# EUROPEAN PATENT APPLICATION

(11) **EP 3 800 248 A2**
(43) Date of publication of application: **07.04.2021**
(21) Application number: 20190783.9
(22) Date of filing: 17.04.2015
(51) Int. Cl.: C12N 9/22, C12N 5/0783, C12N 15/864, C12N 15/90, C12N 15/10, C12N 15/113, C12N 15/63, C07K 14/725, A61P 35/00, C12N 15/11

(54) **CRISPR-CAS-RELATED METHODS, COMPOSITIONS AND COMPONENTS FOR CANCER IMMUNOTHERAPY**

(30) Priority: 18.04.2014 US 201461981636 P; 25.03.2015 US 201562138246 P
(62) Divisional of application: 15721400.8
(71) Applicant: Editas Medicine, Inc., Cambridge, MA 02142 (US)
(72) Inventor: WELSTEAD, G. Grant, Newton, MA 02459 (US); FRIEDLAND, Ari E., Cambridge, MA 02140 (US); MAEDER, Morgan L., Jamaica Plain, MA 02130 (US); BUMCROT, David A., Belmont, MA 02478 (US)
(74) Representative: Forresters IP LLP

(57) **Abstract**

CRISPR/CAS-related compositions and methods for treatment of cancer.

## Description

### CROSS REFERENCE TO RELATED APPLICATIONS

This application claims the benefit of U.S. Provisional Application Serial No. 61/981,636, filed April 18, 2014, and U.S. Provisional Application Serial No. 62/138,246, filed March 25, 2015, the disclosures of which are hereby incorporated by reference.

### SEQUENCE LISTING

The present specification makes reference to a Sequence Listing (submitted electronically as a .txt file named "SEQUENCE LISTING 2011271-0005 EM034PCT.txt" on April 17, 2015). The .txt file was generated on April 17, 2015 and is 11,400,000 bytes in size. The entire contents of the Sequence Listing are hereby incorporated by reference.

### FIELD OF INVENTION

The invention relates to CRISPR/CAS-related methods, compositions and components for editing a target nucleic acid sequence, or modulating expression of a target nucleic acid sequence, and applications thereof in connection with cancer immunotherapy comprising adoptive transfer of engineered T cells or T cell precursors.

### BACKGROUND

Adoptive transfer of genetically engineered T cells has entered clinical testing as a cancer therapeutic modality. Typically, the approach consists of the following steps: 1) obtaining leukocytes from the subject by apheresis; 2) selecting/enriching for T cells; 3) activating the T cells by cytokine treatment; 4) introducing cloned T cell receptor (TCR) genes or a chimeric antigen receptor (CAR) gene by retroviral transduction, lentiviral transduction or electroporation; 5) expanding the T cells by cytokine treatment; 6) conditioning the subject, usually by lymphodepletion; and 7) infusion of the engineered T cells into the subject.

Sources of cloned TCR genes (TRAC and TRBC) include rare T cell populations isolated from individuals with particular malignancies and T cell clones isolated from T cell receptor-humanized mice immunized with specific tumor antigens or tumor cells. Following adoptive transfer, TCR-engineered T cells recognize their cognate antigen peptides presented by major histocompatibility complex (MHC) proteins on the tumor cell surface. Antigen engagement stimulates signal transduction pathways leading to T cell activation and proliferation. Stimulated T cells then mount a cytotoxic anti-tumor cell response, typically involving a secreted complex comprising Granzyme B, perforin and granulysin, inducing tumor cell apoptosis.

Chimeric antigen receptor (CAR) genes encode artificial T cell receptors comprising an extra-cellular tumor antigen binding domain, typically derived from the single-chain antibody variable fragment (scFv) domain of a monoclonal antibody, fused via hinge and transmembrane domains to a cytoplasmic effector domain. The effector domain is typically derived from the CD3-zeta chain of the T cell co-receptor complex, and can also include domains derived from CD28 and/or CD137 receptor proteins. The CAR extra-cellular domain binds the tumor antigen in an MHC-independent manner leading to T cell activation and proliferation, culminating in cytotoxic anti-tumor activity as described for TCR engineered T cells.

To date, at least 15 different tumor antigens have been targeted in clinical trials of engineered T cells. In several trials, anti-tumor activity has been reported. The greatest success has been achieved in hematologic malignancies. For example, adoptive transfer of CAR-T cells engineered to target the B cell antigen, CD19, led to multiple partial and complete responses in subjects with lymphoma, acute lymphoblastic leukemia, acute lymphocytic leukemia and B-cell acute lymphocytic leukemia. In contrast, trials targeting other tumor types, especially solid tumors, including renal cell carcinoma, neuroblastoma, colorectal cancer, breast cancer, ovarian cancer, melanoma, sarcoma and prostate cancer, have been less successful. In many of these trials, very few patients experienced objective responses. Thus, there is a need to improve the anti-tumor efficacy of adoptively transferred engineered T cells.

### SUMMARY

Methods and compositions discussed herein provide for the treatment of cancer using an immunotherapy approach comprising administration of genetically engineered T cells or T cell precursors to a subject. An approach to treat a subject suffering from cancer is to isolate T cells from the subject, genetically modify them to target an antigen expressed by the cancer cells, then re-introduce them into the subject; a process referred to as adoptive cell transfer. Methods to genetically modify T cells include introduction of T cell receptor (TCR) or chimeric antigen receptor (CAR) genes encoding transmembrane TCR or CAR proteins, respectively, which specifically recognize particular cancer antigens. While not wishing to be bound by theory, it is believed that engagement of the tumor-expressed antigen with the antigen binding domain of the TCR or CAR protein initiates a signaling cascade leading to T cell activation, proliferation and, ultimately, destruction of the cancer cell via a cytotoxic immune response (Kershaw et al., 2013 NatRevCancer 13, 525-541).

Adoptive cell transfer utilizing genetically modified T cells has entered clinical testing as a therapeutic for solid and hematologic malignancies. Results to date have been mixed. In hematologic malignancies (especially lymphoma, CLL and ALL), the majority of patients in several Phase 1 and 2 trials exhibited at least a partial response, with some exhibiting complete responses (Kochenderfer, J. N. et al., 2012 Blood 119, 2709-2720). However, in most tumor types (including melanoma, renal cell carcinoma and colorectal cancer), fewer responses have been observed (Johnson, L. A. et al., 2009 Blood 114, 535-546; Lamers, C. H. et al., 2013 Mol. Ther. 21, 904-912; Warren, R. S. et al., 1998 Cancer Gene Ther. 5, S1-S2). Thus, there exists a need to improve the efficacy of adoptive transfer of modified T cells in cancer treatment.

Factors limiting the efficacy of genetically modified T cells as cancer therapeutics include (1) T cell proliferation, e.g., limited proliferation of T cells following adoptive transfer; (2) T cell survival, e.g., induction of T cell apoptosis by factors in the tumor environment; and (3) T cell function, e.g., inhibition of cytotoxic T cell function by inhibitory factors secreted by host immune cells and cancer cells. The methods and compositions described herein address these limitations by modifying the expression of T cell-expressed genes that influence T cell proliferation, survival and/or function.

In an embodiment, methods and compositions discussed herein can be used to affect T cell proliferation (e.g., by inactivating genes that inhibit T cell proliferation). In an embodiment, methods and compositions discussed herein can be used to affect T cell survival (e.g., by inactivating genes mediating T cell apoptosis). In an embodiment, methods and composition discussed herein can be used to affect T cell function (e.g., by inactivating genes encoding immunosuppressive and inhibitory (e.g., anergy-inducing) signaling factors). It is contemplated herein that the methods and compositions described above can be utilized individually or in combination to affect one or more of the factors limiting the efficacy of genetically modified T cells as cancer therapeutics, e.g., T cell proliferation, T cell survival, T cell function, or any combination thereof.

Methods and compositions discussed herein can be used to affect T cell proliferation, survival and/or function by altering one or more T-cell expressed genes, e.g., one or more of *FAS, BID, CTLA4, PDCD1, CBLB, PTPN6, TRAC* and/or *TRBC* genes. In an embodiment, methods and compositions described herein can be used to affect T cell proliferation by altering one or more T-cell expressed genes, e.g., the *CBLB* and/or *PTPN6* gene. In an embodiment, methods and compositions described herein can be used to affect T cell survival by altering one or more T-cell expressed genes, e.g., *FAS* and/or *BID* gene. In an embodiment, methods and compositions described herein can be used to affect T cell function by altering one or more T-cell expressed gene or genes, e.g., *CTLA4* and/or *PDCD1* and/or *TRAC* and/or *TRBC* gene.

In one approach, one or more T-cell expressed genes, e.g., *FAS, BID, CTLA4, PDCD1, CBLB, PTPN6, TRAC* and/or *TRBC* genes, are independently targeted as a targeted knockout or knockdown, e.g., to influence T cell proliferation, survival and/or function. In an embodiment, the approach comprises knocking out or knocking down one T-cell expressed gene (e.g., *FAS, BID, CTLA4, PDCD1, CBLB, PTPN6, TRAC* or *TRBC* gene). In another embodiment, the approach comprises independently knocking out or knocking down two T-cell expressed genes, e.g., two of *FAS, BID, CTLA4, PDCD1, CBLB, PTPN6, TRAC* or *TRBC* genes. In another embodiment, the approach comprises independently knocking out or knocking down three T-cell expressed genes, e.g., three of *FAS, BID, CTLA4, PDCD1, CBLB, PTPN6, TRAC* or *TRBC* genes. In another embodiment, the approach comprises independently knocking out or knocking down four T-cell expressed genes, e.g., four of *FAS, BID, CTLA4, PDCD1, CBLB, PTPN6, TRAC* or *TRBC* genes. In another embodiment, the approach comprises independently knocking out or knocking down five T-cell expressed genes, e.g., five of *FAS, BID, CTLA4, PDCD1, CBLB, PTPN6, TRAC* or *TRBC* genes. In another embodiment, the approach comprises independently knocking out or knocking down six T-cell expressed genes, e.g., six of *FAS, BID, CTLA4, PDCD1, CBLB, PTPN6, TRAC* or *TRBC* genes. In another embodiment, the approach comprises independently knocking out or knocking down seven T-cell expressed genes, e.g., seven of *FAS, BID, CTLA4, PDCD1, CBLB, PTPN6, TRAC* or *TRBC* genes. In another embodiment, the approach comprises independently knocking out or knocking down eight T-cell expressed genes, e.g., each of *FAS, BID, CTLA4, PDCD1, CBLB, PTPN6, TRAC* and *TRBC* genes.

In addition to the genes described above, a number of other T-cell expressed genes may be targeted to affect the efficacy of engineered T cells. These genes include, but are not limited to *TGFBRI, TGFBRII* and *TGFBRIII* (Kershaw et al. 2013 NatRevCancer 13, 525-541). It is contemplated herein that one or more of *TGFBRI, TGFBRII* or *TGFBRIII* gene can be altered either individually or in combination using the methods disclosed herein. It is further contemplated that one or more of *TGFBRI, TGFBRII* or *TGFBRIII* gene can be altered either individually or in combination with any one or more of the eight genes described above (i.e., *FAS, BID, CTLA4, PDCD1, CBLB, PTPN6, TRAC* or *TRBC* gene) using the methods disclosed herein.

In one aspect, methods and compositions discussed herein may be used to alter one or more of the *FAS, BID, CTLA4, PDCD1, CBLB, PTPN6, TRAC* and/or *TRBC* genes to affect T cell proliferation, survival and/or function by targeting the gene, e.g., the non-coding or coding regions, e.g., the promoter region, or a transcribed sequence, e.g., intronic or exonic sequence. In an embodiment, coding sequence, e.g., a coding region, e.g., an early coding region, of the *FAS, BID, CTLA4, PDCD1, CBLB, PTPN6, TRAC* and/or *TRBC* gene, is targeted for alteration and knockout of expression.

In another aspect, the methods and compositions discussed herein may be used to alter the *FAS, BID, CTLA4, PDCD1, CBLB, PTPN6, TRAC* and/or *TRBC gene* to affect T cell proliferation, survival and/or function by targeting the coding sequence of the *FAS, BID, CTLA4, PDCD1, CBLB, PTPN6, TRAC* and/or *TRBC* gene. In an embodiment, the gene, e.g., the coding sequence of the *FAS, BID, CTLA4, PDCD1, CBLB, PTPN6, TRAC* and/or *TRBC* gene, is targeted to knockout one or more *of FAS, BID, CTLA4, PDCD1, CBLB, PTPN6, TRAC* and/or *TRBC* gene, respectively, e.g., to eliminate expression of one or more of *FAS, BID, CTLA4, PDCD1, CBLB, PTPN6, TRAC* and/or *TRBC* gene, e.g., to knockout one or two alleles of one or more of *FAS, BID, CTLA4, PDCD1, CBLB, PTPN6, TRAC* and/or *TRBC* gene, e.g., by induction of an alteration comprising a deletion or mutation in one or more of *FAS, BID, CTLA4, PDCD1, CBLB, PTPN6, TRAC* and/or *TRBC* gene. In an embodiment, the method provides an alteration that comprises an insertion or deletion. As described herein, a targeted knockout approach is mediated by non-homologous end joining (NHEJ) using a CRISPR/Cas system comprising an enzymatically active Cas9 (eaCas9).

In an embodiment, an early coding sequence of the *FAS, BID, CTLA4, PDCD1, CBLB, PTPN6, TRAC* and/or *TRBC* gene is targeted to knockout one or more of *FAS, BID, CTLA4, PDCD1, CBLB, PTPN6, TRAC* and/or *TRBC* gene, respectively. In an embodiment, targeting affects one or two alleles of the *FAS, BID, CTLA4, PDCD1, CBLB, PTPN6, TRAC* and/or *TRBC* gene. In an embodiment, a targeted knockout approach reduces or eliminates expression of functional *FAS, BID, CTLA4, PDCD1, CBLB, PTPN6, TRAC* and/or *TRBC* gene product. In an embodiment, the method provides an alteration that comprises an insertion or deletion.

In another aspect, the methods and compositions discussed herein may be used to alter the *FAS, BID, CTLA4, PDCD1, CBLB, PTPN6, TRAC* and/or *TRBC* gene to affect T cell function by targeting non-coding sequence of the *FAS, BID, CTLA4, PDCD1, CBLB, PTPN6, TRAC* and/or *TRBC* gene, e.g., promoter, an enhancer, an intron, 3'UTR, and/or polyadenylation signal. In an embodiment, the gene, e.g., the non-coding sequence of the *FAS, BID, CTLA4, PDCD1, CBLB, PTPN6, TRAC* and/or *TRBC* gene, is targeted to knockout the gene, e.g., to eliminate expression of the gene, e.g., to knockout one or two alleles of the *FAS, BID, CTLA4, PDCD1, CBLB, PTPN6, TRAC* and/or *TRBC* gene, e.g., by induction of an alteration comprising a deletion or mutation in the *FAS, BID, CTLA4, PDCD1, CBLB, PTPN6, TRAC* and/or *TRBC* gene. In an embodiment, the method provides an alteration that comprises an insertion or deletion.

"T cell target FAS knockout position", as used herein, refers to a position in the *FAS* gene, which if altered by NHEJ-mediated alteration, results in a reduction or elimination of expression of functional *FAS* gene product (e.g., knockout of expression of functional *FAS* gene product). In an embodiment, the position is in the *FAS* gene coding region, e.g., an early coding region.

"T cell target BID knockout position", as used herein, refers to a position in the *BID* gene, which if altered by NHEJ-mediated alteration, results in a reduction or elimination of expression of functional *BID* gene product (e.g., knockout of expression of functional *BID* gene product). In an embodiment, the position is in the *BID* gene coding region, e.g., an early coding region.

"T cell target CTLA4 knockout position", as used herein, refers to a position in the *CTLA4* gene, which if altered by NHEJ-mediated alteration, results in a reduction or elimination of expression of functional *CTLA4* gene product (e.g., knockout of expression of functional *CTLA4* gene product). In an embodiment, the position is in the *CTLA4* gene coding region, e.g., an early coding region.

"T cell target PDCD1 knockout position", as used herein, refers to a position in the *PDCD1* gene, which if altered by NHEJ-mediated alteration, results in a reduction or elimination of expression of functional *PDCD1* gene product (e.g., knockout of expression of functional *PDCD1* gene product). In an embodiment, the position is in the *PDCD1* gene coding region, e.g., an early coding region.

"T cell target CBLB knockout position", as used herein, refers to a position in the *CBLB* gene, which if altered by NHEJ-mediated alteration, results in a reduction or elimination of expression of functional *CBLB* gene product (e.g., knockout of expression of functional *CBLB* gene product). In an embodiment, the position is in the *CBLB* gene coding region, e.g., an early coding region.

"T cell target PTPN6 knockout position", as used herein, refers to a position in the *PTPN6* gene, which if altered by NHEJ-mediated alteration, results in a reduction or elimination of expression of functional *PTPN6* gene product (e.g., knockout of expression of functional *PTPN6* gene product). In an embodiment, the position is in the *PTPN6* gene coding region, e.g., an early coding region.

"T cell target TRAC knockout position", as used herein, refers to a position in the *TRAC* gene, which if altered by NHEJ-mediated alteration, results in a reduction or elimination of expression of functional *TRAC* gene product (e.g., knockout of expression of functional *TRAC* gene product). In an embodiment, the position is in the *TRAC* gene coding region, e.g., an early coding region.

"T cell target TRBC knockout position", as used herein, refers to a position in the *TRBC* gene, which if altered by NHEJ-mediated alteration, results in a reduction or elimination of expression of functional *TRBC* gene product (e.g., knockout of expression of functional *TRBC* gene product). In an embodiment, the position is in the *TRBC* gene coding region, e.g., an early coding region.

In another aspect, methods and compositions discussed herein may be used to alter the expression of one or more T cell-expressed genes, e.g., the *FAS, BID, CTLA4, PDCD1, CBLB,* or *PTPN6* genes, to affect T cell function by targeting a promoter region of the *FAS, BID, CTLA4, PDCD1, CBLB,* or *PTPN6* gene. In an embodiment, the promoter region of the *FAS, BID, CTLA4, PDCD1, CBLB,* or *PTPN6* gene is targeted to knockdown expression of the *FAS, BID, CTLA4, PDCD1, CBLB,* or *PTPN6* gene. A targeted knockdown approach reduces or eliminates expression of the functional *FAS, BID, CTLA4, PDCD1, CBLB,* and/or *PTPN6* gene product. As described herein, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *FAS, BID, CTLA4, PDCD1, CBLB* and/or *PTPN6* genes.

"T cell target FAS knockdown position", as used herein, refers to a position, e.g., in the *FAS* gene, which if targeted by an eiCas9 or an eiCas9 fusion protein described herein, results in reduction or elimination of expression of functional *FAS* gene product. In an embodiment, transcription is reduced or eliminated. In an embodiment, the position is in the *FAS* promoter sequence. In an embodiment, a position in the promoter sequence of the *FAS* gene is targeted by an enzymatically inactive Cas9 (eiCas9) or an eiCas9-fusion protein, as described herein.

"T cell target BID knockdown position", as used herein, refers to a position, e.g., in the *BID* gene, which if targeted by an eiCas9 or an eiCas9 fusion protein described herein, results in reduction or elimination of expression of functional *BID* gene product. In an embodiment, transcription is reduced or eliminated. In an embodiment, the position is in the *BID* promoter sequence. In an embodiment, a position in the promoter sequence of the *BID* gene is targeted by an enzymatically inactive Cas9 (eiCas9) or an eiCas9-fusion protein, as described herein.

"T cell target CTLA4 knockdown position", as used herein, refers to a position, e.g., in the *CTLA4* gene, which if targeted by an eiCas9 or an eiCas9 fusion protein described herein, results in reduction or elimination of expression of functional *CTLA4* gene product. In an embodiment, transcription is reduced or eliminated. In an embodiment, the position is in the *CTLA4* promoter sequence. In an embodiment, a position in the promoter sequence of the *CTLA4* gene is targeted by an enzymatically inactive Cas9 (eiCas9) or an eiCas9-fusion protein, as described herein.

"T cell target PDCD1 knockdown position", as used herein, refers to a position, e.g., in the *PDCD1* gene, which if targeted by an eiCas9 or an eiCas9 fusion protein described herein, results in reduction or elimination of expression of functional *PDCD1* gene product. In an embodiment, transcription is reduced or eliminated. In an embodiment, the position is in the *PDCD1* promoter sequence. In an embodiment, a position in the promoter sequence of the *PDCD1* gene is targeted by an enzymatically inactive Cas9 (eiCas9) or an eiCas9-fusion protein, as described herein.

"T cell target CBLB knockdown position", as used herein, refers to a position, e.g., in the *CBLB* gene, which if targeted by an eiCas9 or an eiCas9 fusion protein described herein, results in reduction or elimination of expression of functional *CBLB* gene product. In an embodiment, transcription is reduced or eliminated. In an embodiment, the position is in the *CBLB* promoter sequence. In an embodiment, a position in the promoter sequence of the *CBLB* gene is targeted by an enzymatically inactive Cas9 (eiCas9) or an eiCas9-fusion protein, as described herein.

"T cell target PTPN6 knockdown position", as used herein, refers to a position, e.g., in the *PTPN6* gene, which if targeted by an eiCas9 or an eiCas9 fusion protein described herein, results in reduction or elimination of expression of functional *PTPN6* gene product. In an embodiment, transcription is reduced or eliminated. In an embodiment, the position is in the *PTPN6* promoter sequence. In an embodiment, a position in the promoter sequence of the *PTPN6* gene is targeted by an enzymatically inactive Cas9 (eiCas9) or an eiCas9-fusion protein, as described herein.

"T cell target FAS position", as used herein, refers to any of the T cell target FAS knockout position and/or T cell target FAS knockdown position, as described herein.

"T cell target BID position", as used herein, refers to any of the T cell target BID knockout position and/or T cell target BID knockdown position, as described herein.

"T cell target CTLA4 position", as used herein, refers to any of the T cell target CTLA4 knockout position and/or T cell target CTLA4 knockdown position, as described herein.

"T cell target PDCD1 position", as used herein, refers to any of the T cell target PDCD 1 knockout position and/or T cell target PDCD1 knockdown position, as described herein.

"T cell target CBLB position", as used herein, refers to any of the T cell target CBLB knockout position and/or T cell target CBLB knockdown position, as described herein.

"T cell target PTPN6 position", as used herein, refers to any of the T cell target PTPN6 knockout position and/or T cell target PTPN6 knockdown position, as described herein.

"T cell target TRAC position", as used herein, refers to any of the T cell target TRAC knockout position, as described herein.

"T cell target TRBC position", as used herein, refers to any of the T cell target TRBC knockout position, as described herein.

"T cell target knockout position", as used herein, refers to any of the T cell target FAS knockout position, T cell target BID knockout position, T cell target CTLA4 knockout position, T cell target PDCD1 knockout position, T cell target CBLB knockout position, T cell target PTPN6 knockout position, T cell target TRAC knockout position, or T cell target TRBC knockout position, as described herein.

"T cell target knockdown position", as used herein, refers to any of the T cell target FAS knockdown position, T cell target BID knockdown position, T cell target CTLA4 knockdown position, T cell target *PDCD1* knockdown position, T cell target CBLB knockdown position, or T cell target PTPN6 knockdown position, as described herein. "T cell target position", as used in herein, refers to any of a T cell target knockout position or T cell target knockdown position, as described herein.

In one aspect, disclosed herein is a gRNA molecule, e.g., an isolated or non-naturally occurring gRNA molecule, comprising a targeting domain which is complementary with a target domain from the *FAS, BID, CTLA4, PDCD1, CBLB, PTPN6, TRAC* or *TRBC* gene.

In an embodiment, the targeting domain of the gRNA molecule is configured to provide a cleavage event, e.g., a double strand break or a single strand break, sufficiently close to a T cell target position in the *FAS, BID, CTLA4, PDCD1, CBLB, PTPN6, TRAC* or *TRBC* gene to allow alteration, e.g., alteration associated with NHEJ, of a T cell target position in the *FAS, BID, CTLA4, PDCD1, CBLB, PTPN6, TRAC* or *TRBC* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450, or 500 nucleotides es of a T cell target position. The break, e.g., a double strand or single strand break, can be positioned upstream or downstream of a T cell target position in the *FAS, BID, CTLA4, PDCD1, CBLB, PTPN6, TRAC* or *TRBC* gene.

In an embodiment, a second gRNA molecule comprising a second targeting domain is configured to provide a cleavage event, e.g., a double strand break or a single strand break, sufficiently close to the T cell target position in the *FAS, BID, CTLA4, PDCD1, CBLB, PTPN6, TRAC* or *TRBC* gene, to allow alteration, e.g., alteration associated with NHEJ, of the T cell target position in the *FAS, BID, CTLA4, PDCD1, CBLB, PTPN6, TRAC* or *TRBC* gene, either alone or in combination with the break positioned by the first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position. In an embodiment, the breaks, e.g., double strand or single strand breaks, are positioned on both sides of a nucleotide of a T cell target position in the *FAS, BID, CTLA4, PDCD1, CBLB, PTPN6, TRAC* or *TRBC* gene. In an embodiment, the breaks, e.g., double strand or single strand breaks, are positioned on one side, e.g., upstream or downstream, of a nucleotide of a T cell target position in the *FAS, BID, CTLA4, PDCD1, CBLB, PTPN6, TRAC* or *TRBC* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450, or 500 nucleotides of a T cell target position. In an embodiment, the first and second gRNA molecules are configured such that, when guiding a Cas9 nickase, a single strand break will be accompanied by an additional single strand break, positioned by a second gRNA, sufficiently close to one another to result in alteration of a T cell target position in the *FAS, BID, CTLA4, PDCD1, CBLB, PTPN6, TRAC* or *TRBC* gene. In an embodiment, the first and second gRNA molecules are configured such that a single strand break positioned by the second gRNA is within 10, 20, 30, 40, or 50 nucleotides of the break positioned by the first gRNA molecule, e.g., when the Cas9 is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position; and the targeting domain of a second gRNA molecule is configured such that a double strand break is positioned downstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position; and the targeting domains of a second and third gRNA molecule are configured such that two single strand breaks are positioned downstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand break can be accompanied by two additional single strand breaks positioned by a third gRNA molecule and a fourth gRNA molecule. For example, the targeting domain of a first and second gRNA molecule are configured such that two single strand breaks are positioned upstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position; and the targeting domains of a third and fourth gRNA molecule are configured such that two single strand breaks are positioned downstream of a T cell target position in the *FAS, BID, CTLA4, PDCD1, CBLB, PTPN6, TRAC* or *TRBC* gene, e.g., within 1, 2, 3, 4, 5, 10, 15, 20, 25, 30, 35, 40, 45, 50, 60, 70, 80, 90, 100, 150, 200, 250, 300, 350, 400, 450, or 500 nucleotides of the target position.

It is contemplated herein that when multiple gRNAs are used to generate (1) two single stranded breaks in close proximity, (2) two double stranded breaks, e.g., flanking a position (e.g., to remove a piece of DNA, e.g., to create a deletion mutation) or to create more than one indel in the gene, e.g., in a coding region, e.g., an early coding region, (3) one double stranded break and two paired nicks flanking a position (e.g., to remove a piece of DNA, e.g., to insert a deletion) or (4) four single stranded breaks, two on each side of a position, that they are targeting the same T cell target position. It is further contemplated herein that multiple gRNAs may be used to target more than one position in the same gene.

When two or more gRNAs are used to position two or more cleavage events, e.g., double strand or single strand breaks, in a target nucleic acid, it is contemplated that the two or more cleavage events may be made by the same or different Cas9 proteins. For example, when two gRNAs are used to position two double stranded breaks in a target nucleic acid, a single Cas9 nuclease may be used to create both double stranded breaks. When two or more gRNAs are used to position two or more single stranded breaks (also refered to as nicks) in a target nucleic acid, a single Cas9 nickase may be used to create the two or more nicks. When two or more gRNAs are used to position at least one double stranded break and at least one single stranded break in a target nucleic acid, two Cas9 proteins may be used, e.g., one Cas9 nuclease and one Cas9 nickase. It is contemplated that when two or more Cas9 proteins are used that the two or more Cas9 proteins may be delivered sequentially to control specificity of a double stranded versus a single stranded break at the desired position in the target nucleic acid. In another embodiment, when two or more Cas9 proteins are used, the Cas9 proteins may be from different species. For example, when two or more gRNAs are used to position at least one double stranded break and at least one single stranded break in a target nucleic acid, the Cas9 nuclease generating the double stranded break may be from one bacterial species and the Cas9 nickase generating the single stranded break may be from a different bacterial species.

When more than one gene is targeted for alteration in a cell, the targeted nucleic acids may be altered, e.g., cleaved, by one or more Cas9 proteins. For example, if two genes are targeted for alteration, e.g., both genes are targeted for knockout, the same or a different Cas9 protein may be used to target each gene. In one embodiment, both genes (or each gene targeted in a cell), are cleaved by a Cas9 nuclease to generate a double stranded break. In another embodiment, both genes (or each gene targeted in a cell), are cleaved by a Cas9 nuclease to generate a double stranded break. In another embodiment, one or more genes in a cell may be altered by cleavage with a Cas9 nuclease and one or more genes in the same cell may be altered by cleavage with a Cas9 nickase. When two or more Cas9 proteins are used to cut a target nucleic acid, e.g., different genes in a cell, the Cas9 proteins may be from different bacterial species. For example, one or more genes in a cell may be altered by cleavage with a Cas9 protein from one bacterial species, and one or more genes in the same cell may be altered by cleavage with a Cas9 protein from a different bacterial species. It is contemplated that when two or more Cas9 proteins from different species are used that they may be delivered at the same time or delivered sequentially to control specificity of cleavage in the desired gene at the desired position in the target nucleic acid.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., Alu repeats, in the target domain. The gRNA molecule may be a first, second, third and/or fourth gRNA molecule.

In an embodiment, the targeting domain of a gRNA molecule is configured to position a cleavage event sufficiently far from a preselected nucleotide, e.g., the nucleotide of a coding region, such that the nucleotide is not be altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In other embodiments, a position in the coding region, e.g., the early coding region, of the *FAS, BID, CTLA4, PDCD1, CBLB, PTPN6, TRAC* or *TRBC* gene is targeted, e.g., for knockout. In an embodiment, the targeting domain comprises a sequence that is the same as, or differs by no more than 1, 2, 3, 4, or 5 nucleotides from, a targeting domain sequence from any one of **Tables 1A-I, Tables 3A-H, Tables 5A-I, Tables 7A-H, Tables 9A-I, Tables 11A-I, Tables 13A-K, Tables 15A-F, Tables 17A-K, Tables 19A-J, Tables 21A-K, Tables 23A-J, Tables 25A-G, Tables 26A-G, Table 27, Table 29, Table 31, or Table 32.**

In an embodiment, the targeting domain is selected from those in **Tables 1A-I.** In other embodiments, the targeting domain is:
GACUGACAUCAACUCCA (SEQ ID NO:8640);
GACUGCGUGCCCUGCCAAGA (SEQ ID NO:8641);
GAGGGCUCACCAGAGGUAGG (SEQ ID NO:8642);
GCGUGCCCUGCCAAGAA (SEQ ID NO:8643);
GCUAGGGACUGCACAGUCAA (SEQ ID NO:8644);
GCUCACCAGAGGUAGGA (SEQ ID NO:8645);
GGACUGCACAGUCAAUG (SEQ ID NO:8646);
GGCUCACCAGAGGUAGG (SEQ ID NO:8647);
GUGACUGACAUCAACUCCAA (SEQ ID NO:8648);
GUGUAACAUACCUGGAGGAC (SEQ ID NO:8649);
GUUGAGACUCAGAACUUGGA (SEQ ID NO:8650); or
GUUUUGUGUAACAUACC (SEQ ID NO:8651).

In an embodiment, the targeting domain is selected from those in **Tables 3A-H.** In other embodiments, the targeting domain is:
GAACACCAGCCGGUCGG (SEQ ID NO:10760);
GAACACCAGCCGGUCGGAGG (SEQ ID NO: 10761);
GAACCGUUGUUGACCUG (SEQ ID NO:10762);
GAAUGUGCAGCGGUGCU (SEQ ID NO:10763);
GACCGUAGCAUCCCUCC (SEQ ID NO:10764);
GAUGGCUCGGUGGGCAGCGA (SEQ ID NO: 10765);
GCACCUCGCCCAGGUCG (SEQ ID NO: 10766);
GCGGAUUCUGUAAGUGGGCU (SEQ ID NO:10767);
GCUCAUCGUAGCCCUCCCAC (SEQ ID NO:10768);
GCUCGGUGGGCAGCGAG (SEQ ID NO:10769);
GGAACCGUUGUUGACCUGAG (SEQ ID NO:10770);
GGACCGUAGCAUCCCUC (SEQ ID NO: 10771);
GGCCCGGAGGGAUGCUA (SEQ ID NO:10772);
GGGCCUGAGGACCGCGU (SEQ ID NO:10773);
GGGUCAUGAUGGCUCGG (SEQ ID NO:10774);
GGUCAUGAUGGCUCGGU (SEQ ID NO:10775);
GGUCCAUGCUGUCCCCGACC (SEQ ID NO:10776);
GGUUCACAGUGUCCCAG (SEQ ID NO:10777);
GUCCAUGCUGUCCCCGACCU (SEQ ID NO:10778);
GUCCCAGUGGCGACCUGGAA (SEQ ID NO:10779);
GUCCCUUUCCAGGUCGCCAC (SEQ ID NO:10780);
GUGCAUCACAAACCUAC (SEQ ID NO:10781);
GUGCGGAUUCUGUAAGU (SEQ ID NO:10782); or
GUGUCCCAGUGGCGACC (SEQ ID NO:10783).

In an embodiment, the targeting domain is selected from those in **Tables 5A-I.** In other embodiments, the targeting domain is:
GGUGACAGUGCUUCGGC (SEQ ID NO:13286);
GUGCGGCAACCUACAUGAUG (SEQ ID NO:13287);
GCGGCAACCUACAUGAU (SEQ ID NO:13288);
GGCCACGUGCAUUGCUAGCA (SEQ ID NO:13289); or
GCUUUAUGGGAGCGGUGUUC (SEQ ID NO: 13290).

In an embodiment, the targeting domain is selected from those in **Tables 7A-H.** In other embodiments, the targeting domain is:
GUCUGGGCGGUGCUACAACU (SEQ ID NO:563);
GCCCAGACGACUGGCCA (SEQ ID NO:564);
GCUCGUGGUGACCGAAG (SEQ ID NO:565);
GUGUCACACAACUGCCCAAC (SEQ ID NO:566);
GCGUGACUUCCACAUGAGCG (SEQ ID NO:567);
GCCCGGCGCAAUGACAG (SEQ ID NO:568);
GGUGACAGGUGCGGCCUCGG (SEQ ID NO:569);
GACAGGUGCGGCCUCGG (SEQ ID NO:570);
GUGGAAGUCACGCCCGU (SEQ ID NO:571);
GAAGCUCUCCGAUGUGU (SEQ ID NO:572); or
GUCACCACGAGCAGGGC (SEQ ID NO:573).

In an embodiment, the targeting domain is selected from those in **Tables 9A-I.** In other embodiments, the targeting domain is:
GGGTATTATTGATGCTATTC (SEQ ID NO:6119);
GATCTGCGGCAGCTTGCTTA (SEQ ID NO:6120);
GCAGGACCGTGGAGAAGACT (SEQ ID NO:6121);
GGATTTCCTCCTCGACCACC (SEQ ID NO:6122);
GATTTCCTCCTCGACCACCA (SEQ ID NO:6123);
GTACTCATTCTCACTGAGTT (SEQ ID NO:6124);
GTATGAAGAACAGTCAC (SEQ ID NO:6125); or
GCGGCAGCTTGCTTAGG (SEQ ID NO:6126).

In an embodiment, the targeting domain is selected from those in **Tables 11A-I.** In other embodiments, the targeting domain is:
GGUUUCACCGAGACCUCAGU (SEQ ID NO:3749);
GGGAUCAGGUGACCCAUAUU (SEQ ID NO:3750);
GUUUGCGACUCUGACAGAGC (SEQ ID NO:3751);
GAGUACUACACUCAGCAGCA (SEQ ID NO:3752);
GUGGGAUCGGAGCAGUUCAG (SEQ ID NO:3753);
GUCACCCUGGUUCUUGCGAC (SEQ ID NO:3754);
GGACACCUCGGCCCUUGAGC (SEQ ID NO:3755);
GCCCAGUCGCAAGAACC (SEQ ID NO:3756);
GUCCUGCAGGACCGCGA (SEQ ID NO:3757);
GAAAGCACGAAGUCUCC (SEQ ID NO:3758);
GGAUCGGAGCAGUUCAG (SEQ ID NO:3759);
GAUCGGAGCAGUUCAGC (SEQ ID NO:3760);
GGAUGAUGGUGCCGUCG (SEQ ID NO:3761); or
GUUCUGGAUCCGAAUAU (SEQ ID NO:3762).

In an embodiment, when the T cell target knockout position is the *FAS* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 1A-F** or **Tables 13A-K.**

In an embodiment, when the T cell target knockout position is the *BID* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 3A-H** or **Tables 15A-F.**

In an embodiment, when the T cell target knockout position is the *CTLA4* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 5A-I** or **Tables 17A-K.**

In an embodiment, when the T cell target knockout position is the *PDCD1* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 7A-H, Tables 19A-J, Table 31** or **Table 32.**

In other embodiments, the targeting domain is:
GGUGCUACAACUGGGCUGGCGGC (SEQ ID NO:481);
GUGCUACAACUGGGCUGGCGGC (SEQ ID NO:482);
GCUACAACUGGGCUGGCGGC (SEQ ID NO:483);
GACGACUGGCCAGGGCGCCUG (SEQ ID NO:484);
GACUGGCCAGGGCGCCUG (SEQ ID NO:485);
GCAUGCCUGGAGCAGCCCCAC (SEQ ID NO:486);
GGGGGGUUCCAGGGCCUGUCUG (SEQ ID NO:487);
GGGGGUUCCAGGGCCUGUCUG (SEQ ID NO:488);
GGGGUUCCAGGGCCUGUCUG (SEQ TD NO:489);
GAGAGCCUGCGGGCAGAGCU (SEQ ID NO:490);
GAGCCUGCGGGCAGAGCU (SEQ ID NO:491);
GGGGGGGUUCCAGGGCCUGUCUG (SEQ ID NO:492);
GCAGGGCUGGGGAGAAGGUGG (SEQ ID NO:493);
GGGCUGGGGAGAAGGUGG (SEQ ID NO:494);
GAGCAGGGCUGGGGAGAAGGUGG (SEQ ID NO:495);
GGCCGCCCACGACACCAACCAC (SEQ ID NO:496);
GCCGCCCACGACACCAACCAC (SEQ ID NO:497);
GCCCACGACACCAACCAC (SEQ ID NO:498);
GGUUUGGAACUGGCCGGCUGGC (SEQ ID NO:499);
GUUUGGAACUGGCCGGCUGGC (SEQ ID NO:500);
GGGCAGCCUGGUGCUGCUAGU (SEQ ID NO:501);
GGCAGCCUGGUGCUGCUAGU (SEQ ID NO:502);
GCAGCCUGGUGCUGCUAGU (SEQ ID NO:503);
GGCUGGCCUGGGUGAGGGGC (SEQ ID NO:504);
GGGUUUGGAACUGGCCGGCUGGC (SEQ ID NO:505);
GCUGGGCAGCCUGGUGCUGCUAGU (SEQ ID NO:506);
GGCCGGCUGGCCUGGGUGAGGGGC (SEQ ID NO:507);
GUCUGGGCGGUGCUACAACU (SEQ ID NO:508);
GGCGCCCUGGCCAGUCGUCU (SEQ ID NO:509);
GGGCGGUGCUACAACUGGGC (SEQ ID NO:510);
GGCCAGGAUGGUUCUUAGGU (SEQ ID NO:511);
GGAUGGUUCUUAGGUAGGUG (SEQ ID NO:512);
GAAGGCGGCACUCUGGU (SEQ ID NO:513);
GCCCUGGCCAGUCGUCU (SEQ ID NO:514);
GCCCAGACGACUGGCCA (SEQ ID NO:515);
GAUGGUUCUUAGGUAGG (SEQ ID NO:516);
GGCGGAGGUGAGCGGAA (SEQ ID NO:517);
GGAAGGGAAACUGUCCC (SEQ ID NO:518);
GCGUGACUUCCACAUGAGCG (SEQ ID NO:519);
GCCCUGCUCGUGGUGACCGA (SEQ ID NO:520);
GGUGCCGCUGUCAUUGCGCC (SEQ ID NO:521);
GCUCUCUUUGAUCUGCGCCU (SEQ ID NO:522);
GAAGGUGGCGUUGUCCCCUU (SEQ TD NO:523);
GUGUCACACAACUGCCCAAC (SEQ ID NO:524);
GCUUGUCCGUCUGGUUGCUG (SEQ ID NO:525);
GAUCUGCGCCUUGGGGGCCA (SEQ ID NO:526);
GGGCCCUGACCACGCUCAUG (SEQ ID NO:527);
GCAGUUGUGUGACACGGAAG (SEQ ID NO:528);
GACAGCGGCACCUACCUCUG (SEQ ID NO:529);
GUGGAAGUCACGCCCGU (SEQ ID NO:530);
GCUCGUGGUGACCGAAG (SEQ ID NO:531);
GCCCGGCGCAAUGACAG (SEQ ID NO:532);
GCCGCUGUCAUUGCGCC (SEQ ID NO:533);
GCGGCACCUACCUCUGU (SEQ ID NO:534);
GGUGGCGUUGUCCCCUU (SEQ ID NO:535);
GAAGCUCUCCGAUGUGU (SEQ ID NO:536);
GUUGUGUGACACGGAAG (SEQ ID NO:537);
GCUGGCUGCGGUCCUCG (SEQ ID NO:538);
GCUGCGGUCCUCGGGGA (SEQ ID NO:539);
GACGUUACCUCGUGCGGCCC (SEQ ID NO:540);
GGUGUCGUGGGCGGCCUGCU (SEQ ID NO:541);
GCCCACGACACCAACCACCA (SEQ ID NO:542);
GCAGCCUGGUGCUGCUAGUC (SEQ ID NO:543);
GGUGCUGCUAGUCUGGGUCC (SEQ ID NO:544);
GGACCCAGACUAGCAGCACC (SEQ ID NO:545);
GGCACUUCUGCCCUUCUCUC (SEQ ID NO:546);
GGGCGGCCUGCUGGGCAGCC (SEQ ID NO:547);
GCAGCACCAGGCUGCCCAGC (SEQ ID NO:548);
GCUGGCCUGGGUGAGGGGCU (SEQ ID NO:549);
GUUACCUCGUGCGGCCC (SEQ ID NO:550);
GUCGUGGGCGGCCUGCU (SEQ ID NO:551);
GUUUGGAACUGGCCGGC (SEQ ID NO:552);
GGCCGUCAUCUGCUCCC (SEQ ID NO:553);
GCUGCUAGUCUGGGUCC (SEQ ID NO:554); or
GCCUGGUGCUGCUAGUC (SEQ ID NO:555).

Moreover, in another embodiment, when the T cell target knockout position is the *PDCD1* coding region, e.g., an early coding region, e.g., to create one or more indels, in the target nucleic acid sequence, each guide RNA is independently selected from one of **Tables 7A-H, Tables 19A-J, Table 31** or **Table 32** so that the break is generated with over 10% efficiency.

In an embodiment, the targeting domain is:
GCAUGCCUGGAGCAGCCCCAC (SEQ ID NO:486);
GGGGGUUCCAGGGCCUGUCUG (SEQ ID NO:488);
GCAGGGCUGGGGAGAAGGUGG (SEQ ID NO:493);
GGGCUGGGGAGAAGGUGG (SEQ ID NO:494);
GCCGCCCACGACACCAACCAC (SEQ ID NO:497);
GCCCACGACACCAACCAC (SEQ ID NO:498);
GUUUGGAACUGGCCGGCUGGC (SEQ ID NO:500);
GUCUGGGCGGUGCUACAACU (SEQ ID NO:508);
GGCGCCCUGGCCAGUCGUCU (SEQ ID NO:509);
GGCCAGGAUGGUUCUUAGGU (SEQ ID NO:511);
GGAUGGUUCUUAGGUAGGUG (SEQ ID NO:512);
GAAGGCGGCACUCUGGU (SEQ ID NO:513);
GCCCAGACGACUGGCCA (SEQ ID NO:515);
GGCGGAGGUGAGCGGAA (SEQ ID NO:517);
GCCCUGCUCGUGGUGACCGA (SEQ ID NO:520):
GCUCUCUUUGAUCUGCGCCU (SEQ ID NO:522);
GAAGGUGGCGUUGUCCCCUU (SEQ ID NO:523);
GCUUGUCCGUCUGGUUGCUG (SEQ ID NO:525);
GAUCUGCGCCUUGGGGGCCA (SEQ ID NO:526);
GGGCCCUGACCACGCUCAUG (SEQ ID NO:527);
GCAGUUGUGUGACACGGAAG (SEQ ID NO:528);
GACAGCGGCACCUACCUCUG (SEQ ID NO:529);
GCCGCUGUCAUUGCGCC (SEQ ID NO:533);
GGUGGCGUUGUCCCCUU (SEQ ID NO:535);
GAAGCUCUCCGAUGUGU (SEQ ID NO:536);
GUUGUGUGACACGGAAG (SEQ ID NO:537);
GCUGGCUGCGGUCCUCG (SEQ ID NO:538);
GCUGCGGUCCUCGGGGA (SEQ ID NO:539);
GACGUUACCUCGUGCGGCCC (SEQ ID NO:540);
GGUGCUGCUAGUCUGGGUCC (SEQ TD NO:544);
GGACCCAGACUAGCAGCACC (SEQ ID NO:545); or
GGCACUUCUGCCCUUCUCUC (SEQ ID NO:546).

In an embodiment, when the T cell target knockout position is the *CBLB* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 9A-I** or **Tables 21A-K.**

In an embodiment, when the T cell target knockout position is the *PTPN6* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 11A-I** or **Tables 23A-J.**

In an embodiment, when the T cell target knockout position is the *TRAC* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 25A-G** or **Table 29.**

In other embodiments, the targeting domain is:
UCUCUCAGCUGGUACACGGC (SEQ ID NO:449);
UGGAUUUAGAGUCUCUCAGC (SEQ ID NO:450);
ACACGGCAGGGUCAGGGUUC (SEQ ID NO:451);
GAGAAUCAAAAUCGGUGAAU (SEQ ID NO:452);
GCUGGUACACGGCAGGGUCA (SEQ ID NO:453);
CUCAGCUGGUACACGGC (SEQ ID NO:454);
UGGUACACGGCAGGGUC (SEQ ID NO:455);
GCUAGACAUGAGGUCUA (SEQ ID NO:456);
GUCAGAUUUGUUGCUCC (SEQ ID NO:457);
UCAGCUGGUACACGGCA (SEQ ID NO:458);
GCAGACAGACUUGUCAC (SEQ ID NO:459);
GGUACACGGCAGGGUCA (SEQ ID NO:460);
CUUCAAGAGCAACAGUGCUG (SEQ ID NO:461);
AGAGCAACAGUGCUGUGGCC (SEQ ID NO:462);
AAAGUCAGAUUUGUUGCUCC (SEQ ID NO:463);
ACAAAACUGUGCUAGACAUG (SEQ ID NO:464);
AAACUGUGCUAGACAUG (SEQ ID NO:465);
UGUGCUAGACAUGAGGUCUA (SEQ ID NO:466);
GGCUGGGGAAGAAGGUGUCUUC (SEQ ID NO:467);
GCUGGGGAAGAAGGUGUCUUC (SEQ ID NO:468);
GGGGAAGAAGGUGUCUUC (SEQ ID NO:469);
GUUUUGUCUGUGAUAUACACAU (SEQ ID NO:470);
GGCAGACAGACUUGUCACUGGAUU (SEQ ID NO:471);
GCAGACAGACUUGUCACUGGAUU(SEQ ID NO:472);
GACAGACUUGUCACUGGAUU (SEQ ID NO:473);
GUGAAUAGGCAGACAGACUUGUCA (SEQ ID NO:474);
GAAUAGGCAGACAGACUUGUCA (SEQ ID NO:475);
GAGUCUCUCAGCUGGUACACGG (SEQ ID NO:476);
GUCUCUCAGCUGGUACACGG (SEQ ID NO:477); or
GGUACACGGCAGGGUCAGGGUU (SEQ ID NO:478).

In another embodiment, when the T cell target knockout position is the *TRAC* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 25A-G** or **Table 29** so that the break is generated with over 10% efficiency.

In an embodiment, the targeting domain is:
GGCUGGGGAAGAAGGUGUCUUC (SEQ ID NO:467);
GCUGGGGAAGAAGGUGUCUUC (SEQ ID NO:468);
GUUUUGUCUGUGAUAUACACAU (SEQ ID NO:470);
GGCAGACAGACUUGUCACUGGAUU (SEQ ID NO:471);
GCAGACAGACUUGUCACUGGAUU (SEQ ID NO:472);
GACAGACUUGUCACUGGAUU (SEQ ID NO:473);
GUGAAUAGGCAGACAGACUUGUCA (SEQ ID NO:474);
GAAUAGGCAGACAGACUUGUCA (SEQ ID NO:475);
GAGUCUCUCAGCUGGUACACGG (SEQ ID NO:476);
GUCUCUCAGCUGGUACACGG (SEQ ID NO:477);
GGUACACGGCAGGGUCAGGGUU (SEQ ID NO:478);
GUACACGGCAGGGUCAGGGUU (SEQ ID NO:49451);
GAGAAUCAAAAUCGGUGAAU (SEQ ID NO:452);
GCUAGACAUGAGGUCUA (SEQ ID NO:456);
GCAGACAGACUUGUCAC (SEQ ID NO:459);
GGUACACGGCAGGGUCA (SEQ ID NO:460);
CUUCAAGAGCAACAGUGCUG (SEQ ID NO:461);
AGAGCAACAGUGCUGUGGCC (SEQ ID NO:462);
ACAAAACUGUGCUAGACAUG (SEQ ID NO:464);
AAACUGUGCUAGACAUG (SEQ ID NO:465); or
UGUGCUAGACAUGAGGUCUA (SEQ ID NO:466).

In an embodiment, when the T cell target knockout position is the *TRBC* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 26A-G** or **Table 27.**

In other embodiments, the targeting domain is:
CACCCAGAUCGUCAGCGCCG (SEQ ID NO:387);
CAAACACAGCGACCUCGGGU (SEQ ID NO:388);
UGACGAGUGGACCCAGGAUA (SEQ ID NO:389);
GGCUCUCGGAGAAUGACGAG (SEQ ID NO:390);
GGCCUCGGCGCUGACGAUCU (SEQ ID NO:391);
GAAAAACGUGUUCCCACCCG (SEQ ID NO:392);
AUGACGAGUGGACCCAGGAU (SEQ ID NO:393);
AGUCCAGUUCUACGGGCUCU (SEQ ID NO:394);
CGCUGUCAAGUCCAGUUCUA (SEQ ID NO:395);
AUCGUCAGCGCCGAGGCCUG (SEQ ID NO:396);
UCAAACACAGCGACCUCGGG (SEQ ID NO:397);
CGUAGAACUGGACUUGACAG (SEQ ID NO:398);
AGGCCUCGGCGCUGACGAUC (SEQ ID NO:399);
UGACAGCGGAAGUGGUUGCG (SEQ ID NO:400);
UUGACAGCGGAAGUGGUUGC (SEQ ID NO:401);
UCUCCGAGAGCCCGUAGAAC (SEQ ID NO:402);
CGGGUGGGAACACGUUUUUC (SEQ ID NO:403);
GACAGGUUUGGCCCUAUCCU (SEQ ID NO:404);
GAUCGUCAGCGCCGAGGCCU (SEQ ID NO:405);
GGCUCAAACACAGCGACCUC (SEQ ID NO:406);
UGAGGGUCUCGGCCACCUUC (SEQ TD NO:407);
AGGCUUCUACCCCGACCACG (SEQ ID NO:408);
CCGACCACGUGGAGCUGAGC (SEQ ID NO:409);
UGACAGGUUUGGCCCUAUCC (SEQ ID NO:410);
CUUGACAGCGGAAGUGGUUG (SEQ ID NO:411);
AGAUCGUCAGCGCCGAGGCC (SEQ ID NO:412);
GCGCUGACGAUCUGGGUGAC (SEQ ID NO:413);
UGAGGGCGGGCUGCUCCUUG (SEQ ID NO:414);
GUUGCGGGGGUUCUGCCAGA (SEQ ID NO:415);
AGCUCAGCUCCACGUGGUCG (SEQ ID NO:416);
GCGGCUGCUCAGGCAGUAUC (SEQ ID NO:417);
GCGGGGGUUCUGCCAGAAGG (SEQ ID NO:418);
UGGCUCAAACACAGCGACCU (SEQ ID NO:419);
ACUGGACUUGACAGCGGAAG (SEQ ID NO:420);
GACAGCGGAAGUGGUUGCGG (SEQ ID NO:421);
GCUGUCAAGUCCAGUUCUAC (SEQ ID NO:422);
GUAUCUGGAGUCAUUGAGGG (SEQ ID NO:423);
CUCGGCGCUGACGAUCU (SEQ ID NO:424);
CCUCGGCGCUGACGAUC (SEQ ID NO:425);
CCGAGAGCCCGUAGAAC (SEQ ID NO:426);
CCAGAUCGUCAGCGCCG (SEQ ID NO:427);
GAAUGACGAGUGGACCC (SEQ ID NO:428);
GGGUGACAGGUUUGGCCCUAUC (SEQ ID NO:429);
GGUGACAGGUUUGGCCCUAUC (SEQ ID NO:430);
GUGACAGGUUUGGCCCUAUC (SEQ ID NO:431);
GACAGGUUUGGCCCUAUC (SEQ ID NO:432);
GAUACUGCCUGAGCAGCCGCCU (SEQ ID NO:433);
GACCACGUGGAGCUGAGCUGGUGG (SEQ ID NO:434);
GUGGAGCUGAGCUGGUGG (SEQ ID NO:435);
GGGCGGGCUGCUCCUUGAGGGGCU (SEQ ID NO:436);
GGCGGGCUGCUCCUUGAGGGGCU (SEQ ID NO:437);
GCGGGCUGCUCCUUGAGGGGCU (SEQ ID NO:438);
GGGCUGCUCCUUGAGGGGCU (SEQ ID NO:439);
GGCUGCUCCUUGAGGGGCU (SEQ ID NO:440);
GCUGCUCCUUGAGGGGCU (SEQ ID NO:441);
GGUGAAUGGGAAGGAGGUGCACAG (SEQ ID NO:442);
GUGAAUGGGAAGGAGGUGCACAG (SEQ ID NO:443); or
GAAUGGGAAGGAGGUGCACAG (SEQ ID NO:444).

In an embodiment, when the T cell target knockout position is the *TRBC* coding region, e.g., an early coding region, and more than one gRNA is used to position breaks, e.g., two single stranded breaks or two double stranded breaks, or a combination of single strand and double strand breaks, e.g., to create one or more indels, in the target nucleic acid sequence, each guide RNA is independently selected from one of **Tables 26A-G** or **Table 27** so that the break is generated with over 10% efficiency.

In an embodiment, the targeting domain is:
GGGUGACAGGUUUGGCCCUAUC (SEQ ID NO:429);
GGUGACAGGUUUGGCCCUAUC (SEQ ID NO:430);
GUGACAGGUUUGGCCCUAUC (SEQ ID NO:431);
GAUACUGCCUGAGCAGCCGCCU (SEQ ID NO:433);
GACCACGUGGAGCUGAGCUGGUGG (SEQ ID NO:434);
GGGCGGGCUGCUCCUUGAGGGGCU (SEQ ID NO:436);
GGCGGGCUGCUCCUUGAGGGGCU (SEQ ID NO:437);
GCGGGCUGCUCCUUGAGGGGCU (SEQ ID NO:438);
GGGCUGCUCCUUGAGGGGCU (SEQ ID NO:439);
GGCUGCUCCUUGAGGGGCU (SEQ ID NO:440);
GCUGCUCCUUGAGGGGCU (SEQ ID NO:441);
GGUGAAUGGGAAGGAGGUGCACAG (SEQ ID NO:442);
GUGAAUGGGAAGGAGGUGCACAG (SEQ ID NO:443);
GAAUGGGAAGGAGGUGCACAG (SEQ ID NO:444).
UGACGAGUGGACCCAGGAUA (SEQ ID NO:389);
GGCUCUCGGAGAAUGACGAG (SEQ ID NO:390);
AGGCUUCUACCCCGACCACG (SEQ ID NO:408);
UGAGGGCGGGCUGCUCCUUG (SEQ ID NO:414); or
GACAGCGGAAGUGGUUGCGG (SEQ ID NO:421).

In an embodiment, the targeting domain of the gRNA molecule is configured to target an enzymatically inactive Cas9 (eiCas9) or an eiCas9 fusion protein (e.g., an eiCas9 fused to a transcription repressor domain), sufficiently close to a T cell knockdown target position to reduce, decrease or repress expression of the *FAS, BID, CTLA4, PDCD1, CBLB,* or *PTPN6* gene. In an embodiment, the targeting domain is configured to target the promoter region of the *FAS, BID, CTLA4, PDCD1, CBLB,* or *PTPN6* gene to block transcription initation, binding of one or more transcription enhancers or activators, and/or RNA polymerase. One or more gRNA may be used to target an eiCas9 to the promoter region of the *FAS, BID, CTLA4, PDCD1, CBLB,* or *PTPN6* gene.

In an embodiment, when the *FAS, BID, CTLA4, PDCD1, CBLB,* or *PTPN6* promoter region is targeted, e.g., for knockdown, the targeting domain can comprise a sequence that is the same as, or differs by no more than 1, 2, 3, 4, or 5 nucleotides from, a targeting domain sequence from any one of **Tables 2A-I, Tables 4A-I, Tables 6A-I, Tables 8A-H, Tables 10A-I, Tables 12A-I, Tables 14A-K, Table 16A-K, Tables 18A-K, Tables 20A-J, Tables 22A-K, or Tables 24A-K.** In an embodiment, the targeting domain is selected from those in **Tables 2A-I.** In other embodiments, the targeting domain is:
GAAGCGGUUUACGAGUGACU (SEQ ID NO:9236);
GUUGGUGGACCCGCUCAGUA (SEQ ID NO:9237);
GGACCCGCUCAGUACGGAGU (SEQ ID NO:9238);
GACCCGCUCAGUACGGAGUU (SEQ ID NO:9239);
GGGAAGCUCUUUCACUUCGG (SEQ ID NO:9240);
GGAUUGCUCAACAACCAUGC (SEQ ID NO:9241);
GGCUUACCCCGUCUUAGUCC (SEQ ID NO:9242);
GCUUACCCCGUCUUAGUCCC (SEQ ID NO:9243);
GUCCCGGGGAUAGGCAAAGU (SEQ ID NO:9244);
GCGGGACGCGUGCGGGAUUG (SEQ ID NO:9245);
GCCUAUCCCCGGGACUAAGA (SEQ ID NO:9246);
GACGGGGUAAGCCUCCACCC (SEQ ID NO:9247);
GAGCGGGUCCACCAACCCGC (SEQ ID NO:9248);
GACGAGCUCACGAAAAGCCC (SEQ ID NO:9249);
GAGCUCACGAAAAGCCCCGG (SEQ ID NO:9250);
GAAAAGCCCCGGUGGUCAGG (SEQ ID NO:9251);
GCUCCAUUGAUUCAGCAACU (SEQ ID NO:9252);
GUCAGGGUUCGUUGCACAAA (SEQ ID NO:9253);
GUCCCGGGGCGUUCCUGCAG (SEQ ID NO:9254);
GUGGCUGGCAUGCUCACUUC (SEQ ID NO:9255);
GCUUCUGCUGUAGUUCAACC (SEQ ID NO:9256);
GUAGUUCAACCUGGGAAGUU (SEQ ID NO:9257);
GACUUGCGGGGCAUUUGACU (SEQ ID NO:9258);
GAGAGGCUCACAGACGUUUC (SEQ ID NO:9259);
GCGCCAUAGGGCUCUGAAAA (SEQ ID NO:9260);
GCUGGGGCUAUGCGAUU (SEQ TD NO:9261);
GCGCAAGAGUGACACAC (SEQ ID NO:9262);
GUGUUCAAAGACGCUUC (SEQ ID NO:9263);
GCGGUUUACGAGUGACU (SEQ ID NO:9264);
GGUGGACCCGCUCAGUA (SEQ ID NO:9265);
GGGAAGCUCUUUCACUU (SEQ ID NO:9266);
GUCUUAGUCCCGGGGAU (SEQ ID NO:9267);
GGAUAGGCAAAGUGGGG (SEQ ID NO:9268);
GGACGCGUGCGGGAUUG (SEQ ID NO:9269);
GGCGCACGCGGGCACCU (SEQ ID NO:9270);
GGGGUAAGCCUCCACCC (SEQ ID NO:9271);
GCGGGUCCACCAACCCG (SEQ ID NO:9272);
GAGCUCACGAAAAGCCC (SEQ ID NO:9273);
GAAAAGCCCCGGUGGUC (SEQ ID NO:9274);
GCAACUUGGCCUGCGCG (SEQ ID NO:9275);
GGGUUCGUUGCACAAAU (SEQ ID NO:9276);
GCUGGCAUGCUCACUUC (SEQ ID NO:9277);
GUUCAACCUGGGAAGUU (SEQ ID NO:9278); or
GCCAUAGGGCUCUGAAA (SEQ ID NO:9279).

In an embodiment, the targeting domain is selected from those in **Tables 4A-I.** In other embodiments, the targeting domain is:
GAAAACCCGCGGGCCUGGCC (SEQ ID NO:11440);
GAAAGACCACGGUGUGGGCG (SEQ ID NO:11441);
GAAGCGCGCGGGGCGCCAUA (SEQ ID NO:11442);
GAAUGACCCCAGGGGACCCU (SEQ ID NO:11443);
GACUACCCGCUUCCUCCUUA (SEQ ID NO:11444);
GAGGAGAAAACCCGCGGGCC (SEQ ID NO:11445);
GAGGGUCGGGUCCGCCUCGG (SEQ ID NO:11446);
GCGCGCGGGGCGCCAUAAGG (SEQ ID NO:11447);
GCGGAUUCUGUAAGUGGGCU (SEQ ID NO:11448);
GCGGGAGAGACCCGGCCCUC (SEQ ID NO:11449);
GCUCCUCCCGAGCGGAGCUC (SEQ ID NO:11450);
GCUCGGCGCGUACCCCGGGA (SEQ ID NO:11451);
GCUGAGGGUCGGGUCCGCCU (SEQ ID NO:11452);
GGAUUCCGAUCCGCCGGCAA (SEQ TD NO:11453);
GGCCACCCUUGCCGGCGGAU (SEQ ID NO:11454);
GGCUCGGCGCGUACCCCGGG (SEQ ID NO:11455);
GGGAUUCCGAUCCGCCGGCA (SEQ ID NO:11456);
GGGCGCCAUAAGGAGGAAGC (SEQ ID NO:11457);
GGGCGGGGAUUCCGAUCCGC (SEQ ID NO:11458);
GGUCAGAGCGGCCGCUUACU (SEQ ID NO:11459);
GGUCGGGUCCGCCUCGGAGG (SEQ ID NO:11460);
GUCAGAGCGGCCGCUUACUG (SEQ ID NO:11461);
GUCGACCGUGUCCGCGCGCC (SEQ ID NO:11462);
GUCGGGUCCGCCUCGGAGGC (SEQ ID NO:11463);
GUCUCCCAGGCGCGCGGACA (SEQ ID NO:11464);
GUGCAGGCCACCCUUGCCGG (SEQ ID NO:11465);
GAAUGUGCAGCGGUGCU (SEQ ID NO:11466);
GACCGUGUCCGCGCGCC (SEQ ID NO:114(7);
GAGAAAACCCGCGGGCC (SEQ ID NO:11468);
GAGGGUCGGGUCCGCCU (SEQ ID NO:11469);
GAUUCUGUAAGUGGGCU (SEQ ID NO:11470);
GCAGGUGUCUGCGGUGC (SEQ ID NO:11471);
GCCGGUCCAGCCGCAGA (SEQ ID NO:11472);
GCGCGCGGGGCGCCAUA (SEQ ID NO:11473);
GCUCGGGAGGAGCCGGC (SEQ ID NO:11474);
GGAGAGACCCGGCCCUC (SEQ ID NO:11475);
GGAGAGGAGAAAACCCG (SEQ ID NO:11476);
GGAUUCUGUAAGUGGGC (SEQ ID NO:11477);
GGCUCGGCGCGUACCCC (SEQ ID NO:11478);
GGGCGAGCCCCAGUAAG (SEQ ID NO:11479);
GGGCUCGGCGCGUACCC (SEQ ID NO:11480);
GGGUCCGCCUCGGAGGC (SEQ ID NO:11481);
GGGUUUUCUCCUCUCCG (SEQ ID NO:11482);
GGUCAUGAUGGCUCGGU (SEQ ID NO:11483);
GGUCCGCCUCGGAGGCG (SEQ ID NO:11484);
GGUCGGGUCCGCCUCGG (SEQ ID NO:11485); or
GUGCGGAUUCUGUAAGU (SEQ ID NO:11486).

In an embodiment, the targeting domain is selected from those in **Tables 6A-I.** In other embodiments, the targeting domain is:
GCUCAGAACAGAGCGCC (SEQ ID NO:13746);
GAGCGCCAGGUAUUUAGUAG (SEQ ID NO:13747);
GCGCCAGGUAUUUAGUA (SEQ ID NO:13748);
GCUUUAUGGGAGCGGUGUUC (SEQ ID NO: 13749);
GGAGCGGUGUUCAGGUCUUC (SEQ ID NO: 13750);
GCGGUGUUCAGGUCUUC (SEQ ID NO:13751); or
UUAUGGGAGCGGUGUUC (SEQ ID NO:13752).

In an embodiment, the targeting domain is selected from those in **Tables 8A-H.** In other embodiments, the targeting domain is:
GCCCCAAGGACCGGCUGAGA (SEQ ID NO:1517);
GUCUGGGCGGUGCUACAACU (SEQ ID NO:1518);
GGGCGGUGCUACAACUGGGC (SEQ ID NO:1519);
GCGUGUUUCUGUAGAAUGAC (SEQ ID NO:1520);
GCCCAGACGACUGGCCA (SEQ ID NO:1521);
GGGCAGCUCGAACUCCU (SEQ ID NO:1522); or
GGGCGGGAUAUGGAAAG (SEQ ID NO: 1523).

In an embodiment, the targeting domain is selected from those in **Tables 10A-I.** In other embodiments, the targeting domain is:
GAAAGTGAAGTTTCTGCGTA (SEQ ID NO:7635);
GATAGGACCTTCAGTTGTTC (SEQ ID NO:7636);
GGTTTTGCTCTAATCGATGG (SEQ ID NO:7637);
GGGTATTATTGATGCTATTC (SEQ ID NO:7638);
GCAGGACCGTGGAGAAGACT (SEQ ID NO:7639);
GTGTATGGCCATAGAATGCT (SEQ ID NO:7640);
GATCTGCGGCAGCTTGCTTA (SEQ ID NO:7641);
GGATTTCCTCCTCGACCACC (SEQ ID NO:7642);
GATTTCCTCCTCGACCACCA (SEQ ID NO:7643);
GCTCACGTTTCAAGTAGCTA (SEQ ID NO:7644);
GAAGGTCCTATCATACATTT (SEQ ID NO:7645);
GATGAATCCGGAACTTT (SEQ ID NO:7646);
GGTTTTGCTCTAATCGA (SEQ ID NO:7647);
GGAGGAAATCCCCGAAA (SEQ ID NO:7648);
GGACCGTGGAGAAGACT (SEQ ID NO:7649);
GCGGCAGCTTGCTTAGG (SEQ ID NO:7650); or
GGTCCTATCATACATTT (SEQ ID NO:7651).

In an embodiment, the targeting domain is selected from those in **Tables 12A-I.** In other embodiments, the targeting domain is:
GGCCCUUUGGUUUCCGCCUA (SEQ ID NO:4701);
GGGUAAGUCCCGGGCACCAU (SEQ ID NO:4702);
GGUAAGUCCCGGGCACCAUC (SEQ ID NO:4703);
GCCUGCUGUACUCCCGCUUU (SEQ ID NO:4704);
GGGGGAAACCUCUCCGUAGG (SEQ ID NO:4705);
GGUCCUCACACCUUGCGGGA (SEQ ID NO:4706);
GGCGGAAACCAAAGGGCCUA (SEQ ID NO:4707);
GGACGUGGGAGGGCCUUCGC (SEQ ID NO:4708);
GCACUCACGUUCACACACGU (SEQ ID NO:4709);
GUCCUCACACCUUGCGGGAA (SEQ ID NO:4710);
GGUUUCCGCCUACGGAG (SEQ ID NO:4711);
GUCCCGUGGGUAAGUCC (SEQ ID NO:4712);
GGAAACCUCUCCGUAGG (SEQ ID NO:4713);
GACCGGAAACAGGCGCA (SEQ ID NO:4714);
GGGGGAAACCUCUCCGU (SEQ ID NO:4715); or
GGACCGGAAACAGGCGC (SEQ ID NO:4716.

In an embodiment, when the T cell target knockdown position is the *FAS* promoter region and more than one gRNA is used to position an eiCas9 or an eiCas9-fusion protein (e.g., an eiCas9-transcription repressor domain fusion protein), in the target nucleic acid sequence, each guide RNA is independently selected from one of **Tables 2A-I** or **Tables 14A-K.**

In an embodiment, when the T cell target knockdown position is the *BID* promoter region and more than one gRNA is used to position an eiCas9 or an eiCas9-fusion protein (e.g., an eiCas9-transcription repressor domain fusion protein), in the target nucleic acid sequence, each guide RNA is independently selected from one of **Tables 4A-I** or **Tables 16A-K.**

In an embodiment, when the T cell target knockdown position is the *CTLA4* promoter region and more than one gRNA is used to position an eiCas9 or an eiCas9-fusion protein (e.g., an eiCas9-transcription repressor domain fusion protein), in the target nucleic acid sequence, each guide RNA is independently selected from one of **Tables 6A-I** or **Tables 18A-K.**

In an embodiment, when the T cell target knockdown position is the *PDCD1* promoter region and more than one gRNA is used to position an eiCas9 or an eiCas9-fusion protein (e.g., an eiCas9-transcription repressor domain fusion protein), in the target nucleic acid sequence, each guide RNA is independently selected from one of **Tables 8A-H** or **Tables 20A-J.**

In an embodiment, when the T cell target knockdown position is the *CBLB* promoter region and more than one gRNA is used to position an eiCas9 or an eiCas9-fusion protein (e.g., an eiCas9-transcription repressor domain fusion protein), in the target nucleic acid sequence, each guide RNA is independently selected from one of **Tables 10A-I** or **Tables 22A-K.**

In an embodiment, when the T cell target knockdown position is the *PTPD6* promoter region and more than one gRNA is used to position an eiCas9 or an eiCas9-fusion protein (e.g., an eiCas9-transcription repressor domain fusion protein), in the target nucleic acid sequence, each guide RNA is independently selected from one of **Tables 12A-I** or **Tables 24A-K.**

In an embodiment, the gRNA, e.g., a gRNA comprising a targeting domain, which is complementary with the *FAS, BID, CTLA4, PDCD1, CBLB, PTPN6, TRAC* or *TRBC* gene, is a modular gRNA. In other embodiments, the gRNA is a unimolecular or chimeric gRNA.

In an embodiment, the targeting domain which is complementary with the *FAS, BID, CTLA4, PDCD1, CBLB, PTPN6, TRAC* or *TRBC* gene comprises 16 or more nucleotides in length. In an embodiment, the targeting domain which is complementary with a target domain from the *FAS, BID, CTLA4, PDCD1, CBLB, PTPN6, TRAC* or *TRBC* gene is 16 nucleotides or more in length. In an embodiment, the targeting domain is 16 nucleotides in length. In an embodiment, the targeting domain is 17 nucleotides in length. In an embodiment, the targeting domain is 18 nucleotides in length. In an embodiment, the targeting domain is 19 nucleotides in length. In an embodiment, the targeting domain is 20 nucleotides in length. In an embodiment, the targeting domain is 21 nucleotides in length. In an embodiment, the targeting domain is 22 nucleotides in length. In an embodiment, the targeting domain is 23 nucleotides in length. In an embodiment, the targeting domain is 24 nucleotides in length. In an embodiment, the targeting domain is 25 nucleotides in length. In an embodiment, the targeting domain is 26 nucleotides in length. A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain of equal to or greater than 16,17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 30 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 35 nucleotides in length; and a targeting domain of equal to or greater than 16, 17, 18, 19, 20, 21, 22, 23, 24, 25 or 26 nucleotides in length.

In another embodiment, a gRNA comprises a linking domain of no more than 25 nucleotides in length; a proximal and tail domain, that taken together, are at least 40 nucleotides in length; and a targeting domain of equal to or greater than 16, 17, 18, 19, 20, 21, 22, 23, 24, 25 or 26 nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in some embodiments, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

In an embodiment, the eaCas9 molecule catalyzes a double strand break.

In some embodiments, the eaCas9 molecule comprises HNH-like domain cleavage activity but has no, or no significant, N-terminal RuvC-like domain cleavage activity. In this case, the eaCas9 molecule is an HNH-like domain nickase, e.g., the eaCas9 molecule comprises a mutation at D10, e.g., D10A. In other embodiments, the eaCas9 molecule comprises N-terminal RuvC-like domain cleavage activity but has no, or no significant, HNH-like domain cleavage activity. In an embodiment, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., N863A.

In an embodiment, a single strand break is formed in the strand of the target nucleic acid to which the targeting domain of the gRNA is complementary. In another embodiment, a single strand break is formed in the strand of the target nucleic acid other than the strand to which the targeting domain of the gRNA is complementary.

In another aspect, disclosed herein is a nucleic acid, e.g., an isolated or non-naturally occurring nucleic acid, e.g., DNA, that comprises (a) a sequence that encodes a gRNA molecule comprising a targeting domain that is complementary with a target domain, e.g., with a T cell target position, in *FAS, BID, CTLA4, PDCD1, CBLB, PTPN6, TRAC* or *TRBC* gene, as disclosed herein.

In an embodiment, the nucleic acid encodes a gRNA molecule, e.g., a first gRNA molecule, comprising a targeting domain configured to provide a cleavage event, e.g., a double strand break or a single strand break, sufficiently close to a T cell target position in the *FAS, BID, CTLA4, PDCD1, CBLB, PTPN6, TRAC* or *TRBC* gene to allow alteration, e.g., alteration associated with NHEJ, of the a T cell target position in the *FAS, BID, CTLA4, PDCD1, CBLB, PTPN6, TRAC* or *TRBC* gene, respectively.

In an embodiment, the nucleic acid encodes a gRNA molecule, e.g., a first gRNA molecule, comprising a targeting domain configured to target an enzymatically inactive Cas9 (eiCas9) or an eiCas9 fusion protein (e.g., an eiCas9 fused to a transcription repressor domain), sufficiently close to a T cell knockdown target position to reduce, decrease or repress expression of the *FAS, BID, CTLA4, PDCD1, CBLB,* or *PTPN6* gene.

In an embodiment, the nucleic acid encodes a gRNA molecule, e.g., the first gRNA molecule, comprising a targeting domain comprising a sequence that is the same as, or differs by no more than 1, 2, 3, 4, or 5 nucleotides from, a targeting domain sequence from any one of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table** 27, **Table 29, Table 31, or Table 32.** In an embodiment, the nucleic acid encodes a gRNA molecule comprising a targeting domain selected from those in **Tables 1A-I, Tables 2A-I, Tables 3A-H, Tables 4A-I, Tables 5A-I, Tables 6A-I, Tables 7AH, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.**

In an embodiment, the nucleic acid encodes a modular gRNA, e.g., one or more nucleic acids encode a modular gRNA. In other embodiments, the nucleic acid encodes a chimeric gRNA. The nucleic acid may encode a gRNA, e.g., the first gRNA molecule, comprising a targeting domain comprising 16 nucleotides or more in length. The nucleic acid may encode a gRNA, e.g., the first gRNA molecule, comprising a targeting domain comprising 16 nucleotides or more in length. In an embodiment, the nucleic acid encodes a gRNA, e.g., the first gRNA molecule, comprising a targeting domain that is 17 nucleotides in length. In other embodiments, the nucleic acid encodes a gRNA, e.g., the first gRNA molecule, comprising a targeting domain that is 18 nucleotides in length. In still other embodiments, the nucleic acid encodes a gRNA, e.g., the first gRNA molecule, comprising a targeting domain that is 19 nucleotides in length. In still other embodiments, the nucleic acid encodes a gRNA, e.g., the first gRNA molecule, comprising a targeting domain that is 20 nucleotides in length. In still other embodiments, the nucleic acid encodes a gRNA, e.g., the first gRNA molecule, comprising a targeting domain that is 21 nucleotides in length. In still other embodiments, the nucleic acid encodes a gRNA, e.g., the first gRNA molecule, comprising a targeting domain that is 22 nucleotides in length. In still other embodiments, the nucleic acid encodes a gRNA, e.g., the first gRNA molecule, comprising a targeting domain that is 23 nucleotides in length. In still other embodiments, the nucleic acid encodes a gRNA, e.g., the first gRNA molecule, comprising a targeting domain that is 24 nucleotides in length. In still other embodiments, the nucleic acid encodes a gRNA, e.g., the first gRNA molecule, comprising a targeting domain that is 25 nucleotides in length. In still other embodiments, the nucleic acid encodes a gRNA, e.g., the first gRNA molecule, comprising a targeting domain that is 26 nucleotides in length.

In an embodiment, a nucleic acid encodes a gRNA comprising from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a nucleic acid encodes a gRNA e.g., the first gRNA molecule, comprising a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In an embodiment, a nucleic acid encodes a gRNA e.g., the first gRNA molecule, comprising a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In an embodiment, a nucleic acid encodes a gRNA e.g., the first gRNA molecule, comprising a linking domain of no more than 25 nucleotides in length; a proximal and tail domain, that taken together, are at least 35 nucleotides in length; and a targeting domain equal to or greater than 16, 17, 18, 19, 20, 21, 22, 23, 24, 25 or 26 nucleotides in length.

In an embodiment, a nucleic acid encodes a gRNA comprising e.g., the first gRNA molecule, a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

In an embodiment, a nucleic acid comprises (a) a sequence that encodes a gRNA molecule, e.g., the first gRNA molecule, comprising a targeting domain that is complementary with a target domain in the *FAS, BID, CTLA4, PDCD1, CBLB, PTPN6, TRAC* or *TRBC* gene, as disclosed herein, and further comprising (b) a sequence that encodes a Cas9 molecule.

In an embodiment, the nucleic acid further comprises a sequence encoding a governing gRNA molecule.

The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in some embodiments, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

A nucleic acid disclosed herein may comprise (a) a sequence that encodes a gRNA molecule comprising a targeting domain that is complementary with a target domain in the *FAS, BID, CTLA4, PDCD1, CBLB, PTPN6, TRAC* or *TRBC* gene, as disclosed herein; (b) a sequence that encodes a Cas9 molecule; and further comprises (c) (i) a sequence that encodes a second gRNA molecule described herein having a targeting domain that is complementary to a second target domain of the *FAS, BID, CTLA4, PDCD1, CBLB, PTPN6, TRAC* or *TRBC* gene, and optionally, (ii) a sequence that encodes a third gRNA molecule described herein having a targeting domain that is complementary to a third target domain of the *FAS, BID, CTLA4, PDCD1, CBLB, PTPN6, TRAC* or *TRBC* gene; and optionally, (iii) a sequence that encodes a fourth gRNA molecule described herein having a targeting domain that is complementary to a fourth target domain of the *FAS, BID, CTLA4, PDCD1, CBLB, PTPN6, TRAC* or *TRBC* gene.

In an embodiment, a nucleic acid encodes a second gRNA molecule comprising a targeting domain configured to provide a cleavage event, e.g., a double strand break or a single strand break, sufficiently close to a T cell target position in the *FAS, BID, CTLA4, PDCD1, CBLB, PTPN6, TRAC* or *TRBC* gene, to allow alteration, e.g., alteration associated with NHEJ, of the a T cell target position in the *FAS, BID, CTLA4, PDCD1, CBLB, PTPN6, TRAC* or *TRBC* gene, either alone or in combination with the break positioned by the first gRNA molecule.

In an embodiment, the nucleic acid encodes a second gRNA molecule comprising a targeting domain configured to target an enzymatically inactive Cas9 (eiCas9) or an eiCas9 fusion protein (e.g., an eiCas9 fused to a transcription repressor domain), sufficiently close to a T cell knockdown target position to reduce, decrease or repress expression of the *FAS, BID, CTLA4, PDCD1, CBLB,* or *PTPN6* gene.

In an embodiment, a nucleic acid encodes a third gRNA molecule comprising a targeting domain configured to provide a cleavage event, e.g., a double strand break or a single strand break, sufficiently close to a T cell target position in the *FAS, BID, CTLA4, PDCD1, CBLB, PTPN6, TRAC* or *TRBC* gene to allow alteration, e.g., alteration associated with NHEJ, of a T cell target position in the *FAS, BID, CTLA4, PDCD1, CBLB, PTPN6, TRAC* or *TRBC* gene, either alone or in combination with the break positioned by the first and/or second gRNA molecule.

In an embodiment, the nucleic acid encodes a third gRNA molecule comprising a targeting domain configured to target an enzymatically inactive Cas9 (eiCas9) or an eiCas9 fusion protein (e.g., an eiCas9 fused to a transcription repressor domain), sufficiently close to a T cell knockdown target position to reduce, decrease or repress expression of the *FAS, BID, CTLA4, PDCD1, CBLB,* or *PTPN6* gene.

In an embodiment, a nucleic acid encodes a fourth gRNA molecule comprising a targeting domain configured to provide a cleavage event, e.g., a double strand break or a single strand break, sufficiently close to a T cell target position in the *FAS, BID, CTLA4, PDCD1, CBLB, PTPN6, TRAC* or *TRBC* gene to allow alteration, e.g., alteration associated with NHEJ, of a T cell target position in the *FAS, BID, CTLA4, PDCD1, CBLB, PTPN6, TRAC* or *TRBC* gene, either alone or in combination with the break positioned by the first gRNA molecule, the second gRNA molecule and the third gRNA molecule.

In an embodiment, the nucleic acid encodes a fourth gRNA molecule comprising a targeting domain configured to target an enzymatically inactive Cas9 (eiCas9) or an eiCas9 fusion protein (e.g., an eiCas9 fused to a transcription repressor domain), sufficiently close to a T cell knockdown target position to reduce, decrease or repress expression of the *FAS, BID, CTLA4, PDCD1, CBLB,* or *PTPN6* gene.

In an embodiment, the nucleic acid encodes a second gRNA molecule. The second gRNA is selected to target the same T cell target position as the first gRNA molecule. Optionally, the nucleic acid may encode a third gRNA, and further optionally, the nucleic acid may encode a fourth gRNA molecule. In an embodiment, the third gRNA molecule and the fourth gRNA molecule are selected to target the same T cell target position as the first and second gRNA molecules.

In an embodiment, the nucleic acid encodes a second gRNA molecule comprising a targeting domain comprising a sequence that is the same as, or differs by no more than 1, 2, 3, 4, or 5 nucleotides from, a targeting domain sequence from one of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, or Tables 26A-G Tables 26A-G, Table 27, Table 29, Table 31, or Table 32.** In an embodiment, the nucleic acid encodes a second gRNA molecule comprising a targeting domain selected from those in **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, or Tables 26A-G, Table 27, Table 29, Table 31, or Table 32.** In an embodiment, when a third or fourth gRNA molecule are present, the third and fourth gRNA molecules may comprise a targeting domain comprising a sequence that is the same as, or differs by no more than 1, 2, 3, 4, or 5 nucleotides from, a targeting domain sequence independently selected from one of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.** In a further embodiment, when a third or fourth gRNA molecule are present, the third and fourth gRNA molecules may comprise a targeting domain independently selected from those in **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.**

In an embodiment, the nucleic acid encodes a second gRNA which is a modular gRNA, e.g., wherein one or more nucleic acid molecules encode a modular gRNA. In other embodiments, the nucleic acid encoding a second gRNA is a chimeric gRNA. In other embodiments, when a nucleic acid encodes a third or fourth gRNA, the third and fourth gRNA may be a modular gRNA or a chimeric gRNA. When multiple gRNAs are used, any combination of modular or chimeric gRNAs may be used.

A nucleic acid may encode a second, a third, and/or a fourth gRNA comprising a targeting domain comprising 16 nucleotides or more in length. In an embodiment, the nucleic acid encodes a second gRNA comprising a targeting domain that is 16 nucleotides in length. In an embodiment, the nucleic acid encodes a second gRNA comprising a targeting domain that is 17 nucleotides in length. In other embodiments, the nucleic acid encodes a second gRNA comprising a targeting domain that is 18 nucleotides in length. In still other embodiments, the nucleic acid encodes a second gRNA comprising a targeting domain that is 19 nucleotides in length. In still other embodiments, the nucleic acid encodes a second gRNA comprising a targeting domain that is 20 nucleotides in length. In still other embodiments, the nucleic acid encodes a second gRNA comprising a targeting domain that is 21 nucleotides in length. In still other embodiments, the nucleic acid encodes a second gRNA comprising a targeting domain that is 22 nucleotides in length. In still other embodiments, the nucleic acid encodes a second gRNA comprising a targeting domain that is 23 nucleotides in length. In still other embodiments, the nucleic acid encodes a second gRNA comprising a targeting domain that is 24 nucleotides in length. In still other embodiments, the nucleic acid encodes a second gRNA comprising a targeting domain that is 25 nucleotides in length. In still other embodiments, the nucleic acid encodes a second gRNA comprising a targeting domain that is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, a nucleic acid encodes a second, a third, and/or a fourth gRNA comprising from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a nucleic acid encodes a second, a third, and/or a fourth gRNA comprising a linking domain of no more than 25 nucleotides in length; a proximal and tail domain, that taken together, are at least 20 nucleotides in length; and a targeting domain of equal to or greater than 16, 17, 18, 19, 20, 21, 22, 23, 24, 25 or 26 nucleotides in length.

In an embodiment, a nucleic acid encodes a second, a third, and/or a fourth gRNA comprising a linking domain of no more than 25 nucleotides in length; a proximal and tail domain, that taken together, are at least 30 nucleotides in length; and a targeting domain of equal to or greater than 16, 17, 18, 19, 20, 21, 22, 23, 24, 25 or 26 nucleotides in length.

In an embodiment, a nucleic acid encodes a second, a third, and/or a fourth gRNA comprising a linking domain of no more than 25 nucleotides in length; a proximal and tail domain, that taken together, are at least 35 nucleotides in length; and a targeting domain of equal to or greater than 16, 17, 18, 19, 20, 21, 22, 23, 24, 25 or 26 nucleotides in length.

In an embodiment, a nucleic acid encodes a second, a third, and/or a fourth gRNA comprising a linking domain of no more than 25 nucleotides in length; a proximal and tail domain, that taken together, are at least 40 nucleotides in length; and a targeting domain of equal to or greater than 16, 17, 18, 19, 20, 21, 22, 23, 24, 25 or 26 nucleotides in length.

As described above, a nucleic acid may comprise (a) a sequence encoding a gRNA molecule comprising a targeting domain that is complementary with a target domain in the *FAS, BID, CTLA4, PDCD1, CBLB, PTPN6, TRAC* or *TRBC* gene, and (b) a sequence encoding a Cas9 molecule. In some embodiments, (a) and (b) are present on the same nucleic acid molecule, e.g., the same vector, e.g., the same viral vector, e.g., the same adeno-associated virus (AAV) vector. In an embodiment, the nucleic acid molecule is an AAV vector. Exemplary AAV vectors that may be used in any of the described compositions and methods include an AAV1 vector, a modified AAV1 vector, an AAV2 vector, a modified AAV2 vector, an AAV3 vector, an AAV4 vector, a modified AAV4 vector, an AAV5 vector, a modified AAV5 vector, a modified AAV3 vector, an AAV6 vector, a modified AAV6 vector, an AAV7 vector, a modified AAV7 vector, an AAV8 vector,an AAV9 vector an AAV.rh10 vector, a modified AAV.rh10 vector, an AAV.rh32/33 vector, a modified AAV.rh32/33 vector, an AAV.rh43 vector, a modified AAV.rh43 vector, an AAV.rh64R1 vector, and a modified AAV.rh64R1 vector. In other embodiments, (a) is present on a first nucleic acid molecule, e.g. a first vector, e.g., a first viral vector, e.g., a first AAV vector; and (b) is present on a second nucleic acid molecule, e.g., a second vector, e.g., a second vector, e.g., a second AAV vector. The first and second nucleic acid molecules may be AAV vectors.

In other embodiments, the nucleic acid may further comprise (c) (i) a sequence that encodes a second gRNA molecule as described herein. In some embodiments, the nucleic acid comprises (a), (b) and (c)(i). Each of (a) and (c)(i) may be present on the same nucleic acid molecule, e.g., the same vector, e.g., the same viral vector, e.g., the same adeno-associated virus (AAV) vector. In an embodiment, the nucleic acid molecule is an AAV vector.

In other embodiments, (a) and (c)(i) are on different vectors. For example, (a) may be present on a first nucleic acid molecule, e.g. a first vector, e.g., a first viral vector, e.g., a first AAV vector; and (c)(i) may be present on a second nucleic acid molecule, e.g., a second vector, e.g., a second vector, e.g., a second AAV vector. In an embodiment, the first and second nucleic acid molecules are AAV vectors.

In another embodiment, each of (a), (b), and (c)(i) are present on the same nucleic acid molecule, e.g., the same vector, e.g., the same viral vector, e.g., an AAV vector. In an embodiment, the nucleic acid molecule is an AAV vector. In an alternate embodiment, one of (a), (b), and (c)(i) is encoded on a first nucleic acid molecule, e.g., a first vector, e.g., a first viral vector, e.g., a first AAV vector; and a second and third of (a), (b), and (c)(i) is encoded on a second nucleic acid molecule, e.g., a second vector, e.g., a second vector, e.g., a second AAV vector. The first and second nucleic acid molecule may be AAV vectors.

In an embodiment, (a) is present on a first nucleic acid molecule, e.g., a first vector, e.g., a first viral vector, a first AAV vector; and (b) and (c)(i) are present on a second nucleic acid molecule, e.g., a second vector, e.g., a second vector, e.g., a second AAV vector. The first and second nucleic acid molecule may be AAV vectors.

In other embodiments, (b) is present on a first nucleic acid molecule, e.g., a first vector, e.g., a first viral vector, e.g., a first AAV vector; and (a) and (c)(i) are present on a second nucleic acid molecule, e.g., a second vector, e.g., a second vector, e.g., a second AAV vector. The first and second nucleic acid molecule may be AAV vectors.

In other embodiments, (c)(i) is present on a first nucleic acid molecule, e.g., a first vector, e.g., a first viral vector, e.g., a first AAV vector; and (b) and (a) are present on a second nucleic acid molecule, e.g., a second vector, e.g., a second vector, e.g., a second AAV vector. The first and second nucleic acid molecule may be AAV vectors.

In another embodiment, each of (a), (b) and (c)(i) are present on different nucleic acid molecules, e.g., different vectors, e.g., different viral vectors, e.g., different AAV vector. For example, (a) may be on a first nucleic acid molecule, (b) on a second nucleic acid molecule, and (c)(i) on a third nucleic acid molecule. The first, second and third nucleic acid molecule may be AAV vectors.

In another embodiment, when a third and/or fourth gRNA molecule are present, each of (a), (b), (c)(i), (c) (ii) and (c)(iii) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), (c)(i), (c) (ii) and (c)(iii) may be present on the different nucleic acid molecules, e.g., different vectors, e.g., the different viral vectors, e.g., different AAV vectors. In further embodiments, each of (a), (b), (c)(i), (c) (ii) and (c)(iii) may be present on more than one nucleic acid molecule, but fewer than five nucleic acid molecules, e.g., AAV vectors.

The nucleic acids described herein may comprise a promoter operably linked to the sequence that encodes the gRNA molecule of (a), e.g., a promoter described herein. The nucleic acid may further comprise a second promoter operably linked to the sequence that encodes the second, third and/or fourth gRNA molecule of (c), e.g., a promoter described herein. The promoter and second promoter differ from one another. In some embodiments, the promoter and second promoter are the same.

The nucleic acids described herein may further comprise a promoter operably linked to the sequence that encodes the Cas9 molecule of (b), e.g., a promoter described herein.

In another aspect, disclosed herein is a composition comprising (a) a gRNA molecule comprising a targeting domain that is complementary with a target domain in the *FAS, BID, CTLA4, PDCD1, CBLB, PTPN6, TRAC* or *TRBC* gene, as described herein. The composition of (a) may further comprise (b) a Cas9 molecule, e.g., a Cas9 molecule as described herein. A composition of (a) and (b) may further comprise (c) a second, third and/or fourth gRNA molecule, e.g., a second, third and/or fourth gRNA molecule described herein. In an embodiment, a composition may comprise at least two gRNA molecules to target two or more of *FAS, BID, CTLA4, PDCD1, CBLB, PTPN6, TRAC* and *TRBC* genes. In an embodiment, the composition further comprises a governing gRNA molecule, or a nucleic acid that encodes a governing gRNA molecule.

In another aspect, disclosed herein is a method of altering a cell, e.g., altering the structure, e.g., altering the sequence, of a target nucleic acid of a cell, comprising contacting the cell with: (a) a gRNA that targets the *FAS, BID, CTLA4, PDCD1, CBLB, PTPN6, TRAC* or *TRBC* gene, e.g., a gRNA as described herein; (b) a Cas9 molecule, e.g., a Cas9 molecule as described herein; and optionally, (c) a second, third and/or fourth gRNA that targets *FAS, BID, CTLA4, PDCD1, CBLB, PTPN6, TRAC* or *TRBC* gene, e.g., a gRNA, as described herein. In an embodiment, the method further comprises, introducing into the cell, a governing gRNA molecule, or a nucleic acid that encodes a governing gRNA molecule, into the cell.

In some embodiments, the method comprises contacting the cell with (a) and (b).

In some embodiments, the method comprises contacting the cell with (a), (b), and (c).

The gRNA of (a) and optionally (c) may be independently selected from any of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32,** or a gRNA that differs by no more than 1, 2, 3, 4, or 5 nucleotides from, a targeting domain sequence independently selected from any of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.**

In an embodiment, the method of altering a cell, e.g., altering the structure, e.g., altering the sequence, of a target nucleic acid of a cell, comprising altering two or more of *FAS, BID, CTLA4, PDCD1, CBLB, PTPN6, TRAC* or *TRBC* genes. When two or more genes are altered in a cell, the cell is contacted with: (a) gRNAs that target two or more of the *FAS, BID, CTLA4, PDCD1, CBLB, PTPN6, TRAC* or *TRBC* genes, e.g., two or more of the gRNA as described herein; (b) a Cas9 molecule, e.g., a Cas9 molecule as described herein; and optionally, (c) second, third and/or fourth gRNAs that respectively targets the two or more genes selected in (a), e.g., a gRNA, as described herein. In an embodiment, the method further comprises, introducing into the cell, a governing gRNA molecule, or a nucleic acid that encodes a governing gRNA molecule, into the cell.

In an embodiment, the method of altering a cell comprises altering two or more of *FAS, BID, CTLA4, PDCD1, CBLB, PTPN6, TRAC* or *TRBC* genes.

In an embodiment, the method of altering a cell comprises altering three or more of *FAS, BID, CTLA4, PDCD1, CBLB, PTPN6, TRAC* or *TRBC* genes.

In an embodiment, the method of altering a cell comprises altering four or more of *FAS, BID, CTLA4, PDCD1, CBLB, PTPN6, TRAC* or *TRBC* genes.

In an embodiment, the method of altering a cell comprises altering five or more of *FAS, BID, CTLA4, PDCD1, CBLB, PTPN6, TRAC* or *TRBC* genes.

In an embodiment, the method of altering a cell comprises altering six or more of *FAS, BID, CTLA4, PDCD1, CBLB, PTPN6, TRAC* or *TRBC* genes.

In an embodiment, the method of altering a cell comprises altering seven or more of *FAS, BID, CTLA4, PDCD1, CBLB, PTPN6, TRAC* or *TRBC* genes.

In an embodiment, the method of altering a cell comprises altering each of *FAS, BID, CTLA4, PDCD1, CBLB, PTPN6, TRAC* or *TRBC* genes.

In some embodiments, the method comprises contacting a cell from a subject suffering from cancer. The cell may be from a subject that would benefit from having a mutation at a T cell target position.

In some embodiments, the cell being contacted in the disclosed method is a T cell. The contacting may be performed *ex vivo* and the contacted cell may be returned to the subject's body after the contacting step. The T cell may be an engineered T cell, e.g., an engineered CAR (chimeric antigen receptor) T cell or an engineered TCR (T-cell receptor) T cell. A T cell may engineered to express a TCR or a CAR prior to, after, or at the same time as introducing a T cell target position mutation in one or more of the *FAS, BID, CTLA4, PDCD1, CBLB, PTPN6, TRAC* or *TRBC* gene.

In some embodiments, the method of altering a cell as described herein comprises acquiring knowledge of the sequence of a T cell target position in the cell, prior to the contacting step. Acquiring knowledge of the sequence of a T cell target position in the cell may be by sequencing the *FAS, BID, CTLA4, PDCD1, CBLB, PTPN6, TRAC* or *TRBC* gene, or a portion of the *FAS, BID, CTLA4, PDCD1, CBLB, PTPN6, TRAC* or *TRBC* gene.

In some embodiments, the contacting step of the method comprises contacting the cell with a nucleic acid, e.g., a vector, e.g., an AAV vector, e.g., an AAV vector described herein, that expresses at least one of (a), (b), and (c). In some embodiments, the contacting step of the method comprises contacting the cell with a nucleic acid, e.g., a vector, e.g., an AAV vector, that expresses each of (a), (b), and (c). In another embodiment, the contacting step of the method comprises delivering to the cell a Cas9 molecule of (b) and a nucleic acid which encodes a gRNA (a) and optionally, a second gRNA (c)(i) (and further optionally, a third gRNA (c)(iv) and/or fourth gRNA (c)(iii).

In some embodiments, the contacting step of the method comprises contacting the cell with a nucleic acid, e.g., a vector, e.g., an AAV vector, that expresses at least one of (a), (b), and (c). In some embodiments, the contacting step of the method comprises contacting the cell with a nucleic acid, e.g., a vector, e.g., an AAV vector, that expresses each of (a), (b), and (c). In an embodiment, contacting comprises contacting the cell with a nucleic acid, e.g., a vector, e.g., an AAV vector an AAV1 vector, a modified AAV1 vector, an AAV2 vector, a modified AAV2 vector, an AAV3 vector, a modified AAV3 vector, an AAV4 vector, a modified AAV4 vector, an AAV5 vector, a modified AAV5 vector, an AAV6 vector, a modified AAV6 vector, an AAV7 vector, a modified AAV7 vector, an AAV8 vector, an AAV9 vector, an AAV.rh10 vector, a modified AAV.rh10 vector, an AAV.rh32/33 vector, a modified AAV.rh32/33 vector, an AAV.rh43vector, a modified AAV.rh43vector, an AAV.rh64R1vector, and a modified AAV.rh64R1vector.

In an embodiment, contacting comprises delivering to the cell a Cas9 molecule of (b), as a protein or an mRNA, and a nucleic acid which encodes (a) and optionally (c).

In an embodiment, contacting comprises delivering to the cell a Cas9 molecule of (b), as a protein or an mRNA, the gRNA of (a), as an RNA, and optionally the second gRNA of (c), as an RNA.

In an embodiment, contacting comprises delivering to the cell a gRNA of (a) as an RNA, optionally the second gRNA of (c) as an RNA, and a nucleic acid that encodes the Cas9 molecule of (b).

In another aspect, disclosed herein is a method of treating a subject suffering from cancer, e.g., altering the structure, e.g., sequence, of a target nucleic acid of the subject, comprising contacting the subject (or a cell from the subject) with:
(a) a gRNA that targets the *FAS, BID, CTLA4, PDCD1, CBLB, PTPN6, TRAC* or *TRBC* gene, e.g., a gRNA disclosed herein;
(b) a Cas9 molecule, e.g., a Cas9 molecule disclosed herein; and
optionally, (c)(i) a second gRNA that targets the *FAS, BID, CTLA4, PDCD1, CBLB, PTPN6, TRAC* or *TRBC* gene, e.g., a second gRNA disclosed herein, and
further optionally, (c)(ii) a third gRNA, and still further optionally, (c)(iii) a fourth gRNA that target the *FAS, BID, CTLA4, PDCD1, CBLB, PTPN6, TRAC* or *TRBC* gene, e.g., a third and fourth gRNA disclosed herein. In an embodiment, the method further comprises, introducing into the subject, or a cell of the subject, a governing gRNA molecule, or a nucleic acid that encodes a governing gRNA molecule.

In some embodiments, contacting comprises contacting with (a) and (b).

In some embodiments, contacting comprises contacting with (a), (b), and (c)(i).

In some embodiments, contacting comprises contacting with (a), (b), (c)(i) and (c)(ii).

In some embodiments, contacting comprises contacting with (a), (b), (c)(i), (c)(ii) and (c)(iii).

The gRNA of (a) or (c) (e.g., (c)(i), (c)(ii), or (c)(iii) may be independently selected from any of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32,** or a gRNA that differs by no more than 1, 2, 3, 4, or 5 nucleotides from, a targeting domain sequence independently selected from any of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.**

In an embodiment, the method of treating a subject suffering from cancer, comprises altering two or more of *FAS, BID, CTLA4, PDCD1, CBLB, PTPN6, TRAC* or *TRBC* genes, e.g., altering the structure, e.g., sequence, of two or more target nucleic acids of the subject (e.g., two or more of *FAS, BID, CTLA4, PDCD1, CBLB, PTPN6, TRAC* or *TRBC* genes). When two or more genes are altered in a subject (or a cell from the subject), the subject (or a cell from the subject) is contacted with: (a) gRNAs that target two or more of the *FAS, BID, CTLA4, PDCD1, CBLB, PTPN6, TRAC* or *TRBC* genes, e.g., two or more of the gRNA as described herein; (b) a Cas9 molecule, e.g., a Cas9 molecule as described herein; and optionally, (c) second, third and/or fourth gRNAs that respectively targets the two or more genes selected in (a), e.g., a gRNA, as described herein. In an embodiment, the method further comprises, introducing into the subject, or a cell of the subject, a governing gRNA molecule, or a nucleic acid that encodes a governing gRNA molecule.

In an embodiment, the method of treating a subject suffering from cancer comprises altering two or more of *FAS, BID, CTLA4, PDCD1, CBLB, PTPN6, TRAC* or *TRBC* genes.

In an embodiment, the method of treating a subject suffering from cancer comprises altering three or more of *FAS, BID, CTLA4, PDCD1, CBLB, PTPN6, TRAC* or *TRBC* genes.

In an embodiment, the method of treating a subject suffering from cancer comprises altering four or more of *FAS, BID, CTLA4, PDCD1, CBLB, PTPN6, TRAC* or *TRBC* genes.

In an embodiment, the method of treating a subject suffering from cancer comprises altering five or more of *FAS, BID, CTLA4, PDCD1, CBLB, PTPN6, TRAC* or *TRBC* genes.

In an embodiment, the method of treating a subject suffering from cancer comprises altering six or more of *FAS, BID, CTLA4, PDCD1, CBLB, PTPN6, TRAC* or *TRBC* genes.

In an embodiment, the method of treating a subject suffering from cancer comprises altering seven or more of *FAS, BID, CTLA4, PDCD1, CBLB, PTPN6, TRAC* or *TRBC* genes.

In an embodiment, the method of treating a subject suffering from cancer comprises altering each of *FAS, BID, CTLA4, PDCD1, CBLB, PTPN6, TRAC* or *TRBC* genes.

In an embodiment, the method comprises acquiring knowledge of the sequence at a T cell target position in the subject.

In an embodiment, the method comprises acquiring knowledge of the sequence at a T cell target position in the subject by sequencing one or more of the *FAS, BID, CTLA4, PDCD1, CBLB, PTPN6, TRAC* or *TRBC* gene or a portion of one or more of the *FAS, BID, CTLA4, PDCD1, CBLB, PTPN6, TRAC* or *TRBC* gene.

In an embodiment, the method comprises inducing a mutation at a T cell target position in the *FAS, BID, CTLA4, PDCD1, CBLB, PTPN6, TRAC* or *TRBC* gene.

In an embodiment, the method comprises inducing a mutation at a T cell target position in one or more of the *FAS, BID, CTLA4, PDCD1, CBLB, PTPN6, TRAC* and *TRBC* genes.

In an embodiment, the method comprises inducing a mutation at a T cell target position in two or more of the *FAS, BID, CTLA4, PDCD1, CBLB, PTPN6, TRAC* and *TRBC* genes.

In an embodiment, the method comprises inducing a mutation at a T cell target position in three or more of the *FAS, BID, CTLA4, PDCD1, CBLB, PTPN6, TRAC* and *TRBC* genes.

In an embodiment, the method comprises inducing a mutation at a T cell target position in four or more of the *FAS, BID, CTLA4, PDCD1, CBLB, PTPN6, TRAC* and *TRBC* genes.

In an embodiment, the method comprises inducing a mutation at a T cell target position in five or more of the *FAS, BID, CTLA4, PDCD1, CBLB, PTPN6, TRAC* and *TRBC* genes.

In an embodiment, the method comprises inducing a mutation at a T cell target position in six or more of the *FAS, BID, CTLA4, PDCD1, CBLB, PTPN6, TRAC* and *TRBC* genes.

In an embodiment, the method comprises inducing a mutation at a T cell target position in seven or more of the *FAS, BID, CTLA4, PDCD1, CBLB, PTPN6, TRAC* and *TRBC* genes.

In an embodiment, the method comprises inducing a mutation at a T cell target position in each of *the FAS, BID, CTLA4, PDCD1, CBLB, PTPN6, TRAC and TRBC* genes.

In an embodiment, the method comprises inducing a mutation at a T cell target position in one or more of the *FAS, BID, CTLA4, PDCD1, CBLB, PTPN6, TRAC* or *TRBC* gene by NHEJ.

In an embodiment, a cell of the subject is contacted *ex vivo* with (a), (b), and optionally (c). In an embodiment, the cell is returned to the subject's body. In an embodiment, the cell of the subject being contacted *ex vivo* is a T cell. The T cell may be an engineered T cell, e.g., an engineered CAR (chimeric antigen receptor) T cell or an engineered TCR (T-cell receptor) T cell. A T cell may engineered to express a TCR or a CAR prior to, after, or at the same time as introducing a T cell target position mutation in one or more of the *FAS, BID, CTLA4, PDCD1, CBLB, PTPN6, TRAC* or *TRBC* gene.

When the method comprises (1) inducing a mutation at a T cell target position by NHEJ or (2) knocking down expression of the *FAS, BID, CTLA4, PDCD1, CBLB,* or *PTPN6* gene, e.g., by targeting the promoter region, a Cas9 of (b) and at least one guide RNA, e.g., a guide RNA of (a) are included in the contacting step.

In another aspect, disclosed herein is a reaction mixture comprising a, gRNA, a nucleic acid, or a composition described herein, and a cell, e.g., a cell from a subject having cancer, or a subject which would benefit from a mutation at a T cell target position.

In another aspect, disclosed herein is a kit comprising, (a) gRNA molecule described herein, or nucleic acid that encodes the gRNA, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein or a nucleic acid that encodes (c)(i);
(c)(ii) a third gRNA molecule, e.g., a second gRNA molecule described herein or a nucleic acid that encodes (c)(ii);
(c)(iii) a fourth gRNA molecule, e.g., a second gRNA molecule described herein or a nucleic acid that encodes (c)(iii).

In an embodiment, the kit comprises nucleic acid, e.g., an AAV vector, e.g., an AAV vector described herein, that encodes one or more of (a), (b), (c)(i), (c)(ii), and (c)(iii). In an embodiment, the kit further comprises a governing gRNA molecule, or a nucleic acid that encodes a governing gRNA molecule.

In an aspect, the disclosure features a gRNA molecule, referred to herein as a governing gRNA molecule, comprising a targeting domain which is complementary to a target domain on a nucleic acid that encodes a component of the CRISPR/Cas system introduced into a cell or subject. In an embodiment, the governing gRNA molecule targets a nucleic acid that encodes a Cas9 molecule or a nucleic acid that encodes a target gene gRNA molecule. In an embodiment, the governing gRNA comprises a targeting domain that is complementary to a target domain in a sequence that encodes a Cas9 component, e.g., a Cas9 molecule or target gene gRNA molecule. In an embodiment, the target domain is designed with, or has, minimal homology to other nucleic acid sequences in the cell, e.g., to minimize off-target cleavage. For example, the targeting domain on the governing gRNA can be selected to reduce or minimize off-target effects. In an embodiment, a target domain for a governing gRNA can be disposed in the control or coding region of a Cas9 molecule or disposed between a control region and a transcribed region. In an embodiment, a target domain for a governing gRNA can be disposed in the control or coding region of a target gene gRNA molecule or disposed between a control region and a transcribed region for a target gene gRNA. While not wishing to be bound by theory, in an embodiment, it is believed that altering, e.g., inactivating, a nucleic acid that encodes a Cas9 molecule or a nucleic acid that encodes a target gene gRNA molecule can be effected by cleavage of the targeted nucleic acid sequence or by binding of a Cas9 molecule/governing gRNA molecule complex to the targeted nucleic acid sequence.

The compositions, reaction mixtures and kits, as disclosed herein, can also include a governing gRNA molecule, e.g., a governing gRNA molecule disclosed herein.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting.

Headings, including numeric and alphabetical headings and subheadings, are for organization and presentation and are not intended to be limiting.

Other features and advantages of the invention will be apparent from the detailed description, drawings, and from the claims.

### DESCRIPTION

The drawings are first briefly described.
**Figs. 1A-1G** are representations of several exemplary gRNAs.
**Fig. 1A** depicts a modular gRNA molecule derived in part (or modeled on a sequence in part) from *Streptococcus pyogenes (S. pyogenes)* as a duplexed structure (SEQ ID NO:42 and 43, respectively, in order of appearance);
**Fig. 1B** depicts a unimolecular (or chimeric) gRNA molecule derived in part from *S. pyogenes* as a duplexed structure (SEQ ID NO:44);
**Fig. 1C** depicts a unimolecular gRNA molecule derived in part from *S. pyogenes* as a duplexed structure (SEQ ID NO:45);
**Fig. 1D** depicts a unimolecular gRNA molecule derived in part from *S. pyogenes* as a duplexed structure (SEQ ID NO:46);
**Fig. 1E** depicts a unimolecular gRNA molecule derived in part from *S. pyogenes* as a duplexed structure (SEQ ID NO:47);
**Fig. 1F** depicts a modular gRNA molecule derived in part from *Streptococcus thermophilus (S. thermophilus)* as a duplexed structure (SEQ ID NO:48 and 49, respectively, in order of appearance);
**Fig. 1G** depicts an alignment of modular gRNA molecules of *S*. *pyogenes* and *S. thermophilus* (SEQ ID NO:50-53, respectively, in order of appearance).

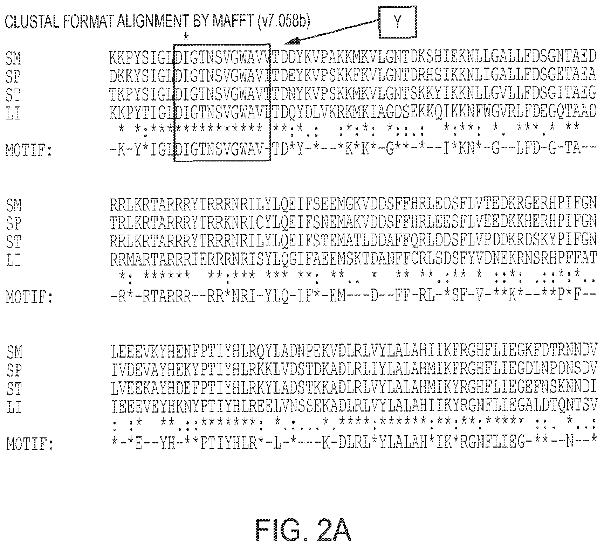

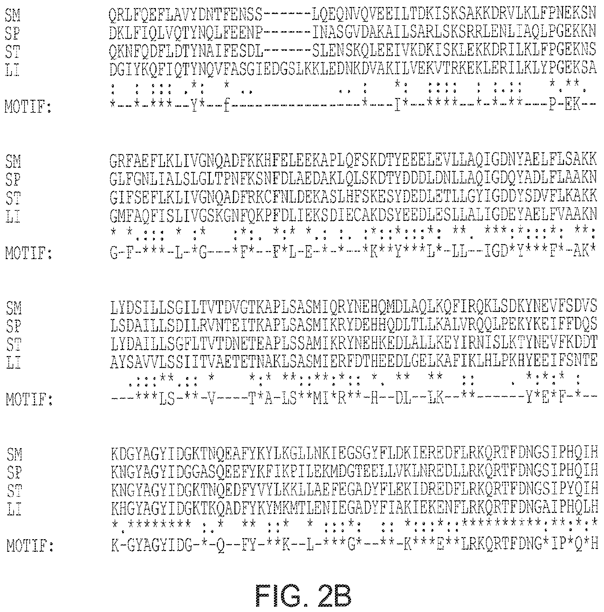

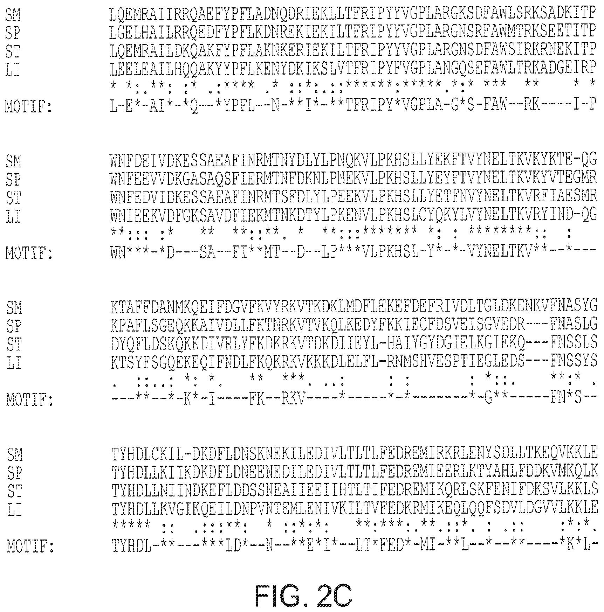

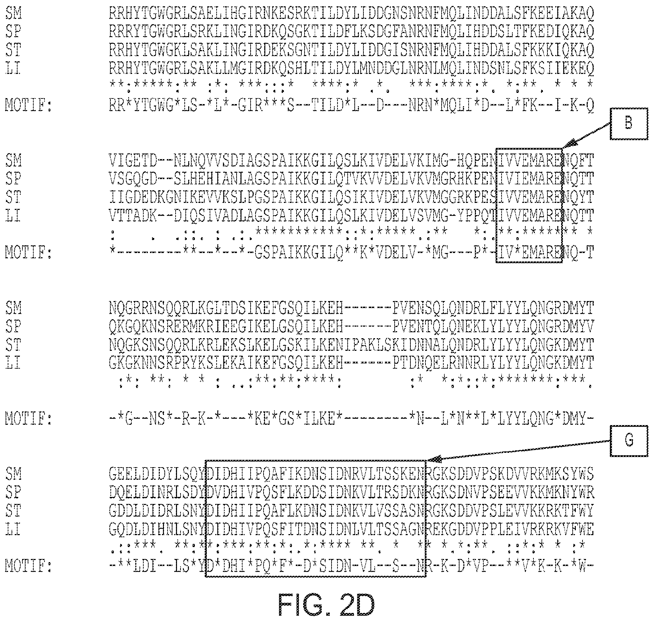

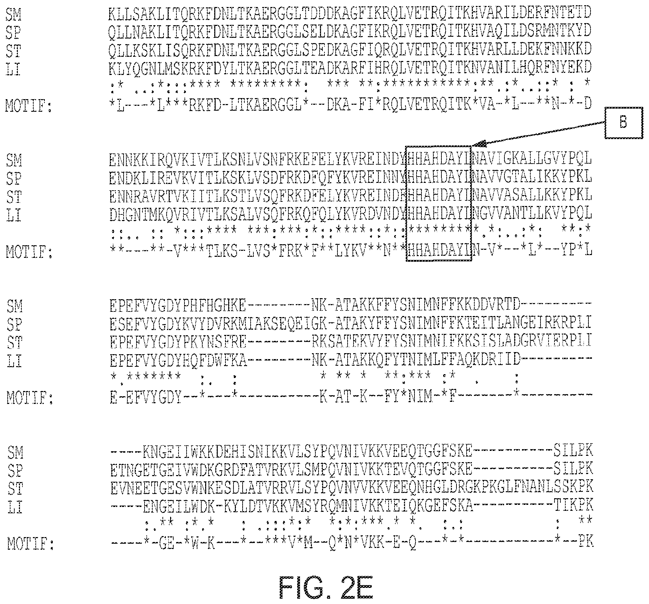

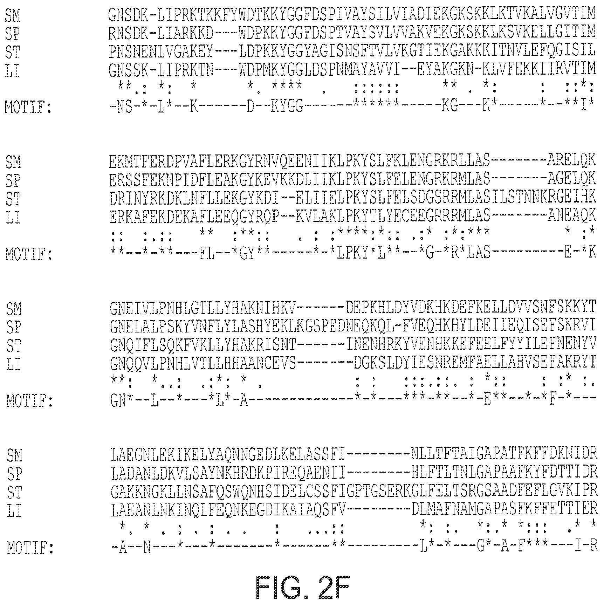

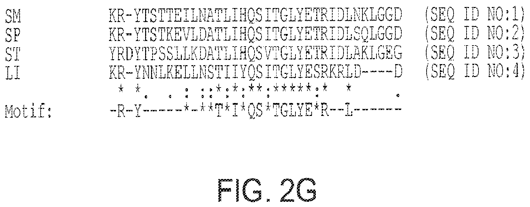

**Figs. 2A-2G** depict an alignment of Cas9 sequences from Chylinski et al. (RNA Biol. 2013; 10(5): 726-737). The N-terminal RuvC-like domain is boxed and indicated with a "y". The other two RuvC-like domains are boxed and indicated with a "b". The HNH-like domain is boxed and indicated by a "g". Sm: *S*. *mutans* (SEQ ID NO: 1); Sp: *S. pyogenes* (SEQ ID NO:2); St: *S. thermophilus* (SEQ ID NO:3); Li: *L. innocua* (SEQ ID NO:4). Motif: this is a motif based on the four sequences: residues conserved in all four sequences are indicated by single letter amino acid abbreviation; "*" indicates any amino acid found in the corresponding position of any of the four sequences; and "-" indicates any amino acid, e.g., any of the 20 naturally occurring amino acids.
**Figs. 3A-3B** show an alignment of the N-terminal RuvC-like domain from the Cas9 molecules disclosed in Chylinski *et al* (SEQ ID NO:54-103, respectively, in order of appearance). The last line of **Fig. 3B** identifies 4 highly conserved residues.
**Figs. 4A-4B** show an alignment of the N-terminal RuvC-like domain from the Cas9 molecules disclosed in Chylinski *et al.* with sequence outliers removed (SEQ ID NO:104-177, respectively, in order of appearance). The last line of **Fig. 4B** identifies 3 highly conserved residues.
**Figs. 5A-5C** show an alignment of the HNH-like domain from the Cas9 molecules disclosed in Chylinski *et al* (SEQ ID NO:178-252, respectively, in order of appearance). The last line of **Fig. 5C** identifies conserved residues.
**Figs. 6A-6B** show an alignment of the HNH-like domain from the Cas9 molecules disclosed in Chylinski *et al.* with sequence outliers removed (SEQ ID NO:253-302, respectively, in order of appearance). The last line of **Fig. 6B** identifies 3 highly conserved residues.

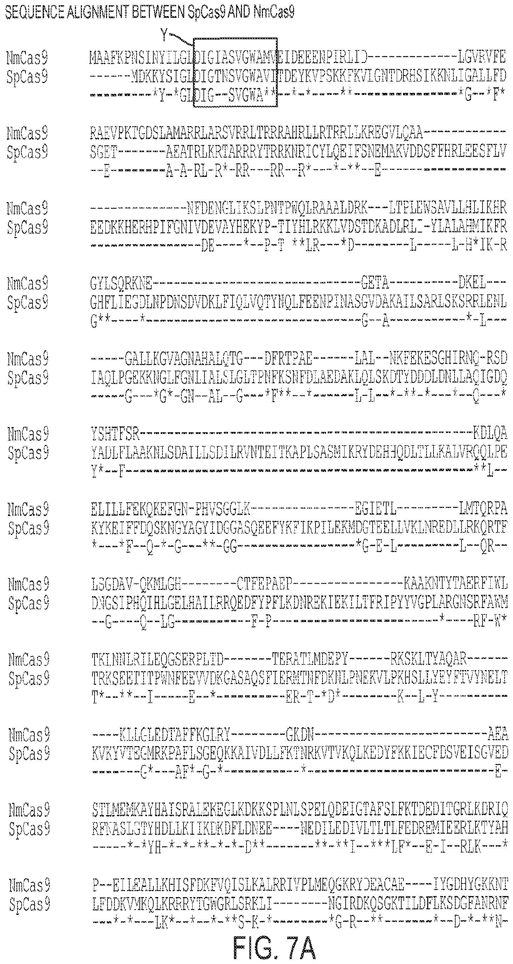

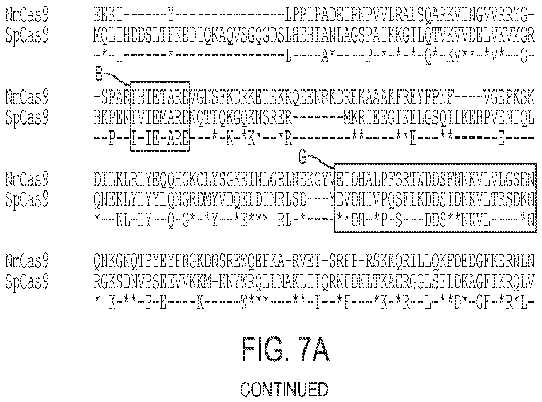

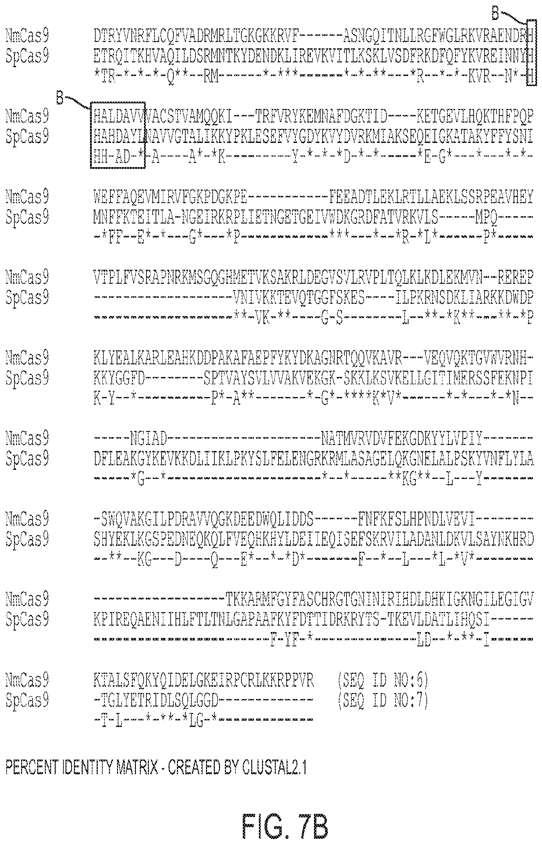

**Figs. 7A-7B** depict an alignment of Cas9 sequences from *S. pyogenes* and *Neisseria meningitidis (N. meningitidis).* The N-terminal RuvC-like domain is boxed and indicated with a "Y". The other two RuvC-like domains are boxed and indicated with a "B". The HNH-like domain is boxed and indicated with a "G". Sp: *S. pyogenes;* Nm: *N. meningitidis.* Motif: this is a motif based on the two sequences: residues conserved in both sequences are indicated by a single amino acid designation; "*" indicates any amino acid found in the corresponding position of any of the two sequences; "-" indicates any amino acid, e.g., any of the 20 naturally occurring amino acids, and "-" indicates any amino acid, e.g., any of the 20 naturally occurring amino acids, or absent.
**Fig. 8** shows a nucleic acid sequence encoding Cas9 of *N*. *meningitidis* (SEQ ID NO:303). Sequence indicated by an "R" is an SV40 NLS; sequence indicated as "G" is an HA tag; and sequence indicated by an "O" is a synthetic NLS sequence; the remaining (unmarked) sequence is the open reading frame (ORF).

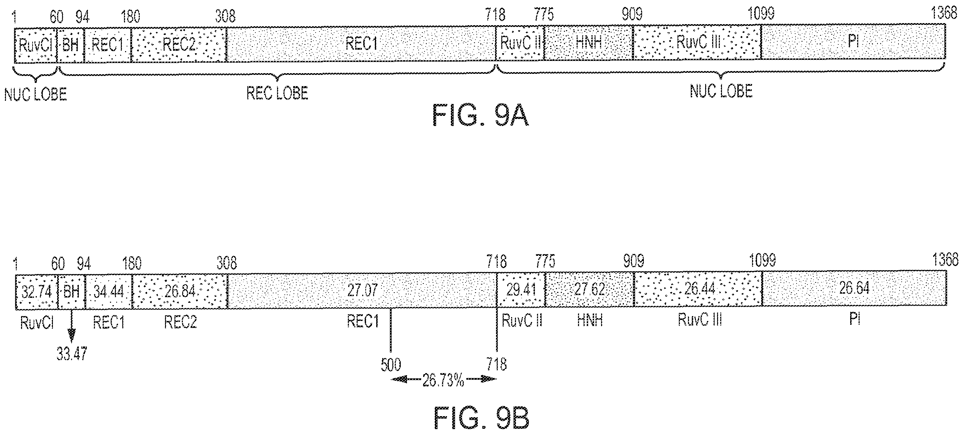

**Fig. 9A** shows schematic representations of the domain organization *of S. pyogenes* Cas 9 and the organization of the Cas9 domains, including amino acid positions, in reference to the two lobes of Cas9 (recognition (REC) and nuclease (NUC) lobes).
**Fig. 9B** shows schematic representations of the domain organization *of S. pyogenes* Cas 9 and the percent homology of each domain across 83 Cas9 orthologs.
**Fig. 10A** shows an exemplary structure of a unimolecular gRNA molecule derived in part from S. *pyogenes* as a duplexed structure (SEQ ID NO:40).
**Fig. 10B** shows an exemplary structure of a unimolecular gRNA molecule derived in part from S. *aureus* as a duplexed structure (SEQ ID NO:41).
**Fig. 11** shows results from an experiment assesssing the activity of gRNAs directed against *TRBC* gene in 293 cells using *S. aureus* Cas9. 293s were transfected with two plasmids - one encoding *S*. *aureus* Cas9 and the other encoding the listed gRNA. The graph summarizes the average %NHEJ observed at the TRBC2 locus for each gRNA, which was calculated from a T7E1 assay performed on genomic DNA isolated from duplicate samples.
**Fig. 12** shows results from an experiment assesssing the activity of gRNAs directed against *TRBC* gene in 293 cells using *S. pyrogenes* Cas9. 293 cells were transfected with two plasmids - one encoding *S. pyogenes* Cas9 and the other encoding the listed gRNA. The graph shows the average %NHEJ observed at both the *TRBC1* and *TRBC2* loci for each gRNA, which was calculated from a T7E1 assay performed on genomic DNA isolated from duplicate samples.
**Fig. 13** shows results from an experiment assesssing the activity of gRNAs directed against *TRAC* gene in 293 cells using *S. aureus* Cas9. 293 cells were transfected with two plasmids - one encoding *S. aureus* Cas9 and the other encoding the listed gRNA. The graph shows the average %NHEJ observed at the *TRAC* locus for each gRNA, which was calculated from a T7E1 assay performed on genomic DNA isolated from duplicate samples.
**Fig. 14** shows results from an experiment assesssing the activity of gRNAs directed against *TRAC* gene in 293 cells using *S. pyogenes* Cas9. 293 cells were transfected with two plasmids - one encoding *S. pyogenes* Cas9 and the other encoding the listed gRNA. The graph shows the average %NHEJ observed at the *TRAC* locus for each gRNA, which was calculated from a T7E1 assay performed on genomic DNA isolated from duplicate samples.
**Fig. 15** shows results from an experiment assesssing the activity of gRNAs directed against *PDCD1* gene in 293 cells using *S. aureus* Cas9. 293 cells were transfected with two plasmids - one encoding *S. aureus* Cas9 and the other encoding the listed gRNA. The graph shows the average %NHEJ observed at the *PDCD1* locus for each gRNA, which was calculated from a T7E1 assay performed on genomic DNA isolated from duplicate samples.
**Fig. 16** shows results from an experiment assesssing the activity of gRNAs directed against *PDCD1* gene in 293 cells using *S. pyogenes* Cas9. 293 cells were transfected with two plasmids - one encoding *S. pyogenes* Cas9 and the other encoding the listed gRNA. The graph shows the average %NHEJ observed at the *PDCD1* locus for each gRNA, which was calculated from a T7E1 assay performed on genomic DNA isolated from duplicate samples.
**Figs. 17A-C** depict results showing a loss of CD3 expression in CD4+ T cells due to delivery of *S. pyogenes* Cas9 mRNA and *TRBC* and *TRAC* gene specific gRNAs

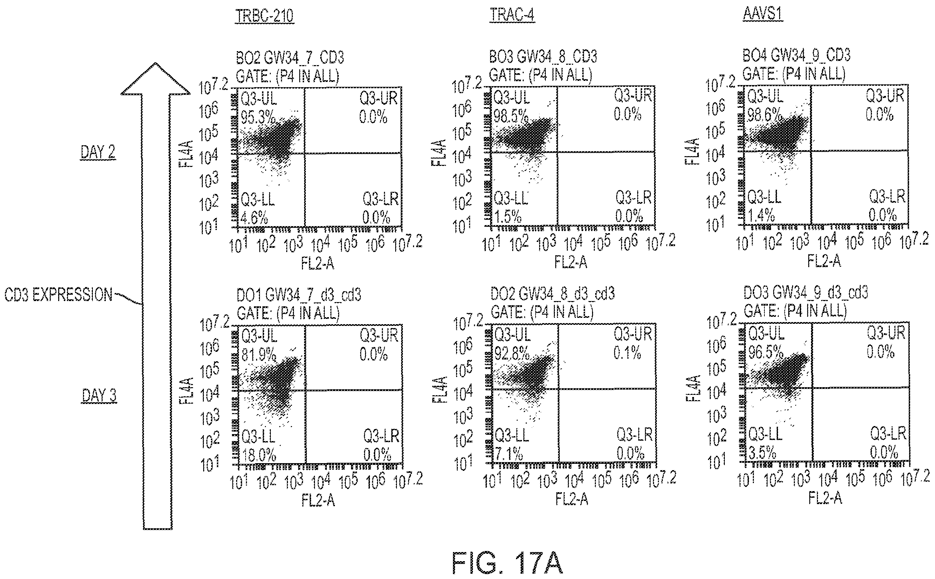

**Fig. 17A** shows CD4+ T cells electroporated with *S. pyogenes* Cas9 mRNA and the gRNA indicated (TRBC-210 (GCGCUGACGAUCUGGGUGAC) (SEQ ID NO:413), TRAC-4 (GCUGGUACACGGCAGGGUCA) (SEQ ID NO:453) or AAVS1 (GUCCCCUCCACCCCACAGUG) (SEQ ID NO:51201)) and stained with an APC-CD3 antibody and analyzed by FACS. The cells were analyzed on day 2 and day 3 after the electroporation.

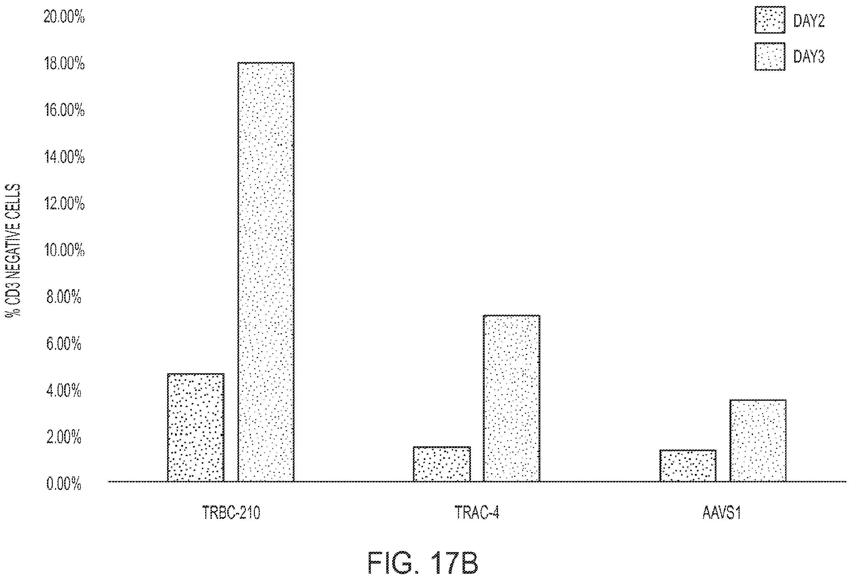

**Fig. 17B** shows quantification of the CD3 negative population from the plots in (A).

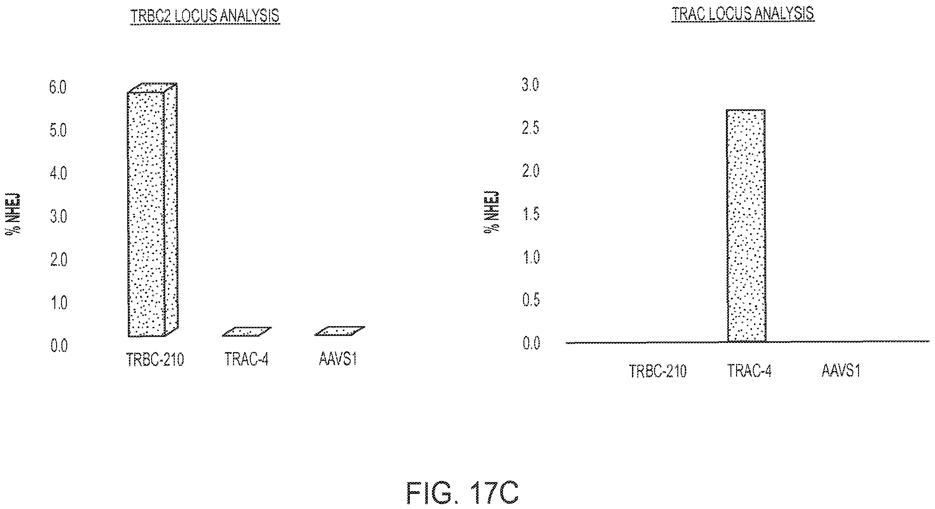

**Fig. 17C** shows %NHEJ results from the T7E1 assay performed on *TRBC2* and *TRAC* loci.

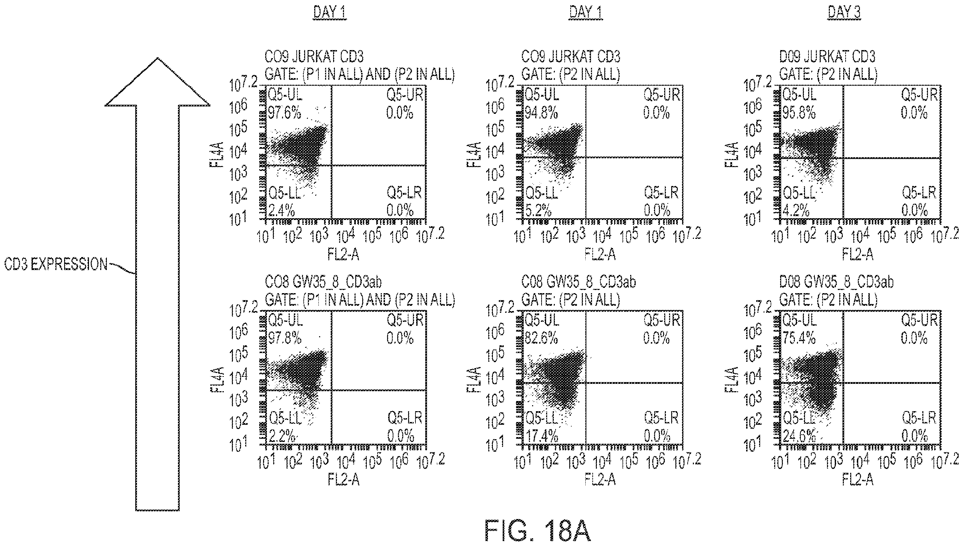

**Figs. 18A-C** depict results showing a loss of CD3 expression in Jurkat T cells due to delivery of *S. aureus* Cas9/gRNA RNP targeting *TRAC* gene
**Fig. 18A** shows Jurkat T cells electroporated with *S. aureus* Cas9/gRNA TRAC-233 (GUGAAUAGGCAGACAGACUUGUCA) (SEQ ID NO:474) RNPs targeting *TRAC* gene and stained with an APC-CD3 antibody and analyzed by FACS. The cells were analyzed on day 1, day 2 and day 3 after the electroporation.

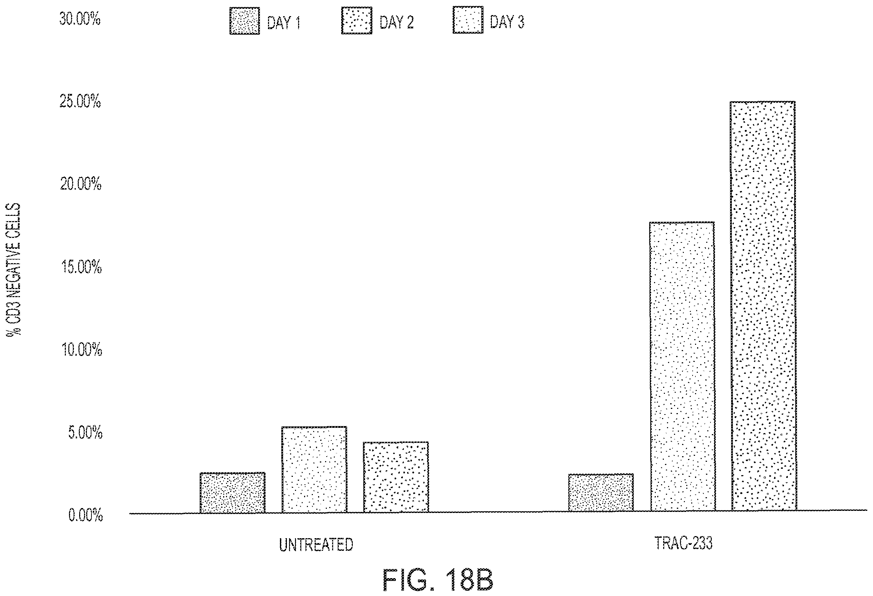

**Fig. 18B** shows quantification of the CD3 negative population from the plots in (A).

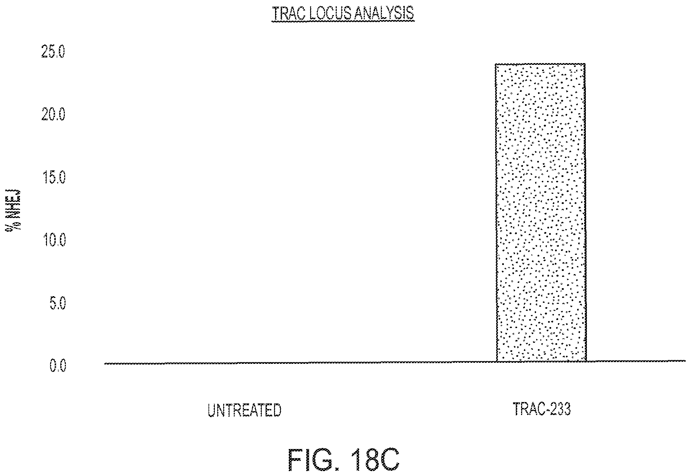

**Fig. 18C** shows % NHEJ results from the T7E1 assay performed on the *TRAC* locus

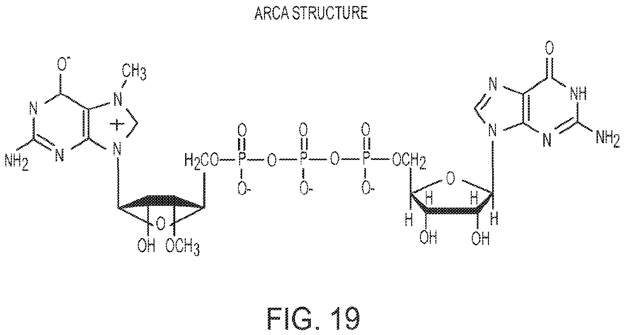

**Fig. 19** shows the structure of the 5' ARCA cap.

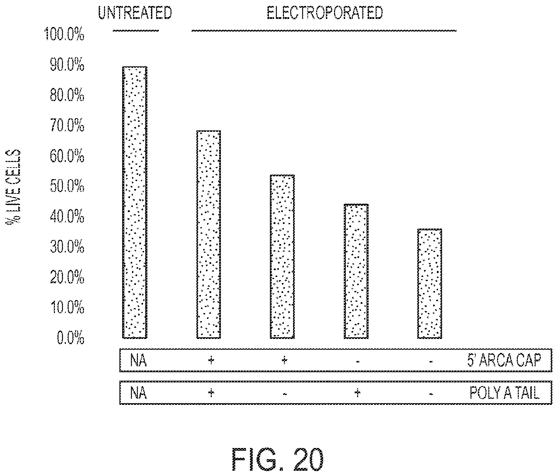

**Fig. 20** depicts results from the quantification of live Jurkat T cells post electroporation with Cas9 mRNA and AAVS1 gRNAs. Jurkat T cells were electroporated with *S*. *pyogenes* Cas9 mRNA and the respective modified gRNA. 24 hours after electroporation, 1 x 10⁵ cells were stained with FITC-conjugated Annexin-V specific antibody for 15 minutes at room temperature followed by staining with propidium iodide immediately before analysis by flow cytometry. The percentage of cells that did not stain for either Annexin-V or PI is presented in the bar graph.

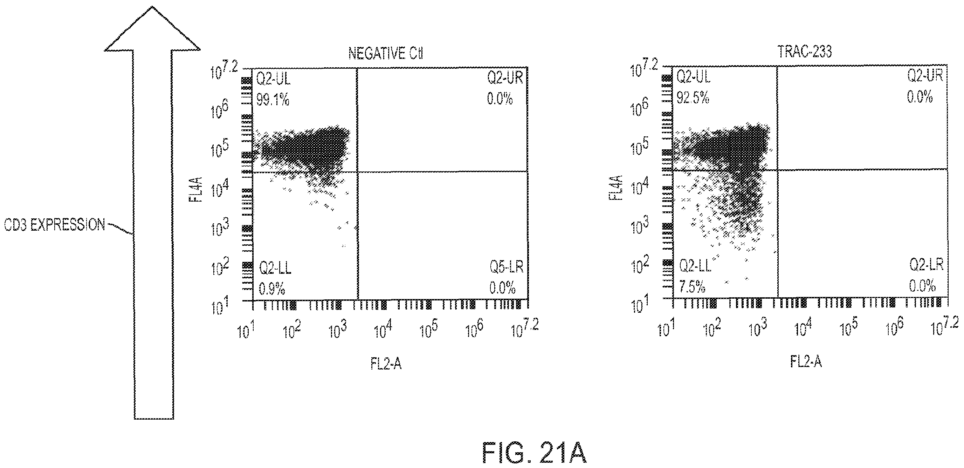

**Figs. 21A-C** depict loss of CD3 expression in Naive CD3+ T cells due to delivery of *S. aureus* Cas9/gRNA RNP targeting TRAC.
**Fig. 21A** depicts naive CD3+ T cells electroporated with *S. aureus* Cas9/gRNA (with targeting domain GUGAAUAGGCAGACAGACUUGUCA (SEQ ID NO:474) RNPs targeting TRAC were stained with an APC-CD3 antibody and analyzed by FACS. The cells were analyzed on day 4 after the electroporation. The negative control are cells with the gRNA with the targeting domain GUGAAUAGGCAGACAGACUUGUCA (SEQ ID NO:474) without a functional Cas9.

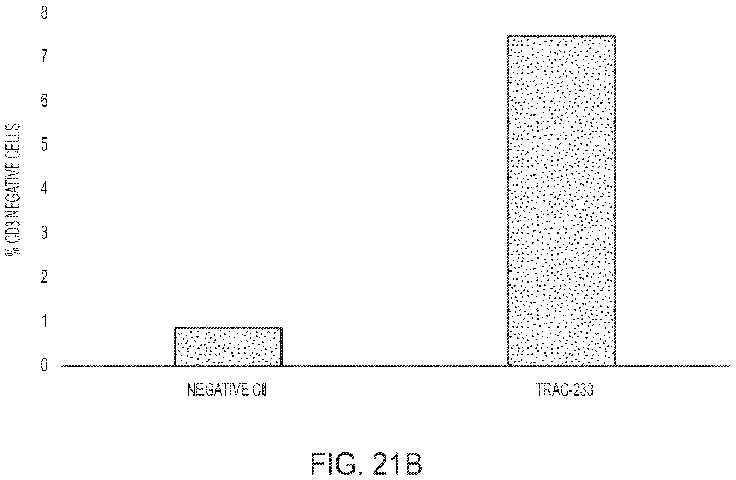

**Fig. 21B** depicts quantification of the CD3 negative population from the plots in Fig. 21A.

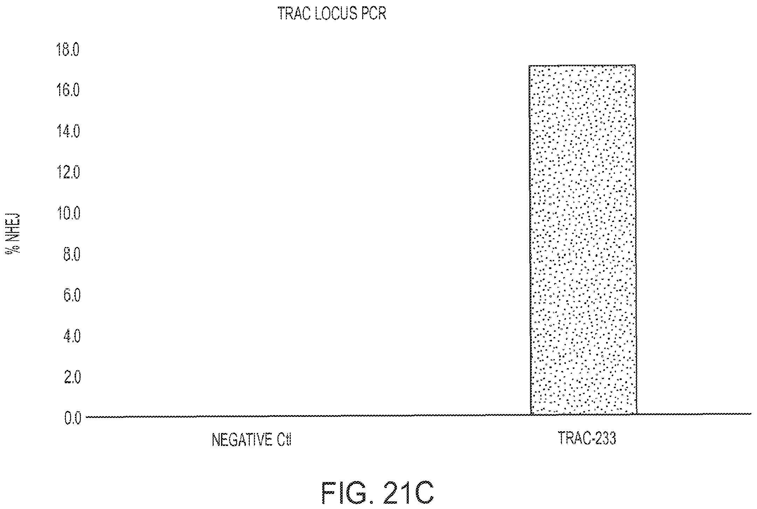

**Fig. 21C** depicts % NHEJ results from the T7E1 assay performed on the TRAC locus.

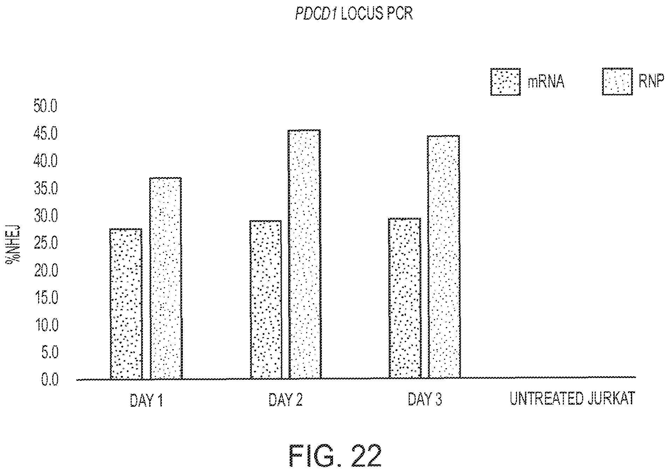

**Fig. 22** depicts genomic editing at the PDCD1 locus in Jurkat T cells after delivery of *S. pyogenes* Cas9 mRNA and PDCD1 gRNA (with a targeting domain GUCUGGGCGGUGCUACAACU (SEQ ID NO:508)) or *S. pyogenes* Cas9/gRNA (with a targeting domain GUCUGGGCGGUGCUACAACU (SEQ ID NO:508)) RNP targeting PDCD1. Quantification of %NHEJ results from the T7E1 assay performed on the PDCD1 locus at 24, 48, and 72 hours. Higher levels of %NHEJ were detected with RNP vs mRNA delivery.

### Definitions

"Domain", as used herein, is used to describe segments of a protein or nucleic acid. Unless otherwise indicated, a domain is not required to have any specific functional property.

Calculations of homology or sequence identity between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Governing gRNA molecule", as used herein, refers to a gRNA molecule that comprises a targeting domain that is complementary to a target domain on a nucleic acid that comprises a sequence that encodes a component of the CRISPR/Cas system that is introduced into a cell or subject. A governing gRNA does not target an endogenous cell or subject sequence. In an embodiment, a governing gRNA molecule comprises a targeting domain that is complementary with a target sequence on: (a) a nucleic acid that encodes a Cas9 molecule; (b) a nucleic acid that encodes a gRNA which comprises a targeting domain that targets *FAS, BID, CTLA4, PDCD1, CBLB, PTPN6, TRAC* or *TRBC* gene (a target gene gRNA); or on more than one nucleic acid that encodes a CRISPR/Cas component, e.g., both (a) and (b). In an embodiment, a nucleic acid molecule that encodes a CRISPR/Cas component, e.g., that encodes a Cas9 molecule or a target gene gRNA, comprises more than one target domain that is complementary with a governing gRNA targeting domain. While not wishing to be bound by theory, it is believed that a governing gRNA molecule complexes with a Cas9 molecule and results in Cas9 mediated inactivation of the targeted nucleic acid, e.g., by cleavage or by binding to the nucleic acid, and results in cessation or reduction of the production of a CRISPR/Cas system component. In an embodiment, the Cas9 molecule forms two complexes: a complex comprising a Cas9 molecule with a target gene gRNA, which complex will alter the *FAS, BID, CTLA4, PDCD1, CBLB, PTPN6, TRAC* or *TRBC* gene; and a complex comprising a Cas9 molecule with a governing gRNA molecule, which complex will act to prevent further production of a CRISPR/Cas system component, e.g., a Cas9 molecule or a target gene gRNA molecule. In an embodiment, a governing gRNA molecule/Cas9 molecule complex binds to or promotes cleavage of a control region sequence, e.g., a promoter, operably linked to a sequence that encodes a Cas9 molecule, a sequence that encodes a transcribed region, an exon, or an intron, for the Cas9 molecule. In an embodiment, a governing gRNA molecule/Cas9 molecule complex binds to or promotes cleavage of a control region sequence, e.g., a promoter, operably linked to a gRNA molecule, or a sequence that encodes the gRNA molecule. In an embodiment, the governing gRNA, e.g., a Cas9-targeting governing gRNA molecule, or a target gene gRNA-targeting governing gRNA molecule, limits the effect of the Cas9 molecule/target gene gRNA molecule complex-mediated gene targeting. In an embodiment, a governing gRNA places temporal, level of expression, or other limits, on activity of the Cas9 molecule/target gene gRNA molecule complex. In an embodiment, a governing gRNA reduces off-target or other unwanted activity. In an embodiment, a governing gRNA molecule inhibits, e.g., entirely or substantially entirely inhibits, the production of a component of the Cas9 system and thereby limits, or governs, its activity.

"Modulator", as used herein, refers to an entity, e.g., a drug, which can alter the activity (e.g., enzymatic activity, transcriptional activity, or translational activity), amount, distribution, or structure of a subject molecule or genetic sequence. In an embodiment, modulation comprises cleavage, e.g., breaking of a covalent or non-covalent bond, or the forming of a covalent or non-covalent bond, e.g., the attachment of a moiety, to the subject molecule. In an embodiment, a modulator alters the, three dimensional, secondary, tertiary, or quaternary structure, of a subject molecule. A modulator can increase, decrease, initiate, or eliminate a subject activity.

"Large molecule", as used herein, refers to a molecule having a molecular weight of at least 2, 3, 5, 10, 20, 30, 40, 50, 60, 70, 80, 90, or 100 kD. Large molecules include proteins, polypeptides, nucleic acids, biologics, and carbohydrates.

"Polypeptide", as used herein, refers to a polymer of amino acids having less than 100 amino acid residues. In an embodiment, it has less than 50, 20, or 10 amino acid residues.

"Non-homologous end joining" or "NHEJ", as used herein, refers to ligation mediated repair and/or non-template mediated repair including, e.g., canonical NHEJ (cNHEJ), alternative NHEJ (altNHEJ), microhomology-mediated end joining (MMEJ), single-strand annealing (SSA), and synthesis-dependent microhomology-mediated end joining (SD-MMEJ).

"Reference molecule", e.g., a reference Cas9 molecule or reference gRNA, as used herein, refers to a molecule to which a subject molecule, e.g., a subject Cas9 molecule of subject gRNA molecule, e.g., a modified or candidate Cas9 molecule is compared. For example, a Cas9 molecule can be characterized as having no more than 10% of the nuclease activity of a reference Cas9 molecule. Examples of reference Cas9 molecules include naturally occurring unmodified Cas9 molecules, e.g., a naturally occurring Cas9 molecule such as a Cas9 molecule of *S. pyogenes*, *S. aureus*, or *S. thermophilus.* In an embodiment, the reference Cas9 molecule is the naturally occurring Cas9 molecule having the closest sequence identity or homology with the Cas9 molecule to which it is being compared. In an embodiment, the reference Cas9 molecule is a sequence, e.g., a naturally occurring or known sequence, which is the parental form on which a change, e.g., a mutation has been made.

"Replacement", or "replaced", as used herein with reference to a modification of a molecule does not require a process limitation but merely indicates that the replacement entity is present.

"Small molecule", as used herein, refers to a compound having a molecular weight less than about 2 kD, e.g., less than about 2 kD, less than about 1.5 kD, less than about 1 kD, or less than about 0.75 kD.

"Subject", as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human. In other embodiments, the subject is poultry.

"Treat", "treating" and "treatment", as used herein, mean the treatment of a disease in a mammal, e.g., in a human, including (a) inhibiting the disease, i.e., arresting or preventing its development; (b) relieving the disease, i.e., causing regression of the disease state; and (c) curing the disease.

"X" as used herein in the context of an amino acid sequence, refers to any amino acid (e.g., any of the twenty natural amino acids) unless otherwise specified.

### Improving cancer immunotherapy

In an aspect, compositions and methods described herein can be used to affect proliferation of engineered T cells by altering one ore more T cell-expressed genes, e.g., the FAS, BID, CTLA4, PDCD1, CBLB, PTPN6, TRAC and/or TRBC gene. In order for engineered T cells to mount an effective anti-tumor response they need to: 1) proliferate adequately following transfer to the subject to provide a sufficient number of specific tumor-targeting T cells; 2) survive in the subject for a length of time sufficient to maintain the required anti-tumor activity; and 3) evade the influence of suppressive factors produced by immune cells, tumor cells and other cells in the tumor environment so that the engineered T cells maintain a functional anti-tumor phenotype. Insufficient proliferation and/or survival, as well as susceptibility to inhibitory factors, can contribute to lack of efficacy of engineered T cells in subjects suffering from cancer. The methods and compositions disclosed herein address these issues in order to improve efficacy of engineered T cells as a cancer therapeutic modality.

In an embodiment, compositions and methods described herein can be used to affect proliferation of engineered T cells by altering the *CBLB* gene. While not wishing to be bound by theory, it is considered that reduced or absent expression of Casitas B-lineage lymphoma b protein (encoded by *CBLB)* reduces the requirement for exogenous interleukin signaling to promote proliferation of engineered T cells following transfer to the subject (Stromnes, I. M. et al., 2010 J. Clin. Tnvest. 120, 3722-3734).

In an embodiment, compositions and methods described herein can be used to affect proliferation of engineered T cells by altering the *PTPN6* gene. While not wishing to be bound by theory, it is considered that reduced or absent expression of Src homology region 2 domain-containing phosphatase-1 protein (encoded by *PTPN6*) leads to increased short-term accumulation of transferred T cells with subsequently improved anti-tumor activity (Stromnes, I. M. et al., 2012 J. Immunol. 189, 1812-1825).

In an embodiment, compositions and methods described herein can be used to affect proliferation of engineered T cells by altering the *FAS* gene. While not wishing to be bound by theory, it is considered that reduced or absent expression of the Fas protein will inhibit induction of T cell apoptosis by Fasligand; a factor expressed by many cancer types (Dotti, G. et al., 2005 Blood 105, 4677-4684).

In an embodiment, compositions and methods described herein can be used to affect proliferation of engineered T cells by altering the *BID* gene. While not wishing to be bound by theory, it is considered that reduced or absent expression of the Bid protein prevents the induction of T cell apoptosis following activation of the Fas pathway (Lei, X. Y. et al., 2009 Immunol. Lett. 122, 30-36).

In an embodiment, compositions and methods described herein can be used to decrease the effect of immune suppressive factors on engineered T cells by altering the *CTLA4* gene. While not wishing to be bound by theory, it is considered that reduced or absent expression of cytotoxic T-lymphocyte-associated antigen 4 (encoded by *CTLA4*) abrogates the induction of a non-responsive state ("anergy") following binding of CD80 or CD86 expressed by antigen presenting cells in the tumor environment (Shrikant, P. et al, 1999 Immunity 11, 483-493).

In an embodiment, compositions and methods described herein can be used to decrease the effect of immune suppressive factors on engineered T cells by altering the *PDCD1* gene. While not wishing to be bound by theory, it is considered that reduced or absent expression of the Programmed Cell Death Protein 1 (encoded by *PDCD1*) prevents induction of T cell apoptosis by engagement of PD1 Ligand expressed by tumor cells or cells in the tumor environment (Topalian, S. L. et al., 2012 N. Engl. J. Med. 366, 2443-2454).

In an embodiment, compositions and methods described herein can be used to improve T cell specificity and safety by altering the *TRAC* and/or *TRBC* gene. While not wishing to be bound by theory, it is considered that reduced or absent expression of T-cell receptors (encoded by *TRAC* and *TRBC*) prevents graft vs. host disease by eliminating T cell receptor recognition of and response to host tissues. This approach, therefore, could be used to generate "off the shelf' T cells (Torikai et al., 2012 Blood 119, 5697-5705). Also, while not wishing to be bound by theory, it is considered that reduced or absent expression of the *TRAC* and/or *TRBC* gene will reduce or eliminate mis-pairing of endogenous T cell receptors with exogenously introduced engineered T cell receptors, thus improving therapeutic efficacy (Provasi et al., 2012, Nature Medicine 18, 807-815).

In an embodiment, compositions and methods described herein can be used to decrease one or more of *FAS, BID, CTLA4, PDCD1, CBLB, PTPN6, TRAC* and *TRBC* genes to improve treatment of cancer immunotherapy using engineered T cells.

Disclosed herein are the approaches to treat cancer via immunotherapy, using the compositions and methods described herein.

In one approach, one or more of the *FAS, BID, CTLA4, PDCD1, CBLB, PTPN6, TRAC* and *TRBC* genes are targeted as a targeted knockout or knockdown, e.g., to affect T cell proliferation, survival and/or function. In an embodiment, said approach comprises knocking out or knocking down one T-cell expressed gene (e.g., *FAS, BID, CTLA4, PDCD1, CBLB, PTPN6, TRAC* or *TRBC* gene). In another embodiment, the approach comprises knocking out or knocking down two T-cell expressed genes, e.g., two of *FAS, BID, CTLA4, PDCD1, CBLB, PTPN6, TRAC* or *TRBC* genes. In another embodiment, the approach comprises knocking out or knocking down three T-cell expressed genes, e.g., three of *FAS*, *BID*, *CTLA4, PDCD1, CBLB, PTPN6, TRAC* or *TRBC* genes. In another embodiment, the approach comprises knocking out or knocking down four T-cell expressed genes, e.g., four of *FAS*, *BID*, *CTLA4*, *PDCD1, CBLB, PTPN6, TRAC* or *TRBC* genes. In another embodiment, the approach comprises knocking out or knocking down five T-cell expressed genes, e.g., five of *FAS, BID, CTLA4, PDCD1, CBLB, PTPN6, TRAC* or *TRBC* genes. In another embodiment, the approach comprises knocking out or knocking down six T-cell expressed genes, e.g., six of *FAS, BID, CTLA4, PDCD1, CBLB, PTPN6, TRAC* or *TRBC* genes. In another embodiment, the approach comprises knocking out or knocking down seven T-cell expressed genes, e.g., seven of *FAS, BID, CTLA4, PDCD1, CBLB, PTPN6, TRAC* or *TRBC* genes. In another embodiment, the approach comprises knocking out or knocking down eight T-cell expressed genes, e.g., each of *FAS, BID, CTLA4, PDCD1, CBLB, PTPN6, TRAC and TRBC* genes.

In an embodiment, the methods comprise initiating treatment of a subject after disease onset. In an embodiment, the method comprises initiating treatment of a subject well after disease onset, e.g., 1, 2, 3, 4, 5, 6, 7, 8, 9, 10, 12, 24, or 36 months after onset of cancer.

In an embodiment, the method comprises initiating treatment of a subject in an advanced stage of disease.

Overall, initiation of treatment for subjects at all stages of disease is expected to be of benefit to subjects.

Cancers that may be treated using the compositions and methods disclosed herein include cancers of the blood and solid tumors. For example, cancers that may be treated using the compositions and methods disclosed herein include, but are not limited to, lymphoma, chronic lymphocytic leukemia (CLL), B cell acute lymphocytic leukemia (B-ALL), acute lymphoblastic leukemia, acute myeloid leukemia, non-Hodgkin's lymphoma (NHL), diffuse large cell lymphoma (DELL), multiple myeloma, renal cell carcinoma (RCC), neuroblastoma, colorectal cancer, breast cancer, ovarian cancer, melanoma, sarcoma, prostate cancer, lung cancer, esophageal cancer, hepatocellular carcinoma, pancreatic cancer, astrocytoma, mesothelioma, head and neck cancer, and medulloblastoma.

### Methods of altering one or more T cell-expressed genes, e.g., the FAS, BID, CTLA4, PDCD1, CBLB. PTPN6, TRAC and/or TRBC gene

As disclosed herein, one or more T cell-expressed genes, e.g., the FAS, BID, CTLA4, PDCD1, CBLB, PTPN6, TRAC and/or TRBC gene can be targeted (e.g., altered) by gene editing, e.g., using CRISPR-Cas9 mediated methods as described herein.

Methods and compositions discussed herein, provide for targeting (e.g., altering) a T cell target position in one or more T cell-expressed genes, e.g., the FAS, BID, CTLA4, PDCD1, CBLB, PTPN6, TRAC and/or TRBC gene. A T cell target position can be targeted (e.g., altered) by gene editing, e.g., using CRISPR-Cas9 mediated methods to target (e.g. alter) in one or more T cell-expressed genes, e.g., the FAS, BID, CTLA4, PDCD1, CBLB, PTPN6, TRAC and/or TRBC gene.
Disclosed herein are methods for targeting (e.g., altering) a T cell target position in one or more T cell-expressed genes, e.g., the FAS, BID, CTLA4, PDCD1, CBLB, PTPN6, TRAC and/or TRBC gene.

Targeting (e.g., altering) a T cell target position is achieved, e.g., by:
(1) knocking out one or more T cell-expressed genes, e.g., the FAS, BID, CTLA4, PDCD1, CBLB, PTPN6, TRAC and/or TRBC gene:
   (a) insertion or deletion (e.g., NHEJ-mediated insertion or deletion) of one or more nucleotides in close proximity to or within the coding region of one or more T cell-expressed genes, e.g., the FAS, BID, CTLA4, PDCD1, CBLB, PTPN6, TRAC and/or TRBC gene, or
   (b) deletion (e.g., NHEJ-mediated deletion) of a genomic sequence including at least a portion of one or more T cell-expressed genes, e.g., the FAS, BID, CTLA4, PDCD1, CBLB, PTPN6, TRAC and/or TRBC gene, or
(2) knocking down one or more T cell-expressed genes, e.g., the FAS, BID, CTLA4, PDCD1, CBLB, and/or PTPN6 gene mediated by enzymatically inactive Cas9 (eiCas9) molecule or an eiCas9-fusion protein by targeting non-coding region, e.g., a promoter region, of the gene.

All approaches give rise to targeting (e.g., alteration) of one or more T cell-expressed genes, e.g., the FAS, BID, CTLA4, PDCD1, CBLB, PTPN6, TRAC and/or TRBC gene.

In one embodiment, methods described herein introduce one or more breaks near the coding region in at least one allele of one or more T cell-expressed genes, e.g., the FAS, BID, CTLA4, PDCD1, CBLB, PTPN6, TRAC and/or TRBC gene. In another embodiment, methods described herein introduce two or more breaks to flank at least a portion of one or more T cell-expressed genes, e.g., the FAS, BID, CTLA4, PDCD1, CBLB, PTPN6, TRAC and/or TRBC gene. The two or more breaks remove (e.g., delete) a genomic sequence including at least a portion of one or more T cell-expressed genes, e.g., the FAS, BID, CTLA4, PDCD1, CBLB, PTPN6, TRAC and/or TRBC gene. In another embodiment, methods described herein comprise knocking down one or more T cell-expressed genes, e.g., the FAS, BID, CTLA4, PDCD1, CBLB, and/or PTPN6 gene mediated by enzymatically inactive Cas9 (eiCas9) molecule or an eiCas9-fusion protein by targeting the promoter region of T cell target knockdown position. All methods described herein result in targeting (e.g., alteration) of one or more T cell-expressed genes, e.g., the FAS, BID, CTLA4, PDCD1, CBLB, PTPN6, TRAC and/or TRBC gene.

The targeting (e.g., alteration) of one or more T cell-expressed genes, e.g., the FAS, BID, CTLA4, PDCD1, CBLB, PTPN6, TRAC and/or TRBC gene can be mediated by any mechanism. Exemplary mechanisms that can be associated with the alteration of one or more T cell-expressed genes, e.g., the FAS, BID, CTLA4, PDCD1, CBLB, PTPN6, TRAC and/or TRBC gene include, but are not limited to, non-homologous end joining (e.g., classical or alternative), microhomology-mediated end joining (MMEJ), homology-directed repair (e.g., endogenous donor template mediated), SDSA (synthesis dependent strand annealing), single strand annealing or single strand invasion.

### Knocking out one or more T cell-expressed genes, e.g., the FAS, BID, CTLA4, PDCD1, CBLB, PTPN6, TRAC and/or TRBC gene by introducing an indel or a deletion in one or more T cell-expressed genes, e.g., the FAS, BID, CTLA4, PDCD1, CBLB, PTPN6, TRAC and/or TRBC gene

In an embodiment, the method comprises introducing an insertion or deletion of one more nucleotides in close proximity to the T cell target knockout position (e.g., the early coding region) of one or more T cell-expressed genes, e.g., the FAS, BID, CTLA4, PDCD1, CBLB, PTPN6, TRAC and/or TRBC gene genes. As described herein, in one embodiment, the method comprises the introduction of one or more breaks (e.g., single strand breaks or double strand breaks) to the T cell target knockout position, e.g., the coding region (e.g., the early coding region, e.g., within 500bp from the start codon or the remaining coding sequence, e.g., downstream of the first 500bp from the start codon) of one or more T cell-expressed genes, e.g., the FAS, BID, CTLA4, PDCD1, CBLB, PTPN6, TRAC and/or TRBC gene. While not wishing to be bound by theory, it is believed that NHEJ-mediated repair of the break(s) allows for the NHEJ-mediated introduction of an indel in close proximity to within the T cell target knockout position.

In an embodiment, a single strand break is introduced (e.g., positioned by one gRNA molecule) at or in close proximity to a T cell target knockout position in one or more T cell-expressed genes, e.g., the FAS, BTD, CTLA4, PDCD1, CBLB, PTPN6, TRAC and/or TRBC gene. In an embodiment, a single gRNA molecule (e.g., with a Cas9 nickase) is used to create a single strand break at or in close proximity to the T cell target knockout position, e.g., the coding region (e.g., the early coding region, e.g., within 500bp from the start codon or the remaining coding sequence, e.g., downstream of the first 500bp from the start codon). In an embodiment, the break is positioned to avoid unwanted target chromosome elements, such as repeat elements, e.g., an *Alu* repeat.

In an embodiment, a double strand break is introduced (e.g., positioned by one gRNA molecule) at or in close proximity to a T cell target knockout position in one or more T cell-expressed genes, e.g., the FAS, BID, CTLA4, PDCD1, CBLB, PTPN6, TRAC and/or TRBC gene. In an embodiment, a single gRNA molecule (e.g., with a Cas9 nuclease other than a Cas9 nickase) is used to create a double strand break at or in close proximity to the T cell target knockout position, e.g., the coding region (e.g., the early coding region, e.g., within 500bp from the start codon or the remaining coding sequence, e.g., downstream of the first 500bp from the start codon). In an embodiment, the break is positioned to avoid unwanted target chromosome elements, such as repeat elements, e.g., an *Alu* repeat.

In an embodiment, two single strand breaks are introduced (e.g., positioned by two gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, two gRNA molecules (e.g., with one or two Cas9 nickcases) are used to create two single strand breaks at or in close proximity to the T cell target knockout position, e.g., the coding region (e.g., the early coding region, e.g., within 500bp from the start codon or the remaining coding sequence, e.g., downstream of the first 500bp from the start codon). In an embodiment, the gRNAs molecules are configured such that both single strand breaks are positioned upstream or downstream of the T cell target knockout position. In another embodiment, two gRNA molecules (e.g., with two Cas9 nickcases) are used to create two single strand breaks at or in close proximity to the T cell target knockout position, e.g., the gRNAs molecules are configured such that one single strand break is positioned upstream and a second single strand break is positioned downstream of the T cell target knockout position. In an embodiment, the breaks are positioned to avoid unwanted target chromosome elements, such as repeat elements, e.g., an *Alu* repeat.

In an embodiment, two sets of breaks (e.g., two double strand breaks) are introduced (e.g., positioned by two gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, two gRNA molecules (e.g., with one or two Cas9 nucleases that are not Cas9 nickases) are used to create two double strand breaks to flank a T cell target knockout position, e.g., the coding region (e.g., the early coding region, e.g., within 500bp from the start codon or the remaining coding sequence, e.g., downstream of the first 500bp from the start codon). In an embodiment, the gRNAs molecules are configured such that both sets of breaks are positioned upstream or downstream of the T cell target knockout position. In an embodiment, the gRNA molecules are configured such that one set of break(s) are positioned upstream and a second set of break(s) are positioned downstream of the T cell target knockout position. In an embodiment, the breaks are positioned to avoid unwanted target chromosome elements, such as repeat elements, e.g., an *Alu* repeat.

In an embodiment, two sets of breaks (e.g., one double strand breaks and a pair of single strand breaks) are introduced (e.g., positioned by three gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, three gRNA molecules (e.g., with a Cas9 nuclease and one or two Cas9 nickases) are used to create two sets of breaks to flank a T cell target knockout position, e.g., the coding region (e.g., the early coding region, e.g., within 500bp from the start codon or the remaining coding sequence, e.g., downstream of the first 500bp from the start codon). In an embodiment, the gRNAs molecules are configured such that both sets of breaks are positioned upstream or downstream of the T cell target knockout position. In an embodiment, the gRNA molecules are configured such that oneset of break(s) are positioned upstream and a second set of break(s) are positioned downstream of the T cell target knockout position. In an embodiment, the breaks are positioned to avoid unwanted target chromosome elements, such as repeat elements, e.g., an *Alu* repeat.

In an embodiment, two sets of breaks (e.g., two pairs of single strand breaks) are introduced (e.g., positioned by four gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, four gRNA molecules (e.g., with one or more Cas9 nickases) are used to create two sets of breaks to flank a T cell target knockout position, e.g., the coding region (e.g., the early coding region, e.g., within 500bp from the start codon or the remaining coding sequence, e.g., downstream of the first 500bp from the start codon). In an embodiment, the gRNAs molecules are configured such that both sets of breaks are positioned upstream or downstream of the T cell target knockout position. In an embodiment, the gRNA molecules are configured such that one set of break(s) are positioned upstream and a second set of break(s) are positioned downstream of the T cell target knockout position. In an embodiment, the breaks are positioned to avoid unwanted target chromosome elements, such as repeat elements, e.g., an *Alu* repeat.

In an embodiment, two or more (e.g., three or four) gRNA molecules are used with one Cas9 molecule. In another embodiment, when two ore more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

When more than one gene is targeted for alteration in a cell, the targeted nucleic acids may be altered, e.g., cleaved, by one or more Cas9 proteins. For example, if two genes are targeted for alteration, e.g., both genes are targeted for knockout, the same or a different Cas9 protein may be used to target each gene. In one embodiment, both genes (or each gene targeted in a cell), are cleaved by a Cas9 nuclease to generate a double stranded break. In another embodiment, both genes (or each gene targeted in a cell), are cleaved by a Cas9 nuclease to generate a double stranded break. In another embodiment, one or more genes in a cell may be altered by cleavage with a Cas9 nuclease and one or more genes in the same cell may be altered by cleavage with a Cas9 nickase. When two or more Cas9 proteins are used to cut a target nucleic acid, e.g., different genes in a cell, the Cas9 proteins may be from different bacterial species. For example, one or more genes in a cell may be altered by cleavage with a Cas9 protein from one bacterial species, and one or more genes in the same cell may be altered by cleavage with a Cas9 protein from a different bacterial species. It is contemplated that when two or more Cas9 proteins from different species are used that they may be delivered at the same time or delivered sequentially to control specificity of cleavage in the desired gene at the desired position in the target nucleic acid.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward.

### Knocking out one or more T cell-expressed genes, e.g., the FAS, BID, CTLA4, PDCD1, CBLB, PTPN6, TRAC and/or TRBC gene by deleting (e.g., NHEJ-mediated deletion) a genomic sequence including at least a portion of one or more T cell-expressed genes, e.g., the FAS, BID, CTLA4, PDCD1, CBLB. PTPN6, TRAC and/or TRBC gene genes

In an embodiment, the method comprises introducing a deletion of a genomic sequence comprising at least a portion of one or more T cell-expressed genes, e.g., the FAS, BID, CTLA4, PDCD1, CBLB, PTPN6, TRAC and/or TRBC gene genes. As described herein, in an embodiment, the method comprises the introduction of two double stand breaks - one 5' and the other 3' to (i.e., flanking) T cell target knockout position. In an embodiment, two gRNAs, e.g., unimolecular (or chimeric) or modular gRNA molecules, are configured to position the two sets of breaks (e.g., two double strand breaks, one double strand break and a pair of single strand breaks or two pairs of single strand breaks) on opposite sides of the T cell target knockout position in one or more T cell-expressed genes, e.g., the FAS, BID, CTLA4, PDCD1, CBLB, PTPN6, TRAC and/or TRBC gene.

In an embodiment, the method comprises deleting (e.g., NHEJ-mediated deletion) a genomic sequence including at least a portion of one or more T cell-expressed genes, e.g., the FAS, BID, CTLA4, PDCD1, CBLB, PTPN6, TRAC and/or TRBC gene genes. As described herein, in one embodiment, the method comprises the introduction two sets of breaks (e.g., a pair of double strand breaks, one double strand break or a pair of single strand breaks, or two pairs of single strand breaks) to flank a region in one or more T cell-expressed genes, e.g., the FAS, BID, CTLA4, PDCD1, CBLB, PTPN6, TRAC and/or TRBC gene genes (e.g., a coding region, e.g., an early coding region, or a non-coding region, e.g., a non-coding sequence of one or more T cell-expressed genes, e.g., the FAS, BID, CTLA4, PDCD1, CBLB, PTPN6, TRAC and/or TRBC gene genes, e.g., a promoter, an enhancer, an intron, a 3'UTR, and/or a polyadenylation signal). While not wishing to be bound by theory, it is believed that NHEJ-mediated repair of the break(s) allows for alteration of one or more T cell-expressed genes, e.g., the FAS, BID, CTLA4, PDCD1, CBLB, PTPN6, TRAC and/or TRBC gene genes as described herein, which reduces or eliminates expression of the gene, e.g., to knock out one or both alleles of one or more T cell-expressed genes, e.g., the FAS, BID, CTLA4, PDCD1, CBLB, PTPN6, TRAC and/or TRBC gene genes.

In an embodiment, two sets of breaks (e.g., two double strand breaks) are introduced (e.g., positioned by two gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, two gRNA molecules (e.g., with one or two Cas9 nucleases that are not Cas9 nickases) are used to create two sets of breaks to flank a T cell target knockout position, e.g., the gRNA molecules are configured such that one set of break(s) are positioned upstream and a a second set of break(s) are positioned downstream of the T cell target knockout position. In an embodiment, the breaks are positioned to avoid unwanted target chromosome elements, such as repeat elements, e.g., an *Alu* repeat.

In an embodiment, two sets of breaks (e.g., one double strand breaks and a pair of single strand breaks) are introduced (e.g., positioned by three gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, three gRNA molecules (e.g., with a Cas9 nuclease and one or two Cas9 nickases) are used to create two sets of breaks to flank a T cell target knockout position, e.g., the gRNA molecules are configured such that one set of break(s) are positioned upstream and a second set of break(s) are positioned downstream of the T cell target knockout position. In an embodiment, the breaks are positioned to avoid unwanted target chromosome elements, such as repeat elements, e.g., an *Alu* repeat.

In an embodiment, two sets of breaks (e.g., two pairs of single strand breaks) are introduced (e.g., positioned by four gRNA molecules) at or in close proximity to a T cell target knockout position in one or more T cell-expressed genes, e.g., the FAS, BID, CTLA4, PDCD1, CBLB, PTPN6, TRAC and/or TRBC gene. In an embodiment, four gRNA molecules (e.g., with a Cas9 nuclease and one or two Cas9 nickases) are used to create two sets of breaks to flank a T cell target knockout position, e.g., the gRNA molecules are configured such that one set of break(s) are positioned upstream and a a second set of break(s) are positioned downstream of the T cell target knockout position. In an embodiment, the breaks are positioned to avoid unwanted target chromosome elements, such as repeat elements, e.g., an *Alu* repeat.

In an embodiment, two or more (e.g., three or four) gRNA molecules are used with one Cas9 molecule. In another embodiment, when two ore more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

When more than one gene is targeted for alteration in a cell, the targeted nucleic acids may be altered, e.g., cleaved, by one or more Cas9 proteins. For example, if two genes are targeted for alteration, e.g., both genes are targeted for knockout, the same or a different Cas9 protein may be used to target each gene. In one embodiment, both genes (or each gene targeted in a cell), are cleaved by a Cas9 nuclease to generate a double stranded break. In another embodiment, both genes (or each gene targeted in a cell), are cleaved by a Cas9 nuclease to generate a double stranded break. In another embodiment, one or more genes in a cell may be altered by cleavage with a Cas9 nuclease and one or more genes in the same cell may be altered by cleavage with a Cas9 nickase. When two or more Cas9 proteins are used to cut a target nucleic acid, e.g., different genes in a cell, the Cas9 proteins may be from different bacterial species. For example, one or more genes in a cell may be altered by cleavage with a Cas9 protein from one bacterial species, and one or more genes in the same cell may be altered by cleavage with a Cas9 protein from a different bacterial species. It is contemplated that when two or more Cas9 proteins from different species are used that they may be delivered at the same time or delivered sequentially to control specificity of cleavage in the desired gene at the desired position in the target nucleic acid.

In some embodiments, the targeting domain of the first gRNA molecule and the targeting domain of the second gRNA molecules are complementary to opposite strands of the target nucleic acid molecule. In some embodiments, the gRNA molecule and the second gRNA molecule are configured such that the PAMs are oriented outward.

### Knocking down one or more T cell-expressed genes, e.g., the FAS, BID, CTLA4, PDCD1, CBLB. and/or PTPN6 gene mediated by an enzymatically inactive Cas9 (eiCas9) molecule

A targeted knockdown approach reduces or eliminates expression of functional FAS, BID, CTLA4, PDCD1, CBLB, and/or PTPN6 gene product. As described herein, in an embodiment, a targeted knockdown is mediated by targeting an enzymatically inactive Cas9 (eiCas9) molecule or an eiCas9 fused to a transcription repressor domain or chromatin modifying protein to alter transcription, e.g., to block, reduce, or decrease transcription, of one or more T cell-expressed genes, e.g., the FAS, BID, CTLA4, PDCD1, CBLB, and/or PTPN6 gene.

Methods and compositions discussed herein may be used to alter the expression of one or more T cell-expressed genes, e.g., the FAS, BID, CTLA4, PDCD1, CBLB, and/or PTPN6 gene to treat or prevent HIV infection or AIDS by targeting a promoter region of one or more T cell-expressed genes, e.g., the FAS, BID, CTLA4, PDCD1, CBLB, and/or PTPN6 gene. In an embodiment, the promoter region is targeted to knock down expression of one or more T cell-expressed genes, e.g., the FAS, BID, CTLA4, PDCD1, CBLB, and/or PTPN6 gene. A targeted knockdown approach reduces or eliminates expression of functional FAS, BID, CTLA4, PDCD1, CBLB, and/or PTPN6 gene product. As described herein, in an embodiment, a targeted knockdown is mediated by targeting an enzymatically inactive Cas9 (eiCas9) or an eiCas9 fusion protein (e.g., an eiCas9 fused to a transcription repressor domain or chromatin modifying protein) to alter transcription, e.g., to block, reduce, or decrease transcription, of one or more T cell-expressed genes, e.g., the FAS, BID, CTLA4, PDCD1, CBLB, P and/or PTPN6 gene.

In an embodiment, one or more eiCas9s may be used to block binding of one or more endogenous transcription factors. In another embodiment, an eiCas9 can be fused to a chromatin modifying protein. Altering chromatin status can result in decreased expression of the target gene. One or more eiCas9s fused to one or more chromatin modifying proteins may be used to alter chromatin status.

When more than one gene is targeted for alteration in a cell, the targeted nucleic acids may be altered, e.g., by one or more eiCas9 proteins or eiCas9 fusion proteins. When two or more eiCas9 proteins or eiCas9 fusion proteins are used the eiCas9 proteins or eiCas9 fusion proteins may be from different bacterial species. For example, one or more genes in a cell may be altered with an eiCas9 protein or eiCas9 fusion protein from one bacterial species, and one or more genes in the same cell may be altered with an eiCas9 protein or eiCas9 fusion protein from a different bacterial species. It is contemplated that when two or more eiCas9 proteins or eiCas9 fusion proteins from different species are used that they may be delivered at the same time or delivered sequentially to control specificity of cleavage in the desired gene at the desired position in the target nucleic acid.

While not wishing to be bound by theory, it is considered that adoptive transfer of genetically engineered T cells may provide a potential treatment for cancer. Genes encoding cell surface receptors are inserted into the T cells. The genetically engineered T cells are able to detect tumor associated antigens, which can be used to discriminate tumor cells from most normal tissues.

Knockout or knockdown of one or two alleles of the target gene (e.g., *FAS, BID, CTLA4, PDCD1, CBLB, PTPN6, TRAC* or *TRBC* gene) may be performed after disease onset, but preferably early in the disease course.

### I. gRNA Molecules

A gRNA molecule, as that term is used herein, refers to a nucleic acid that promotes the specific targeting or homing of a gRNA molecule/Cas9 molecule complex to a target nucleic acid. gRNA molecules can be unimolecular (having a single RNA molecule), sometimes referred to herein as "chimeric" gRNAs, or modular (comprising more than one, and typically two, separate RNA molecules). A gRNA molecule comprises a number of domains. The gRNA molecule domains are described in more detail below.

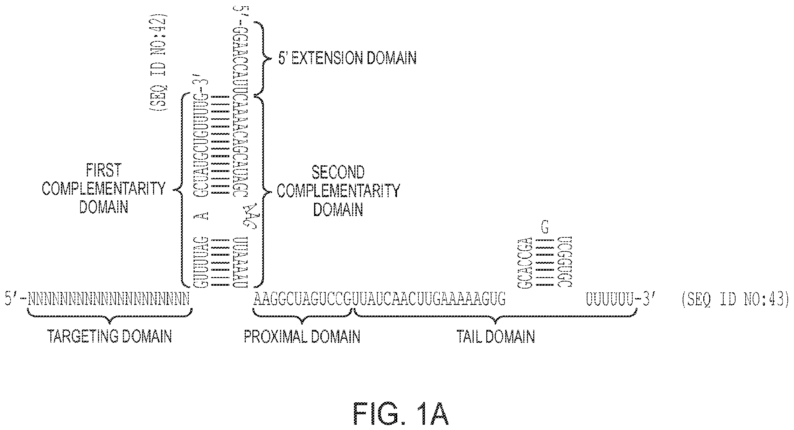

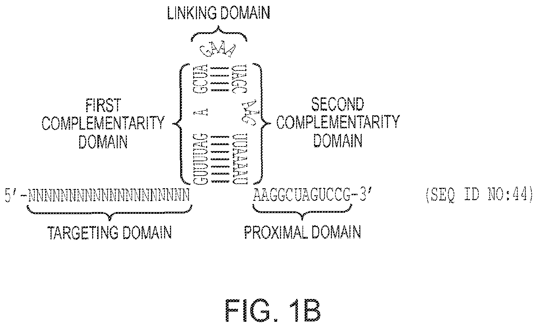

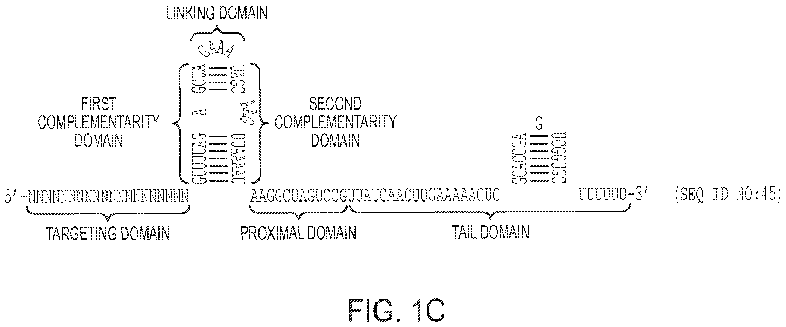

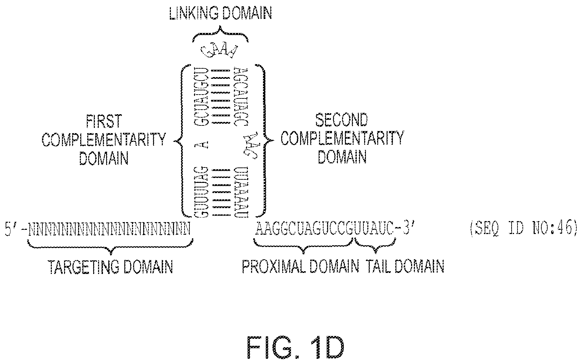

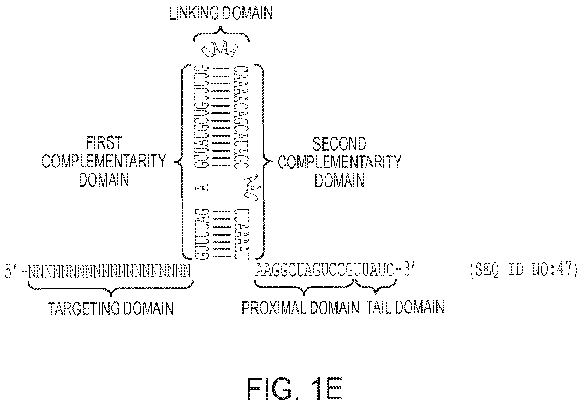

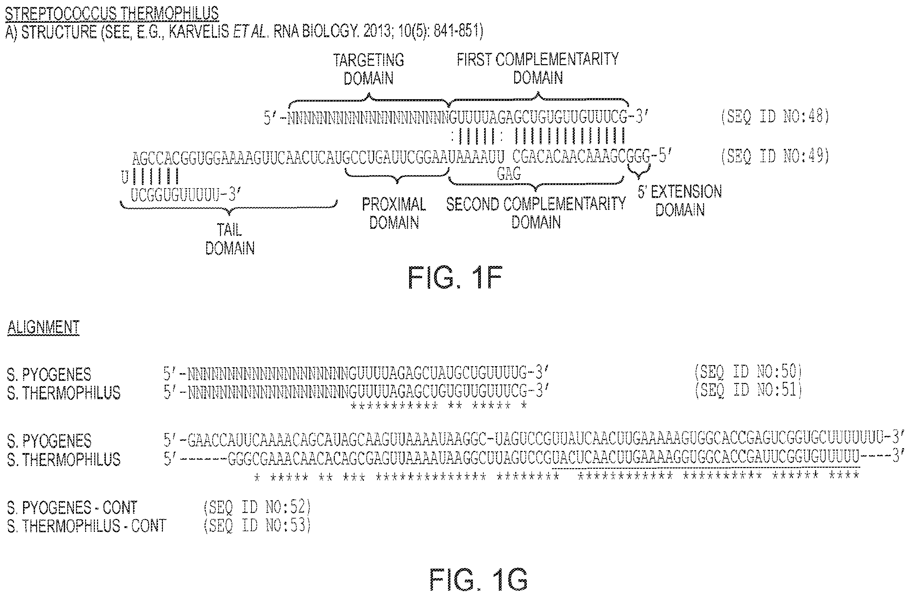

Several exemplary gRNA structures, with domains indicated thereon, are provided in **Fig. 1****.** While not wishing to be bound by theory, with regard to the three dimensional form, or intra- or inter-strand interactions of an active form of a gRNA, regions of high complementarity are sometimes shown as duplexes in Fig. 1 and other depictions provided herein.

In an embodiment, a unimolecular, or chimeric, gRNA comprises, preferably from 5' to 3':
a targeting domain (which is complementary to a target nucleic acid in the *FAS, BID, CTLA4, PDCD1, CBLB, PTPN6, TRAC* or *TRBC* gene, e.g., a targeting domain from any of **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table** 32;
a first complementarity domain;
a linking domain;
a second complementarity domain (which is complementary to the first complementarity domain);
a proximal domain; and
optionally, a tail domain.

In an embodiment, a modular gRNA comprises:
a first strand comprising, preferably from 5' to 3';
   a targeting domain (which is complementary to a target nucleic acid in the *FAS, BID, CTLA4, PDCD1, CBLB, PTPN6, TRAC* or *TRBC* gene, e.g., a targeting domain from **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32**; and
   a first complementarity domain; and
a second strand, comprising, preferably from 5' to 3':
   optionally, a 5' extension domain;
   a second complementarity domain;
   a proximal domain; and
   optionally, a tail domain.

The domains are discussed briefly below:

### The Targeting Domain

**Fig. 1** provides examples of the placement of targeting domains.

The targeting domain comprises a nucleotide sequence that is complementary, e.g., at least 80, 85, 90, 95, 98 or 99% complementary, e.g., fully complementary, to the target sequence on the target nucleic acid. The targeting domain is part of an RNA molecule and will therefore comprise the base uracil (U), while any DNA encoding the gRNA molecule will comprise the base thymine (T). While not wishing to be bound by theory, in an embodiment, it is believed that the complementarity of the targeting domain with the target sequence contributes to specificity of the interaction of the gRNA molecule/Cas9 molecule complex with a target nucleic acid. It is understood that in a targeting domain and target sequence pair, the uracil bases in the targeting domain will pair with the adenine bases in the target sequence. In an embodiment, the target domain itself comprises in the 5' to 3' direction, an optional secondary domain, and a core domain. In an embodiment, the core domain is fully complementary with the target sequence. In an embodiment, the targeting domain is 5 to 50 nucleotides in length. The strand of the target nucleic acid with which the targeting domain is complementary is referred to herein as the complementary strand. Some or all of the nucleotides of the domain can have a modification, e.g., modification found in Section VIII herein.
In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

Targeting domains are discussed in more detail below.

### The First Complementarity Domain

**Figs. 1A-1G** provide examples of first complementarity domains.

The first complementarity domain is complementary with the second complementarity domain, and in an embodiment, has sufficient complementarity to the second complementarity domain to form a duplexed region under at least some physiological conditions. In an embodiment, the first complementarity domain is 5 to 30 nucleotides in length. In an embodiment, the first complementarity domain is 5 to 25 nucleotides in length. In an embodiment, the first complementary domain is 7 to 25 nucleotides in length. In an embodiment, the first complementary domain is 7 to 22 nucleotides in length. In an embodiment, the first complementary domain is 7 to 18 nucleotides in length. In an embodiment, the first complementary domain is 7 to 15 nucleotides in length. In an embodiment, the first complementary domain is 5, 6, 7, 8, 9, 10, 11, 12, 13, 14, 15, 16, 17, 18, 19, 20, 21, 22, 23, 24, or 25 nucleotides in length.

In an embodiment, the first complementarity domain comprises 3 subdomains, which, in the 5' to 3' direction are: a 5' subdomain, a central subdomain, and a 3' subdomain. In an embodiment, the 5' subdomain is 4-9, e.g., 4, 5, 6, 7, 8 or 9 nucleotides in length. In an embodiment, the central subdomain is 1, 2, or 3, e.g., 1, nucleotide in length. In an embodiment, the 3' subdomain is 3 to 25, e.g., 4-22, 4-18, or 4 to 10, or 3, 4, 5, 6, 7, 8, 9, 10, 11, 12, 13, 14, 15, 16, 17, 18, 19, 20, 21, 22, 23, 24, or 25, nucleotides in length.

The first complementarity domain can share homology with, or be derived from, a naturally occurring first complementarity domain. In an embodiment, it has at least 50% homology with a first complementarity domain disclosed herein, e.g., an *S. pyogenes, S. aureus, N. meningtidis,* or *S. thermophilus,* first complementarity domain.

Some or all of the nucleotides of the first complementarity domain can have a modification, e.g., a modification found in Section VIII herein.

First complementarity domains are discussed in more detail below.

### The Linking Domain

**Figs. 1A-1G** provide examples of linking domains.

A linking domain serves to link the first complementarity domain with the second complementarity domain of a unimolecular gRNA. The linking domain can link the first and second complementarity domains covalently or non-covalently. In an embodiment, the linkage is covalent. In an embodiment, the linking domain covalently couples the first and second complementarity domains, see, e.g., **Figs. 1B-1E****.** In an embodiment, the linking domain is, or comprises, a covalent bond interposed between the first complementarity domain and the second complementarity domain. Typically the linking domain comprises one or more, e.g., 2, 3, 4, 5, 6, 7, 8, 9, or 10 nucleotides.

In modular gRNA molecules the two molecules are associated by virtue of the hybridization of the complementarity domains see e.g., **Fig. 1A**.

A wide variety of linking domains are suitable for use in unimolecular gRNA molecules. Linking domains can consist of a covalent bond, or be as short as one or a few nucleotides, e.g., 1, 2, 3, 4, or 5 nucleotides in length. In an embodiment, a linking domain is 2, 3, 4, 5, 6, 7, 8, 9, 10, 15, 20, or 25 or more nucleotides in length. In an embodiment, a linking domain is 2 to 50, 2 to 40, 2 to 30, 2 to 20, 2 to 10, or 2 to 5 nucleotides in length. In an embodiment, a linking domain shares homology with, or is derived from, a naturally occurring sequence, e.g., the sequence of a tracrRNA that is 5' to the second complementarity domain. In an embodiment, the linking domain has at least 50% homology with a linking domain disclosed herein.

Some or all of the nucleotides of the linking domain can have a modification, e.g., a modification found in Section VIII herein.

Linking domains are discussed in more detail below.

### The 5' Extension Domain

In an embodiment, a modular gRNA can comprise additional sequence, 5' to the second complementarity domain, referred to herein as the 5' extension domain, see, e.g., **Fig. 1A****.** In an embodiment, the 5' extension domain is, 2-10, 2-9, 2-8, 2-7, 2-6, 2-5, or 2-4 nucleotides in length. In an embodiment, the 5' extension domain is 2, 3, 4, 5, 6, 7, 8, 9, or 10 or more nucleotides in length.

### The Second Complementarity Domain

**Figs. 1A-1G** provide examples of second complementarity domains.

The second complementarity domain is complementary with the first complementarity domain, and in an embodiment, has sufficient complementarity to the second complementarity domain to form a duplexed region under at least some physiological conditions. In an embodiment, e.g., as shown in Figs. **1A-1B****,** the second complementarity domain can include sequence that lacks complementarity with the first complementarity domain, e.g., sequence that loops out from the duplexed region.

In an embodiment, the second complementarity domain is 5 to 27 nucleotides in length. In an embodiment, it is longer than the first complementarity region. In an embodiment the second complementary domain is 7 to 27 nucleotides in length. In an embodiment, the second complementary domain is 7 to 25 nucleotides in length. In an embodiment, the second complementary domain is 7 to 20 nucleotides in length. In an embodiment, the second complementary domain is 7 to 17 nucleotides in length. In an embodiment, the complementary domain is 5, 6, 7, 8, 9, 10, 11, 12, 13, 14, 15, 16, 17, 18, 19, 20, 21, 22, 23, 24, 25, or 26 nucleotides in length.

In an embodiment, the second complementarity domain comprises 3 subdomains, which, in the 5' to 3' direction are: a 5' subdomain, a central subdomain, and a 3' subdomain. In an embodiment, the 5' subdomain is 3 to 25, e.g., 4 to 22, 4 to 18, or 4 to 10, or 3, 4, 5, 6, 7, 8, 9, 10, 11, 12, 13, 14, 15, 16, 17, 18, 19, 20, 21, 22, 23, 24, or 25 nucleotides in length. In an embodiment, the central subdomain is 1, 2, 3, 4 or 5, e.g., 3, nucleotides in length. In an embodiment, the 3' subdomain is 4 to 9, e.g., 4, 5, 6, 7, 8 or 9 nucleotides in length.

In an embodiment, the 5' subdomain and the 3' subdomain of the first complementarity domain, are respectively, complementary, e.g., fully complementary, with the 3' subdomain and the 5' subdomain of the second complementarity domain.

The second complementarity domain can share homology with or be derived from a naturally occurring second complementarity domain. In an embodiment, it has at least 50% homology with a second complementarity domain disclosed herein, e.g., an *S. pyogenes, S. aureus, N. meningtidis,* or *S. thermophilus,* first complementarity domain.

Some or all of the nucleotides of the second complementarity domain can have a modification, e.g., a modification found in Section VIII herein.

### A Proximal domain

**Figs. 1A-1G** provide examples of proximal domains.

In an embodiment, the proximal domain is 5 to 20 nucleotides in length. In an embodiment, the proximal domain can share homology with or be derived from a naturally occurring proximal domain. In an embodiment, it has at least 50% homology with a proximal domain disclosed herein, e.g., an *S*. *pyogenes, S. aureus, N. meningtidis,* or *S. thermophilus,* proximal domain.

Some or all of the nucleotides of the proximal domain can have a modification, e.g., a modification found in Section VIII herein.

### A Tail Domain

**Figs. 1A-1G** provide examples of tail domains.

As can be seen by inspection of the tail domains in **Fig. 1A** and **Figs. 1B****-1F,** a broad spectrum of tail domains are suitable for use in gRNA molecules. In an embodiment, the tail domain is 0 (absent), 1, 2, 3, 4, 5, 6, 7, 8, 9, or 10 nucleotides in length. In embodiment, the tail domain nucleotides are from or share homology with sequence from the 5' end of a naturally occurring tail domain, see e.g., **Fig. 1D** **or** **1E**. In an embodiment, the tail domain includes sequences that are complementary to each other and which, under at least some physiological conditions, form a duplexed region.

In an embodiment, the tail domain is absent or is 1 to 50 nucleotides in length. In an embodiment, the tail domain can share homology with or be derived from a naturally occurring proximal tail domain. In an embodiment, it has at least 50% homology with a tail domain disclosed herein, e.g., an *S. pyogenes, S. aureus, N. meningtidis,* or *S. thermophilus,* tail domain.

In an embodiment, the tail domain includes nucleotides at the 3' end that are related to the method of in vitro or in vivo transcription. When a T7 promoter is used for in vitro transcription of the gRNA, these nucleotides may be any nucleotides present before the 3' end of the DNA template. When a U6 promoter is used for in vivo transcription, these nucleotides may be the sequence UUUUUU. When alternate pol-III promoters are used, these nucleotides may be various numbers or uracil bases or may include alternate bases.

The domains of gRNA molecules are described in more detail below.

### The Targeting Domain

The "targeting domain" of the gRNA is complementary to the "target domain" on the target nucleic acid. The strand of the target nucleic acid comprising the core domain target is referred to herein as the "complementary strand" of the target nucleic acid. Guidance on the selection of targeting domains can be found, e.g., in Fu Y et al., Nat Biotechnol 2014 (doi: 10.1038/nbt.2808) and Sternberg SH et al., Nature 2014 (doi: 10.1038/naturel3011).

In an embodiment, the targeting domain is 16, 17, 18, 19, 20, 21, 22, 23, 24, 25 or 26 nucleotides in length.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides. In an embodiment, the targeting domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the targeting domain is 20+/-5 nucleotides in length.

In an embodiment, the targeting domain is 20+/-10, 30+/-10, 40+/-10, 50+/-10, 60+/-10, 70+/-10, 80+/-10, 90+/-10, or 100+/-10 nucleotides, in length.

In an embodiment, the targeting domain is 30+/-10 nucleotides in length.

In an embodiment, the targeting domain is 10 to 100, 10 to 90, 10 to 80, 10 to 70, 10 to 60, 10 to 50, 10 to 40, 10 to 30, 10 to 20 or 10 to 15 nucleotides in length.

In other embodiments, the targeting domain is 20 to 100, 20 to 90, 20 to 80, 20 to 70, 20 to 60, 20 to 50, 20 to 40, 20 to 30, or 20 to 25 nucleotides in length.

Typically the targeting domain has full complementarity with the target sequence. In some embodiments the targeting domain has or includes 1, 2, 3, 4, 5, 6, 7 or 8 nucleotides that are not complementary with the corresponding nucleotide of the targeting domain.

In an embodiment, the target domain includes 1, 2, 3, 4 or 5 nucleotides that are complementary with the corresponding nucleotide of the targeting domain within 5 nucleotides of its 5' end. In an embodiment, the target domain includes 1, 2, 3, 4 or 5 nucleotides that are complementary with the corresponding nucleotide of the targeting domain within 5 nucleotides of its 3' end.

In an embodiment, the target domain includes 1, 2, 3, or 4 nucleotides that are not complementary with the corresponding nucleotide of the targeting domain within 5 nucleotides of its 5' end. In an embodiment, the target domain includes 1, 2, 3, or 4 nucleotides that are not complementary with the corresponding nucleotide of the targeting domain within 5 nucleotides of its 3' end.

In an embodiment, the degree of complementarity, together with other properties of the gRNA, is sufficient to allow targeting of a Cas9 molecule to the target nucleic acid.

In some embodiments, the targeting domain comprises two consecutive nucleotides that are not complementary to the target domain ("non-complementary nucleotides"), e.g., two consecutive noncomplementary nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides within 5 nucleotides of the 5' end of the targeting domain, within 5 nucleotides of the 3' end of the targeting domain, or within a region that is more than 5 nucleotides away from one or both ends of the targeting domain, are not complementary to the targeting domain.

In an embodiment, there are no noncomplementary nucleotides within 5 nucleotides of the 5' end of the targeting domain, within 5 nucleotides of the 3' end of the targeting domain, or within a region that is more than 5 nucleotides away from one or both ends of the targeting domain.

In an embodiment, the targeting domain nucleotides do not comprise modifications, e.g., modifications of the type provided in Section VIII. However, in an embodiment, the targeting domain comprises one or more modifications, e.g., modifications that render it less susceptible to degradation or more bio-compatible, e.g., less immunogenic. By way of example, the backbone of the targeting domain can be modified with a phosphorothioate, or other modification(s) from Section VIII. In an embodiment, a nucleotide of the targeting domain can comprise a 2' modification, e.g., a 2-acetylation, e.g., a 2' methylation, or other modification(s) from Section VIII.

In some embodiments, the targeting domain includes 1, 2, 3, 4, 5, 6, 7 or 8 or more modifications. In an embodiment, the targeting domain includes 1, 2, 3, or 4 modifications within 5 nucleotides of its 5' end. In an embodiment, the targeting domain comprises as many as 1, 2, 3, or 4 modifications within 5 nucleotides of its 3' end.

In some embodiments, the targeting domain comprises modifications at two consecutive nucleotides, e.g., two consecutive nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides are modified within 5 nucleotides of the 5' end of the targeting domain, within 5 nucleotides of the 3' end of the targeting domain, or within a region that is more than 5 nucleotides away from one or both ends of the targeting domain. In an embodiment, no nucleotide is modified within 5 nucleotides of the 5' end of the targeting domain, within 5 nucleotides of the 3' end of the targeting domain, or within a region that is more than 5 nucleotides away from one or both ends of the targeting domain.

Modifications in the targeting domain can be selected to not interfere with targeting efficacy, which can be evaluated by testing a candidate modification in the system described in Section IV. gRNAs having a candidate targeting domain having a selected length, sequence, degree of complementarity, or degree of modification, can be evaluated in a system in Section IV. The candidate targeting domain can be placed, either alone, or with one or more other candidate changes in a gRNA molecule/Cas9 molecule system known to be functional with a selected target and evaluated.

In some embodiments, all of the modified nucleotides are complementary to and capable of hybridizing to corresponding nucleotides present in the target domain. In other embodiments, 1, 2, 3, 4, 5, 6, 7 or 8 or more modified nucleotides are not complementary to or capable of hybridizing to corresponding nucleotides present in the target domain.

In an embodiment, the targeting domain comprises, preferably in the 5'→3' direction: a secondary domain and a core domain. These domains are discussed in more detail below.

### The Core Domain and Secondary Domain of the Targeting Domain

The "core domain" of the targeting domain is complementary to the "core domain target" on the target nucleic acid. In an embodiment, the core domain comprises about 8 to about 13 nucleotides from the 3' end of the targeting domain (e.g., the most 3' 8 to 13 nucleotides of the targeting domain).

In an embodiment, the secondary domain is absent or optional.

In an embodiment, the core domain and targeting domain are independently 6 +/-2, 7+/-2, 8+/-2, 9+/-2, 10+/-2, 11+/-2, 12+/-2, 13+/-2, 14+/-2, 15+/-2, or 16+-2 nucleotides in length.

In an embodiment, the core domain and targeting domain are independently 10+/-2 nucleotides in length.

In an embodiment, the core domain and targeting domain are independently 10+/-4 nucleotides in length.

In an embodiment, the core domain and targeting domain are independently 6, 7, 8, 9, 10, 11, 12, 13, 14, 15 or 16 nucleotides in length.

In an embodiment, the core and targeting domain are independently 3 to 20, 4 to 20, 5 to 20, 6 to 20, 7 to 20, 8 to 20, 9 to 20 10 to 20 or 15 to 20 nucleotides in length.

In an embodiment, the core and targeting domain, are independently 3 to 15, e.g., 6 to 15, 7 to 14, 7 to 13, 6 to 12, 7 to 12, 7 to 11, 7 to 10, 8 to 14, 8 to 13, 8 to 12, 8 to 11, 8 to 10 or 8 to 9 nucleotides in length.

The core domain is complementary with the core domain target. Typically the core domain has exact complementarity with the core domain target. In some embodiments, the core domain can have 1, 2, 3, 4 or 5 nucleotides that are not complementary with the corresponding nucleotide of the core domain. In an embodiment, the degree of complementarity, together with other properties of the gRNA, is sufficient to allow targeting of a Cas9 molecule to the target nucleic acid.

The "secondary domain" of the targeting domain of the gRNA is complementary to the "secondary domain target" of the target nucleic acid.

In an embodiment, the secondary domain is positioned 5' to the core domain.

In an embodiment, the secondary domain is absent or optional.

In an embodiment, if the targeting domain is 26 nucleotides in length and the core domain (counted from the 3' end of the targeting domain) is 8 to 13 nucleotides in length, the secondary domain is 12 to 17 nucleotides in length.

In an embodiment, if the targeting domain is 25 nucleotides in length and the core domain (counted from the 3' end of the targeting domain) is 8 to 13 nucleotides in length, the secondary domain is 12 to 17 nucleotides in length.

In an embodiment, if the targeting domain is 24 nucleotides in length and the core domain (counted from the 3' end of the targeting domain) is 8 to 13 nucleotides in length, the secondary domain is 11 to 16 nucleotides in length.

In an embodiment, if the targeting domain is 23 nucleotides in length and the core domain (counted from the 3' end of the targeting domain) is 8 to 13 nucleotides in length, the secondary domain is 10 to 15 nucleotides in length.

In an embodiment, if the targeting domain is 22 nucleotides in length and the core domain (counted from the 3' end of the targeting domain) is 8 to 13 nucleotides in length, the secondary domain is 9 to 14 nucleotides in length.

In an embodiment, if the targeting domain is 21 nucleotides in length and the core domain (counted from the 3' end of the targeting domain) is 8 to 13 nucleotides in length, the secondary domain is 8 to 13 nucleotides in length.

In an embodiment, if the targeting domain is 20 nucleotides in length and the core domain (counted from the 3' end of the targeting domain) is 8 to 13 nucleotides in length, the secondary domain is 7 to 12 nucleotides in length.

In an embodiment, if the targeting domain is 19 nucleotides in length and the core domain (counted from the 3' end of the targeting domain) is 8 to 13 nucleotides in length, the secondary domain is 6 to 11 nucleotides in length.

In an embodiment, if the targeting domain is 18 nucleotides in length and the core domain (counted from the 3' end of the targeting domain) is 8 to 13 nucleotides in length, the secondary domain is 5 to 10 nucleotides in length.

In an embodiment, if the targeting domain is 17 nucleotides in length and the core domain (counted from the 3' end of the targeting domain) is 8 to 13 nucleotides in length, the secondary domain is 4 to 9 nucleotides in length.

In an embodiment, if the targeting domain is 16 nucleotides in length and the core domain (counted from the 3' end of the targeting domain) is 8 to 13 nucleotides in length, the secondary domain is 3 to 8 nucleotides in length.

In an embodiment, the secondary domain is 0, 1, 2, 3, 4, 5, 6, 7, 8, 9, 10, 11, 12, 13, 14 or 15 nucleotides in length.

The secondary domain is complementary with the secondary domain target. Typically the secondary domain has exact complementarity with the secondary domain target. In some embodiments the secondary domain can have 1, 2, 3, 4 or 5 nucleotides that are not complementary with the corresponding nucleotide of the secondary domain. In an embodiment, the degree of complementarity, together with other properties of the gRNA, is sufficient to allow targeting of a Cas9 molecule to the target nucleic acid.

In an embodiment, the core domain nucleotides do not comprise modifications, e.g., modifications of the type provided in Section VIII. However, in an embodiment, the core domain comprises one or more modifications, e.g., modifications that render it less susceptible to degradation or more bio-compatible, e.g., less immunogenic. By way of example, the backbone of the core domain can be modified with a phosphorothioate, or other modification(s) from Section VIII. In an embodiment a nucleotide of the core domain can comprise a 2' modification (e.g., a modification at the 2' position on ribose), e.g., a 2-acetylation, e.g., a 2' methylation, or other modification(s) from Section VIII. Typically, a core domain will contain no more than 1, 2, or 3 modifications.

Modifications in the core domain can be selected to not interfere with targeting efficacy, which can be evaluated by testing a candidate modification in the system described in Section IV. gRNAs having a candidate core domain having a selected length, sequence, degree of complementarity, or degree of modification, can be evaluated in the system described at Section IV. The candidate core domain can be placed, either alone, or with one or more other candidate changes in a gRNA molecule/Cas9 molecule system known to be functional with a selected target and evaluated.

In an embodiment, the secondary domain nucleotides do not comprise modifications, e.g., modifications of the type provided in Section VIII. However, in an embodiment, the secondary domain comprises one or more modifications, e.g., modifications that render it less susceptible to degradation or more bio-compatible, e.g., less immunogenic. By way of example, the backbone of the secondary domain can be modified with a phosphorothioate, or other modification(s) from Section VIII. In an embodiment a nucleotide of the secondary domain can comprise a 2' modification, e.g., a 2-acetylation, e.g., a 2' methylation, or other modification(s) from Section VIII. Typically, a secondary domain will contain no more than 1, 2, or 3 modifications.

Modifications in the secondary domain can be selected to not interfere with targeting efficacy, which can be evaluated by testing a candidate modification in the system described in Section IV. gRNAs having a candidate secondary domain having a selected length, sequence, degree of complementarity, or degree of modification, can be evaluated in the system described at Section IV. The candidate secondary domain can be placed, either alone, or with one or more other candidate changes in a gRNA molecule/Cas9 molecule system known to be functional with a selected target and evaluated.

In an embodiment, (1) the degree of complementarity between the core domain and its target, and (2) the degree of complementarity between the secondary domain and its target, may differ. In an embodiment, (1) may be greater than (2). Tn an embodiment, (1) may be less than (2). In an embodiment, (1) and (2) are the same, e.g., each may be completely complementary with its target.

In an embodiment, (1) the number of modifications (e.g., modifications from Section VIII) of the nucleotides of the core domain and (2) the number of modification (e.g., modifications from Section VIII) of the nucleotides of the secondary domain, may differ. In an embodiment, (1) may be less than (2). In an embodiment, (1) may be greater than (2). In an embodiment, (1) and (2) may be the same, e.g., each may be free of modifications.

### The First and Second Complementarity Domains

The first complementarity domain is complementary with the second complementarity domain.

Typically the first complementarity domain does not have exact complementarity with the second complementarity domain target. In some embodiments, the first complementarity domain can have 1, 2, 3, 4 or 5 nucleotides that are not complementary with the corresponding nucleotide of the second complementarity domain. In an embodiment, 1, 2, 3, 4, 5 or 6, e.g., 3 nucleotides, will not pair in the duplex, and, e.g., form a non-duplexed or looped-out region. In an embodiment, an unpaired, or loop-out, region, e.g., a loop-out of 3 nucleotides, is present on the second complementarity domain. In an embodiment, the unpaired region begins 1, 2, 3, 4, 5, or 6, e.g., 4, nucleotides from the 5' end of the second complementarity domain.

In an embodiment, the degree of complementarity, together with other properties of the gRNA, is sufficient to allow targeting of a Cas9 molecule to the target nucleic acid.

In an embodiment, the first and second complementarity domains are:
independently, 6 +/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length;
independently, 6, 7, 8, 9, 10, 11, 12, 13, 14, 14, 16, 17, 18, 19, 20, 21, 22, 23, 24, 25, or 26, nucleotides in length; or
independently, 5 to 24, 5 to 23, 5 to 22, 5 to 21, 5 to 20, 7 to 18, 9 to 16, or 10 to 14 nucleotides in length.

In an embodiment, the second complementarity domain is longer than the first complementarity domain, e.g., 2, 3, 4, 5, or 6, e.g., 6, nucleotides longer.

In an embodiment, the first and second complementary domains, independently, do not comprise modifications, e.g., modifications of the type provided in Section VIII.

In an embodiment, the first and second complementary domains, independently, comprise one or more modifications, e.g., modifications that the render the domain less susceptible to degradation or more bio-compatible, e.g., less immunogenic. By way of example, the backbone of the domain can be modified with a phosphorothioate, or other modification(s) from Section VTTT. In an embodiment a nucleotide of the domain can comprise a 2' modification, e.g., a 2-acetylation, e.g., a 2' methylation, or other modification(s) from Section VIII.

In an embodiment, the first and second complementary domains, independently, include 1, 2, 3, 4, 5, 6, 7 or 8 or more modifications. In an embodiment, the first and second complementary domains, independently, include 1, 2, 3, or 4 modifications within 5 nucleotides of its 5' end. In an embodiment, the first and second complementary domains, independently, include as many as 1, 2, 3, or 4 modifications within 5 nucleotides of its 3' end.

In an embodiment, the first and second complementary domains, independently, include modifications at two consecutive nucleotides, e.g., two consecutive nucleotides that are within 5 nucleotides of the 5' end of the domain, within 5 nucleotides of the 3' end of the domain, or more than 5 nucleotides away from one or both ends of the domain. In an embodiment, the first and second complementary domains, independently, include no two consecutive nucleotides that are modified, within 5 nucleotides of the 5' end of the domain, within 5 nucleotides of the 3' end of the domain, or within a region that is more than 5 nucleotides away from one or both ends of the domain. In an embodiment, the first and second complementary domains, independently, include no nucleotide that is modified within 5 nucleotides of the 5' end of the domain, within 5 nucleotides of the 3' end of the domain, or within a region that is more than 5 nucleotides away from one or both ends of the domain.

Modifications in a complementarity domain can be selected to not interfere with targeting efficacy, which can be evaluated by testing a candidate modification in the system described in Section IV. gRNAs having a candidate complementarity domain having a selected length, sequence, degree of complementarity, or degree of modification, can be evaluated in the system described in Section IV. The candidate complementarity domain can be placed, either alone, or with one or more other candidate changes in a gRNA molecule/Cas9 molecule system known to be functional with a selected target and evaluated.

In an embodiment, the first complementarity domain has at least 60, 70, 80, 85%, 90% or 95% homology with, or differs by no more than 1, 2, 3, 4, 5, or 6 nucleotides from, a reference first complementarity domain, e.g., a naturally occurring, e.g., an *S. pyogenes, S. aureus, N. meningtidis,* or *S. thermophilus,* first complementarity domain, or a first complementarity domain described herein, e.g., from **Figs. 1A-1G****.**

In an embodiment, the second complementarity domain has at least 60, 70, 80, 85%, 90%, or 95% homology with, or differs by no more than 1, 2, 3, 4, 5, or 6 nucleotides from, a reference second complementarity domain, e.g., a naturally occurring, e.g., an *S. pyogenes, S. aureus, N. meningtidis,* or *S. thermophilus,* second complementarity domain, or a second complementarity domain described herein, e.g., from **Figs. 1A-1G****.**

The duplexed region formed by first and second complementarity domains is typically 6, 7, 8, 9, 10, 11, 12, 13, 14, 15, 16, 17, 18, 19, 20, 21 or 22 base pairs in length (excluding any looped out or unpaired nucleotides).

In some embodiments, the first and second complementarity domains, when duplexed, comprise 11 paired nucleotides, for example, in the gRNA sequence (one paired strand underlined, one bolded):

NNNNNNNNNNNNNNNNNNNNGUUUUAGAGCUAGAAAUAGCAAGUUAAAAUAAGGCUAG
UCCGUUAUCAACUUGAAAAAGUGGCACCGAGUCGGUGC (SEQ ID NO:5).

In some embodiments, the first and second complementarity domains, when duplexed, comprise 15 paired nucleotides, for example in the gRNA sequence (one paired strand underlined, one bolded):

NNNNNNNNNNNNNNNNNNNNGUUUUAGAGCUAUGCUGAAAAGCAUAGCAAGUUAAAAU
AAGGCUAGUCCGUUAUCAACUUGAAAAAGUGGCACCGAGUCGGUGC (SEQ ID NO:27).

In some embodiments the first and second complementarity domains, when duplexed, comprise 16 paired nucleotides, for example in the gRNA sequence (one paired strand underlined, one bolded):

NNNNNNNNNNNNNNNNNNNNGUUUUAGAGCUAUGCUGGAAACAGCAUAGCAAGUUAAA
AUAAGGCUAGUCCGUUAUCAACUUGAAAAAGUGGCACCGAGUCGGUGC (SEQ ID NO:28).

In some embodiments the first and second complementarity domains, when duplexed, comprise 21 paired nucleotides, for example in the gRNA sequence (one paired strand underlined, one bolded):

NNNNNNNNNNNNNNNNNNNNGUUUUAGAGCUAUGCUGUUUUGGAAACAAAACAGCAUA
GCAAGUUAAAAUAAGGCUAGUCCGUUAUCAACUUGAAAAAGUGGCACCGAGUCGGUGC
(SEQ ID NO:29).

In some embodiments, nucleotides are exchanged to remove poly-U tracts, for example in the gRNA sequences (exchanged nucleotides underlined): 
NNNNNNNNNNNNNNNNNNNNGUAUUAGAGCUAGAAAUAGCAAGUUAAUAUAAGGCUAGU
CCGUUAUCAACUUGAAAAAGUGGCACCGAGUCGGUGC (SEQ ID NO:30);
 and

NNNNNNNNNNNNNNNNNNNNGUUUAAGAGCUAGAAAUAGCAAGUUUAAAUAAGGCUAGU
CCGUUAUCAACUUGAAAAAGUGGCACCGAGUCGGUGC (SEQ ID NO:31);

NNNNNNNNNNNNNNNNNNNNGUAUUAGAGCUAUGCUGUAUUGGAAACAAUACAGCAUAG
CAAGUUAAUAUAAGGCUAGUCCGUUAUCAACUUGAAAAAGUGGCACCGAGUCGGUGC
(SEQ ID NO:32).

### The 5' Extension Domain

In an embodiment, a modular gRNA can comprise additional sequence, 5' to the second complementarity domain. In an embodiment, the 5' extension domain is 2 to 10, 2 to 9, 2 to 8, 2 to 7, 2 to 6, 2 to 5, or 2 to 4 nucleotides in length. In an embodiment, the 5' extension domain is 2, 3, 4, 5, 6, 7, 8, 9, or 10 or more nucleotides in length.

In an embodiment, the 5' extension domain nucleotides do not comprise modifications, e.g., modifications of the type provided in Section VIII. However, in an embodiment, the 5' extension domain comprises one or more modifications, e.g., modifications that render it less susceptible to degradation or more bio-compatible, e.g., less immunogenic. By way of example, the backbone of the 5' extension domain can be modified with a phosphorothioate, or other modification(s) from Section VIII. In an embodiment, a nucleotide of the 5' extension domain can comprise a 2' modification, e.g., a 2-acetylation, e.g., a 2' methylation, or other modification(s) from Section VIII.

In some embodiments, the 5' extension domain can comprise as many as 1, 2, 3, 4, 5, 6, 7 or 8 modifications. In an embodiment, the 5' extension domain comprises as many as 1, 2, 3, or 4 modifications within 5 nucleotides of its 5' end, e.g., in a modular gRNA molecule. In an embodiment, the 5' extension domain comprises as many as 1, 2, 3, or 4 modifications within 5 nucleotides of its 3' end, e.g., in a modular gRNA molecule.

In some embodiments, the 5' extension domain comprises modifications at two consecutive nucleotides, e.g., two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In an embodiment, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In an embodiment, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected to not interfere with gRNA molecule efficacy, which can be evaluated by testing a candidate modification in the system described in Section IV. gRNAs having a candidate 5' extension domain having a selected length, sequence, degree of complementarity, or degree of modification, can be evaluated in the system described at Section IV. The candidate 5' extension domain can be placed, either alone, or with one or more other candidate changes in a gRNA molecule/Cas9 molecule system known to be functional with a selected target and evaluated.

In an embodiment, the 5' extension domain has at least 60, 70, 80, 85, 90, 95, 98 or 99% homology with, or differs by no more than 1, 2, 3, 4, 5, or 6 nucleotides from, a reference 5' extension domain, e.g., a naturally occurring, e.g., an *S. pyogenes, S. aureus, N. meningtidis,* or *S. thermophilus, 5'* extension domain, or a 5' extension domain described herein, e.g., from **Figs. 1A-1G****.**

### The Linking Domain

In a unimolecular gRNA molecule the linking domain is disposed between the first and second complementarity domains. In a modular gRNA molecule, the two molecules are associated with one another by the complementarity domains.

In an embodiment, the linking domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the linking domain is 20+/-10, 30+/-10, 40+/-10, 50+/-10, 60+/-10, 70+/-10, 80+/-10, 90+/-10, or 100+/-10 nucleotides, in length.

In an embodiment, the linking domain is 10 to 100, 10 to 90, 10 to 80, 10 to 70, 10 to 60, 10 to 50, 10 to 40, 10 to 30, 10 to 20 or 10 to 15 nucleotides in length. In other embodiments, the linking domain is 20 to 100, 20 to 90, 20 to 80, 20 to 70, 20 to 60, 20 to 50, 20 to 40, 20 to 30, or 20 to 25 nucleotides in length.

In an embodiment, the linking domain is 1, 2, 3, 4, 5, 6, 7, 8, 9, 10, 11, 12, 13, 14, 15, 16 17, 18, 19, or 20 nucleotides in length.

In an embodiment, the linking domain is a covalent bond.

In an embodiment, the linking domain comprises a duplexed region, typically adjacent to or within 1, 2, or 3 nucleotides of the 3' end of the first complementarity domain and/or the 5- end of the second complementarity domain. In an embodiment, the duplexed region can be 20+/-10 base pairs in length. In an embodiment, the duplexed region can be 10+/-5, 15+/-5, 20+/-5, or 30+/-5 base pairs in length. In an embodiment, the duplexed region can be 1, 2, 3, 4, 5, 6, 7, 8, 9, 10, 11, 12, 13, 14, or 15 base pairs in length.

Typically the sequences forming the duplexed region have exact complementarity with one another, though in some embodiments as many as 1, 2, 3, 4, 5, 6, 7 or 8 nucleotides are not complementary with the corresponding nucleotides.

In an embodiment, the linking domain nucleotides do not comprise modifications, e.g., modifications of the type provided in Section VIII. However, in an embodiment, the linking domain comprises one or more modifications, e.g., modifications that render it less susceptible to degradation or more bio-compatible, e.g., less immunogenic. By way of example, the backbone of the linking domain can be modified with a phosphorothioate, or other modification(s) from Section VIII. In an embodiment a nucleotide of the linking domain can comprise a 2' modification, e.g., a 2-acetylation, e.g., a 2' methylation, or other modification(s) from Section VIII. In some embodiments, the linking domain can comprise as many as 1, 2, 3, 4, 5, 6, 7 or 8 modifications.

Modifications in a linking domain can be selected to not interfere with targeting efficacy, which can be evaluated by testing a candidate modification in the system described in Section IV. gRNAs having a candidate linking domain having a selected length, sequence, degree of complementarity, or degree of modification, can be evaluated a system described in Section IV. A candidate linking domain can be placed, either alone, or with one or more other candidate changes in a gRNA molecule/Cas9 molecule system known to be functional with a selected target and evaluated.

In an embodiment, the linking domain has at least 60, 70, 80, 85, 90, 95, 98 or 99% homology with, or differs by no more than 1, 2, 3, 4, 5 ,or 6 nucleotides from, a reference linking domain, e.g., a linking domain described herein, e.g., from **Figs. 1A-1G****.**

### The Proximal Domain

In an embodiment, the proximal domain is 6 +/-2, 7+/-2, 8+/-2, 9+/-2, 10+/-2, 11+/-2, 12+/-2, 13+/-2, 14+/-2, 14+/-2, 16+/-2, 17+/-2, or 18+/-2 nucleotides in length.

In an embodiment, the proximal domain is 6, 7, 8, 9, 10, 11, 12, 13, 14, 14, 16, 17, 18, 19, or 20 nucleotides in length.

In an embodiment, the proximal domain is 5 to 20, 7, to 18, 9 to 16, or 10 to 14 nucleotides in length.

In an embodiment, the proximal domain nucleotides do not comprise modifications, e.g., modifications of the type provided in Section VIII. However, in an embodiment, the proximal domain comprises one or more modifications, e.g., modifications that render it less susceptible to degradation or more bio-compatible, e.g., less immunogenic. By way of example, the backbone of the proximal domain can be modified with a phosphorothioate, or other modification(s) from Section VIII. In an embodiment a nucleotide of the proximal domain can comprise a 2' modification, e.g., a 2-acetylation, e.g., a 2' methylation, or other modification(s) from Section VIII.

In some embodiments, the proximal domain can comprise as many as 1, 2, 3, 4, 5, 6, 7 or 8 modifications. In an embodiment, the proximal domain comprises as many as 1, 2, 3, or 4 modifications within 5 nucleotides of its 5' end, e.g., in a modular gRNA molecule. In an embodiment, the target domain comprises as many as 1, 2, 3, or 4 modifications within 5 nucleotides of its 3' end, e.g., in a modular gRNA molecule.

In some embodiments, the proximal domain comprises modifications at two consecutive nucleotides, e.g., two consecutive nucleotides that are within 5 nucleotides of the 5' end of the proximal domain, within 5 nucleotides of the 3' end of the proximal domain, or more than 5 nucleotides away from one or both ends of the proximal domain. In an embodiment, no two consecutive nucleotides are modified within 5 nucleotides of the 5' end of the proximal domain, within 5 nucleotides of the 3' end of the proximal domain, or within a region that is more than 5 nucleotides away from one or both ends of the proximal domain. In an embodiment, no nucleotide is modified within 5 nucleotides of the 5' end of the proximal domain, within 5 nucleotides of the 3' end of the proximal domain, or within a region that is more than 5 nucleotides away from one or both ends of the proximal domain.

Modifications in the proximal domain can be selected to not interfere with gRNA molecule efficacy, which can be evaluated by testing a candidate modification in the system described in Section IV. gRNAs having a candidate proximal domain having a selected length, sequence, degree of complementarity, or degree of modification, can be evaluated in the system described at Section IV. The candidate proximal domain can be placed, either alone, or with one or more other candidate changes in a gRNA molecule/Cas9 molecule system known to be functional with a selected target and evaluated.

In an embodiment, the proximal domain has at least 60, 70, 80, 85 90, 95, 98 or 99% homology with, or differs by no more than 1, 2, 3, 4, 5 ,or 6 nucleotides from, a reference proximal domain, e.g., a naturally occurring, e.g., an *S. pyogenes, S. aureus, N. meningtidis,* or *S. thermophilus,* proximal domain, or a proximal domain described herein, e.g., from **Figs. 1A-1G****.**

### The Tail Domain

In an embodiment, the tail domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the tail domain is 20+/-5 nucleotides in length.

In an embodiment, the tail domain is 20+/-10, 30+/-10, 40+/-10, 50+/-10, 60+/-10, 70+/-10, 80+/- 10, 90+/-10, or 100+/-10 nucleotides, in length.

In an embodiment, the tail domain is 25+/-10 nucleotides in length.

In an embodiment, the tail domain is 10 to 100, 10 to 90, 10 to 80, 10 to 70, 10 to 60, 10 to 50, 10 to 40, 10 to 30, 10 to 20 or 10 to 15 nucleotides in length.

In other embodiments, the tail domain is 20 to 100, 20 to 90, 20 to 80, 20 to 70, 20 to 60, 20 to 50, 20 to 40, 20 to 30, or 20 to 25 nucleotides in length.

In an embodiment, the tail domain is 1 to 20, 1 to 1, 1 to 10, or 1 to 5 nucleotides in length.

In an embodiment, the tail domain nucleotides do not comprise modifications, e.g., modifications of the type provided in Section VIII. However, in an embodiment, the tail domain comprises one or more modifications, e.g., modifications that render it less susceptible to degradation or more bio-compatible, e.g., less immunogenic. By way of example, the backbone of the tail domain can be modified with a phosphorothioate, or other modification(s) from Section VIII. In an embodiment a nucleotide of the tail domain can comprise a 2' modification, e.g., a 2-acetylation, e.g., a 2' methylation, or other modification(s) from Section VIII.

In some embodiments, the tail domain can have as many as 1, 2, 3, 4, 5, 6, 7 or 8 modifications. In an embodiment, the target domain comprises as many as 1, 2, 3, or 4 modifications within 5 nucleotides of its 5' end. In an embodiment, the target domain comprises as many as 1, 2, 3, or 4 modifications within 5 nucleotides of its 3' end.

In an embodiment, the tail domain comprises a tail duplex domain, which can form a tail duplexed region. In an embodiment, the tail duplexed region can be 3, 4, 5, 6, 7, 8, 9, 10, 11, or 12 base pairs in length. In an embodiment, a further single stranded domain, exists 3' to the tail duplexed domain. In an embodiment, this domain is 3, 4, 5, 6, 7, 8, 9, or 10 nucleotides in length. In an embodiment it is 4 to 6 nucleotides in length.

In an embodiment, the tail domain has at least 60, 70, 80, 90, 95, 98 or 99% homology with, or differs by no more than 1, 2, 3, 4, 5 ,or 6 nucleotides from, a reference tail domain, e.g., a naturally occurring, e.g., an *S. pyogenes, S. aureus, N. meningtidis,* or *S. thermophilus,* tail domain, or a tail domain described herein, e.g., from **Figs. 1A-1G****.**

In an embodiment, the proximal and tail domain, taken together comprise the following sequences:
AAGGCUAGUCCGUUAUCAACUUGAAAAAGUGGCACCGAGUCGGUGCU (SEQ ID NO:33),
AAGGCUAGUCCGUUAUCAACUUGAAAAAGUGGCACCGAGUCGGUGGUGC (SEQ ID NO:34),
AAGGCUAGUCCGUUAUCAACUUGAAAAAGUGGCACCGAGUCGGUGCGGAUC (SEQ ID NO:35),
AAGGCUAGUCCGUUAUCAACUUGAAAAAGUG (SEQ ID NO:36),
AAGGCUAGUCCGUUAUCA (SEQ ID NO:37), or
AAGGCUAGUCCG (SEQ ID NO:38).

In an embodiment, the tail domain comprises the 3' sequence UUUUUU, e.g., if a U6 promoter is used for transcription.

In an embodiment, the tail domain comprises the 3' sequence UUUU, e.g., if an HI promoter is used for transcription.

In an embodiment, tail domain comprises variable numbers of 3' Us depending, e.g., on the termination signal of the pol-III promoter used.

In an embodiment, the tail domain comprises variable 3' sequence derived from the DNA template if a T7 promoter is used.

In an embodiment, the tail domain comprises variable 3' sequence derived from the DNA template, e.g., if in vitro transcription is used to generate the RNA molecule.

In an embodiment, the tail domain comprises variable 3' sequence derived from the DNA template, e.g., if a pol-II promoter is used to drive transcription.

Modifications in the tail domain can be selected to not interfere with targeting efficacy, which can be evaluated by testing a candidate modification in the system described in Section IV. gRNAs having a candidate tail domain having a selected length, sequence, degree of complementarity, or degree of modification, can be evaluated in the system described in Section IV. The candidate tail domain can be placed, either alone, or with one or more other candidate changes in a gRNA molecule/Cas9 molecule system known to be functional with a selected target and evaluated.

In some embodiments, the tail domain comprises modifications at two consecutive nucleotides, e.g., two consecutive nucleotides that are within 5 nucleotides of the 5' end of the tail domain, within 5 nucleotides of the 3' end of the tail domain, or more than 5 nucleotides away from one or both ends of the tail domain. In an embodiment, no two consecutive nucleotides are modified within 5 nucleotides of the 5' end of the tail domain, within 5 nucleotides of the 3' end of the tail domain, or within a region that is more than 5 nucleotides away from one or both ends of the tail domain. In an embodiment, no nucleotide is modified within 5 nucleotides of the 5' end of the tail domain, within 5 nucleotides of the 3' end of the tail domain, or within a region that is more than 5 nucleotides away from one or both ends of the tail domain.

In an embodiment a gRNA has the following structure:
5' [targeting domain]-[first complementarity domain]-[linking domain]-[second complementarity domain]-[proximal domain]-[tail domain]-3'
wherein, the targeting domain comprises a core domain and optionally a secondary domain, and is 10 to 50 nucleotides in length;
the first complementarity domain is 5 to 25 nucleotides in length and, In an embodiment has at least 50, 60, 70, 80, 85, 90, 95, 98 or 99% homology with a reference first complementarity domain disclosed herein;
the linking domain is 1 to 5 nucleotides in length;
the proximal domain is 5 to 20 nucleotides in length and, in an embodiment has at least 50, 60, 70, 80, 85, 90, 95, 98 or 99% homology with a reference proximal domain disclosed herein; and
the tail domain is absent or a nucleotide sequence is 1 to 50 nucleotides in length and, in an embodiment has at least 50, 60, 70, 80, 85, 90, 95, 98 or 99% homology with a reference tail domain disclosed herein.

### Exemplary Chimeric gRNAs

In an embodiment, a unimolecular, or chimeric, gRNA comprises, preferably from 5' to 3':
a targeting domain, e.g., comprising 15, 16, 17, 18, 19, 20, 21, 22, 23, 24, 25, or 26 nucleotides (which is complementary to a target nucleic acid);
   a first complementarity domain;
   a linking domain;
a second complementarity domain (which is complementary to the first complementarity domain);
   a proximal domain; and
   a tail domain,
   wherein,
   (a) the proximal and tail domain, when taken together, comprise
      at least 15, 18, 20, 25, 30, 31, 35, 40, 45, 49, 50, or 53 nucleotides;
   (b) there are at least 15, 18, 20, 25, 30, 31, 35, 40, 45, 49, 50, or 53 nucleotides 3' to the last nucleotide of the second complementarity domain; or
   (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment, there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number: 
NNNNNNNNNNNNNNNNNNNNGUUUUAGAGCUAGAAAUAGCAAGUUAAAAUAAGG
CUAGUCCGUUAUCAACUUGAAAAAGUGGCACCGAGUCGGUGCUUUUUU (SEQ ID NO:40).
 In an embodiment, the unimolecular, or chimeric, gRNA molecule is a *S. pyogenes* gRNA molecule.

In some embodiments, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number: 
NNNNNNNNNNNNNNNNNNNNGUUUUAGUACUCUGGAAACAGAAUCUACUAAAAC
AAGGCAAAAUGCCGUGUUUAUCUCGUCAACUUGUUGGCGAGAUUUUUU (SEQ ID NO:41).
 In an embodiment, the unimolecular, or chimeric, gRNA molecule is a *S*. *aureus* gRNA molecule.

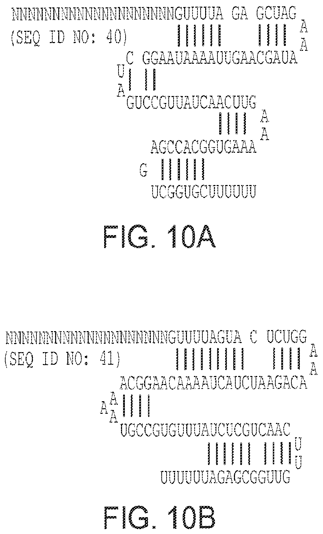

The sequences and structures of exemplary chimeric gRNAs are also shown in **Figs. 10A-10B**.

### Exemplary Modular gRNAs

In an embodiment, a modular gRNA comprises:
a first strand comprising, preferably from 5' to 3';
   a targeting domain, e.g., comprising 15, 16, 17, 18, 19, 20, 21, 22, 23, 24, 25, or 26 nucleotides;
   a first complementarity domain; and
   a second strand, comprising, preferably from 5' to 3':
   optionally a 5' extension domain;
   a second complementarity domain;
   a proximal domain; and
   a tail domain,
wherein:
   (a) the proximal and tail domain, when taken together, comprise
      at least 15, 18, 20, 25, 30, 31, 35, 40, 45, 49, 50, or 53 nucleotides;
   (b) there are at least 15, 18, 20, 25, 30, 31, 35, 40, 45, 49, 50, or 53 nucleotides 3' to the last nucleotide of the second complementarity domain; or
   (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### II. Methods for Designing gRNAs

Methods for designing gRNAs are described herein, including methods for selecting, designing and validating targeting domains. Exemplary targeting domains are also provided herein. Targeting domains discussed herein can be incorporated into the gRNAs described herein.

Methods for selection and validation of target sequences as well as off-target analyses are described, e.g., in Mali et al., 2013 SCIENCE 339(6121): 823-826; Hsu et al. NAT BIOTECHNOL, 31(9): 827-32; Fu et al., 2014 NAT BIOTECHNOL, doi: 10.1038/nbt.2808. PubMed PMID: 24463574; Heigwer et al., 2014 NAT METHODS 11(2):122-3. doi: 10.1038/nmeth.2812. PubMed PMID: 24481216; Bae et al., 2014 BIOINFORMATICS PubMed PMID: 24463181; Xiao A et al., 2014 BIOINFORMATICS PubMed PMID: 24389662.

In some embodiments, a software tool can be used to optimize the choice of gRNA within a user's target sequence, e.g., to minimize total off-target activity across the genome. Off target activity may be other than cleavage. For example, for each possible gRNA choice using *S. pyogenes* Cas9, software tools can identify all potential off-target sequences (preceding either NAG or NGG PAMs) across the genome that contain up to a certain number (e.g., 1, 2, 3, 4, 5, 6, 7, 8, 9, or 10) of mismatched base-pairs. The cleavage efficiency at each off-target sequence can be predicted, e.g., using an experimentally-derived weighting scheme. Each possible gRNA can then be ranked according to its total predicted off-target cleavage; the top-ranked gRNAs represent those that are likely to have the greatest on-target and the least off-target cleavage. Other functions, e.g., automated reagent design for gRNA vector construction, primer design for the on-target Surveyor assay, and primer design for high-throughput detection and quantification of off-target cleavage via next-generation sequencing, can also be included in the tool. Candidate gRNA molecules can be evaluated by art-known methods or as described in Section IV herein.

In some embodiments, gRNAs for use with *S. pyogenes, S. aureus,* and *N. meningitidis* Cas9s were identified using a DNA sequence searching algorithm, e.g., using a custom gRNA design software based on the public tool cas-offinder (Bae et al. Bioinformatics. 2014; 30(10): 1473-1475). Said custom gRNA design software scores guides after calculating their genomewide off-target propensity. Typically matches ranging from perfect matches to 7 mismatches are considered for guides ranging in length from 17 to 24. Once the off-target sites were computationally determined, an aggregate score was calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene were obtained from the UCSC Genome browser and sequences were screened for repeat elements using the publicly available RepeatMasker program. RepeatMasker searches input DNA sequences for repeated elements and regions of low complexity. The output is a detailed annotation of the repeats present in a given query sequence.

Following identification, gRNAs were ranked into tiers based on on one or more of their distance to the target site, their orthogonality and presence of a 5' G (based on identification of close matches in the human genome containing a relavant PAM, e.g., in the case of *S. pyogenes,* a NGG PAM, in the case of *S. aureus,* NNGRR (e.g, a NNGRRT or NNGRRV) PAM, and in the case of *N. meningtidis*, a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer targeting domains that have no identical sequences in the human genome besides the intended target, nor any sequences that contain one or two mismatches in the target sequence. Targeting domains with good orthogonality are selected to minimize off-target DNA cleavage. It is to be understood that this is a non-limiting example and that a variety of strategies could be utilized to identify gRNAs for use with *S. pyogenes, S. aureus* and *N. meningitidis* or other Cas9 enzymes.

Two design and tiering strategies were utilized to identify exemplary gRNAs for use with *S*. *pyogenes, S. aureus* and *N. meningitidis* Cas9 enzymes.

### First Strategy for Designing and Tiering gRNAs

In the first strategy, gRNAs for use with the *S. pyogenes* Cas9 were identified using the publicly available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequences for each gene were obtained from the UCSC Genome browser and sequences were screened for repeat elements using the publicly available Repeat-Masker program. RepeatMasker searches input DNA sequences for repeated elements and regions of low complexity. The output is a detailed annotation of the repeats present in a given query sequence. Following identification, gRNAs for use with a *S*. *pyogenes* Cas9 were ranked into 5 tiers.

The targeting domains for first tier gRNA molecules were selected based on their distance to the target site, their orthogonality and presence of a 5' G (based on the ZiFiT identification of close matches in the human genome containing an NGG PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome nor any sequences that contain one or two mismatches in the target sequence. Targeting domains with good orthogonality are selected to miminize off-target DNA cleavage. For all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs canalso often result in indel mutations at the site of only one of the gRNAs. Candidate pair members can be tested for how efficiently they remove the entire sequence versus just causing indel mutations at the site of one gRNA.

The targeting domains for first tier gRNA molecules were selected based on (1) a reasonable distance to the target position, e.g., within the first 500bp of coding sequence downstream of start codon, (2) a high level of orthogonality, and (3) the presence of a 5' G. For selection of second tier gRNAs, the requirement for a 5'G was removed, but the distance restriction was required and a high level of orthogonality was required. Third tier selection used the same distance restriction and the requirement for a 5'G, but removed the requirement of good orthogonality. Fourth tier selection used the same distance restriction but remove the requirement of good orthogonality and start with a 5'G. Fifth tier selection removed the requirement of good orthogonality and a 5'G, and a longer sequence (e.g., the rest of the coding sequence, e.g., additional 500 bp upstream or downstream to the transcription target site) was scanned. Note that tiers are non-inclusive (each gRNA is listed only once). In certain instances, no gRNA was identified based on the criteria of the particular tier.

As discussed above, gRNAs were identified for single-gRNA nuclease cleavage as well as for a dual-gRNA paired "nickase" strategy, as indicated.

gRNAs for use with the *N*. *meningitidis* and *S. aureus* Cas9s were identified manually by scanning genomic DNA sequence for the presence of PAM sequences. These gRNAs were separated into two tiers. For first tier gRNAs, targeting domains were selected within the first 500bp of coding sequence downstream of start codon. For second tier gRNAs, targeting domains were selected within the remaining coding sequence (downstream of the first 500bp). Note that tiers are non-inclusive (each gRNA is listed only once for the strategy). In certain instances, no gRNA was identified based on the criteria of the particular tier.

### Exemplary Targeting Domains (First Strategy)

Below are tables for providing exemplary targeting domains according to the first design and tiering strategy. As an example, for *S. pyogenes, S. aureus* and *N. meningtidis* targets, 17-mer, or 20-mer targeting domains were designed.

**Table 1A** provides targeting domains for knocking out the *FAS* gene using *S. pyogenes* Cas9 selected according to first tier parameters. The targeting domains bind within the first 500bp of coding sequence downstream of start codon, have good orthogonality, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with any targeting domain from Group B as shown in **Table 1.**

**Table 1**

| Group A | Group B |
|---|---|
| CACUUGGGCAUUAACACUUU (SEQ ID NO:8655); | GUGACUGACAUCAACUCCAA (SEQ ID NO:8648); |
| | GUUGAGACUCAGAACUUGGA (SEQ ID NO:8650); |
| UUGGGCAUUAACACUUU (SEQ ID NO:8735) | |
| | ACUGACAUCAACUCCAA (SEQ ID NO:8686); |
| | GAGACUCAGAACUUGGA (SEQ ID NO:8668) |

**Table 1A**

| 1st Tier |
|---|
| Targeting domains for knocking out the *FAS* gene using *S. pyogenes* Cas9 selected according to first tier parameters are presented in SEQ ID NO:8640-8651. |

**Table 1B** provides targeting domains for knocking out the *FAS* gene using *S. pyogenes* Cas9 selected according to second tier parameters. The targeting domains bind within the first 500bp of coding sequence downstream of start codon, have good orthogonality, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1B**

| 2nd Tier |
|---|
| Targeting domains for knocking out the *FAS* gene using *S. pyogenes* Cas9 selected according to second tier parameters are presented in SEQ ID NO:8652-8665. |

**Table 1C** provides targeting domains for knocking out the *FAS* gene using *S. pyogenes* Cas9 selected according to third tier parameters. The targeting domains bind within the first 500bp of coding sequence downstream of start codon, and start with G. It is contemplated herein that the targeting domain hybridizes to the target domain through complementary base pairing Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1C**

| 3rd Tier |
|---|
| Targeting domains for knocking out the *FAS* gene using *S. pyogenes* Cas9 selected according to third tier parameters are presented in SEQ ID NO:8666-8681. |

**Table 1D** provides targeting domains for knocking out the *FAS* gene using *S. pyogenes* Cas9 selected according to fourth tier parameters. The targeting domains bind within the first 500bp of coding sequence downstream of start codon, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1D**

| 4th Tier |
|---|
| Targeting domains for knocking out the *FAS* gene using *S. pyogenes* Cas9 selected according to fourth tier parameters are presented in SEQ ID NO:8682-8737. |

**Table 1E** provides targeting domains for knocking out the *FAS* gene using *S. pyogenes* Cas9 selected according to fifth tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1E**

| 5th Tier |
|---|
| Targeting domains for knocking out the *FAS* gene using *S. pyogenes* Cas9 selected according to fifth tier parameters are presented in SEQ ID NO:8738-8859. |

**Table 1F** provides targeting domains for knocking out the *FAS* gene using *S. aureus* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1F**

| 1st Tier |
|---|
| Targeting domains for knocking out the *FAS* gene using *S. aureus* Cas9 selected according to first tier parameters are presented in SEQ ID NO:8860-8997. |

**Table 1G** provides targeting domains for knocking out the *FAS* gene using *S. aureus* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1G**

| 2nd Tier |
|---|
| Targeting domains for knocking out the *FAS* gene using *S. aureus* Cas9 selected according to second tier parameters are presented in SEQ ID NO:8998-9213. |

**Table 1H** provides targeting domains for knocking out the *FAS* gene using *N. meningitidis* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1H**

| 1st Tier |
|---|
| Targeting domains for knocking out the *FAS* gene using *N. meningitidismeningitidis* Cas9 selected according to first tier parameters are presented in SEQ ID NO:9214-9221. |

**Table 1I** provides targeting domains for knocking out the *FAS* gene using *N. meningitidis* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 1I**

| 2nd Tier |
|---|
| Targeting domains for knocking out the *FAS* gene using *N. meningitidismeningitidis* Cas9 selected according to second tier parameters are presented in SEQ ID NO:9222-9235. |

**Table 2A** provides targeting domains for knocking down the *FAS* gene using *S. pyogenes* Cas9 selected according to first tier parameters. The targeting domains bind within the 500bp upstream and downstream of transcription start site, have good orthogonality, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 2A**

| 1st Tier |
|---|
| Targeting domains for knocking out the *FAS* gene using *S. pyogenes* Cas9 selected according to first tier parameters are presented in SEQ ID NO:9236-9279. |

**Table 2B** provides targeting domains for knocking down the *FAS* gene using *S. pyogenes* Cas9 selected according to second tier parameters. The targeting domains bind within the 500bp upstream and downstream of transcription start site, have good orthogonality, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 2B**

| 2nd Tier |
|---|
| Targeting domains for knocking out the *FAS* gene using *S. pyogenes* Cas9 selected according to second tier parameters are presented in SEQ ID NO:9280-9351. |

**Table 2C** provides targeting domains for knocking down the *FAS* gene using *S. pyogenes* Cas9 selected according to third tier parameters. The targeting domains bind within the 500bp upstream and downstream of transcription start site, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 2C**

| 3rd Tier |
|---|
| Targeting domains for knocking out the *FAS* gene using *S. pyogenes* Cas9 selected according to third tier parameters are presented in SEQ ID NO:9352-9442. |

**Table 2D** provides targeting domains for knocking down the *FAS* gene using *S. pyogenes* Cas9 selected according to fourth tier parameters. The targeting domains bind within the 500bp upstream and downstream of transcription start site, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 2D**

| 4th Tier |
|---|
| Targeting domains for knocking out the *FAS* gene using *S. pyogenes* Cas9 selected according to fourth tier parameters are presented in SEQ ID NO:9443-9617. |

**Table 2E** provides targeting domains for knocking down the *FAS* gene using *S. pyogenes* Cas9 selected according to fifth tier parameters. The targeting domains bind within the additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 2E**

| 5th Tier |
|---|
| Targeting domains for knocking out the *FAS* gene using *S. pyogenes* Cas9 selected according to fifth tier parameters are presented in SEQ ID NO:9618-9959. |

**Table 2F** provides targeting domains for knocking down the *FAS* gene using *S. aureus* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 2F**

| 1st Tier |
|---|
| Targeting domains for knocking out the *FAS* gene using *S. aureus* Cas9 selected according to first tier parameters are presented in SEQ ID NO:9960-10369. |

**Table 2G** provides targeting domains for knocking down the *FAS* gene using *S. aureus* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 2G**

| 2nd Tier |
|---|
| Targeting domains for knocking out the *FAS* gene using *S. aureus* Cas9 selected according to second tier parameters are presented in SEQ ID NO: 10370-10745. |

**Table 2H** provides targeting domains for knocking down the *FAS* gene using *N. meningitidis* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 2H**

| 1st Tier |
|---|
| Targeting domains for knocking out the *FAS* gene using *S. meningitidis* Cas9 selected according to first tier parameters are presented in SEQ ID NO:10746-10753. |

**Table 2I** provides targeting domains for knocking down the *FAS* gene using *N. meningitidis* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 2I**

| 2nd Tier |
|---|
| Targeting domains for knocking out the *FAS* gene using *S. meningitidis* Cas9 selected according to first tier parameters are presented in SEQ ID NO:10754-10759. |

**Table 3A** provides targeting domains for knocking out the *BID* gene using *S. pyogenes* Cas9 selected according to first tier parameters. The targeting domains bind within the first 500bp of coding sequence downstream of start codon, have good orthogonality, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). Tn an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with any targeting domain from Group B as shown in **Table 3** or a gRNA with a targeting domain from Group C can be paired with a gRNA with any targeting domain from Group D as shown in **Table 3.**

**Table 3**

| Group A | Group B |
|---|---|
| GUCCCAGUGGCGACCUGGAA (SEQ ID NO: 10779); | UCGGAGCUGCCGCGCUGCCC (SEQ ID NO:10833) |
| GUGUCCCAGUGGCGACC (SEQ ID NO: 10783); | |
| GGUUCACAGUGUCCCAG (SEQ ID NO:10777) | |

| Group C | Group D |
|---|---|
| GCUCAUCGUAGCCCUCCCAC (SEQ ID NO: 10768); | CGCAGCAGCCACUCCCGCUU (SEQ ID NO:10815); |
| CAUCGUAGCCCUCCCAC (SEQ ID NO:10803) | AGCAGCCACUCCCGCUU (SEQ ID NO:10792) |

**Table 3A**

| 1st Tier |
|---|
| Targeting domains for knocking out the *BID* gene using *S. pyogenes* Cas9 selected according to first tier parameters are presented in SEQ ID NO: 10760-10783. |

**Table 3B** provides targeting domains for knocking out the *BID* gene using *S. pyogenes* Cas9 selected according to second tier parameters. The targeting domains bind within the first 500bp of coding sequence downstream of start codon, have good orthogonality, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3B**

| 2nd Tier |
|---|
| Targeting domains for knocking out the *BID* gene using *S. pyogenes* Cas9 selected according to second tier parameters are presented in SEQ ID NO:10784-10839. |

**Table 3C** provides targeting domains for knocking out the *BID* gene using *S. pyogenes* Cas9 selected according to third tier parameters. The targeting domains bind within the first 500bp of coding sequence downstream of start codon, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3C**

| 3rd Tier |
|---|
| Targeting domains for knocking out the *BID* gene using *S. pyogenes* Cas9 selected according to third tier parameters are presented in SEQ ID NO:10840-10898. |

**Table 3D** provides targeting domains for knocking out the *BID* gene using *S. pyogenes* Cas9 selected according to fourth tier parameters. The targeting domains bind within the first 500bp of coding sequence downstream of start codon, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3D**

| 4th Tier |
|---|
| Targeting domains for knocking out the *BID* gene using *S. pyogenes* Cas9 selected according to fourth tier parameters are presented in SEQ ID NO:10899-11033. |

**Table 3E** provides targeting domains for knocking out the *BID* gene using *S. pyogenes* Cas9 selected according to fifth tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3E**

| 5th Tier |
|---|
| Targeting domains for knocking out the *BID* gene using *S. pyogenes* Cas9 selected according to fifth tier parameters are presented in SEQ ID NO:11034-11115. |

**Table 3F** provides targeting domains for knocking out the *BID* gene using *S. aureus* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3F**

| 1st Tier |
|---|
| Targeting domains for knocking out the *BID* gene using *S. aureus* Cas9 selected according to first tier parameters are presented in SEQ ID NO:11116-11367. |

**Table 3G** provides targeting domains for knocking out the *BID* gene using *S. aureus* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3G**

| 2nd Tier |
|---|
| Targeting domains for knocking out the *BID* gene using *S. aureus* Cas9 selected according to second tier parameters are presented in SEQ ID NO:11368-11435. |

**Table 3H** provides targeting domains for knocking out the *BID* gene using *N. meningitidis* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 3H**

| 1st Tier |
|---|
| Targeting domains for knocking out the *BID* gene using *N. meningitidismeningitidis* Cas9 selected according to first tier parameters are presented in SEQ ID NO:11436-11439. |

**Table 4A** provides targeting domains for knocking down the *BID* gene using *S. pyogenes* Cas9 selected according to first tier parameters. The targeting domains bind within the 500bp upstream and downstream of transcription start site, have good orthogonality, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 4A**

| 1st Tier |
|---|
| Targeting domains for knocking down the *BID* gene using *S. pyogenes* Cas9 selected according to first tier parameters are presented in SEQ ID NO:11440-11486. |

**Table 4B** provides targeting domains for knocking down the *BID* gene using *S. pyogenes* Cas9 selected according to second tier parameters. The targeting domains bind within the 500bp upstream and downstream of transcription start site, have good orthogonality, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 4B**

| 2nd Tier |
|---|
| Targeting domains for knocking down the *BID* gene using *S. pyogenes* Cas9 selected according to second tier parameters are presented in SEQ ID NO:11487-11559. |

**Table 4C** provides targeting domains for knocking down the *BID* gene using *S. pyogenes* Cas9 selected according to third tier parameters. The targeting domains bind within the 500bp upstream and downstream of transcription start site, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 4C**

| 3rd Tier |
|---|
| Targeting domains for knocking down the *BID* gene using *S. pyogenes* Cas9 selected according to third tier parameters are presented in SEQ ID NO:11560-11721. |

**Table 4D** provides targeting domains for knocking down the *BID* gene using *S. pyogenes* Cas9 selected according to fourth tier parameters. The targeting domains bind within the 500bp upstream and downstream of transcription start site, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 4D**

| 4th Tier |
|---|
| Targeting domains for knocking down the *BID* gene using *S. pyogenes* Cas9 selected according to fourth tier parameters are presented in SEQ ID NO:11722-11983. |

**Table 4E** provides targeting domains for knocking down the *BID* gene using *S. pyogenes* Cas9 selected according to fifth tier parameters. The targeting domains bind within the additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 4E**

| 5th Tier |
|---|
| Targeting domains for knocking down the *BID* gene using *S. pyogenes* Cas9 selected according to fifth tier parameters are presented in SEQ ID NO:11984-12411. |

**Table 4F** provides targeting domains for knocking down the *BID* gene using *S*. *aureus* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 4F**

| 1st Tier |
|---|
| Targeting domains for knocking down the *BID* gene using *S. aureus* Cas9 selected according to first tier parameters are presented in SEQ ID NO:12412-12841. |

**Table 4G** provides targeting domains for knocking down the *BID* gene using *S. aureus* Cas9 selected according to second tier parameters. The targeting domains bind within additional 500bp upstream and downstream of transcription start site (extending to 1kb up and downstream of the Transcription start site). It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 4G**

| 2nd Tier |
|---|
| Targeting domains for knocking down the *BID* gene using *S. aureus* Cas9 selected according to second tier parameters are presented in SEQ ID NO:12842-13269. |

**Table 4H** provides targeting domains for knocking down the *BID* gene using *N. meningitidis* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 4H**

| 1st Tier |
|---|
| Targeting domains for knocking down the *BID* gene using *N. meningitidismeningitidis* Cas9 selected according to first tier parameters are presented in SEQ ID NO:13270-13275. |

**Table 4I** provides targeting domains for knocking down the *BID* gene using *N. meningitidis* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 4I**

| 2nd Tier |
|---|
| Targeting domains for knocking down the *BID* gene using *N. meningitidismeningitidis*Cas9 selected according to second tier parameters are presented in SEQ ID NO: 13276-13285. |

**Table 5A** provides targeting domains for knocking out the *CTLA4* gene using *S. pyogenes* Cas9 selected according to first tier parameters. The targeting domains bind within the first 500bp of coding sequence downstream of start codon, have good orthogonality, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with any targeting domain from Group B as shown in **Table 5** or a gRNA with a targeting domain from Group C can be paired with a gRNA with any targeting domain from Group D as shown in **Table 5.**

**Table 5**

| Group A | Group B |
|---|---|
| CCUUGGAUUUCAGCGGCACA (SEQ ID NO: 13293); | GCUUUAUGGGAGCGGUGUUC (SEQ ID NO: 13290); |
| UGGAUUUCAGCGGCACA (SEQ ID NO: 13294) | UUAUGGGAGCGGUGUUC (SEQ ID NO: 13307) |

| Group C | Group D |
|---|---|
| UGAACCUGGCUACCAGGACC (SEQ ID NO: 13295); | CCUUGUGCCGCUGAAAUCCA (SEQ ID NO: 13305); |
| ACCUGGCUACCAGGACC (SEQ ID NO: 13296) | UGUGCCGCUGAAAUCCA (SEQ ID NO: 13306) |

**Table 5A**

| 1st Tier |
|---|
| Targeting domains for knocking out the *CTLA4* gene using *S. pyogenes* Cas9 selected according to first tier parameters are presented in SEQ ID NO:13286-13290. |

**Table 5B** provides targeting domains for knocking out the *CTLA4* gene using *S. pyogenes* Cas9 selected according to second tier parameters. The targeting domains bind within the first 500bp of coding sequence downstream of start codon, have good orthogonality, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5B**

| 2nd Tier |
|---|
| Targeting domains for knocking out the *CTLA4* gene using *S. pyogenes* Cas9 selected according to second tier parameters are presented in SEQ ID NO:13291-13307. |

**Table 5C** provides targeting domains for knocking out the *CTLA4* gene using *S. pyogenes* Cas9 selected according to third tier parameters. The targeting domains bind within the first 500bp of coding sequence downstream of start codon, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5C**

| 3rd Tier |
|---|
| Targeting domains for knocking out the *CTLA4* gene using *S. pyogenes* Cas9 selected according to third tier parameters are presented in SEQ ID NO:13308-13340. |

**Table 5D** provides targeting domains for knocking out the *CTLA4* gene using *S. pyogenes* Cas9 selected according to fourth tier parameters. The targeting domains bind within the first 500bp of coding sequence downstream of start codon, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). Tn an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5D**

| 4th Tier |
|---|
| Targeting domains for knocking out the *CTLA4* gene using *S. pyogenes* Cas9 selected according to fourth tier parameters are presented in SEQ ID NO:13341-13429. |

**Table 5E** provides targeting domains for knocking out the *CTLA4* gene using *S. pyogenes* Cas9 selected according to fifth tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5E**

| 5th Tier |
|---|
| Targeting domains for knocking out the *CTLA4* gene using *S. pyogenes* Cas9 selected according to fifth tier parameters are presented in SEQ ID NO:13430-13500. |

**Table 5F** provides targeting domains for knocking out the *CTLA4* gene using *S. aureus* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5F**

| 1st Tier |
|---|
| Targeting domains for knocking out the *CTLA4* gene using *S. aureus* Cas9 selected according to first tier parameters are presented in SEQ ID NO: 13501-13630. |

**Table 5G** provides targeting domains for knocking out the *CTLA4* gene using *S. aureus* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5G**

| 2nd Tier |
|---|
| Targeting domains for knocking out the *CTLA4* gene using *S. aureus* Cas9 selected according to second tier parameters are presented in SEQ ID NO:13631-13735. |

**Table 5H** provides targeting domains for knocking out the *CTLA4* gene using *N. meningitidis* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5H**

| 1st Tier |
|---|
| Targeting domains for knocking out the *CTLA4* gene using *N. meningitidismeningitidis* Cas9 selected according to first tier parameters are presented in SEQ ID NO:13736-13741. |

**Table 5I** provides targeting domains for knocking out the *CTLA4* gene using *N. meningitidis* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *N*. *meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 5I**

| 2nd Tier |
|---|
| Targeting domains for knocking out the *CTLA4* gene using *N. meningitidismeningitidis*Cas9 selected according to second tier parameters are presented in SEQ ID NO: 13742-13745. |

**Table 6A** provides targeting domains for knocking down the *CTLA4* gene using *S. pyogenes* Cas9 selected according to first tier parameters. The targeting domains bind within the 500bp upstream and downstream of transcription start site, have good orthogonality, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 6A**

| 1st Tier |
|---|
| Targeting domains for knocking down the *CTLA4* gene using *S. pyogenes* Cas9 selected according to first tier parameters are presented in SEQ ID NO: 13746-13752. |

**Table 6B** provides targeting domains for knocking down the *CTLA4* gene using *S*. *pyogenes* Cas9selected according to second tier parameters. The targeting domains bind within the 500bp upstream and downstream of transcription start site, have good orthogonality, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 6B**

| 2nd Tier |
|---|
| Targeting domains for knocking down the *CTLA4* gene using *S. pyogenes* Cas9 selected according to second tier parameters are presented in SEQ ID NO:13753-13771. |

**Table 6C** provides targeting domains for knocking down the *CTLA4* gene using *S. pyogenes* Cas9selected according to third tier parameters. The targeting domains bind within the 500bp upstream and downstream of transcription start site, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 6C**

| 3rd Tier |
|---|
| Targeting domains for knocking down the *CTLA4* gene using *S. pyogenes* Cas9 selected according to third tier parameters are presented in SEQ ID NO: 13772-13818. |

**Table 6D** provides targeting domains for knocking down the *CTLA4* gene using *S. pyogenes* Cas9selected according to fourth tier parameters. The targeting domains bind within the 500bp upstream and downstream of transcription start site, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 6D**

| 4th Tier |
|---|
| Targeting domains for knocking down the *CTLA4* gene using *S. pyogenes* Cas9 selected according to fourth tier parameters are presented in SEQ ID NO:13819-13969. |

**Table 6E** provides targeting domains for knocking down the *CTLA4* gene using *S. pyogenes* Cas9selected according to fifth tier parameters. The targeting domains bind within the additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 6E**

| 5th Tier |
|---|
| Targeting domains for knocking down the *CTLA4* gene using *S. pyogenes* Cas9 selected according to fifth tier parameters are presented in SEQ ID NO:13970-14107. |

**Table 6F** provides targeting domains for knocking down the *CTLA4* gene using *S. aureus* Cas9selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 6F**

| 1st Tier |
|---|
| Targeting domains for knocking down the *CTLA4* gene using *S. aureus* Cas9 selected according to first tier parameters are presented in SEQ ID NO:14108-14400. |

**Table 6G** provides targeting domains for knocking down the *CTLA4* gene using *S. aureus* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 6G**

| 2nd Tier |
|---|
| Targeting domains for knocking down the *CTLA4* gene using *S. aureus* Cas9 selected according to second tier parameters are presented in SEQ ID NO:14401-14616. |

**Table 6H** provides targeting domains for knocking down the *CTLA4* gene using *N. meningitidis* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 6H**

| 1st Tier |
|---|
| Targeting domains for knocking down the *CTLA4* gene using *N. meningitidismeningitidis* Cas9 selected according to first tier parameters are presented in SEQ ID NO:14617-14634. |

**Table 6I** provides targeting domains for knocking down the *CTLA4* gene using *N. meningitidis* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 6I**

| 2nd Tier |
|---|
| Targeting domains for knocking down the *CTLA4* gene using *N. meningitidismeningitidis* Cas9 selected according to second tier parameters are presented in SEQ ID NO: 14635-14656. |

**Table 7A** provides targeting domains for knocking out the *PDCD1* gene using *S. pyogenes* Cas9 selected according to first tier parameters. The targeting domains bind within the first 500bp of coding sequence downstream of start codon, have good orthogonality, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with any targeting domain from Group B as shown in **Table 7** or a gRNA with a targeting domain from Group C can be paired with a gRNA with any targeting domain from Group D as shown in **Table 7.**

**Table 7**

| Group A | Group B |
|---|---|
| | GCGUGACUUCCACAUGAGCG (SEQ ID NO:567); |
| UGACACGGAAGCGGCAGUCC (SEQ ID NO:600); | ACUUCCACAUGAGCGUGGUC (SEQ ID NO:590); |
| CACGGAAGCGGCAGUCC (SEQ ID NO:601) | UGACUUCCACAUGAGCG (SEQ ID NO:592); |
| | UCCACAUGAGCGUGGUC (SEQ ID NO:593) |

| Group C | Group D |
|---|---|
| CAUGUGGAAGUCACGCCCGU (SEQ ID NO:597); | |
| AUGUGGAAGUCACGCCCGUU (SEQ ID NO:598); | AGGGCCCGGCGCAAUGACAG (SEQ ID NO:594); |
| GUGGAAGUCACGCCCGU (SEQ ID NO:571); | GCCCGGCGCAAUGACAG (SEQ ID NO:568) |
| UGGAAGUCACGCCCGUU (SED ID NO:599) | |

**Table 7A**

| 1st Tier |
|---|
| Targeting domains for knocking out the *PDCD1* gene using *S. pyogenes* Cas9 selected according to first tier parameters are presented in SEQ ID NO:563-573. |

**Table 7B** provides targeting domains for knocking out the *PDCD1* gene using *S. pyogenes* Cas9 selected according to second tier parameters. The targeting domains bind within the first 500bp of coding sequence downstream of start codon, have good orthogonality, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7B**

| 2nd Tier |
|---|
| Targeting domains for knocking out the *PDCD1* gene using *S. pyogenes* Cas9 selected according to second tier parameters are presented in SEQ ID NO:574-607. |

**Table 7C** provides targeting domains for knocking out the *PDCD1* gene using *S. pyogenes* Cas9 selected according to third tier parameters. The targeting domains bind within the first 500bp of coding sequence downstream of start codon, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7C**

| 3rd Tier |
|---|
| Targeting domains for knocking out the *PDCD1* gene using *S. pyogenes* Cas9 selected according to third tier parameters are presented in SEQ ID NO:608-687. |

**Table 7D** provides targeting domains for knocking out the *PDCD1* gene using *S. pyogenes* Cas9 selected according to fourth tier parameters. The targeting domains bind within the first 500bp of coding sequence downstream of start codon, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7D**

| 4th Tier |
|---|
| Targeting domains for knocking out the *PDCD1* gene using *S. pyogenes* Cas9 selected according to fourth tier parameters are presented in SEQ ID NO:688-816. |

**Table 7E** provides targeting domains for knocking out the *PDCD1* gene using *S. pyogenes* Cas9 selected according to fifth tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7E**

| 5th Tier |
|---|
| Targeting domains for knocking out the *PDCD1* gene using *S. pyogenes* Cas9 selected according to fifth tier parameters are presented in SEQ ID NO:817-1090. |

**Table 7F** provides targeting domains for knocking out the *PDCD1* gene using *S. aureus* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7F**

| 1st Tier |
|---|
| Targeting domains for knocking out the *PDCD1* gene using *S. aureus* Cas9 selected according to first tier parameters are presented in SEQ ID NO:1091-1268. |

**Table 7G** provides targeting domains for knocking out the *PDCD1* gene using *S. aureus* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7G**

| 2nd Tier |
|---|
| Targeting domains for knocking out the *PDCD1* gene using *S*. *aureus* Cas9 selected according to second tier parameters are presented in SEQ ID NO:1269-1514. |

**Table 7H** provides targeting domains for knocking out the *PDCD1* gene using *N. meningitidis* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 7H**

| 2nd Tier |
|---|
| Targeting domains for knocking out the *PDCD1* gene using *N. meningitidismeningitidis* Cas9 selected according to second tier parameters are presented in SEQ ID NO: 1515-1516. |

**Table 8A** provides targeting domains for knocking down the *PDCD1* gene using *S. pyogenes* Cas9 selected according to first tier parameters. The targeting domains bind within the 500bp upstream and downstream of transcription start site, have good orthogonality, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 8A**

| 1st Tier |
|---|
| Targeting domains for knocking down the *PDCD1* gene using *S. pyogenes* Cas9 selected according to first tier parameters are presented in SEQ ID NO:1517-1523. |

**Table 8B** provides targeting domains for knocking down the *PDCD1* gene using *S. pyogenes* Cas9 selected according to second tier parameters. The targeting domains bind within the 500bp upstream and downstream of transcription start site, have good orthogonality, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 8B**

| 2nd Tier |
|---|
| Targeting domains for knocking down the *PDCD1* gene using *S. pyogenes* Cas9 selected according to second tier parameters are presented in SEQ ID NO: 1524-1566. |

**Table 8C** provides targeting domains for knocking down the *PDCD1* gene using *S. pyogenes* Cas9 selected according to third tier parameters. The targeting domains bind within the 500bp upstream and downstream of transcription start site, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 8C**

| 3rd Tier |
|---|
| Targeting domains for knocking down the *PDCD1* gene using *S. pyogenes* Cas9 selected according to third tier parameters are presented in SEQ ID NO: 1567-1753. |

**Table 8D** provides targeting domains for knocking down the *PDCD1* gene using *S. pyogenes* Cas9 selected according to fourth tier parameters. The targeting domains bind within the 500bp upstream and downstream of transcription start site, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 8D**

| 4th Tier |
|---|
| Targeting domains for knocking down the *PDCD1* gene using *S. pyogenes* Cas9 selected according to fourth tier parameters are presented in SEQ ID NO:1754-8682-2130. |

**Table 8E** provides targeting domains for knocking down the *PDCD1* gene using *S. pyogenes* Cas9 selected according to fifth tier parameters. The targeting domains bind within the additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 8E**

| 5th Tier |
|---|
| Targeting domains for knocking down the *PDCD1* gene using *S. pyogenes* Cas9 selected according to fifth tier parameters are presented in SEQ ID NO:2131-2620. |

**Table 8F** provides targeting domains for knocking down the *PDCD1* gene using *S. aureus* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 8F**

| 1st Tier |
|---|
| Targeting domains for knocking down the *PDCD1* gene using *S. aureus* Cas9 selected according to first tier parameters are presented in SEQ ID NO:2621-3320. |

**Table 8G** provides targeting domains for knocking down the *PDCD1* gene using *S. aureus* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 8G**

| 2nd Tier |
|---|
| Targeting domains for knocking down the *PDCD1* gene using *S. aureus* Cas9 selected according to second tier parameters are presented in SEQ ID NO:3321-3742. |

**Table 8H** provides targeting domains for knocking down the *PDCD1* gene using *N. meningitidis* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 8H**

| 2nd Tier |
|---|
| Targeting domains for knocking down the *PDCD1* gene using *N. meningitidismeningitidis* Cas9 selected according to second tier parameters are presented in SEQ ID NO:3743-3748. |

**Table 9A** provides targeting domains for knocking out the *CBLB* gene using *S. pyogenes* Cas9 selected according to first tier parameters. The targeting domains bind within the first 500bp of coding sequence downstream of start codon, have good orthogonality, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with any targeting domain from Group B as shown in **Table 9** or a gRNA with a targeting domain from Group C can be paired with a gRNA with any targeting domain from Group D as shown in **Table 9.**

**Table 9**

| Group A | Group B |
|---|---|
| | GCGUGACUUCCACAUGAGCG (SEQ ID NO:567); |
| UGACACGGAAGCGGCAGUCC (SEQ ID NO:600); | ACUUCCACAUGAGCGUGGUC (SEQ ID NO:590); |
| CACGGAAGCGGCAGUCC (SEQ ID NO:601) | UGACUUCCACAUGAGCG (SEQ ID NO:592); |
| | UCCACAUGAGCGUGGUC (SEQ ID NO:593) |
| Group C | Group D |
| CAUGUGGAAGUCACGCCCGU (SEQ ID NO:597); | |
| AUGUGGAAGUCACGCCCGUU (SEQ ID NO:598); | AGGGCCCGGCGCAAUGACAG (SEQ ID NO:594); |
| GUGGAAGUCACGCCCGU (SEQ ID NO:571); | GCCCGGCGCAAUGACAG (SEQ ID NO:568) |
| UGGAAGUCACGCCCGUU (SED ID NO:599) | |

**Table 9A**

| 1st Tier |
|---|
| Targeting domains for knocking out the *CBLB* gene using *S. pyogenes* Cas9 selected according to first tier parameters are presented in SEQ ID NO:6119-6126. |

**Table 9B** provides targeting domains for knocking out the *CBLB* gene using *S. pyogenes* Cas9 selected according to second tier parameters. The targeting domains bind within the first 500bp of coding sequence downstream of start codon, have good orthogonality, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9B**

| 2nd Tier |
|---|
| Targeting domains for knocking out the *CBLB* gene using *S. pyogenes* Cas9 selected according to second tier parameters are presented in SEQ ID NO:6127-6151. |

**Table 9C** provides targeting domains for knocking out the *CBLB* gene using *S. pyogenes* Cas9 selected according to third tier parameters. The targeting domains bind within the first 500bp of coding sequence downstream of start codon, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9C**

| 3rd Tier |
|---|
| Targeting domains for knocking out the *CBLB* gene using *S. pyogenes* Cas9 selected according to third tier parameters are presented in SEQ ID NO:6152-6166. |

**Table 9D** provides targeting domains for knocking out the *CBLB* gene using *S. pyogenes* Cas9 selected according to fourth tier parameters. The targeting domains bind within the first 500bp of coding sequence downstream of start codon, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9D**

| 4th Tier |
|---|
| Targeting domains for knocking out the *CBLB* gene using *S. pyogenes* Cas9 selected according to fourth tier parameters are presented in SEQ ID NO:6167-6202. |

**Table 9E** provides targeting domains for knocking out the *CBLB* gene using *S. pyogenes* Cas9 selected according to fifth tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9E**

| 5th Tier |
|---|
| Targeting domains for knocking out the *CBLB* gene using *S. pyogenes* Cas9 selected according to fifth tier parameters are presented in SEQ ID NO:6203-7006. |

**Table 9F** provides targeting domains for knocking out the *CBLB* gene using *S. aureus* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). Tn an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9F**

| 1st Tier |
|---|
| Targeting domains for knocking out the *CBLB* gene using *S. aureus* Cas9 selected according to first tier parameters are presented in SEQ ID NO:7007-7116. |

**Table 9G** provides targeting domains for knocking out the *CBLB* gene using *S. aureus* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9G**

| 2nd Tier |
|---|
| Targeting domains for knocking out the *CBLB* gene using *S. aureus* Cas9 selected according to second tier parameters are presented in SEQ ID NO:7117-7566. |

**Table 9H** provides targeting domains for knocking out the *CBLB* gene using *N. meningitidis* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9H**

| 1st Tier |
|---|
| Targeting domains for knocking out the *CBLB* gene using *N. meningitidismeningitidis* Cas9 selected according to first tier parameters are presented in SEQ ID NO:7567-7574. |

**Table 9I** provides targeting domains for knocking out the *CBLB* gene using *N. meningitidis* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). n an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 9I**

| 2nd Tier |
|---|
| Targeting domains for knocking out the *CBLB* gene using *N. meningitidismeningitidis* Cas9 selected according to second tier parameters are presented in SEQ ID NO:7575-7634. |

**Table 10A** provides targeting domains for knocking down the *CBLB* gene using *S. pyogenes* Cas9 selected according to first tier parameters. The targeting domains bind within the 500bp upstream and downstream of transcription start site, have good orthogonality, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 10A**

| 1st Tier |
|---|
| Targeting domains for knocking down the *CBLB* gene using *S. pyogenes* Cas9 selected according to first tier parameters are presented in SEQ ID NO:7635-7651. |

**Table 10B** provides targeting domains for knocking down the *CBLB* gene using *S. pyogenes* Cas9 selected according to second tier parameters. The targeting domains bind within the 500bp upstream and downstream of transcription start site, have good orthogonality, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 10B**

| 2nd Tier |
|---|
| Targeting domains for knocking down the *CBLB* gene using *S. pyogenes* Cas9 selected according to second tier parameters are presented in SEQ ID NO:7652-7682. |

**Table 10C** provides targeting domains for knocking down the *CBLB* gene using *S. pyogenes* Cas9 selected according to third tier parameters. The targeting domains bind within the 500bp upstream and downstream of transcription start site, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 10C**

| 3rd Tier |
|---|
| Targeting domains for knocking down the *CBLB* gene using *S. pyogenes* Cas9 selected according to third tier parameters are presented in SEQ ID NO:7683-7701. |

**Table 10D** provides targeting domains for knocking down the *CBLB* gene using *S. pyogenes* Cas9 selected according to fourth tier parameters. The targeting domains bind within the 500bp upstream and downstream of transcription start site, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 10D**

| 4th Tier |
|---|
| Targeting domains for knocking down the *CBLB* gene using *S. pyogenes* Cas9 selected according to fourth tier parameters are presented in SEQ ID NO:7702-7790. |

**Table 10E** provides targeting domains for knocking down the *CBLB* gene using *S. pyogenes* Cas9 selected according to fifth tier parameters. The targeting domains bind within the additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 10E**

| 5th Tier |
|---|
| Targeting domains for knocking down the *CBLB* gene using *S. pyogenes* Cas9 selected according to fifth tier parameters are presented in SEQ ID NO:7791-8042. |

**Table 10F** provides targeting domains for knocking down the *CBLB* gene using *S*. *aureus* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 10F**

| 1st Tier |
|---|
| Targeting domains for knocking down the *CBLB* gene using *S. aureus* Cas9 selected according to first tier parameters are presented in SEQ ID NO:8043-8277. |

**Table 10G** provides targeting domains for knocking down the *CBLB* gene using *S. aureus* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 10G**

| 2nd Tier |
|---|
| Targeting domains for knocking down the *CBLB* gene using *S. aureus* Cas9 selected according to second tier parameters are presented in SEQ ID NO:8278-8599. |

**Table 10H** provides targeting domains for knocking down the *CBLB* gene using *N. meningitidis* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 10H**

| 1st Tier |
|---|
| Targeting domains for knocking down the *CBLB* gene using *N. meningitidismeningitidis* Cas9 selected according to first tier parameters are presented in SEQ ID NO:8600-8623. |

**Table 10I** provides targeting domains for knocking down the *CBLB* gene using *N. meningitidis* Cas9 selected according to second tier parameters. The targeting domains bind within additional 500bp upstream and downstream of transcription start site (extending to 1kb up and downstream of the transcription start site). It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 10I**

| 2nd Tier |
|---|
| Targeting domains for knocking down the *CBLB* gene using *N. meningitidismeningitidis* Cas9 selected according to second tier parameters are presented in SEQ ID NO:8624-8639. |

**Table 11A** provides targeting domains for knocking out the *PTPN6* gene using *S. pyogenes* Cas9 selected according to first tier parameters. The targeting domains bind within the first 500bp of coding sequence downstream of start codon, have good orthogonality, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with any targeting domain from Group B as shown in **Table 11.**

**Table 11**

| Group A | Group B |
|---|---|
| GGACACCUCGGCCCUUGAGC (SEQ ID NO:3755); | GCCCAGUCGCAAGAACC (SEQ ID NO:3756) |
| GUUCUGGAUCCGAAUAU (SEQ ID NO:3762) | GUUUGCGACUCUGACAGAGC (SEQ ID NO:3751) |

**Table 11A**

| 1st Tier |
|---|
| Targeting domains for knocking out the *PTPN6* gene using *S. pyogenes* Cas9 selected according to first tier parameters are presented in SEQ ID NO:3749-3762. |

**Table 11B** provides targeting domains for knocking out the *PTPN6* gene using *S. pyogenes* Cas9 selected according to second tier parameters. The targeting domains bind within the first 500bp of coding sequence downstream of start codon, have good orthogonality, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11B**

| 2nd Tier |
|---|
| Targeting domains for knocking out the *PTPN6* gene using *S. pyogenes* Cas9 selected according to second tier parameters are presented in SEQ ID NO:3763-3806. |

**Table 11C** provides targeting domains for knocking out the *PTPN6* gene using *S. pyogenes* Cas9 selected according to third tier parameters. The targeting domains bind within the first 500bp of coding sequence downstream of start codon, and start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11C**

| 3rd Tier |
|---|
| Targeting domains for knocking out the *PTPN6* gene using *S. pyogenes* Cas9 selected according to third tier parameters are presented in SEQ ID NO:3807-3838. |

**Table 11D** provides targeting domains for knocking out the *PTPN6* gene using *S. pyogenes* Cas9 selected according to fourth tier parameters. The targeting domains bind within the first 500bp of coding sequence downstream of start codon, and do not start with G. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11D**

| 4th Tier |
|---|
| Targeting domains for knocking out the *PTPN6* gene using *S. pyogenes* Cas9 selected according to fourth tier parameters are presented in SEQ ID NO:3839-3894. |

**Table 11E** provides targeting domains for knocking out the *PTPN6* gene using *S. pyogenes* Cas9 selected according to fifth tier parameters. The targeting domains bind within the remaining coding sequence (downstream of the first 500bp). It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11E**

| 5th Tier |
|---|
| Targeting domains for knocking out the *PTPN6* gene using *S. pyogenes* Cas9 selected according to fifth tier parameters are presented in SEQ ID NO:3895-4250. |

**Table 11F** provides targeting domains for knocking out the *PTPN6* gene using *S. aureus* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11F**

| 1st Tier |
|---|
| Targeting domains for knocking out the *PTPN6* gene using *S. aureus* Cas9 selected according to first tier parameters are presented in SEQ ID NO:4251-4378. |

**Table 11G** provides targeting domains for knocking out the *PTPN6* gene using *S. aureus* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11G**

| 2nd Tier |
|---|
| Targeting domains for knocking out the *PTPN6* gene using *S. aureus* Cas9 selected according to second tier parameters are presented in SEQ ID NO:4379-4692. |

**Table 11H** provides targeting domains for knocking out the *PTPN6* gene using *N. meningitidis* Cas9 selected according to first tier parameters. The targeting domains bind within the first 500bp of coding sequence downstream of start codon. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11H**

| 1st Tier |
|---|
| Targeting domains for knocking out the *PTPN6* gene using *N. meningitidismeningitidis* Cas9 selected according to first tier parameters are presented in SEQ ID NO:4693-4696. |

**Table 11I** provides targeting domains for knocking out the *PTPN6* gene using *N. meningitidis* Cas9 selected according to second tier parameters. The targeting domains bind within the remaining coding sequence (downstream of the first 500bp). It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 11I**

| 2nd Tier |
|---|
| Targeting domains for knocking out the *PTPN6* gene using *N. meningitidismeningitidis* Cas9 selected according to second tier parameters are presented in SEQ ID NO:4697-4700. |

**Table 12A** provides targeting domains for knocking down the *PTPN6* gene using *S. pyogenes* Cas9 selected according to first tier parameters. The targeting domains bind within the 500bp upstream and downstream of transcription start site, have good orthogonality, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 12A**

| 1st Tier |
|---|
| Targeting domains for knocking down the *PTPN6* gene using *S. pyogenes* Cas9 selected according to first tier parameters are presented in SEQ ID NO:4701-4716. |

**Table 12B** provides targeting domains for knocking down the *PTPN6* gene using *S. pyogenes* Cas9 selected according to second tier parameters. The targeting domains bind within the 500bp upstream and downstream of transcription start site, have good orthogonality, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 12B**

| 2nd Tier |
|---|
| Targeting domains for knocking down the *PTPN6* gene using *S. pyogenes* Cas9 selected according to second tier parameters are presented in SEQ ID NO:4717-4750. |

**Table 12C** provides targeting domains for knocking down the *PTPN6* gene using *S. pyogenes* Cas9 selected according to third tier parameters. The targeting domains bind within the 500bp upstream and downstream of transcription start site, and start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 12C**

| 3rd Tier |
|---|
| Targeting domains for knocking down the *PTPN6* gene using *S. pyogenes* Cas9 selected according to third tier parameters are presented in SEQ ID NO:4751-4883. |

**Table 12D** provides targeting domains for knocking down the *PTPN6* gene using *S. pyogenes* Cas9 selected according to fourth tier parameters. The targeting domains bind within the 500bp upstream and downstream of transcription start site, and do not start with G. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 12D**

| 4th Tier |
|---|
| Targeting domains for knocking down the *PTPN6* gene using *S. pyogenes* Cas9 selected according to fourth tier parameters are presented in SEQ ID NO:4884-5137. |

**Table 12E** provides targeting domains for knocking down the *PTPN6* gene using *S. pyogenes* Cas9 selected according to fifth tier parameters. The targeting domains bind within the additional 222bp upstream and downstream of transcription start site (extending to 722bp upstream and 1kb downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 12E**

| 5th Tier |
|---|
| Targeting domains for knocking down the *PTPN6* gene using *S. pyogenes* Cas9 selected according to fifth tier parameters are presented in SEQ ID NO:5138-5399. |

**Table 12F** provides targeting domains for knocking down the *PTPN6* gene using *S. aureus* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 12F**

| 1st Tier |
|---|
| Targeting domains for knocking down the *PTPN6* gene using *S. aureus* Cas9 selected according to first tier parameters are presented in SEQ ID NO:5400-5845. |

**Table 12G** provides targeting domains for knocking down the *PTPN6* gene using *S. aureus* Cas9 selected according to second tier parameters. The targeting domains bind within additional 222bp upstream and downstream of transcription start site (extending to 722bp upstream and 1kb downstream of the transcription start site). It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 12G**

| 2nd Tier |
|---|
| Targeting domains for knocking down the *PTPN6* gene using *S. aureus* Cas9 selected according to second tier parameters are presented in SEQ ID NO:5846-6094. |

**Table 12H** provides targeting domains for knocking down the *PTPN6* gene using *N. meningitidis* Cas9 selected according to first tier parameters. The targeting domains bind within 500bp upstream and downstream of transcription start site. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 12H**

| 1st Tier |
|---|
| Targeting domains for knocking down the *PTPN6* gene using *N. meningitidismeningitidis* Cas9 selected according to first tier parameters are presented in SEQ ID NO:6095-6102. |

**Table 12I** provides targeting domains for knocking down the *PTPN6* gene using *N. meningitidis* Cas9 selected according to second tier parameters. The targeting domains bind within additional 222bp upstream and downstream of transcription start site (extending to 722bp upstream and 1kb downstream of the transcription start site). It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 12I**

| 2nd Tier |
|---|
| Targeting domains for knocking down the *PTPN6* gene using *N. meningitidismeningitidis* Cas9 selected according to second tier parameters are presented in SEQ ID NO:6103-6118. |

Targeting domains, disclosed herein, may comprise a **17-mer** described in Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, e.g., the targeting domains of 18 or more nucleotides may comprise the 17-mer gRNAs described in Tables 1A-T, Tables 2A-T, Tables 3A-H, Tables 4A-T, Tables 5A-I, Tables 6A-I, Tables 7A-H, Tables 8A-H, Tables 9A-I, Tables 10A-I, Tables 11A-I, Tables 12A-I.

Targeting domains, disclosed herein, may comprise a **20-mer** gRNAs described in Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, e.g., the targeting domains of 21 or more nucleotides may comprise the 20-mer gRNAs described in Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I.

### Second Strategy for Designing and Tiering gRNAs

The second strategy used to identify gRNAs for use with S. pyogenes, S. aureus and N. meningtidis Cas9 enzymes differed from the first strategy as follows.

Guide RNAs (gRNAs) for use with S. pyogenes, S. aureus and N. meningtidis Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined , an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site or their orthogonality (based on identification of close matches in the human genome containing a relavant PAM, e.g., in the case of S. pyogenes, a NGG PAM, in the case of S. aureus, NNGRR (e.g, a NNGRRT or NNGRRV) PAM, and in the case of N. meningtidiss, a NNNNGATT or NNNNGCTT PAM. Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for S. pyogenes and N. meningtidiss targets, 17-mer, or 20-mer gRNAs were designed. As another example, for S. aureus targets, 18-mer, 19-mer, 20-mer, 21-mer, 22-mer, 23-mer and 24-mer gRNAs were designed. Targeting domains, disclosed herein, may comprise the 17-mer described in Targeting domains, disclosed herein, may comprise a 17-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 18 or more nucleotides may comprise the 17-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a 18-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 19 or more nucleotides may comprise the 18-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, Tables 25A-G, Tables 26A-G, Table 27, Table 29, Table 31, or Table 32.

Targeting domains, disclosed herein, may comprise a 19-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 20 or more nucleotides may comprise the 19-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a 20-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 21 or more nucleotides may comprise the 20-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a 21-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 22 or more nucleotides may comprise the 21-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a 22-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 23 or more nucleotides may comprise the 22-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a 23-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 24 or more nucleotides may comprise the 23-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a 24-mer described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 25 or more nucleotides may comprise the 24-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

gRNAs were identified for both single-gRNA nuclease cleavage and for a dual-gRNA paired "nickase" strategy. Criteria for selecting gRNAs and the determination for which gRNAs can be used for which strategy is based on several considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

The Targeting Domains discussed herein can be incorporated into the gRNAs described herein.

For designing knock out strategies, in some embodiments, the targeting domains for tier 1 gRNA molecules for S. pyogenes were selected based on their distance to the target site and their orthogonality (PAM is NGG). The targeting domains for tier 1 gRNA molecules were selected based on (1) a reasonable distance to the target position, e.g., within the first 500bp of coding sequence downstream of start codon and (2) a high level of orthogonality. For selection of tier 2 gRNAs, a high level of orthogonality was not required. Tier 3 gRNAs removed the requirement of good orthogonality and a longer sequence (e.g., the rest of the coding sequence) was scanned. Note that tiers are non-inclusive (each gRNA is listed only once). In certain instances, no gRNA was identified based on the criteria of the particular tier.

For designing knock out strategies, in some embodiments, the targeting domain for tier 1 gRNA molecules for N. meningtidis were selected within the first 500bp of the coding sequence and had a high level of orthogonality. The targeting domain for tier 2 gRNA molecules for N. meningtidis were selected within the first 500bp of the coding sequence and did not require high orthogonality. The targeting domain for tier 3 gRNA molecules for N. meningtidis were selected within a remainder of coding sequence downstream of the 500bp. Note that tiers are non-inclusive (each gRNA is listed only once). In certain instances, no gRNA was identified based on the criteria of the particular tier.

For designing knock out strategies, in some embodiments, the targeting domain for tier 1 grNA molecules for S. aureus were selected within the first 500bp of the coding sequence, had a high level of orthogonality, and contained a NNGRRT PAM. The targeting domain for tier 2 grNA molecules for S. aureus were selected within the first 500bp of the coding sequence, no level of orthogonality was required, and contained a NNGRRT PAM. The targeting domain for tier 3 gRNA molecules for S. aureus were selected within the remainder of the coding sequence downstream and contained a NNGRRT PAM. The targeting domain for tier 4 gRNA molecules for S. aureus were selected within the first 500bp of the coding sequence and contained a NNGRRV PAM. The targeting domain for tier 5 gRNA molecules for S. aureus were selected within the remainder of the coding sequence downstream and contained a NNGRRV PAM. Note that tiers are non-inclusive (each gRNA is listed only once). In certain instances, no gRNA was identified based on the criteria of the particular tier.

For designing of gRNA molecules for knocking down strategies, in some embodiments, the targeting domain for tier 1 gRNA molecules for S. pyogenes were selected within the first 500bp upstream and downstream of the transcription start site and had a high level of orthogonality. The targeting domain for tier 2 gRNA molecules for S. pyogenes were selected within the first 500bp upstream and downstream of the transcription start site and did not require high orthogonality. The targeting domain for tier 3 gRNA molecules for S. pyogenes were selected within the additional 500bp upstream and downstream of transcription start site (e.g., extending to 1kb up and downstream of the transcription start site). Note that tiers are non-inclusive (each gRNA is listed only once). In certain instances, no gRNA was identified based on the criteria of the particular tier.

For designing of gRNA molecules for knocking down strategies, in some embodiments, the targeting domain for tier 1 gRNA molecules for N. meningtidis were selected within the first 500bp upstream and downstream of the transcription start site and had a high level of orthogonality. The targeting domain for tier 2 gRNA molecules for N. meningtidis were selected within the first 500bp upstream and downstream of the transcription start site and did not require high orthogonality. The targeting domain for tier 3 gRNA molecules for N. meningtidis were selected within the additional 500bp upstream and downstream of transcription start site (e.g., extending to 1kb up and downstream of the transcription start site). Note that tiers are non-inclusive (each gRNA is listed only once). In certain instances, no gRNA was identified based on the criteria of the particular tier.

For designing of gRNA molecules for knocking down strategies, in some embodiments, the targeting domain for tier 1 gRNA molecules for S. aureus were selected within 500bp upstream and downstream of transcription start site, a high level of orthogonality and PAM is NNGRRT. The targeting domain for tier 2 gRNA molecules for S. aureus were selected within 500bp upstream and downstream of transcription start site, no orthogonality requirement and PAM is NNGRRT. The targeting domain for tier 3 gRNA molecules for S. aureus were selected within the additional 500bp upstream and downstream of transcription start site (e.g., extending to 1kb up and downstream of the transcription start site) and PAM is NNGRRT. The targeting domain for tier 4 gRNA molecules for S. aureus were selected within 500bp upstream and downstream of transcription start site and PAM is NNGRRV. The targeting domain for tier 5 gRNA molecules for S. aureus were selected within the additional 500bp upstream and downstream of transcription start site (extending to 1kb up and downstream of the transcription start site) and PAM is NNGRRV. Note that tiers are non-inclusive (each gRNA is listed only once). In certain instances, no gRNA was identified based on the criteria of the particular tier.

### Exemplary Targeting Domains (Second Strategy)

Below are tables for providing exemplary targeting domains according to the second design and tiering strategy. As an example, for *S. pyogenes* and *N. meningtidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed.

**Table** 13A provides targeting domains for knocking out the *FAS* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13A**

| 1st Tier |
|---|
| Targeting domains for knocking out the *FAS* gene using *S. pyogenes* Cas9 selected according to first tier parameters are presented in SEQ ID NO:27729-27741. |

**Table 13B** provides targeting domains for knocking out the *FAS* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13B**

| 2nd Tier |
|---|
| Targeting domains for knocking out the *FAS* gene using *S. pyogenes* Cas9 selected according to second tier parameters are presented in SEQ ID NO:27742-27888. |

**Table 13C** provides targeting domains for knocking out the *FAS* gene using *S. pyogenes* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13C**

| 3rd Tier |
|---|
| Targeting domains for knocking out the *FAS* gene using *S. pyogenes* Cas9 selected according to third tier parameters are presented in SEQ ID NO:27889-27968. |

**Table 13D** provides targeting domains for knocking out the *FAS* gene using *S. aureus* Cas9 selected according to first tier parameters. The targeting domains were selected within the first 500bp of the coding sequence, had a high level of orthogonality, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13D**

| 1st Tier |
|---|
| Targeting domains for knocking out the *FAS* gene using *S. aureus* Cas9 selected according to first tier parameters are presented in SEQ ID NO:27969-28034. |

**Table 13E** provides targeting domains for knocking out the *FAS* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13E**

| 2nd Tier |
|---|
| Targeting domains for knocking out the *FAS* gene using *S. aureus* Cas9 selected according to second tier parameters are presented in SEQ ID NO:28035-28101. |

**Table 13F** provides targeting domains for knocking out the *FAS* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13F**

| 3rd Tier |
|---|
| Targeting domains for knocking out the *FAS* gene using *S. aureus* Cas9 selected according to third tier parameters are presented in SEQ ID NO:28102-28639. |

**Table 13G** provides targeting domains for knocking out the *FAS* gene using *S. aureus* Cas9 selected according to fourth tier parameters. The targeting domains were selected within the first 500bp of the coding sequence and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13G**

| 4th Tier | |
|---|---|
| Targeting domains for knocking out the *FAS* gene using *S. aureus* Cas9 selected according to fourth tier parameters are presented in SEQ ID NO:28640-28772. | |

**Table 13H** provides targeting domains for knocking out the *FAS* gene using *S. aureus* Cas9 selected according to fifth tier parameters. The targeting domains were selected within the remainder of the coding sequence downstream and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13H**

| 5th Tier |
|---|
| Targeting domains for knocking out the *FAS* gene using *S. aureus* Cas9 selected according to fifth tier parameters are presented in SEQ ID NO:28773-29209. |

**Table 13I** provides targeting domains for knocking out the *FAS* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13I**

| 1st Tier |
|---|
| Targeting domains for knocking out the *FAS* gene using *N. meningitidismeningitidis* Cas9 selected according to first tier parameters are presented in SEQ ID NO:29210-29229. |

**Table 13J** provides targeting domains for knocking out the *FAS* gene using *N. meningitidis* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13J**

| 2nd Tier |
|---|
| Targeting domains for knocking out the *FAS* gene using *N. meningitidismeningitidis* Cas9 selected according to second tier parameters are presented in SEQ ID NO:29230-29231. |

**Table 13K** provides targeting domains for knocking out the *FAS* gene using *N. meningitidis* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 13K**

| 3rd Tier |
|---|
| Targeting domains for knocking out the *FAS* gene using *N. meningitidismeningitidis* Cas9 selected according to third tier parameters are presented in SEQ ID NO:29232-29266. |

**Table 14A** provides targeting domains for knocking down the *FAS* gene using *S. pyogenes* Cas9 selected according to first tier parameters. The targeting domains were selected within the first 500bp upstream and downstream of the transcription start site and had a high level of orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 14A**

| 1st Tier |
|---|
| Targeting domains for knocking down the *FAS* gene using *S. pyogenes* Cas9 selected according to first tier parameters are presented in SEQ ID NO:29267-29363. |

**Table 14B** provides targeting domains for knocking down the *FAS* gene using *S. pyogenes* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 14B**

| 2nd Tier |
|---|
| Targeting domains for knocking down the *FAS* gene using *S. pyogenes* Cas9 selected according to second tier parameters are presented in SEQ ID NO:29364-29648. |

**Table 14C** provides targeting domains for knocking down the *FAS* gene using *S. pyogenes* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 14C**

| 3rd Tier |
|---|
| Targeting domains for knocking down the *FAS* gene using *S. pyogenes* Cas9 selected according to third tier parameters are presented in SEQ ID NO:29649-29960. |

**Table 14D** provides targeting domains for knocking down the *FAS* gene using *S. aureus* Cas9 selected according to first tier parameters. The targeting domains were selected within 500bp upstream and downstream of transcription start site, a high level of orthogonality and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 14D**

| 1st Tier |
|---|
| Targeting domains for knocking down the *FAS* gene using *S. aureus* Cas9 selected according to first tier parameters are presented in SEQ ID NO:29961-30083. |

**Table 14E** provides targeting domains for knocking down the *FAS* gene using *S. aureus* Cas9 selected according to second tier parameters. The targeting domains were selected within 500bp upstream and downstream of transcription start site, no orthogonality requirement and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 14E**

| 2nd Tier |
|---|
| Targeting domains for knocking down the *FAS* gene using *S. aureus* Cas9 selected according to second tier parameters are presented in SEQ ID NO:30084-30149. |

**Table 14F** provides targeting domains for knocking down the *FAS* gene using *S. aureus* Cas9 selected according to third tier parameters. The targeting domains were selected within the additional 500bp upstream and downstream of transcription start site (e.g. extending to 1kb up and downstream of the transcription start site) and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 14F**

| 3rd Tier |
|---|
| Targeting domains for knocking down the *FAS* gene using *S. aureus* Cas9 selected according to third tier parameters are presented in SEQ ID NO:30150-31395. |

**Table 14G** provides targeting domains for knocking down the *FAS* gene using *S. aureus* Cas9 selected according to fourth tier parameters. The targeting domains were selected within 500bp upstream and downstream of transcription start site and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 14G**

| 4th Tier |
|---|
| Targeting domains for knocking down the *FAS* gene using *S. aureus* Cas9 selected according to fourth tier parameters are presented in SEQ ID NO:31396-31556. |

**Table 14H** provides targeting domains for knocking down the *FAS* gene using *S. aureus* Cas9 selected according to fifth tier parameters. The targeting domains were selected within the additional 500bp upstream and downstream of transcription start site (extending to 1kb up and downstream of the transcription start site) and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 14H**

| 5th Tier |
|---|
| Targeting domains for knocking down the *FAS* gene using *S. aureus* Cas9 selected according to fifth tier parameters are presented in SEQ ID NO:31557-32573. |

**Table 14I** provides targeting domains for knocking down the *FAS* gene using *N. meningitidis* Cas9 selected according to first tier parameters. The targeting were selected within the first 500bp upstream and downstream of the transcription start site and had a high level of orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 14I**

| 1st Tier |
|---|
| Targeting domains for knocking down the *FAS* gene using *N. meningitidismeningitidis* Cas9 selected according to first tier parameters are presented in SEQ ID NO:32574-32597. |

**Table 14J** provides targeting domains for knocking down the *FAS* gene using *N. meningitidis* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 14J**

| 2nd Tier |
|---|
| Targeting domains for knocking down the *FAS* gene using *N. meningitidismeningitidis* Cas9 selected according to second tier parameters are presented in SEQ ID NO:32598-32599. |

**Table 14K** provides targeting domains for knocking down the *FAS* gene using *N. meningitidis* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 14K**

| 3rd Tier |
|---|
| Targeting domains for knocking down the *FAS* gene using *N. meningitidismeningitidis* Cas9 selected according to third tier parameters are presented in SEQ ID NO:32600-32635. |

**Table 15A** provides targeting domains for knocking out the *BID* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 15A**

| 1st Tier |
|---|
| Targeting domains for knocking out the *BID* gene using *S. pyogenes* Cas9 selected according to first tier parameters are presented in SEQ ID NO:40252-40513. |

**Table 15B** provides targeting domains for knocking out the *BID* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 15B**

| 2nd Tier |
|---|
| Targeting domains for knocking out the *BID* gene using *S. pyogenes* Cas9 selected according to second tier parameters are presented in SEQ ID NO:40514-40627. |

**Table 15C** provides targeting domains for knocking out the *BID* gene using *S. aureus* Cas9 selected according to first tier parameters. The targeting domains were selected within the first 500bp of the coding sequence, had a high level of orthogonality, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 15C**

| 1st Tier |
|---|
| Targeting domains for knocking out the *BID* gene using *S. aureus* Cas9 selected according to first tier parameters are presented in SEQ ID NO:40628-40675. |

**Table 15D** provides targeting domains for knocking out the *BID* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 15D**

| 2nd Tier |
|---|
| Targeting domains for knocking out the *BID* gene using *S. aureus* Cas9 selected according to second tier parameters are presented in SEQ ID NO:40676-40802. |

**Table 15E** provides targeting domains for knocking out the *BID* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 15E**

| 3rd Tier |
|---|
| Targeting domains for knocking out the *BID* gene using *S. aureus* Cas9 selected according to third tier parameters are presented in SEQ ID NO:40803-41834. |

**Table 15F** provides targeting domains for knocking out the *BID* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 15F**

| 1st Tier |
|---|
| Targeting domains for knocking out the *BID* gene using *N. meningitidismeningitidis* Cas9 selected according to first tier parameters are presented in SEQ ID NO:41835-41848. |

**Table 16A** provides targeting domains for knocking down the *BID* gene using *S. pyogenes* Cas9 selected according to first tier parameters. The targeting domains were selected within the first 500bp upstream and downstream of the transcription start site and had a high level of orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 16A**

| 1st Tier |
|---|
| Targeting domains for knocking down the *BID* gene using *S. pyogenes* Cas9 selected according to first tier parameters are presented in SEQ ID NO:41849-41956. |

**Table 16B** provides targeting domains for knocking down the *BID* gene using *S. pyogenes* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 16B**

| 2nd Tier |
|---|
| Targeting domains for knocking down the *BID* gene using *S. pyogenes* Cas9 selected according to second tier parameters are presented in SEQ ID NO:41957-42424. |

**Table 16C** provides targeting domains for knocking down the *BID* gene using *S. pyogenes* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 16C**

| 3rd Tier |
|---|
| Targeting domains for knocking down the *BID* gene using *S. pyogenes* Cas9 selected according to third tier parameters are presented in SEQ ID NO:42425-42854. |

**Table 16D** provides targeting domains for knocking down the *BID* gene using *S. aureus* Cas9 selected according to first tier parameters. The targeting domains were selected within 500bp upstream and downstream of transcription start site, a high level of orthogonality and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 16D**

| 1st Tier |
|---|
| Targeting domains for knocking down the *BID* gene using *S. aureus* Cas9 selected according to first tier parameters are presented in SEQ ID NO:42855-42963. |

**Table 16E** provides targeting domains for knocking down the *BID* gene using *S. aureus* Cas9 selected according to second tier parameters. The targeting domains were selected within 500bp upstream and downstream of transcription start site, no orthogonality requirement and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 16E**

| 2nd Tier |
|---|
| Targeting domains for knocking down the *BID* gene using *S. aureus* Cas9 selected according to second tier parameters are presented in SEQ ID NO:42964-43092. |

**Table 16F** provides targeting domains for knocking down the *BID* gene using *S. aureus* Cas9 selected according to third tier parameters. The targeting domains were selected within the additional 500bp upstream and downstream of transcription start site (e.g. extending to 1kb up and downstream of the transcription start site) and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 16F**

| 3rd Tier |
|---|
| Targeting domains for knocking down the *BID* gene using *S. aureus* Cas9 selected according to third tier parameters are presented in SEQ ID NO:43093-44450. |

**Table 16G** provides targeting domains for knocking down the *BID* gene using *S. aureus* Cas9 selected according to fourth tier parameters. The targeting domains were selected within the first 500bp of the coding sequence and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 16G**

| 4th Tier |
|---|
| Targeting domains for knocking down the *BID* gene using *S. aureus* Cas9 selected according to fourth tier parameters are presented in SEQ ID NO:44451-44744. |

**Table 16H** provides targeting domains for knocking down the *BID* gene using *S. aureus* Cas9 selected according to fifth tier parameters. The targeting domains were selected within the remainder of the coding sequence downstream and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 16H**

| 5th Tier |
|---|
| Targeting domains for knocking down the *BID* gene using *S. aureus* Cas9 selected according to fifth tier parameters are presented in SEQ ID NO:44745-45940. |

**Table 16I** provides targeting domains for knocking down the *BID* gene using *N. meningitidis* Cas9 selected according to first tier parameters. The targeting were selected within the first 500bp upstream and downstream of the transcription start site and had a high level of orthogonality. It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 16I**

| 1st Tier |
|---|
| Targeting domains for knocking down the *BID* gene using *N. meningitidismeningitidis* Cas9 selected according to first tier parameters are presented in SEQ ID NO:45941-45955. |

**Table 16J** provides targeting domains for knocking down the *BID* gene using *N*. *meningitidis* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 16J**

| 2nd Tier |
|---|
| Targeting domains for knocking down the *BID* gene using *N. meningitidismeningitidis* Cas9 selected according to second tier parameters are presented in SEQ ID NO:45956-45958. |

**Table 16K** provides targeting domains for knocking down the *BID* gene using *N. meningitidis* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 16K**

| 3rd Tier |
|---|
| Targeting domains for knocking down the *BID* gene using *N. meningitidismeningitidis* Cas9 selected according to third tier parameters are presented in SEQ ID NO:45959-45980. |

**Table 17A** provides targeting domains for knocking out the *CTLA4* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17A**

| 1st Tier |
|---|
| Targeting domains for knocking out the *CTLA4* gene using *S. pyogenes* Cas9 selected according to first tier parameters are presented in SEQ ID NO:45981-46023. |

**Table 17B** provides targeting domains for knocking out the *CTLA4* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17B**

| 2nd Tier |
|---|
| Targeting domains for knocking out the *CTLA4* gene using *S. pyogenes* Cas9 selected according to second tier parameters are presented in SEQ ID NO:46024-46149. |

**Table 17C** provides targeting domains for knocking out the *CTLA4* gene using *S. pyogenes* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17C**

| 3rd Tier |
|---|
| Targeting domains for knocking out the *CTLA4* gene using *S. pyogenes* Cas9 selected according to third tier parameters are presented in SEQ ID NO:46150-46207. |

**Table 17D** provides targeting domains for knocking out the *CTLA4* gene using *S. aureus* Cas9 selected according to first tier parameters. The targeting domains were selected within the first 500bp of the coding sequence, had a high level of orthogonality, and contained a NNGRRT PAM.. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17D**

| 1st Tier |
|---|
| Targeting domains for knocking out the *CTLA4* gene using *S. aureus* Cas9 selected according to first tier parameters are presented in SEQ ID NO:46208-46269. |

**Table 17E** provides targeting domains for knocking out the *CTLA4* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17E**

| 2nd Tier |
|---|
| Targeting domains for knocking out the *CTLA4* gene using *S. aureus* Cas9 selected according to second tier parameters are presented in SEQ ID NO:46270-46305. |

**Table 17F** provides targeting domains for knocking out the *CTLA4* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17F**

| 3rd Tier |
|---|
| Targeting domains for knocking out the *CTLA4* gene using *S. aureus* Cas9 selected according to third tier parameters are presented in SEQ ID NO:46306-46718. |

**Table 17G** provides targeting domains for knocking out the *CTLA4* gene using *S. aureus* Cas9 selected according to fourth tier parameters. The targeting domains were selected within the first 500bp of the coding sequence and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17G**

| 4th Tier |
|---|
| Targeting domains for knocking out the *CTLA4* gene using *S. aureus* Cas9 selected according to fourth tier parameters are presented in SEQ ID NO:46719-46788. |

**Table 17H** provides targeting domains for knocking out the *CTLA4* gene using *S. aureus* Cas9 selected according to fifth tier parameters. The targeting domains were selected within the remainder of the coding sequence downstream and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17H**

| 5th Tier |
|---|
| Targeting domains for knocking out the *CTLA4* gene using *S. aureus* Cas9 selected according to fifth tier parameters are presented in SEQ ID NO:46789-47047. |

**Table 17I** provides targeting domains for knocking out the *CTLA4* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17I**

| 1st Tier |
|---|
| Targeting domains for knocking out the *CTLA4* gene using *N. meningitidismeningitidis* Cas9 selected according to first tier parameters are presented in SEQ ID NO:47048-47064. |

**Table 17J** provides targeting domains for knocking out the *CTLA4* gene using *N. meningitidis* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17J**

| 2nd Tier |
|---|
| A targeting domains for knocking out the *CTLA4* gene using *N. meningitidismeningitidis* Cas9 selected according to second tier parameters is presented in SEQ ID NO:47065. |

**Table 17K** provides targeting domains for knocking out the *CTLA4* gene using *N. meningitidis* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 17K**

| 3rd Tier |
|---|
| Targeting domains for knocking out the *CTLA4* gene using *N. meningitidismeningitidis* Cas9 selected according to third tier parameters are presented in SEQ ID NO:47066-47073. |

**Table 18A** provides targeting domains for knocking down the *CTLA4* gene using *S. pyogenes* Cas9 selected according to first tier parameters. The targeting domains were selected within the first 500bp upstream and downstream of the transcription start site and had a high level of orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 18A**

| 1st Tier |
|---|
| Targeting domains for knocking down the *CTLA4* gene using *S. pyogenes* Cas9 selected according to first tier parameters are presented in SEQ ID NO:47074-47104. |

**Table 18B** provides targeting domains for knocking down the *CTLA4* gene using *S. pyogenes* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 18B**

| 2nd Tier |
|---|
| Targeting domains for knocking down the *CTLA4* gene using *S. pyogenes* Cas9 selected according to second tier parameters are presented in SEQ ID NO:47105-47291. |

**Table 18C** provides targeting domains for knocking down the *CTLA4* gene using *S. pyogenes* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 18C**

| 3rd Tier |
|---|
| Targeting domains for knocking down the *CTLA4* gene using *S. pyogenes* Cas9 selected according to third tier parameters are presented in SEQ ID NO:47292-47446. |

**Table 18D** provides targeting domains for knocking down the *CTLA4* gene using *S. aureus* Cas9 selected according to first tier parameters. The targeting domains were selected within 500bp upstream and downstream of transcription start site, a high level of orthogonality and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 18D**

| 1st Tier |
|---|
| Targeting domains for knocking down the *CTLA4* gene using *S. aureus* Cas9 selected according to first tier parameters are presented in SEQ ID NO:47447-47574. |

**Table 18E** provides targeting domains for knocking down the *CTLA4* gene using *S. aureus* Cas9 selected according to second tier parameters. The targeting domains were selected within 500bp upstream and downstream of transcription start site, no orthogonality requirement and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 18E**

| 2nd Tier |
|---|
| Targeting domains for knocking down the *CTLA4* gene using *S. aureus* Cas9 selected according to second tier parameters are presented in SEQ ID NO:47575-47677. |

**Table 18F** provides targeting domains for knocking down the *CTLA4* gene using *S. aureus* Cas9 selected according to third tier parameters. The targeting domains were selected within the additional 500bp upstream and downstream of transcription start site (e.g. extending to 1kb up and downstream of the transcription start site) and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 18F**

| 3rd Tier |
|---|
| Targeting domains for knocking down the *CTLA4* gene using *S. aureus* Cas9 selected according to third tier parameters are presented in SEQ ID NO:47678-48391. |

**Table 18G** provides targeting domains for knocking down the *CTLA4* gene using *S. aureus* Cas9 selected according to fourth tier parameters. The targeting domains were selected within 500bp upstream and downstream of transcription start site and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 18G**

| 4th Tier |
|---|
| Targeting domains for knocking down the *CTLA4* gene using *S. aureus* Cas9 selected according to fourth tier parameters are presented in SEQ ID NO:48392-48618. |

**Table 18H** provides targeting domains for knocking down the *CTLA4* gene using *S. aureus* Cas9 selected according to fifth tier parameters. The targeting domains were selected within the additional 500bp upstream and downstream of transcription start site (extending to 1kb up and downstream of the transcription start site) and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 18H**

| 5th Tier |
|---|
| Targeting domains for knocking down the *CTLA4* gene using *S. aureus* Cas9 selected according to fifth tier parameters are presented in SEQ ID NO:48619-49187. |

**Table 18I** provides targeting domains for knocking down the *CTLA4* gene using *N. meningitidis* Cas9 selected according to first tier parameters. The targeting were selected within the first 500bp upstream and downstream of the transcription start site and had a high level of orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 18I**

| 1st Tier |
|---|
| Targeting domains for knocking down the *CTLA4* gene using *N. meningitidismeningitidis* Cas9 selected according to first tier parameters are presented in SEQ ID NO:49188-49232. |

**Table 18J** provides targeting domains for knocking down the *CTLA4* gene using *N. meningitidis* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 18J**

| 2nd Tier |
|---|
| Targeting domains for knocking down the *CTLA4* gene using *N. meningitidismeningitidis* Cas9 selected according to second tier parameters are presented in SEQ ID NO:49233-49239. |

**Table 18K** provides targeting domains for knocking down the *CTLA4* gene using *N. meningitidis* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 18K**

| 3rd Tier |
|---|
| Targeting domains for knocking down the *CTLA4* gene using *N. meningitidismeningitidis* Cas9 selected according to third tier parameters are presented in SEQ ID NO:49240-49273. |

**Table 19A** provides targeting domains for knocking out the *PDCD1* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19A**

| 1st Tier |
|---|
| Targeting domains for knocking out the *PDCD1* gene using *S. pyogenes* Cas9 selected according to first tier parameters are presented in SEQ ID NO:14657-14724. |

**Table 19B** provides targeting domains for knocking out the *PDCD1* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19B**

| 2nd Tier |
|---|
| Targeting domains for knocking out the *PDCD1* gene using *S. pyogenes* Cas9 selected according to second tier parameters are presented in SEQ ID NO:14725-14910. |

**Table 19C** provides targeting domains for knocking out the *PDCD1* gene using *S. pyogenes* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19C**

| 3rd Tier |
|---|
| Targeting domains for knocking out the *PDCD1* gene using *S. pyogenes* Cas9 selected according to third tier parameters are presented in SEQ ID NO:14911-15185. |

**Table 19D** provides targeting domains for knocking out the *PDCD1* gene using *S. aureus* Cas9 selected according to first tier parameters. The targeting domains were selected within the first 500bp of the coding sequence, had a high level of orthogonality, and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19D**

| 1st Tier |
|---|
| Targeting domains for knocking out the *PDCD1* gene using *S. aureus* Cas9 selected according to first tier parameters are presented in SEQ ID NO:15186-15214. |

**Table 19E** provides targeting domains for knocking out the *PDCD1* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19E**

| 2nd Tier |
|---|
| Targeting domains for knocking out the *PDCD1* gene using *S. aureus* Cas9 selected according to second tier parameters are presented in SEQ ID NO:15215-15241. |

**Table 19F** provides targeting domains for knocking out the *PDCD1* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19F**

| 3rd Tier |
|---|
| Targeting domains for knocking out the *PDCD1* gene using *S. aureus* Cas9 selected according to third tier parameters are presented in SEQ ID NO:15242-15857. |

**Table 19G** provides targeting domains for knocking out the *PDCD1* gene using *S. aureus* Cas9 selected according to fourth tier parameters. The targeting domains were selected within the first 500bp of the coding sequence and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19G**

| 4th Tier |
|---|
| Targeting domains for knocking out the *PDCD1* gene using *S. aureus* Cas9 selected according to fourth tier parameters are presented in SEQ ID NO:15858-15969. |

**Table 19H** provides targeting domains for knocking out the *PDCD1* gene using *S. aureus* Cas9 selected according to fifth tier parameters. The targeting domains were selected within the remainder of the coding sequence downstream and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19H**

| 5th Ti er |
|---|
| Targeting domains for knocking out the *PDCD1* gene using *S. aureus* Cas9 selected according to fifth tier parameters are presented in SEQ ID NO:15970-16658. |

**Table 19I** provides targeting domains for knocking out the *PDCD1* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19I**

| 1st Tier |
|---|
| Targeting domains for knocking out the *PDCD1* gene using *N. meningitidismeningitidis* Cas9 selected according to first tier parameters are presented in SEQ ID NO:16659-16666. |

**Table 19J** provides targeting domains for knocking out the *PDCD1* gene using *N. meningitidis* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 19J**

| 3rd Tier |
|---|
| Targeting domains for knocking out the *PDCD1* gene using *N. meningitidismeningitidis* Cas9 selected according to third tier parameters are presented in SEQ ID NO:16667-16670. |

**Table 20A** provides targeting domains for knocking down the *PDCD1* gene using *S. pyogenes* Cas9 selected according to first tier parameters. The targeting domains were selected within the first 500bp upstream and downstream of the transcription start site and had a high level of orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 20A**

| 1st Tier |
|---|
| Targeting domains for knocking down the *PDCD1* gene using *S. pyogenes* Cas9 selected according to first tier parameters are presented in SEQ ID NO: 16671-16718. |

**Table 20B** provides targeting domains for knocking down the *PDCD1* gene using *S. pyogenes* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 20B**

| 2nd Tier |
|---|
| Targeting domains for knocking down the *PDCD1* gene using *S. pyogenes* Cas9 selected according to second tier parameters are presented in SEQ ID NO:16719-17276. |

**Table 20C** provides targeting domains for knocking down the *PDCD1* gene using *S. pyogenes* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 20C**

| 3rd Tier |
|---|
| Targeting domains for knocking down the *PDCD1* gene using *S. pyogenes* Cas9 selected according to third tier parameters are presented in SEQ ID NO: 17277-17713. |

**Table 20D** provides targeting domains for knocking down the *PDCD1* gene using *S. aureus* Cas9 selected according to first tier parameters. The targeting domains were selected within 500bp upstream and downstream of transcription start site, a high level of orthogonality and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 20D**

| 1st Tier |
|---|
| Targeting domains for knocking down the *PDCD1* gene using *S. aureus* Cas9 selected according to first tier parameters are presented in SEQ ID NO:17714-17818. |

**Table 20E** provides targeting domains for knocking down the *PDCD1* gene using *S. aureus* Cas9 selected according to second tier parameters. The targeting domains were selected within 500bp upstream and downstream of transcription start site, no orthogonality requirement and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 20E**

| 2nd Tier |
|---|
| Targeting domains for knocking down the *PDCD1* gene using *S. aureus* Cas9 selected according to second tier parameters are presented in SEQ ID NO:17819-17921. |

**Table 20F** provides targeting domains for knocking down the *PDCD1* gene using *S. aureus* Cas9 selected according to third tier parameters. The targeting domains were selected within the additional 500bp upstream and downstream of transcription start site (e.g. extending to 1kb up and downstream of the transcription start site) and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 20F**

| 3rd Tier |
|---|
| Targeting domains for knocking down the *PDCD1* gene using *S. aureus* Cas9 selected according to third tier parameters are presented in SEQ ID NO:17922-19687. |

**Table 20G** provides targeting domains for knocking down the *PDCD1* gene using *S. aureus* Cas9 selected according to fourth tier parameters. The targeting domains were selected within 500bp upstream and downstream of transcription start site and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 20G**

| 4th Tier |
|---|
| Targeting domains for knocking down the *PDCD1* gene using *S. aureus* Cas9 selected according to fourth tier parameters are presented in SEQ ID NO:19688-19904. |

**Table 20H** provides targeting domains for knocking down the *PDCD1* gene using *S. aureus* Cas9 selected according to fifth tier parameters. The targeting domains were selected within the additional 500bp upstream and downstream of transcription start site (extending to 1kb up and downstream of the transcription start site) and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 20H**

| 5th Tier |
|---|
| Targeting domains for knocking down the *PDCD1* gene using *S. aureus* Cas9 selected according to fifth tier parameters are presented in SEQ ID NO:19905-21017. |

**Table 20I** provides targeting domains for knocking down the *PDCD1* gene using *N. meningitidis* Cas9 selected according to first tier parameters. The targeting were selected within the first 500bp upstream and downstream of the transcription start site and had a high level of orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 20I**

| 1st Tier |
|---|
| Targeting domains for knocking down the *PDCD1* gene using *N. meningitidismeningitidis* Cas9 selected according to first tier parameters are presented in SEQ ID NO:21018-21019. |

**Table 20J** provides targeting domains for knocking down the *PDCD1* gene using *N. meningitidis* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 20J**

| 3rd Tier |
|---|
| Targeting domains for knocking down the *PDCD1* gene using *N. meningitidismeningitidis* Cas9 selected according to third tier parameters are presented in SEQ ID NO:21020-21037. |

**Table 21A** provides targeting domains for knocking out the *CBLB* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21A**

| 1st Tier |
|---|
| Targeting domains for knocking out the *CBLB* gene using *S. pyogenes* Cas9 selected according to first tier parameters are presented in SEQ ID NO:32636-32652. |

**Table 21B** provides targeting domains for knocking out the *CBLB* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21B**

| 2nd Tier |
|---|
| Targeting domains for knocking out the *CBLB* gene using *S. pyogenes* Cas9 selected according to second tier parameters are presented in SEQ ID NO:32653-32731. |

**Table 21C** provides targeting domains for knocking out the *CBLB* gene using *S. pyogenes* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21C**

| 3rd Tier |
|---|
| Targeting domains for knocking out the *CBLB* gene using *S. pyogenes* Cas9 selected according to third tier parameters are presented in SEQ ID NO:32732-33533. |

**Table 21D** provides targeting domains for knocking out the *CBLB* gene using *S. aureus* Cas9 selected according to first tier parameters. The targeting domains were selected within the first 500bp of the coding sequence, had a high level of orthogonality, and contained a NNGRRT PAM.. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21D**

| 1st Tier |
|---|
| Targeting domains for knocking out the *CBLB* gene using *S. aureus* Cas9 selected according to first tier parameters are presented in SEQ ID NO:33534-33622. |

**Table 21E** provides targeting domains for knocking out the *CBLB* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21E**

| 2nd Tier |
|---|
| Targeting domains for knocking out the *CBLB* gene using *S. aureus* Cas9 selected according to second tier parameters are presented in SEQ ID NO:33623-33659. |

**Table 21F** provides targeting domains for knocking out the *CBLB* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21F**

| 3rd Tier |
|---|
| Targeting domains for knocking out the *CBLB* gene using *S*. *aureus* Cas9 selected according to third tier parameters are presented in SEQ ID NO:33660-33935. |

**Table 21G** provides targeting domains for knocking out the *CBLB* gene using *S. aureus* Cas9 selected according to fourth tier parameters. The targeting domains were selected within the first 500bp of the coding sequence and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21G**

| 4th Tier |
|---|
| Targeting domains for knocking out the *CBLB* gene using *S. aureus* Cas9 selected according to fourth tier parameters are presented in SEQ ID NO:33936-34600. |

**Table 21H** provides targeting domains for knocking out the *CBLB* gene using *S. aureus* Cas9 selected according to fifth tier parameters. The targeting domains were selected within the remainder of the coding sequence downstream and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21H**

| 5th Tier |
|---|
| Targeting domains for knocking out the *CBLB* gene using *S. aureus* Cas9 selected according to fifth tier parameters are presented in SEQ ID NO:34601-37358. |

**Table 21I** provides targeting domains for knocking out the *CBLB* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21I**

| 1st Tier |
|---|
| Targeting domains for knocking out the *CBLB* gene using *N. meningitidismeningitidis* Cas9 selected according to first tier parameters are presented in SEQ ID NO:37359-37369. |

**Table 21J** provides targeting domains for knocking out the *CBLB* gene using *N. meningitidis* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21J**

| 2nd Tier |
|---|
| Targeting domains for knocking out the *CBLB* gene using *N. meningitidismeningitidis* Cas9 selected according to second tier parameters are presented in SEQ ID NO:37370-37372. |

**Table 21K** provides targeting domains for knocking out the *CBLB* gene using *N. meningitidis* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N*. *meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 21K**

| 3rd Tier |
|---|
| Targeting domains for knocking out the *CBLB* gene using *N. meningitidismeningitidis* Cas9 selected according to third tier parameters are presented in SEQ ID NO:37373-37471. |

**Table 22A** provides targeting domains for knocking down the *CBLB* gene using *S. pyogenes* Cas9 selected according to first tier parameters. The targeting domains were selected within the first 500bp upstream and downstream of the transcription start site and had a high level of orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 22A**

| 1st Tier |
|---|
| Targeting domains for knocking down the *CBLB* gene using *S. pyogenes* Cas9 selected according to first tier parameters are presented in SEQ ID NO:37472-37530. |

**Table 22B** provides targeting domains for knocking down the *CBLB* gene using *S. pyogenes* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 22B**

| 2nd Tier |
|---|
| Targeting domains for knocking down the *CBLB* gene using *S. pyogenes* Cas9 selected according to second tier parameters are presented in SEQ ID NO:37531-37754. |

**Table 22C** provides targeting domains for knocking down the *CBLB* gene using *S. pyogenes* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 22C**

| 3rd Tier |
|---|
| Targeting domains for knocking down the *CBLB* gene using *S. pyogenes* Cas9 selected according to third tier parameters are presented in SEQ ID NO:37755-38052. |

**Table 22D** provides targeting domains for knocking down the *CBLB* gene using *S. aureus* Cas9 selected according to first tier parameters. The targeting domains were selected within 500bp upstream and downstream of transcription start site, a high level of orthogonality and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 22D**

| 1st Tier |
|---|
| Targeting domains for knocking down the *CBLB* gene using *S. aureus* Cas9 selected according to first tier parameters are presented in SEQ ID NO:38053-38141. |

**Table 22E** provides targeting domains for knocking down the *CBLB* gene using *S. aureus* Cas9 selected according to second tier parameters. The targeting domains were selected within 500bp upstream and downstream of transcription start site, no orthogonality requirement and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 22E**

| 2nd Tier |
|---|
| Targeting domains for knocking down the *CBLB* gene using *S. aureus* Cas9 selected according to second tier parameters are presented in SEQ ID NO:38142-38206. |

**Table 22F** provides targeting domains for knocking down the *CBLB* gene using *S. aureus* Cas9 selected according to third tier parameters. The targeting domains were selected within the additional 500bp upstream and downstream of transcription start site (e.g. extending to 1kb up and downstream of the transcription start site) and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 22F**

| 3rd Tier |
|---|
| Targeting domains for knocking down the *CBLB* gene using *S. aureus* Cas9 selected according to third tier parameters are presented in SEQ ID NO:38207-39105. |

**Table 22G** provides targeting domains for knocking down the *CBLB* gene using *S. aureus* Cas9 selected according to fourth tier parameters. The targeting domains were selected within 500bp upstream and downstream of transcription start site and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 22G**

| 4th Tier |
|---|
| Targeting domains for knocking down the *CBLB* gene using *S. aureus* Cas9 selected according to fourth tier parameters are presented in SEQ ID NO:39106-39259. |

**Table 22H** provides targeting domains for knocking down the *CBLB* gene using *S. aureus* Cas9 selected according to fifth tier parameters. The targeting domains were selected within the additional 500bp upstream and downstream of transcription start site (extending to 1kb up and downstream of the transcription start site) and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 22H**

| 5th Tier |
|---|
| Targeting domains for knocking down the *CBLB* gene using *S. aureus* Cas9 selected according to fifth tier parameters are presented in SEQ ID NO:39260-40211. |

**Table 22I** provides targeting domains for knocking down the *CBLB* gene using *N. meningitidis* Cas9 selected according to first tier parameters. The targeting were selected within the first 500bp upstream and downstream of the transcription start site and had a high level of orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 22I**

| 1 st Tier |
|---|
| Targeting domains for knocking down the *CBLB* gene using *N. meningitidismeningitidis* Cas9 selected according to first tier parameters are presented in SEQ ID NO:40212-40230. |

**Table 22J** provides targeting domains for knocking down the *CBLB* gene using *N. meningitidis* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *N. meningitidis* eiCas9 molecule, e.g., an eiCas9 fusion protein, as described herein.

**Table 22J**

| 2nd Tier |
|---|
| Targeting domains for knocking down the *CBLB* gene using *N. meningitidismeningitidis* Cas9 selected according to second tier parameters are presented in SEQ ID NO:40231-40235. |

**Table 22K** provides targeting domains for knocking down the *CBLB* gene using *N. meningitidis* Cas9 selected according to third tier parameters. The targeting domains were selected within the additional 500bp upstream and downstream of transcription start site (e.g. extending to 1kb up and downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 22K**

| 3rd Tier |
|---|
| Targeting domains for knocking down the *CBLB* gene using *N. meningitidismeningitidis* Cas9 selected according to third tier parameters are presented in SEQ ID NO:40236-40251. |

**Table 23A** provides targeting domains for knocking out the *PTPN6* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23A**

| 1st Tier |
|---|
| Targeting domains for knocking out the *PTPN6* gene using *S. pyogenes* Cas9 selected according to first tier parameters are presented in SEQ ID NO:21038-21112. |

**Table 23B** provides targeting domains for knocking out the *PTPN6* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23B**

| 2nd Tier |
|---|
| Targeting domains for knocking out the *PTPN6* gene using *S. pyogenes* Cas9 selected according to second tier parameters are presented in SEQ ID NO:21113-21332. |

**Table 23C** provides targeting domains for knocking out the *PTPN6* gene using *S. pyogenes* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23C**

| 3rd Tier |
|---|
| Targeting domains for knocking out the *PTPN6* gene using *S. pyogenes* Cas9 selected according to third tier parameters are presented in SEQ ID NO:21333-21976. |

**Table 23D** provides targeting domains for knocking out the *PTPN6* gene using *S. aureus* Cas9 selected according to first tier parameters. The targeting domains were selected within the first 500bp of the coding sequence, had a high level of orthogonality, and contained a NNGRRT PAM.. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp

**Table 23D**

| 1st Tier |
|---|
| Targeting domains for knocking out the *PTPN6* gene using *S. aureus* Cas9 selected according to first tier parameters are presented in SEQ ID NO:21977-22077. |

**Table 23E** provides targeting domains for knocking out the *PTPN6* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23E**

| 2nd Tier |
|---|
| Targeting domains for knocking out the *PTPN6* gene using *S. aureus* Cas9 selected according to second tier parameters are presented in SEQ ID NO:22078-22137. |

**Table 23F** provides targeting domains for knocking out the *PTPN6* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23F**

| 3rd Tier |
|---|
| Targeting domains for knocking out the *PTPN6* gene using *S. aureus* Cas9 selected according to third tier parameters are presented in SEQ ID NO:22138-22815. |

**Table 23G** provides targeting domains for knocking out the *PTPN6* gene using *S. aureus* Cas9 selected according to fourth tier parameters. The targeting domains were selected within the first 500bp of the coding sequence and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23G**

| 4th Tier |
|---|
| Targeting domains for knocking out the *PTPN6* gene using *S. aureus* Cas9 selected according to fourth tier parameters are presented in SEQ ID NO:22816-23105. |

**Table 23H** provides targeting domains for knocking out the *PTPN6* gene using *S. aureus* Cas9 selected according to fifth tier parameters. The targeting domains were selected within the remainder of the coding sequence downstream and contained a NNGRRV PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23H**

| 5th Tier |
|---|
| Targeting domains for knocking out the *PTPN6* gene using *S. aureus* Cas9 selected according to fifth tier parameters are presented in SEQ TD NO:23106-24749. |

**Table 231** provides targeting domains for knocking out the *PTPN6* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 231**

| 1st Tier |
|---|
| Targeting domains for knocking out the *PTPN6* gene using *N. meningitidismeningitidis* Cas9 selected according to first tier parameters are presented in SEQ ID NO:24750-24755. |

**Table 23J** provides targeting domains for knocking out the *PTPN6* gene using *N. meningitidis* Cas9 selected according to third tier parameters. The targeting domains are beyond the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 23J**

| 3rd Tier |
|---|
| Targeting domains for knocking out the *PTPN6* gene using *N. meningitidismeningitidis* Cas9 selected according to third tier parameters are presented in SEQ ID NO:24756-24777. |

**Table 24A** provides targeting domains for knocking down the *PTPN6* gene using *S. pyogenes* Cas9 selected according to first tier parameters. The targeting domains were selected within the first 500bp upstream and downstream of the transcription start site and had a high level of orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 24A**

| 1st Tier |
|---|
| Targeting domains for knocking down the *PTPN6* gene using *S. pyogenes* Cas9 selected according to first tier parameters are presented in SEQ ID NO:24778-24841. |

**Table 24B** provides targeting domains for knocking down the *PTPN6* gene using *S. pyogenes* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 24B**

| 2nd Tier |
|---|
| Targeting domains for knocking down the *PTPN6* gene using *S. pyogenes* Cas9 selected according to second tier parameters are presented in SEQ ID NO:24842-25280. |

**Table 24C** provides targeting domains for knocking down the *PTPN6* gene using *S. pyogenes* Cas9 selected according to third tier parameters. The targeting domains were selected within the additional 73bp upstream and 500bp downstream of transcription start site (extending to 573bp up and 1kb downstream of the transcription start site). It is contemplated herein that the targeting domain hybridizes to the target domain through complementary base pairing. Any of the targeting domains in the table can be used with a *S. pyogenes* eiCas9 molecule, e.g., an eiCas9 fusion protein, as described herein.

**Table 24C**

| 3rd Tier |
|---|
| Targeting domains for knocking down the *PTPN6* gene using *S. pyogenes* Cas9 selected according to third tier parameters are presented in SEQ ID NO:25281-25439. |

**Table 24D** provides targeting domains for knocking down the *PTPN6* gene using *S. aureus* Cas9 selected according to first tier parameters. The targeting domains were selected within 500bp upstream and downstream of transcription start site, a high level of orthogonality and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 24D**

| 1st Tier |
|---|
| Targeting domains for knocking down the *PTPN6* gene using *S. aureus* Cas9 selected according to first tier parameters are presented in SEQ ID NO:25440-25521. |

**Table 24E** provides targeting domains for knocking down the *PTPN6* gene using *S. aureus* Cas9 selected according to second tier parameters. The targeting domains were selected within 500bp upstream and downstream of transcription start site, no orthogonality requirement and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 24E**

| 2nd Tier |
|---|
| Targeting domains for knocking down the *PTPN6* gene using *S. aureus* Cas9 selected according to second tier parameters are presented in SEQ ID NO:25522-25593. |

**Table 24F** provides targeting domains for knocking down the *PTPN6* gene using *S*. *aureus* Cas9 selected according to third tier parameters. The targeting domains were selected within the additional 500bp upstream and downstream of transcription start site (e.g. extending to 1kb up and downstream of the transcription start site) and PAM is NNGRRT. It is contemplated herein that the targeting domain hybridizes to the target domain through complementary base pairing. Any of the targeting domains in the table can be used with a *S*. *aureus* eiCas9 molecule, e.g., an eiCas9 fusion protein, as described herein.

**Table 24F**

| 3rd TiEr |
|---|
| Targeting domains for knocking down the *PTPN6* gene using *S. aureus* Cas9 selected according to third tier parameters are presented in SEQ ID NO:25594-27119. |

**Table 24G** provides targeting domains for knocking down the *PTPN6* gene using *S. aureus* Cas9 selected according to fourth tier parameters. The targeting domains were selected Within the additional 73bp upstream and 500bp downstream of transcription start site (extending to 573bp up and 1kb downstream of the transcription start site) and PAM is NNGRRT. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 24G**

| 4th Tier |
|---|
| Targeting domains for knocking down the *PTPN6* gene using *S. aureus* Cas9 selected according to fourth tier parameters are presented in SEQ ID NO:27120-27224. |

**Table 24H** provides targeting domains for knocking down the *PTPN6* gene using *S. aureus* Cas9 selected according to fifth tier parameters. The targeting domains were selected within the additional 73bp upstream and 500bp downstream of transcription start site (extending to 573bp up and 1kb downstream of the transcription start site) and PAM is NNGRRV. It is contemplated herein that the targeting domain hybridizes to the target domain through complementary base pairing. Any of the targeting domains in the table can be used with a *S. aureus* eiCas9 molecule, e.g., an eiCas9 fusion protein, as described herein.

**Table 24H**

| 5th Tier |
|---|
| Targeting domains for knocking down the *PTPN6* gene using *S. aureus* Cas9 selected according to fifth tier parameters are presented in SEQ ID NO:27225-27698. |

**Table 24I** provides targeting domains for knocking down the *PTPN6* gene using *N. meningitidis* Cas9 selected according to first tier parameters. The targeting were selected within the first 500bp upstream and downstream of the transcription start site and had a high level of orthogonality. It is contemplated herein that the targeting domain hybridizes to the target domain through complementary base pairing. Any of the targeting domains in the table can be used with a N. *meningitidis* eiCas9 molecule, e.g., an eiCas9 fusion protein, as described herein.

**Table 24I**

| 1st Tier |
|---|
| Targeting domains for knocking down the *PTPN6* gene using *N. meningitidismeningitidis* Cas9 selected according to first tier parameters are presented in SEQ ID NO:27699-27715. |

**Table 24J** provides targeting domains for knocking down the *PTPN6* gene using *N. meningitidis* Cas9 selected according to second tier parameters. The targeting domains were selected within the first 500bp upstream and downstream of the transcription start site and did not require high orthogonality. It is contemplated herein that the targeting domain hybridizes to the target domain through complementary base pairing. Any of the targeting domains in the table can be used with a N. *meningitidis* eiCas9 molecule, e.g., an eiCas9 fusion protein, as described herein.

**Table 24J**

| 2nd Tier |
|---|
| Targeting domains for knocking down the *PTPN6* gene using *N. meningitidismeningitidis* Cas9 selected according to second tier parameters are presented in SEQ ID NO:27716-27718. |

**Table 24K** provides targeting domains for knocking down the *PTPN6* gene using *N. meningitidis* Cas9 selected according to third tier parameters. The targeting domains were selected within the additional 73bp upstream and 500bp downstream of transcription start site (extending to 573bp up and 1kb downstream of the transcription start site). It is contemplated herein that the targeting domain hybridizes to the target domain through complementary base pairing. Any of the targeting domains in the table can be used with a *N*. *meningitidis* eiCas9 molecule, e.g., an eiCas9 fusion protein, as described herein.

**Table 24K**

| 3rd Tier |
|---|
| Targeting domains for knocking down the *PTPN6* gene using *N. meningitidismeningitidis* Cas9 selected according to third tier parameters are presented in SEQ ID NO:27719-27728. |

**Table 25A** provides targeting domains for knocking out the *TRAC* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with a targeting domain from Group B as shown in **Table 25-1.**

**Table 25-1**

| Group A | Group B |
|---|---|
| GCUAGACAUGAGGUCUA (SEQ ID NO:49303) | AAAGUCAGAUUUGUUGCUCC (SEQ ID NO:49379) |

**Table 25A**

| 1st Tier |
|---|
| Targeting domains for knocking out the *TRAC* gene using *S. pyogenes* Cas9 selected according to first tier parameters are presented in SEQ ID NO:49274-49291. |

**Table 25B** provides targeting domains for knocking out the *TRAC* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 25B**

| 2nd Tier |
|---|
| Targeting domains for knocking out the *TRAC* gene using *S. pyogenes* Cas9 selected according to second tier parameters are presented in SEQ ID NO:49292-49415. |

**Table 25C** provides targeting domains for knocking out the *TRAC* gene using *S. aureus* Cas9 selected according to first tier parameters. The targeting domains were selected within the first 500bp of the coding sequence, had a high level of orthogonality, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with a targeting domain from Group B as shown in **Table 25-2.**

**Table 25-2**

| Group A | Group B |
|---|---|
| GUUUUGUCUGUGAUAUACACAU (SEQ ID NO:49440) | UCUCAAACAAAUGUGUCACAAAGU (SEQ ID NO:49489) |

**Table 25C**

| 1st Tier |
|---|
| Targeting domains for knocking out the *TRAC* gene using *S. aureus* Cas9 selected according to first tier parameters are presented in SEQ ID NO:49416-49496. |

**Table 25D** provides targeting domains for knocking out the *TRAC* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 25D**

| 2nd Tier |
|---|
| Targeting domains for knocking out the *TRAC* gene using *S. aureus* Cas9 selected according to second tier parameters are presented in SEQ ID NO:49497-49555. |

**Table 25E** provides targeting domains for knocking out the *TRAC* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 25E**

| 3rd Tier |
|---|
| Targeting domains for knocking out the *TRAC* gene using *S. aureus* Cas9 selected according to third tier parameters are presented in SEQ ID NO:49556-49926. |

**Table 25F** provides targeting domains for knocking out the *TRAC* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 25F**

| 1st Tier |
|---|
| Targeting domains for knocking out the *TRAC* gene using *N. meningitidismeningitidis* Cas9 selected according to first tier parameters are presented in SEQ ID NO:49927-49949. |

**Table 25G** provides targeting domains for knocking out the *TRAC* gene using *N. meningitidis* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 25G**

| 2nd Tier |
|---|
| A targeting domains for knocking out the *TRAC* gene using *N. meningitidismeningitidis* Cas9 selected according to second tier parameters is presented in SEQ ID NO:49950. |

**Table 26A** provides targeting domains for knocking out the *TRBC* gene using *S. pyogenes* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with a targeting domain from Group B as shown in **Table 26-1.**

**Table 26-1**

| Group A | Group B |
|---|---|
| AUGACGAGUGGACCCAGGAU (SEQ ID NO:49999) | GCGCUGACGAUCUGGGUGAC (SEQ ID NO:50160) |

**Table 26A**

| 1st Tier |
|---|
| Targeting domains for knocking out the *TRBC* gene using *S. pyogenes* Cas9 selected according to first tier parameters are presented in SEQ ID NO:49951-50002. |

**Table 26B** provides targeting domains for knocking out the *TRBC* gene using *S. pyogenes* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 26B**

| 2nd Tier |
|---|
| Targeting domains for knocking out the *TRBC* gene using *S. pyogenes* Cas9 selected according to second tier parameters are presented in SEQ ID NO:50003-50240. |

**Table 26C** provides targeting domains for knocking out the *TRBC* gene using *S. aureus* Cas9 selected according to first tier parameters. The targeting domains were selected within the first 500bp of the coding sequence, had a high level of orthogonality, and contained a NNGRRT PAM.. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp. In an embodiment, two gRNAs are used to target two Cas9 nucleases or two Cas9 nickases, e.g., a gRNA with a targeting domain from Group A can be paired with a gRNA with a targeting domain from Group B as shown in **Table 26-2.**

**Table 26-2**

| Group A | Group B |
|---|---|
| GUGAAUGGGAAGGAGGUGCACAG (SEQ ID NO:50401) | GGCGGGCUGCUCCUUGAGGGGCU (SEQ ID NO:50378) |

**Table 26C**

| 1st Tier |
|---|
| Targeting domains for knocking out the *TRBC* gene using *S. aureus* Cas9 selected according to first tier parameters are presented in SEQ ID NO:50241-50358. |

**Table 26D** provides targeting domains for knocking out the *TRBC* gene using *S. aureus* Cas9 selected according to second tier parameters. The targeting domains were selected within the first 500bp of the coding sequence, no level of orthogonality was required, and contained a NNGRRT PAM. It is contemplated herein that the targeting domain hybridizes to the target domain through complementary base pairing. Any of the targeting domains in the table can be used with a *S*. *aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 26D**

| 2nd Tier |
|---|
| Targeting domains for knocking out the *TRBC* gene using *S. aureus* Cas9 selected according to second tier parameters are presented in SEQ ID NO:50359-50429. |

**Table 26E** provides targeting domains for knocking out the *TRBC* gene using *S. aureus* Cas9 selected according to third tier parameters. The targeting domains were selected within the remainder of the coding sequence downstream and contained a NNGRRT PAM. It is contemplated herein that the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 26E**

| 3rd Tier |
|---|
| Targeting domains for knocking out the *TRBC* gene using *S. aureus* Cas9 selected according to third tier parameters are presented in SEQ ID NO:50430-51192. |

**Table 26F** provides targeting domains for knocking out the *TRBC* gene using *N. meningitidis* Cas9 selected according to first tier parameters. The targeting domains bind within the first 500bp of coding sequence downstream of start codon and have good orthogonality. It is contemplated herein that the targeting domain hybridizes to the target domain through complementary base pairing. Any of the targeting domains in the table can be used with a N. *meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 26F**

| 1st Tier |
|---|
| Targeting domains for knocking out the *TRBC* gene using *N. meningitidismeningitidis* Cas9 selected according to first tier parameters are presented in SEQ ID NO:51193-51198. |

**Table 26G** provides targeting domains for knocking out the *TRBC* gene using *N. meningitidis* Cas9 selected according to second tier parameters. The targeting domains bind within the first 500bp of coding sequence downstream of start codon and good orthogonality is not required. It is contemplated herein that the targeting domain hybridizes to the target domain through complementary base pairing. Any of the targeting domains in the table can be used with a *N. meningitidis* Cas9 molecule that generates a double stranded break (Cas9 nuclease) or a single-stranded break (Cas9 nickase). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired with any gRNA comprising a plus strand targeting domain provided that the two gRNAs are oriented on the DNA such that PAMs face outward and the distance between the 5' ends of the gRNAs is 0-50bp.

**Table 26G**

| 2nd Tier |
|---|
| Targeting domains for knocking out the *TRBC* gene using *N. meningitidismeningitidis* Cas9 selected according to second tier parameters are presented in SEQ ID NO:51199-51200. |

Targeting domains, disclosed herein, may comprise a **17-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 18 or more nucleotides may comprise the 17-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a **18-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 19 or more nucleotides may comprise the 18-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, Tables 25A-G, Tables 26A-G, Table 27, Table 29, Table 31, or Table 32.

Targeting domains, disclosed herein, may comprise a **19-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 20 or more nucleotides may comprise the 19-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a **20-mer** gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 21 or more nucleotides may comprise the 20-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a **21-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 22 or more nucleotides may comprise the 21-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a **22-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 23 or more nucleotides may comprise the 22-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a **23-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 24 or more nucleotides may comprise the 23-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

Targeting domains, disclosed herein, may comprise a **24-mer** described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32, e.g., the targeting domains of 25 or more nucleotides may comprise the 24-mer gRNAs described in Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.

### III. Cas9 Molecules

Cas9 molecules of a variety of species can be used in the methods and compositions described herein. While the *S. pyogenes, S. aureus, N. meningitidis,* and *S. thermophilus* Cas9 molecules are the subject of much of the disclosure herein, Cas9 molecules of, derived from, or based on the Cas9 proteins of other species listed herein can be used as well. In other words, while the much of the description herein uses *S. pyogenes, S. aureus, N. meningitidis,* and *S. thermophilus* Cas9 molecules, Cas9 molecules from the other species can replace them. Such species include: *Acidovorax avenae, Actinobacillus pleuropneumoniae, Actinobacillus succinogenes, Actinobacillus suis, Actinomyces sp*., *Cycliphilusdenitrificans, Aminomonas paucivorans, Bacillus cereus, Bacillus smithii, Bacillus thuringiensis, Bacteroides sp*., *Blastopirellula marina, Bradyrhizobium sp., Brevibacillus laterosp*or*us, Campylobacter coli, Campylobacter jejuni, Campylobacter lari, Candidatus puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, Gammaproteobacterium*, *Gluconacetobacter diazotrophicus, Haemophilus parainfluenzae*, *Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosp*or*ium, Mobiluncus mulieris, Neisseria bacillif*or*mis, Neisseria cinerea, Neisseria flavescens, Neisseria lactamica, Neisseria meningitidis, Neisseria sp., Neisseria wadsw*or*thii, Nitrosomonas sp*., *Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus aureus, Staphylococcus lugdunensis, Streptococcus sp., Subdoligranulum sp., Tistrella mobilis, Treponema sp.,* or *Verminephrobacter eiseniae.*

A Cas9 molecule, or Cas9 polypeptide, as that term is used herein, refers to a molecule or polypeptide that can interact with a gRNA molecule and, in concert with the gRNA molecule, homes or localizes to a site which comprises a target domain and PAM sequence. Cas9 molecule and Cas9 polypeptide, as those terms are used herein, refer to naturally occurring Cas9 molecules and to engineered, altered, or modified Cas9 molecules or Cas9 polypeptides that differ, e.g., by at least one amino acid residue, from a reference sequence, e.g., the most similar naturally occurring Cas9 molecule or a sequence of **Table 100.**

### Cas9 Domains

Crystal structures have been determined for two different naturally occurring bacterial Cas9 molecules (Jinek et al., Science, 343(6176):1247997, 2014) and for *S. pyogenes* Cas9 with a guide RNA (e.g., a synthetic fusion of crRNA and tracrRNA) (Nishimasu et al., Cell, 156:935-949, 2014; and Anders et al., Nature, 2014, doi: 10.1038/nature13579).

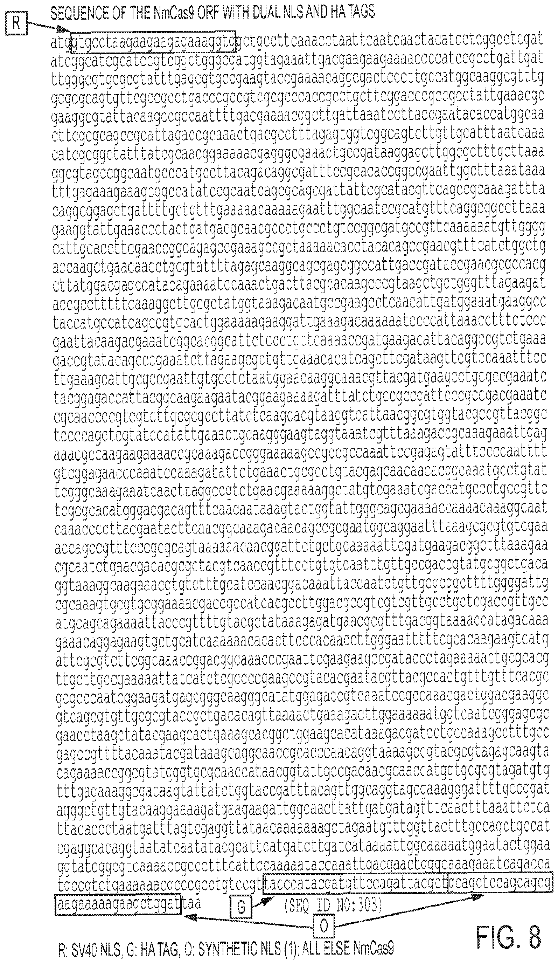

A naturally occurring Cas9 molecule comprises two lobes: a recognition (REC) lobe and a nuclease (NUC) lobe; each of which further comprises domains described herein. **Figs. 8A-8B** provide a schematic of the organization of important Cas9 domains in the primary structure. The domain nomenclature and the numbering of the amino acid residues encompassed by each domain used throughout this disclosure is as described in Nishimasu et al. The numbering of the amino acid residues is with reference to Cas9 from *S. pyogenes.*

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long α-helix and arginine rich region and comprises amino acids 60-93 of the sequence *of S. pyogenes* Cas9. The REC1 domain is important for recognition of the repeat: anti-repeat duplex, e.g., of a gRNA or a tracrRNA, and is therefore critical for Cas9 activity by recognizing the target sequence. The REC1 domain comprises two REC1 motifs at amino acids 94 to 179 and 308 to 717 of the sequence *of S. pyogenes* Cas9. These two REC1 domains, though separated by the REC2 domain in the linear primary structure, assemble in the tertiary structure to form the REC 1 domain. The REC2 domain, or parts thereof, may also play a role in the recognition of the repeat:anti-repeat duplex. The REC2 domain comprises amino acids 180-307 of the sequence of *S*. *pyogenes* Cas9.

The NUC lobe comprises the RuvC domain (also referred to herein as RuvC-like domain), the HNH domain (also referred to herein as HNH-like domain), and the PAM-interacting (PI) domain. The RuvC domain shares structural similarity to retroviral integrase superfamily members and cleaves a single strand, e.g., the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC 1 domain, the three RuvC motifs are linearly separated by other domains in the primary structure, however in the tertiary structure, the three RuvC motifs assemble and form the RuvC domain. The HNH domain shares structural similarity with HNH endonucleases, and cleaves a single strand, e.g., the complementary strand of the target nucleic acid molecule. The HNH domain lies between the RuvC II-III motifs and comprises amino acids 775-908 of the sequence *of S. pyogenes* Cas9. The PI domain interacts with the PAM of the target nucleic acid molecule, and comprises amino acids 1099-1368 of the sequence of *S. pyogenes* Cas9.

### A RuvC-like domain and an HNH-like domain

In an embodiment, a Cas9 molecule or Cas9 polypeptide comprises an HNH-like domain and a RuvC-like domain. In an embodiment, cleavage activity is dependent on a RuvC-like domain and an HNH-like domain. A Cas9 molecule or Cas9 polypeptide, e.g., an eaCas9 molecule or eaCas9 polypeptide, can comprise one or more of the following domains: a RuvC-like domain and an HNH-like domain. In an embodiment, a Cas9 molecule or Cas9 polypeptide is an eaCas9 molecule or eaCas9 polypeptide and the eaCas9 molecule or eaCas9 polypeptide comprises a RuvC-like domain, e.g., a RuvC-like domain described below, and/or an HNH-like domain, e.g., an HNH-like domain described below.

### RuvC-like domains

In an embodiment, a RuvC-like domain cleaves, a single strand, e.g., the non-complementary strand of the target nucleic acid molecule. The Cas9 molecule or Cas9 polypeptide can include more than one RuvC-like domain (e.g., one, two, three or more RuvC-like domains). In an embodiment, a RuvC-like domain is at least 5, 6, 7, 8 amino acids in length but not more than 20, 19, 18, 17, 16 or 15 amino acids in length. In an embodiment, the Cas9 molecule or Cas9 polypeptide comprises an N-terminal RuvC-like domain of about 10 to 20 amino acids, e.g., about 15 amino acids in length.

### N-terminal RuvC-like domains

Some naturally occurring Cas9 molecules comprise more than one RuvC-like domain with cleavage being dependent on the N-terminal RuvC-like domain. Accordingly, Cas9 molecules or Cas9 polypeptide can comprise an N-terminal RuvC-like domain. Exemplary N-terminal RuvC-like domains are described below.

In an embodiment, an eaCas9 molecule or eaCas9 polypeptide comprises an N-terminal RuvC-like domain comprising an amino acid sequence of formula I:
D-X1-G-X2-X3-X4-X5-G-X6-X7-X8-X9 (SEQ ID NO:8),
wherein,
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X4 is selected from S, Y, N and F (e.g., S);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent, designated by L1 (e.g., selected from T, V, I, L, Δ, F, S, A, Y, M and R, or, e.g., selected from T, V, I, L and Δ).

In an embodiment, the N-terminal RuvC-like domain differs from a sequence of SEQ TD NO:8, by as many as 1 but no more than 2, 3, 4, or 5 residues.

In embodiment, the N-terminal RuvC-like domain is cleavage competent.

In embodiment, the N-terminal RuvC-like domain is cleavage incompetent.

In an embodiment, a eaCas9 molecule or eaCas9 polypeptide comprises an N-terminal RuvC-like domain comprising an amino acid sequence of formula II:
D-X1-G-X2-X3-S-X5-G-X6-X7-X8-X9 (SEQ ID NO:9),
wherein
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:9 by as many as 1 but no more than 2, 3, 4, or 5 residues.

In an embodiment, the N-terminal RuvC-like domain comprises an amino acid sequence of formula III:
D-1-G-X2-X3-S-V-G-W-A-X8-X9 (SEQ ID NO:10),
wherein
X2 is selected from T, I, V, S, N, Y, E and L (e.g., selected from T, V, and I);
X3 is selected from N, S, G, A, D, T, R, M and F (e.g., A or N);
   X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:10 by as many as 1 but no more than, 2, 3, 4, or 5 residues.

In an embodiment, the N-terminal RuvC-like domain comprises an amino acid sequence of formula III:
D-1-G-T-N-S-V-G-W-A-V-X (SEQ ID NO:11),
wherein
X is a non-polar alkyl amino acid or a hydroxyl amino acid, e.g., X is selected from V, T, L and T (e.g., the eaCas9 molecule can comprise an N-terminal RuvC-like domain shown in **Figs. 2A-2G** (is depicted as Y)).

In an embodiment, the N-terminal RuvC-like domain differs from a sequence of SEQ ID NO:11 by as many as 1 but no more than, 2, 3, 4, or 5 residues.

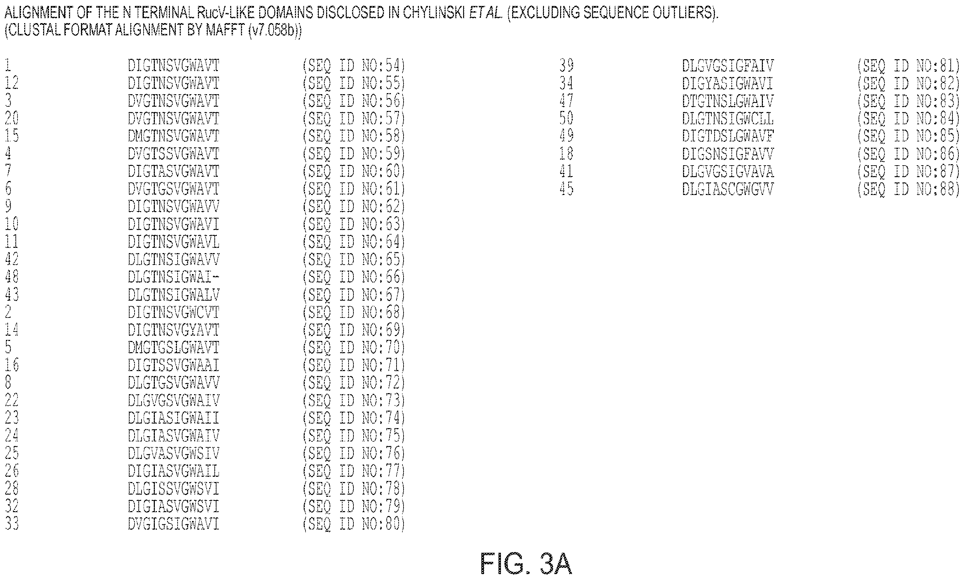

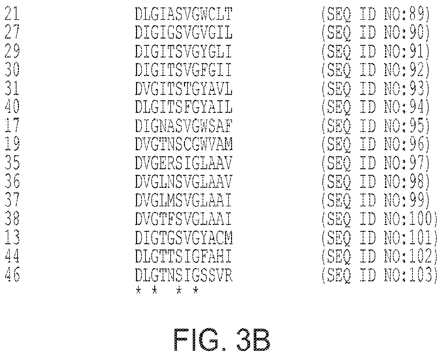

In an embodiment, the N-terminal RuvC-like domain differs from a sequence of an N-terminal RuvC like domain disclosed herein, e.g., in **Figs. 3A-3B** or **Figs. 7A-7B****,** as many as 1 but no more than 2, 3, 4, or 5 residues. In an embodiment, 1, 2, or all 3 of the highly conserved residues identified **in** **Figs. 3A-3B** or **Figs. 7A-7B** are present.

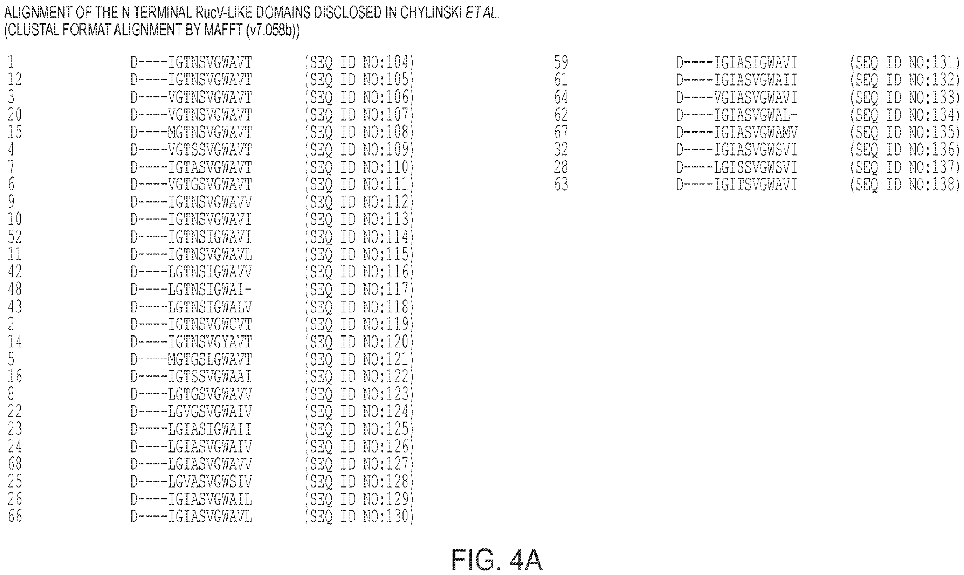

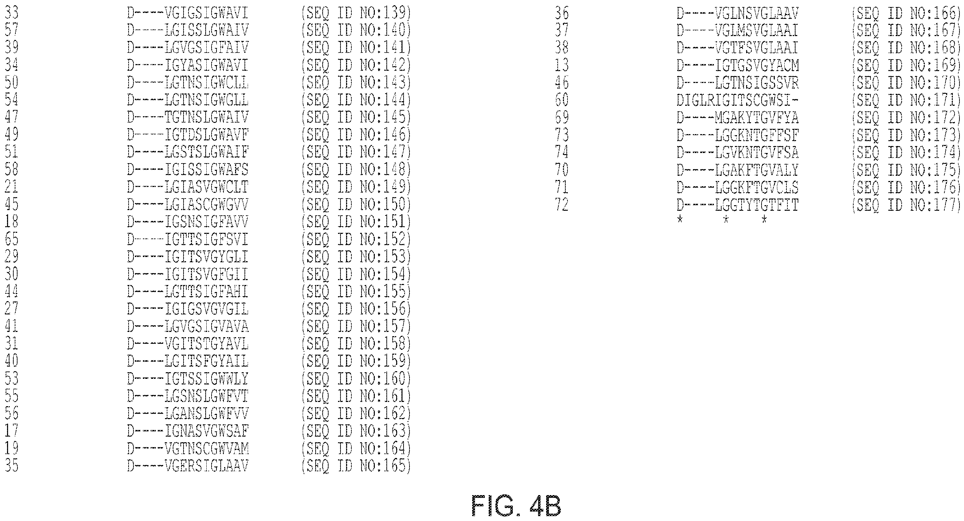

In an embodiment, the N-terminal RuvC-like domain differs from a sequence of an N-terminal RuvC-like domain disclosed herein, e.g., in **Figs. 4A-4B** or **Figs. 7A-7B****,** as many as 1 but no more than 2, 3, 4, or 5 residues. In an embodiment, 1, 2, 3 or all 4 of the highly conserved residues identified in **Figs. 4A-4B** or **Figs. 7A-7B** are present.

### Additional RuvC-like domains

In addition to the N-terminal RuvC-like domain, the Cas9 molecule or Cas9 polypeptide, e.g., an eaCas9 molecule or eaCas9 polypeptide, can comprise one or more additional RuvC-like domains. In an embodiment, the Cas9 molecule or Cas9 polypeptide can comprise two additional RuvC-like domains. Preferably, the additional RuvC-like domain is at least 5 amino acids in length and, e.g., less than 15 amino acids in length, e.g., 5 to 10 amino acids in length, e.g., 8 amino acids in length.

An additional RuvC-like domain can comprise an amino acid sequence:
I-X1-X2-E-X3-A-R-E (SEQ ID NO:12),
wherein
X1 is V or H,
X2 is I, L or V (e.g., I or V); and
X3 is M or T.

In an embodiment, the additional RuvC-like domain comprises the amino acid sequence:
I-V-X2-E-M-A-R-E (SEQ ID NO:13),
wherein
X2 is I, L or V (e.g., I or V) (e.g., the eaCas9 molecule or eaCas9 polypeptide can comprise an additional RuvC-like domain shown in **Figs. 2A-2G** or **Figs. 7A-7B** (depicted as B)).

An additional RuvC-like domain can comprise an amino acid sequence:
H-H-A-X1-D-A-X2-X3 (SEQ ID NO:14),
wherein
X1 isH orL;
X2 is R or V; and
X3 is E or V.

In an embodiment, the additional RuvC-like domain comprises the amino acid sequence: H-H-AH-D-A-Y-L (SEQ ID NO:15).

In an embodiment, the additional RuvC-like domain differs from a sequence of SEQ ID NO:12, 13, 14 or 15 by as many as 1 but no more than 2, 3, 4, or 5 residues.

In some embodiments, the sequence flanking the N-terminal RuvC-like domain is a sequence of formula V:
K-X1'-Y-X2'-X3'-X4'-Z-T-D-X9'-Y (SEQ ID NO:16),
wherein
X1' is selected from K and P,
X2' is selected from V, L, I, and F (e.g., V, I and L);
X3' is selected from G, A and S (e.g., G),
X4' is selected from L, I, V and F (e.g., L);
X9' is selected from D, E, N and Q; and
Z is an N-terminal RuvC-like domain, e.g., as described above.

### HNH-like domains

In an embodiment, an HNH-like domain cleaves a single stranded complementary domain, e.g., a complementary strand of a double stranded nucleic acid molecule. In an embodiment, an HNH-like domain is at least 15, 20, 25 amino acids in length but not more than 40, 35 or 30 amino acids in length, e.g., 20 to 35 amino acids in length, e.g., 25 to 30 amino acids in length. Exemplary HNH-like domains are described below.

In an embodiment, an eaCas9 molecule or eaCas9 polypeptide comprises an HNH-like domain having an amino acid sequence of formula VI:
X1-X2-X3-H-X4-X5-P-X6-X7-X8-X9-X10-X11-X12-X13-X14-X15-N-X16-X17-X18-X19-X20-X21-X22-X23-N (SEQ ID NO:17),
wherein
X1 is selected from D, E, Q and N (e.g., D and E);
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X7 is selected from S, A, D, T and K (e.g., S and A);
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X11 is selected from D, S, N, R, L and T (e.g., D);
X12 is selected from D, N and S;
X13 is selected from S, A, T, G and R (e.g., S);
X14 is selected from I, L, F, S, R, Y, Q, W, D, K and H (e.g., I, L and F);
X15 is selected from D, S, I, N, E, A, H, F, L, Q, M, G, Y and V;
X16 is selected from K, L, R, M, T and F (e.g., L, R and K);
X17 is selected from V, L, I, A and T;
X18 is selected from L, I, V and A (e.g., L and I);
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, a HNH-like domain differs from a sequence of SEQ ID NO:17 by at least one but no more than, 2, 3, 4, or 5 residues.

In an embodiment, the HNH-like domain is cleavage competent.

In an embodiment, the HNH-like domain is cleavage incompetent.

In an embodiment, an eaCas9 molecule or eaCas9 polypeptide comprises an HNH-like domain comprising an amino acid sequence of formula VII:
X1-X2-X3-H-X4-X5-P-X6-S-X8-X9-X10-D-D-S-X14-X15-N-K-V-L-X19-X20-X21-X22-X23-N (SEQ ID NO:18),
wherein
X1 is selected from D and E;
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO:18 by 1, 2, 3, 4, or 5 residues.

In an embodiment, an eaCas9 molecule or eaCas9 polypeptide comprises an HNH-like domain comprising an amino acid sequence of formula VII:
X1-V-X3-H-I-V-P-X6-S-X8-X9-X10-D-D-S-X14-X15-N-K-V-L-T-X20-X21-X22-X23-N (SEQ ID NO:19),
wherein
X1 is selected from D and E;
X3 is selected from D and E;
X6 is selected from Q, H, R, K, Y, I, L and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO:19 by 1, 2, 3, 4, or 5 residues.

In an embodiment, an eaCas9 molecule or eaCas9 polypeptide comprises an HNH-like domain having an amino acid sequence of formula VIII:
D-X2-D-H-I-X5-P-Q-X7-F-X9-X10-D-X12-S-I-D-N-X16-V-L-X19-X20-S-X22-X23-N (SEQ ID NO:20),
wherein
X2 is selected from I and V;
X5 is selected from I and V;
X7 is selected from A and S;
X9 is selected from I and L;
X10 is selected from K and T;
X12 is selected from D and N;
X16 is selected from R, K and L; X19 is selected from T and V;
X20 is selected from S and R;
X22 is selected from K, D and A; and
X23 is selected from E, K, G and N (e.g., the eaCas9 molecule or eaCas9 polypeptide can comprise an HNH-like domain as described herein).

In an embodiment, the HNH-like domain differs from a sequence of SEQ ID NO:20 by as many as 1 but no more than 2, 3, 4, or 5 residues.

In an embodiment, an eaCas9 molecule or eaCas9 polypeptide comprises the amino acid sequence of formula IX:
L-Y-Y-L-Q-N-G-X1'-D-M-Y-X2'-X3'-X4'-X5'-L-D-I-X6'-X7'-L-S-X8'-Y-Z-N-R-X9'-K-X10'-D-X11'-V-P (SEQ ID NO:21),
wherein
X1' is selected from K and R;
X2' is selected from V and T;
X3' is selected from G and D;
X4' is selected from E, Q and D;
X5' is selected from E and D;
X6' is selected from D, N and H;
X7' is selected from Y, R and N;
X8' is selected from Q, D and N; X9' is selected from G and E;
X10' is selected from S and G;
X11' is selected from D and N; and
Z is an HNH-like domain, e.g., as described above.

In an embodiment, the eaCas9 molecule or eaCas9 polypeptide comprises an amino acid sequence that differs from a sequence of SEQ ID NO:21 by as many as 1 but no more than 2, 3, 4, or 5 residues.

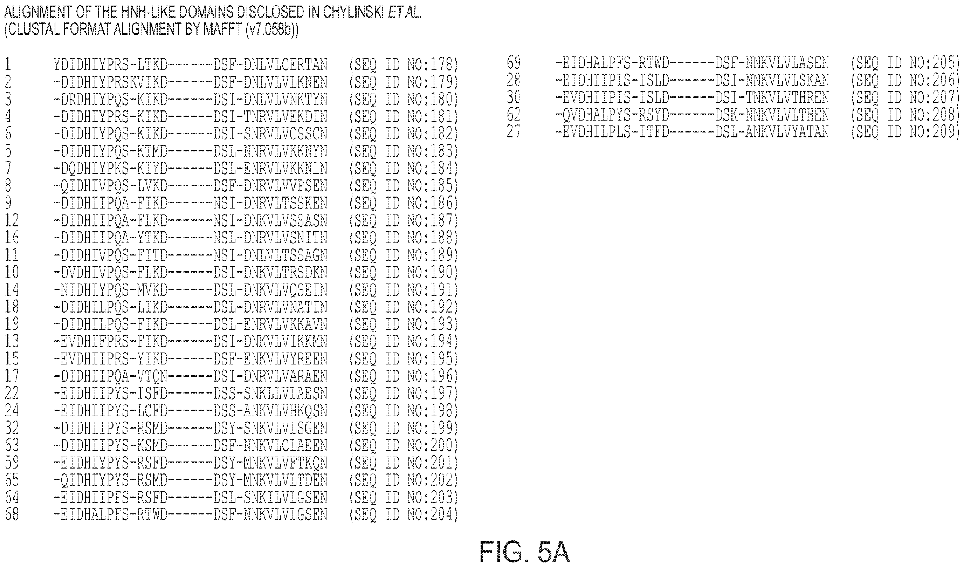

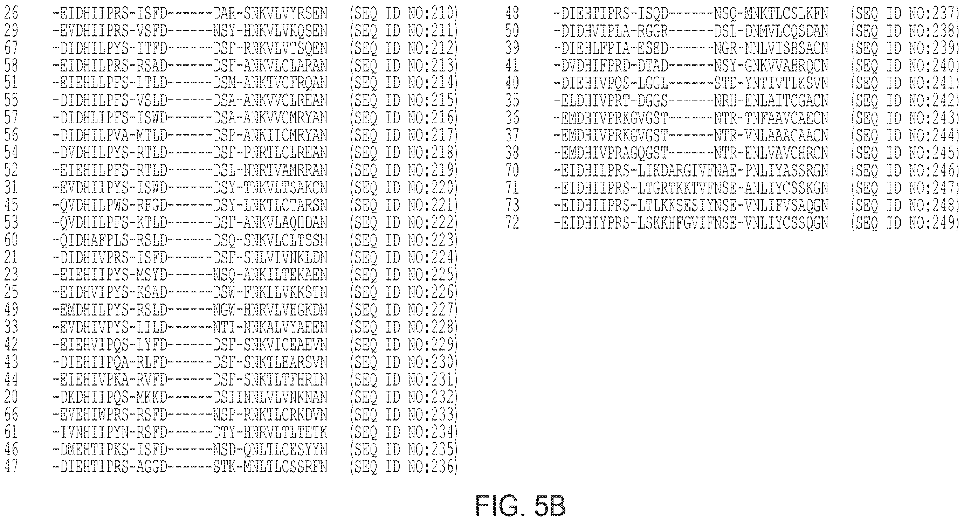

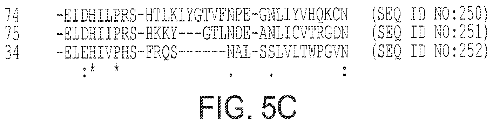

In an embodiment, the HNH-like domain differs from a sequence of an HNH-like domain disclosed herein, e.g., in **Figs. 5A-5C** or **Figs. 7A-7B**, as many as 1 but no more than 2, 3, 4, or 5 residues. In an embodiment, 1 or both of the highly conserved residues identified in **Figs. 5A-5C** or **Figs. 7A-7B** are present.

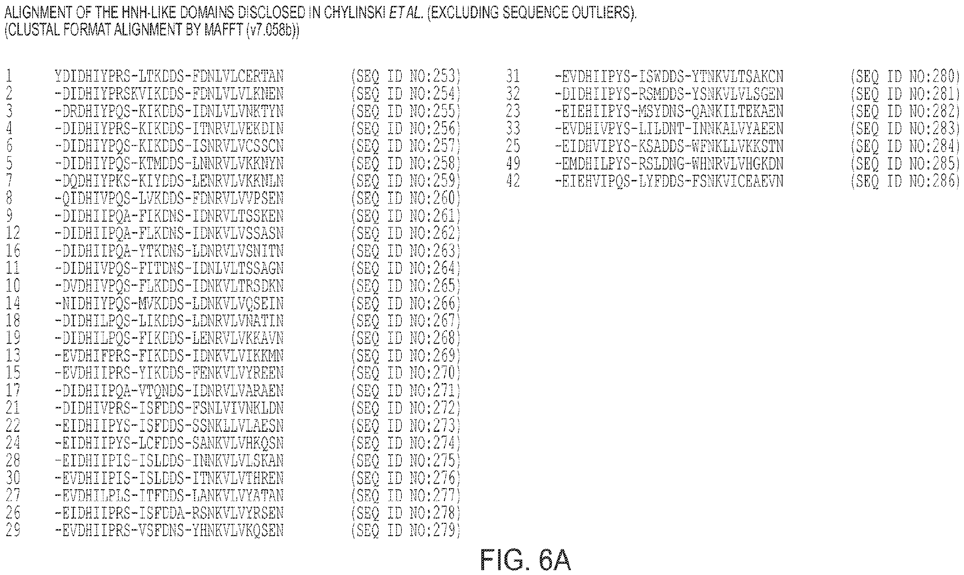

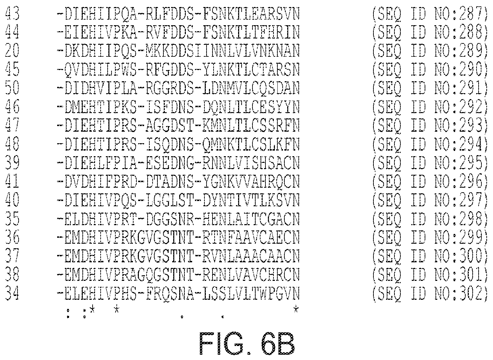

In an embodiment, the HNH -like domain differs from a sequence of an HNH-like domain disclosed herein, e.g., in **Figs. 6A-6B** or **Figs. 7A-7B**, as many as 1 but no more than 2, 3, 4, or 5 residues. In an embodiment, 1, 2, all 3 of the highly conserved residues identified in **Figs. 6A-6B** or **Figs. 7A-7B** are present.

### Cas9 Activities

### Nuclease and Helicase Activities

In an embodiment, the Cas9 molecule or Cas9 polypeptide is capable of cleaving a target nucleic acid molecule. Typically wild type Cas9 molecules cleave both strands of a target nucleic acid molecule. Cas9 molecules and Cas9 polypeptides can be engineered to alter nuclease cleavage (or other properties), e.g., to provide a Cas9 molecule or Cas9 peolypeptide which is a nickase, or which lacks the ability to cleave target nucleic acid. A Cas9 molecule or Cas9 polypeptide that is capable of cleaving a target nucleic acid molecule is referred to herein as an eaCas9 molecule or eaCas9 polypeptide

In an embodiment, an eaCas9 molecule or eaCas9 polypeptide comprises one or more of the following activities:
a nickase activity, i.e., the ability to cleave a single strand, e.g., the non-complementary strand or the complementary strand, of a nucleic acid molecule;
a double stranded nuclease activity, i.e., the ability to cleave both strands of a double stranded nucleic acid and create a double stranded break, which in an embodiment is the presence of two nickase activities;
an endonuclease activity;
an exonuclease activity; and
a helicase activity, i.e., the ability to unwind the helical structure of a double stranded nucleic acid.

In an embodiment, an enzymatically active or eaCas9 molecule or eaCas9 polypeptide cleaves both strands and results in a double stranded break. In an embodiment, an eaCas9 molecule cleaves only one strand, e.g., the strand to which the gRNA hybridizes to, or the strand complementary to the strand the gRNA hybridizes with. In an embodiment, an eaCas9 molecule or eaCas9 polypeptide comprises cleavage activity associated with an HNH-like domain. In an embodiment, an eaCas9 molecule or eaCas9 polypeptide comprises cleavage activity associated with an N-terminal RuvC-like domain. In an embodiment, an eaCas9 molecule or eaCas9 polypeptide comprises cleavage activity associated with an HNH-like domain and cleavage activity associated with an N-terminal RuvC-like domain. In an embodiment, an eaCas9 molecule or eaCas9 polypeptide comprises an active, or cleavage competent, HNH-like domain and an inactive, or cleavage incompetent, N-terminal RuvC-like domain. In an embodiment, an eaCas9 molecule or eaCas9 polypeptide comprises an inactive, or cleavage incompetent, HNH-like domain and an active, or cleavage competent, N-terminal RuvC-like domain.

Some Cas9 molecules or Cas9 polypeptides have the ability to interact with a gRNA molecule, and in conjunction with the gRNA molecule localize to a core target domain, but are incapable of cleaving the target nucleic acid, or incapable of cleaving at efficient rates. Cas9 molecules having no, or no substantial, cleavage activity are referred to herein as an eiCas9 molecule or eiCas9 polypeptide. For example, an eiCas9 molecule or eiCas9 polypeptide can lack cleavage activity or have substantially less, e.g., less than 20, 10, 5, 1 or 0.1 % of the cleavage activity of a reference Cas9 molecule or eiCas9 polypeptide, as measured by an assay described herein.

### Targeting and PAMs

A Cas9 molecule or Cas9 polypeptide, is a polypeptide that can interact with a guide RNA (gRNA) molecule and, in concert with the gRNA molecule, localizes to a site which comprises a target domain and a PAM sequence.

In an embodiment, the ability of an eaCas9 molecule or eaCas9 polypeptide to interact with and cleave a target nucleic acid is PAM sequence dependent. A PAM sequence is a sequence in the target nucleic acid. In an embodiment, cleavage of the target nucleic acid occurs upstream from the PAM sequence. EaCas9 molecules from different bacterial species can recognize different sequence motifs (e.g., PAM sequences). In an embodiment, an eaCas9 molecule *of S. pyogenes* recognizes the sequence motif NGG, NAG, NGA and directs cleavage of a target nucleic acid sequence 1 to 10, e.g., 3 to 5, base pairs upstream from that sequence. See, e.g., Mali et al., SCIENCE 2013; 339(6121): 823-826. In an embodiment, an eaCas9 molecule of *S. thermophilus* recognizes the sequence motif NGGNG and/or NNAGAAW (W = A or T) and directs cleavage of a target nucleic acid sequence 1 to 10, e.g., 3 to 5, base pairs upstream from these sequences. See, e.g., Horvath et al., SCIENCE 2010; 327(5962):167-170, and Deveau et al., J BACTERIOL 2008; 190(4): 1390-1400. In an embodiment, an eaCas9 molecule of *S. mutans* recognizes the sequence motif NGG and/or NAAR (R = A or G)) and directs cleavage of a core target nucleic acid sequence 1 to 10, e.g., 3 to 5 base pairs, upstream from this sequence. See, e.g., Deveau et al., J BACTERIOL 2008; 190(4): 1390-1400. In an embodiment, an eaCas9 molecule of *S. aureus* recognizes the sequence motif NNGRR (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRT (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRV (R = A or G) and directs cleavage of a target nucleic acid sequence 1 to 10, e.g., 3 to 5, base pairs upstream from that sequence. In an embodiment, an eaCas9 molecule of *N. meningitidis* recognizes the sequence motif NNNNGATT or NNNGCTT (R = A or G, V = A, G or C and directs cleavage of a target nucleic acid sequence 1 to 10, e.g., 3 to 5, base pairs upstream from that sequence. See, e.g., Hou et al., PNAS EARLY EDITION 2013, 1-6. The ability of a Cas9 molecule to recognize a PAM sequence can be determined, e.g., using a transformation assay described in Jinek et al., SCIENCE 2012 337:816. In the aforementioned embodiments, N can be any nucleotide residue, e.g., any of A, G, C or T.

As is discussed herein, Cas9 molecules can be engineered to alter the PAM specificity of the Cas9 molecule.

Exemplary naturally occurring Cas9 molecules are described in Chylinski et al., RNA Biology 2013 10:5, 727-737. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. pyogenes* (e.g., strain SF370, MGAS10270, MGAS10750, MGAS2096, MGAS315, MGAS5005, MGAS6180, MGAS9429, NZ131 and SSI-1), *S. thermophilus* (e.g., strain LMD-9), *S. pseudoporcinus* (e.g., strain SPIN 20026), *S. mutans* (e.g., strain UA159, NN2025), *S. macacae* (e.g., strain NCTC11558), *S. gallolyticus* (e.g., strain UCN34, ATCC BAA-2069), *S. equines* (e.g., strain ATCC 9812, MGCS 124), *S. dysdalactiae* (e.g., strain GGS 124), *S. bovis* (e.g., strain ATCC 700338), *S. anginosus* (e.g., strain F0211), *S. agalactiae* (e.g., strain NEM316, A909), *Listeria monocytogenes* (e.g., strain F6854), *Listeria innocua* (*L. innocua*, e.g., strain Clip11262), *Enterococcus italicus* (e.g., strain DSM 15952), or *Enterococcus faecium* (e.g., strain 1,231,408). Another exemplary Cas9 molecule is a Cas9 molecule of *Neisseria meningitidis* (Hou et al., PNAS Early Edition 2013, 1-6).

In an embodiment, a Cas9 molecule or Cas9 polypeptide, e.g., an eaCas9 molecule or eaCas9 polypeptide, comprises an amino acid sequence:
having 60%, 65%, 70%, 75%, 80%, 85%, 90%, 95%, 96%, 97%, 98% or 99% homology with;
differs at no more than, 2, 5, 10, 15, 20, 30, or 40% of the amino acid residues when compared with;
differs by at least 1, 2, 5, 10 or 20 amino acids but by no more than 100, 80, 70, 60, 50, 40 or 30 amino acids from; or
is identical to any Cas9 molecule sequence described herein, or a naturally occurring Cas9 molecule sequence, e.g., a Cas9 molecule from a species listed herein or described in Chylinski et al., RNA Biology 2013 10:5, 727-737; Hou et al., PNAS Early Edition 2013, 1-6; SEQ ID NOS:1-4. In an embodiment, the Cas9 molecule or Cas9 polypeptide comprises one or more of the following activities: a nickase activity; a double stranded cleavage activity (e.g., an endonuclease and/or exonuclease activity); a helicase activity; or the ability, together with a gRNA molecule, to home to a target nucleic acid.

In an embodiment, a Cas9 molecule or Cas9 polypeptide comprises the amino acid sequence of the consensus sequence of **Figs. 2A-2G**, wherein "*" indicates any amino acid found in the corresponding position in the amino acid sequence of a Cas9 molecule of *S. pyogenes, S. thermophilus, S. mutans and L. innocua*, and "-" indicates any amino acid. In an embodiment, a Cas9 molecule or Cas9 polypeptide differs from the sequence of the consensus sequence disclosed in **Figs. 2A-2G** by at least 1, but no more than 2, 3, 4, 5, 6, 7, 8, 9, or 10 amino acid residues. In an embodiment, a Cas9 molecule or Cas9 polypeptide comprises the amino acid sequence of SEQ ID NO:7 of **Figs. 7A-7B**, wherein "*" indicates any amino acid found in the corresponding position in the amino acid sequence of a Cas9 molecule of *S*. *pyogenes*, or *N. meningitidis,* "-" indicates any amino acid, and "-" indicates any amino acid or absent. In an embodiment, a Cas9 molecule or Cas9 polypeptide differs from the sequence of SEQ ID NO:6 or 7 disclosed in **Figs. 7A-7B** by at least 1, but no more than 2, 3, 4, 5, 6, 7, 8, 9, or 10 amino acid residues.

A comparison of the sequence of a number of Cas9 molecules indicate that certain regions are conserved. These are identified below as:
region 1 (residues 1 to 180, or in the case of region 1'residues 120 to 180)
region 2 (residues360 to 480);
region 3 (residues 660 to 720);
region 4 (residues 817 to 900); and
region 5 (residues 900 to 960);

In an embodiment, a Cas9 molecule or Cas9 polypeptide comprises regions 1-5, together with sufficient additional Cas9 molecule sequence to provide a biologically active molecule, e.g., a Cas9 molecule having at least one activity described herein. In an embodiment, each of regions 1-6, independently, have, 50%, 60%, 70%, or 80% homology with the corresponding residues of a Cas9 molecule or Cas9 polypeptide described herein, e.g., a sequence from **Figs. 2A-2G** or from **Figs. 7A-7B**.

In an embodiment, a Cas9 molecule or Cas9 polypeptide, e.g., an eaCas9 molecule or eaCas9 polypeptide, comprises an amino acid sequence referred to as region 1:
having 50%, 60%, 70%, 80%, 85%, 90%, 95%, 96%, 97%, 98% or 99% homology with amino acids 1-180 (the numbering is according to the motif sequence in **Figs. 2A-2G**; 52% of residues in the four Cas9 sequences in **Figs. 2A-2G** are conserved) of the amino acid sequence of Cas9 of *S. pyogenes;*
differs by at least 1, 2, 5, 10 or 20 amino acids but by no more than 90, 80, 70, 60, 50, 40 or 30 amino acids from amino acids 1-180 of the amino acid sequence of Cas9 of *S. pyogenes, S. thermophilus, S. mutans or L. innocua;* or
is identical to 1-180 of the amino acid sequence of Cas9 of *S. pyogenes*, *S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 1':
having 55%, 60%, 65%, 70%, 75%, 80%, 85%, 90%, 95%, 96%, 97%, 98% or 99% homology with amino acids 120-180 (55% of residues in the four Cas9 sequences in **Figs. 2A-2G** are conserved) of the amino acid sequence of Cas9 of *S. pyogenes, S. thermophilus, S. mutans or L. innocua;*
differs by at least 1, 2, or 5 amino acids but by no more than 35, 30, 25, 20 or 10 amino acids from amino acids 120-180 of the amino acid sequence of Cas9 of *S. pyogenes*, *S. thermophilus, S. mutans* or *L. innocua*; or
is identical to 120-180 of the amino acid sequence of Cas9 of *S. pyogenes*, *S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 2:
having 50%, 55%, 60%, 65%, 70%, 75%, 80%, 85%, 90%, 95%, 96%, 97%, 98% or 99% homology with amino acids 360-480 (52% of residues in the four Cas9 sequences in **Figs. 2A-2G** are conserved) of the amino acid sequence of Cas9 of *S. pyogenes, S. thermophilus, S. mutans or L. innocua;*
differs by at least 1, 2, or 5 amino acids but by no more than 35, 30, 25, 20 or 10 amino acids from amino acids 360-480 of the amino acid sequence of Cas9 of *S. pyogenes, S. thermophilus, S. mutans or L. innocua*; or
is identical to 360-480 of the amino acid sequence of Cas9 of *S. pyogenes, S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 3:
having 55%, 60%, 65%, 70%, 75%, 80%, 85%, 90%, 95%, 96%, 97%, 98%, or 99% homology with amino acids 660-720 (56% of residues in the four Cas9 sequences in **Figs. 2A-2G** are conserved) of the amino acid sequence of Cas9 *of S. pyogenes,* S. thermophilus, S. mutans or L. innocua;
differs by at least 1, 2, or 5 amino acids but by no more than 35, 30, 25, 20 or 10 amino acids from amino acids 660-720 of the amino acid sequence of Cas9 of *S. pyogenes, S. thermophilus, S. mutans or L. innocua;* or
is identical to 660-720 of the amino acid sequence of Cas9 of *S. pyogenes, S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 4:
having 50%, 55%, 60%, 65%, 70%, 75%, 80%, 85%, 90%, 95%, 96%, 97%, 98%, or 99% homology with amino acids 817-900 (55% of residues in the four Cas9 sequences in **Figs. 2A-2G** are conserved) of the amino acid sequence of Cas9 of *S. pyogenes, S. thermophilus, S. mutans or L. innocua;*
differs by at least 1, 2, or 5 amino acids but by no more than 35, 30, 25, 20 or 10 amino acids from amino acids 817-900 of the amino acid sequence of Cas9 of *S. pyogenes*, *S. thermophilus, S. mutans or L. innocua;* or
is identical to 817-900 of the amino acid sequence of Cas9 of *S. pyogenes, S. thermophilus, S. mutans or L. innocua.*

In an embodiment, a Cas9 molecule or Cas9 polypeptide, e.g., an eaCas9 molecule or eaCas9 polypeptide, comprises an amino acid sequence referred to as region 5:
having 50%, 55%, 60%, 65%, 70%, 75%, 80%, 85%, 90%, 95%, 96%, 97%, 98%, or 99% homology with amino acids 900-960 (60% of residues in the four Cas9 sequences in **Figs. 2A-2G** are conserved) of the amino acid sequence of Cas9 of *S*. *pyogenes, S. thermophilus, S. mutans or L. innocua;*
differs by at least 1, 2, or 5 amino acids but by no more than 35, 30, 25, 20 or 10 amino acids from amino acids 900-960 of the amino acid sequence of Cas9 of *S. pyogenes, S. thermophilus, S. mutans or L. innocua;* or
is identical to 900-960 of the amino acid sequence of Cas9 of *S. pyogenes, S. thermophilus, S. mutans or L. innocua.*

### Engineered or Altered Cas9 Molecules and Cas9 Polypeptides

Cas9 molecules and Cas9 polypeptides described herein, e.g., naturally occurring Cas9 molecules, can possess any of a number of properties, including: nickase activity, nuclease activity (e.g., endonuclease and/or exonuclease activity); helicase activity; the ability to associate functionally with a gRNA molecule; and the ability to target (or localize to) a site on a nucleic acid (e.g., PAM recognition and specificity). In an embodiment, a Cas9 molecule or Cas9 polypeptide can include all or a subset of these properties. In typical embodiments, a Cas9 molecule or Cas9 polypeptide has the ability to interact with a gRNA molecule and, in concert with the gRNA molecule, localize to a site in a nucleic acid. Other activities, e.g., PAM specificity, cleavage activity, or helicase activity can vary more widely in Cas9 molecules and Cas9 polypeptides.

Cas9 molecules include engineered Cas9 molecules and engineered Cas9 polypeptides (engineered, as used in this context, means merely that the Cas9 molecule or Cas9 polypeptide differs from a reference sequences, and implies no process or origin limitation). An engineered Cas9 molecule or Cas9 polypeptide can comprise altered enzymatic properties, e.g., altered nuclease activity, (as compared with a naturally occurring or other reference Cas9 molecule) or altered helicase activity. As discussed herein, an engineered Cas9 molecule or Cas9 polypeptide can have nickase activity (as opposed to double strand nuclease activity). In an embodiment an engineered Cas9 molecule or Cas9 polypeptide can have an alteration that alters its size, e.g., a deletion of amino acid sequence that reduces its size, e.g., without significant effect on one or more, or any Cas9 activity. In an embodiment, an engineered Cas9 molecule or Cas9 polypeptide can comprise an alteration that affects PAM recognition. E.g., an engineered Cas9 molecule can be altered to recognize a PAM sequence other than that recognized by the endogenous wild-type PI domain. In an embodiment a Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, e.g., by alteration of a parental, e.g., naturally occurring, Cas9 molecules or Cas9 polypeptides, to provide an altered Cas9 molecule or Cas9 polypeptide having a desired property. For example, one or more mutations or differences relative to a parental Cas9 molecule, e.g., a naturally occurring or engineered Cas9 molecule, can be introduced. Such mutations and differences comprise: substitutions (e.g., conservative substitutions or substitutions of non-essential amino acids); insertions; or deletions. In an embodiment, a Cas9 molecule or Cas9 polypeptide can comprises one or more mutations or differences, e.g., at least 1, 2, 3, 4, 5, 10, 15, 20, 30, 40 or 50 mutations but less than 200, 100, or 80 mutations relative to a reference, e.g., a parental, Cas9 molecule.

In an embodiment, a mutation or mutations do not have a substantial effect on a Cas9 activity, e.g. a Cas9 activity described herein. In an embodiment, a mutation or mutations have a substantial effect on a Cas9 activity, e.g. a Cas9 activity described herein.

### Non-Cleaving and Modified-Cleavage Cas9 Molecules and Cas9 Polypeptides

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S. pyogenes,* as follows: its ability to modulate, e.g., decreased or increased, cleavage of a double stranded nucleic acid (endonuclease and/or exonuclease activity), e.g., as compared to a naturally occurring Cas9 molecule (e.g., a Cas9 molecule of *S. pyogenes*); its ability to modulate, e.g., decreased or increased, cleavage of a single strand of a nucleic acid, e.g., a non-complementary strand of a nucleic acid molecule or a complementary strand of a nucleic acid molecule (nickase activity), e.g., as compared to a naturally occurring Cas9 molecule (e.g., a Cas9 molecule of *S. pyogenes*); or the ability to cleave a nucleic acid molecule, e.g., a double stranded or single stranded nucleic acid molecule, can be eliminated.

### Modified Cleavage eaCas9 Molecules and eaCas9 Polypeptides

In an embodiment, an eaCas9 molecule or eaCas9 polypeptide comprises one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

In an embodiment, an eaCas9 molecule or eaCas9 polypeptide comprises an active, or cleavage competent, HNH-like domain (e.g., an HNH-like domain described herein, e.g., SEQ ID NO:17, SEQ ID NO:18, SEQ ID NO:19, SEQ ID NO:20, or SEQ ID NO:21) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, e.g., an aspartic acid at position 9 of the consensus sequence disclosed in **Figs. 2A-2G** or an aspartic acid at position 10 of SEQ ID NO:7, e.g., can be substituted with an alanine. In an embodiment, the eaCas9 molecule or eaCas9 polypeptide differs from wild type in the N-terminal RuvC-like domain and does not cleave the target nucleic acid, or cleaves with significantly less efficiency, e.g., less than 20, 10, 5, 1 or .1 % of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule *of S. pyogenes,* or S. thermophilus. In an embodiment, the reference Cas9 molecule is the naturally occurring Cas9 molecule having the closest sequence identity or homology.

In an embodiment, an eaCas9 molecule or eaCas9 polypeptide comprises an inactive, or cleavage incompetent, HNH domain and an active, or cleavage competent, N-terminal RuvC-like domain (e.g., an N-terminal RuvC-like domain described herein, e.g., SEQ ID NO:8, SEQ ID NO:9, SEQ ID NO:10, SEQ ID NO:11, SEQ ID NO:12, SEQ ID NO:13, SEQ ID NO:14, SEQ ID NO:15, or SEQ ID NO:16). Exemplary inactive, or cleavage incompetent HNH-like domains can have a mutation at one or more of: a histidine in an HNH-like domain, e.g., a histidine shown at position 856 of **Figs. 2A-2G**, e.g., can be substituted with an alanine; and one or more asparagines in an HNH-like domain, e.g., an asparagine shown at position 870 of **Figs. 2A-2G** and/or at position 879 of **Figs. 2A-2G**, e.g., can be substituted with an alanine. In an embodiment, the eaCas9 differs from wild type in the HNH-like domain and does not cleave the target nucleic acid, or cleaves with significantly less efficiency, e.g., less than 20, 10, 5, 1 or 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes*, *or S. thermophilus.* In an embodiment, the reference Cas9 molecule is the naturally occurring Cas9 molecule having the closest sequence identity or homology.

In an embodiment, an eaCas9 molecule or eaCas9 polypeptide comprises an inactive, or cleavage incompetent, HNH domain and an active, or cleavage competent, N-terminal RuvC-like domain (e.g., an N-terminal RuvC-like domain described herein, e.g., SEQ ID NO:8, SEQ ID NO:9, SEQ ID NO:10, SEQ ID NO:11, SEQ ID NO:12, SEQ ID NO:13, SEQ ID NO:14, SEQ ID NO:15, or SEQ ID NO:16). Exemplary inactive, or cleavage incompetent HNH-like domains can have a mutation at one or more of: a histidine in an HNH-like domain, e.g., a histidine shown at position 856 of **Figs. 2A-2G**, e.g., can be substituted with an alanine; and one or more asparagines in an HNH-like domain, e.g., an asparagine shown at position 870 of **Figs. 2A-2G** and/or at position 879 of **Figs. 2A-2G**, e.g., can be substituted with an alanine. In an embodiment, the eaCas9 differs from wild type in the HNH-like domain and does not cleave the target nucleic acid, or cleaves with significantly less efficiency, e.g., less than 20, 10, 5, 1 or 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes, or S. thermophilus.* In an embodiment, the reference Cas9 molecule is the naturally occurring Cas9 molecule having the closest sequence identity or homology.

### Alterations in the Ability to Cleave One or Both Strands of a Target Nucleic Acid

In an embodiment, exemplary Cas9 activities comprise one or more of PAM specificity, cleavage activity, and helicase activity. A mutation(s) can be present, e.g., in: one or more RuvC-like domain, e.g., an N-terminal RuvC-like domain; an HNH-like domain; a region outside the RuvC-like domains and the HNH-like domain. In some embodiments, a mutation(s) is present in a RuvC-like domain, e.g., an N-terminal RuvC-like. In some embodiments, a mutation(s) is present in an HNH-like domain. In some embodiments, mutations are present in both a RuvC-like domain, e.g., an N-terminal RuvC-like domain, and an HNH-like domain.

Exemplary mutations that may be made in the RuvC domain or HNH domain with reference to the *S. pyogenes* sequence include: D10A, E762A, H840A, N854A, N863A and/or D986A.

In an embodiment, a Cas9 molecule or Cas9 polypeptide is an eiCas9 molecule or eiCas9 polypeptide comprising one or more differences in a RuvC domain and/or in an HNH domain as compared to a reference Cas9 molecule, and the eiCas9 molecule or eiCas9 polypeptide does not cleave a nucleic acid, or cleaves with significantly less efficiency than does wildype, e.g., when compared with wild type in a cleavage assay, e.g., as described herein, cuts with less than 50, 25, 10, or 1% of a reference Cas9 molecule, as measured by an assay described herein.

Whether or not a particular sequence, e.g., a substitution, may affect one or more activity, such as targeting activity, cleavage activity, etc, can be evaluated or predicted, e.g., by evaluating whether the mutation is conservative or by the method described in Section IV. In an embodiment, a "non-essential" amino acid residue, as used in the context of a Cas9 molecule, is a residue that can be altered from the wild-type sequence of a Cas9 molecule, e.g., a naturally occurring Cas9 molecule, e.g., an eaCas9 molecule, without abolishing or more preferably, without substantially altering a Cas9 activity (e.g., cleavage activity), whereas changing an "essential" amino acid residue results in a substantial loss of activity (e.g., cleavage activity).

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S aureus, S. pyogenes,* or *C. jejuni* as follows: its ability to modulate, e.g., decreased or increased, cleavage of a double stranded break (endonuclease and/or exonuclease activity), e.g., as compared to a naturally occurring Cas9 molecule (e.g., a Cas9 molecule of *S aureus, S. pyogenes,* or *C. jejuni)*; its ability to modulate, e.g., decreased or increased, cleavage of a single strand of a nucleic acid, e.g., a non-complementary strand of a nucleic acid molecule or a complementary strand of a nucleic acid molecule (nickase activity), e.g., as compared to a naturally occurring Cas9 molecule (e.g., a Cas9 molecule of *S aureus, S. pyogenes,* or *C. jejuni*); or the ability to cleave a nucleic acid molecule, e.g., a double stranded or single stranded nucleic acid molecule, can be eliminated.

In an embodiment, the altered Cas9 molecule or Cas9 polypeptide is an eaCas9 molecule or eaCas9 polypeptide comprising one or more of the following activities: cleavage activity associated with a RuvC domain; cleavage activity associated with an HNH domain; cleavage activity associated with an HNH domain and cleavage activity associated with a RuvC domain.

In an embodiment, the altered Cas9 molecule or Cas9 polypeptide is an eiCas9 molecule or eaCas9 polypeptide which does not cleave a nucleic acid molecule (either double stranded or single stranded nucleic acid molecules) or cleaves a nucleic acid molecule with significantly less efficiency, e.g., less than 20, 10, 5, 1 or 0.1% of the cleavage activity of a reference Cas9 molecule, e.g., as measured by an assay described herein. The reference Cas9 molecule can be a naturally occurring unmodified Cas9 molecule, e.g., a naturally occurring Cas9 molecule such as a Cas9 molecule of *S. pyogenes, S. thermophilus, S. aureus, C. jejuni* or *N. meningitidis.* In an embodiment, the reference Cas9 molecule is the naturally occurring Cas9 molecule having the closest sequence identity or homology. In an embodiment, the eiCas9 molecule or eiCas9 polypeptide lacks substantial cleavage activity associated with a RuvC domain and cleavage activity associated with an HNH domain.

In an embodiment, the altered Cas9 molecule or Cas9 polypeptide is an eaCas9 molecule or eaCas9 polypeptide comprising the fixed amino acid residues of *S. pyogenes* shown in the consensus sequence disclosed in **Figs. 2A-2G****,** and has one or more amino acids that differ from the amino acid sequence of *S. pyogenes* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G** or SEQ ID NO:7.

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S*. *pyogenes* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. pyogenes* Cas9 molecule.

In an embodiment, the altered Cas9 molecule or Cas9 polypeptide is an eaCas9 molecule or eaCas9 polypeptide comprising the fixed amino acid residues of *S. thermophilus* shown in the consensus sequence disclosed in **Figs. 2A-2G****,** and has one or more amino acids that differ from the amino acid sequence of *S. thermophilus* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*"in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S*. *thermophilus* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g*.,* an *S. thermophilus* Cas9 molecule.

In an embodiment, the altered Cas9 molecule or Cas9 polypeptide is an eaCas9 molecule or eaCas9 polypeptide comprising the fixed amino acid residues of *S. mutans* shown in the consensus sequence disclosed in **Figs. 2A-2G****,** and has one or more amino acids that differ from the amino acid sequence of S. mutans (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S. mutans* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S*. *mutans* Cas9 molecule.

In an embodiment, the altered Cas9 molecule or Cas9 polypeptide is an eaCas9 molecule or eaCas9 polypeptide comprising the fixed amino acid residues of *L. innocula* shown in the consensus sequence disclosed in **Figs. 2A-2G****,** and has one or more amino acids that differ from the amino acid sequence of *L. innocula* (e.g., has a substitution) at one or more residue (e.g., 2, 3, 5, 10, 15, 20, 30, 50, 70, 80, 90, 100, 200 amino acid residues) represented by an "-"in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *L. innocula* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *L. innocula* Cas9 molecule.

In an embodiment, the altered Cas9 molecule or Cas9 polypeptide, e.g., an eaCas9 molecule, can be a fusion, e.g., of two of more different Cas9 molecules or Cas9 polypeptides, e.g., of two or more naturally occurring Cas9 molecules of different species. For example, a fragment of a naturally occurring Cas9 molecule of one species can be fused to a fragment of a Cas9 molecule of a second species. As an example, a fragment of Cas9 molecule *of S. pyogenes* comprising an N-terminal RuvC-like domain can be fused to a fragment of Cas9 molecule of a species other than *S. pyogenes* (e.g., *S. thermophilus)* comprising an HNH-like domain.

### Cas9 Molecules With Altered PAM Recognition Or No PAM Recognition

Naturally occurring Cas9 molecules can recognize specific PAM sequences, for example the PAM recognition sequences described above for, e.g., *S. pyogenes, S. thermophilus, S. mutans, S. aureus* and *N. meningitidis.*

In an embodiment, a Cas9 molecule or Cas9 polypeptide has the same PAM specificities as a naturally occurring Cas9 molecule. In other embodiments, a Cas9 molecule or Cas9 polypeptide has a PAM specificity not associated with a naturally occurring Cas9 molecule, or a PAM specificity not associated with the naturally occurring Cas9 molecule to which it has the closest sequence homology. For example, a naturally occurring Cas9 molecule can be altered, e.g., to alter PAM recognition, e.g., to alter the PAM sequence that the Cas9 molecule or Cas9 polypeptide recognizes to decrease off target sites and/or improve specificity; or eliminate a PAM recognition requirement. In an embodiment, a Cas9 molecule can be altered, e.g., to increase length of PAM recognition sequence and/or improve Cas9 specificity to high level of identity, e.g., to decrease off target sites and increase specificity. In an embodiment, the length of the PAM recognition sequence is at least 4, 5, 6, 7, 8, 9, 10 or 15 amino acids in length.

Cas9 molecules or Cas9 polypeptides that recognize different PAM sequences and/or have reduced off-target activity can be generated using directed evolution. Exemplary methods and systems that can be used for directed evolution of Cas9 molecules are described, e.g., in Esvelt et al. Nature 2011, 472(7344): 499-503. Candidate Cas9 molecules can be evaluated, e.g., by methods described in Section IV.

Alterations of the PI domain, which mediates PAM recognition, are discussed below.

### Synthetic Cas9 Molecules and Cas9 Polypeptides with Altered PI Domains

Current genome-editing methods are limited in the diversity of target sequences that can be targeted by the PAM sequence that is recognized by the Cas9 molecule utilized. A synthetic Cas9 molecule (or Syn-Cas9 molecule), or synthetic Cas9 polypeptide (or Syn-Cas9 polypeptide), as that term is used herein, refers to a Cas9 molecule or Cas9 polypeptide that comprises a Cas9 core domain from one bacterial species and a functional altered PI domain, i.e., a PI domain other than that naturally associated with the Cas9 core domain, e.g., from a different bacterial species.

In an embodiment, the altered PI domain recognizes a PAM sequence that is different from the PAM sequence recognized by the naturally-occurring Cas9 from which the Cas9 core domain is derived. In an embodiment, the altered PT domain recognizes the same PAM sequence recognized by the naturally-occurring Cas9 from which the Cas9 core domain is derived, but with different affinity or specificity. A Syn-Cas9 molecule or Syn-Cas9 polypetide can be, respectively, a Syn-eaCas9 molecule or Syn-eaCas9 polypeptide or a Syn-eiCas9 molecule Syn-eiCas9 polypeptide.

An exemplary Syn-Cas9 molecule or Syn-Cas9 polypetide comprises:
a) a Cas9 core domain, e.g., a Cas9 core domain from **Table 100** or **200,** e.g., a *S. aureus, S. pyogenes,* or *C. jejuni* Cas9 core domain; and
b) an altered PI domain from a species X Cas9 sequence selected from **Tables 400** and **500.**

In an embodiment, the RKR motif (the PAM binding motif) of said altered PI domain comprises: differences at 1, 2, or 3 amino acid residues; a difference in amino acid sequence at the first, second, or third position; differences in amino acid sequence at the first and second positions, the first and third positions, or the second and third positions; as compared with the sequence of the RKR motif of the native or endogenous PI domain associated with the Cas9 core domain.

In an embodiment, the Cas9 core domain comprises the Cas9 core domain from a species X Cas9 from **Table 100** and said altered PI domain comprises a PI domain from a species Y Cas9 from **Table 100.**

In an embodiment, the RKR motif of the species X Cas9 is other than the RKR motif of the species Y Cas9.

In an embodiment, the RKR motif of the altered PI domain is selected from XXY, XNG, and XNQ.

In an embodiment, the altered PI domain has at least 60, 70, 80, 90, 95, or 100% homology with the amino acid sequence of a naturally occurring PI domain of said species Y from **Table 100.**

In an embodiment, the altered PI domain differs by no more than 50, 40, 30, 25, 20, 15, 10, 5, 4, 3, 2, or 1 amino acid residue from the amino acid sequence of a naturally occurring PI domain of said second species from **Table 100.**

In an embodiment, the Cas9 core domain comprises a *S. aureus* core domain and altered PI domain comprises: an *A. denitrificans* PI domain; a *C. jejuni* PI domain; a *H. mustelae* PI domain; or an altered PI domain of species X PI domain, wherein species X is selected from **Table 500.**

In an embodiment, the Cas9 core domain comprises a *S. pyogenes* core domain and the altered PI domain comprises: an *A. denitrificans* PI domain; a *C. jejuni* PI domain; a *H. mustelae* PI domain; or an altered PI domain of species X PI domain, wherein species X is selected from **Table 500.**

In an embodiment, the Cas9 core domain comprises a *C. jejuni* core domain and the altered PI domain comprises: an *A. denitrificans* PI domain; a *H. mustelae* PI domain; or an altered PI domain of species X PI domain, wherein species X is selected from **Table 500.**

In an embodiment, the Cas9 molecule or Cas9 polypeptide further comprises a linker disposed between said Cas9 core domain and said altered PI domain.

In an embodiment, the linker comprises: a linker described elsewhere herein disposed between the Cas9 core domain and the heterologous PI domain. Suitable linkers are further described in Section V.

Exemplary altered PI domains for use in Syn-Cas9 molecules are described in **Tables 400** and **500.** The sequences for the 83 Cas9 orthologs referenced in **Tables 400** and **500** are provided in **Table 100. Table 300** provides the Cas9 orthologs with known PAM sequences and the corresponding RKR motif.

In an embodiment, a Syn-Cas9 molecule or Syn-Cas9 polypeptide may also be size-optimized, e.g., the Syn-Cas9 molecule or Syn-Cas9 polypeptide comprises one or more deletions, and optionally one or more linkers disposed between the amino acid residues flanking the deletions. In an embodiment, a Syn-Cas9 molecule or Syn-Cas9 polypeptide comprises a REC deletion.

### Size-Optimized Cas9 Molecules and Cas9 Polypeptides

Engineered Cas9 molecules and engineered Cas9 polypeptides described herein include a Cas9 molecule or Cas9 polypeptide comprising a deletion that reduces the size of the molecule while still retaining desired Cas9 properties, e.g., essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, e.g., a *S*. *aureus, S. pyogenes,* or *C. jejuni,* Cas9 molecule, having a deletion is smaller, e.g., has reduced number of amino acids, than the corresponding naturally-occurring Cas9 molecule. The smaller size of the Cas9 molecules allows increased flexibility for delivery methods, and thereby increases utility for genome-editing. A Cas9 molecule or Cas9 polypeptide can comprise one or more deletions that do not substantially affect or decrease the activity of the resultant Cas9 molecules or Cas9 polypeptides described herein. Activities that are retained in the Cas9 molecules or Cas9 polypeptides comprising a deletion as described herein include one or more of the following:
a nickase activity, i.e., the ability to cleave a single strand, e.g., the non-complementary strand or the complementary strand, of a nucleic acid molecule; a double stranded nuclease activity, i.e., the ability to cleave both strands of a double stranded nucleic acid and create a double stranded break, which in an embodiment is the presence of two nickase activities;
an endonuclease activity;
an exonuclease activity;
a helicase activity, i.e., the ability to unwind the helical structure of a double stranded nucleic acid;
and recognition activity of a nucleic acid molecule, e.g., a target nucleic acid or a gRNA.

Activity of the Cas9 molecules or Cas9 polypeptides described herein can be assessed using the activity assays described herein or in the art.

### Identifying regions suitable for deletion

Suitable regions of Cas9 molecules for deletion can be identified by a variety of methods. Naturally-occurring orthologous Cas9 molecules from various bacterial species, e.g., any one of those listed in **Table 100,** can be modeled onto the crystal structure of *S. pyogenes* Cas9 (Nishimasu et al., Cell, 156:935-949, 2014) to examine the level of conservation across the selected Cas9 orthologs with respect to the three-dimensional conformation of the protein. Less conserved or unconserved regions that are spatially located distant from regions involved in Cas9 activity, e.g., interface with the target nucleic acid molecule and/or gRNA, represent regions or domains are candidates for deletion without substantially affecting or decreasing Cas9 activity.

### REC-Optimized Cas9 Molecules and Cas9 Polypeptides

A REC-optimized Cas9 molecule, or a REC-optimized Cas9 polypeptide, as that term is used herein, refers to a Cas9 molecule or Cas9 polypeptide that comprises a deletion in one or both of the REC2 domain and the RE1_{CT} domain (collectively a REC deletion), wherein the deletion comprises at least 10% of the amino acid residues in the cognate domain. A REC-optimized Cas9 molecule or Cas9 polypeptide can be an eaCas9 molecule or eaCas9 polypetide, or an eiCas9 molecule or eiCas9 polypeptide. An exemplary REC-optimized Cas9 molecule or REC-optimized Cas9 polypeptide comprises:
a) a deletion selected from:
   i) a REC2 deletion;
   ii) a REC1_{CT} deletion; or
   iii) a REC1_{SUB} deletion.

Optionally, a linker is disposed between the amino acid residues that flank the deletion. In an embodiment a Cas9 molecule or Cas9 polypeptide includes only one deletion, or only two deletions. A Cas9 molecule or Cas9 polypeptide can comprise a REC2 deletion and a REC1_{CT} deletion. A Cas9 molecule or Cas9 polypeptide can comprise a REC2 deletion and a REC1_{SUB} deletion.

Generally, the deletion will contain at least 10% of the amino acids in the cognate domain, e.g., a REC2 deletion will include at least 10% of the amino acids in the REC2 domain. A deletion can comprise: at least 10, 20, 30, 40, 50, 60, 70, 80, or 90% of the amino acid residues of its cognate domain; all of the amino acid residues of its cognate domain; an amino acid residue outside its cognate domain; a plurality of amino acid residues outside its cognate domain; the amino acid residue immediately N terminal to its cognate domain; the amino acid residue immediately C terminal to its cognate domain; the amino acid residue immediately N terminal to its cognate and the amino acid residue immediately C terminal to its cognate domain; a plurality of, e.g., up to 5, 10, 15, or 20, amino acid residues N terminal to its cognate domain; a plurality of, e.g., up to 5, 10, 15, or 20, amino acid residues C terminal to its cognate domain; a plurality of, e.g., up to 5, 10, 15, or 20, amino acid residues N terminal to to its cognate domain and a plurality of e.g., up to 5, 10, 15, or 20, amino acid residues C terminal to its cognate domain.

In an embodiment, a deletion does not extend beyond: its cognate domain; the N terminal amino acid residue of its cognate domain; the C terminal amino acid residue of its cognate domain.

A REC-optimized Cas9 molecule or REC-optimized Cas9 polypeptide can include a linker disposed between the amino acid residues that flank the deletion. Suitable linkers for use between the amino acid resides that flank a REC deletion in a REC-optimized Cas9 molecule is disclosed in Section V.

In an embodiment, a REC-optimized Cas9 molecule or REC-optimized Cas9 polypeptide comprises an amino acid sequence that, other than any REC deletion and associated linker, has at least 50, 55, 60, 65, 70, 75, 80, 85, 90, 95, 99, or 100% homology with the amino acid sequence of a naturally occurring Cas 9, e.g., a Cas9 molecule described in **Table 100,** e.g., a *S. aureus* Cas9 molecule, a *S. pyogenes* Cas9 molecule, or a *C. jejuni* Cas9 molecule.

In an embodiment, a a REC-optimized Cas9 molecule or REC-optimized Cas9 polypeptide comprises an amino acid sequence that, other than any REC deletion and associated linker, differs by no more than 1, 2, 3, 4, 5, 6, 7, 8, 9, 10, 15, 20, or 25, amino acid residues from the amino acid sequence of a naturally occurring Cas 9, e.g., a Cas9 molecule described in **Table 100,** e.g., a *S. aureus* Cas9 molecule, a *S. pyogenes* Cas9 molecule, or a *C. jejuni* Cas9 molecule.

In an embodiment, a REC-optimized Cas9 molecule or REC-optimized Cas9 polypeptide comprises an amino acid sequence that, other than any REC deletion and associate linker, differs by no more than 1, 2, 3, 4, 5, 6, 7, 8, 9, 10, 15, 20, or 25% of the, amino acid residues from the amino acid sequence of a naturally occurring Cas 9, e.g., a Cas9 molecule described in Table **100,** e.g., a *S. aureus* Cas9 molecule, a *S. pyogenes* Cas9 molecule, or a *C. jejuni* Cas9 molecule.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Sequence information for exemplary REC deletions are provided for 83 naturally-occurring Cas9 orthologs in **Table 100.** The amino acid sequences of exemplary Cas9 molecules from different bacterial species are shown below.

**Table 100. Amino Acid Sequence of Cas9 Orthologs**

| | | REC2 | | | REC1_{CT} | | | REC1_{SUB} | | |
|---|---|---|---|---|---|---|---|---|---|---|
| **Species / Composite ID** | **Amino acid sequence** | start (AA pos) | stop (AA pos) | # AA delete d(n) | start (AA pos) | stop (AA pos) | # AA delet ed (n) | start (AA pos) | stop (AA pos) | # AA delet ed (n) |
| Staphylococcus Aureus tr\|J7RUA5\|J7RUA 5_STAAU | SEQ ID NO:304 | 126 | 166 | 41 | 296 | 352 | 57 | 296 | 352 | 57 |
| Streptococcus Pyogenes sp\|Q99ZW2\|CAS9 _STRP1 | SEQ ID NO:305 | 176 | 314 | 139 | 511 | 592 | 82 | 511 | 592 | 82 |
| Campylobacter jejuni NCTC 11168 gi\|218563121\|ref\| YP_002344900.1 | SEQ ID NO:306 | 137 | 181 | 45 | 316 | 360 | 45 | 316 | 360 | 45 |
| Bacteroides fragilis NCTC 9343 gi\|60683389\|ref\|Y P_213533.1\| | SEQ ID NO:307 | 148 | 339 | 192 | 524 | 617 | 84 | 524 | 617 | 84 |
| Bifidobacterium bifidum S17 gi\|310286728\|ref\| YP_003937986. | SEQ ID NO:308 | 173 | 335 | 163 | 516 | 607 | 87 | 516 | 607 | 87 |
| Veillonella atypica ACS-134-V-Col7a gi\|303229466\|ref\|Z P_07316256.1 | SEQ ID NO:309 | 185 | 339 | 155 | 574 | 663 | 79 | 574 | 663 | 79 |
| Lactobacillus rhamnosus GG gi\|258509199\|ref\| YP_003171950.1 | SEQ ID NO:310 | 169 | 320 | 152 | 559 | 645 | 78 | 559 | 645 | 78 |
| Filifactor alocis ATCC 35896 gi\|374307738\|ref\| YP_005054169.1 | SEQ ID NO:311 | 166 | 314 | 149 | 508 | 592 | 76 | 508 | 592 | 76 |
| Oenococcus kitaharae DSM 17330 gi\|366983953\|gb\|E HN59352.1\| | SEQ ID NO:312 | 169 | 317 | 149 | 555 | 639 | 80 | 555 | 639 | 80 |
| Fructobacillus fructosus KCTC 3544 gi\|339625081\|ref\|Z P_08660870.1 | SEQ ID NO:313 | 168 | 314 | 147 | 488 | 571 | 76 | 488 | 571 | 76 |
| Catenibacterium mitsuokai DSM 15897 gi\|224543312\|ref\|Z P_ 03683851.1 | SEQ ID NO:314 | 173 | 318 | 146 | 511 | 594 | 78 | 511 | 594 | 78 |
| Finegoldia magna ATCC 29328 gi\|169823755\|ref\| YP_001691366.1 | SEQ ID NO:315 | 168 | 313 | 146 | 452 | 534 | 77 | 452 | 534 | 77 |
| Coriobacteriumglo meransPW2 gi\|328956315\|ref\| YP_004373648.1 | SEQ ID NO:316 | 175 | 318 | 144 | 511 | 592 | 82 | 511 | 592 | 82 |
| Eubacterium yurii ATCC 43715 gi\|306821691\|ref\|Z P_07455288.1 | SEQ ID NO:317 | 169 | 310 | 142 | 552 | 633 | 76 | 552 | 633 | 76 |
| Peptoniphilus duerdenii ATCC BAA-1640 gi\|304438954\|ref\|Z P_07398877.1 | SEQ ID NO:318 | 171 | 311 | 141 | 535 | 615 | 76 | 535 | 615 | 76 |
| Acidaminococcus sp. D21 gi\|227824983\|ref\|Z P_03989815.1 | SEQ ID NO:319 | 167 | 306 | 140 | 511 | 591 | 75 | 511 | 591 | 75 |
| Lactobacillus farciminis KCTC 3681 gi\|336394882\|ref\|Z P_ 08576281.1 | SEQ ID NO:320 | 171 | 310 | 140 | 542 | 621 | 85 | 542 | 621 | 85 |
| Streptococcus sanguinis SK49 gi\|422884106\|ref\|Z P_16930555.1 | SEQ ID NO:321 | 185 | 324 | 140 | 411 | 490 | 85 | 411 | 490 | 85 |
| Coprococcus catus GD-7 gi\|291520705\|emb\| CBK78998.1\| | SEQ ID NO:322 | 172 | 310 | 139 | 556 | 634 | 76 | 556 | 634 | 76 |
| Streptococcus mutans UA159 gi\|24379809\|ref\|N P_721764.1\| | SEQ ID NO:323 | 176 | 314 | 139 | 392 | 470 | 84 | 392 | 470 | 84 |
| Streptococcus pyogenes M1GAS gi\|13622193\|gb\|A AK33936.1\| | SEQ ID NO:324 | 176 | 314 | 139 | 523 | 600 | 82 | 523 | 600 | 82 |
| Streptococcus thermophilus LMD-9 gi\|116628213\|ref\| YP_ 820832.1\| | SEQ ID NO:325 | 176 | 314 | 139 | 481 | 558 | 81 | 481 | 558 | 81 |
| Fusobacteriumnucl eatum ATCC49256 gi\|34762592\|ref\|ZP _00143587.1\| | SEQ ID NO:326 | 171 | 308 | 138 | 537 | 614 | 76 | 537 | 614 | 76 |
| Planococcus antarcticus DSM 14505 gi\|389815359\|ref\|Z P_10206685.1 | SEQ ID NO:327 | 162 | 299 | 138 | 538 | 614 | 94 | 538 | 614 | 94 |
| Treponema denticola ATCC 35405 gi\|42525843\|ref\|N P_970941.1\| | SEQ ID NO:328 | 169 | 305 | 137 | 524 | 600 | 81 | 524 | 600 | 81 |
| Solobacterium moorei F0204 gi\|320528778\|ref\|Z P_08029929.1 | SEQ ID NO:329 | 179 | 314 | 136 | 544 | 619 | 77 | 544 | 619 | 77 |
| Staphylococcus pseudintermedius ED99 gi\|323463801\|gb\|A DX75954.1\| | SEQ ID NO:330 | 164 | 299 | 136 | 531 | 606 | 92 | 531 | 606 | 92 |
| Flavobacterium branchiophilum FL-15 gi\|347536497\|ref\| YP_004843922.1 | SEQ ID NO:331 | 162 | 286 | 125 | 538 | 613 | 63 | 538 | 613 | 63 |
| Ignavibacterium album JCM 16511 gi\|385811609\|ref\| YP_005848005.1 | SEQ ID NO:332 | 223 | 329 | 107 | 357 | 432 | 90 | 357 | 432 | 90 |
| Bergeyella zoohelcum ATCC 43767 gi\|423317190\|ref]Z P_17295095.1 | SEQ ID NO:333 | 165 | 261 | 97 | 529 | 604 | 56 | 529 | 604 | 56 |
| Nitrobacter hamburgensis X14 gi\|92109262\|ref\|Y P_571550.1\| | SEQ ID NO:334 | 169 | 253 | 85 | 536 | 611 | 48 | 536 | 611 | 48 |
| Odoribacter laneus YIT 12061 gi\|374384763\|ref\|Z P_09642280.1 | SEQ ID NO:335 | 164 | 242 | 79 | 535 | 610 | 63 | 535 | 610 | 63 |
| Legionella pneumophila str. Paris gi\|54296138\|ref\|Y P_122507.1\| | SEQ ID NO:336 | 164 | 239 | 76 | 402 | 476 | 67 | 402 | 476 | 67 |
| Bacteroides sp. 20 3 gi\|301311869\|ref\|Z P_07217791.1 | SEQ ID NO:337 | 198 | 269 | 72 | 530 | 604 | 83 | 530 | 604 | 83 |
| Akkermansia muciniphila ATCC BAA-835 gi\|187736489\|ref\| YP_001878601. | SEQ ID NO:338 | 136 | 202 | 67 | 348 | 418 | 62 | 348 | 418 | 62 |
| Prevotella sp. C561 gi\|345885718\|ref]Z P_08837074.1 | SEQ ID NO:339 | 184 | 250 | 67 | 357 | 425 | 78 | 357 | 425 | 78 |
| Wolinella succinogenes DSM 1740 gi\|34557932\|ref\|N P_907747.1\| | SEQ ID NO:340 | 157 | 218 | 36 | 401 | 468 | 60 | 401 | 468 | 60 |
| Alicyclobacillus hesperidum URH17-3-68 gi\|403744858\|ref\|Z P_10953934.1 | SEQ ID NO:341 | 142 | 196 | 55 | 416 | 482 | 61 | 416 | 482 | 61 |
| Caenispirillum salinarum AK4 gi\|427429481\|ref\|Z P_ 18919511.1 | SEQ ID NO:342 | 161 | 214 | 54 | 330 | 393 | 68 | 330 | 393 | 68 |
| Eubacterium rectale ATCC 33656 gi\|238924075\|ref\| YP_002937591.1 | SEQ ID NO:343 | 133 | 185 | 53 | 322 | 384 | 60 | 322 | 384 | 60 |
| Mycoplasma synoviae 53 gi\|71894592\|ref\|Y P_278700.1\| | SEQ ID NO:344 | 187 | 239 | 53 | 319 | 381 | 80 | 319 | 381 | 80 |
| Porphyromonas sp. oral taxon 279 str. F0450 gi\|402847315\|ref\|Z P_10895610.1 | SEQ ID NO:345 | 150 | 202 | 53 | 309 | 371 | 60 | 309 | 371 | 60 |
| Streptococcus thermophilus LMD-9 gi\|116627542\|ref\| YP_ 820161.1 | SEQ ID NO:346 | 127 | 178 | 139 | 424 | 486 | 81 | 424 | 486 | 81 |
| Roseburia inulinivorans DSM 16841 gi\|225377804\|ref\|Z P_03755025.1 | SEQ ID NO:347 | 154 | 204 | 51 | 318 | 380 | 69 | 318 | 380 | 69 |
| Methylosinus trichosporium OB3b gi\|296446027\|ref\|Z P_06887976.1 | SEQ ID NO:348 | 144 | 193 | 50 | 426 | 488 | 64 | 426 | 488 | 64 |
| Ruminococcus albus 8 gi\|325677756\|ref\|Z P_08157403.1 | SEQ ID NO:349 | 139 | 187 | 49 | 351 | 412 | 55 | 351 | 412 | 55 |
| Bifidobacterium longum DJO10A gi\|189440764\|ref\| YP_001955845. | SEQ ID NO:350 | 183 | 230 | 48 | 370 | 431 | 44 | 370 | 431 | 44 |
| Enterococcus faecalis TX0012 gi\|315149830\|gb\|E FT93846.1\| | SEQ ID NO:351 | 123 | 170 | 48 | 327 | 387 | 60 | 327 | 387 | 60 |
| Mycoplasma mobile 163K gi\|47458868\|ref\|Y P_015730.1\| | SEQ ID NO:352 | 179 | 226 | 48 | 314 | 374 | 79 | 314 | 374 | 79 |
| Actinomyces coleocanis DSM 15436 gi\|227494853\|ref\|Z P_03925169.1 | SEQ ID NO:353 | 147 | 193 | 47 | 358 | 418 | 40 | 358 | 418 | 40 |
| Dinoroseobacter shibae DFL 12 gi\|159042956\|ref\| YP_001531750.1 | SEQ ID NO:354 | 138 | 184 | 47 | 338 | 398 | 48 | 338 | 398 | 48 |
| Actinomyces sp. oral taxon 180 str. F0310 gi\|315605738\|ref\|Z P_07880770.1 | SEQ ID NO:355 | 183 | 228 | 46 | 349 | 409 | 40 | 349 | 409 | 40 |
| Alcanivorax sp. W11-5 gi\|407803669\|ref\|Z P_11150502.1 | SEQ ID NO:356 | 139 | 183 | 45 | 344 | 404 | 61 | 344 | 404 | 61 |
| Aminomonas paucivorans DSM 12260 gi\|312879015\|ref\|Z P_07738815.1 | SEQ ID NO:357 | 134 | 178 | 45 | 341 | 401 | 63 | 341 | 401 | 63 |
| Mycoplasma canis PG 14 gi\|384393286\|gb\|E IE39736.1\| | SEQ ID NO:358 | 139 | 183 | 45 | 319 | 379 | 76 | 319 | 379 | 76 |
| Lactobacillus coryniformis KCTC 3535 gi\|336393381\|ref\|Z P_08574780.1 | SEQ ID NO:359 | 141 | 184 | 44 | 328 | 387 | 61 | 328 | 387 | 61 |
| Elusimicrobium minutum Pei191 gi\|187250660\|ref\| YP_001875142.1 | SEQ ID NO:360 | 177 | 219 | 43 | 322 | 381 | 47 | 322 | 381 | 47 |
| Neisseria meningitidis Z2491 gi\|218767588\|ref\| YP_002342100.1 | SEQ ID NO:361 | 147 | 189 | 43 | 360 | 419 | 61 | 360 | 419 | 61 |
| Pasteurella multocida str. Pm70 gi\|15602992\|ref\|N P_246064.1\| | SEQ ID NO:362 | 139 | 181 | 43 | 319 | 378 | 61 | 319 | 378 | 61 |
| Rhodovulum sp. PH10 gi\|402849997\|ref\|Z P_10898214.1 | SEQ ID NO:363 | 141 | 183 | 43 | 319 | 378 | 48 | 319 | 378 | 48 |
| Eubacterium dolichum DSM 3991 gi\|160915782\|ref\|Z P_02077990.1 | SEQ ID NO:364 | 131 | 172 | 42 | 303 | 361 | 59 | 303 | 361 | 59 |
| Nitratifractor salsuginis DSM 16511 gi\|319957206\|ref\| YP_004168469.1 | SEQ ID NO:365 | 143 | 184 | 42 | 347 | 404 | 61 | 347 | 404 | 61 |
| Rhodospirillum rubrum ATCC 11170 gi\|83591793\|ref\|Y P_ 425545.1\| | SEQ ID NO:366 | 139 | 180 | 42 | 314 | 371 | 55 | 314 | 371 | 55 |
| Clostridium cellulolyticum H10 gi\|220930482\|ref\| YP_002507391.1 | SEQ ID NO:367 | 137 | 176 | 40 | 320 | 376 | 61 | 320 | 376 | 61 |
| Helicobacter mustelae 12198 gi\|291276265\|ref\| YP_003516037.1 | SEQ ID NO:368 | 148 | 187 | 40 | 298 | 354 | 48 | 298 | 354 | 48 |
| Ilyobacter polytropus DSM 2926 gi\|310780384\|ref\| YP_003968716.1 | SEQ ID NO:369 | 134 | 173 | 40 | 462 | 517 | 63 | 462 | 517 | 63 |
| Sphaerochaeta globus str. Buddy gi\|325972003\|ref\| YP_004248194.1 | SEQ ID NO:370 | 163 | 202 | 40 | 335 | 389 | 45 | 335 | 389 | 45 |
| S Staphylococcus lugdunensis M23590 gi\|315659848\|ref\|Z P_07912707.1 | SEQ ID NO:371 | 128 | 167 | 40 | 337 | 391 | 57 | 337 | 391 | 57 |
| Treponema sp. JC4 gi\|384109266\|ref\|Z P_10010146.1 | SEQ ID NO:372 | 144 | 183 | 40 | 328 | 382 | 63 | 328 | 382 | 63 |
| uncultured delta proteobacterium HF0070 07E19 gi\|297182908\|gb\|A DI19058.1\| | SEQ ID NO:373 | 154 | 193 | 40 | 313 | 365 | 55 | 313 | 365 | 55 |
| Alicycliphilus denitrificans K601 gi\|330822845\|ref\| YP_004386148.1 | SEQ ID NO:374 | 140 | 178 | 39 | 317 | 366 | 48 | 317 | 366 | 48 |
| Azospirillum sp. B510 gi\|288957741\|ref\| YP_003448082.1 | SEQ ID NO:375 | 205 | 243 | 39 | 342 | 389 | 46 | 342 | 389 | 46 |
| Bradyrhizobium sp. BTAi1 gi\|148255343\|ref\| YP_001239928.1 | SEQ ID NO:376 | 143 | 181 | 39 | 323 | 370 | 48 | 323 | 370 | 48 |
| Parvibaculum lavamentivorans DS-1 gi\|154250555\|ref\| YP_001411379.1 | SEQ ID NO:377 | 138 | 176 | 39 | 327 | 374 | 58 | 327 | 374 | 58 |
| Prevotella timonensis CRIS 5C-B1 gi\|282880052\|ref\|Z P_06288774.1 | SEQ ID NO:378 | 170 | 208 | 39 | 328 | 375 | 61 | 328 | 375 | 61 |
| Bacillus smithii 7 3 47FAA gi\|365156657\|ref\|Z P_09352959.1 | SEQ ID NO:379 | 134 | 171 | 38 | 401 | 448 | 63 | 401 | 448 | 63 |
| Cand. Puniceispirillum marinum IMCC1322 gi\|294086111\|ref\| YP_003552871.1 | SEQ ID NO:380 | 135 | 172 | 38 | 344 | 391 | 53 | 344 | 391 | 53 |
| Barnesiella intestinihominis YIT 11860 gi\|404487228\|ref\|Z P_11022414.1 | SEQ ID NO:381 | 140 | 176 | 37 | 371 | 417 | 60 | 371 | 417 | 60 |
| Ralstonia syzygii R24 gi\|344171927\|emb\| CCA84553.1\| | SEQ ID NO:382 | 140 | 176 | 37 | 395 | 440 | 50 | 395 | 440 | 50 |
| Wolinella succinogenes DSM 1740 gi\|34557790\|ref\|N P_907605.1\| | SEQ ID NO:383 | 145 | 180 | 36 | 348 | 392 | 60 | 348 | 392 | 60 |
| Mycoplasma gallisepticum str. F gi\|284931710\|gb\|A DC31648.1\| | SEQ ID NO:384 | 144 | 177 | 34 | 373 | 416 | 71 | 373 | 416 | 71 |
| Acidothermus cellulolyticus 11B gi\|117929158\|ref\| YP_873709.1\| | SEQ ID NO:385 | 150 | 182 | 33 | 341 | 380 | 58 | 341 | 380 | 58 |
| Mycoplasma ovipneumoniae SC01 gi\|363542550\|ref\|Z P_09312133.1 | SEQ ID NO:386 | 156 | 184 | 29 | 381 | 420 | 62 | 381 | 420 | 62 |

**Table 200. Amino Acid Sequence of Cas9 Core Domains**

| **Strain Name** | **Cas9 Start (AA pos)** | **Cas9 Stop (AA pos)** |
|---|---|---|
| | **Start and Stop numbers refer to the sequence in Table 100** | |
| Staphylococcus Aureus | 1 | 772 |
| Streptococcus Pyogenes | 1 | 1099 |
| Campulobacter Jejuni | 1 | 741 |

**Table 300. Identified PAM sequences and corresponding RKR motifs.**

| **Strain Name** | **PAM sequence (NA)** | **RKR motif (AA)** |
|---|---|---|
| Streptococcus pyogenes | NGG | RKR |
| Streptococcus mutans | NGG | RKR |
| Streptococcus thermophilus A | NGGNG | RYR |
| Treponema denticola | NAAAAN | VAK |
| Streptococcus thermophilus B | NNAAAAW | IYK |
| Campylobacter jejuni | NNNNACA | NLK |
| Pasteurella multocida | GNNNCNNA | KDG |
| Neisseria meningitidis | NNNNGATT or NNGRRT (R = A or G) | IGK |
| Staphylococcus aureus | NNGRRV (R = A or G; V = A. G or C) or NNGRRT (R = A or G) | NDK |

PI domains are provided in **Tables 400** and **500.**

**Table 400. Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 100** | | | |
| Alicycliphilus denitrificans K601 | 837 | 1029 | 193 | --Y |
| Campylobacter jejuni NCTC 11168 | 741 | 984 | 244 | -NG |
| Helicobacter mustelae 12198 | 771 | 1024 | 254 | -NQ |

**Table 500. Other Altered PI Domains**

| **Strain Name** | **PI Start (AA pos)** | **PI Stop Length of PI (AA pos) (AA)** | | **RKR motif (AA)** |
|---|---|---|---|---|
| | **Start and Stop numbers refer to the sequences in Table 100** | | | |
| Akkermansia muciniphila ATCC BAA-835 | 871 | 1101 | 231 | ALK |
| Ralstonia syzygii R24 | 821 | 1062 | 242 | APY |
| Cand. Puniceispirillum marinum IMCC1322 | 815 | 1035 | 221 | AYK |
| Fructobacillus fructosus KCTC 3544 | 1074 | 1323 | 250 | DGN |
| Eubacterium yurii ATCC 43715 | 1107 | 1391 | 285 | DGY |
| Eubacterium dolichum DSM 3991 | 779 | 1096 | 318 | DKK |
| Dinoroseobacter shibae DFL 12 | 851 | 1079 | 229 | DPI |
| Clostridium cellulolyticum H10 | 767 | 1021 | 255 | EGK |
| Pasteurella multocida str. Pm70 | 815 | 1056 | 242 | ENN |
| Mycoplasma canis PG 14 | 907 | 1233 | 327 | EPK |
| Porphyromonas sp. oral taxon 279 str. F0450 | 935 | 1197 | 263 | EPT |
| Filifactor alocis ATCC 35896 | 1094 | 1365 | 272 | EVD |
| Aminomonas paucivorans DSM 12260 | 801 | 1052 | 252 | EVY |
| Wolinella succinogenes DSM 1740 | 1034 | 1409 | 376 | EYK |
| Oenococcus kitaharae DSM 17330 | 1119 | 1389 | 271 | GAL |
| CoriobacteriumglomeransPW2 | 1126 | 1384 | 259 | GDR |
| Peptoniphilus duerdenii ATCC BAA-1640 | 1091 | 1364 | 274 | GDS |
| Bifidobacterium bifidum S17 | 1138 | 1420 | 283 | GGL |
| Alicyclobacillus hesperidum URH17-3-68 | 876 | 1146 | 271 | GGR |
| Roseburia inulinivorans DSM 16841 | 895 | 1152 | 258 | GGT |
| Actinomyces coleocanis DSM 15436 | 843 | 1105 | 263 | GKK |
| Odoribacter laneus YIT 12061 | 1103 | 1498 | 396 | GKV |
| Coprococcus catus GD-7 | 1063 | 1338 | 276 | GNQ |
| Enterococcus faecalis TX0012 | 829 | 1150 | 322 | GRK |
| Bacillus smithii 7 3 47FAA | 809 | 1088 | 280 | GSK |
| Legionella pneumophila str. Paris | 1021 | 1372 | 352 | GTM |
| Bacteroides fragilis NCTC 9343 | 1140 | 1436 | 297 | IPV |
| Mycoplasma ovipneumoniae SC01 | 923 | 1265 | 343 | IRI |
| Actinomyces sp. oral taxon 180 str. F0310 | 895 | 1181 | 287 | KEK |
| Treponema sp. JC4 | 832 | 1062 | 231 | KIS |
| Fusobacteriumnucleatum ATCC49256 | 1073 | 1374 | 302 | KKV |
| Lactobacillus farciminis KCTC 3681 | 1101 | 1356 | 256 | KKV |
| Nitratifractor salsuginis DSM 16511 | 840 | 1132 | 293 | KMR |
| Lactobacillus coryniformis KCTC 3535 | 850 | 1119 | 270 | KNK |
| Mycoplasma mobile 163K | 916 | 1236 | 321 | KNY |
| Flavobacterium branchiophilum FL-15 | 1182 | 1473 | 292 | KQK |
| Prevotella timonensis CRIS 5C-B1 | 957 | 1218 | 262 | KQQ |
| Methylosinus trichosporium OB3b | 830 | 1082 | 253 | KRP |
| Prevotella sp. C561 | 1099 | 1424 | 326 | KRY |
| Mycoplasma gallisepticum str. F | 911 | 1269 | 359 | KTA |
| Lactobacillus rhamnosus GG | 1077 | 1363 | 287 | KYG |
| Wolinella succinogenes DSM 1740 | 811 | 1059 | 249 | LPN |
| Streptococcus thermophilus LMD-9 | 1099 | 1388 | 290 | MLA |
| Treponema denticola ATCC 35405 | 1092 | 1395 | 304 | NDS |
| Bergeyella zoohelcum ATCC 43767 | 1098 | 1415 | 318 | NEK |
| Veillonella atypica ACS-134-V-Col7a | 1107 | 1398 | 292 | NGF |
| Neisseria meningitidis Z2491 | 835 | 1082 | 248 | NHN |
| Ignavibacterium album JCM 16511 | 1296 | 1688 | 393 | NKK |
| Ruminococcus albus 8 | 853 | 1156 | 304 | NNF |
| Streptococcus thermophilus LMD-9 | 811 | 1121 | 311 | NNK |
| Barnesiella intestinihominis YIT 11860 | 871 | 1153 | 283 | NPV |
| Azospirillum sp. B510 | 911 | 1168 | 258 | PFH |
| Rhodospirillum rubrum ATCC 11170 | 863 | 1173 | 311 | PRG |
| Planococcus antarcticus DSM 14505 | 1087 | 1333 | 247 | PYY |
| Staphylococcus pseudintermedius ED99 | 1073 | 1334 | 262 | QIV |
| Alcanivorax sp. W11-5 | 843 | 1113 | 271 | RIE |
| Bradyrhizobium sp. BTAi1 | 811 | 1064 | 254 | RIY |
| Streptococcus pyogenes M1 GAS | 1099 | 1368 | 270 | RKR |
| Streptococcus mutans UA159 | 1078 | 1345 | 268 | RKR |
| Streptococcus Pyogenes | 1099 | 1368 | 270 | RKR |
| Bacteroides sp. 20 3 | 1147 | 1517 | 371 | RNI |
| *S. aureus* | 772 | 1053 | 282 | RNK |
| Solobacterium moorei F0204 | 1062 | 1327 | 266 | RSG |
| Finegoldia magna ATCC 29328 | 1081 | 1348 | 268 | RTE |
| uncultured delta proteobacterium HF0070 07E19 | 770 | 1011 | 242 | SGG |
| Acidaminococcus sp. D21 | 1064 | 1358 | 295 | SIG |
| Eubacterium rectale ATCC 33656 | 824 | 1114 | 291 | SKK |
| Caenispirillum salinarum AK4 | 1048 | 1442 | 395 | SLV |
| Acidothermus cellulolyticus 11B | 830 | 1138 | 309 | SPS |
| Catenibacterium mitsuokai DSM 15897 | 1068 | 1329 | 262 | SPT |
| Parvibaculum lavamentivorans DS-1 | 827 | 1037 | 211 | TGN |
| Staphylococcus lugdunensis M23590 | 772 | 1054 | 283 | TKK |
| Streptococcus sanguinis SK49 | 1123 | 1421 | 299 | TRM |
| Elusimicrobium minutum Pei191 | 910 | 1195 | 286 | TTG |
| Nitrobacter hamburgensis X14 | 914 | 1166 | 253 | VAY |
| Mycoplasma synoviae 53 | 991 | 1314 | 324 | VGF |
| Sphaerochaeta globus str. Buddy | 877 | 1179 | 303 | VKG |
| Ilyobacter polytropus DSM 2926 | 837 | 1092 | 256 | VNG |
| Rhodovulum sp. PH10 | 821 | 1059 | 239 | VPY |
| Bifidobacterium longum DJO10A | 904 | 1187 | 284 | VRK |

Below are the amino acid sequences for the Cas9 orthologs listed in Table 100.

SEQ ID NO:304
MKRNYILGLDIGITSVGYGIIDYETRDVIDAGVRLFKEANVENNEGRRSKRGARRLKRRRRHRIQRVKKL
LFDYNLLTDHSELSGINPYEARVKGLSQKLSEEEFSAALLHLAKRRGVHNVNEVEEDTGNELSTKEQISR
NSKALEEKYVAELQLERLKKDGEVRGSINRFKTSDYVKEAKQLLKVQKAYHQLDQSFIDTYIDLLETRRT
YYEGPGEGSPFGWKDIKEWYEMLMGHCTYFPEELRSVKYAYNADLYNALNDLNNLVITRDENEKLEYYEK
FQIIENVFKQKKKPTLKQIAKEILVNEEDIKGYRVTSTGKPEFTNLKVYHDIKDITARKEIIENAELLDQ
IAKILTIYQSSEDIQEELTNLNSELTQEEIEQISNLKGYTGTHNLSLKAINLILDELWHTNDNQIAIFNR
LKLVPKKVDLSQQKEIPTTLVDDFILSPVVKRSFIQSIKVINAIIKKYGLPNDIIIELAREKNSKDAQKM
INEMQKRNRQTNERIEEIIRTTGKENAKYLIEKIKLHDMQEGKCLYSLEAIPLEDLLNNPFNYEVDHIIP
RSVSFDNSFNNKVLVKQEENSKKGNRTPFQYLSSSDSKISYETFKKHILNLAKGKGRISKTKKEYLLEER
DINRFSVQKDFINRNLVDTRYATRGLMNLLRSYFRVNNLDVKVKSINGGFTSFLRRKWKFKKERNKGYKH
HAEDALIIANADFIFKEWKKLDKAKKVMENQMFEEKQAESMPEIETEQEYKEIFITPHQIKHIKDFKDYK
YSHRVDKKPNRELINDTLYSTRKDDKGNTLIVNNLNGLYDKDNDKLKKLINKSPEKLLMYHHDPQTYQKL
KLIMEQYGDEKNPLYKYYEETGNYLTKYSKKDNGPVIKKIKYYGNKLNAHLDITDDYPNSRNKVVKLSLK
PYRFDVYLDNGVYKFVTVKNLDVIKKENYYEVNSKCYEEAKKLKKISNQAEFIASFYNNDLIKINGELYR
VIGVNNDLLNRIEVNMIDITYREYLENMNDKRPPRIIKTIASKTQSIKKYSTDILGNLYEVKSKKHPQII
KKG

SEQ ID NO:305






















SEQ ID NO:306
MARILAFDIGISSIGWAFSENDELKDCGVRIFTKVENPKTGESLALPRRLARSARKRLARRKARLNHLKH
LIANEFKLNYEDYQSFDESLAKAYKGSLISPYELRFRALNELLSKQDFARVILHIAKRRGYDDIKNSDDK
EKGAILKAIKQNEEKLANYQSVGEYLYKEYFQKFKENSKEFTNVRNKKESYERCIAQSFLKDELKLIFKK
QREFGFSFSKKFEEEVLSVAFYKRALKDFSHLVGNCSFFTDEKRAPKNSPLAFMFVALTRIINLLNNLKN
TEGILYTKDDLNALLNEVLKNGTLTYKQTKKLLGLSDDYEFKGEKGTYFIEFKKYKEFIKALGEHNLSQD
DLNEIAKDITLIKDEIKLKKALAKYDLNQNQIDSLSKLEFKDHLNISFKALKLVTPLMLEGKKYDEACNE
LNLKVAINEDKKDFLPAFNETYYKDEVTNPVVLRAIKEYRKVLNALLKKYGKVHKINIELAREVGKNHSQ
RAKIEKEQNENYKAKKDAELECEKLGLKINSKNILKLRLFKEQKEFCAYSGEKIKISDLQDEKMLEIDHI
YPYSRSFDDSYMNKVLVFTKQNQEKLNQTPFEAFGNDSAKWQKIEVLAKNLPTKKQKRILDKNYKDKEQK
NFKDRNLNDTRYIARLVLNYTKDYLDFLPLSDDENTKLNDTQKGSKVHVEAKSGMLTSALRHTWGFSAKD
RNNHLHHAIDAVIIAYANNSIVKAFSDFKKEQESNSAELYAKKISELDYKNKRKFFEPFSGFRQKVLDKI
DEIFVSKPERKKPSGALHEETFRKEEEFYQSYGGKEGVLKALELGKIRKVNGKIVKNGDMFRVDIFKHKK
TNKFYAVPIYTMDFALKVLPNKAVARSKKGEIKDWILMDENYEFCFSLYKDSLILIQTKDMQEPEFVYYN
AFTSSTVSLIVSKHDNKFETLSKNQKILFKNANEKEVIAKSIGIQNLKVFEKYIVSALGEVTKAEFRQRE
DFKK

SEQ ID NO:307
MKRILGLDLGTNSIGWALVNEAENKDERSSIVKLGVRVNPLTVDELTNFEKGKSITTNADRTLKRGMRRN
LQRYKLRRETLTEVLKEHKLITEDTILSENGNRTTFETYRLRAKAVTEEISLEEFARVLLMINKKRGYKS
SRKAKGVEEGTLIDGMDIARELYNNNLTPGELCLQLLDAGKKFLPDFYRSDLQNELDRIWEKQKEYYPEI
LTDVLKEELRGKKRDAVWAICAKYFVWKENYTEWNKEKGKTEQQEREHKLEGIYSKRKRDEAKRENLQWR
VNGLKEKLSLEQLVIVFQEMNTQINNSSGYLGAISDRSKELYFNKQTVGQYQMEMLDKNPNASLRNMVFY
RQDYLDEFNMLWEKQAVYHKELTEELKKEIRDIIIFYQRRLKSQKGLIGFCEFESRQIEVDIDGKKKIKT
VGNRVISRSSPLFQEFKIWQILNNIEVTVVGKKRKRRKLKENYSALFEELNDAEQLELNGSRRLCQEEKE
LLAQELFIRDKMTKSEVLKLLFDNPQELDLNFKTIDGNKTGYALFQAYSKMIEMSGHEPVDFKKPVEKVV
EYIKAVFDLLNWNTDILGFNSNEELDNQPYYKLWHLLYSFEGDNTPTGNGRLIQKMTELYGFEKEYATIL
ANVSFQDDYGSLSAKAIHKILPHLKEGNRYDVACVYAGYRHSESSLTREEIANKVLKDRLMLLPKNSLHN
PVVEKILNQMVNVINVIIDIYGKPDEIRVELARELKKNAKEREELTKSIAQTTKAHEEYKTLLQTEFGLT
NVSRTDILRYKLYKELESCGYKTLYSNTYISREKLFSKEFDIEHIIPQARLFDDSFSNKTLEARSVNIEK
GNKTAYDFVKEKFGESGADNSLEHYLNNIEDLFKSGKISKTKYNKLKMAEQDIPDGFIERDLRNTQYIAK
KALSMLNEISHRVVATSGSVTDKLREDWQLIDVMKELNWEKYKALGLVEYFEDRDGRQIGRIKDWTKRND
HRHHAMDALTVAFTKDVFIQYFNNKNASLDPNANEHAIKNKYFQNGRAIAPMPLREFRAEAKKHLENTLI
SIKAKNKVITGNINKTRKKGGVNKNMQQTPRGQLHLETIYGSGKQYLTKEEKVNASFDMRKIGTVSKSAY
RDALLKRLYENDNDPKKAFAGKNSLDKQPIWLDKEQMRKVPEKVKIVTLEAIYTIRKEISPDLKVDKVID

VGVRKILIDRLNEYGNDAKKAFSNLDKNPIWLNKEKGISIKRVTISGISNAQSLHVKKDKDGKPILDENG
RNIPVDFVNTGNNHHVAVYYRPVIDKRGQLVVDEAGNPKYELEEVVVSFFEAVTRANLGLPIIDKDYKTT
EGWQFLFSMKQNEYFVFPNEKTGFNPKEIDLLDVENYGLISPNLFRVQKFSLKNYVFRHHLETTIKDTSS
ILRGITWIDFRSSKGLDTIVKVRVNHIGQIVSVGEY

SEQ ID NO:308
MSRKNYVDDYAISLDIGNASVGWSAFTPNYRLVRAKGHELIGVRLFDPADTAESRRMARTTRRRYSRRRW
RLRLLDALFDQALSEIDPSFLARRKYSWVHPDDENNADCWYGSVLFDSNEQDKRFYEKYPTIYHLRKALM
EDDSQHDIREIYLAIHHMVKYRGNFLVEGTLESSNAFKEDELLKLLGRITRYEMSEGEQNSDIEQDDENK
LVAPANGQLADALCATRGSRSMRVDNALEALSAVNDLSREQRAIVKAIFAGLEGNKLDLAKIFVSKEFSS
ENKKILGIYFNKSDYEEKCVQIVDSGLLDDEEREFLDRMQGQYNAIALKQLLGRSTSVSDSKCASYDAHR
ANWNLIKLQLRTKENEKDINENYGILVGWKIDSGQRKSVRGESAYENMRKKANVFFKKMIETSDLSETDK
NRLIHDIEEDKLFPIQRDSDNGVIPHQLHQNELKQIIKKQGKYYPFLLDAFEKDGKQINKIEGLLTFRVP
YFVGPLVVPEDLQKSDNSENHWMVRKKKGEITPWNFDEMVDKDASGRKFIERLVGTDSYLLGEPTLPKNS
LLYQEYEVLNELNNVRLSVRTGNHWNDKRRMRLGREEKTLLCQRLFMKGQTVTKRTAENLLRKEYGRTYE
LSGLSDESKFTSSLSTYGKMCRIFGEKYVNEHRDLMEKIVELQTVFEDKETLLHQLRQLEGISEADCALL
VNTHYTGWGRLSRKLLTTKAGECKISDDFAPRKHSIIEIMRAEDRNLMEIITDKQLGFSDWIEQENLGAE
NGSSLMEVVDDLRVSPKVKRGIIQSIRLIDDISKAVGKRPSRIFLELADDIQPSGRTISRKSRLQDLYRN
ANLGKEFKGIADELNACSDKDLQDDRLFLYYTQLGKDMYTGEELDLDRLSSAYDIDHIIPQAVTQNDSID
NRVLVARAENARKTDSFTYMPQIADRMRNFWQILLDNGLISRVKFERLTRQNEFSEREKERFVQRSLVET
RQIMKNVATLMRQRYGNSAAVIGLNAELTKEMHRYLGFSHKNRDINDYHHAQDALCVGIAGQFAANRGFF
ADGEVSDGAQNSYNQYLRDYLRGYREKLSAEDRKQGRAFGFIVGSMRSQDEQKRVNPRTGEVVWSEEDKD
YLRKVMNYRKMLVTQKVGDDFGALYDETRYAATDPKGIKGIPFDGAKQDTSLYGGFSSAKPAYAVLIESK
GKTRLVNVTMQEYSLLGDRPSDDELRKVLAKKKSEYAKANILLRHVPKMQLIRYGGGLMVIKSAGELNNA
QQLWLPYEEYCYFDDLSQGKGSLEKDDLKKLLDSILGSVQCLYPWHRFTEEELADLHVAFDKLPEDEKKN
VITGIVSALHADAKTANLSIVGMTGSWRRMNNKSGYTFSDEDEFIFQSPSGLFEKRVTVGELKRKAKKEV
NSKYRTNEKRLPTLSGASQP

SEQ ID NO:309
METQTSNQLITSHLKDYPKQDYFVGLDIGTNSVGWAVTNTSYELLKFHSHKMWGSRLFEEGESAVTRRGF
RSMRRRLERRKLRLKLLEELFADAMAQVDSTFFIRLHESKYHYEDKTTGHSSKHILFIDEDYTDQDYFTE
YPTIYHLRKDLMENGTDDIRKLFLAVHHILKYRGNFLYEGATFNSNAFTFEDVLKQALVNITFNCFDTNS
AISSISNILMESGKTKSDKAKAIERLVDTYTVFDEVNTPDKPQKEQVKEDKKTLKAFANLVLGLSANLID
LFGSVEDIDDDLKKLQIVGDTYDEKRDELAKVWGDEIHIIDDCKSVYDAIILMSIKEPGLTISQSKVKAF
DKHKEDLVILKSLLKLDRNVYNEMFKSDKKGLHNYVHYIKQGRTEETSCSREDFYKYTKKIVEGLADSKD
KEYILNEIELQTLLPLQRIKDNGVIPYQLHLEELKVILDKCGPKFPFLHTVSDGFSVTEKLIKMLEFRIP
YYVGPLNTHHNIDNGGFSWAVRKQAGRVTPWNFEEKIDREKSAAAFIKNLTNKCTYLFGEDVLPKSSLLY
SEFMLLNELNNVRIDGKALAQGVKQHLIDSIFKQDHKKMTKNRIELFLKDNNYITKKHKPEITGLDGEIK
NDLTSYRDMVRILGNNFDVSMAEDIITDITIFGESKKMLRQTLRNKFGSQLNDETIKKLSKLRYRDWGRL
SKKLLKGIDGCDKAGNGAPKTIIELMRNDSYNLMEILGDKFSFMECIEEENAKLAQGQVVNPHDIIDELA
LSPAVKRAVWQALRIVDEVAHIKKALPSRIFVEVARTNKSEKKKKDSRQKRLSDLYSAIKKDDVLQSGLQ
DKEFGALKSGLANYDDAALRSKKLYLYYTQMGRCAYTGNIIDLNQLNTDNYDIDHIYPRSLTKDDSFDNL
VLCERTANAKKSDIYPIDNRIQTKQKPFWAFLKHQGLISERKYERLTRIAPLTADDLSGFIARQLVETNQ
SVKATTTLLRRLYPDIDVVFVKAENVSDFRHNNNFIKVRSLNHHHHAKDAYLNIVVGNVYHEKFTRNFRL
FFKKNGANRTYNLAKMFNYDVICTNAQDGKAWDVKTSMNTVKKMMASNDVRVTRRLLEQSGALADATIYK
ASVAAKAKDGAYIGMKTKYSVFADVTKYGGMTKIKNAYSIIVQYTGKKGEEIKEIVPLPIYLINRNATDI
ELIDYVKSVIPKAKDISIKYRKLCINQLVKVNGFYYYLGGKTNDKIYIDNAIELVVPHDIATYIKLLDKY
DLLRKENKTLKASSITTSIYNINTSTVVSLNKVGIDVFDYFMSKLRTPLYMKMKGNKVDELSSTGRSKFI
KMTLEEQSIYLLEVLNLLTNSKTTFDVKPLGITGSRSTIGVKIHNLDEFKIINESITGLYSNEVTIV

SEQ ID NO:310

MTKLNQPYGIGLDIGSNSIGFAVVDANSHLLRLKGETAIGARLFREGQSAADRRGSRTTRRRLSRTRWRL
SFLRDFFAPHITKIDPDFFLRQKYSEISPKDKDRFKYEKRLFNDRTDAEFYEDYPSMYHLRLHLMTHTHK
ADPREIFLAIHHILKSRGHFLTPGAAKDFNTDKVDLEDIFPALTEAYAQVYPDLELTFDLAKADDFKAKL
LDEQATPSDTQKALVNLLLSSDGEKEIVKKRKQVLTEFAKAITGLKTKFNLALGTEVDEADASNWQFSMG
QLDDKWSNIETSMTDQGTEIFEQIQELYRARLLNGIVPAGMSLSQAKVADYGQHKEDLELFKTYLKKLND
HELAKTIRGLYDRYINGDDAKPFLREDFVKALTKEVTAHPNEVSEQLLNRMGQANFMLKQRTKANGAIPI
QLQQRELDQIIANQSKYYDWLAAPNPVEAHRWKMPYQLDELLNFHIPYYVGPLITPKQQAESGENVFAWM
VRKDPSGNITPYNFDEKVDREASANTFIQRMKTTDTYLIGEDVLPKQSLLYQKYEVLNELNNVRINNECL
GTDQKQRLIREVFERHSSVTIKQVADNLVAHGDFARRPEIRGLADEKRFLSSLSTYHQLKEILHEAIDDP
TKLLDIENIITWSTVFEDHTIFETKLAEIEWLDPKKINELSGIRYRGWGQFSRKLLDGLKLGNGHTVIQE
LMLSNHNLMQILADETLKETMTELNQDKLKTDDIEDVINDAYTSPSNKKALRQVLRVVEDIKHAANGQDP
SWLFIETADGTGTAGKRTQSRQKQIQTVYANAAQELIDSAVRGELEDKIADKASFTDRLVLYFMQGGRDI
YTGAPLNIDQLSHYDIDHILPQSLIKDDSLDNRVLVNATINREKNNVFASTLFAGKMKATWRKWHEAGLI
SGRKLRNLMLRPDEIDKFAKGFVARQLVETRQIIKLTEQIAAAQYPNTKIIAVKAGLSHQLREELDFPKN
RDVNHYHHAFDAFLAARIGTYLLKRYPKLAPFFTYGEFAKVDVKKFREFNFIGALTHAKKNIIAKDTGEI
VWDKERDIRELDRIYNFKRMLITHEVYFETADLFKQTIYAAKDSKERGGSKQLIPKKQGYPTQVYGGYTQ
ESGSYNALVRVAEADTTAYQVIKISAQNASKIASANLKSREKGKQLLNEIVVKQLAKRRKNWKPSANSFK
IVIPRFGMGTLFQNAKYGLFMVNSDTYYRNYQELWLSRENQKLLKKLFSIKYEKTQMNHDALQVYKAIID
QVEKFFKLYDINQFRAKLSDAIERFEKLPINTDGNKIGKTETLRQILIGLQANGTRSNVKNLGIKTDLGL
LQVGSGIKLDKDTQIVYQSPSGLFKRRIPLADL

SEQ ID NO:311
MTKEYYLGLDVGTNSVGWAVTDSQYNLCKFKKKDMWGIRLFESANTAKDRRLQRGNRRRLERKKQRIDLL
QEIFSPEICKIDPTFFIRLNESRLHLEDKSNDFKYPLFIEKDYSDIEYYKEFPTIFHLRKHLIESEEKQD
IRLIYLALHNIIKTRGHFLIDGDLQSAKQLRPILDTFLLSLQEEQNLSVSLSENQKDEYEEILKNRSIAK
SEKVKKLKNLFEISDELEKEEKKAQSAVIENFCKFIVGNKGDVCKFLRVSKEELEIDSFSFSEGKYEDDI
VKNLEEKVPEKVYLFEQMKAMYDWNILVDILETEEYISFAKVKQYEKHKTNLRLLRDIILKYCTKDEYNR
MFNDEKEAGSYTAYVGKLKKNNKKYWIEKKRNPEEFYKSLGKLLDKIEPLKEDLEVLTMMIEECKNHTLL
PIQKNKDNGVIPHQVHEVELKKILENAKKYYSFLTETDKDGYSVVQKIESIFRFRIPYYVGPLSTRHQEK
GSNVWMVRKPGREDRIYPWNMEEIIDFEKSNENFITRMTNKCTYLIGEDVLPKHSLLYSKYMVLNELNNV
KVRGKKLPTSLKQKVFEDLFENKSKVTGKNLLEYLQIQDKDIQIDDLSGFDKDFKTSLKSYLDFKKQIFG
EEIEKESIQNMIEDIIKWITIYGNDKEMLKRVIRANYSNQLTEEQMKKITGFQYSGWGNFSKMFLKGISG
SDVSTGETFDIITAMWETDNNLMQILSKKFTFMDNVEDFNSGKVGKIDKITYDSTVKEMFLSPENKRAVW
QTIQVAEEIKKVMGCEPKKIFIEMARGGEKVKKRTKSRKAQLLELYAACEEDCRELIKEIEDRDERDFNS
MKLFLYYTQFGKCMYSGDDIDINELIRGNSKWDRDHIYPQSKIKDDSIDNLVLVNKTYNAKKSNELLSED
IQKKMHSFWLSLLNKKLITKSKYDRLTRKGDFTDEELSGFIARQLVETRQSTKAIADIFKQIYSSEVVYV
KSSLVSDFRKKPLNYLKSRRVNDYHHAKDAYLNIVVGNVYNKKFTSNPIQWMKKNRDTNYSLNKVFEHDV
VINGEVIWEKCTYHEDTNTYDGGTLDRIRKIVERDNILYTEYAYCEKGELFNATIQNKNGNSTVSLKKGL
DVKKYGGYFSANTSYFSLIEFEDKKGDRARHIIGVPIYIANMLEHSPSAFLEYCEQKGYQNVRILVEKIK
KNSLLIINGYPLRIRGENEVDTSFKRAIQLKLDQKNYELVRNIEKFLEKYVEKKGNYPIDENRDHITHEK
MNQLYEVLLSKMKKFNKKGMADPSDRIEKSKPKFIKLEDLIDKINVINKMLNLLRCDNDTKADLSLIELP
KNAGSFVVKKNTIGKSKIILVNQSVTGLYENRREL

SEQ ID NO:312
MARDYSVGLDIGTSSVGWAAIDNKYHLIRAKSKNLIGVRLFDSAVTAEKRRGYRTTRRRLSRRHWRLRLL
NDIFAGPLTDFGDENFLARLKYSWVHPQDQSNQAHFAAGLLFDSKEQDKDFYRKYPTIYHLRLALMNDDQ
KHDLREVYLAIHHLVKYRGHFLIEGDVKADSAFDVHTFADAIQRYAESNNSDENLLGKIDEKKLSAALTD
KHGSKSQRAETAETAFDILDLQSKKQIQAILKSVVGNQANLMAIFGLDSSAISKDEQKNYKFSFDDADID
EKIADSEALLSDTEFEFLCDLKAAFDGLTLKMLLGDDKTVSAAMVRRFNEHQKDWEYIKSHIRNAKNAGN
GLYEKSKKFDGINAAYLALQSDNEDDRKKAKKIFQDEISSADIPDDVKADFLKKIDDDQFLPIQRTKNNG
TIPHQLHRNELEQIIEKQGIYYPFLKDTYQENSHELNKITALINFRVPYYVGPLVEEEQKIADDGKNIPD
PTNHWMVRKSNDTITPWNLSQVVDLDKSGRRFIERLTGTDTYLIGEPTLPKNSLLYQKFDVLQELNNIRV

SGRRLDIRAKQDAFEHLFKVQKTVSATNLKDFLVQAGYISEDTQIEGLADVNGKNFNNALTTYNYLVSVL
GREFVENPSNEELLEEITELQTVFEDKKVLRRQLDQLDGLSDHNREKLSRKHYTGWGRISKKLLTTKIVQ
NADKIDNQTFDVPRMNQSIIDTLYNTKMNLMEIINNAEDDFGVRAWIDKQNTTDGDEQDVYSLIDELAGP
KEIKRGIVQSFRILDDITKAVGYAPKRVYLEFARKTQESHLTNSRKNQLSTLLKNAGLSELVTQVSQYDA
AALQNDRLYLYFLQQGKDMYSGEKLNLDNLSNYDIDHIIPQAYTKDNSLDNRVLVSNITNRRKSDSSNYL
PALIDKMRPFWSVLSKQGLLSKHKFANLTRTRDFDDMEKERFIARSLVETRQIIKNVASLIDSHFGGETK
AVAIRSSLTADMRRYVDIPKNRDINDYHHAFDALLFSTVGQYTENSGLMKKGQLSDSAGNQYNRYIKEWI
HAARLNAQSQRVNPFGFVVGSMRNAAPGKLNPETGEITPEENADWSIADLDYLHKVMNFRKITVTRRLKD
QKGQLYDESRYPSVLHDAKSKASINFDKHKPVDLYGGFSSAKPAYAALIKFKNKFRLVNVLRQWTYSDKN
SEDYILEQIRGKYPKAEMVLSHIPYGQLVKKDGALVTISSATELHNFEQLWLPLADYKLINTLLKTKEDN
LVDILHNRLDLPEMTIESAFYKAFDSILSFAFNRYALHQNALVKLQAHRDDFNALNYEDKQQTLERILDA
LHASPASSDLKKINLSSGFGRLFSPSHFTLADTDEFIFQSVTGLFSTQKTVAQLYQETK

SEQ ID NO:313
MVYDVGLDIGTGSVGWVALDENGKLARAKGKNLVGVRLFDTAQTAADRRGFRTTRRRLSRRKWRLRLLDE
LFSAEINEIDSSFFQRLKYSYVHPKDEENKAHYYGGYLFPTEEETKKFHRSYPTIYHLRQELMAQPNKRF
DIREIYLAIHHLVKYRGHFLSSQEKITIGSTYNPEDLANAIEVYADEKGLSWELNNPEQLTEIISGEAGY
GLNKSMKADEALKLFEFDNNQDKVAIKTLLAGLTGNQIDFAKLFGKDISDKDEAKLWKLKLDDEALEEKS
QTILSQLTDEEIELFHAVVQAYDGFVLIGLLNGADSVSAAMVQLYDQHREDRKLLKSLAQKAGLKHKRFS
EIYEQLALATDEATIKNGISTARELVEESNLSKEVKEDTLRRLDENEFLPKQRTKANSVIPHQLHLAELQ
KILQNQGQYYPFLLDTFEKEDGQDNKIEELLRFRIPYYVGPLVTKKDVEHAGGDADNHWVERNEGFEKSR
VTPWNFDKVFNRDKAARDFIERLTGNDTYLIGEKTLPQNSLRYQLFTVLNELNNVRVNGKKFDSKTKADL
INDLFKARKTVSLSALKDYLKAQGKGDVTITGLADESKFNSSLSSYNDLKKTFDAEYLENEDNQETLEKI
IEIQTVFEDSKIASRELSKLPLDDDQVKKLSQTHYTGWGRLSEKLLDSKIIDERGQKVSILDKLKSTSQN
FMSIINNDKYGVQAWITEQNTGSSKLTFDEKVNELTTSPANKRGIKQSFAVLNDIKKAMKEEPRRVYLEF
AREDQTSVRSVPRYNQLKEKYQSKSLSEEAKVLKKTLDGNKNKMSDDRYFLYFQQQGKDMYTGRPINFER
LSQDYDIDHIIPQAFTKDDSLDNRVLVSRPENARKSDSFAYTDEVQKQDGSLWTSLLKSGFINRKKYERL
TKAGKYLDGQKTGFIARQLVETRQIIKNVASLIEGEYENSKAVAIRSEITADMRLLVGIKKHREINSFHH
AFDALLITAAGQYMQNRYPDRDSTNVYNEFDRYTNDYLKNLRQLSSRDEVRRLKSFGFVVGTMRKGNEDW
SEENTSYLRKVMMFKNILTTKKTEKDRGPLNKETIFSPKSGKKLIPLNSKRSDTALYGGYSNVYSAYMTL
VRANGKNLLIKIPISIANQIEVGNLKINDYIVNNPAIKKFEKILISKLPLGQLVNEDGNLIYLASNEYRH
NAKQLWLSTTDADKIASISENSSDEELLEAYDILTSENVKNRFPFFKKDIDKLSQVRDEFLDSDKRIAVI
QTILRGLQIDAAYQAPVKIISKKVSDWHKLQQSGGIKLSDNSEMIYQSATGIFETRVKISDLL

SEQ ID NO:314
IVDYCIGLDLGTGSVGWAVVDMNHRLMKRNGKHLWGSRLFSNAETAANRRASRSIRRRYNKRRERIRLLR
AILQDMVLEKDPTFFIRLEHTSFLDEEDKAKYLGTDYKDNYNLFIDEDFNDYTYYHKYPTIYHLRKALCE
STEKADPRLIYLALHHIVKYRGNFLYEGQKFNMDASNIEDKLSDIFTQFTSFNNIPYEDDEKKNLEILEI
LKKPLSKKAKVDEVMTLIAPEKDYKSAFKELVTGIAGNKMNVTKMILCEPIKQGDSEIKLKFSDSNYDDQ
FSEVEKDLGEYVEFVDALHNVYSWVELQTIMGATHTDNASISEAMVSRYNKHHDDLKLLKDCIKNNVPNK
YFDMFRNDSEKSKGYYNYINRPSKAPVDEFYKYVKKCIEKVDTPEAKQILNDIELENFLLKQNSRTNGSV
PYQMQLDEMIKIIDNQAEYYPILKEKREQLLSILTFRIPYYFGPLNETSEHAWIKRLEGKENQRILPWNY
QDIVDVDATAEGFIKRMRSYCTYFPDEEVLPKNSLIVSKYEVYNELNKIRVDDKLLEVDVKNDIYNELFM
KNKTVTEKKLKNWLVNNQCCSKDAEIKGFQKENQFSTSLTPWIDFTNIFGKIDQSNFDLIENIIYDLTVF
EDKKIMKRRLKKKYALPDDKVKQILKLKYKDWSRLSKKLLDGIVADNRFGSSVTVLDVLEMSRLNLMEII
NDKDLGYAQMIEEATSCPEDGKFTYEEVERLAGSPALKRGIWQSLQIVEEITKVMKCRPKYIYIEFERSE
EAKERTESKIKKLENVYKDLDEQTKKEYKSVLEELKGFDNTKKISSDSLFLYFTQLGKCMYSGKKLDIDS
LDKYQIDHIVPQSLVKDDSFDNRVLVVPSENQRKLDDLVVPFDIRDKMYRFWKLLFDHELISPKKFYSLI
KTEYTERDEERFINRQLVETRQITKNVTQIIEDHYSTTKVAAIRANLSHEFRVKNHIYKNRDINDYHHAH
DAYIVALIGGFMRDRYPNMHDSKAVYSEYMKMFRKNKNDQKRWKDGFVINSMNYPYEVDGKLIWNPDLIN
EIKKCFYYKDCYCTTKLDQKSGQLFNLTVLSNDAHADKGVTKAVVPVNKNRSDVHKYGGFSGLQYTIVAI
EGQKKKGKKTELVKKISGVPLHLKAASINEKINYIEEKEGLSDVRIIKDNIPVNQMIEMDGGEYLLTSPT

EYVNARQLVLNEKQCALIADIYNAIYKQDYDNLDDILMIQLYIELTNKMKVLYPAYRGIAEKFESMNENY
VVISKEEKANIIKQMLIVMHRGPQNGNIVYDDFKISDRIGRLKTKNHNLNNIVFISQSPTGIYTKKYKL

SEQ ID NO:315
MKSEKKYYIGLDVGTNSVGWAVTDEFYNILRAKGKDLWGVRLFEKADTAANTRIFRSGRRRNDRKGMRLQ
ILREIFEDEIKKVDKDFYDRLDESKFWAEDKKVSGKYSLFNDKNFSDKQYFEKFPTIFHLRKYLMEEHGK
VDIRYYFLAINQMMKRRGHFLIDGQISHVTDDKPLKEQLILLINDLLKIELEEELMDSIFEILADVNEKR
TDKKNNLKELIKGQDFNKQEGNILNSIFESIVTGKAKIKNIISDEDILEKIKEDNKEDFVLTGDSYEENL
QYFEEVLQENITLFNTLKSTYDFLILQSILKGKSTLSDAQVERYDEHKKDLEILKKVIKKYDEDGKLFKQ
VFKEDNGNGYVSYIGYYLNKNKKITAKKKISNIEFTKYVKGILEKQCDCEDEDVKYLLGKIEQENFLLKQ
ISSINSVIPHQIHLFELDKILENLAKNYPSFNNKKEEFTKIEKIRKTFTFRIPYYVGPLNDYHKNNGGNA
WIFRNKGEKIRPWNFEKIVDLHKSEEEFIKRMLNQCTYLPEETVLPKSSILYSEYMVLNELNNLRINGKP
LDTDVKLKLIEELFKKKTKVTLKSIRDYMVRNNFADKEDFDNSEKNLEIASNMKSYIDFNNILEDKFDVE
MVEDLIEKITIHTGNKKLLKKYIEETYPDLSSSQIQKIINLKYKDWGRLSRKLLDGIKGTKKETEKTDTV
INFLRNSSDNLMQIIGSQNYSFNEYIDKLRKKYIPQEISYEVVENLYVSPSVKKMIWQVIRVTEEITKVM
GYDPDKIFIEMAKSEEEKKTTISRKNKLLDLYKAIKKDERDSQYEKLLTGLNKLDDSDLRSRKLYLYYTQ
MGRDMYTGEKIDLDKLFDSTHYDKDHIIPQSMKKDDSIINNLVLVNKNANQTTKGNIYPVPSSIRNNPKI
YNYWKYLMEKEFISKEKYNRLIRNTPLTNEELGGFINRQLVETRQSTKAIKELFEKFYQKSKIIPVKASL
ASDLRKDMNTLKSREVNDLHHAHDAFLNIVAGDVWNREFTSNPINYVKENREGDKVKYSLSKDFTRPRKS
KGKVIWTPEKGRKLIVDTLNKPSVLISNESHVKKGELFNATIAGKKDYKKGKIYLPLKKDDRLQDVSKYG
GYKAINGAFFFLVEHTKSKKRIRSIELFPLHLLSKFYEDKNTVLDYAINVLQLQDPKIIIDKINYRTEII
IDNFSYLISTKSNDGSITVKPNEQMYWRVDEISNLKKIENKYKKDAILTEEDRKIMESYIDKIYQQFKAG
KYKNRRTTDTIIEKYEIIDLDTLDNKQLYQLLVAFISLSYKTSNNAVDFTVIGLGTECGKPRITNLPDNT
YLVYKSITGIYEKRIRIK

SEQ ID NO:316
MKLRGIEDDYSIGLDMGTSSVGWAVTDERGTLAHFKRKPTWGSRLFREAQTAAVARMPRGQRRRYVRRRW
RLDLLQKLFEQQMEQADPDFFIRLRQSRLLRDDRAEEHADYRWPLFNDCKFTERDYYQRFPTIYHVRSWL
METDEQADIRLIYLALHNIVKHRGNFLREGQSLSAKSARPDEALNHLRETLRVWSSERGFECSIADNGSI
LAMLTHPDLSPSDRRKKIAPLFDVKSDDAAADKKLGIALAGAVIGLKTEFKNIFGDFPCEDSSIYLSNDE
AVDAVRSACPDDCAELFDRLCEVYSAYVLQGLLSYAPGQTISANMVEKYRRYGEDLALLKKLVKIYAPDQ
YRMFFSGATYPGTGIYDAAQARGYTKYNLGPKKSEYKPSESMQYDDFRKAVEKLFAKTDARADERYRMMM
DRFDKQQFLRRLKTSDNGSIYHQLHLEELKAIVENQGRFYPFLKRDADKLVSLVSFRIPYYVGPLSTRNA
RTDQHGENRFAWSERKPGMQDEPIFPWNWESIIDRSKSAEKFILRMTGMCTYLQQEPVLPKSSLLYEEFC
VLNELNGAHWSIDGDDEHRFDAADREGIIEELFRRKRTVSYGDVAGWMERERNQIGAHVCGGQGEKGFES
KLGSYIFFCKDVFKVERLEQSDYPMIERIILWNTLFEDRKILSQRLKEEYGSRLSAEQIKTICKKRFTGW
GRLSEKFLTGITVQVDEDSVSIMDVLREGCPVSGKRGRAMVMMEILRDEELGFQKKVDDFNRAFFAENAQ
ALGVNELPGSPAVRRSLNQSIRIVDEIASIAGKAPANIFIEVTRDEDPKKKGRRTKRRYNDLKDALEAFK
KEDPELWRELCETAPNDMDERLSLYFMQRGKCLYSGRAIDIHQLSNAGIYEVDHIIPRTYVKDDSLENKA
LVYREENQRKTDMLLIDPEIRRRMSGYWRMLHEAKLIGDKKFRNLLRSRIDDKALKGFIARQLVETGQMV
KLVRSLLEARYPETNIISVKASISHDLRTAAELVKCREANDFHHAHDAFLACRVGLFIQKRHPCVYENPI
GLSQVVRNYVRQQADIFKRCRTIPGSSGFIVNSFMTSGFDKETGEIFKDDWDAEAEVEGIRRSLNFRQCF
ISRMPFEDHGVFWDATIYSPRAKKTAALPLKQGLNPSRYGSFSREQFAYFFIYKARNPRKEQTLFEFAQV
PVRLSAQIRQDENALERYARELAKDQGLEFIRIERSKILKNQLIEIDGDRLCITGKEEVRNACELAFAQD
EMRVIRMLVSEKPVSRECVISLFNRILLHGDQASRRLSKQLKLALLSEAFSEASDNVQRNVVLGLIAIFN
GSTNMVNLSDIGGSKFAGNVRIKYKKELASPKVNVHLIDQSVTGMFERRTKIGL

SEQ ID NO:317
MENKQYYIGLDVGTNSVGWAVTDTSYNLLRAKGKDMWGARLFEKANTAAERRTKRTSRRRSEREKARKAM
LKELFADEINRVDPSFFIRLEESKFFLDDRSENNRQRYTLFNDATFTDKDYYEKYKTIFHLRSALINSDE
KFDVRLVFLAILNLFSHRGHFLNASLKGDGDIQGMDVFYNDLVESCEYFEIELPRITNIDNFEKILSQKG
KSRTKILEELSEELSISKKDKSKYNLIKLISGLEASVVELYNIEDIQDENKKIKIGFRESDYEESSLKVK

EIIGDEYFDLVERAKSVHDMGLLSNIIGNSKYLCEARVEAYENHHKDLLKIKELLKKYDKKAYNDMFRKM
TDKNYSAYVGSVNSNIAKERRSVDKRKIEDLYKYIEDTALKNIPDDNKDKIEILEKIKLGEFLKKQLTAS
NGVIPNQLQSRELRAILKKAENYLPFLKEKGEKNLTVSEMIIQLFEFQIPYYVGPLDKNPKKDNKANSWA
KIKQGGRILPWNFEDKVDVKGSRKEFIEKMVRKCTYISDEHTLPKQSLLYEKFMVLNEINNIKIDGEKIS
VEAKQKIYNDLFVKGKKVSQKDIKKELISLNIMDKDSVLSGTDTVCNAYLSSIGKFTGVFKEEINKQSIV
DMIEDIIFLKTVYGDEKRFVKEEIVEKYGDEIDKDKIKRILGFKFSNWGNLSKSFLELEGADVGTGEVRS
IIQSLWETNFNLMELLSSRFTYMDELEKRVKKLEKPLSEWTIEDLDDMYLSSPVKRMIWQSMKIVDEIQT
VIGYAPKRIFVEMTRSEGEKVRTKSRKDRLKELYNGIKEDSKQWVKELDSKDESYFRSKKMYLYYLQKGR
CMYSGEVIELDKLMDDNLYDIDHIYPRSFVKDDSLDNLVLVKKEINNRKQNDPITPQIQASCQGFWKILH
DQGFMSNEKYSRLTRKTQEFSDEEKLSFINRQIVETGQATKCMAQILQKSMGEDVDVVFSKARLVSEFRH
KFELFKSRLINDFHHANDAYLNIVVGNSYFVKFTRNPANFIKDARKNPDNPVYKYHMDRFFERDVKSKSE
VAWIGQSEGNSGTIVIVKKTMAKNSPLITKKVEEGHGSITKETIVGVKEIKFGRNKVEKADKTPKKPNLQ
AYRPIKTSDERLCNILRYGGRTSISISGYCLVEYVKKRKTIRSLEAIPVYLGRKDSLSEEKLLNYFRYNL
NDGGKDSVSDIRLCLPFISTNSLVKIDGYLYYLGGKNDDRIQLYNAYQLKMKKEEVEYIRKIEKAVSMSK
FDEIDREKNPVLTEEKNIELYNKIQDKFENTVFSKRMSLVKYNKKDLSFGDFLKNKKSKFEEIDLEKQCK
VLYNIIFNLSNLKEVDLSDIGGSKSTGKCRCKKNITNYKEFKLIQQSITGLYSCEKDLMTI

SEQ ID NO:318
MKNLKEYYIGLDIGTASVGWAVTDESYNIPKFNGKKMWGVRLFDDAKTAEERRTQRGSRRRLNRRKERIN
LLQDLFATEISKVDPNFFLRLDNSDLYREDKDEKLKSKYTLFNDKDFKDRDYHKKYPTIHHLIMDLIEDE
GKKDIRLLYLACHYLLKNRGHFIFEGQKFDTKNSFDKSINDLKIHLRDEYNIDLEFNNEDLIEIITDTTL
NKTNKKKELKNIVGDTKFLKAISAIMIGSSQKLVDLFEDGEFEETTVKSVDFSTTAFDDKYSEYEEALGD
TISLLNILKSIYDSSILENLLKDADKSKDGNKYISKAFVKKFNKHGKDLKTLKRIIKKYLPSEYANIFRN
KSINDNYVAYTKSNITSNKRTKASKFTKQEDFYKFIKKHLDTIKETKLNSSENEDLKLIDEMLTDIEFKT
FIPKLKSSDNGVIPYQLKLMELKKILDNQSKYYDFLNESDEYGTVKDKVESIMEFRIPYYVGPLNPDSKY
AWIKRENTKITPWNFKDIVDLDSSREEFIDRLIGRCTYLKEEKVLPKASLIYNEFMVLNELNNLKLNEFL
ITEEMKKAIFEELFKTKKKVTLKAVSNLLKKEFNLTGDILLSGTDGDFKQGLNSYIDFKNIIGDKVDRDD
YRIKIEEIIKLIVLYEDDKTYLKKKIKSAYKNDFTDDEIKKIAALNYKDWGRLSKRFLTGIEGVDKTTGE
KGSIIYFMREYNLNLMELMSGHYTFTEEVEKLNPVENRELCYEMVDELYLSPSVKRMLWQSLRVVDEIKR
IIGKDPKKIFIEMARAKEAKNSRKESRKNKLLEFYKFGKKAFINEIGEERYNYLLNEINSEEESKFRWDN
LYLYYTQLGRCMYSLEPIDLADLKSNNIYDQDHIYPKSKIYDDSLENRVLVKKNLNHEKGNQYPIPEKVL
NKNAYGFWKILFDKGLIGQKKYTRLTRRTPFEERELAEFIERQIVETRQATKETANLLKNICQDSEIVYS
KAENASRFRQEFDIIKCRTVNDLHHMHDAYLNIVVGNVYNTKFTKNPLNFIKDKDNVRSYNLENMFKYDV
VRGSYTAWIADDSEGNVKAATIKKVKRELEGKNYRFTRMSYIGTGGLYDQNLMRKGKGQIPQKENTNKSN
IEKYGGYNKASSAYFALIESDGKAGRERTLETIPIMVYNQEKYGNTEAVDKYLKDNLELQDPKILKDKIK
INSLIKLDGFLYNIKGKTGDSLSIAGSVQLIVNKEEQKLIKKMDKFLVKKKDNKDIKVTSFDNIKEEELI
KLYKTLSDKLNNGIYSNKRNNQAKNISEALDKFKEISIEEKIDVLNQIILLFQSYNNGCNLKSIGLSAKT
GVVFIPKKLNYKECKLINQSITGLFENEVDLLNL

SEQ ID NO:319
MGKMYYLGLDIGTNSVGYAVTDPSYHLLKFKGEPMWGAHVFAAGNQSAERRSFRTSRRRLDRRQQRVKLV
QEIFAPVISPIDPRFFIRLHESALWRDDVAETDKHIFFNDPTYTDKEYYSDYPTIHHLIVDLMESSEKHD
PRLVYLAVAWLVAHRGHFLNEVDKDNIGDVLSFDAFYPEFLAFLSDNGVSPWVCESKALQATLLSRNSVN
DKYKALKSLIFGSQKPEDNFDANISEDGLIQLLAGKKVKVNKLFPQESNDASFTLNDKEDAIEEILGTLT
PDECEWIAHIRRLFDWAIMKHALKDGRTISESKVKLYEQHHHDLTQLKYFVKTYLAKEYDDIFRNVDSET
TKNYVAYSYHVKEVKGTLPKNKATQEEFCKYVLGKVKNIECSEADKVDFDEMIQRLTDNSFMPKQVSGEN
RVIPYQLYYYELKTILNKAASYLPFLTQCGKDAISNQDKLLSIMTFRIPYFVGPLRKDNSEHAWLERKAG
KIYPWNFNDKVDLDKSEEAFIRRMTNTCTYYPGEDVLPLDSLIYEKFMILNEINNIRIDGYPISVDVKQQ
VFGLFEKKRRVTVKDIQNLLLSLGALDKHGKLTGIDTTIHSNYNTYHHFKSLMERGVLTRDDVERIVERM
TYSDDTKRVRLWLNNNYGTLTADDVKHISRLRKHDFGRLSKMFLTGLKGVHKETGERASILDFMWNTNDN
LMQLLSECYTFSDEITKLQEAYYAKAQLSLNDFLDSMYISNAVKRPIYRTLAVVNDIRKACGTAPKRIFI
EMARDGESKKKRSVTRREQIKNLYRSIRKDFQQEVDFLEKILENKSDGQLQSDALYLYFAQLGRDMYTGD

PIKLEHIKDQSFYNIDHIYPQSMVKDDSLDNKVLVQSEINGEKSSRYPLDAAIRNKMKPLWDAYYNHGLI
SLKKYQRLTRSTPFTDDEKWDFINRQLVETRQSTKALAILLKRKFPDTEIVYSKAGLSSDFRHEFGLVKS
RNINDLHHAKDAFLAIVTGNVYHERFNRRWFMVNQPYSVKTKTLFTHSIKNGNFVAWNGEEDLGRIVKML
KQNKNTIHFTRFSFDRKEGLFDIQPLKASTGLVPRKAGLDVVKYGGYDKSTAAYYLLVRFTLEDKKTQHK
LMMIPVEGLYKARIDHDKEFLTDYAQTTISEILQKDKQKVINIMFPMGTRHIKLNSMISIDGFYLSIGGK
SSKGKSVLCHAMVPLIVPHKIECYIKAMESFARKFKENNKLRIVEKFDKITVEDNLNLYELFLQKLQHNP
YNKFFSTQFDVLTNGRSTFTKLSPEEQVQTLLNILSIFKTCRSSGCDLKSINGSAQAARIMISADLTGLS
KKYSDIRLVEQSASGLFVSKSQNLLEYL

SEQ ID NO:320
MTKKEQPYNIGLDIGTSSVGWAVTNDNYDLLNIKKKNLWGVRLFEEAQTAKETRLNRSTRRRYRRRKNRI
NWLNEIFSEELAKTDPSFLIRLQNSWVSKKDPDRKRDKYNLFIDGPYTDKEYYREFPTIFHLRKELILNK
DKADIRLIYLALHNILKYRGNFTYEHQKFNISNLNNNLSKELIELNQQLIKYDISFPDDCDWNHISDILI
GRGNATQKSSNILKDFTLDKETKKLLKEVINLILGNVAHLNTIFKTSLTKDEEKLNFSGKDIESKLDDLD
SILDDDQFTVLDAANRIYSTITLNEILNGESYFSMAKVNQYENHAIDLCKLRDMWHTTKNEEAVEQSRQA
YDDYINKPKYGTKELYTSLKKFLKVALPTNLAKEAEEKISKGTYLVKPRNSENGVVPYQLNKIEMEKIID
NQSQYYPFLKENKEKLLSILSFRIPYYVGPLQSAEKNPFAWMERKSNGHARPWNFDEIVDREKSSNKFIR
RMTVTDSYLVGEPVLPKNSLIYQRYEVLNELNNIRITENLKTNPIGSRLTVETKQRIYNELFKKYKKVTV
KKLTKWLIAQGYYKNPILIGLSQKDEFNSTLTTYLDMKKIFGSSFMEDNKNYDQIEELIEWLTIFEDKQI
LNEKLHSSKYSYTPDQIKKISNMRYKGWGRLSKKILMDITTETNTPQLLQLSNYSILDLMWATNNNFISI
MSNDKYDFKNYIENHNLNKNEDQNISDLVNDIHVSPALKRGITQSIKIVQEIVKFMGHAPKHIFIEVTRE
TKKSEITTSREKRIKRLQSKLLNKANDFKPQLREYLVPNKKIQEELKKHKNDLSSERIMLYFLQNGKSLY
SEESLNINKLSDYQVDHILPRTYIPDDSLENKALVLAKENQRKADDLLLNSNVIDRNLERWTYMLNNNMI
GLKKFKNLTRRVITDKDKLGFIHRQLVQTSQMVKGVANILDNMYKNQGTTCIQARANLSTAFRKALSGQD
DTYHFKHPELVKNRNVNDFHHAQDAYLASFLGTYRLRRFPTNEMLLMNGEYNKFYGQVKELYSKKKKLPD
SRKNGFIISPLVNGTTQYDRNTGEIIWNVGFRDKILKIFNYHQCNVTRKTEIKTGQFYDQTIYSPKNPKY
KKLIAQKKDMDPNIYGGFSGDNKSSITIVKIDNNKIKPVAIPIRLINDLKDKKTLQNWLEENVKHKKSIQ
IIKNNVPIGQIIYSKKVGLLSLNSDREVANRQQLILPPEHSALLRLLQIPDEDLDQILAFYDKNILVEIL
QELITKMKKFYPFYKGEREFLIANIENFNQATTSEKVNSLEELITLLHANSTSAHLIFNNIEKKAFGRKT
HGLTLNNTDFIYQSVTGLYETRIHIE

SEQ ID NO:321
MTKFNKNYSIGLDIGVSSVGYAVVTEDYRVPAFKFKVLGNTEKEKIKKNLIGSTTFVSAQPAKGTRVFRV
NRRRIDRRNHRITYLRDIFQKEIEKVDKNFYRRLDESFRVLGDKSEDLQIKQPFFGDKELETAYHKKYPT
IYHLRKHLADADKNSPVADIREVYMAISHILKYRGHFLTLDKINPNNINMQNSWIDFIESCQEVFDLEIS
DESKNIADIFKSSENRQEKVKKILPYFQQELLKKDKSIFKQLLQLLFGLKTKFKDCFELEEEPDLNFSKE
NYDENLENFLGSLEEDFSDVFAKLKVLRDTILLSGMLTYTGATHARFSATMVERYEEHRKDLQRFKFFIK
QNLSEQDYLDIFGRKTQNGFDVDKETKGYVGYITNKMVLTNPQKQKTIQQNFYDYISGKITGIEGAEYFL
NKISDGTFLRKLRTSDNGAIPNQIHAYELEKIIERQGKDYPFLLENKDKLLSILTFKIPYYVGPLAKGSN
SRFAWIKRATSSDILDDNDEDTRNGKIRPWNYQKLINMDETRDAFITNLIGNDIILLNEKVLPKRSLIYE
EVMLQNELTRVKYKDKYGKAHFFDSELRQNIINGLFKNNSKRVNAKSLIKYLSDNHKDLNAIEIVSGVEK
GKSFNSTLKTYNDLKTIFSEELLDSEIYQKELEEIIKVITVFDDKKSIKNYLTKFFGHLEILDEEKINQL
SKLRYSGWGRYSAKLLLDIRDEDTGFNLLQFLRNDEENRNLTKLISDNTLSFEPKIKDIQSKSTIEDDIF
DEIKKLAGSPAIKRGILNSIKIVDELVQIIGYPPHNIVIEMARENMTTEEGQKKAKTRKTKLESALKNIE
NSLLENGKVPHSDEQLQSEKLYLYYLQNGKDMYTLDKTGSPAPLYLDQLDQYEVDHIIPYSFLPIDSIDN
KVLTHRENNQQKLNNIPDKETVANMKPFWEKLYNAKLISQTKYQRLTTSERTPDGVLTESMKAGFIERQL
VETRQIIKHVARILDNRFSDTKIITLKSQLITNFRNTFHIAKIRELNDYHHAHDAYLAVVVGQTLLKVYP
KLAPELIYGHHAHFNRHEENKATLRKHLYSNIMRFFNNPDSKVSKDIWDCNRDLPIIKDVIYNSQINFVK
RTMIKKGAFYNQNPVGKFNKQLAANNRYPLKTKALCLDTSIYGGYGPMNSALSIIIIAERFNEKKGKIET
VKEFHDIFIIDYEKFNNNPFQFLNDTSENGFLKKNNINRVLGFYRIPKYSLMQKIDGTRMLFESKSNLHK
ATQFKLTKTQNELFFHMKRLLTKSNLMDLKSKSAIKESQNFILKHKEEFDNISNQLSAFSQKMLGNTTSL

KNLIKGYNERKIKEIDIRDETIKYFYDNFIKMFSFVKSGAPKDINDFFDNKCTVARMRPKPDKKLLNATL
IHQSITGLYETRIDLSKLGED

SEQ ID NO:322
MKQEYFLGLDMGTGSLGWAVTDSTYQVMRKHGKALWGTRLFESASTAEERRMFRTARRRLDRRNWRIQVL
QEIFSEEISKVDPGFFLRMKESKYYPEDKRDAEGNCPELPYALFVDDNYTDKNYHKDYPTIYHLRKMLME
TTEIPDIRLVYLVLHHMMKHRGHFLLSGDISQIKEFKSTFEQLIQNIQDEELEWHISLDDAAIQFVEHVL
KDRNLTRSTKKSRLIKQLNAKSACEKAILNLLSGGTVKLSDIFNNKELDESERPKVSFADSGYDDYIGIV
EAELAEQYYIIASAKAVYDWSVLVEILGNSVSISEAKIKVYQKHQADLKTLKKIVRQYMTKEDYKRVFVD
TEEKLNNYSAYIGMTKKNGKKVDLKSKQCTQADFYDFLKKNVIKVIDHKEITQEIESEIEKENFLPKQVT
KDNGVIPYQVHDYELKKILDNLGTRMPFIKENAEKIQQLFEFRIPYYVGPLNRVDDGKDGKFTWSVRKSD
ARIYPWNFTEVIDVEASAEKFIRRMTNKCTYLVGEDVLPKDSLVYSKFMVLNELNNLRLNGEKISVELKQ
RIYEELFCKYRKVTRKKLERYLVIEGIAKKGVEITGIDGDFKASLTAYHDFKERLTDVQLSQRAKEAIVL
NVVLFGDDKKLLKQRLSKMYPNLTTGQLKGICSLSYQGWGRLSKTFLEEITVPAPGTGEVWNIMTALWQT
NDNLMQLLSRNYGFTNEVEEFNTLKKETDLSYKTVDELYVSPAVKRQIWQTLKVVKEIQKVMGNAPKRVF
VEMAREKQEGKRSDSRKKQLVELYRACKNEERDWITELNAQSDQQLRSDKLFLYYIQKGRCMYSGETIQL
DELWDNTKYDIDHIYPQSKTMDDSLNNRVLVKKNYNAIKSDTYPLSLDIQKKMMSFWKMLQQQGFITKEK
YVRLVRSDELSADELAGFIERQIVETRQSTKAVATILKEALPDTEIVYVKAGNVSNFRQTYELLKVREMN
DLHHAKDAYLNIVVGNAYFVKFTKNAAWFIRNNPGRSYNLKRMFEFDIERSGEIAWKAGNKGSIVTVKKV
MQKNNILVTRKAYEVKGGLFDQQIMKKGKGQVPIKGNDERLADIEKYGGYNKAAGTYFMLVKSLDKKGKE
IRTIEFVPLYLKNQIEINHESAIQYLAQERGLNSPEILLSKIKIDTLFKVDGFKMWLSGRTGNQLIFKGA
NQLILSHQEAAILKGVVKYVNRKNENKDAKLSERDGMTEEKLLQLYDTFLDKLSNTVYSIRLSAQIKTLT
EKRAKFIGLSNEDQCIVLNEILHMFQCQSGSANLKLIGGPGSAGILVMNNNITACKQISVINQSPTGIYE
KEIDLIKL

SEQ ID NO:323
MKKPYSIGLDIGTNSVGWAVVTDDYKVPAKKMKVLGNTDKSHIEKNLLGALLFDSGNTAEDRRLKRTARR
RYTRRRNRILYLQEIFSEEMGKVDDSFFHRLEDSFLVTEDKRGERHPIFGNLEEEVKYHENFPTIYHLRQ
YLADNPEKVDLRLVYLALAHIIKFRGHFLIEGKFDTRNNDVQRLFQEFLAVYDNTFENSSLQEQNVQVEE
ILTDKISKSAKKDRVLKLFPNEKSNGRFAEFLKLIVGNQADFKKHFELEEKAPLQFSKDTYEEELEVLLA
QIGDNYAELFLSAKKLYDSILLSGILTVTDVGTKAPLSASMIQRYNEHQMDLAQLKQFIRQKLSDKYNEV
FSDVSKDGYAGYIDGKTNQEAFYKYLKGLLNKIEGSGYFLDKIEREDFLRKQRTFDNGSIPHQIHLQEMR
AIIRRQAEFYPFLADNQDRIEKLLTFRIPYYVGPLARGKSDFAWLSRKSADKITPWNFDEIVDKESSAEA
FINRMTNYDLYLPNQKVLPKHSLLYEKFTVYNELTKVKYKTEQGKTAFFDANMKQEIFDGVFKVYRKVTK
DKLMDFLEKEFDEFRIVDLTGLDKENKVFNASYGTYHDLCKILDKDFLDNSKNEKILEDIVLTLTLFEDR
EMIRKRLENYSDLLTKEQVKKLERRHYTGWGRLSAELIHGIRNKESRKTILDYLIDDGNSNRNFMQLIND
DALSFKEEIAKAQVIGETDNLNQVVSDIAGSPAIKKGILQSLKIVDELVKIMGHQPENIVVEMARENQFT
NQGRRNSQQRLKGLTDSIKEFGSQILKEHPVENSQLQNDRLFLYYLQNGRDMYTGEELDIDYLSQYDIDH
IIPQAFIKDNSIDNRVLTSSKENRGKSDDVPSKDVVRKMKSYWSKLLSAKLITQRKFDNLTKAERGGLTD
DDKAGFIKRQLVETRQITKHVARILDERFNTETDENNKKIRQVKIVTLKSNLVSNFRKEFELYKVREIND
YHHAHDAYLNAVIGKALLGVYPQLEPEFVYGDYPHFHGHKENKATAKKFFYSNIMNFFKKDDVRTDKNGE
IIWKKDEHISNIKKVLSYPQVNIVKKVEEQTGGFSKESILPKGNSDKLIPRKTKKFYWDTKKYGGFDSPI
VAYSILVIADIEKGKSKKLKTVKALVGVTIMEKMTFERDPVAFLERKGYRNVQEENIIKLPKYSLFKLEN
GRKRLLASARELQKGNEIVLPNHLGTLLYHAKNIHKVDEPKHLDYVDKHKDEFKELLDVVSNFSKKYTLA
EGNLEKIKELYAQNNGEDLKELASSFINLLTFTAIGAPATFKFFDKNIDRKRYTSTTEILNATLIHQSIT
GLYETRIDLNKLGGD

SEQ ID NO:324






















SEQ ID NO:325
MTKPYSIGLDIGTNSVGWAVTTDNYKVPSKKMKVLGNTSKKYIKKNLLGVLLFDSGITAEGRRLKRTARR
RYTRRRNRILYLQEIFSTEMATLDDAFFQRLDDSFLVPDDKRDSKYPIFGNLVEEKAYHDEFPTIYHLRK
YLADSTKKADLRLVYLALAHMIKYRGHFLIEGEFNSKNNDIQKNFQDFLDTYNAIFESDLSLENSKQLEE
IVKDKISKLEKKDRILKLFPGEKNSGIFSEFLKLIVGNQADFRKCFNLDEKASLHFSKESYDEDLETLLG
YIGDDYSDVFLKAKKLYDAILLSGFLTVTDNETEAPLSSAMIKRYNEHKEDLALLKEYIRNISLKTYNEV
FKDDTKNGYAGYIDGKTNQEDFYVYLKKLLAEFEGADYFLEKIDREDFLRKQRTFDNGSIPYQIHLQEMR
AILDKQAKFYPFLAKNKERIEKILTFRIPYYVGPLARGNSDFAWSIRKRNEKITPWNFEDVIDKESSAEA
FINRMTSFDLYLPEEKVLPKHSLLYETFNVYNELTKVRFIAESMRDYQFLDSKQKKDIVRLYFKDKRKVT
DKDIIEYLHAIYGYDGIELKGIEKQFNSSLSTYHDLLNIINDKEFLDDSSNEAIIEEIIHTLTIFEDREM
IKQRLSKFENIFDKSVLKKLSRRHYTGWGKLSAKLINGIRDEKSGNTILDYLIDDGISNRNFMQLIHDDA
LSFKKKIQKAQIIGDEDKGNIKEVVKSLPGSPAIKKGILQSIKIVDELVKVMGGRKPESIVVEMARENQY
TNQGKSNSQQRLKRLEKSLKELGSKILKENIPAKLSKIDNNALQNDRLYLYYLQNGKDMYTGDDLDIDRL
SNYDIDHIIPQAFLKDNSIDNKVLVSSASNRGKSDDVPSLEVVKKRKTFWYQLLKSKLISQRKFDNLTKA
ERGGLSPEDKAGFIQRQLVETRQITKHVARLLDEKFNNKKDENNRAVRTVKIITLKSTLVSQFRKDFELY
KVREINDFHHAHDAYLNAVVASALLKKYPKLEPEFVYGDYPKYNSFRERKSATEKVYFYSNIMNIFKKSI
SLADGRVIERPLIEVNEETGESVWNKESDLATVRRVLSYPQVNVVKKVEEQNHGLDRGKPKGLFNANLSS
KPKPNSNENLVGAKEYLDPKKYGGYAGISNSFTVLVKGTIEKGAKKKITNVLEFQGISILDRINYRKDKL
NFLLEKGYKDIELIIELPKYSLFELSDGSRRMLASILSTNNKRGEIHKGNQIFLSQKFVKLLYHAKRISN
TINENHRKYVENHKKEFEELFYYILEFNENYVGAKKNGKLLNSAFQSWQNHSIDELCSSFIGPTGSERKG
LFELTSRGSAADFEFLGVKIPRYRDYTPSSLLKDATLIHQSVTGLYETRIDLAKLGEG

SEQ ID NO:326
MKKQKFSDYYLGFDIGTNSVGWCVTDLDYNVLRFNKKDMWGSRLFDEAKTAAERRVQRNSRRRLKRRKWR
LNLLEEIFSDEIMKIDSNFFRRLKESSLWLEDKNSKEKFTLFNDDNYKDYDFYKQYPTIFHLRDELIKNP
EKKDIRLIYLALHSIFKSRGHFLFEGQNLKEIKNFETLYNNLISFLEDNGINKSIDKDNIEKLEKIICDS
GKGLKDKEKEFKGIFNSDKQLVAIFKLSVGSSVSLNDLFDTDEYKKEEVEKEKISFREQIYEDDKPIYYS
ILGEKIELLDIAKSFYDFMVLNNILSDSNYISEAKVKLYEEHKKDLKNLKYIIRKYNKENYDKLFKDKNE
NNYPAYIGLNKEKDKKEVVEKSRLKIDDLIKVIKGYLPKPERIEEKDTIFNEILNKIELKTILPKQRIS
DNGTLPYQIHEVELEKILENQSKYYDFLNYEENGVSTKDKLLKTFKFRIPYYVGPLNSYHKDKGGNSWIV
RKEEGKILPWNFEQKVDIEKSAEEFIKRMTNKCTYLNGEDVIPKDSFLYSEYIILNELNKVQVNDEFLNE
ENKRKIIDELFKENKKVSEKKFKEYLLVNQIANRTVELKGIKDSFNSNYVSYIKFKDIFGEKLNLDIYKE
ISEKSILWKCLYGDDKKIFEKKIKNEYGDILNKDEIKKINSFKFNTWGRLSEKLLTGIEFINLETGECYS
SVMEALRRTNYNLMELLSSKFTLQESIDNENKEMNEVSYRDLIEESYVSPSLKRAILQTLKIYEEIKKIT
GRVPKKVFIEMARGGDESMKNKKIPARQEQLKKLYDSCGNDIANFSIDIKEMKNSLSSYDNNSLRQKKLY

LYYLQFGKCMYTGREIDLDRLLQNNDTYDIDHIYPRSKVIKDDSFDNLVLVLKNENAEKSNEYPVKKEIQ
EKMKSFWRFLKEKNFISDEKYKRLTGKDDFELRGFMARQLVNVRQTTKEVGKILQQIEPEIKIVYSKAEI
ASSFREMFDFIKVRELNDTHHAKDAYLNIVAGNVYNTKFTEKPYRYLQEIKENYDVKKIYNYDIKNAWDK
ENSLEIVKKNMEKNTVNITRFIKEEKGELFNLNPIKKGETSNEIISIKPKLYDGKDNKLNEKYGYYTSLK
AAYFIYVEHEKKNKKVKTFERITRIDSTLIKNEKNLIKYLVSQKKLLNPKIIKKIYKEQTLIIDSYPYTF
TGVDSNKKVELKNKKQLYLEKKYEQILKNALKFVEDNQGETEENYKFIYLKKRNNNEKNETIDAVKERYN
IEFNEMYDKFLEKLSSKDYKNYINNKLYTNFLNSKEKFKKLKLWEKSLILREFLKIFNKNTYGKYEIKDS
QTKEKLFSFPEDTGRIRLGQSSLGNNKELLEESVTGLFVKKIKL

SEQ ID NO:327
MKNYTIGLDIGVASVGWVCIDENYKILNYNNRHAFGVHEFESAESAAGRRLKRGMRRRYNRRKKRLQLLQ
SLFDSYITDSGFFSKTDSQHFWKNNNEFENRSLTEVLSSLRISSRKYPTIYHLRSDLIESNKKMDLRLVY
LALHNLVKYRGHFLQEGNWSEAASAEGMDDQLLELVTRYAELENLSPLDLSESQWKAAETLLLNRNLTKT
DQSKELTAMFGKEYEPFCKLVAGLGVSLHQLFPSSEQALAYKETKTKVQLSNENVEEVMELLLEEESALL
EAVQPFYQQVVLYELLKGETYVAKAKVSAFKQYQKDMASLKNLLDKTFGEKVYRSYFISDKNSQREYQKS
HKVEVLCKLDQFNKEAKFAETFYKDLKKLLEDKSKTSIGTTEKDEMLRIIKAIDSNQFLQKQKGIQNAAI
PHQNSLYEAEKILRNQQAHYPFITTEWIEKVKQILAFRIPYYIGPLVKDTTQSPFSWVERKGDAPITPWN
FDEQIDKAASAEAFISRMRKTCTYLKGQEVLPKSSLTYERFEVLNELNGIQLRTTGAESDFRHRLSYEMK
CWIIDNVFKQYKTVSTKRLLQELKKSPYADELYDEHTGEIKEVFGTQKENAFATSLSGYISMKSILGAVV
DDNPAMTEELIYWIAVFEDREILHLKIQEKYPSITDVQRQKLALVKLPGWGRFSRLLIDGLPLDEQGQSV
LDHMEQYSSVFMEVLKNKGFGLEKKIQKMNQHQVDGTKKIRYEDIEELAGSPALKRGIWRSVKIVEELVS
IFGEPANIVLEVAREDGEKKRTKSRKDQWEELTKTTLKNDPDLKSFIGEIKSQGDQRFNEQRFWLYVTQQ
GKCLYTGKALDIQNLSMYEVDHILPQNFVKDDSLDNLALVMPEANQRKNQVGQNKMPLEIIEANQQYAMR
TLWERLHELKLISSGKLGRLKKPSFDEVDKDKFIARQLVETRQIIKHVRDLLDERFSKSDIHLVKAGIVS
KFRRFSEIPKIRDYNNKHHAMDALFAAALIQSILGKYGKNFLAFDLSKKDRQKQWRSVKGSNKEFFLFKN
FGNLRLQSPVTGEEVSGVEYMKHVYFELPWQTTKMTQTGDGMFYKESIFSPKVKQAKYVSPKTEKFVHDE
VKNHSICLVEFTFMKKEKEVQETKFIDLKVIEHHQFLKEPESQLAKFLAEKETNSPIIHARIIRTIPKYQ
KIWIEHFPYYFISTRELHNARQFEISYELMEKVKQLSERSSVEELKIVFGLLIDQMNDNYPIYTKSSIQD
RVQKFVDTQLYDFKSFEIGFEELKKAVAANAQRSDTFGSRISKKPKPEEVAIGYESITGLKYRKPRSVVG
TKR

SEQ ID NO:328
MKKEIKDYFLGLDVGTGSVGWAVTDTDYKLLKANRKDLWGMRCFETAETAEVRRLHRGARRRIERRKKRI
KLLQELFSQEIAKTDEGFFQRMKESPFYAEDKTILQENTLFNDKDFADKTYHKAYPTINHLIKAWIENKV
KPDPRLLYLACHNIIKKRGHFLFEGDFDSENQFDTSIQALFEYLREDMEVDIDADSQKVKEILKDSSLKN
SEKQSRLNKILGLKPSDKQKKAITNLISGNKINFADLYDNPDLKDAEKNSISFSKDDFDALSDDLASILG
DSFELLLKAKAVYNCSVLSKVIGDEQYLSFAKVKIYEKHKTDLTKLKNVIKKHFPKDYKKVFGYNKNEKN
NNNYSGYVGVCKTKSKKLIINNSVNQEDFYKFLKTILSAKSEIKEVNDILTEIETGTFLPKQISKSNAEI
PYQLRKMELEKILSNAEKHFSFLKQKDEKGLSHSEKIIMLLTFKIPYYIGPINDNHKKFFPDRCWVVKKE
KSPSGKTTPWNFFDHIDKEKTAEAFITSRTNFCTYLVGESVLPKSSLLYSEYTVLNEINNLQIIIDGKNI
CDIKLKQKIYEDLFKKYKKITQKQISTFIKHEGICNKTDEVIILGIDKECTSSLKSYIELKNIFGKQVDE
ISTKNMLEEIIRWATIYDEGEGKTILKTKIKAEYGKYCSDEQIKKILNLKFSGWGRLSRKFLETVTSEMP
GFSEPVNIITAMRETQNNLMELLSSEFTFTENIKKINSGFEDAEKQFSYDGLVKPLFLSPSVKKMLWQTL
KLVKEISHITQAPPKKIFIEMAKGAELEPARTKTRLKILQDLYNNCKNDADAFSSEIKDLSGKIENEDNL
RLRSDKLYLYYTQLGKCMYCGKPIEIGHVFDTSNYDIDHIYPQSKIKDDSISNRVLVCSSCNKNKEDKYP
LKSEIQSKQRGFWNFLQRNNFISLEKLNRLTRATPISDDETAKFIARQLVETRQATKVAAKVLEKMFPET
KIVYSKAETVSMFRNKFDIVKCREINDFHHAHDAYLNIVVGNVYNTKFTNNPWNFIKEKRDNPKIADTYN
YYKVFDYDVKRNNITAWEKGKTIITVKDMLKRNTPIYTRQAACKKGELFNQTIMKKGLGQHPLKKEGPFS
NISKYGGYNKVSAAYYTLIEYEEKGNKIRSLETIPLYLVKDIQKDQDVLKSYLTDLLGKKEFKILVPKIK
INSLLKINGFPCHITGKTNDSFLLRPAVQFCCSNNEVLYFKKIIRFSEIRSQREKIGKTISPYEDLSFRS
YIKENLWKKTKNDEIGEKEFYDLLQKKNLEIYDMLLTKHKDTIYKKRPNSATIDILVKGKEKFKSLIIEN
QFEVILEILKLFSATRNVSDLQHIGGSKYSGVAKIGNKISSLDNCILIYQSITGIFEKRIDLLKV

SEQ ID NO:329
MEGQMKNNGNNLQQGNYYLGLDVGTSSVGWAVTDTDYNVLKFRGKSMWGARLFDEASTAEERRTHRGNRR
RLARRKYRLLLLEQLFEKEIRKIDDNFFVRLHESNLWADDKSKPSKFLLFNDTNFTDKDYLKKYPTIYHL
RSDLIHNSTEHDIRLVFLALHHLIKYRGHFIYDNSANGDVKTLDEAVSDFEEYLNENDIEFNIENKKEFI
NVLSDKHLTKKEKKISLKKLYGDITDSENINISVLIEMLSGSSISLSNLFKDIEFDGKQNLSLDSDIEET
LNDVVDILGDNIDLLIHAKEVYDIAVLTSSLGKHKYLCDAKVELFEKNKKDLMILKKYIKKNHPEDYKKI
FSSPTEKKNYAAYSQTNSKNVCSQEEFCLFIKPYIRDMVKSENEDEVRIAKEVEDKSFLTKLKGTNNSVV
PYQIHERELNQILKNIVAYLPFMNDEQEDISVVDKIKLIFKFKIPYYVGPLNTKSTRSWVYRSDEKIYPW
NFSNVIDLDKTAHEFMNRLIGRCTYTNDPVLPMDSLLYSKYNVLNEINPIKVNGKAIPVEVKQAIYTDLF
ENSKKKVTRKSIYIYLLKNGYIEKEDIVSGIDIEIKSKLKSHHDFTQIVQENKCTPEEIERIIKGILVYS
DDKSMLRRWLKNNIKGLSENDVKYLAKLNYKEWGRLSKTLLTDIYTINPEDGEACSILDIMWNTNATLME
ILSNEKYQFKQNIENYKAENYDEKQNLHEELDDMYISPAARRSIWQALRIVDEIVDIKKSAPKKIFIEMA
REKKSAMKKKRTESRKDTLLELYKSCKSQADGFYDEELFEKLSNESNSRLRRDQLYLYYTQMGRSMYTGK
RIDFDKLINDKNTYDIDHIYPRSKIKDDSITNRVLVEKDINGEKTDIYPISEDIRQKMQPFWKILKEKGL
INEEKYKRLTRNYELTDEELSSFVARQLVETQQSTKALATLLKKEYPSAKIVYSKAGNVSEFRNRKDKEL
PKFREINDLHHAKDAYLNIVVGNVYDTKFTEKFFNNIRNENYSLKRVFDFSVPGAWDAKGSTFNTIKKYM
AKNNPIIAFAPYEVKGELFDQQIVPKGKGQFPIKQGKDIEKYGGYNKLSSAFLFAVEYKGKKARERSLET
VYIKDVELYLQDPIKYCESVLGLKEPQIIKPKILMGSLFSINNKKLVVTGRSGKQYVCHHIYQLSINDED
SQYLKNIAKYLQEEPDGNIERQNILNITSVNNIKLFDVLCTKFNSNTYEIILNSLKNDVNEGREKFSELD
ILEQCNILLQLLKAFKCNRESSNLEKLNNKKQAGVIVIPHLFTKCSVFKVIHQSITGLFEKEMDLLK

SEQ ID NO:330
MGRKPYILSLDIGTGSVGYACMDKGFNVLKYHDKDALGVYLFDGALTAQERRQFRTSRRRKNRRIKRLGL
LQELLAPLVQNPNFYQFQRQFAWKNDNMDFKNKSLSEVLSFLGYESKKYPTIYHLQEALLLKDEKFDPEL
IYMALYHLVKYRGHFLFDHLKIENLTNNDNMHDFVELIETYENLNNIKLNLDYEKTKVIYEILKDNEMTK
NDRAKRVKNMEKKLEQFSIMLLGLKFNEGKLFNHADNAEELKGANQSHTFADNYEENLTPFLTVEQSEFI
ERANKIYLSLTLQDILKGKKSMAMSKVAAYDKFRNELKQVKDIVYKADSTRTQFKKIFVSSKKSLKQYDA
TPNDQTFSSLCLFDQYLIRPKKQYSLLIKELKKIIPQDSELYFEAENDTLLKVLNTTDNASIPMQINLYE
AETILRNQQKYHAEITDEMIEKVLSLIQFRIPYYVGPLVNDHTASKFGWMERKSNESIKPWNFDEVVDRS
KSATQFIRRMTNKCSYLINEDVLPKNSLLYQEMEVLNELNATQIRLQTDPKNRKYRMMPQIKLFAVEHIF
KKYKTVSHSKFLEIMLNSNHRENFMNHGEKLSIFGTQDDKKFASKLSSYQDMTKIFGDIEGKRAQIEEII
QWITIFEDKKILVQKLKECYPELTSKQINQLKKLNYSGWGRLSEKLLTHAYQGHSIIELLRHSDENFMEI
LTNDVYGFQNFIKEENQVQSNKIQHQDIANLTTSPALKKGIWSTIKLVRELTSIFGEPEKIIMEFATEDQ
QKGKKQKSRKQLWDDNIKKNKLKSVDEYKYIIDVANKLNNEQLQQEKLWLYLSQNGKCMYSGQSIDLDAL
LSPNATKHYEVDHIFPRSFIKDDSIDNKVLVIKKMNQTKGDQVPLQFIQQPYERIAYWKSLNKAGLISDS
KLHKLMKPEFTAMDKEGFIQRQLVETRQISVHVRDFLKEEYPNTKVIPMKAKMVSEFRKKFDIPKIRQMN
DAHHAIDAYLNGVVYHGAQLAYPNVDLFDFNFKWEKVREKWKALGEFNTKQKSRELFFFKKLEKMEVSQG
ERLISKIKLDMNHFKINYSRKLANIPQQFYNQTAVSPKTAELKYESNKSNEVVYKGLTPYQTYVVAIKSV
NKKGKEKMEYQMIDHYVFDFYKFQNGNEKELALYLAQRENKDEVLDAQIVYSLNKGDLLYINNHPCYFVS
RKEVINAKQFELTVEQQLSLYNVMNNKETNVEKLLIEYDFIAEKVINEYHHYLNSKLKEKRVRTFFSESN
QTHEDFIKALDELFKVVTASATRSDKIGSRKNSMTHRAFLGKGKDVKIAYTSISGLKTTKPSLFKLAES
RNEL

SEQ ID NO:331
MAKILGLDLGTNSIGWAVVERENIDFSLIDKGVRIFSEGVKSEKGIESSRAAERTGYRSARKIKYRRKLR
KYETLKVLSLNRMCPLSIEEVEEWKKSGFKDYPLNPEFLKWLSTDEESNVNPYFFRDRASKHKVSLFELG
RAFYHIAQRRGFLSNRLDQSAEGILEEHCPKIEAIVEDLISIDEISTNITDYFFETGILDSNEKNGYAKD
LDEGDKKLVSLYKSLLAILKKNESDFENCKSEIIERLNKKDVLGKVKGKIKDISQAMLDGNYKTLGQYFY
SLYSKEKIRNQYTSREEHYLSEFITICKVQGIDQINEEEKINEKKFDGLAKDLYKAIFFQRPLKSQKGLI
GKCSFEKSKSRCAISHPDFEEYRMWTYLNTIKIGTQSDKKLRFLTQDEKLKLVPKFYRKNDFNFDVLAKE
LIEKGSSFGFYKSSKKNDFFYWFNYKPTDTVAACQVAASLKNAIGEDWKTKSFKYQTINSNKEQVSRTVD

YKDLWHLLTVATSDVYLYEFAIDKLGLDEKNAKAFSKTKLKKDFASLSLSAINKILPYLKEGLLYSHAVF
VANIENIVDENIWKDEKQRDYIKTQISEIIENYTLEKSRFEIINGLLKEYKSENEDGKRVYYSKEAEQSF
ENDLKKKLVLFYKSNEIENKEQQETIFNELLPIFIQQLKDYEFIKIQRLDQKVLIFLKGKNETGQIFCTE
EKGTAEEKEKKIKNRLKKLYHPSDIEKFKKKIIKDEFGNEKIVLGSPLTPSIKNPMAMRALHQLRKVLNA
LILEGQIDEKTIIHIEMARELNDANKRKGIQDYQNDNKKFREDAIKEIKKLYFEDCKKEVEPTEDDILRY
QLWMEQNRSEIYEEGKNISICDIIGSNPAYDIEHTIPRSRSQDNSQMNKTLCSQRFNREVKKQSMPIELN
NHLEILPRIAHWKEEADNLTREIEIISRSIKAAATKEIKDKKIRRRHYLTLKRDYLQGKYDRFIWEEPKV
GFKNSQIPDTGIITKYAQAYLKSYFKKVESVKGGMVAEFRKIWGIQESFIDENGMKHYKVKDRSKHTHHT
IDAITIACMTKEKYDVLAHAWTLEDQQNKKEARSIIEASKPWKTFKEDLLKIEEEILVSHYTPDNVKKQA
KKIVRVRGKKQFVAEVERDVNGKAVPKKAASGKTIYKLDGEGKKLPRLQQGDTIRGSLHQDSIYGAIKNP
LNTDEIKYVIRKDLESIKGSDVESIVDEVVKEKIKEAIANKVLLLSSNAQQKNKLVGTVWMNEEKRIAIN
KVRIYANSVKNPLHIKEHSLLSKSKHVHKQKVYGQNDENYAMAIYELDGKRDFELINIFNLAKLIKQGQG
FYPLHKKKEIKGKIVFVPIEKRNKRDVVLKRGQQVVFYDKEVENPKDISEIVDFKGRIYIIEGLSIQRIV
RPSGKVDEYGVIMLRYFKEARKADDIKQDNFKPDGVFKLGENKPTRKMNHQFTAFVEGIDFKVLPSGKFE
KI

SEQ ID NO:332
MEFKKVLGLDIGTNSIGCALLSLPKSIQDYGKGGRLEWLTSRVIPLDADYMKAFIDGKNGLPQVITPAGK
RRQKRGSRRLKHRYKLRRSRLIRVFKTLNWLPEDFPLDNPKRIKETISTEGKFSFRISDYVPISDESYRE
FYREFGYPENEIEQVIEEINFRRKTKGKNKNPMIKLLPEDWVVYYLRKKALIKPTTKEELIRIIYLFNQR
RGFKSSRKDLTETAILDYDEFAKRLAEKEKYSAENYETKFVSITKVKEVVELKTDGRKGKKRFKVILEDS
RIEPYEIERKEKPDWEGKEYTFLVTQKLEKGKFKQNKPDLPKEEDWALCTTALDNRMGSKHPGEFFFDEL
LKAFKEKRGYKIRQYPVNRWRYKKELEFIWTKQCQLNPELNNLNINKEILRKLATVLYPSQSKFFGPKIK
EFENSDVLHIISEDIIYYQRDLKSQKSLISECRYEKRKGIDGEIYGLKCIPKSSPLYQEFRIWQDIHNIK
VIRKESEVNGKKKINIDETQLYINENIKEKLFELFNSKDSLSEKDILELISLNIINSGIKISKKEEETTH
RINLFANRKELKGNETKSRYRKVFKKLGFDGEYILNHPSKLNRLWHSDYSNDYADKEKTEKSILSSLGWK
NRNGKWEKSKNYDVFNLPLEVAKAIANLPPLKKEYGSYSALAIRKMLVVMRDGKYWQHPDQIAKDQENTS
LMLFDKNLIQLTNNQRKVLNKYLLTLAEVQKRSTLIKQKLNEIEHNPYKLELVSDQDLEKQVLKSFLEKK
NESDYLKGLKTYQAGYLIYGKHSEKDVPIVNSPDELGEYIRKKLPNNSLRNPIVEQVIRETIFIVRDVWK
SFGIIDEIHIELGRELKNNSEERKKTSESQEKNFQEKERARKLLKELLNSSNFEHYDENGNKIFSSFTVN
PNPDSPLDIEKFRIWKNQSGLTDEELNKKLKDEKIPTEIEVKKYILWLTQKCRSPYTGKIIPLSKLFDSN
VYEIEHIIPRSKMKNDSTNNLVICELGVNKAKGDRLAANFISESNGKCKFGEVEYTLLKYGDYLQYCKDT
FKYQKAKYKNLLATEPPEDFIERQINDTRYIGRKLAELLTPVVKDSKNIIFTIGSITSELKITWGLNGVW
KDILRPRFKRLESIINKKLIFQDEDDPNKYHFDLSINPQLDKEGLKRLDHRHHALDATIIAATTREHVRY
LNSLNAADNDEEKREYFLSLCNHKIRDFKLPWENFTSEVKSKLLSCVVSYKESKPILSDPFNKYLKWEYK
NGKWQKVFAIQIKNDRWKAVRRSMFKEPIGTVWIKKIKEVSLKEAIKIQAIWEEVKNDPVRKKKEKYIYD
DYAQKVIAKIVQELGLSSSMRKQDDEKLNKFINEAKVSAGVNKNLNTTNKTIYNLEGRFYEKIKVAEYVL
YKAKRMPLNKKEYIEKLSLQKMFNDLPNFILEKSILDNYPEILKELESDNKYIIEPHKKNNPVNRLLLEH
ILEYHNNPKEAFSTEGLEKLNKKAINKIGKPIKYITRLDGDINEEEIFRGAVFETDKGSNVYFVMYENNQ
TKDREFLKPNPSISVLKAIEHKNKIDFFAPNRLGFSRIILSPGDLVYVPTNDQYVLIKDNSSNETIINWD
DNEFISNRIYQVKKFTGNSCYFLKNDIASLILSYSASNGVGEFGSQNISEYSVDDPPIRIKDVCIKIRVD
RLGNVRPL

SEQ ID NO:333
MKHILGLDLGTNSIGWALIERNIEEKYGKIIGMGSRIVPMGAELSKFEQGQAQTKNADRRTNRGARRLNK
RYKQRRNKLIYILQKLDMLPSQIKLKEDFSDPNKIDKITILPISKKQEQLTAFDLVSLRVKALTEKVGLE
DLGKIIYKYNQLRGYAGGSLEPEKEDIFDEEQSKDKKNKSFIAFSKIVFLGEPQEEIFKNKKLNRRAIIV
ETEEGNFEGSTFLENIKVGDSLELLINISASKSGDTITIKLPNKTNWRKKMENIENQLKEKSKEMGREFY
ISEFLLELLKENRWAKIRNNTILRARYESEFEAIWNEQVKHYPFLENLDKKTLIEIVSFIFPGEKESQKK
YRELGLEKGLKYIIKNQVVFYQRELKDQSHLISDCRYEPNEKAIAKSHPVFQEYKVWEQINKLIVNTKIE
AGTNRKGEKKYKYIDRPIPTALKEWIFEELQNKKEITFSAIFKKLKAEFDLREGIDFLNGMSPKDKLKGN
ETKLQLQKSLGELWDVLGLDSINRQIELWNILYNEKGNEYDLTSDRTSKVLEFINKYGNNIVDDNAEETA

IRISKIKFARAYSSLSLKAVERILPLVRAGKYFNNDFSQQLQSKILKLLNENVEDPFAKAAQTYLDNNQS
VLSEGGVGNSIATILVYDKHTAKEYSHDELYKSYKEINLLKQGDLRNPLVEQIINEALVLIRDIWKNYGI
KPNEIRVELARDLKNSAKERATIHKRNKDNQTINNKIKETLVKNKKELSLANIEKVKLWEAQRHLSPYTG
QPIPLSDLFDKEKYDVDHIIPISRYFDDSFTNKVISEKSVNQEKANRTAMEYFEVGSLKYSIFTKEQFIA
HVNEYFSGVKRKNLLATSIPEDPVQRQIKDTQYIAIRVKEELNKIVGNENVKTTTGSITDYLRNHWGLTD
KFKLLLKERYEALLESEKFLEAEYDNYKKDFDSRKKEYEEKEVLFEEQELTREEFIKEYKENYIRYKKNK
LIIKGWSKRIDHRHHAIDALIVACTEPAHIKRLNDLNKVLQDWLVEHKSEFMPNFEGSNSELLEEILSLP
ENERTEIFTQIEKFRAIEMPWKGFPEQVEQKLKEIIISHKPKDKLLLQYNKAGDRQIKLRGQLHEGTLYG
ISQGKEAYRIPLTKFGGSKFATEKNIQKIVSPFLSGFIANHLKEYNNKKEEAFSAEGIMDLNNKLAQYRN
EKGELKPHTPISTVKIYYKDPSKNKKKKDEEDLSLQKLDREKAFNEKLYVKTGDNYLFAVLEGEIKTKKT
SQIKRLYDIISFFDATNFLKEEFRNAPDKKTFDKDLLFRQYFEERNKAKLLFTLKQGDFVYLPNENEEVI
LDKESPLYNQYWGDLKERGKNIYVVQKFSKKQIYFIKHTIADIIKKDVEFGSQNCYETVEGRSIKENCFK
LEIDRLGNIVKVIKR

SEQ ID NO:334
MHVEIDFPHFSRGDSHLAMNKNEILRGSSVLYRLGLDLGSNSLGWFVTHLEKRGDRHEPVALGPGGVRIF
PDGRDPQSGTSNAVDRRMARGARKRRDRFVERRKELIAALIKYNLLPDDARERRALEVLDPYALRKTALT
DTLPAHHVGRALFHLNQRRGFQSNRKTDSKQSEDGAIKQAASRLATDKGNETLGVFFADMHLRKSYEDRQ
TAIRAELVRLGKDHLTGNARKKIWAKVRKRLFGDEVLPRADAPHGVRARATITGTKASYDYYPTRDMLRD
EFNAIWAGQSAHHATITDEARTEIEHIIFYQRPLKPAIVGKCTLDPATRPFKEDPEGYRAPWSHPLAQRF
RILSEARNLEIRDTGKGSRRLTKEQSDLVVAALLANREVKFDKLRTLLKLPAEARFNLESDRRAALDGDQ
TAARLSDKKGFNKAWRGFPPERQIAIVARLEETEDENELIAWLEKECALDGAAAARVANTTLPDGHCRLG
LRAIKKIVPIMQDGLDEDGVAGAGYHIAAKRAGYDHAKLPTGEQLGRLPYYGQWLQDAVVGSGDARDQKE
KQYGQFPNPTVHIGLGQLRRVVNDLIDKYGPPTEISIEFTRALKLSEQQKAERQREQRRNQDKNKARAEE
LAKFGRPANPRNLLKMRLWEELAHDPLDRKCVYTGEQISIERLLSDEVDIDHILPVAMTLDDSPANKIIC
MRYANRHKRKQTPSEAFGSSPTLQGHRYNWDDIAARATGLPRNKRWRFDANAREEFDKRGGFLARQLNET
GWLARLAKQYLGAVTDPNQIWVVPGRLTSMLRGKWGLNGLLPSDNYAGVQDKAEEFLASTDDMEFSGVKN
RADHRHHAIDGLVTALTDRSLLWKMANAYDEEHEKFVIEPPWPTMRDDLKAALEKMVVSHKPDHGIEGKL
HEDSAYGFVKPLDATGLKEEEAGNLVYRKAIESLNENEVDRIRDIQLRTIVRDHVNVEKTKGVALADALR
QLQAPSDDYPQFKHGLRHVRILKKEKGDYLVPIANRASGVAYKAYSAGENFCVEVFETAGGKWDGEAVRR
FDANKKNAGPKIAHAPQWRDANEGAKLVMRIHKGDLIRLDHEGRARIMVVHRLDAAAGRFKLADHNETGN
LDKRHATNNDIDPFRWLMASYNTLKKLAAVPVRVDELGRVWRVMPN

SEQ ID NO:335
METTLGIDLGTNSIGLALVDQEEHQILYSGVRIFPEGINKDTIGLGEKEESRNATRRAKRQMRRQYFRKK
LRKAKLLELLIAYDMCPLKPEDVRRWKNWDKQQKSTVRQFPDTPAFREWLKQNPYELRKQAVTEDVTRPE
LGRILYQMIQRRGFLSSRKGKEEGKIFTGKDRMVGIDETRKNLQKQTLGAYLYDIAPKNGEKYRFRTERV
RARYTLRDMYIREFEIIWQRQAGHLGLAHEQATRKKNIFLEGSATNVRNSKLITHLQAKYGRGHVLIEDT
RITVTFQLPLKEVLGGKIEIEEEQLKFKSNESVLFWQRPLRSQKSLLSKCVFEGRNFYDPVHQKWIIAGP
TPAPLSHPEFEEFRAYQFINNIIYGKNEHLTAIQREAVFELMCTESKDFNFEKIPKHLKLFEKFNFDDTT
KVPACTTISQLRKLFPHPVWEEKREEIWHCFYFYDDNTLLFEKLQKDYALQTNDLEKIKKIRLSESYGNV
SLKAIRRINPYLKKGYAYSTAVLLGGIRNSFGKRFEYFKEYEPEIEKAVCRILKEKNAEGEVIRKIKDYL
VHNRFGFAKNDRAFQKLYHHSQAITTQAQKERLPETGNLRNPIVQQGLNELRRTVNKLLATCREKYGPSF
KFDHIHVEMGRELRSSKTEREKQSRQIRENEKKNEAAKVKLAEYGLKAYRDNIQKYLLYKEIEEKGGTVC
CPYTGKTLNISHTLGSDNSVQIEHIIPYSISLDDSLANKTLCDATFNREKGELTPYDFYQKDPSPEKWGA
SSWEEIEDRAFRLLPYAKAQRFIRRKPQESNEFISRQLNDTRYISKKAVEYLSAICSDVKAFPGQLTAEL
RHLWGLNNILQSAPDITFPLPVSATENHREYYVITNEQNEVIRLFPKQGETPRTEKGELLLTGEVERKVF
RCKGMQEFQTDVSDGKYWRRIKLSSSVTWSPLFAPKPISADGQIVLKGRIEKGVFVCNQLKQKLKTGLPD
GSYWISLPVISQTFKEGESVNNSKLTSQQVQLFGRVREGIFRCHNYQCPASGADGNFWCTLDTDTAQPAF
TPIKNAPPGVGGGQIILTGDVDDKGIFHADDDLHYELPASLPKGKYYGIFTVESCDPTLIPIELSAPKTS
KGENLIEGNIWVDEHTGEVRFDPKKNREDQRHHAIDAIVIALSSQSLFQRLSTYNARRENKKRGLDSTEH
FPSPWPGFAQDVRQSVVPLLVSYKQNPKTLCKISKTLYKDGKKIHSCGNAVRGQLHKETVYGQRTAPGAT

EKSYHIRKDIRELKTSKHIGKVVDITIRQMLLKHLQENYHIDITQEFNIPSNAFFKEGVYRIFLPNKHGE
PVPIKKIRMKEELGNAERLKDNINQYVNPRNNHHVMIYQDADGNLKEEIVSFWSVIERQNQGQPIYQLPR
EGRNIVSILQINDTFLIGLKEEEPEVYRNDLSTLSKHLYRVQKLSGMYYTFRHHLASTLNNEREEFRIQS
LEAWKRANPVKVQIDEIGRITFLNGPLC

SEQ ID NO:336
MESSQILSPIGIDLGGKFTGVCLSHLEAFAELPNHANTKYSVILIDHNNFQLSQAQRRATRHRVRNKKRN
QFVKRVALQLFQHILSRDLNAKEETALCHYLNNRGYTYVDTDLDEYIKDETTINLLKELLPSESEHNFID
WFLQKMQSSEFRKILVSKVEEKKDDKELKNAVKNIKNFITGFEKNSVEGHRHRKVYFENIKSDITKDNQL
DSIKKKIPSVCLSNLLGHLSNLQWKNLHRYLAKNPKQFDEQTFGNEFLRMLKNFRHLKGSQESLAVRNLI
QQLEQSQDYISILEKTPPEITIPPYEARTNTGMEKDQSLLLNPEKLNNLYPNWRNLIPGIIDAHPFLEKD
LEHTKLRDRKRIISPSKQDEKRDSYILQRYLDLNKKIDKFKIKKQLSFLGQGKQLPANLIETQKEMETHF
NSSLVSVLIQIASAYNKEREDAAQGIWFDNAFSLCELSNINPPRKQKILPLLVGAILSEDFINNKDKWAK
FKIFWNTHKIGRTSLKSKCKEIEEARKNSGNAFKIDYEEALNHPEHSNNKALIKIIQTIPDIIQAIQSHL
GHNDSQALIYHNPFSLSQLYTILETKRDGFHKNCVAVTCENYWRSQKTEIDPEISYASRLPADSVRPFDG
VLARMMQRLAYEIAMAKWEQIKHIPDNSSLLIPIYLEQNRFEFEESFKKIKGSSSDKTLEQAIEKQNIQW
EEKFQRIINASMNICPYKGASIGGQGEIDHIYPRSLSKKHFGVIFNSEVNLIYCSSQGNREKKEEHYLLE
HLSPLYLKHQFGTDNVSDIKNFISQNVANIKKYISFHLLTPEQQKAARHALFLDYDDEAFKTITKFLMSQ
QKARVNGTQKFLGKQIMEFLSTLADSKQLQLEFSIKQITAEEVHDHRELLSKQEPKLVKSRQQSFPSHAI
DATLTMSIGLKEFPQFSQELDNSWFINHLMPDEVHLNPVRSKEKYNKPNISSTPLFKDSLYAERFIPVWV
KGETFAIGFSEKDLFEIKPSNKEKLFTLLKTYSTKNPGESLQELQAKSKAKWLYFPINKTLALEFLHHYF
HKEIVTPDDTTVCHFINSLRYYTKKESITVKILKEPMPVLSVKFESSKKNVLGSFKHTIALPATKDWERL
FNHPNFLALKANPAPNPKEFNEFIRKYFLSDNNPNSDIPNNGHNIKPQKHKAVRKVFSLPVIPGNAGTMM
RIRRKDNKGQPLYQLQTIDDTPSMGIQINEDRLVKQEVLMDAYKTRNLSTIDGINNSEGQAYATFDNWLT
LPVSTFKPEIIKLEMKPHSKTRRYIRITQSLADFIKTIDEALMIKPSDSIDDPLNMPNEIVCKNKLFGNE
LKPRDGKMKIVSTGKIVTYEFESDSTPQWIQTLYVTQLKKQP

SEQ ID NO:337
MKKIVGLDLGTNSIGWALINAYINKEHLYGIEACGSRIIPMDAAILGNFDKGNSISQTADRTSYRGIRRL
RERHLLRRERLHRILDLLGFLPKHYSDSLNRYGKFLNDIECKLPWVKDETGSYKFIFQESFKEMLANFTE
HHPILIANNKKVPYDWTIYYLRKKALTQKISKEELAWILLNFNQKRGYYQLRGEEEETPNKLVEYYSLKV
EKVEDSGERKGKDTWYNVHLENGMIYRRTSNIPLDWEGKTKEFIVTTDLEADGSPKKDKEGNIKRSFRAP
KDDDWTLIKKKTEADIDKIKMTVGAYIYDTLLQKPDQKIRGKLVRTIERKYYKNELYQILKTQSEFHEEL
RDKQLYIACLNELYPNNEPRRNSISTRDFCHLFIEDIIFYQRPLKSKKSLIDNCPYEENRYIDKESGEIK
HASIKCIAKSHPLYQEFRLWQFIVNLRIYRKETDVDVTQELLPTEADYVTLFEWLNEKKEIDQKAFFKYP
PFGFKKTTSNYRWNYVEDKPYPCNETHAQIIARLGKAHIPKAFLSKEKEETLWHILYSIEDKQEIEKALH
SFANKNNLSEEFIEQFKNFPPFKKEYGSYSAKAIKKLLPLMRMGKYWSIENIDNGTRIRINKIIDGEYDE
NIRERVRQKAINLTDITHFRALPLWLACYLVYDRHSEVKDIVKWKTPKDIDLYLKSFKQHSLRNPIVEQV
ITETLRTVRDIWQQVGHIDEIHIELGREMKNPADKRARMSQQMIKNENTNLRIKALLTEFLNPEFGIENV
RPYSPSQQDLLRIYEEGVLNSILELPEDIGIILGKFNQTDTLKRPTRSEILRYKLWLEQKYRSPYTGEMI
PLSKLFTPAYEIEHIIPQSRYFDDSLSNKVICESEINKLKDRSLGYEFIKNHHGEKVELAFDKPVEVLSV
EAYEKLVHESYSHNRSKMKKLLMEDIPDQFIERQLNDSRYISKVVKSLLSNIVREENEQEAISKNVIPCT
GGITDRLKKDWGINDVWNKIVLPRFIRLNELTESTRFTSINTNNTMIPSMPLELQKGFNKKRIDHRHHAM
DAIIIACANRNIVNYLNNVSASKNTKITRRDLQTLLCHKDKTDNNGNYKWVIDKPWETFTQDTLTALQKI
TVSFKQNLRVINKTTNHYQHYENGKKIVSNQSKGDSWAIRKSMHKETVHGEVNLRMIKTVSFNEALKKPQ
AIVEMDLKKKILAMLELGYDTKRIKNYFEENKDTWQDINPSKIKVYYFTKETKDRYFAVRKPIDTSFDKK
KIKESITDTGIQQIMLRHLETKDNDPTLAFSPDGIDEMNRNILILNKGKKHQPIYKVRVYEKAEKFTVGQ
KGNKRTKFVEAAKGTNLFFAIYETEEIDKDTKKVIRKRSYSTIPLNVVIERQKQGLSSAPEDENGNLPKY
ILSPNDLVYVPTQEEINKGEVVMPIDRDRIYKMVDSSGITANFIPASTANLIFALPKATAEIYCNGENCI
QNEYGIGSPQSKNQKAITGEMVKEICFPIKVDRLGNIIQVGSCILTN

SEQ ID NO:338

MSRSLTFSFDIGYASIGWAVIASASHDDADPSVCGCGTVLFPKDDCQAFKRREYRRLRRNIRSRRVRIER
IGRLLVQAQIITPEMKETSGHPAPFYLASEALKGHRTLAPIELWHVLRWYAHNRGYDNNASWSNSLSEDG
GNGEDTERVKHAQDLMDKHGTATMAETICRELKLEEGKADAPMEVSTPAYKNLNTAFPRLIVEKEVRRIL
ELSAPLIPGLTAEIIELIAQHHPLTTEQRGVLLQHGIKLARRYRGSLLFGQLIPRFDNRIISRCPVTWAQ
VYEAELKKGNSEQSARERAEKLSKVPTANCPEFYEYRMARILCNIRADGEPLSAEIRRELMNQARQEGKL
TKASLEKAISSRLGKETETNVSNYFTLHPDSEEALYLNPAVEVLQRSGIGQILSPSVYRIAANRLRRGKS
VTPNYLLNLLKSRGESGEALEKKIEKESKKKEADYADTPLKPKYATGRAPYARTVLKKVVEEILDGEDPT
RPARGEAHPDGELKAHDGCLYCLLDTDSSVNQHQKERRLDTMTNNHLVRHRMLILDRLLKDLIQDFADGQ
KDRISRVCVEVGKELTTFSAMDSKKIQRELTLRQKSHTDAVNRLKRKLPGKALSANLIRKCRIAMDMNWT
CPFTGATYGDHELENLELEHIVPHSFRQSNALSSLVLTWPGVNRMKGQRTGYDFVEQEQENPVPDKPNLH
ICSLNNYRELVEKLDDKKGHEDDRRRKKKRKALLMVRGLSHKHQSQNHEAMKEIGMTEGMMTQSSHLMKL
ACKSIKTSLPDAHIDMIPGAVTAEVRKAWDVFGVFKELCPEAADPDSGKILKENLRSLTHLHHALDACVL
GLIPYIIPAHHNGLLRRVLAMRRIPEKLIPQVRPVANQRHYVLNDDGRMMLRDLSASLKENIREQLMEQR
VIQHVPADMGGALLKETMQRVLSVDGSGEDAMVSLSKKKDGKKEKNQVKASKLVGVFPEGPSKLKALKAA
IEIDGNYGVALDPKPVVIRHIKVFKRIMALKEQNGGKPVRILKKGMLIHLTSSKDPKHAGVWRIESIQDS
KGGVKLDLQRAHCAVPKNKTHECNWREVDLISLLKKYQMKRYPTSYTGTPR

SEQ ID NO:339
MTQKVLGLDLGTNSIGSAVRNLDLSDDLQWQLEFFSSDIFRSSVNKESNGREYSLAAQRSAHRRSRGLNE
VRRRRLWATLNLLIKHGFCPMSSESLMRWCTYDKRKGLFREYPIDDKDFNAWILLDFNGDGRPDYSSPYQ
LRRELVTRQFDFEQPIERYKLGRALYHIAQHRGFKSSKGETLSQQETNSKPSSTDEIPDVAGAMKASEEK
LSKGLSTYMKEHNLLTVGAAFAQLEDEGVRVRNNNDYRAIRSQFQHEIETIFKFQQGLSVESELYERLIS
EKKNVGTIFYKRPLRSQRGNVGKCTLERSKPRCAIGHPLFEKFRAWTLINNIKVRMSVDTLDEQLPMKLR
LDLYNECFLAFVRTEFKFEDIRKYLEKRLGIHFSYNDKTINYKDSTSVAGCPITARFRKMLGEEWESFRV
EGQKERQAHSKNNISFHRVSYSIEDIWHFCYDAEEPEAVLAFAQETLRLERKKAEELVRIWSAMPQGYAM
LSQKAIRNINKILMLGLKYSDAVILAKVPELVDVSDEELLSIAKDYYLVEAQVNYDKRINSIVNGLIAKY
KSVSEEYRFADHNYEYLLDESDEKDIIRQIENSLGARRWSLMDANEQTDILQKVRDRYQDFFRSHERKFV
ESPKLGESFENYLTKKFPMVEREQWKKLYHPSQITIYRPVSVGKDRSVLRLGNPDIGAIKNPTVLRVLNT
LRRRVNQLLDDGVISPDETRVVVETARELNDANRKWALDTYNRIRHDENEKIKKILEEFYPKRDGISTDD
IDKARYVIDQREVDYFTGSKTYNKDIKKYKFWLEQGGQCMYTGRTINLSNLFDPNAFDIEHTIPESLSFD
SSDMNLTLCDAHYNRFIKKNHIPTDMPNYDKAITIDGKEYPAITSQLQRWVERVERLNRNVEYWKGQARR
AQNKDRKDQCMREMHLWKMELEYWKKKLERFTVTEVTDGFKNSQLVDTRVITRHAVLYLKSIFPHVDVQR
GDVTAKFRKILGIQSVDEKKDRSLHSHHAIDATTLTIIPVSAKRDRMLELFAKIEEINKMLSFSGSEDRT
GLIQELEGLKNKLQMEVKVCRIGHNVSEIGTFINDNIIVNHHIKNQALTPVRRRLRKKGYIVGGVDNPRW
QTGDALRGEIHKASYYGAITQFAKDDEGKVLMKEGRPQVNPTIKFVIRRELKYKKSAADSGFASWDDLGK
AIVDKELFALMKGQFPAETSFKDACEQGIYMIKKGKNGMPDIKLHHIRHVRCEAPQSGLKIKEQTYKSEK
EYKRYFYAAVGDLYAMCCYTNGKIREFRIYSLYDVSCHRKSDIEDIPEFITDKKGNRLMLDYKLRTGDMI
LLYKDNPAELYDLDNVNLSRRLYKINRFESQSNLVLMTHHLSTSKERGRSLGKTVDYQNLPESIRSSVKS
LNFLIMGENRDFVIKNGKIIFNHR

SEQ ID NO:340
MLVSPISVDLGGKNTGFFSFTDSLDNSQSGTVIYDESFVLSQVGRRSKRHSKRNNLRNKLVKRLFLLILQ
EHHGLSIDVLPDEIRGLFNKRGYTYAGFELDEKKKDALESDTLKEFLSEKLQSIDRDSDVEDFLNQIASN
AESFKDYKKGFEAVFASATHSPNKKLELKDELKSEYGENAKELLAGLRVTKEILDEFDKQENQGNLPRAK
YFEELGEYIATNEKVKSFFDSNSLKLTDMTKLIGNISNYQLKELRRYFNDKEMEKGDIWIPNKLHKITER
FVRSWHPKNDADRQRRAELMKDLKSKEIMELLTTTEPVMTIPPYDDMNNRGAVKCQTLRLNEEYLDKHLP
NWRDIAKRLNHGKFNDDLADSTVKGYSEDSTLLHRLLDTSKEIDIYELRGKKPNELLVKTLGQSDANRLY
GFAQNYYELIRQKVRAGIWVPVKNKDDSLNLEDNSNMLKRCNHNPPHKKNQIHNLVAGILGVKLDEAKFA
EFEKELWSAKVGNKKLSAYCKNIEELRKTHGNTFKIDIEELRKKDPAELSKEEKAKLRLTDDVILNEWSQ
KIANFFDIDDKHRQRFNNLFSMAQLHTVIDTPRSGFSSTCKRCTAENRFRSETAFYNDETGEFHKKATAT
CQRLPADTQRPFSGKIERYIDKLGYELAKIKAKELEGMEAKEIKVPIILEQNAFEYEESLRKSKTGSNDR
VINSKKDRDGKKLAKAKENAEDRLKDKDKRIKAFSSGICPYCGDTIGDDGEIDHILPRSHTLKIYGTVFN

PEGNLIYVHQKCNQAKADSIYKLSDIKAGVSAQWIEEQVANIKGYKTFSVLSAEQQKAFRYALFLQNDNE
AYKKVVDWLRTDQSARVNGTQKYLAKKIQEKLTKMLPNKHLSFEFILADATEVSELRRQYARQNPLLAKA
EKQAPSSHAIDAVMAFVARYQKVFKDGTPPNADEVAKLAMLDSWNPASNEPLTKGLSTNQKIEKMIKSGD
YGQKNMREVFGKSIFGENAIGERYKPIVVQEGGYYIGYPATVKKGYELKNCKVVTSKNDIAKLEKIIKNQ
DLISLKENQYIKIFSINKQTISELSNRYFNMNYKNLVERDKEIVGLLEFIVENCRYYTKKVDVKFAPKYI
HETKYPFYDDWRRFDEAWRYLQENQNKTSSKDRFVIDKSSLNEYYQPDKNEYKLDVDTQPIWDDFCRWYF
LDRYKTANDKKSIRIKARKTFSLLAESGVQGKVFRAKRKIPTGYAYQALPMDNNVIAGDYANILLEANSK
TLSLVPKSGISIEKQLDKKLDVIKKTDVRGLAIDNNSFFNADFDTHGIRLIVENTSVKVGNFPISAIDKS
AKRMIFRALFEKEKGKRKKKTTISFKESGPVQDYLKVFLKKIVKIQLRTDGSISNIVVRKNAADFTLSFR
SEHIQKLLK

SEQ ID NO:341
MAYRLGLDIGITSVGWAVVALEKDESGLKPVRIQDLGVRIFDKAEDSKTGASLALPRREARSARRRTRRR
RHRLWRVKRLLEQHGILSMEQIEALYAQRTSSPDVYALRVAGLDRCLIAEEIARVLIHIAHRRGFQSNRK
SEIKDSDAGKLLKAVQENENLMQSKGYRTVAEMLVSEATKTDAEGKLVHGKKHGYVSNVRNKAGEYRHTV
SRQAIVDEVRKIFAAQRALGNDVMSEELEDSYLKILCSQRNFDDGPGGDSPYGHGSVSPDGVRQSIYERM
VGSCTFETGEKRAPRSSYSFERFQLLTKVVNLRIYRQQEDGGRYPCELTQTERARVIDCAYEQTKITYGK
LRKLLDMKDTESFAGLTYGLNRSRNKTEDTVFVEMKFYHEVRKALQRAGVFIQDLSIETLDQIGWILSVW
KSDDNRRKKLSTLGLSDNVIEELLPLNGSKFGHLSLKAIRKILPFLEDGYSYDVACELAGYQFQGKTEYV
KQRLLPPLGEGEVTNPVVRRALSQAIKVVNAVIRKHGSPESIHIELARELSKNLDERRKIEKAQKENQKN
NEQIKDEIREILGSAHVTGRDIVKYKLFKQQQEFCMYSGEKLDVTRLFEPGYAEVDHIIPYGISFDDSYD
NKVLVKTEQNRQKGNRTPLEYLRDKPEQKAKFIALVESIPLSQKKKNHLLMDKRAIDLEQEGFRERNLSD
TRYITRALMNHIQAWLLFDETASTRSKRVVCVNGAVTAYMRARWGLTKDRDAGDKHHAADAVVVACIGDS
LIQRVTKYDKFKRNALADRNRYVQQVSKSEGITQYVDKETGEVFTWESFDERKFLPNEPLEPWPFFRDEL
LARLSDDPSKNIRAIGLLTYSETEQIDPIFVSRMPTRKVTGAAHKETIRSPRIVKVDDNKGTEIQVVVSK
VALTELKLTKDGEIKDYFRPEDDPRLYNTLRERLVQFGGDAKAAFKEPVYKISKDGSVRTPVRKVKIQEK
LTLGVPVHGGRGIAENGGMVRIDVFAKGGKYYFVPIYVADVLKRELPNRLATAHKPYSEWRVVDDSYQFK
FSLYPNDAVMIKPSREVDITYKDRKEPVGCRIMYFVSANIASASISLRTHDNSGELEGLGIQGLEVFEKY
VVGPLGDTHPVYKERRMPFRVERKMN

SEQ ID NO:342
MPVLSPLSPNAAQGRRRWSLALDIGEGSIGWAVAEVDAEGRVLQLTGTGVTLFPSAWSNENGTYVAHGAA
DRAVRGQQQRHDSRRRRLAGLARLCAPVLERSPEDLKDLTRTPPKADPRAIFFLRADAARRPLDGPELFR
VLHHMAAHRGIRLAELQEVDPPPESDADDAAPAATEDEDGTRRAAADERAFRRLMAEHMHRHGTQPTCGE
IMAGRLRETPAGAQPVTRARDGLRVGGGVAVPTRALIEQEFDAIRAIQAPRHPDLPWDSLRRLVLDQAPI
AVPPATPCLFLEELRRRGETFQGRTITREAIDRGLTVDPLIQALRIRETVGNLRLHERITEPDGRQRYVP
RAMPELGLSHGELTAPERDTLVRALMHDPDGLAAKDGRIPYTRLRKLIGYDNSPVCFAQERDTSGGGITV
NPTDPLMARWIDGWVDLPLKARSLYVRDVVARGADSAALARLLAEGAHGVPPVAAAAVPAATAAILESDI
MQPGRYSVCPWAAEAILDAWANAPTEGFYDVTRGLFGFAPGEIVLEDLRRARGALLAHLPRTMAAARTPN
RAAQQRGPLPAYESVIPSQLITSLRRAHKGRAADWSAADPEERNPFLRTWTGNAATDHILNQVRKTANEV
ITKYGNRRGWDPLPSRITVELAREAKHGVIRRNEIAKENRENEGRRKKESAALDTFCQDNTVSWQAGGLP
KERAALRLRLAQRQEFFCPYCAERPKLRATDLFSPAETEIDHVIERRMGGDGPDNLVLAHKDCNNAKGKK
TPHEHAGDLLDSPALAALWQGWRKENADRLKGKGHKARTPREDKDFMDRVGWRFEEDARAKAEENQERRG
RRMLHDTARATRLARLYLAAAVMPEDPAEIGAPPVETPPSPEDPTGYTAIYRTISRVQPVNGSVTHMLRQ
RLLQRDKNRDYQTHHAEDACLLLLAGPAVVQAFNTEAAQHGADAPDDRPVDLMPTSDAYHQQRRARALGR
VPLATVDAALADIVMPESDRQDPETGRVHWRLTRAGRGLKRRIDDLTRNCVILSRPRRPSETGTPGALHN
ATHYGRREITVDGRTDTVVTQRMNARDLVALLDNAKIVPAARLDAAAPGDTILKEICTEIADRHDRVVDP
EGTHARRWISARLAALVPAHAEAVARDIAELADLDALADADRTPEQEARRSALRQSPYLGRAISAKKADG
RARAREQEILTRALLDPHWGPRGLRHLIMREARAPSLVRIRANKTDAFGRPVPDAAVWVKTDGNAVSQLW
RLTSVVTDDGRRIPLPKPIEKRIEISNLEYARLNGLDEGAGVTGNNAPPRPLRQDIDRLTPLWRDHGTAP
GGYLGTAVGELEDKARSALRGKAMRQTLTDAGITAEAGWRLDSEGAVCDLEVAKGDTVKKDGKTYKVGVI
TQGIFGMPVDAAGSAPRTPEDCEKFEEQYGIKPWKAKGIPLA

SEQ ID NO:343
MNYTEKEKLFMKYILALDIGIASVGWAILDKESETVIEAGSNIFPEASAADNQLRRDMRGAKRNNRRLKT
RINDFIKLWENNNLSIPQFKSTEIVGLKVRAITEEITLDELYLILYSYLKHRGISYLEDALDDTVSGSSA
YANGLKLNAKELETHYPCEIQQERLNTIGKYRGQSQIINENGEVLDLSNVFTIGAYRKEIQRVFEIQKKY
HPELTDEFCDGYMLIFNRKRKYYEGPGNEKSRTDYGRFTTKLDANGNYITEDNIFEKLIGKCSVYPDELR
AAAASYTAQEYNVLNDLNNLTINGRKLEENEKHEIVERIKSSNTINMRKIISDCMGENIDDFAGARIDKS
GKEIFHKFEVYNKMRKALLEIGIDISNYSREELDEIGYIMTINTDKEAMMEAFQKSWIDLSDDVKQCLIN
MRKTNGALFNKWQSFSLKIMNELIPEMYAQPKEQMTLLTEMGVTKGTQEEFAGLKYIPVDVVSEDIFNPV
VRRSVRISFKILNAVLKKYKALDTIVIEMPRDRNSEEQKKRINDSQKLNEKEMEYIEKKLAVTYGIKLSP
SDFSSQKQLSLKLKLWNEQDGICLYSGKTIDPNDIINNPQLFEIDHIIPRSISFDDARSNKVLVYRSENQ
KKGNQTPYYYLTHSHSEWSFEQYKATVMNLSKKKEYAISRKKIQNLLYSEDITKMDVLKGFINRNINDTS
YASRLVLNTIQNFFMANEADTKVKVIKGSYTHQMRCNLKLDKNRDESYSHHAVDAMLIGYSELGYEAYHK
LQGEFIDFETGEILRKDMWDENMSDEVYADYLYGKKWANIRNEVVKAEKNVKYWHYVMRKSNRGLCNQTI
RGTREYDGKQYKINKLDIRTKEGIKVFAKLAFSKKDSDRERLLVYLNDRRTFDDLCKIYEDYSDAANPFV
QYEKETGDIIRKYSKKHNGPRIDKLKYKDGEVGACIDISHKYGFEKGSKKVILESLVPYRMDVYYKEENH
SYYLVGVKQSDIKFEKGRNVIDEEAYARILVNEKMIQPGQSRADLENLGFKFKLSFYKNDIIEYEKDGKI
YTERLVSRTMPKQRNYIETKPIDKAKFEKQNLVGLGKTKFIKKYRYDILGNKYSCSEEKFTSFC

SEQ ID NO:344
MLRLYCANNLVLNNVQNLWKYLLLLIFDKKIIFLFKIKVILIRRYMENNNKEKIVIGFDLGVASVGWSIV
NAETKEVIDLGVRLFSEPEKADYRRAKRTTRRLLRRKKFKREKFHKLILKNAEIFGLQSRNEILNVYKDQ
SSKYRNILKLKINALKEEIKPSELVWILRDYLQNRGYFYKNEKLTDEFVSNSFPSKKLHEHYEKYGFFRG
SVKLDNKLDNKKDKAKEKDEEEESDAKKESEELIFSNKQWINEIVKVFENQSYLTESFKEEYLKLFNYVR
PFNKGPGSKNSRTAYGVFSTDIDPETNKFKDYSNIWDKTIGKCSLFEEEIRAPKNLPSALIFNLQNEICT
IKNEFTEFKNWWLNAEQKSEILKFVFTELFNWKDKKYSDKKFNKNLQDKIKKYLLNFALENFNLNEEILK
NRDLENDTVLGLKGVKYYEKSNATADAALEFSSLKPLYVFIKFLKEKKLDLNYLLGLENTEILYFLDSIY
LAISYSSDLKERNEWFKKLLKELYPKIKNNNLEIIENVEDIFEITDQEKFESFSKTHSLSREAFNHIIPL
LLSNNEGKNYESLKHSNEELKKRTEKAELKAQQNQKYLKDNFLKEALVPLSVKTSVLQAIKIFNQIIKNF
GKKYEISQVVIEMARELTKPNLEKLLNNATNSNIKILKEKLDQTEKFDDFTKKKFIDKIENSVVFRNKLF
LWFEQDRKDPYTQLDIKINEIEDETEIDHVIPYSKSADDSWFNKLLVKKSTNQLKKNKTVWEYYQNESDP
EAKWNKFVAWAKRIYLVQKSDKESKDNSEKNSIFKNKKPNLKFKNITKKLFDPYKDLGFLARNLNDTRYA
TKVFRDQLNNYSKHHSKDDENKLFKVVCMNGSITSFLRKSMWRKNEEQVYRFNFWKKDRDQFFHHAVDAS
IIAIFSLLTKTLYNKLRVYESYDVQRREDGVYLINKETGEVKKADKDYWKDQHNFLKIRENAIEIKNVLN
NVDFQNQVRYSRKANTKLNTQLFNETLYGVKEFENNFYKLEKVNLFSRKDLRKFILEDLNEESEKNKKNE
NGSRKRILTEKYIVDEILQILENEEFKDSKSDINALNKYMDSLPSKFSEFFSQDFINKCKKENSLILTFD
AIKHNDPKKVIKIKNLKFFREDATLKNKQAVHKDSKNQIKSFYESYKCVGFIWLKNKNDLEESIFVPINS
RVIHFGDKDKDIFDFDSYNKEKLLNEINLKRPENKKFNSINEIEFVKFVKPGALLLNFENQQIYYISTLE
SSSLRAKIKLLNKMDKGKAVSMKKITNPDEYKIIEHVNPLGINLNWTKKLENNN

SEQ ID NO:345
MLMSKHVLGLDLGVGSIGWCLIALDAQGDPAEILGMGSRVVPLNNATKAIEAFNAGAAFTASQERTARRT
MRRGFARYQLRRYRLRRELEKVGMLPDAALIQLPLLELWELRERAATAGRRLTLPELGRVLCHINQKRGY
RHVKSDAAAIVGDEGEKKKDSNSAYLAGIRANDEKLQAEHKTVGQYFAEQLRQNQSESPTGGISYRIKDQ
IFSRQCYIDEYDQIMAVQRVHYPDILTDEFIRMLRDEVIFMQRPLKSCKHLVSLCEFEKQERVMRVQQDD
GKGGWQLVERRVKFGPKVAPKSSPLFQLCCIYEAVNNIRLTRPNGSPCDITPEERAKIVAHLQSSASLSF
AALKKLLKEKALIADQLTSKSGLKGNSTRVALASALQPYPQYHHLLDMELETRMMTVQLTDEETGEVTER
EVAVVTDSYVRKPLYRLWHILYSIEEREAMRRALITQLGMKEEDLDGGLLDQLYRLDFVKPGYGNKSAKF
ICKLLPQLQQGLGYSEACAAVGYRHSNSPTSEEITERTLLEKIPLLQRNELRQPLVEKILNQMINLVNAL
KAEYGIDEVRVELARELKMSREERERMARNNKDREERNKGVAAKIRECGLYPTKPRIQKYMLWKEAGRQC
LYCGRSIEEEQCLREGGMEVEHIIPKSVLYDDSYGNKTCACRRCNKEKGNRTALEYIRAKGREAEYMKRI
NDLLKEKKISYSKHQRLRWLKEDIPSDFLERQLRLTQYISRQAMAILQQGIRRVSASEGGVTARLRSLWG

YGKILHTLNLDRYDSMGETERVSREGEATEELHITNWSKRMDHRHHAIDALVVACTRQSYIQRLNRLSSE
FGREDKKKEDQEAQEQQATETGRLSNLERWLTQRPHFSVRTVSDKVAEILISYRPGQRVVTRGRNIYRKK
MADGREVSCVQRGVLVPRGELMEASFYGKILSQGRVRIVKRYPLHDLKGEVVDPHLRELITTYNQELKSR
EKGAPIPPLCLDKDKKQEVRSVRCYAKTLSLDKAIPMCFDEKGEPTAFVKSASNHHLALYRTPKGKLVES
IVTFWDAVDRARYGIPLVITHPREVMEQVLQRGDIPEQVLSLLPPSDWVFVDSLQQDEMVVIGLSDEELQ
RALEAQNYRKISEHLYRVQKMSSSYYVFRYHLETSVADDKNTSGRIPKFHRVQSLKAYEERNIRKVRVDL
LGRISLL

SEQ ID NO:346
MSDLVLGLDIGIGSVGVGILNKVTGEIIHKNSRIFPAAQAENNLVRRTNRQGRRLARRKKHRRVRLNRLF
EESGLITDFTKISINLNPYQLRVKGLTDELSNEELFIALKNMVKHRGISYLDDASDDGNSSVGDYAQIVK
ENSKQLETKTPGQIQLERYQTYGQLRGDFTVEKDGKKHRLINVFPTSAYRSEALRILQTQQEFNPQITDE
FINRYLEILTGKRKYYHGPGNEKSRTDYGRYRTSGETLDNIFGILIGKCTFYPDEFRAAKASYTAQEFNL
LNDLNNLTVPTETKKLSKEQKNQIINYVKNEKAMGPAKLFKYIAKLLSCDVADIKGYRIDKSGKAEIHTF
EAYRKMKTLETLDIEQMDRETLDKLAYVLTLNTEREGIQEALEHEFADGSFSQKQVDELVQFRKANSSIF
GKGWHNFSVKLMMELIPELYETSEEQMTILTRLGKQKTTSSSNKTKYIDEKLLTEEIYNPVVAKSVRQAI
KIVNAAIKEYGDFDNIVIEMARETNEDDEKKAIQKIQKANKDEKDAAMLKAANQYNGKAELPHSVFHGHK
QLATKIRLWHQQGERCLYTGKTISIHDLINNSNQFEVDHILPLSITFDDSLANKVLVYATANQEKGQRTP
YQALDSMDDAWSFRELKAFVRESKTLSNKKKEYLLTEEDISKFDVRKKFIERNLVDTRYASRVVLNALQE
HFRAHKIDTKVSVVRGQFTSQLRRHWGIEKTRDTYHHHAVDALIIAASSQLNLWKKQKNTLVSYSEDQLL
DIETGELISDDEYKESVFKAPYQHFVDTLKSKEFEDSILFSYQVDSKFNRKISDATIYATRQAKVGKDKA
DETYVLGKIKDIYTQDGYDAFMKIYKKDKSKFLMYRHDPQTFEKVIEPILENYPNKQINEKGKEVPCNPF
LKYKEEHGYIRKYSKKGNGPEIKSLKYYDSKLGNHIDITPKDSNNKVVLQSVSPWRADVYFNKTTGKYEI
LGLKYADLQFEKGTGTYKISQEKYNDIKKKEGVDSDSEFKFTLYKNDLLLVKDTETKEQQLFRFLSRTMP
KQKHYVELKPYDKQKFEGGEALIKVLGNVANSGQCKKGLGKSNISIYKVRTDVLGNQHIIKNEGDKPKLD
F

SEQ ID NO:347
MNAEHGKEGLLIMEENFQYRIGLDIGITSVGWAVLQNNSQDEPVRITDLGVRIFDVAENPKNGDALAAPR
RDARTTRRRLRRRRHRLERIKFLLQENGLIEMDSFMERYYKGNLPDVYQLRYEGLDRKLKDEELAQVLIH
IAKHRGFRSTRKAETKEKEGGAVLKATTENQKIMQEKGYRTVGEMLYLDEAFHTECLWNEKGYVLTPRNR
PDDYKHTILRSMLVEEVHAIFAAQRAHGNQKATEGLEEAYVEIMTSQRSFDMGPGLQPDGKPSPYAMEGF
GDRVGKCTFEKDEYRAPKATYTAELFVALQKINHTKLIDEFGTGRFFSEEERKTIIGLLLSSKELKYGTI
RKKLNIDPSLKFNSLNYSAKKEGETEEERVLDTEKAKFASMFWTYEYSKCLKDRTEEMPVGEKADLFDRI
GEILTAYKNDDSRSSRLKELGLSGEEIDGLLDLSPAKYQRVSLKAMRKMQPYLEDGLIYDKACEAAGYDF
RALNDGNKKHLLKGEEINAIVNDITNPVVKRSVSQTIKVINAIIQKYGSPQAVNIELAREMSKNFQDRTN
LEKEMKKRQQENERAKQQIIELGKQNPTGQDILKYRLWNDQGGYCLYSGKKIPLEELFDGGYDIDHILPY
SITFDDSYRNKVLVTAQENRQKGNRTPYEYFGADEKRWEDYEASVRLLVRDYKKQQKLLKKNFTEEERKE
FKERNLNDTKYITRVVYNMIRQNLELEPFNHPEKKKQVWAVNGAVTSYLRKRWGLMQKDRSTDRHHAMDA
VVIACCTDGMIHKISRYMQGRELAYSRNFKFPDEETGEILNRDNFTREQWDEKFGVKVPLPWNSFRDELD
IRLLNEDPKNFLLTHADVQRELDYPGWMYGEEESPIEEGRYINYIRPLFVSRMPNHKVTGSAHDATIRSA
RDYETRGVVITKVPLTDLKLNKDNEIEGYYDKDSDRLLYQALVRQLLLHGNDGKKAFAEDFHKPKADGTE
GPVVRKVKIEKKQTSGVMVRGGTGIAANGEMVRIDVFRENGKYYFVPVYTADVVRKVLPNRAATHTKPYS
EWRVMDDANFVFSLYSRDLIHVKSKKDIKTNLVNGGLLLQKEIFAYYTGADIATASIAGFANDSNFKFRG
LGIQSLEIFEKCQVDILGNISVVRHENRQEFH

SEQ ID NO:348
MRVLGLDAGIASLGWALIEIEESNRGELSQGTIIGAGTWMFDAPEEKTQAGAKLKSEQRRTFRGQRRVVR
RRRQRMNEVRRILHSHGLLPSSDRDALKQPGLDPWRIRAEALDRLLGPVELAVALGHIARHRGFKSNSKG
AKTNDPADDTSKMKRAVNETREKLARFGSAAKMLVEDESFVLRQTPTKNGASEIVRRFRNREGDYSRSLL
RDDLAAEMRALFTAQARFQSAIATADLQTAFTKAAFFQRPLQDSEKLVGPCPFEVDEKRAPKRGYSFELF
RFLSRLNHVTLRDGKQERTLTRDELALAAADFGAAAKVSFTALRKKLKLPETTVFVGVKADEESKLDVVA

RSGKAAEGTARLRSVIVDALGELAWGALLCSPEKLDKIAEVISFRSDIGRISEGLAQAGCNAPLVDALTA
AASDGRFDPFTGAGHISSKAARNILSGLRQGMTYDKACCAADYDHTASRERGAFDVGGHGREALKRILQE
ERISRELVGSPTARKALIESIKQVKAIVERYGVPDRIHVELARDVGKSIEEREEITRGIEKRNRQKDKLR
GLFEKEVGRPPQDGARGKEELLRFELWSEQMGRCLYTDDYISPSQLVATDDAVQVDHILPWSRFADDSYA
NKTLCMAKANQDKKGRTPYEWFKAEKTDTEWDAFIVRVEALADMKGFKKRNYKLRNAEEAAAKFRNRNLN
DTRWACRLLAEALKQLYPKGEKDKDGKERRRVFSRPGALTDRLRRAWGLQWMKKSTKGDRIPDDRHHALD
AIVIAATTESLLQRATREVQEIEDKGLHYDLVKNVTPPWPGFREQAVEAVEKVFVARAERRRARGKAHDA
TIRHIAVREGEQRVYERRKVAELKLADLDRVKDAERNARLIEKLRNWIEAGSPKDDPPLSPKGDPIFKVR
LVTKSKVNIALDTGNPKRPGTVDRGEMARVDVFRKASKKGKYEYYLVPIYPHDIATMKTPPIRAVQAYKP
EDEWPEMDSSYEFCWSLVPMTYLQVISSKGEIFEGYYRGMNRSVGAIQLSAHSNSSDVVQGIGARTLTEF
KKFNVDRFGRKHEVERELRTWRGETWRGKAYI

SEQ ID NO:349
MGNYYLGLDVGIGSIGWAVINIEKKRIEDFNVRIFKSGEIQEKNRNSRASQQCRRSRGLRRLYRRKSHRK
LRLKNYLSIIGLTTSEKIDYYYETADNNVIQLRNKGLSEKLTPEEIAACLIHICNNRGYKDFYEVNVEDI
EDPDERNEYKEEHDSIVLISNLMNEGGYCTPAEMICNCREFDEPNSVYRKFHNSAASKNHYLITRHMLVK
EVDLILENQSKYYGILDDKTIAKIKDIIFAQRDFEIGPGKNERFRRFTGYLDSIGKCQFFKDQERGSRFT
VIADIYAFVNVLSQYTYTNNRGESVFDTSFANDLINSALKNGSMDKRELKAIAKSYHIDISDKNSDTSLT
KCFKYIKVVKPLFEKYGYDWDKLIENYTDTDNNVLNRIGIVLSQAQTPKRRREKLKALNIGLDDGLINEL
TKLKLSGTANVSYKYMQGSIEAFCEGDLYGKYQAKFNKEIPDIDENAKPQKLPPFKNEDDCEFFKNPVVF
RSINETRKLINAIIDKYGYPAAVNIETADELNKTFEDRAIDTKRNNDNQKENDRIVKEIIECIKCDEVHA
RHLIEKYKLWEAQEGKCLYSGETITKEDMLRDKDKLFEVDHIVPYSLILDNTINNKALVYAEENQKKGQR
TPLMYMNEAQAADYRVRVNTMFKSKKCSKKKYQYLMLPDLNDQELLGGWRSRNLNDTRYICKYLVNYLRK
NLRFDRSYESSDEDDLKIRDHYRVFPVKSRFTSMFRRWWLNEKTWGRYDKAELKKLTYLDHAADAIIIAN
CRPEYVVLAGEKLKLNKMYHQAGKRITPEYEQSKKACIDNLYKLFRMDRRTAEKLLSGHGRLTPIIPNLS
EEVDKRLWDKNIYEQFWKDDKDKKSCEELYRENVASLYKGDPKFASSLSMPVISLKPDHKYRGTITGEEA
IRVKEIDGKLIKLKRKSISEITAESINSIYTDDKILIDSLKTIFEQADYKDVGDYLKKTNQHFFTTSSGK
RVNKVTVIEKVPSRWLRKEIDDNNFSLLNDSSYYCIELYKDSKGDNNLQGIAMSDIVHDRKTKKLYLKPD
FNYPDDYYTHVMYIFPGDYLRIKSTSKKSGEQLKFEGYFISVKNVNENSFRFISDNKPCAKDKRVSITKK
DIVIKLAVDLMGKVQGENNGKGISCGEPLSLLKEKN

SEQ ID NO:350
MLSRQLLGASHLARPVSYSYNVQDNDVHCSYGERCFMRGKRYRIGIDVGLNSVGLAAVEVSDENSPVRLL
NAQSVIHDGGVDPQKNKEAITRKNMSGVARRTRRMRRRKRERLHKLDMLLGKFGYPVIEPESLDKPFEEW
HVRAELATRYIEDDELRRESISIALRHMARHRGWRNPYRQVDSLISDNPYSKQYGELKEKAKAYNDDATA
AEEESTPAQLVVAMLDAGYAEAPRLRWRTGSKKPDAEGYLPVRLMQEDNANELKQIFRVQRVPADEWKPL
FRSVFYAVSPKGSAEQRVGQDPLAPEQARALKASLAFQEYRIANVITNLRIKDASAELRKLTVDEKQSIY
DQLVSPSSEDITWSDLCDFLGFKRSQLKGVGSLTEDGEERISSRPPRLTSVQRIYESDNKIRKPLVAWWK
SASDNEHEAMIRLLSNTVDIDKVREDVAYASAIEFIDGLDDDALTKLDSVDLPSGRAAYSVETLQKLTRQ
MLTTDDDLHEARKTLFNVTDSWRPPADPIGEPLGNPSVDRVLKNVNRYLMNCQQRWGNPVSVNIEHVRSS
FSSVAFARKDKREYEKNNEKRSIFRSSLSEQLRADEQMEKVRESDLRRLEAIQRQNGQCLYCGRTITFRT
CEMDHIVPRKGVGSTNTRTNFAAVCAECNRMKSNTPFAIWARSEDAQTRGVSLAEAKKRVTMFTFNPKSY
APREVKAFKQAVIARLQQTEDDAAIDNRSIESVAWMADELHRRIDWYFNAKQYVNSASIDDAEAETMKTT
VSVFQGRVTASARRAAGIEGKIHFIGQQSKTRLDRRHHAVDASVIAMMNTAAAQTLMERESLRESQRLIG
LMPGERSWKEYPYEGTSRYESFHLWLDNMDVLLELLNDALDNDRIAVMQSQRYVLGNSIAHDATIHPLEK
VPLGSAMSADLIRRASTPALWCALTRLPDYDEKEGLPEDSHREIRVHDTRYSADDEMGFFASQAAQIAVQ
EGSADIGSAIHHARVYRCWKTNAKGVRKYFYGMIRVFQTDLLRACHDDLFTVPLPPQSISMRYGEPRVVQ
ALQSGNAQYLGSLVVGDEIEMDFSSLDVDGQIGEYLQFFSQFSGGNLAWKHWVVDGFFNQTQLRIRPRYL
AAEGLAKAFSDDVVPDGVQKIVTKQGWLPPVNTASKTAVRIVRRNAFGEPRLSSAHHMPCSWQWRHE

SEQ ID NO:351

MYSIGLDLGISSVGWSVIDERTGNVIDLGVRLFSAKNSEKNLERRTNRGGRRLIRRKTNRLKDAKKILAA
VGFYEDKSLKNSCPYQLRVKGLTEPLSRGEIYKVTLHILKKRGISYLDEVDTEAAKESQDYKEQVRKNAQ
LLTKYTPGQIQLQRLKENNRVKTGINAQGNYQLNVFKVSAYANELATILKTQQAFYPNELTDDWIALFVQ
PGIAEEAGLIYRKRPYYHGPGNEANNSPYGRWSDFQKTGEPATNIFDKLIGKDFQGELRASGLSLSAQQY
NLLNDLTNLKIDGEVPLSSEQKEYILTELMTKEFTRFGVNDVVKLLGVKKERLSGWRLDKKGKPEIHTLK
GYRNWRKIFAEAGIDLATLPTETIDCLAKVLTLNTEREGIENTLAFELPELSESVKLLVLDRYKELSQSI
STQSWHRFSLKTLHLLIPELMNATSEQNTLLEQFQLKSDVRKRYSEYKKLPTKDVLAEIYNPTVNKTVSQ
AFKVIDALLVKYGKEQIRYITIEMPRDDNEEDEKKRIKELHAKNSQRKNDSQSYFMQKSGWSQEKFQTTI
QKNRRFLAKLLYYYEQDGICAYTGLPISPELLVSDSTEIDHIIPISISLDDSINNKVLVLSKANQVKGQQ
TPYDAWMDGSFKKINGKFSNWDDYQKWVESRHFSHKKENNLLETRNIFDSEQVEKFLARNLNDTRYASRL
VLNTLQSFFTNQETKVRVVNGSFTHTLRKKWGADLDKTRETHHHHAVDATLCAVTSFVKVSRYHYAVKEE
TGEKVMREIDFETGEIVNEMSYWEFKKSKKYERKTYQVKWPNFREQLKPVNLHPRIKFSHQVDRKANRKL
SDATIYSVREKTEVKTLKSGKQKITTDEYTIGKIKDIYTLDGWEAFKKKQDKLLMKDLDEKTYERLLSIA
ETTPDFQEVEEKNGKVKRVKRSPFAVYCEENDIPAIQKYAKKNNGPLIRSLKYYDGKLNKHINITKDSQG
RPVEKTKNGRKVTLQSLKPYRYDIYQDLETKAYYTVQLYYSDLRFVEGKYGITEKEYMKKVAEQTKGQVV
RFCFSLQKNDGLEIEWKDSQRYDVRFYNFQSANSINFKGLEQEMMPAENQFKQKPYNNGAINLNIAKYGK
EGKKLRKFNTDILGKKHYLFYEKEPKNIIK

SEQ ID NO:352
MYFYKNKENKLNKKVVLGLDLGIASVGWCLTDISQKEDNKFPIILHGVRLFETVDDSDDKLLNETRRKKR
GQRRRNRRLFTRKRDFIKYLIDNNIIELEFDKNPKILVRNFIEKYINPFSKNLELKYKSVTNLPIGFHNL
RKAAINEKYKLDKSELIVLLYFYLSLRGAFFDNPEDTKSKEMNKNEIEIFDKNESIKNAEFPIDKIIEFY
KISGKIRSTINLKFGHQDYLKEIKQVFEKQNIDFMNYEKFAMEEKSFFSRIRNYSEGPGNEKSFSKYGLY
ANENGNPELIINEKGQKIYTKIFKTLWESKIGKCSYDKKLYRAPKNSFSAKVFDITNKLTDWKHKNEYIS
ERLKRKILLSRFLNKDSKSAVEKILKEENIKFENLSEIAYNKDDNKINLPIINAYHSLTTIFKKHLINFE
NYLISNENDLSKLMSFYKQQSEKLFVPNEKGSYEINQNNNVLHIFDAISNILNKFSTIQDRIRILEGYFE
FSNLKKDVKSSEIYSEIAKLREFSGTSSLSFGAYYKFIPNLISEGSKNYSTISYEEKALQNQKNNFSHSN
LFEKTWVEDLIASPTVKRSLRQTMNLLKEIFKYSEKNNLEIEKIVVEVTRSSNNKHERKKIEGINKYRKE
KYEELKKVYDLPNENTTLLKKLWLLRQQQGYDAYSLRKIEANDVINKPWNYDIDHIVPRSISFDDSFSNL
VIVNKLDNAKKSNDLSAKQFIEKIYGIEKLKEAKENWGNWYLRNANGKAFNDKGKFIKLYTIDNLDEFDN
SDFINRNLSDTSYITNALVNHLTFSNSKYKYSVVSVNGKQTSNLRNQIAFVGIKNNKETEREWKRPEGFK
SINSNDFLIREEGKNDVKDDVLIKDRSFNGHHAEDAYFITIISQYFRSFKRIERLNVNYRKETRELDDLE
KNNIKFKEKASFDNFLLINALDELNEKLNQMRFSRMVITKKNTQLFNETLYSGKYDKGKNTIKKVEKLNL
LDNRTDKIKKIEEFFDEDKLKENELTKLHIFNHDKNLYETLKIIWNEVKIEIKNKNLNEKNYFKYFVNKK
LQEGKISFNEWVPILDNDFKIIRKIRYIKFSSEEKETDEIIFSQSNFLKIDQRQNFSFHNTLYWVQIWVY
KNQKDQYCFISIDARNSKFEKDEIKINYEKLKTQKEKLQIINEEPILKINKGDLFENEEKELFYIVGRDE
KPQKLEIKYILGKKIKDQKQIQKPVKKYFPNWKKVNLTYMGEIFKK

SEQ ID NO:353
MDNKNYRIGIDVGLNSIGFCAVEVDQHDTPLGFLNLSVYRHDAGIDPNGKKTNTTRLAMSGVARRTRRLF
RKRKRRLAALDRFIEAQGWTLPDHADYKDPYTPWLVRAELAQTPIRDENDLHEKLAIAVRHIARHRGWRS
PWVPVRSLHVEQPPSDQYLALKERVEAKTLLQMPEGATPAEMVVALDLSVDVNLRPKNREKTDTRPENKK
PGFLGGKLMQSDNANELRKIAKIQGLDDALLRELIELVFAADSPKGASGELVGYDVLPGQHGKRRAEKAH
PAFQRYRIASIVSNLRIRHLGSGADERLDVETQKRVFEYLLNAKPTADITWSDVAEEIGVERNLLMGTAT
QTADGERASAKPPVDVTNVAFATCKIKPLKEWWLNADYEARCVMVSALSHAEKLTEGTAAEVEVAEFLQN
LSDEDNEKLDSFSLPIGRAAYSVDSLERLTKRMIENGEDLFEARVNEFGVSEDWRPPAEPIGARVGNPAV
DRVLKAVNRYLMAAEAEWGAPLSVNIEHVREGFISKRQAVEIDRENQKRYQRNQAVRSQIADHINATSGV
RGSDVTRYLAIQRQNGECLYCGTAITFVNSEMDHIVPRAGLGSTNTRDNLVATCERCNKSKSNKPFAVWA
AECGIPGVSVAEALKRVDFWIADGFASSKEHRELQKGVKDRLKRKVSDPEIDNRSMESVAWMARELAHRV
QYYFDEKHTGTKVRVFRGSLTSAARKASGFESRVNFIGGNGKTRLDRRHHAMDAATVAMLRNSVAKTLVL
RGNIRASERAIGAAETWKSFRGENVADRQIFESWSENMRVLVEKFNLALYNDEVSIFSSLRLQLGNGKAH
DDTITKLQMHKVGDAWSLTEIDRASTPALWCALTRQPDFTWKDGLPANEDRTIIVNGTHYGPLDKVGIFG

KAAASLLVRGGSVDIGSAIHHARIYRIAGKKPTYGMVRVFAPDLLRYRNEDLFNVELPPQSVSMRYAEPK
VREAIREGKAEYLGWLVVGDELLLDLSSETSGQIAELQQDFPGTTHWTVAGFFSPSRLRLRPVYLAQEGL
GEDVSEGSKSIIAGQGWRPAVNKVFGSAMPEVIRRDGLGRKRRFSYSGLPVSWQG

SEQ ID NO:354
MRLGLDIGTSSIGWWLYETDGAGSDARITGVVDGGVRIFSDGRDPKSGASLAVDRRAARAMRRRRDRYLR
RRATLMKVLAETGLMPADPAEAKALEALDPFALRAAGLDEPLPLPHLGRALFHLNQRRGFKSNRKTDRGD
NESGKIKDATARLDMEMMANGARTYGEFLHKRRQKATDPRHVPSVRTRLSIANRGGPDGKEEAGYDFYPD
RRHLEEEFHKLWAAQGAHHPELTETLRDLLFEKIFFQRPLKEPEVGLCLFSGHHGVPPKDPRLPKAHPLT
QRRVLYETVNQLRVTADGREARPLTREERDQVIHALDNKKPTKSLSSMVLKLPALAKVLKLRDGERFTLE
TGVRDAIACDPLRASPAHPDRFGPRWSILDADAQWEVISRIRRVQSDAEHAALVDWLTEAHGLDRAHAEA
TAHAPLPDGYGRLGLTATTRILYQLTADVVTYADAVKACGWHHSDGRTGECFDRLPYYGEVLERHVIPGS
YHPDDDDITRFGRITNPTVHIGLNQLRRLVNRIIETHGKPHQIVVELARDLKKSEEQKRADIKRIRDTTE
AAKKRSEKLEELEIEDNGRNRMLLRLWEDLNPDDAMRRFCPYTGTRISAAMIFDGSCDVDHILPYSRTLD
DSFPNRTLCLREANRQKRNQTPWQAWGDTPHWHAIAANLKNLPENKRWRFAPDAMTRFEGENGFLDRALK
DTQYLARISRSYLDTLFTKGGHVWVVPGRFTEMLRRHWGLNSLLSDAGRGAVKAKNRTDHRHHAIDAAVI
AATDPGLLNRISRAAGQGEAAGQSAELIARDTPPPWEGFRDDLRVRLDRIIVSHRADHGRIDHAARKQGR
DSTAGQLHQETAYSIVDDIHVASRTDLLSLKPAQLLDEPGRSGQVRDPQLRKALRVATGGKTGKDFENAL
RYFASKPGPYQAIRRVRIIKPLQAQARVPVPAQDPIKAYQGGSNHLFEIWRLPDGEIEAQVITSFEAHTL
EGEKRPHPAAKRLLRVHKGDMVALERDGRRVVGHVQKMDIANGLFIVPHNEANADTRNNDKSDPFKWIQI
GARPAIASGIRRVSVDEIGRLRDGGTRPI

SEQ ID NO:355
MLHCIAVIRVPPSEEPGFFETHADSCALCHHGCMTYAANDKAIRYRVGIDVGLRSIGFCAVEVDDEDHPI
RILNSVVHVHDAGTGGPGETESLRKRSGVAARARRRGRAEKQRLKKLDVLLEELGWGVSSNELLDSHAPW
HIRKRLVSEYIEDETERRQCLSVAMAHIARHRGWRNSFSKVDTLLLEQAPSDRMQGLKERVEDRTGLQFS
EEVTQGELVATLLEHDGDVTIRGFVRKGGKATKVHGVLEGKYMQSDLVAELRQICRTQRVSETTFEKLVL
SIFHSKEPAPSAARQRERVGLDELQLALDPAAKQPRAERAHPAFQKFKVVATLANMRIREQSAGERSLTS
EELNRVARYLLNHTESESPTWDDVARKLEVPRHRLRGSSRASLETGGGLTYPPVDDTTVRVMSAEVDWLA
DWWDCANDESRGHMIDAISNGCGSEPDDVEDEEVNELISSATAEDMLKLELLAKKLPSGRVAYSLKTLRE
VTAAILETGDDLSQAITRLYGVDPGWVPTPAPIEAPVGNPSVDRVLKQVARWLKFASKRWGVPQTVNIEH
TREGLKSASLLEEERERWERFEARREIRQKEMYKRLGISGPFRRSDQVRYEILDLQDCACLYCGNEINFQ
TFEVDHIIPRVDASSDSRRTNLAAVCHSCNSAKGGLAFGQWVKRGDCPSGVSLENAIKRVRSWSKDRLGL
TEKAMGKRKSEVISRLKTEMPYEEFDGRSMESVAWMAIELKKRIEGYFNSDRPEGCAAVQVNAYSGRLTA
CARRAAHVDKRVRLIRLKGDDGHHKNRFDRRNHAMDALVIALMTPAIARTIAVREDRREAQQLTRAFESW
KNFLGSEERMQDRWESWIGDVEYACDRLNELIDADKIPVTENLRLRNSGKLHADQPESLKKARRGSKRPR
PQRYVLGDALPADVINRVTDPGLWTALVRAPGFDSQLGLPADLNRGLKLRGKRISADFPIDYFPTDSPAL
AVQGGYVGLEFHHARLYRIIGPKEKVKYALLRVCAIDLCGIDCDDLFEVELKPSSISMRTADAKLKEAMG
NGSAKQIGWLVLGDEIQIDPTKFPKQSIGKFLKECGPVSSWRVSALDTPSKITLKPRLLSNEPLLKTSRV
GGHESDLVVAECVEKIMKKTGWVVEINALCQSGLIRVIRRNALGEVRTSPKSGLPISLNLR

SEQ ID NO:356
MRYRVGLDLGTASVGAAVFSMDEQGNPMELIWHYERLFSEPLVPDMGQLKPKKAARRLARQQRRQIDRRA
SRLRRIAIVSRRLGIAPGRNDSGVHGNDVPTLRAMAVNERIELGQLRAVLLRMGKKRGYGGTFKAVRKVG
EAGEVASGASRLEEEMVALASVQNKDSVTVGEYLAARVEHGLPSKLKVAANNEYYAPEYALFRQYLGLPA
IKGRPDCLPNMYALRHQIEHEFERIWATQSQFHDVMKDHGVKEEIRNAIFFQRPLKSPADKVGRCSLQTN
LPRAPRAQIAAQNFRIEKQMADLRWGMGRRAEMLNDHQKAVIRELLNQQKELSFRKIYKELERAGCPGPE
GKGLNMDRAALGGRDDLSGNTTLAAWRKLGLEDRWQELDEVTQIQVINFLADLGSPEQLDTDDWSCRFMG
KNGRPRNFSDEFVAFMNELRMTDGFDRLSKMGFEGGRSSYSIKALKALTEWMIAPHWRETPETHRVDEEA
AIRECYPESLATPAQGGRQSKLEPPPLTGNEVVDVALRQVRHTINMMIDDLGSVPAQIVVEMAREMKGGV
TRRNDIEKQNKRFASERKKAAQSIEENGKTPTPARILRYQLWIEQGHQCPYCESNISLEQALSGAYTNFE
HILPRTLTQIGRKRSELVLAHRECNDEKGNRTPYQAFGHDDRRWRIVEQRANALPKKSSRKTRLLLLKDF

EGEALTDESIDEFADRQLHESSWLAKVTTQWLSSLGSDVYVSRGSLTAELRRRWGLDTVIPQVRFESGMP
VVDEEGAEITPEEFEKFRLQWEGHRVTREMRTDRRPDKRIDHRHHLVDAIVTALTSRSLYQQYAKAWKVA
DEKQRHGRVDVKVELPMPILTIRDIALEAVRSVRISHKPDRYPDGRFFEATAYGIAQRLDERSGEKVDWL
VSRKSLTDLAPEKKSIDVDKVRANISRIVGEAIRLHISNIFEKRVSKGMTPQQALREPIEFQGNILRKVR
CFYSKADDCVRIEHSSRRGHHYKMLLNDGFAYMEVPCKEGILYGVPNLVRPSEAVGIKRAPESGDFIRFY
KGDTVKNIKTGRVYTIKQILGDGGGKLILTPVTETKPADLLSAKWGRLKVGGRNIHLLRLCAE

SEQ ID NO:357
MIGEHVRGGCLFDDHWTPNWGAFRLPNTVRTFTKAENPKDGSSLAEPRRQARGLRRRLRRKTQRLEDLRR
LLAKEGVLSLSDLETLFRETPAKDPYQLRAEGLDRPLSFPEWVRVLYHITKHRGFQSNRRNPVEDGQERS
RQEEEGKLLSGVGENERLLREGGYRTAGEMLARDPKFQDHRRNRAGDYSHTLSRSLLLEEARRLFQSQRT
LGNPHASSNLEEAFLHLVAFQNPFASGEDIRNKAGHCSLEPDQIRAPRRSASAETFMLLQKTGNLRLIHR
RTGEERPLTDKEREQIHLLAWKQEKVTHKTLRRHLEIPEEWLFTGLPYHRSGDKAEEKLFVHLAGIHEIR
KALDKGPDPAVWDTLRSRRDLLDSIADTLTFYKNEDEILPRLESLGLSPENARALAPLSFSGTAHLSLSA
LGKLLPHLEEGKSYTQARADAGYAAPPPDRHPKLPPLEEADWRNPVVFRALTQTRKVVNALVRRYGPPWC
IHLETARELSQPAKVRRRIETEQQANEKKKQQAEREFLDIVGTAPGPGDLLKMRLWREQGGFCPYCEEYL
NPTRLAEPGYAEMDHILPYSRSLDNGWHNRVLVHGKDNRDKGNRTPFEAFGGDTARWDRLVAWVQASHLS
APKKRNLLREDFGEEAERELKDRNLTDTRFITKTAATLLRDRLTFHPEAPKDPVMTLNGRLTAFLRKQWG
LHKNRKNGDLHHALDAAVLAVASRSFVYRLSSHNAAWGELPRGREAENGFSLPYPAFRSEVLARLCPTRE
EILLRLDQGGVGYDEAFRNGLRPVFVSRAPSRRLRGKAHMETLRSPKWKDHPEGPRTASRIPLKDLNLEK
LERMVGKDRDRKLYEALRERLAAFGGNGKKAFVAPFRKPCRSGEGPLVRSLRIFDSGYSGVELRDGGEVY
AVADHESMVRVDVYAKKNRFYLVPVYVADVARGIVKNRAIVAHKSEEEWDLVDGSFDFRFSLFPGDLVEI
EKKDGAYLGYYKSCHRGDGRLLLDRHDRMPRESDCGTFYVSTRKDVLSMSKYQVDPLGEIRLVGSEKPPF
VL

SEQ ID NO:358
MEKKRKVTLGFDLGIASVGWAIVDSETNQVYKLGSRLFDAPDTNLERRTQRGTRRLLRRRKYRNQKFYNL
VKRTEVFGLSSREAIENRFRELSIKYPNIIELKTKALSQEVCPDEIAWILHDYLKNRGYFYDEKETKEDF
DQQTVESMPSYKLNEFYKKYGYFKGALSQPTESEMKDNKDLKEAFFFDFSNKEWLKEINYFFNVQKNILS
ETFIEEFKKIFSFTRDISKGPGSDNMPSPYGIFGEFGDNGQGGRYEHIWDKNIGKCSIFTNEQRAPKYLP
SALIFNFLNELANIRLYSTDKKNIQPLWKLSSVDKLNILLNLFNLPISEKKKKLTSTNINDIVKKESIKS
IMISVEDIDMIKDEWAGKEPNVYGVGLSGLNIEESAKENKFKFQDLKILNVLINLLDNVGIKFEFKDRND
IIKNLELLDNLYLFLIYQKESNNKDSSIDLFIAKNESLNIENLKLKLKEFLLGAGNEFENHNSKTHSLSK
KAIDEILPKLLDNNEGWNLEAIKNYDEEIKSQIEDNSSLMAKQDKKYLNDNFLKDAILPPNVKVTFQQAI
LIFNKIIQKFSKDFEIDKVVIELAREMTQDQENDALKGIAKAQKSKKSLVEERLEANNIDKSVFNDKYEK
LIYKIFLWISQDFKDPYTGAQISVNEIVNNKVEIDHIIPYSLCFDDSSANKVLVHKQSNQEKSNSLPYEY
IKQGHSGWNWDEFTKYVKRVFVNNVDSILSKKERLKKSENLLTASYDGYDKLGFLARNLNDTRYATILFR
DQLNNYAEHHLIDNKKMFKVIAMNGAVTSFIRKNMSYDNKLRLKDRSDFSHHAYDAAIIALFSNKTKTLY
NLIDPSLNGIISKRSEGYWVIEDRYTGEIKELKKEDWTSIKNNVQARKIAKEIEEYLIDLDDEVFFSRKT
KRKTNRQLYNETIYGIATKTDEDGITNYYKKEKFSILDDKDIYLRLLREREKFVINQSNPEVIDQIIEII
ESYGKENNIPSRDEAINIKYTKNKINYNLYLKQYMRSLTKSLDQFSEEFINQMIANKTFVLYNPTKNTTR
KIKFLRLVNDVKINDIRKNQVINKFNGKNNEPKAFYENINSLGAIVFKNSANNFKTLSINTQIAIFGDKN
WDIEDFKTYNMEKIEKYKEIYGIDKTYNFHSFIFPGTILLDKQNKEFYYISSIQTVRDIIEIKFLNKIEF
KDENKNQDTSKTPKRLMFGIKSIMNNYEQVDISPFGINKKIFE

SEQ ID NO:359
MGYRIGLDVGITSTGYAVLKTDKNGLPYKILTLDSVIYPRAENPQTGASLAEPRRIKRGLRRRTRRTKFR
KQRTQQLFIHSGLLSKPEIEQILATPQAKYSVYELRVAGLDRRLTNSELFRVLYFFIGHRGFKSNRKAEL
NPENEADKKQMGQLLNSIEEIRKAIAEKGYRTVGELYLKDPKYNDHKRNKGYIDGYLSTPNRQMLVDEIK
QILDKQRELGNEKLTDEFYATYLLGDENRAGIFQAQRDFDEGPGAGPYAGDQIKKMVGKDIFEPTEDRAA
KATYTFQYFNLLQKMTSLNYQNTTGDTWHTLNGLDRQAIIDAVFAKAEKPTKTYKPTDFGELRKLLKLPD
DARFNLVNYGSLQTQKEIETVEKKTRFVDFKAYHDLVKVLPEEMWQSRQLLDHIGTALTLYSSDKRRRRY

FAEELNLPAELIEKLLPLNFSKFGHLSIKSMQNIIPYLEMGQVYSEATTNTGYDFRKKQISKDTIREEIT
NPVVRRAVTKTIKIVEQIIRRYGKPDGINIELARELGRNFKERGDIQKRQDKNRQTNDKIAAELTELGIP
VNGQNIIRYKLHKEQNGVDPYTGDQIPFERAFSEGYEVDHIIPYSISWDDSYTNKVLTSAKCNREKGNRI
PMVYLANNEQRLNALTNIADNIIRNSRKRQKLLKQKLSDEELKDWKQRNINDTRFITRVLYNYFRQAIEF
NPELEKKQRVLPLNGEVTSKIRSRWGFLKVREDGDLHHAIDATVIAAITPKFIQQVTKYSQHQEVKNNQA
LWHDAEIKDAEYAAEAQRMDADLFNKIFNGFPLPWPEFLDELLARISDNPVEMMKSRSWNTYTPIEIAKL
KPVFVVRLANHKISGPAHLDTIRSAKLFDEKGIVLSRVSITKLKINKKGQVATGDGIYDPENSNNGDKVV
YSAIRQALEAHNGSGELAFPDGYLEYVDHGTKKLVRKVRVAKKVSLPVRLKNKAAADNGSMVRIDVFNTG
KKFVFVPIYIKDTVEQVLPNKAIARGKSLWYQITESDQFCFSLYPGDMVHIESKTGIKPKYSNKENNTSV
VPIKNFYGYFDGADIATASILVRAHDSSYTARSIGIAGLLKFEKYQVDYFGRYHKVHEKKRQLFVKRDE

SEQ ID NO:360
MQKNINTKQNHIYIKQAQKIKEKLGDKPYRIGLDLGVGSIGFAIVSMEENDGNVLLPKEIIMVGSRIFKA
SAGAADRKLSRGQRNNHRHTRERMRYLWKVLAEQKLALPVPADLDRKENSSEGETSAKRFLGDVLQKDIY
ELRVKSLDERLSLQELGYVLYHIAGHRGSSAIRTFENDSEEAQKENTENKKIAGNIKRLMAKKNYRTYGE
YLYKEFFENKEKHKREKISNAANNHKFSPTRDLVIKEAEAILKKQAGKDGFHKELTEEYIEKLTKAIGYE
SEKLIPESGFCPYLKDEKRLPASHKLNEERRLWETLNNARYSDPIVDIVTGEITGYYEKQFTKEQKQKLF
DYLLTGSELTPAQTKKLLGLKNTNFEDIILQGRDKKAQKIKGYKLIKLESMPFWARLSEAQQDSFLYDWN
SCPDEKLLTEKLSNEYHLTEEEIDNAFNEIVLSSSYAPLGKSAMLIILEKIKNDLSYTEAVEEALKEGKL
TKEKQAIKDRLPYYGAVLQESTQKIIAKGFSPQFKDKGYKTPHTNKYELEYGRIANPVVHQTLNELRKLV
NEIIDILGKKPCEIGLETARELKKSAEDRSKLSREQNDNESNRNRIYEIYIRPQQQVIITRRENPRNYIL
KFELLEEQKSQCPFCGGQISPNDIINNQADIEHLFPIAESEDNGRNNLVISHSACNADKAKRSPWAAFAS
AAKDSKYDYNRILSNVKENIPHKAWRFNQGAFEKFIENKPMAARFKTDNSYISKVAHKYLACLFEKPNII
CVKGSLTAQLRMAWGLQGLMIPFAKQLITEKESESFNKDVNSNKKIRLDNRHHALDAIVIAYASRGYGNL
LNKMAGKDYKINYSERNWLSKILLPPNNIVWENIDADLESFESSVKTALKNAFISVKHDHSDNGELVKGT
MYKIFYSERGYTLTTYKKLSALKLTDPQKKKTPKDFLETALLKFKGRESEMKNEKIKSAIENNKRLFDVI
QDNLEKAKKLLEEENEKSKAEGKKEKNINDASIYQKAISLSGDKYVQLSKKEPGKFFAISKPTPTTTGYG
YDTGDSLCVDLYYDNKGKLCGEIIRKIDAQQKNPLKYKEQGFTLFERIYGGDILEVDFDIHSDKNSFRNN
TGSAPENRVFIKVGTFTEITNNNIQIWFGNIIKSTGGQDDSFTINSMQQYNPRKLILSSCGFIKYRSPIL
KNKEG

SEQ ID NO:361
MAAFKPNPINYILGLDIGIASVGWAMVEIDEDENPICLIDLGVRVFERAEVPKTGDSLAMARRLARSVRR
LTRRRAHRLLRARRLLKREGVLQAADFDENGLIKSLPNTPWQLRAAALDRKLTPLEWSAVLLHLIKHRGY
LSQRKNEGETADKELGALLKGVADNAHALQTGDFRTPAELALNKFEKESGHIRNQRGDYSHTFSRKDLQA
ELILLFEKQKEFGNPHVSGGLKEGIETLLMTQRPALSGDAVQKMLGHCTFEPAEPKAAKNTYTAERFIWL
TKLNNLRILEQGSERPLTDTERATLMDEPYRKSKLTYAQARKLLGLEDTAFFKGLRYGKDNAEASTLMEM
KAYHAISRALEKEGLKDKKSPLNLSPELQDEIGTAFSLFKTDEDITGRLKDRIQPEILEALLKHISFDKF
VQISLKALRRIVPLMEQGKRYDEACAEIYGDHYGKKNTEEKIYLPPIPADEIRNPVVLRALSQARKVING
VVRRYGSPARIHIETAREVGKSFKDRKEIEKRQEENRKDREKAAAKFREYFPNFVGEPKSKDILKLRLYE
QQHGKCLYSGKEINLGRLNEKGYVEIDHALPFSRTWDDSFNNKVLVLGSENQNKGNQTPYEYFNGKDNSR
EWQEFKARVETSRFPRSKKQRILLQKFDEDGFKERNLNDTRYVNRFLCQFVADRMRLTGKGKKRVFASNG
QITNLLRGFWGLRKVRAENDRHHALDAVVVACSTVAMQQKITRFVRYKEMNAFDGKTIDKETGEVLHQKT
HFPQPWEFFAQEVMIRVFGKPDGKPEFEEADTPEKLRTLLAEKLSSRPEAVHEYVTPLFVSRAPNRKMSG
QGHMETVKSAKRLDEGVSVLRVPLTQLKLKDLEKMVNREREPKLYEALKARLEAHKDDPAKAFAEPFYKY
DKAGNRTQQVKAVRVEQVQKTGVWVRNHNGIADNATMVRVDVFEKGDKYYLVPIYSWQVAKGILPDRAVV
QGKDEEDWQLIDDSFNFKFSLHPNDLVEVITKKARMFGYFASCHRGTGNINIRIHDLDHKIGKNGILEGI
GVKTALSFQKYQIDELGKEIRPCRLKKRPPVR

SEQ ID NO:362
MQTTNLSYILGLDLGIASVGWAVVEINENEDPIGLIDVGVRIFERAEVPKTGESLALSRRLARSTRRLIR
RRAHRLLLAKRFLKREGILSTIDLEKGLPNQAWELRVAGLERRLSAIEWGAVLLHLIKHRGYLSKRKNES

QTNNKELGALLSGVAQNHQLLQSDDYRTPAELALKKFAKEEGHIRNQRGAYTHTFNRLDLLAELNLLFAQ
QHQFGNPHCKEHIQQYMTELLMWQKPALSGEAILKMLGKCTHEKNEFKAAKHTYSAERFVWLTKLNNLRI
LEDGAERALNEEERQLLINHPYEKSKLTYAQVRKLLGLSEQAIFKHLRYSKENAESATFMELKAWHAIRK
ALENQGLKDTWQDLAKKPDLLDEIGTAFSLYKTDEDIQQYLTNKVPNSVINALLVSLNFDKFIELSLKSL
RKILPLMEQGKRYDQACREIYGHHYGEANQKTSQLLPAIPAQEIRNPVVLRTLSQARKVINAIIRQYGSP
ARVHIETGRELGKSFKERREIQKQQEDNRTKRESAVQKFKELFSDFSSEPKSKDILKFRLYEQQHGKCLY
SGKEINIHRLNEKGYVEIDHALPFSRTWDDSFNNKVLVLASENQNKGNQTPYEWLQGKINSERWKNFVAL
VLGSQCSAAKKQRLLTQVIDDNKFIDRNLNDTRYIARFLSNYIQENLLLVGKNKKNVFTPNGQITALLRS
RWGLIKARENNNRHHALDAIVVACATPSMQQKITRFIRFKEVHPYKIENRYEMVDQESGEIISPHFPEPW
AYFRQEVNIRVFDNHPDTVLKEMLPDRPQANHQFVQPLFVSRAPTRKMSGQGHMETIKSAKRLAEGISVL
RIPLTQLKPNLLENMVNKEREPALYAGLKARLAEFNQDPAKAFATPFYKQGGQQVKAIRVEQVQKSGVLV
RENNGVADNASIVRTDVFIKNNKFFLVPIYTWQVAKGILPNKAIVAHKNEDEWEEMDEGAKFKFSLFPND
LVELKTKKEYFFGYYIGLDRATGNISLKEHDGEISKGKDGVYRVGVKLALSFEKYQVDELGKNRQICRPQ
QRQPVR

SEQ ID NO:363
MGIRFAFDLGTNSIGWAVWRTGPGVFGEDTAASLDGSGVLIFKDGRNPKDGQSLATMRRVPRQSRKRRDR
FVLRRRDLLAALRKAGLFPVDVEEGRRLAATDPYHLRAKALDESLTPHEMGRVIFHLNQRRGFRSNRKAD
RQDREKGKIAEGSKRLAETLAATNCRTLGEFLWSRHRGTPRTRSPTRIRMEGEGAKALYAFYPTREMVRA
EFERLWTAQSRFAPDLLTPERHEEIAGILFRQRDLAPPKIGCCTFEPSERRLPRALPSVEARGIYERLAH
LRITTGPVSDRGLTRPERDVLASALLAGKSLTFKAVRKTLKILPHALVNFEEAGEKGLDGALTAKLLSKP
DHYGAAWHGLSFAEKDTFVGKLLDEADEERLIRRLVTENRLSEDAARRCASIPLADGYGRLGRTANTEIL
AALVEETDETGTVVTYAEAVRRAGERTGRNWHHSDERDGVILDRLPYYGEILQRHVVPGSGEPEEKNEAA
RWGRLANPTVHIGLNQLRKVVNRLIAAHGRPDQIVVELARELKLNREQKERLDRENRKNREENERRTAIL
AEHGQRDTAENKIRLRLFEEQARANAGIALCPYTGRAIGIAELFTSEVEIDHILPVSLTLDDSLANRVLC
RREANREKRRQTPFQAFGATPAWNDIVARAAKLPPNKRWRFDPAALERFEREGGFLGRQLNETKYLSRLA
KIYLGKICDPDRVYVTPGTLTGLLRARWGLNSILSDSNFKNRSDHRHHAVDAVVIGVLTRGMIQRIAHDA
ARAEDQDLDRVFRDVPVPFEDFRDHVRERVSTITVAVKPEHGKGGALHEDTSYGLVPDTDPNAALGNLVV
RKPIRSLTAGEVDRVRDRALRARLGALAAPFRDESGRVRDAKGLAQALEAFGAENGIRRVRILKPDASVV
TIADRRTGVPYRAVAPGENHHVDIVQMRDGSWRGFAASVFEVNRPGWRPEWEVKKLGGKLVMRLHKGDMV
ELSDKDGQRRVKVVQQIEISANRVRLSPHNDGGKLQDRHADADDPFRWDLATIPLLKDRGCVAVRVDPIG
VVTLRRSNV

SEQ ID NO:364
MMEVFMGRLVLGLDIGITSVGFGIIDLDESEIVDYGVRLFKEGTAAENETRRTKRGGRRLKRRRVTRRED
MLHLLKQAGIISTSFHPLNNPYDVRVKGLNERLNGEELATALLHLCKHRGSSVETIEDDEAKAKEAGETK
KVLSMNDQLLKSGKYVCEIQKERLRTNGHIRGHENNFKTRAYVDEAFQILSHQDLSNELKSAIITIISRK
RMYYDGPGGPLSPTPYGRYTYFGQKEPIDLIEKMRGKCSLFPNEPRAPKLAYSAELFNLLNDLNNLSIEG
EKLTSEQKAMILKIVHEKGKITPKQLAKEVGVSLEQIRGFRIDTKGSPLLSELTGYKMIREVLEKSNDEH
LEDHVFYDEIAEILTKTKDIEGRKKQISELSSDLNEESVHQLAGLTKFTAYHSLSFKALRLINEEMLKTE
LNQMQSITLFGLKQNNELSVKGMKNIQADDTAILSPVAKRAQRETFKVVNRLREIYGEFDSIVVEMAREK
NSEEQRKAIRERQKFFEMRNKQVADIIGDDRKINAKLREKLVLYQEQDGKTAYSLEPIDLKLLIDDPNAY
EVDHIIPISISLDDSITNKVLVTHRENQEKGNLTPISAFVKGRFTKGSLAQYKAYCLKLKEKNIKTNKGY
RKKVEQYLLNENDIYKYDIQKEFINRNLVDTSYASRVVLNTLTTYFKQNEIPTKVFTVKGSLTNAFRRKI
NLKKDRDEDYGHHAIDALIIASMPKMRLLSTIFSRYKIEDIYDESTGEVFSSGDDSMYYDDRYFAFIASL
KAIKVRKFSHKIDTKPNRSVADETIYSTRVIDGKEKVVKKYKDIYDPKFTALAEDILNNAYQEKYLMALH
DPQTFDQIVKVVNYYFEEMSKSEKYFTKDKKGRIKISGMNPLSLYRDEHGMLKKYSKKGDGPAITQMKYF
DGVLGNHIDISAHYQVRDKKVVLQQISPYRTDFYYSKENGYKFVTIRYKDVRWSEKKKKYVIDQQDYAMK
KAEKKIDDTYEFQFSMHRDELIGITKAEGEALIYPDETWHNFNFFFHAGETPEILKFTATNNDKSNKIEV
KPIHCYCKMRLMPTISKKIVRIDKYATDVVGNLYKVKKNTLKFEFD

SEQ ID NO:365

MKKILGVDLGITSFGYAILQETGKDLYRCLDNSVVMRNNPYDEKSGESSQSIRSTQKSMRRLIEKRKKRI
RCVAQTMERYGILDYSETMKINDPKNNPIKNRWQLRAVDAWKRPLSPQELFAIFAHMAKHRGYKSIATED
LIYELELELGLNDPEKESEKKADERRQVYNALRHLEELRKKYGGETIAQTIHRAVEAGDLRSYRNHDDYE
KMIRREDIEEEIEKVLLRQAELGALGLPEEQVSELIDELKACITDQEMPTIDESLFGKCTFYKDELAAPA
YSYLYDLYRLYKKLADLNIDGYEVTQEDREKVIEWVEKKIAQGKNLKKITHKDLRKILGLAPEQKIFGVE
DERIVKGKKEPRTFVPFFFLADIAKFKELFASIQKHPDALQIFRELAEILQRSKTPQEALDRLRALMAGK
GIDTDDRELLELFKNKRSGTRELSHRYILEALPLFLEGYDEKEVQRILGFDDREDYSRYPKSLRHLHLRE
GNLFEKEENPINNHAVKSLASWALGLIADLSWRYGPFDEIILETTRDALPEKIRKEIDKAMREREKALDK
IIGKYKKEFPSIDKRLARKIQLWERQKGLDLYSGKVINLSQLLDGSADIEHIVPQSLGGLSTDYNTIVTL
KSVNAAKGNRLPGDWLAGNPDYRERIGMLSEKGLIDWKKRKNLLAQSLDEIYTENTHSKGIRATSYLEAL
VAQVLKRYYPFPDPELRKNGIGVRMIPGKVTSKTRSLLGIKSKSRETNFHHAEDALILSTLTRGWQNRLH
RMLRDNYGKSEAELKELWKKYMPHIEGLTLADYIDEAFRRFMSKGEESLFYRDMFDTIRSISYWVDKKPL
SASSHKETVYSSRHEVPTLRKNILEAFDSLNVIKDRHKLTTEEFMKRYDKEIRQKLWLHRIGNTNDESYR
AVEERATQIAQILTRYQLMDAQNDKEIDEKFQQALKELITSPIEVTGKLLRKMRFVYDKLNAMQIDRGLV
ETDKNMLGIHISKGPNEKLIFRRMDVNNAHELQKERSGILCYLNEMLFIFNKKGLIHYGCLRSYLEKGQG
SKYIALFNPRFPANPKAQPSKFTSDSKIKQVGIGSATGIIKAHLDLDGHVRSYEVFGTLPEGSIEWFKEE
SGYGRVEDDPHH

SEQ ID NO:366
MRPIEPWILGLDIGTDSLGWAVFSCEEKGPPTAKELLGGGVRLFDSGRDAKDHTSRQAERGAFRRARRQT
RTWPWRRDRLIALFQAAGLTPPAAETRQIALALRREAVSRPLAPDALWAALLHLAHHRGFRSNRIDKRER
AAAKALAKAKPAKATAKATAPAKEADDEAGFWEGAEAALRQRMAASGAPTVGALLADDLDRGQPVRMRYN
QSDRDGVVAPTRALIAEELAEIVARQSSAYPGLDWPAVTRLVLDQRPLRSKGAGPCAFLPGEDRALRALP
TVQDFIIRQTLANLRLPSTSADEPRPLTDEEHAKALALLSTARFVEWPALRRALGLKRGVKFTAETERNG
AKQAARGTAGNLTEAILAPLIPGWSGWDLDRKDRVFSDLWAARQDRSALLALIGDPRGPTRVTEDETAEA
VADAIQIVLPTGRASLSAKAARAIAQAMAPGIGYDEAVTLALGLHHSHRPRQERLARLPYYAAALPDVGL
DGDPVGPPPAEDDGAAAEAYYGRIGNISVHIALNETRKIVNALLHRHGPILRLVMVETTRELKAGADERK
RMIAEQAERERENAEIDVELRKSDRWMANARERRQRVRLARRQNNLCPYTSTPIGHADLLGDAYDIDHVI
PLARGGRDSLDNMVLCQSDANKTKGDKTPWEAFHDKPGWIAQRDDFLARLDPQTAKALAWRFADDAGERV
ARKSAEDEDQGFLPRQLTDTGYIARVALRYLSLVTNEPNAVVATNGRLTGLLRLAWDITPGPAPRDLLPT
PRDALRDDTAARRFLDGLTPPPLAKAVEGAVQARLAALGRSRVADAGLADALGLTLASLGGGGKNRADHR
HHFIDAAMIAVTTRGLINQINQASGAGRILDLRKWPRTNFEPPYPTFRAEVMKQWDHIHPSIRPAHRDGG
SLHAATVFGVRNRPDARVLVQRKPVEKLFLDANAKPLPADKIAEIIDGFASPRMAKRFKALLARYQAAHP
EVPPALAALAVARDPAFGPRGMTANTVIAGRSDGDGEDAGLITPFRANPKAAVRTMGNAVYEVWEIQVKG
RPRWTHRVLTRFDRTQPAPPPPPENARLVMRLRRGDLVYWPLESGDRLFLVKKMAVDGRLALWPARLATG
KATALYAQLSCPNINLNGDQGYCVQSAEGIRKEKIRTTSCTALGRLRLSKKAT

SEQ ID NO:367
MKYTLGLDVGIASVGWAVIDKDNNKIIDLGVRCFDKAEESKTGESLATARRIARGMRRRISRRSQRLRLV
KKLFVQYEIIKDSSEFNRIFDTSRDGWKDPWELRYNALSRILKPYELVQVLTHITKRRGFKSNRKEDLST
TKEGVVITSIKNNSEMLRTKNYRTIGEMIFMETPENSNKRNKVDEYIHTIAREDLLNEIKYIFSIQRKLG
SPFVTEKLEHDFLNIWEFQRPFASGDSILSKVGKCTLLKEELRAPTSCYTSEYFGLLQSINNLVLVEDNN
TLTLNNDQRAKIIEYAHFKNEIKYSEIRKLLDIEPEILFKAHNLTHKNPSGNNESKKFYEMKSYHKLKST
LPTDIWGKLHSNKESLDNLFYCLTVYKNDNEIKDYLQANNLDYLIEYIAKLPTFNKFKHLSLVAMKRIIP
FMEKGYKYSDACNMAELDFTGSSKLEKCNKLTVEPIIENVTNPVVIRALTQARKVINAIIQKYGLPYMVN
IELAREAGMTRQDRDNLKKEHENNRKAREKISDLIRQNGRVASGLDILKWRLWEDQGGRCAYSGKPIPVC
DLLNDSLTQIDHIYPYSRSMDDSYMNKVLVLTDENQNKRSYTPYEVWGSTEKWEDFEARIYSMHLPQSKE
KRLLNRNFITKDLDSFISRNLNDTRYISRFLKNYIESYLQFSNDSPKSCVVCVNGQCTAQLRSRWGLNKN
REESDLHHALDAAVIACADRKIIKEITNYYNERENHNYKVKYPLPWHSFRQDLMETLAGVFISRAPRRKI
TGPAHDETIRSPKHFNKGLTSVKIPLTTVTLEKLETMVKNTKGGISDKAVYNVLKNRLIEHNNKPLKAFA
EKIYKPLKNGTNGAIIRSIRVETPSYTGVFRNEGKGISDNSLMVRVDVFKKKDKYYLVPIYVAHMIKKEL

PSKAIVPLKPESQWELIDSTHEFLFSLYQNDYLVIKTKKGITEGYYRSCHRGTGSLSLMPHFANNKNVKI
DIGVRTAISIEKYNVDILGNKSIVKGEPRRGMEKYNSFKSN

SEQ ID NO:368
MIRTLGIDIGIASIGWAVIEGEYTDKGLENKEIVASGVRVFTKAENPKNKESLALPRTLARSARRRNARK
KGRIQQVKHYLSKALGLDLECFVQGEKLATLFQTSKDFLSPWELRERALYRVLDKEELARVILHIAKRRG
YDDITYGVEDNDSGKIKKAIAENSKRIKEEQCKTIGEMMYKLYFQKSLNVRNKKESYNRCVGRSELREEL
KTIFQIQQELKSPWVNEELIYKLLGNPDAQSKQEREGLIFYQRPLKGFGDKIGKCSHIKKGENSPYRACK
HAPSAEEFVALTKSINFLKNLTNRHGLCFSQEDMCVYLGKILQEAQKNEKGLTYSKLKLLLDLPSDFEFL
GLDYSGKNPEKAVFLSLPSTFKLNKITQDRKTQDKIANILGANKDWEAILKELESLQLSKEQIQTIKDAK
LNFSKHINLSLEALYHLLPLMREGKRYDEGVEILQERGIFSKPQPKNRQLLPPLSELAKEESYFDIPNPV
LRRALSEFRKVVNALLEKYGGFHYFHIELTRDVCKAKSARMQLEKINKKNKSENDAASQLLEVLGLPNTY
NNRLKCKLWKQQEEYCLYSGEKITIDHLKDQRALQIDHAFPLSRSLDDSQSNKVLCLTSSNQEKSNKTPY
EWLGSDEKKWDMYVGRVYSSNFSPSKKRKLTQKNFKERNEEDFLARNLVDTGYIGRVTKEYIKHSLSFLP
LPDGKKEHIRIISGSMTSTMRSFWGVQEKNRDHHLHHAQDAIIIACIEPSMIQKYTTYLKDKETHRLKSH
QKAQILREGDHKLSLRWPMSNFKDKIQESIQNIIPSHHVSHKVTGELHQETVRTKEFYYQAFGGEEGVKK
ALKFGKIREINQGIVDNGAMVRVDIFKSKDKGKFYAVPIYTYDFAIGKLPNKAIVQGKKNGIIKDWLEMD
ENYEFCFSLFKNDCIKIQTKEMQEAVLAIYKSTNSAKATIELEHLSKYALKNEDEEKMFTDTDKEKNKTM
TRESCGIQGLKVFQKVKLSVLGEVLEHKPRNRQNIALKTTPKHV

SEQ ID NO:369
MKYSIGLDIGIASVGWSVINKDKERIEDMGVRIFQKAENPKDGSSLASSRREKRGSRRRNRRKKHRLDRI
KNILCESGLVKKNEIEKIYKNAYLKSPWELRAKSLEAKISNKEIAQILLHIAKRRGFKSFRKTDRNADDT
GKLLSGIQENKKIMEEKGYLTIGDMVAKDPKFNTHVRNKAGSYLFSFSRKLLEDEVRKIQAKQKELGNTH
FTDDVLEKYIEVFNSQRNFDEGPSKPSPYYSEIGQIAKMIGNCTFESSEKRTAKNTWSGERFVFLQKLNN
FRIVGLSGKRPLTEEERDIVEKEVYLKKEVRYEKLRKILYLKEEERFGDLNYSKDEKQDKKTEKTKFISL
IGNYTIKKLNLSEKLKSEIEEDKSKLDKIIEILTFNKSDKTIESNLKKLELSREDIEILLSEEFSGTLNL
SLKAIKKILPYLEKGLSYNEACEKADYDYKNNGIKFKRGELLPVVDKDLIANPVVLRAISQTRKVVNAII
RKYGTPHTIHVEVARDLAKSYDDRQTIIKENKKRELENEKTKKFISEEFGIKNVKGKLLLKYRLYQEQEG
RCAYSRKELSLSEVILDESMTDIDHIIPYSRSMDDSYSNKVLVLSGENRKKSNLLPKEYFDRQGRDWDTF
VLNVKAMKIHPRKKSNLLKEKFTREDNKDWKSRALNDTRYISRFVANYLENALEYRDDSPKKRVFMIPGQ
LTAQLRARWRLNKVRENGDLHHALDAAVVAVTDQKAINNISNISRYKELKNCKDVIPSIEYHADEETGEV
YFEEVKDTRFPMPWSGFDLELQKRLESENPREEFYNLLSDKRYLGWFNYEEGFIEKLRPVFVSRMPNRGV
KGQAHQETIRSSKKISNQIAVSKKPLNSIKLKDLEKMQGRDTDRKLYEALKNRLEEYDDKPEKAFAEPFY
KPTNSGKRGPLVRGIKVEEKQNVGVYVNGGQASNGSMVRIDVFRKNGKFYTVPIYVHQTLLKELPNRAIN
GKPYKDWDLIDGSFEFLYSFYPNDLIEIEFGKSKSIKNDNKLTKTEIPEVNLSEVLGYYRGMDTSTGAAT
IDTQDGKIQMRIGIKTVKNIKKYQVDVLGNVYKVKREKRQTF

SEQ ID NO:370
MSKKVSRRYEEQAQEICQRLGSRPYSIGLDLGVGSIGVAVAAYDPIKKQPSDLVFVSSRIFIPSTGAAER
RQKRGQRNSLRHRANRLKFLWKLLAERNLMLSYSEQDVPDPARLRFEDAVVRANPYELRLKGLNEQLTLS
ELGYALYHIANHRGSSSVRTFLDEEKSSDDKKLEEQQAMTEQLAKEKGISTFIEVLTAFNTNGLIGYRNS
ESVKSKGVPVPTRDIISNEIDVLLQTQKQFYQEILSDEYCDRIVSAILFENEKIVPEAGCCPYFPDEKKL
PRCHFLNEERRLWEAINNARIKMPMQEGAAKRYQSASFSDEQRHILFHIARSGTDITPKLVQKEFPALKT
SIIVLQGKEKAIQKIAGFRFRRLEEKSFWKRLSEEQKDDFFSAWTNTPDDKRLSKYLMKHLLLTENEVVD
ALKTVSLIGDYGPIGKTATQLLMKHLEDGLTYTEALERGMETGEFQELSVWEQQSLLPYYGQILTGSTQA
LMGKYWHSAFKEKRDSEGFFKPNTNSDEEKYGRIANPVVHQTLNELRKLMNELITILGAKPQEITVELAR
ELKVGAEKREDIIKQQTKQEKEAVLAYSKYCEPNNLDKRYIERFRLLEDQAFVCPYCLEHISVADIAAGR
ADVDHIFPRDDTADNSYGNKVVAHRQCNDIKGKRTPYAAFSNTSAWGPIMHYLDETPGMWRKRRKFETNE
EEYAKYLQSKGFVSRFESDNSYIAKAAKEYLRCLFNPNNVTAVGSLKGMETSILRKAWNLQGIDDLLGSR
HWSKDADTSPTMRKNRDDNRHHGLDAIVALYCSRSLVQMINTMSEQGKRAVEIEAMIPIPGYASEPNLSF
EAQRELFRKKILEFMDLHAFVSMKTDNDANGALLKDTVYSILGADTQGEDLVFVVKKKIKDIGVKIGDYE

EVASAIRGRITDKQPKWYPMEMKDKIEQLQSKNEAALQKYKESLVQAAAVLEESNRKLIESGKKPIQLSE
KTISKKALELVGGYYYLISNNKRTKTFVVKEPSNEVKGFAFDTGSNLCLDFYHDAQGKLCGEIIRKIQAM
NPSYKPAYMKQGYSLYVRLYQGDVCELRASDLTEAESNLAKTTHVRLPNAKPGRTFVIIITFTEMGSGYQ
IYFSNLAKSKKGQDTSFTLTTIKNYDVRKVQLSSAGLVRYVSPLLVDKIEKDEVALCGE

SEQ ID NO:371
MNQKFILGLDIGITSVGYGLIDYETKNIIDAGVRLFPEANVENNEGRRSKRGSRRLKRRRIHRLERVKKL
LEDYNLLDQSQIPQSTNPYAIRVKGLSEALSKDELVIALLHIAKRRGIHKIDVIDSNDDVGNELSTKEQL
NKNSKLLKDKFVCQIQLERMNEGQVRGEKNRFKTADIIKEIIQLLNVQKNFHQLDENFINKYIELVEMRR
EYFEGPGKGSPYGWEGDPKAWYETLMGHCTYFPDELRSVKYAYSADLFNALNDLNNLVIQRDGLSKLEYH
EKYHIIENVFKQKKKPTLKQIANEINVNPEDIKGYRITKSGKPQFTEFKLYHDLKSVLFDQSILENEDVL
DQIAEILTIYQDKDSIKSKLTELDILLNEEDKENIAQLTGYTGTHRLSLKCIRLVLEEQWYSSRNQMEIF
THLNIKPKKINLTAANKIPKAMIDEFILSPVVKRTFGQAINLINKIIEKYGVPEDIIIELARENNSKDKQ
KFINEMQKKNENTRKRINEIIGKYGNQNAKRLVEKIRLHDEQEGKCLYSLESIPLEDLLNNPNHYEVDHI
IPRSVSFDNSYHNKVLVKQSENSKKSNLTPYQYFNSGKSKLSYNQFKQHILNLSKSQDRISKKKKEYLLE
ERDINKFEVQKEFINRNLVDTRYATRELTNYLKAYFSANNMNVKVKTINGSFTDYLRKVWKFKKERNHGY
KHHAEDALIIANADFLFKENKKLKAVNSVLEKPEIESKQLDIQVDSEDNYSEMFIIPKQVQDIKDFRNFK
YSHRVDKKPNRQLINDTLYSTRKKDNSTYIVQTIKDIYAKDNTTLKKQFDKSPEKFLMYQHDPRTFEKLE
VIMKQYANEKNPLAKYHEETGEYLTKYSKKNNGPIVKSLKYIGNKLGSHLDVTHQFKSSTKKLVKLSIKP
YRFDVYLTDKGYKFITISYLDVLKKDNYYYIPEQKYDKLKLGKAIDKNAKFIASFYKNDLIKLDGEIYKI
IGVNSDTRNMIELDLPDIRYKEYCELNNIKGEPRIKKTIGKKVNSIEKLTTDVLGNVFTNTQYTKPQLLF
KRGN

SEQ ID NO:372
MIMKLEKWRLGLDLGTNSIGWSVFSLDKDNSVQDLIDMGVRIFSDGRDPKTKEPLAVARRTARSQRKLIY
RRKLRRKQVFKFLQEQGLFPKTKEECMTLKSLNPYELRIKALDEKLEPYELGRALFNLAVRRGFKSNRKD
GSREEVSEKKSPDEIKTQADMQTHLEKAIKENGCRTITEFLYKNQGENGGIRFAPGRMTYYPTRKMYEEE
FNLIRSKQEKYYPQVDWDDIYKAIFYQRPLKPQQRGYCIYENDKERTFKAMPCSQKLRILQDIGNLAYYE
GGSKKRVELNDNQDKVLYELLNSKDKVTFDQMRKALCLADSNSFNLEENRDFLIGNPTAVKMRSKNRFGK
LWDEIPLEEQDLIIETIITADEDDAVYEVIKKYDLTQEQRDFIVKNTILQSGTSMLCKEVSEKLVKRLEE
IADLKYHEAVESLGYKFADQTVEKYDLLPYYGKVLPGSTMEIDLSAPETNPEKHYGKISNPTVHVALNQT
RVVVNALIKEYGKPSQIAIELSRDLKNNVEKKAEIARKQNQRAKENIAINDTISALYHTAFPGKSFYPNR
NDRMKYRLWSELGLGNKCIYCGKGISGAELFTKEIEIEHILPFSRTLLDAESNLTVAHSSCNAFKAERSP
FEAFGTNPSGYSWQEIIQRANQLKNTSKKNKFSPNAMDSFEKDSSFIARQLSDNQYIAKAALRYLKCLVE
NPSDVWTTNGSMTKLLRDKWEMDSILCRKFTEKEVALLGLKPEQIGNYKKNRFDHRHHAIDAVVIGLTDR
SMVQKLATKNSHKGNRIEIPEFPILRSDLIEKVKNIVVSFKPDHGAEGKLSKETLLGKIKLHGKETFVCR
ENIVSLSEKNLDDIVDEIKSKVKDYVAKHKGQKIEAVLSDFSKENGIKKVRCVNRVQTPIEITSGKISRY
LSPEDYFAAVIWEIPGEKKTFKAQYIRRNEVEKNSKGLNVVKPAVLENGKPHPAAKQVCLLHKDDYLEFS
DKGKMYFCRIAGYAATNNKLDIRPVYAVSYCADWINSTNETMLTGYWKPTPTQNWVSVNVLFDKQKARLV
TVSPIGRVFRK

SEQ ID NO:373
MSSKAIDSLEQLDLFKPQEYTLGLDLGIKSIGWAILSGERIANAGVYLFETAEELNSTGNKLISKAAERG
RKRRIRRMLDRKARRGRHIRYLLEREGLPTDELEEVVVHQSNRTLWDVRAEAVERKLTKQELAAVLFHLV
RHRGYFPNTKKLPPDDESDSADEEQGKINRATSRLREELKASDCKTIGQFLAQNRDRQRNREGDYSNLMA
RKLVFEEALQILAFQRKQGHELSKDFEKTYLDVLMGQRSGRSPKLGNCSLIPSELRAPSSAPSTEWFKFL
QNLGNLQISNAYREEWSIDAPRRAQIIDACSQRSTSSYWQIRRDFQIPDEYRFNLVNYERRDPDVDLQEY
LQQQERKTLANFRNWKQLEKIIGTGHPIQTLDEAARLITLIKDDEKLSDQLADLLPEASDKAITQLCELD
FTTAAKISLEAMYRILPHMNQGMGFFDACQQESLPEIGVPPAGDRVPPFDEMYNPVVNRVLSQSRKLINA
VIDEYGMPAKIRVELARDLGKGRELRERIKLDQLDKSKQNDQRAEDFRAEFQQAPRGDQSLRYRLWKEQN
CTCPYSGRMIPVNSVLSEDTQIDHILPISQSFDNSLSNKVLCFTEENAQKSNRTPFEYLDAADFQRLEAI
SGNWPEAKRNKLLHKSFGKVAEEWKSRALNDTRYLTSALADHLRHHLPDSKIQTVNGRITGYLRKQWGLE

KDRDKHTHHAVDAIVVACTTPAIVQQVTLYHQDIRRYKKLGEKRPTPWPETFRQDVLDVEEEIFITRQPK
KVSGGIQTKDTLRKHRSKPDRQRVALTKVKLADLERLVEKDASNRNLYEHLKQCLEESGDQPTKAFKAPF
YMPSGPEAKQRPILSKVTLLREKPEPPKQLTELSGGRRYDSMAQGRLDIYRYKPGGKRKDEYRVVLQRMI
DLMRGEENVHVFQKGVPYDQGPEIEQNYTFLFSLYFDDLVEFQRSADSEVIRGYYRTFNIANGQLKISTY
LEGRQDFDFFGANRLAHFAKVQVNLLGKVIK

SEQ ID NO:374
MRSLRYRLALDLGSTSLGWALFRLDACNRPTAVIKAGVRIFSDGRNPKDGSSLAVTRRAARAMRRRRDRL
LKRKTRMQAKLVEHGFFPADAGKRKALEQLNPYALRAKGLQEALLPGEFARALFHINQRRGFKSNRKTDK
KDNDSGVLKKAIGQLRQQMAEQGSRTVGEYLWTRLQQGQGVRARYREKPYTTEEGKKRIDKSYDLYIDRA
MIEQEFDALWAAQAAFNPTLFHEAARADLKDTLLHQRPLRPVKPGRCTLLPEEERAPLALPSTQRFRIHQ
EVNHLRLLDENLREVALTLAQRDAVVTALETKAKLSFEQIRKLLKLSGSVQFNLEDAKRTELKGNATSAA
LARKELFGAAWSGFDEALQDEIVWQLVTEEGEGALIAWLQTHTGVDEARAQAIVDVSLPEGYGNLSRKAL
ARIVPALRAAVITYDKAVQAAGFDHHSQLGFEYDASEVEDLVHPETGEIRSVFKQLPYYGKALQRHVAFG
SGKPEDPDEKRYGKIANPTVHIGLNQVRMVVNALIRRYGRPTEVVIELARDLKQSREQKVEAQRRQADNQ
RRNARIRRSIAEVLGIGEERVRGSDIQKWICWEELSFDAADRRCPYSGVQISAAMLLSDEVEVEHILPFS
KTLDDSLNNRTVAMRQANRIKRNRTPWDARAEFEAQGWSYEDILQRAERMPLRKRYRFAPDGYERWLGDD
KDFLARALNDTRYLSRVAAEYLRLVCPGTRVIPGQLTALLRGKFGLNDVLGLDGEKNRNDHRHHAVDACV
IGVTDQGLMQRFATASAQARGDGLTRLVDGMPMPWPTYRDHVERAVRHIWVSHRPDHGFEGAMMEETSYG
IRKDGSIKQRRKADGSAGREISNLIRIHEATQPLRHGVSADGQPLAYKGYVGGSNYCIEITVNDKGKWEG
EVISTFRAYGVVRAGGMGRLRNPHEGQNGRKLIMRLVIGDSVRLEVDGAERTMRIVKISGSNGQIFMAPI
HEANVDARNTDKQDAFTYTSKYAGSLQKAKTRRVTISPIGEVRDPGFKG

SEQ ID NO:375
MARPAFRAPRREHVNGWTPDPHRISKPFFILVSWHLLSRVVIDSSSGCFPGTSRDHTDKFAEWECAVQPY
RLSFDLGTNSIGWGLLNLDRQGKPREIRALGSRIFSDGRDPQDKASLAVARRLARQMRRRRDRYLTRRTR
LMGALVRFGLMPADPAARKRLEVAVDPYLARERATRERLEPFEIGRALFHLNQRRGYKPVRTATKPDEEA
GKVKEAVERLEAAIAAAGAPTLGAWFAWRKTRGETLRARLAGKGKEAAYPFYPARRMLEAEFDTLWAEQA
RHHPDLLTAEAREILRHRIFHQRPLKPPPVGRCTLYPDDGRAPRALPSAQRLRLFQELASLRVIHLDLSE
RPLTPAERDRIVAFVQGRPPKAGRKPGKVQKSVPFEKLRGLLELPPGTGFSLESDKRPELLGDETGARIA
PAFGPGWTALPLEEQDALVELLLTEAEPERAIAALTARWALDEATAAKLAGATLPDFHGRYGRRAVAELL
PVLERETRGDPDGRVRPIRLDEAVKLLRGGKDHSDFSREGALLDALPYYGAVLERHVAFGTGNPADPEEK
RVGRVANPTVHIALNQLRHLVNAILARHGRPEEIVIELARDLKRSAEDRRREDKRQADNQKRNEERKRLI
LSLGERPTPRNLLKLRLWEEQGPVENRRCPYSGETISMRMLLSEQVDIDHILPFSVSLDDSAANKVVCLR
EANRIKRNRSPWEAFGHDSERWAGILARAEALPKNKRWRFAPDALEKLEGEGGLRARHLNDTRHLSRLAV
EYLRCVCPKVRVSPGRLTALLRRRWGIDAILAEADGPPPEVPAETLDPSPAEKNRADHRHHALDAVVIGC
IDRSMVQRVQLAAASAEREAAAREDNIRRVLEGFKEEPWDGFRAELERRARTIVVSHRPEHGIGGALHKE
TAYGPVDPPEEGFNLVVRKPIDGLSKDEINSVRDPRLRRALIDRLAIRRRDANDPATALAKAAEDLAAQP
ASRGIRRVRVLKKESNPIRVEHGGNPSGPRSGGPFHKLLLAGEVHHVDVALRADGRRWVGHWVTLFEAHG
GRGADGAAAPPRLGDGERFLMRLHKGDCLKLEHKGRVRVMQVVKLEPSSNSVVVVEPHQVKTDRSKHVKI
SCDQLRARGARRVTVDPLGRVRVHAPGARVGIGGDAGRTAMEPAEDIS

SEQ ID NO:376
MKRTSLRAYRLGVDLGANSLGWFVVWLDDHGQPEGLGPGGVRIFPDGRNPQSKQSNAAGRRLARSARRRR
DRYLQRRGKLMGLLVKHGLMPADEPARKRLECLDPYGLRAKALDEVLPLHHVGRALFHLNQRRGLFANRA
IEQGDKDASAIKAAAGRLQTSMQACGARTLGEFLNRRHQLRATVRARSPVGGDVQARYEFYPTRAMVDAE
FEAIWAAQAPHHPTMTAEAHDTIREAIFSQRAMKRPSIGKCSLDPATSQDDVDGFRCAWSHPLAQRFRIW
QDVRNLAVVETGPTSSRLGKEDQDKVARALLQTDQLSFDEIRGLLGLPSDARFNLESDRRDHLKGDATGA
ILSARRHFGPAWHDRSLDRQIDIVALLESALDEAAIIASLGTTHSLDEAAAQRALSALLPDGYCRLGLRA
IKRVLPLMEAGRTYAEAASAAGYDHALLPGGKLSPTGYLPYYGQWLQNDVVGSDDERDTNERRWGRLPNP
TVHIGIGQLRRVVNELIRWHGPPAEITVELTRDLKLSPRRLAELEREQAENQRKNDKRTSLLRKLGLPAS
THNLLKLRLWDEQGDVASECPYTGEAIGLERLVSDDVDIDHLIPFSISWDDSAANKVVCMRYANREKGNR

TPFEAFGHRQGRPYDWADIAERAARLPRGKRWRFGPGARAQFEELGDFQARLLNETSWLARVAKQYLAAV
THPHRIHVLPGRLTALLRATWELNDLLPGSDDRAAKSRKDHRHHAIDALVAALTDQALLRRMANAHDDTR
RKIEVLLPWPTFRIDLETRLKAMLVSHKPDHGLQARLHEDTAYGTVEHPETEDGANLVYRKTFVDISEKE
IDRIRDRRLRDLVRAHVAGERQQGKTLKAAVLSFAQRRDIAGHPNGIRHVRLTKSIKPDYLVPIRDKAGR
IYKSYNAGENAFVDILQAESGRWIARATTVFQANQANESHDAPAAQPIMRVFKGDMLRIDHAGAEKFVKI
VRLSPSNNLLYLVEHHQAGVFQTRHDDPEDSFRWLFASFDKLREWNAELVRIDTLGQPWRRKRGLETGSE
DATRIGWTRPKKWP

SEQ ID NO:377
MERIFGFDIGTTSIGFSVIDYSSTQSAGNIQRLGVRIFPEARDPDGTPLNQQRRQKRMMRRQLRRRRIRR
KALNETLHEAGFLPAYGSADWPVVMADEPYELRRRGLEEGLSAYEFGRAIYHLAQHRHFKGRELEESDTP
DPDVDDEKEAANERAATLKALKNEQTTLGAWLARRPPSDRKRGIHAHRNVVAEEFERLWEVQSKFHPALK
SEEMRARISDTIFAQRPVFWRKNTLGECRFMPGEPLCPKGSWLSQQRRMLEKLNNLAIAGGNARPLDAEE
RDAILSKLQQQASMSWPGVRSALKALYKQRGEPGAEKSLKFNLELGGESKLLGNALEAKLADMFGPDWPA
HPRKQEIRHAVHERLWAADYGETPDKKRVIILSEKDRKAHREAAANSFVADFGITGEQAAQLQALKLPTG
WEPYSIPALNLFLAELEKGERFGALVNGPDWEGWRRTNFPHRNQPTGEILDKLPSPASKEERERISQLRN
PTVVRTQNELRKVVNNLIGLYGKPDRIRIEVGRDVGKSKREREEIQSGIRRNEKQRKKATEDLIKNGIAN
PSRDDVEKWILWKEGQERCPYTGDQIGFNALFREGRYEVEHIWPRSRSFDNSPRNKTLCRKDVNIEKGNR
MPFEAFGHDEDRWSAIQIRLQGMVSAKGGTGMSPGKVKRFLAKTMPEDFAARQLNDTRYAAKQILAQLKR
LWPDMGPEAPVKVEAVTGQVTAQLRKLWTLNNILADDGEKTRADHRHHAIDALTVACTHPGMTNKLSRYW
QLRDDPRAEKPALTPPWDTIRADAEKAVSEIVVSHRVRKKVSGPLHKETTYGDTGTDIKTKSGTYRQFVT
RKKIESLSKGELDEIRDPRIKEIVAAHVAGRGGDPKKAFPPYPCVSPGGPEIRKVRLTSKQQLNLMAQTG
NGYADLGSNHHIAIYRLPDGKADFEIVSLFDASRRLAQRNPIVQRTRADGASFVMSLAAGEAIMIPEGSK
KGIWIVQGVWASGQVVLERDTDADHSTTTRPMPNPILKDDAKKVSIDPIGRVRPSND

SEQ ID NO:378
MNKRILGLDTGTNSLGWAVVDWDEHAQSYELIKYGDVIFQEGVKIEKGIESSKAAERSGYKAIRKQYFRR
RLRKIQVLKVLVKYHLCPYLSDDDLRQWHLQKQYPKSDELMLWQRTSDEEGKNPYYDRHRCLHEKLDLTV
EADRYTLGRALYHLTQRRGFLSNRLDTSADNKEDGVVKSGISQLSTEMEEAGCEYLGDYFYKLYDAQGNK
VRIRQRYTDRNKHYQHEFDAICEKQELSSELIEDLQRAIFFQLPLKSQRHGVGRCTFERGKPRCADSHPD
YEEFRMLCFVNNIQVKGPHDLELRPLTYEEREKIEPLFFRKSKPNFDFEDIAKALAGKKNYAWIHDKEER
AYKFNYRMTQGVPGCPTIAQLKSIFGDDWKTGIAETYTLIQKKNGSKSLQEMVDDVWNVLYSFSSVEKLK
EFAHHKLQLDEESAEKFAKIKLSHSFAALSLKAIRKFLPFLRKGMYYTHASFFANIPTIVGKEIWNKEQN
RKYIMENVGELVFNYQPKHREVQGTIEMLIKDFLANNFELPAGATDKLYHPSMIETYPNAQRNEFGILQL
GSPRTNAIRNPMAMRSLHILRRVVNQLLKESIIDENTEVHVEYARELNDANKRRAIADRQKEQDKQHKKY
GDEIRKLYKEETGKDIEPTQTDVLKFQLWEEQNHHCLYTGEQIGITDFIGSNPKFDIEHTIPQSVGGDST
QMNLTLCDNRFNREVKKAKLPTELANHEEILTRIEPWKNKYEQLVKERDKQRTFAGMDKAVKDIRIQKRH
KLQMEIDYWRGKYERFTMTEVPEGFSRRQGTGIGLISRYAGLYLKSLFHQADSRNKSNVYVVKGVATAEF
RKMWGLQSEYEKKCRDNHSHHCMDAITIACIGKREYDLMAEYYRMEETFKQGRGSKPKFSKPWATFTEDV
LNIYKNLLVVHDTPNNMPKHTKKYVQTSIGKVLAQGDTARGSLHLDTYYGAIERDGEIRYVVRRPLSSFT
KPEELENIVDETVKRTIKEAIADKNFKQAIAEPIYMNEEKGILIKKVRCFAKSVKQPINIRQHRDLSKKE
YKQQYHVMNENNYLLAIYEGLVKNKVVREFEIVSYIEAAKYYKRSQDRNIFSSIVPTHSTKYGLPLKTKL
LMGQLVLMFEENPDEIQVDNTKDLVKRLYKVVGIEKDGRIKFKYHQEARKEGLPIFSTPYKNNDDYAPIF
RQSINNINILVDGIDFTIDILGKVTLKE

SEQ ID NO:379
MNYKMGLDIGIASVGWAVINLDLKRIEDLGVRIFDKAEHPQNGESLALPRRIARSARRRLRRRKHRLERI
RRLLVSENVLTKEEMNLLFKQKKQIDVWQLRVDALERKLNNDELARVLLHLAKRRGFKSNRKSERNSKES
SEFLKNIEENQSILAQYRSVGEMIVKDSKFAYHKRNKLDSYSNMIARDDLEREIKLIFEKQREFNNPVCT
ERLEEKYLNIWSSQRPFASKEDIEKKVGFCTFEPKEKRAPKATYTFQSFIVWEHINKLRLVSPDETRALT
EIERNLLYKQAFSKNKMTYYDIRKLLNLSDDIHFKGLLYDPKSSLKQIENIRFLELDSYHKIRKCIENVY
GKDGIRMFNETDIDTFGYALTIFKDDEDIVAYLQNEYITKNGKRVSNLANKVYDKSLIDELLNLSFSKFA

HLSMKAIRNILPYMEQGEIYSKACELAGYNFTGPKKKEKALLLPVIPNIANPVVMRALTQSRKVVNAIIK
KYGSPVSIHIELARDLSHSFDERKKIQKDQTENRKKNETAIKQLIEYELTKNPTGLDIVKFKLWSEQQGR
CMYSLKPIELERLLEPGYVEVDHILPYSRSLDDSYANKVLVLTKENREKGNHTPVEYLGLGSERWKKFEK
FVLANKQFSKKKKQNLLRLRYEETEEKEFKERNLNDTRYISKFFANFIKEHLKFADGDGGQKVYTINGKI
TAHLRSRWDFNKNREESDLHHAVDAVIVACATQGMIKKITEFYKAREQNKESAKKKEPIFPQPWPHFADE
LKARLSKFPQESIEAFALGNYDRKKLESLRPVFVSRMPKRSVTGAAHQETLRRCVGIDEQSGKIQTAVKT
KLSDIKLDKDGHFPMYQKESDPRTYEAIRQRLLEHNNDPKKAFQEPLYKPKKNGEPGPVIRTVKIIDTKN
KVVHLDGSKTVAYNSNIVRTDVFEKDGKYYCVPVYTMDIMKGTLPNKAIEANKPYSEWKEMTEEYTFQFS
LFPNDLVRIVLPREKTIKTSTNEEIIIKDIFAYYKTIDSATGGLELISHDRNFSLRGVGSKTLKRFEKYQ
VDVLGNIHKVKGEKRVGLAAPTNQKKGKTVDSLQSVSD

SEQ ID NO:380
MRRLGLDLGTNSIGWCLLDLGDDGEPVSIFRTGARIFSDGRDPKSLGSLKATRREARLTRRRRDRFIQRQ
KNLINALVKYGLMPADEIQRQALAYKDPYPIRKKALDEAIDPYEMGRAIFHINQRRGFKSNRKSADNEAG
VVKQSIADLEMKLGEAGARTIGEFLADRQATNDTVRARRLSGTNALYEFYPDRYMLEQEFDTLWAKQAAF
NPSLYIEAARERLKEIVFFQRKLKPQEVGRCIFLSDEDRISKALPSFQRFRIYQELSNLAWIDHDGVAHR
ITASLALRDHLFDELEHKKKLTFKAMRAILRKQGVVDYPVGFNLESDNRDHLIGNLTSCIMRDAKKMIGS
AWDRLDEEEQDSFILMLQDDQKGDDEVRSILTQQYGLSDDVAEDCLDVRLPDGHGSLSKKAIDRILPVLR
DQGLIYYDAVKEAGLGEANLYDPYAALSDKLDYYGKALAGHVMGASGKFEDSDEKRYGTISNPTVHIALN
QVRAVVNELIRLHGKPDEVVIEIGRDLPMGADGKRELERFQKEGRAKNERARDELKKLGHIDSRESRQKF
QLWEQLAKEPVDRCCPFTGKMMSISDLFSDKVEIEHLLPFSLTLDDSMANKTVCFRQANRDKGNRAPFDA
FGNSPAGYDWQEILGRSQNLPYAKRWRFLPDAMKRFEADGGFLERQLNDTRYISRYTTEYISTIIPKNKI
WVVTGRLTSLLRGFWGLNSILRGHNTDDGTPAKKSRDDHRHHAIDAIVVGMTSRGLLQKVSKAARRSEDL
DLTRLFEGRIDPWDGFRDEVKKHIDAIIVSHRPRKKSQGALHNDTAYGIVEHAENGASTVVHRVPITSLG
KQSDIEKVRDPLIKSALLNETAGLSGKSFENAVQKWCADNSIKSLRIVETVSIIPITDKEGVAYKGYKGD
GNAYMDIYQDPTSSKWKGEIVSRFDANQKGFIPSWQSQFPTARLIMRLRINDLLKLQDGEIEEIYRVQRL
SGSKILMAPHTEANVDARDRDKNDTFKLTSKSPGKLQSASARKVHISPTGLIREG

SEQ ID NO:381
MKNILGLDLGLSSIGWSVIRENSEEQELVAMGSRVVSLTAAELSSFTQGNGVSINSQRTQKRTQRKGYDR
YQLRRTLLRNKLDTLGMLPDDSLSYLPKLQLWGLRAKAVTQRIELNELGRVLLHLNQKRGYKSIKSDFSG
DKKITDYVKTVKTRYDELKEMRLTIGELFFRRLTENAFFRCKEQVYPRQAYVEEFDCIMNCQRKFYPDIL
TDETIRCIRDEIIYYQRPLKSCKYLVSRCEFEKRFYLNAAGKKTEAGPKVSPRTSPLFQVCRLWESINNI
VVKDRRNEIVFISAEQRAALFDFLNTHEKLKGSDLLKLLGLSKTYGYRLGEQFKTGIQGNKTRVEIERAL
GNYPDKKRLLQFNLQEESSSMVNTETGEIIPMISLSFEQEPLYRLWHVLYSIDDREQLQSVLRQKFGIDD
DEVLERLSAIDLVKAGFGNKSSKAIRRILPFLQLGMNYAEACEAAGYNHSNNYTKAENEARALLDRLPAI
KKNELRQPVVEKILNQMVNVVNALMEKYGRFDEIRVELARELKQSKEERSNTYKSINKNQRENEQIAKRI
VEYGVPTRSRIQKYKMWEESKHCCIYCGQPVDVGDFLRGFDVEVEHIIPKSLYFDDSFANKVCSCRSCNK
EKNNRTAYDYMKSKGEKALSDYVERVNTMYTNNQISKTKWQNLLTPVDKISIDFIDRQLRESQYIARKAK
EILTSICYNVTATSGSVTSFLRHVWGWDTVLHDLNFDRYKKVGLTEVIEVNHRGSVIRREQIKDWSKRFD
HRHHAIDALTIACTKQAYIQRLNNLRAEEGPDFNKMSLERYIQSQPHFSVAQVREAVDRILVSFRAGKRA
VTPGKRYIRKNRKRISVQSVLIPRGALSEESVYGVIHVWEKDEQGHVIQKQRAVMKYPITSINREMLDKE
KVVDKRIHRILSGRLAQYNDNPKEAFAKPVYIDKECRIPIRTVRCFAKPAINTLVPLKKDDKGNPVAWVN
PGNNHHVAIYRDEDGKYKERTVTFWEAVDRCRVGIPAIVTQPDTIWDNILQRNDISENVLESLPDVKWQF
VLSLQQNEMFILGMNEEDYRYAMDQQDYALLNKYLYRVQKLSKSDYSFRYHTETSVEDKYDGKPNLKLSM
QMGKLKRVSIKSLLGLNPHKVHISVLGEIKEIS

SEQ ID NO:382
MAEKQHRWGLDIGTNSIGWAVIALIEGRPAGLVATGSRIFSDGRNPKDGSSLAVERRGPRQMRRRRDRYL
RRRDRFMQALINVGLMPGDAAARKALVTENPYVLRQRGLDQALTLPEFGRALFHLNQRRGFQSNRKTDRA
TAKESGKVKNAIAAFRAGMGNARTVGEALARRLEDGRPVRARMVGQGKDEHYELYIAREWIAQEFDALWA
SQQRFHAEVLADAARDRLRAILLFQRKLLPVPVGKCFLEPNQPRVAAALPSAQRFRLMQELNHLRVMTLA

DKRERPLSFQERNDLLAQLVARPKCGFDMLRKIVFGANKEAYRFTIESERRKELKGCDTAAKLAKVNALG
TRWQALSLDEQDRLVCLLLDGENDAVLADALREHYGLTDAQIDTLLGLSFEDGHMRLGRSALLRVLDALE
SGRDEQGLPLSYDKAVVAAGYPAHTADLENGERDALPYYGELLWRYTQDAPTAKNDAERKFGKIANPTVH
IGLNQLRKLVNALIQRYGKPAQIVVELARNLKAGLEEKERIKKQQTANLERNERIRQKLQDAGVPDNREN
RLRMRLFEELGQGNGLGTPCIYSGRQISLQRLFSNDVQVDHILPFSKTLDDSFANKVLAQHDANRYKGNR
GPFEAFGANRDGYAWDDIRARAAVLPRNKRNRFAETAMQDWLHNETDFLARQLTDTAYLSRVARQYLTAI
CSKDDVYVSPGRLTAMLRAKWGLNRVLDGVMEEQGRPAVKNRDDHRHHAIDAVVIGATDRAMLQQVATLA
ARAREQDAERLIGDMPTPWPNFLEDVRAAVARCVVSHKPDHGPEGGLHNDTAYGIVAGPFEDGRYRVHR
VSLFDLKPGDLSNVRCDAPLQAELEPIFEQDDARAREVALTALAERYRQRKVWLEELMSVLPIRPRGEDG
KTLPDSAPYKAYKGDSNYCYELFINERGRWDGELISTFRANQAAYRRFRNDPARFRRYTAGGRPLLMRLC
INDYIAVGTAAERTIFRVVKMSENKITLAEHFEGGTLKQRDADKDDPFKYLTKSPGALRDLGARRIFVDL
IGRVLDPGIKGD

SEQ ID NO:383
MIERILGVDLGISSLGWAIVEYDKDDEAANRIIDCGVRLFTAAETPKKKESPNKARREARGIRRVLNRRR
VRMNMIKKLFLRAGLIQDVDLDGEGGMFYSKANRADVWELRHDGLYRLLKGDELARVLIHIAKHRGYKFI
GDDEADEESGKVKKAGVVLRQNFEAAGCRTVGEWLWRERGANGKKRNKHGDYEISIHRDLLVEEVEAIFV
AQQEMRSTIATDALKAAYREIAFFVRPMQRIEKMVGHCTYFPEERRAPKSAPTAEKFIAISKFFSTVIID
NEGWEQKIIERKTLEELLDFAVSREKVEFRHLRKFLDLSDNEIFKGLHYKGKPKTAKKREATLFDPNEPT
ELEFDKVEAEKKAWISLRGAAKLREALGNEFYGRFVALGKHADEATKILTYYKDEGQKRRELTKLPLEAE
MVERLVKIGFSDFLKLSLKAIRDILPAMESGARYDEAVLMLGVPHKEKSAILPPLNKTDIDILNPTVIRA
FAQFRKVANALVRKYGAFDRVHFELAREINTKGEIEDIKESQRKNEKERKEAADWIAETSFQVPLTRKNI
LKKRLYIQQDGRCAYTGDVIELERLFDEGYCEIDHILPRSRSADDSFANKVLCLARANQQKTDRTPYEWF
GHDAARWNAFETRTSAPSNRVRTGKGKIDRLLKKNFDENSEMAFKDRNLNDTRYMARAIKTYCEQYWVFK
NSHTKAPVQVRSGKLTSVLRYQWGLESKDRESHTHHAVDAIIIAFSTQGMVQKLSEYYRFKETHREKERP
KLAVPLANFRDAVEEATRIENTETVKEGVEVKRLLISRPPRARVTGQAHEQTAKPYPRIKQVKNKKKWRL
APIDEEKFESFKADRVASANQKNFYETSTIPRVDVYHKKGKFHLVPIYLHEMVLNELPNLSLGTNPEAMD
ENFFKFSIFKDDLISIQTQGTPKKPAKIIMGYFKNMHGANMVLSSINNSPCEGFTCTPVSMDKKHKDKCK
LCPEENRIAGRCLQGFLDYWSQEGLRPPRKEFECDQGVKFALDVKKYQIDPLGYYYEVKQEKRLGTIPQM
RSAKKLVKK

SEQ ID NO:384
MNNSIKSKPEVTIGLDLGVGSVGWAIVDNETNIIHHLGSRLFSQAKTAEDRRSFRGVRRLIRRRKYKLKR
FVNLIWKYNSYFGFKNKEDILNNYQEQQKLHNTVLNLKSEALNAKIDPKALSWILHDYLKNRGHFYEDNR
DFNVYPTKELAKYFDKYGYYKGIIDSKEDNDNKLEEELTKYKFSNKHWLEEVKKVLSNQTGLPEKFKEEY
ESLFSYVRNYSEGPGSINSVSPYGIYHLDEKEGKVVQKYNNIWDKTIGKCNIFPDEYRAPKNSPIAMIFN
EINELSTIRSYSIYLTGWFINQEFKKAYLNKLLDLLIKTNGEKPIDARQFKKLREETIAESIGKETLKDV
ENEEKLEKEDHKWKLKGLKLNTNGKIQYNDLSSLAKFVHKLKQHLKLDFLLEDQYATLDKINFLQSLFVY
LGKHLRYSNRVDSANLKEFSDSNKLFERILQKQKDGLFKLFEQTDKDDEKILAQTHSLSTKAMLLAITRM
TNLDNDEDNQKNNDKGWNFEAIKNFDQKFIDITKKNNNLSLKQNKRYLDDRFINDAILSPGVKRILREAT
KVFNAILKQFSEEYDVTKVVIELARELSEEKELENTKNYKKLIKKNGDKISEGLKALGISEDEIKDILKS
PTKSYKFLLWLQQDHIDPYSLKEIAFDDIFTKTEKFEIDHIIPYSISFDDSSSNKLLVLAESNQAKSNQT
PYEFISSGNAGIKWEDYEAYCRKFKDGDSSLLDSTQRSKKFAKMMKTDTSSKYDIGFLARNLNDTRYATI
VFRDALEDYANNHLVEDKPMFKVVCINGSVTSFLRKNFDDSSYAKKDRDKNIHHAVDASIISIFSNETKT
LFNQLTQFADYKLFKNTDGSWKKIDPKTGVVTEVTDENWKQIRVRNQVSEIAKVIEKYIQDSNIERKARY
SRKIENKTNISLFNDTVYSAKKVGYEDQIKRKNLKTLDIHESAKENKNSKVKRQFVYRKLVNVSLLNNDK
LADLFAEKEDILMYRANPWVINLAEQIFNEYTENKKIKSQNVFEKYMLDLTKEFPEKFSEFLVKSMLRNK
TAIIYDDKKNIVHRIKRLKMLSSELKENKLSNVIIRSKNQSGTKLSYQDTINSLALMIMRSIDPTAKKQY
IRVPLNTLNLHLGDHDFDLHNMDAYLKKPKFVKYLKANEIGDEYKPWRVLTSGTLLIHKKDKKLMYISSF
QNLNDVIEIKNLIETEYKENDDSDSKKKKANRFLMTLSTILNDYILLDAKDNFDILGLSKNRIDEILNS
KLGLDKIVK

SEQ ID NO:385
MGGSEVGTVPVTWRLGVDVGERSIGLAAVSYEEDKPKEILAAVSWIHDGGVGDERSGASRLALRGMARRA
RRLRRFRRARLRDLDMLLSELGWTPLPDKNVSPVDAWLARKRLAEEYVVDETERRRLLGYAVSHMARHRG
WRNPWTTIKDLKNLPQPSDSWERTRESLEARYSVSLEPGTVGQWAGYLLQRAPGIRLNPTQQSAGRRAEL
SNATAFETRLRQEDVLWELRCIADVQGLPEDVVSNVIDAVFCQKRPSVPAERIGRDPLDPSQLRASRACL
EFQEYRIVAAVANLRIRDGSGSRPLSLEERNAVIEALLAQTERSLTWSDIALEILKLPNESDLTSVPEED
GPSSLAYSQFAPFDETSARIAEFIAKNRRKIPTFAQWWQEQDRTSRSDLVAALADNSIAGEEEQELLVHL
PDAELEALEGLALPSGRVAYSRLTLSGLTRVMRDDGVDVHNARKTCFGVDDNWRPPLPALHEATGHPVVD
RNLAILRKFLSSATMRWGPPQSIVVELARGASESRERQAEEEAARRAHRKANDRIRAELRASGLSDPSPA
DLVRARLLELYDCHCMYCGAPISWENSELDHIVPRTDGGSNRHENLAITCGACNKEKGRRPFASWAETSN
RVQLRDVIDRVQKLKYSGNMYWTRDEFSRYKKSVVARLKRRTSDPEVIQSIESTGYAAVALRDRLLSYGE
KNGVAQVAVFRGGVTAEARRWLDISIERLFSRVAIFAQSTSTKRLDRRHHAVDAVVLTTLTPGVAKTLAD
ARSRRVSAEFWRRPSDVNRHSTEEPQSPAYRQWKESCSGLGDLLISTAARDSIAVAAPLRLRPTGALHEE
TLRAFSEHTVGAAWKGAELRRIVEPEVYAAFLALTDPGGRFLKVSPSEDVLPADENRHIVLSDRVLGPRD
RVKLFPDDRGSIRVRGGAAYIASFHHARVFRWGSSHSPSFALLRVSLADLAVAGLLRDGVDVFTAELPPW
TPAWRYASIALVKAVESGDAKQVGWLVPGDELDFGPEGVTTAAGDLSMFLKYFPERHWVVTGFEDDKRIN
LKPAFLSAEQAEVLRTERSDRPDTLTEAGEILAQFFPRCWRATVAKVLCHPGLTVIRRTALGQPRWRRGH
LPYSWRPWSADPWSGGTP

SEQ ID NO:386
MHNKKNITIGFDLGIASIGWAIIDSTTSKILDWGTRTFEERKTANERRAFRSTRRNIRRKAYRNQRFINL
ILKYKDLFELKNISDIQRANKKDTENYEKIISFFTEIYKKCAAKHSNILEVKVKALDSKIEKLDLIWILH
DYLENRGFFYDLEEENVADKYEGIEHPSILLYDFFKKNGFFKSNSSIPKDLGGYSFSNLQWVNEIKKLFE
VQEINPEFSEKFLNLFTSVRDYAKGPGSEHSASEYGIFQKDEKGKVFKKYDNIWDKTIGKCSFFVEENRS
PVNYPSYEIFNLLNQLINLSTDLKTTNKKIWQLSSNDRNELLDELLKVKEKAKIISISLKKNEIKKIILK
DFGFEKSDIDDQDTIEGRKIIKEEPTTKLEVTKHLLATIYSHSSDSNWININNILEFLPYLDAICIILDR
EKSRGQDEVLKKLTEKNIFEVLKIDREKQLDFVKSIFSNTKFNFKKIGNFSLKAIREFLPKMFEQNKNSE
YLKWKDEEIRRKWEEQKSKLGKTDKKTKYLNPRIFQDEIISPGTKNTFEQAVLVLNQIIKKYSKENIIDA
IIIESPREKNDKKTIEEIKKRNKKGKGKTLEKLFQILNLENKGYKLSDLETKPAKLLDRLRFYHQQDGID
LYTLDKINIDQLINGSQKYEIEHIIPYSMSYDNSQANKILTEKAENLKKGKLIASEYIKRNGDEFYNKYY
EKAKELFINKYKKNKKLDSYVDLDEDSAKNRFRFLTLQDYDEFQVEFLARNLNDTRYSTKLFYHALVEHF
ENNEFFTYIDENSSKHKVKISTIKGHVTKYFRAKPVQKNNGPNENLNNNKPEKIEKNRENNEHHAVDAAI
VAIIGNKNPQIANLLTLADNKTDKKFLLHDENYKENIETGELVKIPKFEVDKLAKVEDLKKIIQEKYEEA
KKHTAIKFSRKTRTILNGGLSDETLYGFKYDEKEDKYFKIIKKKLVTSKNEELKKYFENPFGKKADGKSE
YTVLMAQSHLSEFNKLKEIFEKYNGFSNKTGNAFVEYMNDLALKEPTLKAEIESAKSVEKLLYYNFKPSD
QFTYHDNINNKSFKRFYKNIRIIEYKSIPIKFKILSKHDGGKSFKDTLFSLYSLVYKVYENGKESYKSIP
VTSQMRNFGIDEFDFLDENLYNKEKLDIYKSDFAKPIPVNCKPVFVLKKGSILKKKSLDIDDFKETKETE
EGNYYFISTISKRFNRDTAYGLKPLKLSVVKPVAEPSTNPIFKEYIPIHLDELGNEYPVKIKEHTDDEKL
MCTIK

### Nucleic Acids Encoding Cas9 Molecules

Nucleic acids encoding the Cas9 molecules or Cas9 polypeptides, e.g., an eaCas9 molecule or eaCas9 polypeptide, are provided herein.

Exemplary nucleic acids encoding Cas9 molecules or Cas9 polypeptides are described in Cong et al., Science 2013, 399(6121):819-823; Wang et al., Cell 2013, 153(4):910-918; Mali et al., Science 2013, 399(6121):823-826; Jinek et al., Science 2012, 337(6096):816-821. Another exemplary nucleic acid encoding a Cas9 molecule or Cas9 polypeptide is shown in black in Fig. 8.

In an embodiment, a nucleic acid encoding a Cas9 molecule or Cas9 polypeptide can be a synthetic nucleic acid sequence. For example, the synthetic nucleic acid molecule can be chemically modified, e.g., as described in Section VIII. In an embodiment, the Cas9 mRNA has one or more (e.g., all of the following properties: it is capped, polyadenylated, substituted with 5-methylcytidine and/or pscudouridinc.

In addition, or alternatively, the synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein.

In addition, or alternatively, a nucleic acid encoding a Cas9 molecule or Cas9 polypeptide may comprise a nuclear localization sequence (NLS). Nuclear localization sequences arc known in the art.

Provided below is an exemplary codon optimized nucleic acid sequence encoding a Cas9 molecule of *S. pyogenes.*

SEQ ID NO:22

ATGGATAAAAAGTACAGCATCGGGCTGGACATCGGTACAAACTCAGTGGGGTGGGCCGTGATTACGGACG
AGTACAAGGTACCCTCCAAAAAATTTAAAGTGCTGGGTAACACGGACAGACACTCTATAAAGAAAAATCT
TATTGGAGCCTTGCTGTTCGACTCAGGCGAGACAGCCGAAGCCACAAGGTTGAAGCGGACCGCCAGGAGG
CGGTATACCAGGAGAAAGAACCGCATATGCTACCTGCAAGAAATCTTCAGTAACGAGATGGCAAAGGTTG
ACGATAGCTTTTTCCATCGCCTGGAAGAATCCTTTCTTGTTGAGGAAGACAAGAAGCACGAACGGCACCC
CATCTTTGGCAATATTGTCGACGAAGTGGCATATCACGAAAAGTACCCGACTATCTACCACCTCAGGAAG
AAGCTGGTGGACTCTACCGATAAGGCGGACCTCAGACTTATTTATTTGGCACTCGCCCACATGATTAAAT
TTAGAGGACATTTCTTGATCGAGGGCGACCTGAACCCGGACAACAGTGACGTCGATAAGCTGTTCATCCA
ACTTGTGCAGACCTACAATCAACTGTTCGAAGAAAACCCTATAAATGCTTCAGGAGTCGACGCTAAAGCA
ATCCTGTCCGCGCGCCTCTCAAAATCTAGAAGACTTGAGAATCTGATTGCTCAGTTGCCCGGGGAAAAGA
AAAATGGATTGTTTGGCAACCTGATCGCCCTCAGTCTCGGACTGACCCCAAATTTCAAAAGTAACTTCGA
CCTGGCCGAAGACGCTAAGCTCCAGCTGTCCAAGGACACATACGATGACGACCTCGACAATCTGCTGGCC
CAGATTGGGGATCAGTACGCCGATCTCTTTTTGGCAGCAAAGAACCTGTCCGACGCCATCCTGTTGAGCG
ATATCTTGAGAGTGAACACCGAAATTACTAAAGCACCCCTTAGCGCATCTATGATCAAGCGGTACGACGA
GCATCATCAGGATCTGACCCTGCTGAAGGCTCTTGTGAGGCAACAGCTCCCCGAAAAATACAAGGAAATC
TTCTTTGACCAGAGCAAAAACGGCTACGCTGGCTATATAGATGGTGGGGCCAGTCAGGAGGAATTCTATA
AATTCATCAAGCCCATTCTCGAGAAAATGGACGGCACAGAGGAGTTGCTGGTCAAACTTAACAGGGAGGA
CCTGCTGCGGAAGCAGCGGACCTTTGACAACGGGTCTATCCCCCACCAGATTCATCTGGGCGAACTGCAC
GCAATCCTGAGGAGGCAGGAGGATTTTTATCCTTTTCTTAAAGATAACCGCGAGAAAATAGAAAAGATTC
TTACATTCAGGATCCCGTACTACGTGGGACCTCTCGCCCGGGGCAATTCACGGTTTGCCTGGATGACAAG
GAAGTCAGAGGAGACTATTACACCTTGGAACTTCGAAGAAGTGGTGGACAAGGGTGCATCTGCCCAGTCT
TTCATCGAGCGGATGACAAATTTTGACAAGAACCTCCCTAATGAGAAGGTGCTGCCCAAACATTCTCTGC
TCTACGAGTACTTTACCGTCTACAATGAACTGACTAAAGTCAAGTACGTCACCGAGGGAATGAGGAAGCC
GGCATTCCTTAGTGGAGAACAGAAGAAGGCGATTGTAGACCTGTTGTTCAAGACCAACAGGAAGGTGACT
GTGAAGCAACTTAAAGAAGACTACTTTAAGAAGATCGAATGTTTTGACAGTGTGGAAATTTCAGGGGTTG
AAGACCGCTTCAATGCGTCATTGGGGACTTACCATGATCTTCTCAAGATCATAAAGGACAAAGACTTCCT
GGACAACGAAGAAAATGAGGATATTCTCGAAGACATCGTCCTCACCCTGACCCTGTTCGAAGACAGGGAA
ATGATAGAAGAGCGCTTGAAAACCTATGCCCACCTCTTCGACGATAAAGTTATGAAGCAGCTGAAGCGCA
GGAGATACACAGGATGGGGAAGATTGTCAAGGAAGCTGATCAATGGAATTAGGGATAAACAGAGTGGCAA

GACCATACTGGATTTCCTCAAATCTGATGGCTTCGCCAATAGGAACTTCATGCAACTGATTCACGATGAC
TCTCTTACCTTCAAGGAGGACATTCAAAAGGCTCAGGTGAGCGGCAGGGAGACTCCCTTCATGAACACA
TCGCGAATTTGGCAGGTTCCCCCGCTATTAAAAAGGGCATCCTTCAAACTGTCAAGGTGGTGGATGAATT
GGTCAAGGTAATGGGCAGACATAAGCCAGAAAATATTGTGATCGAGATGGCCCGCGAAAACCAGACCACA
CAGAAGGGCCAGAAAAATAGTAGAGAGCGGATGAAGAGGATCGAGGAGGGCATCAAAGAGCTGGGATCTC
AGATTCTCAAAGAACACCCCGTAGAAAACACACAGCTGCAGAACGAAAAATTGTACTTGTACTATCTGCA
GAACGGCAGAGACATGTACGTCGACCAAGAACTTGATATTAATAGACTGTCCGACTATGACGTAGACCAT
ATCGTGCCCCAGTCCTTCCTGAAGGACGACTCCATTGATAACAAAGTCTTGACAAGAAGCGACAAGAACA
GGGGTAAAAGTGATAATGTGCCTAGCGAGGAGGTGGTGAAAAAAATGAAGAACTACTGGCGACAGCTGCT
TAATGCAAAGCTCATTACACAACGGAAGTTCGATAATCTGACGAAAGCAGAGAGAGGTGGCTTGTCTGAG
TTGGACAAGGCAGGGTTTATTAAGCGGCAGCTGGTGGAAACTAGGCAGATCACAAAGCACGTGGCGCAGA
TTTTGGACAGCCGGATGAACACAAAATACGACGAAAATGATAAACTGATACGAGAGGTCAAAGTTATCAC
GCTGAAAAGCAAGCTGGTGTCCGATTTTCGGAAAGACTTCCAGTTCTACAAAGTTCGCGAGATTAATAAC
TACCATCATGCTCACGATGCGTACCTGAACGCTGTTGTCGGGACCGCCTTGATAAAGAAGTACCCAAAGC
TGGAATCCGAGTTCGTATACGGGGATTACAAAGTGTACGATGTGAGGAAAATGATAGCCAAGTCCGAGCA
GGAGATTGGAAAGGCCACAGCTAAGTACTTCTTTTATTCTAACATCATGAATTTTTTTAAGACGGAAATT
ACCCTGGCCAACGGAGAGATCAGAAAGCGGCCCCTTATAGAGACAAATGGTGAAACAGGTGAAATCGTCT
GGGATAAGGGCAGGGATTTCGCTACTGTGAGGAAGGTGCTGAGTATGCCACAGGTAAATATCGTGAAAAA
AACCGAAGTACAGACCGGAGGATTTTCCAAGGAAAGCATTTTGCCTAAAAGAAACTCAGACAAGCTCATC
GCCCGCAAGAAAGATTGGGACCCTAAGAAATACGGGGGATTTGACTCACCCACCGTAGCCTATTCTGTGC
TGGTGGTAGCTAAGGTGGAAAAAGGAAAGTCTAAGAAGCTGAAGTCCGTGAAGGAACTCTTGGGAATCAC
TATCATGGAAAGATCATCCTTTGAAAAGAACCCTATCGATTTCCTGGAGGCTAAGGGTTACAAGGAGGTC
AAGAAAGACCTCATCATTAAACTGCCAAAATACTCTCTCTTCGAGCTGGAAAATGGCAGGAAGAGAATGT
TGGCCAGCGCCGGAGAGCTGCAAAAGGGAAACGAGCTTGCTCTGCCCTCCAAATATGTTAATTTTCTCTA
TCTCGCTTCCCACTATGAAAAGCTGAAAGGGTCTCCCGAAGATAACGAGCAGAAGCAGCTGTTCGTCGAA
CAGCACAAGCACTATCTGGATGAAATAATCGAACAAATAAGCGAGTTCAGCAAAAGGGTTATCCTGGCGG
ATGCTAATTTGGACAAAGTACTGTCTGCTTATAACAAGCACCGGGATAAGCCTATTAGGGAACAAGCCGA
GAATATAATTCACCTCTTTACACTCACGAATCTCGGAGCCCCCGCCGCCTTCAAATACTTTGATACGACT
ATCGACCGGAAACGGTATACCAGTACCAAAGAGGTCCTCGATGCCACCCTCATCCACCAGTCAATTACTG
GCCTGTACGAAACACGGATCGACCTCTCTCAACTGGGCGGCGACTAG

Provided below is the corresponding amino acid sequence of a *S. pyogenes* Cas9 molecule.

SEQ ID NO:23





















*

Provided below is an exemplary codon optimized nucleic acid sequence encoding a Cas9 molecule of *N. meningitidis.*

SEQ ID NO:24

ATGGCCGCCTTCAAGCCCAACCCCATCAACTACATCCTGGGCCTGGACATCGGCATCGCCAGCGTGGGCT
GGGCCATGGTGGAGATCGACGAGGACGAGAACCCCATCTGCCTGATCGACCTGGGTGTGCGCGTGTTCGA
GCGCGCTGAGGTGCCCAAGACTGGTGACAGTCTGGCTATGGCTCGCCGGCTTGCTCGCTCTGTTCGGCGC
CTTACTCGCCGGCGCGCTCACCGCCTTCTGCGCGCTCGCCGCCTGCTGAAGCGCGAGGGTGTGCTGCAGG
CTGCCGACTTCGACGAGAACGGCCTGATCAAGAGCCTGCCCAACACTCCTTGGCAGCTGCGCGCTGCCGC
TCTGGACCGCAAGCTGACTCCTCTGGAGTGGAGCGCCGTGCTGCTGCACCTGATCAAGCACCGCGGCTAC
CTGAGCCAGCGCAAGAACGAGGGCGAGACCGCCGACAAGGAGCTGGGTGCTCTGCTGAAGGGCGTGGCCG
ACAACGCCCACGCCCTGCAGACTGGTGACTTCCGCACTCCTGCTGAGCTGGCCCTGAACAAGTTCGAGAA
GGAGAGCGGCCACATCCGCAACCAGCGCGGCGACTACAGCCACACCTTCAGCCGCAAGGACCTGCAGGCC
GAGCTGATCCTGCTGTTCGAGAAGCAGAAGGAGTTCGGCAACCCCCACGTGAGCGGCGGCCTGAAGGAGG
GCATCGAGACCCTGCTGATGACCCAGCGCCCCGCCCTGAGCGGCGACGCCGTGCAGAAGATGCTGGGCCA
CTGCACCTTCGAGCCAGCCGAGCCCAAGGCCGCCAAGAACACCTACACCGCCGAGCGCTTCATCTGGCTG
ACCAAGCTGAACAACCTGCGCATCCTGGAGCAGGGCAGCGAGCGCCCCCTGACCGACACCGAGCGCGCCA
CCCTGATGGACGAGCCCTACCGCAAGAGCAAGCTGACCTACGCCCAGGCCCGCAAGCTGCTGGGTCTGGA
GGACACCGCCTTCTTCAAGGGCCTGCGCTACGGCAAGGACAACGCCGAGGCCAGCACCCTGATGGAGATG
AAGGCCTACCACGCCATCAGCCGCGCCCTGGAGAAGGAGGGCCTGAAGGACAAGAAGAGTCCTCTGAACC
TGAGCCCCGAGCTGCAGGACGAGATCGGCACCGCCTTCAGCCTGTTCAAGACCGACGAGGACATCACCGG
CCGCCTGAAGGACCGCATCCAGCCCGAGATCCTGGAGGCCCTGCTGAAGCACATCAGCTTCGACAAGTTC
GTGCAGATCAGCCTGAAGGCCCTGCGCCGCATCGTGCCCCTGATGGAGCAGGGCAAGCGCTACGACGAGG
CCTGCGCCGAGATCTACGGCGACCACTACGGCAAGAAGAACACCGAGGAGAAGATCTACCTGCCTCCTAT
CCCCGCCGACGAGATCCGCAACCCCGTGGTGCTGCGCGCCCTGAGCCAGGCCCGCAAGGTGATCAACGGC
GTGGTGCGCCGCTACGGCAGCCCCGCCCGCATCCACATCGAGACCGCCCGCGAGGTGGGCAAGAGCTTCA
AGGACCGCAAGGAGATCGAGAAGCGCCAGGAGGAGAACCGCAAGGACCGCGAGAAGGCCGCCGCCAAGTT
CCGCGAGTACTTCCCCAACTTCGTGGGCGAGCCCAAGAGCAAGGACATCCTGAAGCTGCGCCTGTACGAG
CAGCAGCACGGCAAGTGCCTGTACAGCGGCAAGGAGATCAACCTGGGCCGCCTGAACGAGAAGGGCTACG
TGGAGATCGACCACGCCCTGCCCTTCAGCCGCACCTGGGACGACAGCTTCAACAACAAGGTGCTGGTGCT
GGGCAGCGAGAACCAGAACAAGGGCAACCAGACCCCCTACGAGTACTTCAACGGCAAGGACAACAGCCGC
GAGTGGCAGGAGTTCAAGGCCCGCGTGGAGACCAGCCGCTTCCCCCGCAGCAAGAAGCAGCGCATCCTGC
TGCAGAAGTTCGACGAGGACGGCTTCAAGGAGCGCAACCTGAACGACACCCGCTACGTGAACCGCTTCCT
GTGCCAGTTCGTGGCCGACCGCATGCGCCTGACCGGCAAGGGCAAGAAGCGCGTGTTCGCCAGCAACGGC
CAGATCACCAACCTGCTGCGCGGCTTCTGGGGCCTGCGCAAGGTGCGCGCCGAGAACGACCGCCACCACG
CCCTGGACGCCGTGGTGGTGGCCTGCAGCACCGTGGCCATGCAGCAGAAGATCACCCGCTTCGTGCGCTA
CAAGGAGATGAACGCCTTCGACGGTAAAACCATCGACAAGGAGACCGGCGAGGTGCTGCACCAGAAGACC
CACTTCCCCCAGCCCTGGGAGTTCTTCGCCCAGGAGGTGATGATCCGCGTGTTCGGCAAGCCCGACGGCA
AGCCCGAGTTCGAGGAGGCCGACACCCCCGAGAAGCTGCGCACCCTGCTGGCCGAGAAGCTGAGCAGCCG
CCCTGAGGCCGTGCACGAGTACGTGACTCCTCTGTTCGTGAGCCGCGCCCCCAACCGCAAGATGAGCGGT
CAGGGTCACATGGAGACCGTGAAGAGCGCCAAGCGCCTGGACGAGGGCGTGAGCGTGCTGCGCGTGCCCC
TGACCCAGCTGAAGCTGAAGGACCTGGAGAAGATGGTGAACCGCGAGCGCGAGCCCAAGCTGTACGAGGC
CCTGAAGGCCCGCCTGGAGGCCCACAAGGACGACCCCGCCAAGGCCTTCGCCGAGCCCTTCTACAAGTAC

GACAAGGCCGGCAACCGCACCCAGCAGGTGAAGGCCGTGCGCGTGGAGCAGGTGCAGAAGACCGGCGTGT
GGGTGCGCAACCACAACGGCATCGCCGACAACGCCACCATGGTGCGCGTGGACGTGTTCGAGAAGGCGA
CAAGTACTACCTGGTGCCCATCTACAGCTGGCAGGTGGCCAAGGGCATCCTGCCCGACCGCGCCGTGGTG
CAGGGCAAGGACGAGGAGGACTGGCAGCTGATCGACGACAGCTTCAACTTCAAGTTCAGCCTGCACCCCA
ACGACCTGGTGGAGGTGATCACCAAGAAGGCCCGCATGTTCGGCTACTTCGCCAGCTGCCACCGCGGCAC
CGGCAACATCAACATCCGCATCCACGACCTGGACCACAAGATCGGCAAGAACGGCATCCTGGAGGGCATC
GGCGTGAAGACCGCCCTGAGCTTCCAGAAGTACCAGATCGACGAGCTGGGCAAGGAGATCCGCCCCTGCC
GCCTGAAGAAGCGCCCTCCTGTGCGCTAA

Provided below is the corresponding amino acid sequence of a *N*. *meningitidis* Cas9 molecule.

SEQ ID NO:25

MAAFKPNPINYILGLDIGIASVGWAMVEIDEDENPICLIDLGVRVFERAEVPKTGDSLAMARRLARSVRR
LTRRRAHRLLRARRLLKREGVLQAADFDENGLIKSLPNTPWQLRAAALDRKLTPLEWSAVLLHLIKHRGY
LSQRKNEGETADKELGALLKGVADNAHALQTGDFRTPAELALNKFEKESGHIRNQRGDYSHTFSRKDLQA
ELILLFEKQKEFGNPHVSGGLKEGIETLLMTQRPALSGDAVQKMLGHCTFEPAEPKAAKNTYTAERFIWL
TKLNNLRILEQGSERPLTDTERATLMDEPYRKSKLTYAQARKLLGLEDTAFFKGLRYGKDNAEASTLMEM
KAYHAISRALEKEGLKDKKSPLNLSPELQDEIGTAFSLFKTDEDITGRLKDRIQPEILEALLKHISFDKF
VQISLKALRRIVPLMEQGKRYDEACAEIYGDHYGKKNTEEKIYLPPIPADEIRNPVVLRALSQARKVING
VVRRYGSPARIHIETAREVGKSFKDRKEIEKRQEENRKDREKAAAKFREYFPNFVGEPKSKDILKLRLYE
QQHGKCLYSGKEINLGRLNEKGYVEIDHALPFSRTWDDSFNNKVLVLGSENQNKGNQTPYEYFNGKDNSR
EWQEFKARVETSRFPRSKKQRILLQKFDEDGFKERNLNDTRYVNRFLCQFVADRMRLTGKGKKRVFASNG
QITNLLRGFWGLRKVRAENDRHHALDAVVVACSTVAMQQKITRFVRYKEMNAFDGKTIDKETGEVLHQKT
HFPQPWEFFAQEVMIRVFGKPDGKPEFEEADTPEKLRTLLAEKLSSRPEAVHEYVTPLFVSRAPNRKMSG
QGHMETVKSAKRLDEGVSVLRVPLTQLKLKDLEKMVNREREPKLYEALKARLEAHKDDPAKAFAEPFYKY
DKAGNRTQQVKAVRVEQVQKTGVWVRNHNGIADNATMVRVDVFEKGDKYYLVPIYSWQVAKGILPDRAVV
QGKDEEDWQLIDDSFNFKFSLHPNDLVEVITKKARMFGYFASCHRGTGNINIRIHDLDHKIGKNGILEGI
GVKTALSFQKYQIDELGKEIRPCRLKKRPPVR*

Provided below is an amino acid sequence of a *S. aureus* Cas9 molecule.

SEQ ID NO:26

MKRNYILGLDIGITSVGYGIIDYETRDVIDAGVRLFKEANVENNEGRRSKRGARRLKRRRRHRIQRVKKL
LFDYNLLTDHSELSGINPYEARVKGLSQKLSEEEFSAALLHLAKRRGVHNVNEVEEDTGNELSTKEQISR
NSKALEEKYVAELQLERLKKDGEVRGSINRFKTSDYVKEAKQLLKVQKAYHQLDQSFIDTYIDLLETRRT
YYEGPGEGSPFGWKDIKEWYEMLMGHCTYFPEELRSVKYAYNADLYNALNDLNNLVITRDENEKLEYYEK
FQIIENVFKQKKKPTLKQIAKEILVNEEDIKGYRVTSTGKPEFTNLKVYHDIKDITARKEIIENAELLDQ
IAKILTIYQSSEDIQEELTNLNSELTQEEIEQISNLKGYTGTHNLSLKAINLILDELWHTNDNQIAIFNR
LKLVPKKVDLSQQKEIPTTLVDDFILSPVVKRSFIQSIKVINAIIKKYGLPNDIIIELAREKNSKDAQKM
INEMQKRNRQTNERIEEIIRTTGKENAKYLIEKIKLHDMQEGKCLYSLEAIPLEDLLNNPFNYEVDHIIP
RSVSFDNSFNNKVLVKQEENSKKGNRTPFQYLSSSDSKISYETFKKHILNLAKGKGRISKTKKEYLLEER
DINRFSVQKDFINRNLVDTRYATRGLMNLLRSYFRVNNLDVKVKSINGGFTSFLRRKWKFKKERNKGYKH
HAEDALIIANADFIFKEWKKLDKAKKVMENQMFEEKQAESMPEIETEQEYKEIFITPHQIKHIKDFKDYK
YSHRVDKKPNRELINDTLYSTRKDDKGNTLIVNNLNGLYDKDNDKLKKLINKSPEKLLMYHHDPQTYQKL
KLIMEQYGDEKNPLYKYYEETGNYLTKYSKKDNGPVIKKIKYYGNKLNAHLDITDDYPNSRNKVVKLSLK
PYRFDVYLDNGVYKFVTVKNLDVIKKENYYEVNSKCYEEAKKLKKISNQAEFIASFYNNDLIKINGELYR

VIGVNNDLLNRIEVNMIDITYREYLENMNDKRPPRIIKTIASKTQSIKKYSTDILGNLYEVKSKKHPQII
KKG*

Provided below is an exemplary codon optimized nucleic acid sequence encoding a Cas9 molecule of *S. aureus* Cas9.

SEQ ID NO:39

ATGAAAAGGAACTACATTCTGGGGCTGGACATCGGGATTACAAGCGTGGGGTATGGGATTATTGACTATG
AAACAAGGGACGTGATCGACGCAGGCGTCAGACTGTTCAAGGAGGCCAACGTGGAAAACAATGAGGGACG
GAGAAGCAAGAGGGGAGCCAGGCGCCTGAAACGACGGAGAAGGCACAGAATCCAGAGGGTGAAGAAACTG
CTGTTCGATTACAACCTGCTGACCGACCATTCTGAGCTGAGTGGAATTAATCCTTATGAAGCCAGGGTGA
AAGGCCTGAGTCAGAAGCTGTCAGAGGAAGAGTTTTCCGCAGCTCTGCTGCACCTGGCTAAGCGCCGAGG
AGTGCATAACGTCAATGAGGTGGAAGAGGACACCGGCAACGAGCTGTCTACAAAGGAACAGATCTCACGC
AATAGCAAAGCTCTGGAAGAGAAGTATGTCGCAGAGCTGCAGCTGGAACGGCTGAAGAAAGATGGCGAGG
TGAGAGGGTCAATTAATAGGTTCAAGACAAGCGACTACGTCAAAGAAGCCAAGCAGCTGCTGAAAGTGCA
GAAGGCTTACCACCAGCTGGATCAGAGCTTCATCGATACTTATATCGACCTGCTGGAGACTCGGAGAACC
TACTATGAGGGACCAGGAGAAGGGAGCCCCTTCGGATGGAAAGACATCAAGGAATGGTACGAGATGCTGA
TGGGACATTGCACCTATTTTCCAGAAGAGCTGAGAAGCGTCAAGTACGCTTATAACGCAGATCTGTACAA
CGCCCTGAATGACCTGAACAACCTGGTCATCACCAGGGATGAAAACGAGAAACTGGAATACTATGAGAAG
TTCCAGATCATCGAAAACGTGTTTAAGCAGAAGAAAAAGCCTACACTGAAACAGATTGCTAAGGAGATCC
TGGTCAACGAAGAGGACATCAAGGGCTACCGGGTGACAAGCACTGGAAAACCAGAGTTCACCAATCTGAA
AGTGTATCACGATATTAAGGACATCACAGCACGGAAAGAAATCATTGAGAACGCCGAACTGCTGGATCAG
ATTGCTAAGATCCTGACTATCTACCAGAGCTCCGAGGACATCCAGGAAGAGCTGACTAACCTGAACAGCG
AGCTGACCCAGGAAGAGATCGAACAGATTAGTAATCTGAAGGGGTACACCGGAACACACAACCTGTCCCT
GAAAGCTATCAATCTGATTCTGGATGAGCTGTGGCATACAAACGACAATCAGATTGCAATCTTTAACCGG
CTGAAGCTGGTCCCAAAAAAGGTGGACCTGAGTCAGCAGAAAGAGATCCCAACCACACTGGTGGACGATT
TCATTCTGTCACCCGTGGTCAAGCGGAGCTTCATCCAGAGCATCAAAGTGATCAACGCCATCATCAAGAA
GTACGGCCTGCCCAATGATATCATTATCGAGCTGGCTAGGGAGAAGAACAGCAAGGACGCACAGAAGATG
ATCAATGAGATGCAGAAACGAAACCGGCAGACCAATGAACGCATTGAAGAGATTATCCGAACTACCGGGA
AAGAGAACGCAAAGTACCTGATTGAAAAAATCAAGCTGCACGATATGCAGGAGGGAAAGTGTCTGTATTC
TCTGGAGGCCATCCCCCTGGAGGACCTGCTGAACAATCCATTCAACTACGAGGTCGATCATATTATCCCC
AGAAGCGTGTCCTTCGACAATTCCTTTAACAACAAGGTGCTGGTCAAGCAGGAAGAGAACTCTAAAAAGG
GCAATAGGACTCCTTTCCAGTACCTGTCTAGTTCAGATTCCAAGATCTCTTACGAAACCTTTAAAAAGCA
CATTCTGAATCTGGCCAAAGGAAAGGGCCGCATCAGCAAGACCAAAAAGGAGTACCTGCTGGAAGAGCGG
GACATCAACAGATTCTCCGTCCAGAAGGATTTTATTAACCGGAATCTGGTGGACACAAGATACGCTACTC
GCGGCCTGATGAATCTGCTGCGATCCTATTTCCGGGTGAACAATCTGGATGTGAAAGTCAAGTCCATCAA
CGGCGGGTTCACATCTTTTCTGAGGCGCAAATGGAAGTTTAAAAAGGAGCGCAACAAAGGGTACAAGCAC
CATGCCGAAGATGCTCTGATTATCGCAAATGCCGACTTCATCTTTAAGGAGTGGAAAAAGCTGGACAAAG
CCAAGAAAGTGATGGAGAACCAGATGTTCGAAGAGAAGCAGGCCGAATCTATGCCCGAAATCGAGACAGA
ACAGGAGTACAAGGAGATTTTCATCACTCCTCACCAGATCAAGCATATCAAGGATTTCAAGGACTACAAG
TACTCTCACCGGGTGGATAAAAAGCCCAACAGAGAGCTGATCAATGACACCCTGTATAGTACAAGAAAAG
ACGATAAGGGGAATACCCTGATTGTGAACAATCTGAACGGACTGTACGACAAAGATAATGACAAGCTGAA
AAAGCTGATCAACAAAAGTCCCGAGAAGCTGCTGATGTACCACCATGATCCTCAGACATATCAGAAACTG
AAGCTGATTATGGAGCAGTACGGCGACGAGAAGAACCCACTGTATAAGTACTATGAAGAGACTGGGAACT
ACCTGACCAAGTATAGCAAAAAGGATAATGGCCCCGTGATCAAGAAGATCAAGTACTATGGGAACAAGCT
GAATGCCCATCTGGACATCACAGACGATTACCCTAACAGTCGCAACAAGGTGGTCAAGCTGTCACTGAAG
CCATACAGATTCGATGTCTATCTGGACAACGGCGTGTATAAATTTGTGACTGTCAAGAATCTGGATGTCA
TCAAAAAGGAGAACTACTATGAAGTGAATAGCAAGTGCTACGAAGAGGCTAAAAAGCTGAAAAAGATTAG
CAACCAGGCAGAGTTCATCGCCTCCTTTTACAACAACGACCTGATTAAGATCAATGGCGAACTGTATAGG

GTCATCGGGGTGAACAATGATCTGCTGAACCGCATTGAAGTGAATATGATTGACATCACTTACCGAGAGT
ATCTGGAAAACATGAATGATAAGCGCCCCCCTCGAATTATCAAAACAATTGCCTCTAAGACTCAGAGTAT
CAAAAAGTACTCAACCGACATTCTGGGAAACCTGTATGAGGTGAAGAGCAAAAAGCACCCTCAGATTATC
AAAAAGGGC

If any of the above Cas9 sequences are fused with a peptide or polypeptide at the C-terminus, it is understood that the stop codon will be removed.

### Other Cas Molecules and Cas Polypeptides

Various types of Cas molecules or Cas polypeptides can be used to practice the inventions disclosed herein. In some embodiments, Cas molecules of Type II Cas systems are used. In other embodiments, Cas molecules of other Cas systems are used. For example, Type I or Type III Cas molecules may be used. Exemplary Cas molecules (and Cas systems) are described, e.g., in Haft et al., PLoS Computational Biology 2005, 1(6): e60 and Makarova et al., Nature Review Microbiology 2011, 9:467-477, the contents of both references are incorporated herein by reference in their entirety. Exemplary Cas molecules (and Cas systems) are also shown in **Table 600.**

**Table 600. Cas Systems**

| **Gene name**^{‡} | **System type or subtype** | **Name from Haft *et al.***^{§} | **Structure of encoded protein (PDB accessions)**^{¶} | **Families (and superfamily) of encoded protein^{#}**** | **Representatives** |
|---|---|---|---|---|---|
| *cas1* | • Type I | *cas1* | 3GOD, 3LFX and 2YZS | COG1518 | SERP2463, SPy1047 and *ygbT* |
| | • Type II | | | | |
| | • Type III | | | | |
| *cas2* | • Type I | *cas2* | 2IVY, 2I8E and 3EXC | COG1343 and COG3512 | SERP2462, SPy1048, SPy1723 (N-terminal domain) and *ygbF* |
| | • Type II | | | | |
| | • Type III | | | | |
| *cas3'* | • Type I^{‡‡} | *cas3* | NA | COG1203 | APE1232 and *ygcB* |
| *cas3"* | • Subtype I-A | NA | NA | COG2254 | APE1231 and BH0336 |
| | • Subtype I-B | | | | |
| *cas4* | • Subtype I-A | *cas4* and *csa1* | NA | COG1468 | APE1239 and BH0340 |
| | • Subtype I-B | | | | |
| | • Subtype I-C | | | | |
| | • Subtype I-D | | | | |
| | • Subtype II-B | | | | |
| *cas5* | • Subtype I-A | *cas5a*, *cas5d*, *cas5e*, *cas5h*, *cas5p*, *cas5t* and *cmx5* | 3KG4 | COG1688 | APE1234, BH0337, *devS* and *ygcI* |
| | • Subtype I-B | | | (RAMP) | |
| | • Subtype I-C | | | | |
| | • Subtype I-E | | | | |
| *cas6* | • Subtype I-A | *cas6* and *cmx6* | 3I4H | COG1583 and COG5551 (RAMP) | PF1131 and slr7014 |
| | • Subtype I-B | | | | |
| | • Subtype I-D | | | | |
| | • Subtype III-A | | | | |
| | • Subtype III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | y1727 |
| *cas7* | • Subtype I-A | *csa2*, *csd2*, *cse4*, *csh2*, *csp1* and *cst2* | NA | COG1857 and COG3649 (RAMP) | *devR* and *ygcJ* |
| | • Subtype I-B | | | | |
| | • Subtype I-C | | | | |
| | • Subtype I-E | | | | |
| *cas8a1* | • Subtype I-A^{‡‡} | *cmx1*, *cst1*, *csx8*, *csx13* and CXXC-CXXC | NA | BH0338-like | LA3191^{§§} and PG2018^{§§} |
| *cas8a2* | • Subtype I-A^{‡‡} | *csa4* and *csx9* | NA | PH0918 | AF0070, AF1873, MJ0385, PF0637, PH0918 and SSO1401 |
| *cas8b* | • Subtype 1-B^{‡‡} | *csh1* and TM1802 | NA | BH0338-like | MTH1090 and TM1802 |
| *cas8c* | • Subtype I-C^{‡‡} | *csd1* and *csp2* | NA | BH0338-like | BH0338 |
| *cas9* | • Type II^{‡‡} | *csn1* and *csx12* | NA | COG3513 | FTN_0757 and SPy1046 |
| *cas10* | • Type III^{‡‡} | *cmr2*, *csm1* and *csx11* | NA | COG1353 | MTH326, Rv2823c^{§§} and TM1794^{§§} |
| *cas10d* | • Subtype I-D^{‡‡} | *csc3* | NA | COG1353 | slr7011 |
| *csy1* | • Subtype I-F^{‡‡} | *csy1* | NA | y1724-like | y1724 |
| *csy2* | • Subtype I-F | *csy2* | NA | (RAMP) | y1725 |
| *csy3* | • Subtype I-F | *csy3* | NA | (RAMP) | y1726 |
| *cse1* | • Subtype 1-E^{‡‡} | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SSO1398 |
| *csn2* | • Subtype II-A | *csn2* | NA | SPy1049-like | SPy1049 |
| *csm2* | • Subtype III-A^{‡‡} | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B^{‡‡} | *cmr5* | 2ZOP and 2OEB | COG3337 | MTH324 and PF1125 |
| *cmr6* | • Subtype III-B | *cmr6* | NA | COG1604 (RAMP) | PF1124 |
| *csb1* | • Subtype I-U | GSU0053 | NA | (RAMP) | Balac_1306 and GSU0053 |
| *csb2* | • Subtype I-U^{§§} | NA | NA | (RAMP) | Balac_1305 and GSU0054 |
| *csb3* | • Subtype I-U | NA | NA | (RAMP) | Balac_1303^{§§} |
| *csx17* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3*, *csx1*, *csx2*, DXTHG, NE0113 and TIGR02710 | 1XMX and 2171 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE_1037 |

### IV. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule are described, e.g., in Jinek et al., SCIENCE 2012, 337(6096):816-821.

### Binding and Cleavage Assay: Testing the endonuclease activity of Cas9 molecule

The ability of a Cas9 molecule/gRNA molecule complex to bind to and cleave a target nucleic acid can be evaluated in a plasmid cleavage assay. In this assay, synthetic or *in vitro*-transcribed gRNA molecule is pre-annealed prior to the reaction by heating to 95°C and slowly cooling down to room temperature. Native or restriction digest-linearized plasmid DNA (300 ng (∼8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mM KCl, 0.5 mM DTT, 0.1 mM EDTA) with or without 10 mM MgCl₂. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA molecule complex to bind to and cleave a target nucleic acid can be evaluated in an oligonucleotide DNA cleavage assay. In this assay, DNA oligonucleotides (10 pmol) are radiolabeled by incubating with 5 units T4 polynucleotide kinase and ∼3-6 pmol (∼20-40 mCi) [γ-32P]-ATP in IX T4 polynucleotide kinase reaction buffer at 37°C for 30 min, in a 50 µL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA molecules are annealed by heating to 95°C for 30 s, followed by slow cooling to room temperature. Cas9 (500 nM final concentration) is pre-incubated with the annealed gRNA molecules (500 nM) in cleavage assay buffer (20 mM HEPES pH 7.5, 100 mM KCl, 5 mM MgC12, 1 mM DTT, 5% glycerol) in a total volume of 9 µl. Reactions are initiated by the addition of 1 µl target DNA (10 nM) and incubated for 1 h at 37°C. Reactions are quenched by the addition of 20 µl of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA molecule or candidate Cas9 molecule.

### Binding Assay: Testing the binding of Cas9 molecule to target DNA

Exemplary methods for evaluating the binding of Cas9 molecule to target DNA arc described, e.g., in Jinek et al., SCIENCE 2012; 337(6096):816-821.

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing IX TBE. DNA bands are visualized by UV shadowing, excised, and eluted by soaking gel pieces in DEPC-treated H₂O. Eluted DNA is ethanol precipitated and dissolved in DEPC-treated H₂O. DNA samples are 5' end labeled with [γ-32P]-ATP using T4 polynucleotide kinase for 30 min at 37°C. Polynucleotide kinase is heat denatured at 65°C for 20 min, and unincorporated radiolabel is removed using a column. Binding assays are performed in buffer containing 20 mM HEPES pH 7.5, 100 mM KCl, 5 mM MgCl₂, 1 mM DTT and 10% glycerol in a total volume of 10 µl. Cas9 protein molecule is programmed with equimolar amounts of pre-annealed gRNA molecule and titrated from 100 pM to 1 µM. Radiolabeled DNA is added to a final concentration of 20 pM. Samples are incubated for 1 h at 37°C and resolved at 4°C on an 8% native polyacrylamide gel containing IX TBE and 5 mM MgCl₂. Gels are dried and DNA visualized by phosphorimaging.

### Differential Scanning Flourimetry (DSF)

The thermostability of Cas9-gRNA ribonucleoprotein (RNP) complexes can be measured via DSF. This technique measures the thermostability of a protein, which can increase under favorable conditions such as the addition of a binding RNA molecule, e.g., a gRNA.

The assay is performed using two different protocols, one to test the best stoichiometric ratio of gRNA:Cas9 protein and another to determine the best solution conditions for RNP formation.

To determine the best solution to form RNP complexes, a 2uM solution of Cas9 in water+10x SYPRO Orange® (Life Techonologies cat#S-6650) and dispensed into a 384 well plate. An equimolar amount of gRNA diluted in solutions with varied pH and salt is then added. After incubating at room temperature for 10'and brief centrifugation to remove any bubbles,a Bio-Rad CFX384™ Real-Time System C1000 Touch™ Thermal Cycler with the Bio-Rad CFX Manager software is used to run a gradient from 20°C to 90°C with a 1° increase in temperature every 10seconds.

The second assay consists of mixing various concentrations of gRNA with 2uM Cas9 in optimal buffer from assay 1 above and incubating at RT for 10' in a 384 well plate. An equal volume of optimal buffer + 10x SYPRO Orange® (Life Techonologies cat#S-6650) is added and the plate sealed with Microseal® B adhesive (MSB-1001). Following brief centrifugation to remove any bubbles, a Bio-Rad CFX384™ Real-Time System C1000 Touch™ Thermal Cycler with the Bio-Rad CFX Manager software is used to run a gradient from 20°C to 90°C with a 1° increase in temperature every 10seconds.

### V. Genome Editing Approaches

In general, it is to be understood that the alteration of any gene according to the methods described herein can be mediated by any mechanism and that any methods are not limited to a particular mechanism. Exemplary mechanisms that can be associated with the alteration of a gene include, but are not limited to, non-homologous end joining (e.g., classical or alternative), microhomology-mediated end joining (MMEJ), homology-directed repair (e.g., endogenous donor template mediated), SDSA (synthesis dependent strand annealing), single strand annealing or single strand invasion. Described herein are exemplary methods for targeted knockout of one or both alleles of the FAS, BID, CTLA4, PDCD1, CBLB or, PTPN66, TRAC or TRBC gene using NHEJ (see Section V.1). In another embodiment, exemplary methods arc provided for targeted knockdown of the FAS, BID, CTLA4, PDCD1, CBLB, or PTPN6 gene (see Section V.2). It is further contemplated that the disclosed methods may target two or more of the FAS, BID, CTLA4, PDCD1, CBLB, or PTPN6 genes for knockdown or knockout or a combination thereof.

### V.1 NHEJ Approaches for Gene Targeting

As described herein, nuclease-induced non-homologous end-joining (NHEJ) can be used to target gene-specific knockouts. Nuclease-induced NHEJ can also be used to remove (e.g., delete) sequence insertions in a gene of interest.

While not wishing to be bound by theory, it is believed that, in an embodiment, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; most commonly in the 1-50 bp range, but they can easily reach greater than 100-200 bp. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double strand cleaving eaCas9 molecules and single strand, or nickase, eaCas9 molecules can be used in the methods and compositions described herein to generate NHEJ-mediated indels. NHEJ-mediated indels targeted to the gene, e.g., a coding region, e.g., an early coding region of a gene, of interest can be used to knockout (i.e., eliminate expression of) a gene of interest. For example, early coding region of a gene of interest includes sequence immediately following a transcription start site, within a first exon of the coding sequence, or within 500 bp of the transcription start site (e.g., less than 500, 450, 400, 350, 300, 250, 200, 150, 100 or 50 bp).

In an embodiment, NHEJ-mediated indels are introduced into one or more T-cell expressed genes. In an embodiment, in which a Cas9 double-stranded nuclease or a Cas9 single-stranded nickase is used to introduce mutations into two T-cell expressed genes, e.g., any two of *FAS, BID, CTLA4, PDCD1, CBLB, PTPN6, TRAC* or *TRBC* genes, individual gRNAs or gRNA pairs targeting both genes are provided together with the Cas9 double-stranded nuclease or single-stranded nickase. In an embodiment, in which a Cas9 double-stranded nuclease or a Cas9 single-stranded nickase is used to introduce mutations into three T-cell expressed genes, e.g., any three of *FAS, BID, CTLA4, PDCD1, CBLB, PTPN6, TRAC* or *TRBC* genes, individual gRNAs or gRNA pairs targeting all three genes are provided together with the Cas9 double-stranded nuclease or single-stranded nickase. In an embodiment, in which a Cas9 double-stranded nuclease or a Cas9 single-stranded nickase is used to introduce mutations into four T-cell expressed genes, e.g., any four of *FAS, BID, CTLA4, PDCD1, CBLB, PTPN6, TRAC* or *TRBC* genes, individual gRNAs or gRNA pairs targeting all four genes are provided together with the Cas9 double-stranded nuclease or single-stranded nickase. In an embodiment, in which a Cas9 double-stranded nuclease or a Cas9 single-stranded nickase is used to introduce mutations into five T-cell expressed genes, e.g., any five of *FAS, BID, CTLA4, PDCD1, CBLB, PTPN6, TRAC* or *TRBC* genes, individual gRNAs or gRNA pairs targeting all five genes are provided together with the Cas9 double-stranded nuclease or single-stranded nickase. In an embodiment, in which a Cas9 double-stranded nuclease or a Cas9 single-stranded nickase is used to introduce mutations into six T-cell expressed genes, e.g., any six of *FAS, BID, CTLA4, PDCD1, CBLB, PTPN6, TRAC* or *TRBC* genes, individual gRNAs or gRNA pairs targeting all six genes are provided together with the Cas9 double-stranded nuclease or single-stranded nickase. In an embodiment, in which a Cas9 double-stranded nuclease or a Cas9 single-stranded nickase is used to introduce mutations into seven T-cell expressed genes, e.g., any seven of *FAS, BID, CTLA4, PDCD1, CBLB, PTPN6, TRAC* or *TRBC* genes, individual gRNAs or gRNA pairs targeting all seven genes are provided together with the Cas9 double-stranded nuclease or single-stranded nickase. In an embodiment, in which a Cas9 double-stranded nuclease or aCas9 single-stranded nickase is used to introduce mutations into eight T-cell expressed genes, e.g., each of *FAS, BID, CTLA4, PDCD1, CBLB, PTPN6, TRAC* and *TRBC* genes, individual gRNAs or gRNA pairs targeting all eight genes are provided together with the Cas9 double-stranded nuclease or single-stranded nickase.

### Placement of double strand or single strand breaks relative to the target position

In an embodiment, in which a gRNA and Cas9 nuclease generate a double strand break for the purpose of inducing NHEJ-mediated indels, a gRNA, e.g., a unimolecular (or chimeric) or modular gRNA molecule, is configured to position one double-strand break in close proximity to a nucleotide of the target position. In an embodiment, the cleavage site is between 0-30 bp away from the target position (e.g., less than 30, 25, 20, 15, 10, 9, 8, 7, 6, 5, 4, 3, 2 or 1 bp from the target position).

In an embodiment, in which two gRNAs complexing with Cas9 nickases induce two single strand breaks for the purpose of inducing NHEJ-mediated indels, two gRNAs, e.g., independently, unimolecular (or chimeric) or modular gRNA, are configured to position two single-strand breaks to provide for NHEJ repair a nucleotide of the target position. In an embodiment, the gRNAs are configured to position cuts at the same position, or within a few nucleotides of one another, on different strands, essentially mimicking a double strand break. In an embodiment, the closer nick is between 0-30 bp away from the target position (e.g., less than 30, 25, 20, 15, 10, 9, 8, 7, 6, 5, 4, 3, 2 or 1 bp from the target position), and the two nicks are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In an embodiment, the gRNAs are configured to place a single strand break on either side of a nucleotide of the target position.

Both double strand cleaving eaCas9 molecules and single strand, or nickase, eaCas9 molecules can be used in the methods and compositions described herein to generate breaks both sides of a target position. Double strand or paired single strand breaks may be generated on both sides of a target position to remove the nucleic acid sequence between the two cuts (e.g., the region between the two breaks in deleted). In an embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

### V.2 Targeted Knockdown

Unlike CRISPR/Cas-mediated gene knockout, which permanently eliminates or reduces expression by mutating the gene at the DNA level, CRISPR/Cas knockdown allows for temporary reduction of gene expression through the use of artificial transcription factors. Mutating key residues in both DNA cleavage domains of the Cas9 protein (e.g., the D10A and H840A mutations) results in the generation of a catalytically inactive Cas9 (eiCas9 which is also known as dead Cas9 or dCas9). A catalytically inactive Cas9 complexes with a gRNA and localizes to the DNA sequence specified by that gRNA's targeting domain, however, it does not cleave the target DNA. Fusion of the dCas9 to an effector domain, e.g., a transcription repression domain, enables recruitment of the effector to any DNA site specified by the gRNA. While it has been shown that the eiCas9 itself can block transcription when recruited to early regions in the coding sequence, more robust repression can be achieved by fusing a transcriptional repression domain (for example KRAB, SID or ERD) to the Cas9 and recruiting it to the promoter region of a gene. It is likely that targeting DNAseI hypersensitive regions of the promoter may yield more efficient gene repression or activation because these regions are more likely to be accessible to the Cas9 protein and are also more likely to harbor sites for endogenous transcription factors. Especially for gene repression, it is contemplated herein that blocking the binding site of an endogenous transcription factor would aid in downregulating gene expression. In another embodiment, an eiCas9 can be fused to a chromatin modifying protein. Altering chromatin status can result in decreased expression of the target gene.

In an embodiment, a gRNA molecule can be targeted to a known transcription response elements (e.g., promoters, enhancers, etc.), a known upstream activating sequences (UAS), and/or sequences of unknown or known function that are suspected of being able to control expression of the target DNA.

CRISPR/Cas-mcdiatcd gene knockdown can be used to reduce expression of an unwanted allele or transcript. Contemplated herein are scenarios wherein permanent destruction of the gene is not ideal. In these scenarios, site-specific repression may be used to temporarily reduce or eliminate expression. It is also contemplated herein that the off-target effects of a Cas-repressor may be less severe than those of a Cas-nuclease as a nuclease can cleave any DNA sequence and cause mutations whereas a Cas-repressor may only have an effect if it targets the promoter region of an actively transcribed gene. However, while nuclease-mediated knockout is permanent, repression may only persist as long as the Cas-repressor is present in the cells. Once the repressor is no longer present, it is likely that endogenous transcription factors and gene regulatory elements would restore expression to its natural state.

In an embodiment, CRISPR/Cas-mediated gene knockdown can be used to reduce expression one or more T-cell expressed genes. In an embodiment, in which a eiCas9 or an eiCas9 fusion protein described herein is used to knockdown two T-cell expressed genes, e.g., any two of *FAS, BID, CTLA4, PDCD1, CBLB,* or *PTPN6* genes, individual gRNAs or gRNA pairs targeting both genes are provided together with the eiCas9 or eiCas9 fusion protein. In an embodiment, in which a eiCas9 or eiCas9 fusion protein is used to knockdown three T-cell expressed genes, e.g., any three of *FAS, BID, CTLA4, PDCD1, CBLB,* or *PTPN6* genes, individual gRNAs or gRNA pairs targeting all three genes are provided together with the eiCas9 or eiCas9 fusion protein. In an embodiment, in which a eiCas9 or eiCas9 fusion protein is used to knockdown four T-cell expressed genes, e.g., any four of *FAS, BID, CTLA4, PDCD1, CBLB,* or *PTPN6* genes, individual gRNAs or gRNA pairs targeting all four genes are provided together with the eiCas9 or eiCas9 fusion protein. In an embodiment, in which a eiCas9 or eiCas9 fusion protein is used to knockdown five T-cell expressed genes, e.g., any five of *FAS, BID, CTLA4, PDCD1, CBLB,* or *PTPN6* genes, individual gRNAs or gRNA pairs targeting all five genes are provided together with the eiCas9 or eiCas9 fusion protein. In an embodiment, in which a eiCas9 or eiCas9 fusion protein is used to knockdown six T-cell expressed genes, e.g., each of *FAS, BID, CTLA4, PDCD1, CBLB,* or *PTPN6* genes, individual gRNAs or gRNA pairs targeting all six genes are provided together with the eiCas9 or eiCas9 fusion protein.

### V.3 Single-Strand Annealing

Single strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single strand overhangs containing the repeat sequences are coated with RPA protein to prevent the repeats sequences from inappropriate annealing, e.g., to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter or correct a target nucleic acid sequence. Instead, the complementary repeat sequence is utilized.

### V.4 Other DNA Repair Pathways

### SSBR (single strand break repair)

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott, Nature Reviews Genetics 9, 619-631 (August 2008), and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are 'damaged'. End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonculease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5'-residues. Multiple DNA polymerases, including Pol *β*, are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase b, DNA polymerase d, DNA polymerase e, PCNA, LIG1, PNK, PNKP, APE1, APTX, APLF, TDP1, LIG3, FEN1, CtIP, MRN, and ERCC1.

### MMR (mismatch repair)

Cells contain three excision repair pathways: MMR, BER, and NER. The excision repair pathways hace a common feature in that they typically recognize a lesion on one strand of the DNA, then exo/endonucleaseases remove the lesion and leave a 1-30 nucleotide gap that is sub-sequentially filled in by DNA polymerase and finally sealed with ligase. A more complete picture is given in Li, Cell Research (2008) 18:85-98, and a summary is provided here.

Mismatch repair (MMR) operates on mispaired DNA bases.

The MSH2/6 or MSH2/3 complexes both have ATPases activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutL *α* which possesses an ATPase activity and is important for multiple steps of MMR. It possesses a PCNA/replication factor C (RFC)-dependent endonuclease activity which plays an important role in 3' nick-directed MMR involving EXO1. (EXO1 is a participant in both HR and MMR.) It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol d, RPA, HMGB1, RFC, and DNA ligase I.

### Base excision repair (BER)

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott, Nature Reviews Genetics 9, 619-631 (August 2008), and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the correct nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incised the phosphodiester backbone to create a DNA single strand break (SSB). The third step of BER involves cleaning-up of the DNA ends. The fourth step in BER is conducted by Pol β that adds a new complementary nucleotide into the repair gap and in the final step XRCC1/Ligase III seals the remaining nick in the DNA backbone. This completes the short-patch BER pathway in which the majority (∼80%) of damaged DNA bases are repaired. However, if the 5' -ends in step 3 are resistant to end processing activity, following one nucleotide insertion by Pol β there is then a polymerase switch to the replicative DNA polymerases, Pol δ/ε, which then add ∼2-8 more nucleotides into the DNA repair gap. This creates a 5' -flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Polb, Pold, Pole, XRCC1, Ligase III, FEN-1, PCNA, RECQL4, WRN, MYH, PNKP, and APTX.

### Nucleotide excision repair (NER)

Nucleotide excision repair (NER) is an important excision mechanism that removes bulky helix-distorting lesions from DNA. Additional details about NER are given in Marteijn et al., Nature Reviews Molecular Cell Biology 15, 465-481 (2014), and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

### Interstrand Crosslink (ICL)

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, e.g., XPF and RAD51C, endonucleases such as RAD51, translesion polymerases, e.g., DNA polymerase zeta and Rev1), and the Fanconi anemia (FA) proteins, e.g., FancJ.

### Other pathways

Several other DNA repair pathways exist in mammals.

Translesion synthesis (TLS) is a pathway for repairing a single stranded break left after a defective replication event and involves translesion polymerases, e.g., DNA polζ and Rev1.

Error-free postreplication repair (PRR) is another pathway for repairing a single stranded break left after a defective replication event.

### V.5 Examples of gRNAs in Genome Editing Methods

gRNA molecules as described herein can be used with Cas9 molecules that generate a double strand break or a single strand break to alter the sequence of a target nucleic acid, e.g., a target position or target genetic signature. gRNA molecules useful in these methods are described below.

In an embodiment, the gRNA, e.g., a chimeric gRNA, is configured such that it comprises one or more of the following properties;
a) it can position, e.g., when targeting a Cas9 molecule that makes double strand breaks, a double strand break (i) within 50, 100, 150, 200, 250, 300, 350, 400, 450, or 500 nucleotides of a target position, or (ii) sufficiently close that the target position is within the region of end resection;
b) it has a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
c)
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain.

In an embodiment, the gRNA is configured such that it comprises properties: a and b(i).

In an embodiment, the gRNA is configured such that it comprises properties: a and b(ii).

In an embodiment, the gRNA is configured such that it comprises properties: a and b(iii).

In an embodiment, the gRNA is configured such that it comprises properties: a and b(iv).

In an embodiment, the gRNA is configured such that it comprises properties: a and b(v).

In an embodiment, the gRNA is configured such that it comprises properties: a and b(vi).

In an embodiment, the gRNA is configured such that it comprises properties: a and b(vii).

In an embodiment, the gRNA is configured such that it comprises properties: a and b(viii).

In an embodiment, the gRNA is configured such that it comprises properties: a and b(ix).

In an embodiment, the gRNA is configured such that it comprises properties: a and b(x).

In an embodiment, the gRNA is configured such that it comprises properties: a and b(xi).

In an embodiment, the gRNA is configured such that it comprises properties: a and c.

In an embodiment, the gRNA is configured such that in comprises properties: a, b, and c.

In an embodiment, the gRNA is configured such that in comprises properties: a(i), b(i), and c(i).

In an embodiment, the gRNA is configured such that in comprises properties: a(i), b(i), and c(ii).

In an embodiment, the gRNA is configured such that in comprises properties: a(i), b(ii), and c(i).

In an embodiment, the gRNA is configured such that in comprises properties: a(i), b(ii), and c(ii).

In an embodiment, the gRNA is configured such that in comprises properties: a(i), b(iii), and c(i).

In an embodiment, the gRNA is configured such that in comprises properties: a(i), b(iii), and c(ii).

In an embodiment, the gRNA is configured such that in comprises properties: a(i), b(iv), and c(i).

In an embodiment, the gRNA is configured such that in comprises properties: a(i), b(iv), and c(ii).

In an embodiment, the gRNA is configured such that in comprises properties: a(i), b(v), and c(i).

In an embodiment, the gRNA is configured such that in comprises properties: a(i), b(v), and c(ii).

In an embodiment, the gRNA is configured such that in comprises properties: a(i), b(vi), and c(i).

In an embodiment, the gRNA is configured such that in comprises properties: a(i), b(vi), and c(ii).

In an embodiment, the gRNA is configured such that in comprises properties: a(i), b(vii), and c(i).

In an embodiment, the gRNA is configured such that in comprises properties: a(i), b(vii), and c(ii).

In an embodiment, the gRNA is configured such that in comprises properties: a(i), b(viii), and c(i).

In an embodiment, the gRNA is configured such that in comprises properties: a(i), b(viii), and c(ii).

In an embodiment, the gRNA is configured such that in comprises properties: a(i), b(ix), and c(i).

In an embodiment, the gRNA is configured such that in comprises properties: a(i), b(ix), and c(ii).

In an embodiment, the gRNA is configured such that in comprises properties: a(i), b(x), and c(i).

In an embodiment, the gRNA is configured such that in comprises properties: a(i), b(x), and c(ii).

In an embodiment, the gRNA is configured such that in comprises properties: a(i), b(xi), and c(i).

In an embodiment, the gRNA is configured such that in comprises properties: a(i), b(xi), and c(ii).

In an embodiment, the gRNA, e.g., a chimeric gRNA, is configured such that it comprises one or more of the following properties;
a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
c)
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain.

In an embodiment, the gRNA is configured such that it comprises properties: a and b(i).

In an embodiment, the gRNA is configured such that it comprises properties: a and b(ii).

In an embodiment, the gRNA is configured such that it comprises properties: a and b(iii).

In an embodiment, the gRNA is configured such that it comprises properties: a and b(iv).

In an embodiment, the gRNA is configured such that it comprises properties: a and b(v).

In an embodiment, the gRNA is configured such that it comprises properties: a and b(vi).

In an embodiment, the gRNA is configured such that it comprises properties: a and b(vii).

In an embodiment, the gRNA is configured such that it comprises properties: a and b(viii).

In an embodiment, the gRNA is configured such that it comprises properties: a and b(ix).

In an embodiment, the gRNA is configured such that it comprises properties: a and b(x).

In an embodiment, the gRNA is configured such that it comprises properties: a and b(xi).

In an embodiment, the gRNA is configured such that it comprises properties: a and c.

In an embodiment, the gRNA is configured such that in comprises properties: a, b, and c.

In an embodiment, the gRNA is configured such that in comprises properties: a(i), b(i), and c(i).

In an embodiment, the gRNA is configured such that in comprises properties: a(i), b(i), and c(ii).

In an embodiment, the gRNA is configured such that in comprises properties: a(i), b(ii), and c(i).

In an embodiment, the gRNA is configured such that in comprises properties: a(i), b(ii), and c(ii).

In an embodiment, the gRNA is configured such that in comprises properties: a(i), b(iii), and c(i).

In an embodiment, the gRNA is configured such that in comprises properties: a(i), b(iii), and c(ii).

In an embodiment, the gRNA is configured such that in comprises properties: a(i), b(iv), and c(i).

In an embodiment, the gRNA is configured such that in comprises properties: a(i), b(iv), and c(ii).

In an embodiment, the gRNA is configured such that in comprises properties: a(i), b(v), and c(i).

In an embodiment, the gRNA is configured such that in comprises properties: a(i), b(v), and c(ii).

In an embodiment, the gRNA is configured such that in comprises properties: a(i), b(vi), and c(i).

In an embodiment, the gRNA is configured such that in comprises properties: a(i), b(vi), and c(ii).

In an embodiment, the gRNA is configured such that in comprises properties: a(i), b(vii), and c(i).

In an embodiment, the gRNA is configured such that in comprises properties: a(i), b(vii), and c(ii).

In an embodiment, the gRNA is configured such that in comprises properties: a(i), b(viii), and c(i).

In an embodiment, the gRNA is configured such that in comprises properties: a(i), b(viii), and c(ii).

In an embodiment, the gRNA is configured such that in comprises properties: a(i), b(ix), and c(i).

In an embodiment, the gRNA is configured such that in comprises properties: a(i), b(ix), and c(ii).

In an embodiment, the gRNA is configured such that in comprises properties: a(i), b(x), and c(i).

In an embodiment, the gRNA is configured such that in comprises properties: a(i), b(x), and c(ii).

In an embodiment, the gRNA is configured such that in comprises properties: a(i), b(xi), and c(i).

In an embodiment, the gRNA is configured such that in comprises properties: a(i), b(xi), and c(ii).

In an embodiment, the gRNA is used with a Cas9 nickase molecule having HNH activity, e.g., a Cas9 molecule having the RuvC activity inactivated, e.g., a Cas9 molecule having a mutation at D10, e.g., the D10A mutation.

In an embodiment, the gRNA is used with a Cas9 nickase molecule having RuvC activity, e.g., a Cas9 molecule having the HNH activity inactivated, e.g., a Cas9 molecule having a mutation at H840, e.g., a H840A.

In an embodiment, a pair of gRNAs, e.g., a pair of chimeric gRNAs, comprising a first and a second gRNA, is configured such that they comprises one or more of the following properties;
a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides;
c) for one or both:
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes*, *S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain; or, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain;
d) the gRNAs are configured such that, when hybridized to target nucleic acid, they are separated by 0-50, 0-100, 0-200, at least 10, at least 20, at least 30 or at least 50 nucleotides;
e) the breaks made by the first gRNA and second gRNA arc on different strands; and
f) the PAMs are facing outwards.

In an embodiment, one or both of the gRNAs is configured such that it comprises properties: a and b(i).

In an embodiment, one or both of the gRNAs is configured such that it comprises properties: a and b(ii).

In an embodiment, one or both of the gRNAs is configured such that it comprises properties: a and b(iii).

In an embodiment, one or both of the gRNAs is configured such that it comprises properties: a and b(iv).

In an embodiment, one or both of the gRNAs is configured such that it comprises properties: a and b(v).

In an embodiment, one or both of the gRNAs is configured such that it comprises properties: a and b(vi).

In an embodiment, one or both of the gRNAs is configured such that it comprises properties: a and b(vii).

In an embodiment, one or both of the gRNAs is configured such that it comprises properties: a and b(viii).

In an embodiment, one or both of the gRNAs is configured such that it comprises properties: a and b(ix).

In an embodiment, one or both of the gRNAs is configured such that it comprises properties: a and b(x).

In an embodiment, one or both of the gRNAs is configured such that it comprises properties: a and b(xi).

In an embodiment, one or both of the gRNAs configured such that it comprises properties: a and c.

In an embodiment, one or both of the gRNAs is configured such that it comprises properties: a, b, and c.

In an embodiment, one or both of the gRNAs is configured such that it comprises properties: a(i), b(i), and c(i).

In an embodiment, one or both of the gRNAs is configured such that it comprises properties: a(i), b(i), and c(ii).

In an embodiment, one or both of the gRNAs is configured such that it comprises properties: a(i), b(i), c, and d.

In an embodiment, one or both of the gRNAs is configured such that it comprises properties: a(i), b(i), c, and e.

In an embodiment, one or both of the gRNAs is configured such that it comprises properties: a(i), b(i), c, d, and e.

In an embodiment, one or both of the gRNAs is configured such that it comprises properties: a(i), b(ii), and c(i).

In an embodiment, one or both of the gRNAs is configured such that it comprises properties: a(i), b(ii), and c(ii).

In an embodiment, one or both of the gRNAs is configured such that it comprises properties: a(i), b(ii), c, and d.

In an embodiment, one or both of the gRNAs is configured such that it comprises properties: a(i), b(ii), c, and e.

In an embodiment, one or both of the gRNAs is configured such that it comprises properties: a(i), b(ii), c, d, and e.

In an embodiment, one or both of the gRNAs is configured such that it comprises properties: a(i), b(iii), and c(i).

In an embodiment, one or both of the gRNAs is configured such that it comprises properties: a(i), b(iii), and c(ii).

In an embodiment, one or both of the gRNAs is configured such that it comprises properties: a(i), b(iii), c, and d.

In an embodiment, one or both of the gRNAs is configured such that it comprises properties: a(i), b(iii), c, and e.

In an embodiment, one or both of the gRNAs is configured such that it comprises properties: a(i), b(iii), c, d, and e.

In an embodiment, one or both of the gRNAs is configured such that it comprises properties: a(i), b(iv), and c(i).

In an embodiment, one or both of the gRNAs is configured such that it comprises properties: a(i), b(iv), and c(ii).

In an embodiment, one or both of the gRNAs is configured such that it comprises properties: a(i), b(iv), c, and d.

In an embodiment, one or both of the gRNAs is configured such that it comprises properties: a(i), b(iv), c, and e.

In an embodiment, one or both of the gRNAs is configured such that it comprises properties: a(i), b(iv), c, d, and e.

In an embodiment, one or both of the gRNAs is configured such that it comprises properties: a(i), b(v), and c(i).

In an embodiment, one or both of the gRNAs is configured such that it comprises properties: a(i), b(v), and c(ii).

In an embodiment, one or both of the gRNAs is configured such that it comprises properties: a(i), b(v), c, and d.

In an embodiment, one or both of the gRNAs is configured such that it comprises properties: a(i), b(v), c, and e.

In an embodiment, one or both of the gRNAs is configured such that it comprises properties: a(i), b(v), c, d, and e.

In an embodiment, one or both of the gRNAs is configured such that it comprises properties: a(i), b(vi), and c(i).

In an embodiment, one or both of the gRNAs is configured such that it comprises properties: a(i), b(vi), and c(ii).

In an embodiment, one or both of the gRNAs is configured such that it comprises properties: a(i), b(vi), c, and d.

In an embodiment, one or both of the gRNAs is configured such that it comprises properties: a(i), b(vi), c, and e.

In an embodiment, one or both of the gRNAs is configured such that it comprises properties: a(i), b(vi), c, d, and e.

In an embodiment, one or both of the gRNAs is configured such that it comprises properties: a(i), b(vii), and c(i).

In an embodiment, one or both of the gRNAs is configured such that it comprises properties: a(i), b(vii), and c(ii).

In an embodiment, one or both of the gRNAs is configured such that it comprises properties: a(i), b(vii), c, and d.

In an embodiment, one or both of the gRNAs is configured such that it comprises properties: a(i), b(vii), c, and e.

In an embodiment, one or both of the gRNAs is configured such that it comprises properties: a(i), b(vii), c, d, and e.

In an embodiment, one or both of the gRNAs is configured such that it comprises properties: a(i), b(viii), and c(i).

In an embodiment, one or both of the gRNAs is configured such that it comprises properties: a(i), b(viii), and c(ii).

In an embodiment, one or both of the gRNAs is configured such that it comprises properties: a(i), b(viii), c, and d.

In an embodiment, one or both of the gRNAs is configured such that it comprises properties: a(i), b(viii), c, and e.

In an embodiment, one or both of the gRNAs is configured such that it comprises properties: a(i), b(viii), c, d, and e.

In an embodiment, one or both of the gRNAs is configured such that it comprises properties: a(i), b(ix), and c(i).

In an embodiment, one or both of the gRNAs is configured such that it comprises properties: a(i), b(ix), and c(ii).

In an embodiment, one or both of the gRNAs is configured such that it comprises properties: a(i), b(ix), c, and d.

In an embodiment, one or both of the gRNAs is configured such that it comprises properties: a(i), b(ix), c, and e.

In an embodiment, one or both of the gRNAs is configured such that it comprises properties: a(i), b(ix), c, d, and e.

In an embodiment, one or both of the gRNAs is configured such that it comprises properties: a(i), b(x), and c(i).

In an embodiment, one or both of the gRNAs is configured such that it comprises properties: a(i), b(x), and c(ii).

In an embodiment, one or both of the gRNAs is configured such that it comprises properties: a(i), b(x), c, and d.

In an embodiment, one or both of the gRNAs is configured such that it comprises properties: a(i), b(x), c, and e.

In an embodiment, one or both of the gRNAs is configured such that it comprises properties: a(i), b(x), c, d, and c.

In an embodiment, one or both of the gRNAs is configured such that it comprises properties: a(i), b(xi), and c(i).

In an embodiment, one or both of the gRNAs is configured such that it comprises properties: a(i), b(xi), and c(ii).

In an embodiment, one or both of the gRNAs is configured such that it comprises properties: a(i), b(xi), c, and d.

In an embodiment, one or both of the gRNAs is configured such that it comprises properties: a(i), b(xi), c, and e.

In an embodiment, one or both of the gRNAs is configured such that it comprises properties: a(i), b(xi), c, d, and e.

In an embodiment, the gRNAs are used with a Cas9 nickase molecule having HNH activity, e.g., a Cas9 molecule having the RuvC activity inactivated, e.g., a Cas9 molecule having a mutation at D10, e.g., the D10A mutation.

In an embodiment, the gRNAs are used with a Cas9 nickase molecule having RuvC activity, e.g., a Cas9 molecule having the HNH activity inactivated, e.g., a Cas9 molecule having a mutation at H840, e.g., a H840A. In an embodiment, the gRNAs are used with a Cas9 nickase molecule having RuvC activity, e.g., a Cas9 molecule having the HNH activity inactivated, e.g., a Cas9 molecule having a mutation at N863, e.g., N863A.

### VI. Target Cells

Cas9 molecules and gRNA molecules, e.g., a Cas9 molecule/gRNA molecule complex, can be used to manipulate a cell, e.g., to edit a target nucleic acid, in a wide variety of cells.

In an embodiment, a cell is manipulated by editing (e.g., inducing a mutation in) one or more target genes, e.g., as described herein. In some embodiments, the expression of one or more target genes (e.g., *FAS, BID, CTLA4, PDCD1, CBLB, PTPN6, TRAC* or *TRBC* gene) is modulated. In another embodiment, a cell is manipulated *ex vivo* by editing (e.g., inducing a mutation in) one or more target genes and/or modulating the expression of one or more target genes, e.g., *FAS, BID, CTLA4, PDCD1, CBLB, PTPN6, TRAC* or *TRBC* gene, and administered to a subject. Sources of target cells for *ex vivo* manipulation may include, e.g., the subject's blood, the subject's cord blood, or the subject's bone marrow. Sources of target cells for *ex vivo* manipulation may also include, e.g., heterologous donor blood, cord blood, or bone marrow.

The Cas9 and gRNA molecules described herein can be delivered to a target cell. In an embodiment, the target cell is a T cell, e.g., a CD8+ T cell (e.g., a CD8+ naive T cell, central memory T cell, or effector memory T cell), a CD4+ T cell, a natural killer T cell (NKT cells), a regulatory T cell (Treg), a stem cell memory T cell, a lymphoid progenitor cell a hematopoietic stem cell, a natural killer cell (NK cell) or a dendritic cell. In an embodiment, the target cell is an induced pluripotent stem (iPS) cell or a cell derived from an iPS cell, e.g., an iPS cell generated from a subject, manipulated to alter (e.g., induce a mutation in) or manipulate the expression of one or more target genes, e.g., *FAS, BID, CTLA4, PDCD1, CBLB, PTPN6, TRAC* or *TRBC* gene, and differentiated into, e.g., a T cell, e.g., a CD8+ T cell (e.g., a CD8+ naïve T cell, central memory T cell, or effector memory T cell), a CD4+ T cell, a stem cell memory T cell, a lymphoid progenitor cell or a hematopoietic stem cell.

In an embodiment, the target cell has been altered to contain specific T cell receptor (TCR) genes (e.g., a *TRAC* and *TRBC* gene). In another embodiment, the TCR has binding specificity for a tumor associated antigen, e.g., carcinoembryonic antigen (CEA), GP100, melanoma antigen recognized by T cells 1 (MARTI), melanoma antigen A3 (MAGEA3), NYESO1 orp53.

In an embodiment, the target cell has been altered to contain a specific chimeric antigen receptor (CAR). In an embodiment, the CAR has binding specificity for a tumor associated antigen, e.g., CD19, CD20, carbonic anhydrase IX (CAIX), CD171, CEA, ERBB2, GD2, alpha-folate receptor, Lewis Y antigen, prostate specific membrane antigen (PSMA) or tumor associated glycoprotein 72 (TAG72).

In another embodiment, the target cell has been altered to bind one or more of the following tumor antigens, e.g., by a TCR or a CAR. Tumor antigens may include, but are not limited to, AD034, AKT1, BRAP, CAGE, CDX2, CLP, CT-7, CT8/HOM-TES-85, cTAGE-1, Fibulin-1, HAGE, HCA587/MAGE-C2, hCAP-G, HCE661, HER2/neu, HLA-Cw, HOM-HD-21/Galectin9, HOM-MEEL-40/SSX2, HOM-RCC-3.1.3/CAXII, HOXA7, HOXB6, Hu, HUB1, KM-HN-3, KM-KN-1, KOC1, KOC2, KOC3, KOC3, LAGE-1, MAGE-1, MAGE-4a, MPP11, MSLN, NNP-1, NY-BR-1, NY-BR-62, NY-BR-85, NY-CO-37, NY-CO-38, NY-ESO-1, NY-ESO-5, NY-LU-12, NY-REN-10, NY-REN-19/LKB/STK11, NY-REN-21, NY-REN-26/BCR, NY-REN-3/NY-CO-38, NY-REN-33/SNC6, NY-REN-43, NY-REN-65, NY-REN-9, NY-SAR-35, OGFr, PLU-1, Rab38, RBPJkappa, RHAMM, SCP1, SCP-1, SSX3, SSX4, SSX5, TOP2A, TOP2B, or Tyrosinase.

### VII. Ex Vivo Delivery of Components to Target Cells

The components, e.g., a Cas9 molecule and gRNA molecule can be introduced into target cells in a variety of forms using a variety of delivery methods and formulations, see, e.g., **Tables 700** and **800**. When a Cas9 or gRNA component is encoded as DNA for delivery, the DNA may typically but not necessarily include a control region, e.g., comprising a promoter, to effect expression. Useful promoters for Cas9 molecule sequences include, e.g., CMV, EF-1a, EFS, MSCV, PGK, or CAG promoters. Useful promoters for gRNAs include, e.g., H1, EF-1a, tRNA or U6 promoters. Promoters with similar or dissimilar strengths can be selected to tune the expression of components. Sequences encoding a Cas9 molecule may comprise a nuclear localization signal (NLS), e.g., an SV40 NLS. In an embodiment a promoter for a Cas9 molecule or a gRNA molecule may be, independently, inducible, tissue specific, or cell specific.

**Table 700** provides examples of the form in which the components can be delivered to a target cell.

**Table 700**

| **Elements** | | **Comments** |
|---|---|---|
| **Cas9 Molecule(s)** | **gRNA molecule(s)** | |
| DNA | DNA | In this embodiment, a Cas9 molecule, typically an eaCas9 molecule, and a gRNA are transcribed from DNA. In this embodiment, they are encoded on separate molecules. |
| DNA | | In this embodiment, a Cas9 molecule, typically an eaCas9 molecule, and a gRNA are transcribed from DNA, here from a single molecule. |
| DNA | RNA | In this embodiment, a Cas9 molecule, typically an eaCas9 molecule, is transcribed from DNA, and a gRNA is provided as in vitro transcribed or synthesized RNA |
| mRNA | RNA | In this embodiment, a Cas9 molecule, typically an eaCas9 molecule, is translated from in vitro transcribed mRNA, and a gRNA is provided as in |
| | | vitro transcribed or synthesized RNA. |
| mRNA | DNA | In this embodiment, a Cas9 molecule, typically an eaCas9 molecule, is translated from in vitro transcribed mRNA, and a gRNA is transcribed from DNA. |
| Protein | DNA | In this embodiment, a Cas9 molecule, typically an eaCas9 molecule, is provided as a protein, and a gRNA is transcribed from DNA. |
| Protein | RNA | In this embodiment, an eaCas9 molecule is provided as a protein, and a gRNA is provided as transcribed or synthesized RNA. |

**Table 800** summarizes various delivery methods for the components of a Cas system, e.g., the Cas9 molecule component and the gRNA molecule component, as described herein.

**Table 800**

| **Delivery Vector**/**Mode** | | **Delivery into Non-Dividing Cells** | **Duration of Expression** | **Genome Integration** | **Type o**t^{.} **Molecule Delivered** |
|---|---|---|---|---|---|
| **Physical (eg, electroporation, particle gun, Calcium Phosphate transfection, cell compression or squeezing)** | | YES | Transient | NO | Nucleic Acids and Proteins |
| ***Viral*** | **Retrovirus** | NO | Stable | YES | RNA |
| | **Lentivirus** | YES | Stable | YES/NO with modifications | RNA |

| **Delivery Vector/Mode** | | **Delivery Duration of Delivery into Non-Dividing Cells** | **Duration of Expression** | **Genome Integration** | **Type of Molecule Delivered** |
|---|---|---|---|---|---|
| | **Adenovirus** | YES | Transient | NO | DNA |
| | **Adeno-Associated Virus (AAV)** | YES | Stable | NO | DNA |
| | **Vaccinia Virus** | YES | Very Transient | NO | DNA |
| | **Herpes Simplex Virus** | YES | Stable | NO | DNA |
| *Non-Viral* | **Cationic Liposomes** | YES | Transient | Depends on what is delivered | Nucleic Acids and Proteins |
| | **Polymeric Nanoparticles** | YES | Transient | Depends on what is delivered | Nucleic Acids and Proteins |
| ***Biological Non-Viral Delivery Vehicles*** | **Attenuated Bacteria** | YES | Transient | NO | Nucleic Acids |
| | **Engineered Bacteriophages** | YES | Transient | NO | Nucleic Acids |
| | **Mammalian Virus-like Particles** | YES | Transient | NO | Nucleic Acids |

| **Delivery Vector/Mode** | | **Delivery into Non-Dividing Cells** | **Duration of Expression** | **Genome Integration** | **Type of Molecule Delivered** |
|---|---|---|---|---|---|
| | **Biological liposomes: Erythrocyte Ghosts and Exosomes** | YES | Transient | NO | Nucleic Acids |

### DNA-based Delivery of a Cas9 molecule and/or a gRNA molecule

DNA encoding Cas9 molecules (e.g., eaCas9 molecules) and/or gRNA molecules, can be delivered into cells by art-known methods or as described herein. For example, Cas9-encoding and/or gRNA-encoding DNA can be delivered, e.g., by vectors (e.g., viral or non-viral vectors), non-vector based methods (e.g., using naked DNA or DNA complexes), or a combination thereof.

In some embodiments, the Cas9- and/or gRNA-encoding DNA is delivered by a vector (e.g., viral vector/virus or plasmid).

A vector may comprise a sequence that encodes a Cas9 molecule and/or a gRNA molecule. A vector may also comprise a sequence encoding a signal peptide (*e.g.,* for nuclear localization, nucleolar localization, mitochondrial localization), fused, e.g., to a Cas9 molecule sequence. For example, a vector may comprise a nuclear localization sequence (e.g., from SV40) fused to the sequence encoding the Cas9 molecule.

One or more regulatory/control elements, e.g., a promoter, an enhancer, an intron, a polyadenylation signal, a Kozak consensus sequence, internal ribosome entry sites (IRES), a 2A sequence, and splice acceptor or donor can be included in the vectors. In an embodiment, the promoter is recognized by RNA polymerase II (e.g., a CMV promoter). In another embodiment, the promoter is recognized by RNA polymerase III (e.g., a U6 promoter). In another embodiment, the promoter is a regulated promoter (e.g., inducible promoter). In another embodiment, the promoter is a constitutive promoter. In another embodiment, the promoter is a tissue specific promoter. In another embodiment, the promoter is a viral promoter. In another embodiment, the promoter is a non-viral promoter.

In an embodiment, the vector or delivery vehicle is a viral vector (e.g., for generation of recombinant viruses). In an embodiment, the virus is a DNA virus (e.g., dsDNA or ssDNA virus). In an embodiment, the virus is an RNA virus (e.g., an ssRNA virus). Exemplary viral vectors/viruses include, e.g., retroviruses, lentiviruses, adenovirus, adeno-associated virus (AAV), vaccinia viruses, poxviruses, and herpes simplex viruses.

In an embodiment, the virus infects dividing cells. In another embodiment, the virus infects non-dividing cells. In another embodiment, the virus infects both dividing and non-dividing cells. In another embodiment, the virus can integrate into the host genome. In another embodiment, the virus is engineered to have reduced immunity, e.g., in human. In another embodiment, the virus is replication-competent. In another embodiment, the virus is replication-defective, e.g., having one or more coding regions for the genes necessary for additional rounds of virion replication and/or packaging replaced with other genes or deleted. In another embodiment, the virus causes transient expression of the Cas9 molecule and/or the gRNA molecule. In another embodiment, the virus causes long-lasting, e.g., at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, e.g., from at least about 4 kb to at least about 30 kb, e.g., at least about 5 kb, 10 kb, 15 kb, 20 kb, 25 kb, 30 kb, 35 kb, 40 kb, 45 kb, or 50 kb.

In an embodiment, the Cas9- and/or gRNA-encoding DNA is delivered by a recombinant retrovirus. In another embodiment, the retrovirus (e.g., Moloney murine leukemia virus) comprises a reverse transcriptase, e.g., that allows integration into the host genome. In an embodiment, the retrovirus is replication-competent. In another embodiment, the retrovirus is replication-defective, e.g., having one of more coding regions for the genes necessary for additional rounds of virion replication and packaging replaced with other genes, or deleted.

In an embodiment, the Cas9- and/or gRNA-encoding DNA is delivered by a recombinant lentivirus. For example, the lentivirus is replication-defective, e.g., docs not comprise one or more genes required for viral replication.

In an embodiment, the Cas9- and/or gRNA-encoding DNA is delivered by a recombinant adenovirus. In another embodiment, the adenovirus is engineered to have reduced immunity in humans.

In an embodiment, the Cas9- and/or gRNA-encoding DNA is delivered by a recombinant AAV. In an embodiment, the AAV can incorporate its genome into that of a host cell, e.g., a target cell as described herein. In another embodiment, the AAV is a self-complementary adeno-associated virus (scAAV), e.g., a scAAV that packages both strands which anneal together to form double stranded DNA. AAV serotypes that may be used in the disclosed methods, include AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV8, AAV 8.2, AAV9, AAV rh 10, and pseudotyped AAV, such as AAV2/8, AAV2/5 and AAV2/6 can also be used in the disclosed methods.

In an embodiment, the Cas9- and/or gRNA-encoding DNA is delivered by a hybrid virus, e.g., a hybrid of one or more of the viruses described herein.

A packaging cell is used to form a virus particle that is capable of infecting a target cell. Such a cell includes a 293 cell, which can package adenovirus, and a ψ2 cell or a PA317 cell, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed, eg. Cas9. For example, an AAV vector used in gene therapy typically only possesses inverted terminal repeat (ITR) sequences from the AAV genome which are required for packaging and gene expression in the host or target cell. The missing viral functions are supplied *in trans* by the packaging cell line. Henceforth, the viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

In an embodiment, the viral vector has the ability of cell type recognition. For example, the viral vector can be pseudotyped with a different/alternative viral envelope glycoprotein; engineered with a cell type-specific receptor (e.g., genetic modification of the viral envelope glycoproteins to incorporate targeting ligands such as a peptide ligand, a single chain antibody, a growth factor); and/or engineered to have a molecular bridge with dual specificities with one end recognizing a viral glycoprotein and the other end recognizing a moiety of the target cell surface (e.g., ligand-receptor, monoclonal antibody, avidin-biotin and chemical conjugation).

In an embodiment, the viral vector achieves cell type specific expression. For example, a tissue-specific promoter can be constructed to restrict expression of the transgene (Cas 9 and gRNA) in only a specific target cell. The specificity of the vector can also be mediated by microRNA-dependent control of transgene expression. In an embodiment, the viral vector has increased efficiency of fusion of the viral vector and a target cell membrane. For example, a fusion protein such as fusion-competent hemagglutinin (HA) can be incorporated to increase viral uptake into cells. In an embodiment, the viral vector has the ability of nuclear localization. For example, a virus that requires the breakdown of the nuclear membrane (during cell division) and therefore will not infect a non-diving cell can be altered to incorporate a nuclear localization peptide in the matrix protein of the virus thereby enabling the transduction of non-proliferating cells.

In an embodiment, the Cas9- and/or gRNA-encoding DNA is delivered by a non-vector based method (e.g., using naked DNA or DNA complexes). For example, the DNA can be delivered, e.g., by organically modified silica or silicate (Ormosil), electroporation, transient cell compression or squeezing (eg, as described in Lee, et al [2012] Nano Lett 12: 6322-27), gene gun, sonoporation, magnetofection, lipid-mediated transfection, dendrimers, inorganic nanoparticles, calcium phosphates, or a combination thereof.

In an embodiment, delivery via electroporation comprises mixing the cells with the Cas9-and/or gRNA-encoding DNA in a cartridge, chamber or cuvette and applying one or more electrical impulses of defined duration and amplitude. In an embodiment, delivery via electroporation is performed using a system in which cells are mixed with the Cas9-and/or gRNA-encoding DNA in a vessel connected to a device (eg, a pump) which feeds the mixture into a cartridge, chamber or cuvette wherein one or more electrical impulses of defined duration and amplitude are applied, after which the cells are delivered to a second vessel.

In an embodiment, the Cas9- and/or gRNA-encoding DNA is delivered by a combination of a vector and a non-vector based method. For example, a virosome comprises a liposome combined with an inactivated virus (e.g., HIV or influenza virus), which can result in more efficient gene transfer than either a viral or a liposomal method alone.

In an embodiment, the delivery vehicle is a non-viral vector. In an embodiment, the non-viral vector is an inorganic nanoparticle. Exemplary inorganic nanoparticles include, e.g., magnetic nanoparticles (e.g., Fe₃MnO₂) and silica. The outer surface of the nanoparticle can be conjugated with a positively charged polymer (e.g., polyethylenimine, polylysine, polyserine) which allows for attachment (e.g., conjugation or entrapment) of payload. In an embodiment, the non-viral vector is an organic nanoparticle. Exemplary organic nanoparticles include, e.g., SNALP liposomes that contain cationic lipids together with neutral helper lipids which are coated with polyethylene glycol (PEG), and protamine-nucleic acid complexes coated with lipid.

Exemplary lipids for gene transfer are shown below in **Table 900.**

**Table 900. Lipids Used for Gene Transfer**

| **Lipid** | **Abbreviation** | **Feature** |
|---|---|---|
| 1,2-Dioleoyl-sn-glycero-3-phosphatidylcholine | DOPC | Helper |
| 1,2-Dioleoyl-sn-glycero-3-phosphatidylethanolamine | DOPE | Helper |
| Cholesterol | | Helper |
| *N-*[1-(2,3-Dioleyloxy)prophyl]*N,N,N*-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| *N*-(3-Aminopropyl)-*N,N*-dimethyl-2,3-bis(dodecyloxy-propanaminium bromide | GAP-DLRIE | Cationic |
| Cetyltrimethylammonium bromide | CTAB | Cationic |
| 6-Lauroxyhexyl ornithinate | LHON | Cationic |
| 1-(2,3-Dioleoyloxypropyl)-2,4,6-trimethylpyridinium | 2Oc | Cationic |
| 2,3-Dioleyloxy-*N*-[2(sperminecarboxamido-ethyl]-*N*,*N*-dimethyl-1-propanaminium trifluoroacetate | DOSPA | Cationic |
| 1,2-Dioleyl-3-trimethylammonium-propane | DOPA | Cationic |
| *N*-(2-Hydroxyethyl)-*N,N*-dimethyl-2,3-bis(tetradecyloxy)-1-propanaminium bromide | MDRIE | Cationic |
| Dimyristooxypropyl dimethyl hydroxyethyl ammonium bromide | DMRI | Cationic |
| 3β-[*N*-(*N*',*N*'-Dimethylaminoethane)-carbamoyl]cholesterol | DC-Chol | Cationic |
| Bis-guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac-[(2,3-Dioctadecyloxypropyl)(2-hydroxyethyl)]-dimethylammonium chloride | CLIP-1 | Cationic |
| rac-[2(2,3-Dihexadecyloxypropyl-oxymethyloxy)ethyl]trimethylammonium bromide | CLIP-6 | Cationic |
| Ethyldimyristoylphosphatidylcholine | EDMPC | Cationic |
| 1,2-Distearyloxy-*N,N*-dimethyl-3-aminopropane | DSDMA | Cationic |
| 1,2-Dimyristoyl-trimethylammonium propane | DMTAP | Cationic |
| *O,O*'-Dimyristyl-N-lysyl aspartate | DMKE | Cationic |
| 1,2-Distearoyl-sn-glycero-3-ethylphosphocholme | DSEPC | Cationic |
| *N*-Palmitoyl D-erythro-sphingosyl carbamoyl-spermine | CCS | Cationic |
| *N*-*t*-Butyl-*N*0-tetradecy1-3-tetradecylaminopropionamidine | diC14-amidine | Cationic |
| Octadecenolyoxy[ethyl-2-heptadecenyl-3 hydroxyethyl] imidazolinium chloride | DOTIM | Cationic |
| *N*1-Cholesteryloxycarbonyl-3,7-diazanonane-1,9-diamine | CDAN | Cationic |
| 2-(3-[Bis(3-amino-propyl)-amino]propylamino)-*N-*ditetradecylcarbamoylme-ethyl-acetamide | RPR209120 | Cationic |
| 1,2-dilinoleyloxy-3-dimethylaminopropane | DLinDMA | Cationic |
| 2,2-dilinoleyl-4-dimethylaminoethyl-[1,3]-dioxolane | DLin-KC2-DMA | Cationic |
| dilinoleyl-methyl-4-dimethylaminobutyrate | DLin-MC3-DMA | Cationic |

Exemplary polymers for gene transfer are shown below in **Table 1000.**

**Table 1000. Polymers Used for Gene Transfer**

| **Polymer** | **Abbreviation** |
|---|---|
| Poly(ethylene)glycol | PEG |
| Polyethylenimine | PEI |
| Dithiobis(succinimidylpropionate) | DSP |
| Dimethyl-3,3'-dithiobispropionimidate | DTBP |
| Poly(ethylene imine) biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

In an embodiment, the vehicle has targeting modifications to increase target cell update of nanoparticles and liposomes, e.g., cell specific antigens, monoclonal antibodies, single chain antibodies, aptamers, polymers, sugars, and cell penetrating peptides. In an embodiment, the vehicle uses fusogenic and endosome-destabilizing peptides/polymers. In an embodiment, the vehicle undergoes acid-triggered conformational changes (e.g., to accelerate endosomal escape of the cargo). In an embodiment, a stimulus-cleavable polymer is used, e.g., for release in a cellular compartment. For example, disulfide-based cationic polymers that are cleaved in the reducing cellular environment can be used.

In an embodiment, the delivery vehicle is a biological non-viral delivery vehicle. In an embodiment, the vehicle is an attenuated bacterium (e.g., naturally or artificially engineered to be invasive but attenuated to prevent pathogenesis and expressing the transgene (e.g., *Listeria monocytogenes*, certain *Salmonella strains, Bifidobacterium longum*, and modified *Escherichia coli*), bacteria having nutritional and tissue-specific tropism to target specific cells, bacteria having modified surface proteins to alter target cell specificity). In an embodiment, the vehicle is a genetically modified bacteriophage (e.g., engineered phages having large packaging capacity, less immunogenicity, containing mammalian plasmid maintenance sequences and having incorporated targeting ligands). In an embodiment, the vehicle is a mammalian virus-like particle. For example, modified viral particles can be generated (e.g., by purification of the "empty" particles followed by *ex vivo* assembly of the virus with the desired cargo). The vehicle can also be engineered to incorporate targeting ligands to alter target tissue specificity. In an embodiment, the vehicle is a biological liposome. For example, the biological liposome is a phospholipid-based particle derived from human cells (e.g., erythrocyte ghosts, which are red blood cells broken down into spherical structures derived from the subject (e.g., tissue targeting can be achieved by attachment of various tissue or cell-specific ligands), or secretory exosomes -subject-derived membrane-bound nanovescicles (30 -100 nm) of endocytic origin (e.g., can be produced from various cell types and can therefore be taken up by cells without the need for targeting ligands).

In an embodiment, one or more nucleic acid molecules (e.g., DNA molecules) other than the components of a Cas system, e.g., the Cas9 molecule component and/or the gRNA molecule component described herein, are delivered. In an embodiment, the nucleic acid molecule is delivered at the same time as one or more of the components of the Cas system. In an embodiment, the nucleic acid molecule is delivered before or after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In an embodiment, the nucleic acid molecule is delivered by a different means from one or more of the components of the Cas system, e.g., the Cas9 molecule component and/or the gRNA molecule component. The nucleic acid molecule can be delivered by any of the delivery methods described herein. For example, the nucleic acid molecule can be delivered by a viral vector, e.g., a retrovirus or a lentivirus, and the Cas9 molecule component and/or the gRNA molecule component can be delivered by electroporation. In an embodiment, the nucleic acid molecule encodes a *TRAC* gene, a *TRBC* gene or a CAR gene.

### Delivery of RNA encoding a Cas9 molecule

RNA encoding Cas9 molecules (e.g., eaCas9 molecules, eiCas9 molecules or eiCas9 fusion proteins) and/or gRNA molecules, can be delivered into cells, e.g., target cells described herein, by art-known methods or as described herein. For example, Cas9-encoding and/or gRNA-encoding RNA can be delivered, e.g., by microinjection, electroporation, transient cell compression or squeezing (eg, as described in Lee, et al [2012] Nano Lett 12: 6322-27), lipid-mediated transfection, peptide-mediated delivery, or a combination thereof.

In an embodiment, delivery via electroporation comprises mixing the cells with the RNA encoding Cas9 molecules (e.g., eaCas9 molecules, eiCas9 molecules or eiCas9 fusion protiens) and/or gRNA molecules in a cartridge, chamber or cuvette and applying one or more electrical impulses of defined duration and amplitude. In an embodiment, delivery via electroporation is performed using a system in which cells are mixed with the RNA encoding Cas9 molecules (e.g., eaCas9 molecules, eiCas9 molecules or eiCas9 fusion protiens) and/or gRNA molecules in a vessel connected to a device (eg, a pump) which feeds the mixture into a cartridge, chamber or cuvette wherein one or more electrical impulses of defined duration and amplitude are applied, after which the cells are delivered to a second vessel.

### Delivery of Cas9 molecule protein

Cas9 molecules (e.g., eaCas9 molecules, eiCas9 molecules or eiCas9 fusion proteins) can be delivered into cells by art-known methods or as described herein. For example, Cas9 protein molecules can be delivered, e.g., by microinjection, electroporation, transient cell compression or squeezing (eg, as described in Lee, et al [2012] Nano Lett 12: 6322-27), lipid-mediated transfection, peptide-mediated delivery, or a combination thereof. Delivery can be accompanied by DNA encoding a gRNA or by a gRNA.

In an embodiment, delivery via electroporation comprises mixing the cells with the Cas9 molecules (e.g., eaCas9 molecules, eiCas9 molecules or eiCas9 fusion proteins) with or without gRNA molecules in a cartridge, chamber or cuvette and applying one or more electrical impulses of defined duration and amplitude. In an embodiment, delivery via electroporation is performed using a system in which cells are mixed with the Cas9 molecules (e.g., eaCas9 molecules, eiCas9 molecules or eiCas9 fusion protiens) with or without gRNA molecules in a vessel connected to a device (e.g., a pump) which feeds the mixture into a cartridge, chamber or cuvette wherein one or more electrical impulses of defined duration and amplitude are applied, after which the cells are delivered to a second vessel.

### VIII. Modified Nucleosides, Nucleotides, and Nucleic Acids

Modified nucleosides and modified nucleotides can be present in nucleic acids, e.g., particularly gRNA, but also other forms of RNA, e.g., mRNA, RNAi, or siRNA. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, e.g., replacement, of one or both of the non-linking phosphate oxygens and/or of one or more of the linking phosphate oxygens in the phosphodiester backbone linkage;
(ii) alteration, e.g., replacement, of a constituent of the ribose sugar, e.g., of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, e.g., removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. In an embodiment, every base of a gRNA is modified, e.g., all bases have a modified phosphate group, e.g., all are phosphorothioate groups. In an embodiment, all, or substantially all, of the phosphate groups of a unimolecular or modular gRNA molecule are replaced with phosphorothioate groups.

In an embodiment, modified nucleotides, e.g., nucleotides having modifications as described herein, can be incorporated into a nucleic acid, e.g., a "modified nucleic acid." In some embodiments, the modified nucleic acids comprise one, two, three or more modified nucleotides. In some embodiments, at least 5% (e.g., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, e.g., cellular nucleases. For example, nucleases can hydrolyze nucleic acid phosphodiester bonds. Accordingly, in one aspect the modified nucleic acids described herein can contain one or more modified nucleosides or nucleotides, e.g., to introduce stability toward nucleases.

In some embodiments, the modified nucleosides, modified nucleotides, and modified nucleic acids described herein can exhibit a reduced innate immune response when introduced into a population of cells. The term "innate immune response" includes a cellular response to exogenous nucleic acids, including single stranded nucleic acids, generally of viral or bacterial origin, which involves the induction of cytokine expression and release, particularly the interferons, and cell death. In some embodiments, the modified nucleosides, modified nucleotides, and modified nucleic acids described herein can disrupt binding of a major groove interacting partner with the nucleic acid. In some embodiments, the modified nucleosides, modified nucleotides, and modified nucleic acids described herein can exhibit a reduced innate immune response when introduced into a population of cells and also disrupt binding of a major groove interacting partner with the nucleic acid.

### Definitions of Chemical Groups

As used herein, "alkyl" is meant to refer to a saturated hydrocarbon group which is straight-chained or branched. Example alkyl groups include methyl (Me), ethyl (Et), propyl (*e.g*., n-propyl and isopropyl), butyl (*e.g.,* n-butyl, isobutyl, t-butyl), pentyl (*e.g.,* n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (*e.g*., having 2, 3 or 4 fused rings) aromatic hydrocarbons such as, for example, phenyl, naphthyl, anthracenyl, phenanthrenyl, indanyl, indenyl, and the like. In some embodiments, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic non-aromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### Phosphate Backbone Modifications

### The Phosphate Group

In some embodiments, the phosphate group of a modified nucleotide can be modified by replacing one or more of the oxygens with a different substituent. Further, the modified nucleotide, e.g., modified nucleotide present in a modified nucleic acid, can include the wholesale replacement of an unmodified phosphate moiety with a modified phosphate as described herein. In some embodiments, the modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. In some embodiments, one of the non-bridging phosphate oxygen atoms in the phosphate backbone moiety can be replaced by any of the following groups: sulfur (S), selenium (Se), BR₃ (wherein R can be, e.g., hydrogen, alkyl, or aryl), C (e.g., an alkyl group, an aryl group, and the like), H, NR₂ (wherein R can be, e.g., hydrogen, alkyl, or aryl), or OR (wherein R can be, e.g., alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In some embodiments, modifications to one or both non-bridging oxygens can also include the replacement of the non-bridging oxygens with a group independently selected from S, Se, B, C, H, N, and OR (R can be, e.g., alkyl or aryl).

The phosphate linker can also be modified by replacement of a bridging oxygen, (i.e., the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In some embodiments, the charge phosphate group can be replaced by a neutral moiety.

Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In some embodiments, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. In some embodiments, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, e.g., alkyl, cycloalkyl, aryl, aralkyl, heteroaryl or a sugar); polyethyleneglycols (PEG), O(CH₂CH₂O)ₙCH₂CH₂OR wherein R can be, e.g., H or optionally substituted alkyl, and n can be an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In some embodiments, the "oxy"-2' hydroxyl group modification can include "locked" nucleic acids (LNA) in which the 2' hydroxyl can be connected, e.g., by a C₁₋₆ alkylene or C₁₋₆ heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy, O(CH₂)ₙ-amino, (wherein amino can be, e.g., NH₂; alkylamino, dialkylamino, heterocyclyl, arylamino, diarylamino, heteroarylamino, or diheteroarylamino, ethylenediamine, or polyamino). In some embodiments, the "oxy"-2' hydroxyl group modification can include the methoxyethyl group (MOE), (OCH₂CH₂OCH₃, e.g., a PEG derivative).

"Deoxy" modifications can include hydrogen (i.e. deoxyribose sugars, e.g., at the overhang portions of partially ds RNA); halo (e.g., bromo, chloro, fluoro, or iodo); amino (wherein amino can be, e.g., NH₂; alkylamino, dialkylamino, heterocyclyl, arylamino, diarylamino, heteroarylamino, diheteroarylamino, or amino acid); NH(CH₂CH₂NH)ₙCH₂CH₂-amino (wherein amino can be, e.g., as described herein), -NHC(O)R (wherein R can be, e.g., alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino as described herein.

The sugar group can also contain one or more carbons that possess the opposite stereochemical configuration than that of the corresponding carbon in ribose. Thus, a modified nucleic acid can include nucleotides containing e.g., arabinose, as the sugar. The nucleotide "monomer" can have an alpha linkage at the 1' position on the sugar, e.g., alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, e.g. L-nucleosides.

Generally, RNA includes the sugar group ribose, which is a 5-membered ring having an oxygen. Exemplary modified nucleosides and modified nucleotides can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In some embodiments, the modified nucleotides can include multicyclic forms (e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replaced with α-L-threofuranosyl-(3'→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In some embodiments, the nucleobase can include, for example, naturally-occurring and synthetic derivatives of a base.

### Uracil

In some embodiments, the modified nucleobase is a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho⁵U), 5-aminoallyl-uridine, 5-halo-uridine (e.g., 5-iodo-uridine or 5-bromo-uridine), 3-methyl-uridine (m³U), 5-methoxy-uridine (mo⁵U), uridine 5-oxyacetic acid (cmo⁵U), uridine 5-oxyacetic acid methyl ester (memo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s2U), 5-aminomethyl-2-thio-uridine (nm⁵s2U), 5-methylaminomethyl-uridine (mnm⁵U), 5-methylaminomethyl-2-thio-uridine (mnm⁵s2U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm ⁵s2U), 5-propynyl-uridine, 1-propynyl-pseudouridine, 5-taurinomethyl-uridine (τcm⁵U), 1-taurinomethyl-pseudouridine, 5-taurinomethyl-2-thio-uridine(rm⁵s2U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s2U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s2U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s2Um), 5-methoxycarbonylmethyl-2'-O-methyl-uridine (mcm⁵Um), 5-carbamoylmethyl-2'-O-methyl-uridine (ncm ⁵Um), 5-carboxymethylaminomethyl-2'-O-methyl-uridine (cmnm⁵Um), 3,2'-O-dimethyl-uridine (m³Um), 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

### Cytosine

In some embodiments, the modified nucleobase is a modified cytosine. Exemplary nucleobases and nucleosides having a modified cytosine include without limitation 5-aza-cytidine, 6-aza-cytidine, pseudoisocytidine, 3-methyl-cytidine (m³C), N4-acetyl-cytidine (act), 5-formyl-cytidine (f⁵C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k²C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

### Adenine

In some embodiments, the modified nucleobase is a modified adenine. Exemplary nucleobases and nucleosides having a modified adenine include without limitation 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenosine, 7-deaza-8-aza-adenosine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenosine (m²A), N6-methyl-adenosine (m⁶A), 2-methylthio-N6-methyl-adenosine (ms2m⁶A), N6-isopentenyl-adenosine (i⁶A), 2-methylthio-N6-isopentenyl-adenosine (ms²i⁶A), N6-(cis-hydroxyisopentenyl)adenosine (io⁶A), 2-methylthio-N6-(cis-hydroxyisopentenyl)adenosine (ms2io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms2hn⁶A), N6-acetyl-adenosine (ac⁶A), 7-methyl-adenosine, 2-methylthio-adenosine, 2-methoxy-adenosine, α-thio-adenosine, 2'-O-methyl-adenosine (Am), N⁶,2'-O-dimethyl-adenosine (m⁶Am), N⁶-Methyl-2'-deoxyadenosine, N6,N6,2'-O-trimethyl-adenosine (m⁶₂Am), 1,2'-O-dimethyl-adenosine (m¹Am), 2'-O-ribosyladenosine (phosphate) (Ar(p)), 2-amino-N6-methylpurine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

### Guanine

In some embodiments, the modified nucleobase is a modified guanine. Exemplary nucleobases and nucleosides having a modified guanine include without limitation inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-qucuosinc (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m'G), N2-methyl-guanosine (m²G), N2,N2-dimethyl-guanosine (m²₂G), N2,7-dimethyl-guanosine (m²,7G), N2, N2,7-dimethyl-guanosine (m²,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m²₂Gm), 1-methyl-2'-O-methyl-guanosine (m'Gm), N2,7-dimethyl-2'-O-methyl-guanosine (m²,7Gm), 2'-O-methyl-inosine (Im), 1,2'-O-dimethyl-inosine (m'Im), O⁶-phenyl-2'-deoxyinosine, 2'-O-ribosylguanosine (phosphate) (Gr(p)), 1-thio-guanosine, O⁶-methyl-guanosine, O⁶-Methyl-2'-deoxyguanosine, 2'-F-ara-guanosine, and 2'-F-guanosine.

### Exemplary Modified gRNAs

In some embodiments, the modified nucleic acids can be modified gRNAs. It is to be understood that any of the gRNAs described herein can be modified in accordance with this section, including any gRNA that comprises a targeting domain from **Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.** As discussed herein, transiently expressed or delivered nucleic acids can be prone to degradation by, e.g., cellular nucleases. Accordingly, in one aspect the modified gRNAs described herein can contain one or more modified nucleosides or nucleotides which introduce stability toward nucleases. While not wishing to be bound by theory it is also believed that certain modified gRNAs described herein can elicit a reduced innate immune response from certain cells, particularly the cells of the present invention (e.g., T-cells). As noted above, the term "innate immune response" includes a cellular response to exogenous nucleic acids, including single stranded nucleic acids, generally of viral or bacterial origin, which involves the induction of cytokine expression and release, particularly the interferons, and cell death.

For example, as discussed herein, we have seen improvements in *ex vivo* editing of genes in certain cell types (e.g., T cells) when the 5' end of a gRNA is modified by the inclusion of a eukaryotic mRNA cap structure or cap analog. The present invention encompasses the realization that the improvements observed with a 5' capped gRNA can be extended to gRNAs that have been modified in other ways to achieve the same type of structural or functional result (e.g., by the inclusion of modified nucleosides or nucleotides, by the inclusion of a 3' polyA tract and/or when an *in vitro* transcribed gRNA is modified by treatment with a phosphatase such as calf intestinal alkaline phosphatase to remove the 5' triphosphate group).

Thus, in some embodiments, methods and compositions discussed herein provide methods and compositions where gRNAs have been modified at or near their 5' end (e.g., within 1-10, 1-5, or 1-2 nucleotides of their 5' end). In an embodiment, the 5' end of a gRNA is modified by the inclusion of a eukaryotic mRNA cap structure or cap analog (e.g., a *G(5')ppp(5')G* cap analog, a *m7G(5')ppp(5')G* cap analog, or a *3'-O-Me-m7G(5')ppp(5')G* anti reverse cap analog (ARCA)) as depicted in Fig. 19. The cap or cap analog can be included during either chemical synthesis or *in vitro* transcription of the gRNA. In an embodiment, an *in vitro* transcribed gRNA is modified by treatment with a phosphatase (e.g., calf intestinal alkaline phosphatase) to remove the 5' triphosphate group.

In some embodiments, a gRNA comprises a modification at or near its 3' end (e.g., within 1-10, 1-5, or 1-2 nucleotides of its 3' end). For example, in some embodiments, the 3' end of a gRNA is modified by the addition of one or more (e.g., 25-200) adenine (A) residues. The polyA tract can be contained in the nucleic acid (e.g., plasmid, PCR product, viral genome) encoding the gRNA, or can be added to the gRNA during chemical synthesis, or following *in vitro* transcription using a polyadenosine polymerase (e.g., *E. coli* Poly(A)Polymerase). In some embodiments, gRNAs can be modified at a 3' terminal U ribose. For example, the two terminal hydroxyl groups of the U ribose can be oxidized to aldehyde groups and a concomitant opening of the ribose ring to afford a modified nucleoside as shown below: 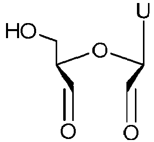 wherein "U" can be an unmodified or modified uridine. In another embodiment, the 3' terminal U can be modified with a 2'3' cyclic phosphate as shown below: 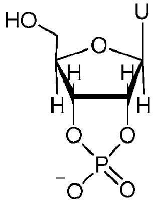 wherein "U" can be an unmodified or modified uridine. In some embodiments, the gRNA molecules may contain 3' nucleotides which can be stabilized against degradation, e.g., by incorporating one or more of the modified nucleotides described herein. In this embodiment, e.g., uridines can be replaced with modified uridines, e.g., 5-(2-amino)propyl uridine, and 5-bromo uridine, or with any of the modified uridines described herein; adenosines and guanosines can be replaced with modified adenosines and guanosines, e.g., with modifications at the 8-position, e.g., 8-bromo guanosine, or with any of the modified adenosines or guanosines described herein.

In some embodiments, a gRNA comprises both a modification at or near its 5' end and a modification at or near its 3' end. In an embodiment, *in vitro* transcribed gRNA contains both a 5' cap structure or cap analog and a 3' polyA tract. In an embodiment, an *in vitro* transcribed gRNA is modified by treatment with a phosphatase (e.g., calf intestinal alkaline phosphatase) to remove the 5' triphosphate group and comprises a 3' polyA tract.

While the foregoing has focused on terminal modifications, it is to be understood that methods and compositions discussed herein may use gRNAs that include one or more modified nucleosides or nucleotides at one or more non-terminal positions and/or one or more terminal positions within the gRNA sequence.

In some embodiments, sugar-modified ribonucleotides can be incorporated into the gRNA, e.g., wherein the 2' OH-group is replaced by a group selected from H, -OR, -R (wherein R can be, e.g., alkyl, cycloalkyl, aryl, aralkyl, heteroaryl or sugar), halo, -SH, -SR (wherein R can be, e.g., alkyl, cycloalkyl, aryl, aralkyl, heteroaryl or sugar), amino (wherein amino can be, e.g., NH₂; alkylamino, dialkylamino, heterocyclyl, arylamino, diarylamino, heteroarylamino, diheteroarylamino, or amino acid); or cyano (-CN). In some embodiments, the phosphate backbone can be modified as described herein, e.g., with a phosphothioate group. In some embodiments, one or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, e.g., 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aco), 2'-O-methoxyethyl-5-methylcytidine (m5Cco), and any combinations thereof.

In some embodiments, a gRNA can include "locked" nucleic acids (LNA) in which the 2' OH-group can be connected, e.g., by a C1-6 alkylene or C1-6 heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy or O(CH₂)ₙ-amino (wherein amino can be, e.g., NH₂; alkylamino, dialkylamino, heterocyclyl, arylamino, diarylamino, heteroarylamino, or diheteroarylamino, ethylenediamine, or polyamino).

In some embodiments, a gRNA can include a modified nucleotide which is multicyclic (e.g., tricyclo; and "unlocked" forms, such as glycol nucleic acid (GNA) (e.g., R-GNA or S-GNA, where ribose is replaced by glycol units attached to phosphodiester bonds), or threose nucleic acid (TNA, where ribose is replaced with α-L-threofuranosyl-(3'→2')).

Generally, gRNA molecules include the sugar group ribose, which is a 5-membered ring having an oxygen. Exemplary modified gRNAs can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. In an embodiment, a gRNA comprises a 4'-S, 4'-Se or a 4'-C-aminomethyl-2'-O-Me modification.

In some embodiments, deaza nucleotides, e.g., 7-deaza-adenosine, can be incorporated into the gRNA. In some embodiments, O- and N-alkylated nucleotides, e.g., N6-methyl adenosine, can be incorporated into the gRNA. In some embodiments, one or more or all of the nucleotides in a gRNA molecule are deoxynucleotides.

### miRNA binding sites

microRNAs (or miRNAs) are naturally occurring cellular 19-25 nucleotide long noncoding RNAs. They bind to nucleic acid molecules having an appropriate miRNA binding site, e.g., in the 3' UTR of an mRNA, and down-regulate gene expression. While not wishing to be bound by theory it is believed that the down regulation is either by reducing nucleic acid molecule stability or by inhibiting translation. An RNA species disclosed herein, e.g., an mRNA encoding Cas9 can comprise an miRNA binding site, e.g., in its 3'UTR. The miRNA binding site can be selected to promote down regulation of expression is a selected cell type. By way of example, the incorporation of a binding site for miR-122, a microRNA abundant in liver, can inhibit the expression of the gene of interest in the liver.

### XI. Governing gRNA molecules and the use thereof to limit the activity of a Cas9 system

Methods and compositions that use, or include, a nucleic acid, e.g., DNA, that encodes a Cas9 molecule or a gRNA molecule, can, in addition, use or include a "governing gRNA molecule." The governing gRNA can limit the activity of the other CRISPR/Cas components introduced into a cell or subject. In an embodiment, a gRNA molecule comprises a targeting domain that is complementary to a target domain on a nucleic acid that comprises a sequence that encodes a component of the CRISPR/Cas system that is introduced into a cell or subject. In an embodiment, a governing gRNA molecule comprises a targeting domain that is complementary with a target sequence on: (a) a nucleic acid that encodes a Cas9 molecule; (b) a nucleic acid that encodes a gRNA which comprises a targeting domain that targets the *FAS, BID, CTLA4, PDCD1, CBLB, PTPN6, TRAC* or *TRBC* gene (a target gene gRNA); or on more than one nucleic acid that encodes a CRISPR/Cas component, e.g., both (a) and (b). The governing gRNA molecule can complex with the Cas9 molecule to inactivate a component of the system. In an embodiment, a Cas9 molecule/governing gRNA molecule complex inactivates a nucleic acid that comprises the sequence encoding the Cas9 molecule. In an embodiment, a Cas9 molecule/governing gRNA molecule complex inactivates the nucleic acid that comprises the sequence encoding a target gene gRNA molecule. In an embodiment, a Cas9 molecule/governing gRNA molecule complex places temporal, level of expression, or other limits, on activity of the Cas9 molecule/target gene gRNA molecule complex. In an embodiment, a Cas9 molecule/governing gRNA molecule complex reduces off-target or other unwanted activity. In an embodiment, a governing gRNA molecule targets the coding sequence, or a control region, e.g., a promoter, for the CRISPR/Cas system component to be negatively regulated. For example, a governing gRNA can target the coding sequence for a Cas9 molecule, or a control region, e.g., a promoter, that regulates the expression of the Cas9 molecule coding sequence, or a sequence disposed between the two. In an embodiment, a governing gRNA molecule targets the coding sequence, or a control region, e.g., a promoter, for a target gene gRNA. In an embodiment, a governing gRNA, e.g., a Cas9-targeting or target gene gRNA-targeting, governing gRNA molecule, or a nucleic acid that encodes it, is introduced separately, e.g., later, than is the Cas9 molecule or a nucleic acid that encodes it. For example, a first vector, e.g., a viral vector, e.g., an AAV vector, can introduce nucleic acid encoding a Cas9 molecule and one or more target gene gRNA molecules, and a second vector, e.g., a viral vector, e.g., an AAV vector, can introduce nucleic acid encoding a governing gRNA molecule, e.g., a Cas9-targeting or target gene gRNA targeting, gRNA molecule. In an embodiment, the second vector can be introduced after the first. In other embodiments, a governing gRNA molecule, e.g., a Cas9-targeting or target gene gRNA targeting, governing gRNA molecule, or a nucleic acid that encodes it, can be introduced together, e.g., at the same time or in the same vector, with the Cas9 molecule or a nucleic acid that encodes it, but, e.g., under transcriptional control elements, e.g., a promoter or an enhancer, that are activated at a later time, e.g., such that after a period of time the transcription of Cas9 is reduced. In an embodiment, the transcriptional control element is activated intrinsically. In an embodiment, the transcriptional element is activated via the introduction of an external trigger.

Typically a nucleic acid sequence encoding a governing gRNA molecule, e.g., a Cas9-targeting gRNA molecule, is under the control of a different control region, e.g., promoter, than is the component it negatively modulates, e.g., a nucleic acid encoding a Cas9 molecule. In an embodiment, "different control region" refers to simply not being under the contol of one control region, e.g., promoter, that is functionally coupled to both controlled sequences. In an embodiment, different refers to "different control region" in kind or type of control region. For example, the sequence encoding a governing gRNA molecule, e.g., a Cas9-targeting gRNA molecule, is under the control of a control region, e.g., a promoter, that has a lower level of expression, or is expressed later than the sequence which encodes is the component it negatively modulates, e.g., a nucleic acid encoding a Cas9 molecule.

By way of example, a sequence that encodes a governing gRNA molecule, e.g., a Cas9-targeting governing gRNA molecule, can be under the control of a control region (e.g., a promoter) described herein, e.g., human U6 small nuclear promoter, or human H1 promoter. In an embodiment, a sequence that encodes the component it negatively regulates, e.g., a nucleic acid encoding a Cas9 molecule, can be under the control of a control region (e.g., a promoter) described herein, e.g., CMV, EF-1a, MSCV, PGK, CAG control promoters.

### EXAMPLES

The following Examples are merely illustrative and are not intended to limit the scope or content of the invention in any way.

### Example 1: Cloning and Initial Screening of gRNAs

The suitability of candidate gRNAs can be evaluated as described in this example. Although described for a chimeric gRNA, the approach can also be used to evaluate modular gRNAs.

### Cloning gRNAs into Vectors

For each gRNA, a pair of overlapping oligonucleotides is designed and obtained. Oligonucleotides are annealed and ligated into a digested vector backbone containing an upstream U6 promoter and the remaining sequence of a long chimeric gRNA. Plasmid is sequence-verified and prepped to generate sufficient amounts of transfection-quality DNA. Alternate promoters may be used to drive *in vivo* transcription (e.g., HI promoter) or for *in vitro* transcription (e.g., a T7 promoter).

### Cloning gRNAs in linear dsDNA molecule (STITCHR)

For each gRNA, a single oligonucleotide is designed and obtained. The U6 promoter and the gRNA scaffold (e.g. including everything except the targeting domain, e.g., including sequences derived from the crRNA and tracrRNA, e.g., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive in vivo transcription (e.g., HI promoter) or for in vitro transcription (e.g., T7 promoter). Any gRNA scaffold may be used to create gRNAs compatible with Cas9s from any bacterial species

### Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, an erythroid cell). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as Cell/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels arc present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1-fraction cleaved)^{½}). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700bp long. For next generation sequencing, PCR amplicons may be 300-500bp long. If the goal is to knockout gene function, sequencing may be used to assess what percent of alleles have undergone NHEJ-induccd indels that result in a frameshift or large deletion or insertion that would be expected to destroy gene function. If the goal is to remove a specific sequence motif, sequencing may be used to assess what percent of alleles have undergone NHEJ-induced deletions that span this sequence.

### Example 3: Screening of gRNAs in 293 cells

### Screening of gRNAs for T cell receptor beta (TRBC)

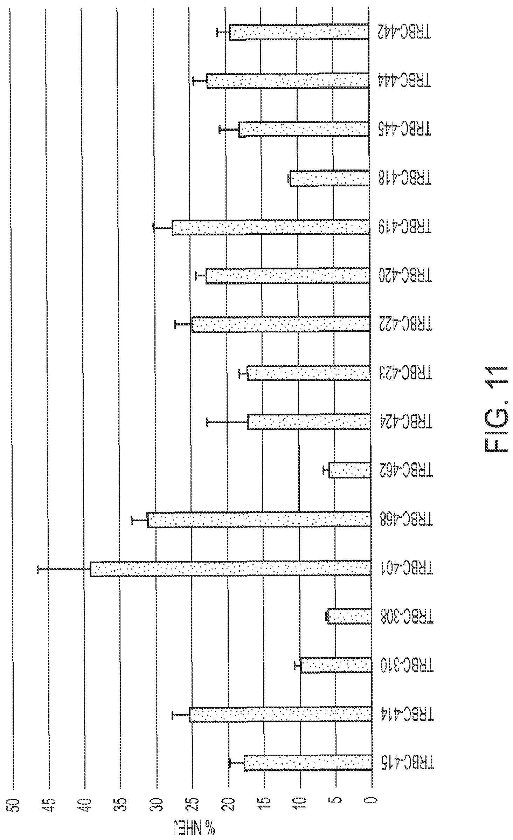

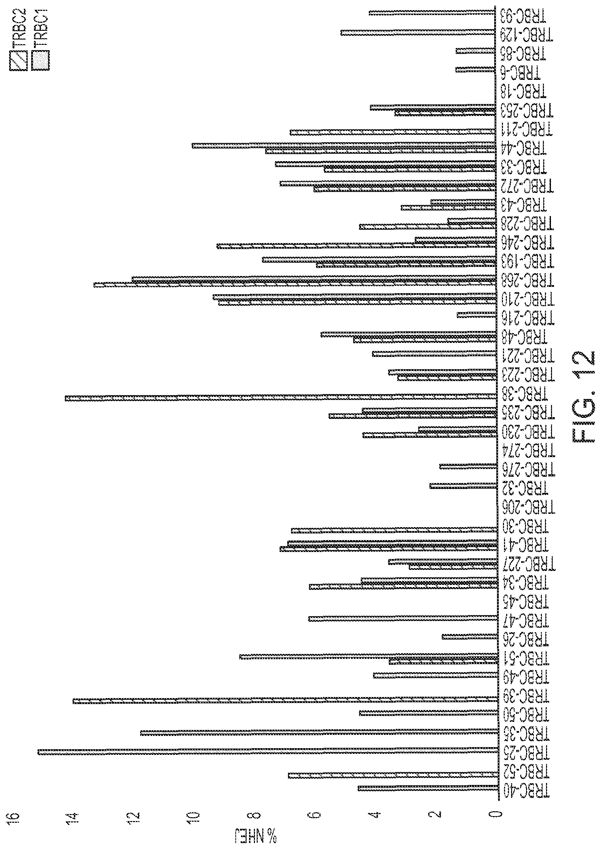

In order to identify gRNAs with the highest on target NHEJ efficiency, 42 *S. pyogenes* and 27 *S*. *aureus* gRNAs were selected (Table 27). A DNA template comprised of a U6 promoter, the gRNA target region and appropriate TRACR sequence (*S. pyogenes* or *S. aureus*) was generated by a PCR STITCHR reaction. This DNA template was subsequently transfected into 293 cells using Lipofectamine 3000 along with a DNA plasmid encoding the appropriate Cas9 (*S. pyogenes* or *S. aureus*) downstream of a CMV promoter. Genomic DNA was isolated from the cells 48-72 hours post transfection. To determine the rate of modification at the T cell receptor beta gene (*TRBC*), the target region was amplified using a locus PCR with the primers listed in Table 28. After PCR amplification, a T7E1 assay was performed on the PCR product. Briefly, this assay involves melting the PCR product followed by a re-annealing step. If gene modification has occurred, there will exist double stranded products that are not perfect matches due to some frequency of insertions or deletions. These double stranded products are sensitive to cleavage by a T7 endonuclease 1 enzyme at the site of mismatch. It is possible, therefore, to determine the efficiency of cutting by the Cas9/gRNA complex by analyzing the amount of T7E1 cleavage. The formula that is used to provide a measure of % NHEJ from the T7E1 cutting is the following: (100^{∗}(1-((1-(fraction cleaved))^0.5))). The results of this analysis are shown in **Fig. 11** and **Fig. 12****.**

**Table 27**

| gRNA Name | Targeting Domain | Cas9 species | SEQ ID NO: |
|---|---|---|---|
| TRBC-40 | CACCCAGAUCGUCAGCGCCG | *S. pyogenes* | 387 |
| TRBC-52 | CAAACACAGCGACCUCGGGU | *S. pyogenes* | 388 |
| TRBC-25 | UGACGAGUGGACCCAGGAUA | *S. pyogenes* | 389 |
| TRBC-35 | GGCUCUCGGAGAAUGACGAG | *S. pyogenes* | 390 |
| TRBC-50 | GGCCUCGGCGCUGACGAUCU | *S. pyogenes* | 391 |
| TRBC-39 | GAAAAACGUGUUCCCACCCG | *S. pyogenes* | 392 |
| TRBC-49 | AUGACGAGUGGACCCAGGAU | *S. pyogenes* | 393 |
| TRBC-51 | AGUCCAGUUCUACGGGCUCU | *S. pyogenes* | 394 |
| TRBC-26 | CGCUGUCAAGUCCAGUUCUA | *S. pyogenes* | 395 |
| TRBC-47 | AUCGUCAGCGCCGAGGCCUG | *S. pyogenes* | 396 |
| TRBC-45 | UCAAACACAGCGACCUCGGG | *S. pyogenes* | 397 |
| TRBC-34 | CGUAGAACUGGACUUGACAG | *S. pyogenes* | 398 |
| TRBC-227 | AGGCCUCGGCGCUGACGAUC | *S. pyogenes* | 399 |
| TRBC-41 | UGACAGCGGAAGUGGUUGCG | *S. pyogenes* | 400 |
| TRBC-30 | UUGACAGCGGAAGUGGUUGC | *S. pyogenes* | 401 |
| TRBC-206 | UCUCCGAGAGCCCGUAGAAC | *S. pyogenes* | 402 |
| TRBC-32 | CGGGUGGGAACACGUUUUUC | *S. pyogenes* | 403 |
| TRBC-276 | GACAGGUUUGGCCCUAUCCU | *S. pyogenes* | 404 |
| TRBC-274 | GAUCGUCAGCGCCGAGGCCU | *S. pyogenes* | 405 |
| TRBC-230 | GGCUCAAACACAGCGACCUC | *S. pyogenes* | 406 |
| TRBC-235 | UGAGGGUCUCGGCCACCUUC | *S. pyogenes* | 407 |
| TRBC-38 | AGGCUUCUACCCCGACCACG | *S. pyogenes* | 408 |
| TRBC-223 | CCGACCACGUGGAGCUGAGC | *S. pyogenes* | 409 |
| TRBC-221 | UGACAGGUUUGGCCCUAUCC | *S. pyogenes* | 410 |
| TRBC-48 | CUUGACAGCGGAAGUGGUUG | *S. pyogenes* | 411 |
| TRBC-216 | AGAUCGUCAGCGCCGAGGCC | *S. pyogenes* | 412 |
| TRBC-210 | GCGCUGACGAUCUGGGUGAC | *S. pyogenes* | 413 |
| TRBC-268 | UGAGGGCGGGCUGCUCCUUG | *S. pyogenes* | 414 |
| TRBC-193 | GUUGCGGGGGUUCUGCCAGA | *S. pyogenes* | 415 |
| TRBC-246 | AGCUCAGCUCCACGUGGUCG | *S. pyogenes* | 416 |
| TRBC-228 | GCGGCUGCUCAGGCAGUAUC | *S. pyogenes* | 417 |
| TRBC-43 | GCGGGGGUUCUGCCAGAAGG | *S. pyogenes* | 418 |
| TRBC-272 | UGGCUCAAACACAGCGACCU | *S. pyogenes* | 419 |
| TRBC-33 | ACUGGACUUGACAGCGGAAG | *S. pyogenes* | 420 |
| TRBC-44 | GACAGCGGAAGUGGUUGCGG | *S. pyogenes* | 421 |
| TRBC-211 | GCUGUCAAGUCCAGUUCUAC | *S. pyogenes* | 422 |
| TRBC-253 | GUAUCUGGAGUCAUUGAGGG | *S. pyogenes* | 423 |
| TRBC-18 | CUCGGCGCUGACGAUCU | *S. pyogenes* | 424 |
| TRBC-6 | CCUCGGCGCUGACGAUC | *S. pyogenes* | 425 |
| TRBC-85 | CCGAGAGCCCGUAGAAC | *S. pyogenes* | 426 |
| TRBC-129 | CCAGAUCGUCAGCGCCG | *S. pyogenes* | 427 |
| TRBC-93 | GAAUGACGAGUGGACCC | *S. pyogenes* | 428 |
| TRBC-415 | GGGUGACAGGUUUGGCCCUAUC | *S. aureus* | 429 |
| TRBC-414 | GGUGACAGGUUUGGCCCUAUC | *S. aureus* | 430 |
| TRBC-310 | GUGACAGGUUUGGCCCUAUC | *S. aureus* | 431 |
| TRBC-308 | GACAGGUUUGGCCCUAUC | *S. aureus* | 432 |
| TRBC-401 | GAUACUGCCUGAGCAGCCGCCU | *S. aureus* | 433 |
| TRBC-468 | GACCACGUGGAGCUGAGCUGGUGG | *S. aureus* | 434 |
| TRBC-462 | GUGGAGCUGAGCUGGUGG | *S. aureus* | 435 |
| TRBC-424 | GGGCGGGCUGCUCCUUGAGGGGCU | *S. aureus* | 436 |
| TRBC-423 | GGCGGGCUGCUCCUUGAGGGGCU | *S. aureus* | 437 |
| TRBC-422 | GCGGGCUGCUCCUUGAGGGGCU | *S. aureus* | 438 |
| TRBC-420 | GGGCUGCUCCUUGAGGGGCU | *S. aureus* | 439 |
| TRBC-419 | GGCUGCUCCUUGAGGGGCU | *S. aureus* | 440 |
| TRBC-418 | GCUGCUCCUUGAGGGGCU | *S. aureus* | 441 |
| TRBC-445 | GGUGAAUGGGAAGGAGGUGCACAG | *S. aureus* | 442 |
| TRBC-444 | GUGAAUGGGAAGGAGGUGCACAG | *S. aureus* | 443 |
| TRBC-442 | GAAUGGGAAGGAGGUGCACAG | *S. aureus* | 444 |

**Table 28**

| Primer name | Sequence | Exon | SEQ ID NO: |
|---|---|---|---|
| GWED133 | ACATCACCTGGAATGTTAGGCA | TRBC2 5' primer | 445 |
| GWED134 | GTGAACCTTGATCATCCCACCT | TRBC2 3' Primer | 446 |
| GWED137 | CCCCAAAACAATGAGGGCCT | TRBC1 5' primer | 447 |
| GWED138 | AGCACCTCCTTTCTTCTGCC | TRBC1 3' primer | 448 |

### Screening of gRNAs for T cell receptor alpha (TRAC)

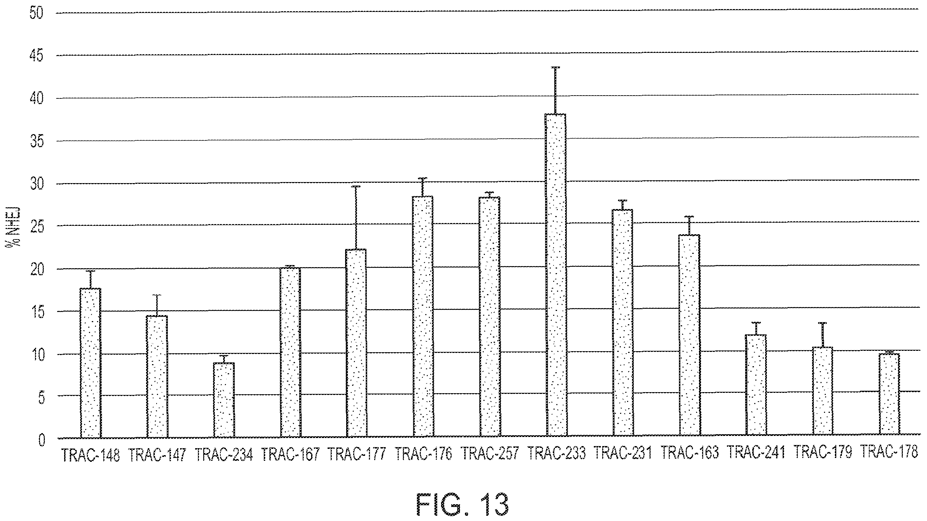

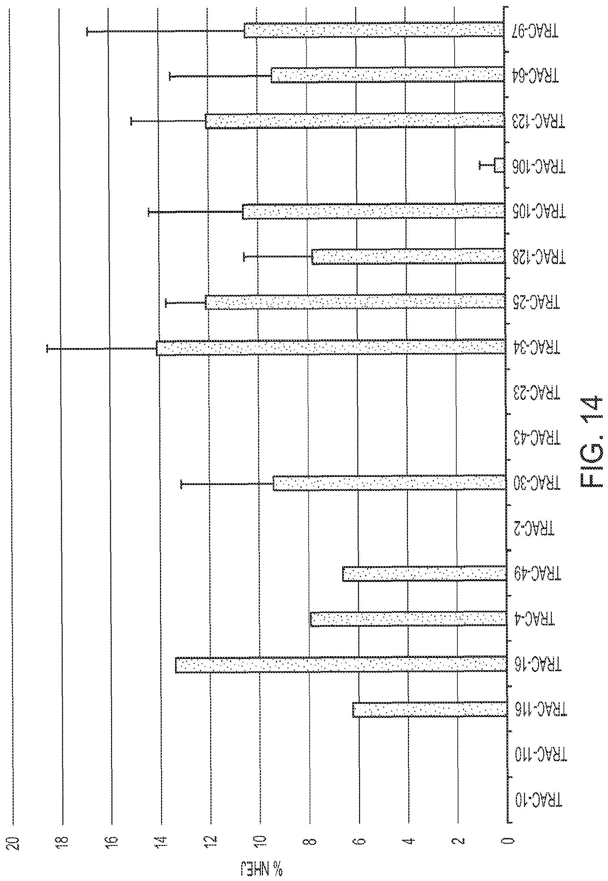

In order to identify gRNAs with the highest on target NHEJ efficiency, 18 *S. pyogenes* and 13 *S. aureus* gRNAs were selected (Table 29). A DNA template comprised of a U6 promoter, the gRNA target region and appropriate TRACR sequence *(S. pyogenes* or *S. aureus)* was generated by a PCR STITCHR reaction. This DNA template was subsequently transfected into 293 cells using Lipofectamine 3000 along with a DNA plasmid encoding the appropriate Cas9 *(S. pyogenes* or *S. aureus)* downstream of a CMV promoter. Genomic DNA was isolated from the cells 48-72 hours post transfection. To determine the rate of modification at the T cell receptor alpha gene (*TRAC*), the target region was amplified using a locus PCR with the primers listed in Table 30. After PCR amplification, a T7E1 assay was performed on the PCR product. Briefly, this assay involves melting the PCR product followed by a re-annealing step. If gene modification has occurred, there will exist double stranded products that are not perfect matches due to some frequency of insertions or deletions. These double stranded products are sensitive to cleavage by a T7 endonuclease 1 enzyme at the site of mismatch. It is possible, therefore, to determine the efficiency of cutting by the Cas9/gRNA complex by analyzing the amount of T7E1 cleavage. The formula that is used to provide a measure of % NHEJ from the T7E1 cutting is the following: (100^{∗}(1-((1-(fraction cleaved))^0.5))). The results of this analysis are shown in **Fig. 13** and **Fig. 14****.**

**Table 29**

| gRNA Name | Targeting Domain | Cas9 species | SEQ ID NO: |
|---|---|---|---|
| TRAC-10 | UCUCUCAGCUGGUACACGGC | *S. pyogenes* | 449 |
| TRAC-110 | UGGAUUUAGAGUCUCUCAGC | *S. pyogenes* | 450 |
| TRAC-116 | ACACGGCAGGGUCAGGGUUC | *S. pyogenes* | 451 |
| TRAC-16 | GAGAAUCAAAAUCGGUGAAU | *S. pyogenes* | 452 |
| TRAC-4 | GCUGGUACACGGCAGGGUCA | *S. pyogenes* | 453 |
| TRAC-49 | CUCAGCUGGUACACGGC | *S. pyogenes* | 454 |
| TRAC-2 | UGGUACACGGCAGGGUC | *S. pyogenes* | 455 |
| TRAC-30 | GCUAGACAUGAGGUCUA | *S. pyogenes* | 456 |
| TRAC-43 | GUCAGAUUUGUUGCUCC | *S. pyogenes* | 457 |
| TRAC-23 | UCAGCUGGUACACGGCA | *S. pyogenes* | 458 |
| TRAC-34 | GCAGACAGACUUGUCAC | *S. pyogenes* | 459 |
| TRAC-25 | GGUACACGGCAGGGUCA | *S. pyogenes* | 460 |
| TRAC-128 | CUUCAAGAGCAACAGUGCUG | *S. pyogenes* | 461 |
| TRAC-105 | AGAGCAACAGUGCUGUGGCC | *S. pyogenes* | 462 |
| TRAC-106 | AAAGUCAGAUUUGUUGCUCC | *S. pyogenes* | 463 |
| TRAC-123 | ACAAAACUGUGCUAGACAUG | *S. pyogenes* | 464 |
| TRAC-64 | AAACUGUGCUAGACAUG | *S. pyogenes* | 465 |
| TRAC-97 | UGUGCUAGACAUGAGGUCUA | *S. pyogenes* | 466 |
| TRAC-148 | GGCUGGGGAAGAAGGUGUCUUC | *S. aureus* | 467 |
| TRAC-147 | GCUGGGGAAGAAGGUGUCUUC | *S. aureus* | 468 |
| TRAC-234 | GGGGAAGAAGGUGUCUUC | *S. aureus* | 469 |
| TRAC-167 | GUUUUGUCUGUGAUAUACACAU | *S. aureus* | 470 |
| TRAC-177 | GGCAGACAGACUUGUCACUGGAUU | *S. aureus* | 471 |
| TRAC-176 | GCAGACAGACUUGUCACUGGAUU | *S. aureus* | 472 |
| TRAC-257 | GACAGACUUGUCACUGGAUU | *S. aureus* | 473 |
| TRAC-233 | GUGAAUAGGCAGACAGACUUGUCA | *S. aureus* | 474 |
| TRAC-231 | GAAUAGGCAGACAGACUUGUCA | *S. aureus* | 475 |
| TRAC-163 | GAGUCUCUCAGCUGGUACACGG | *S. aureus* | 476 |
| TRAC-241 | GUCUCUCAGCUGGUACACGG | *S. aureus* | 477 |
| TRAC-179 | GGUACACGGCAGGGUCAGGGUU | *S. aureus* | 478 |
| TRAC-178 | GUACACGGCAGGGUCAGGGUU | *S. aureus* | 49451 |

**Table 30**

| Primer name | Sequence | Exon | SEQ ID NO: |
|---|---|---|---|
| GWED141 | GTTTGCTTTGCTGGGCCTTT | TRAC 5' primer | 479 |
| GWED142 | CCTCTCCTGCCACCTTCTCT | TRAC 3' primer | 480 |

### Screening of gRNAs for PD-1 receptor

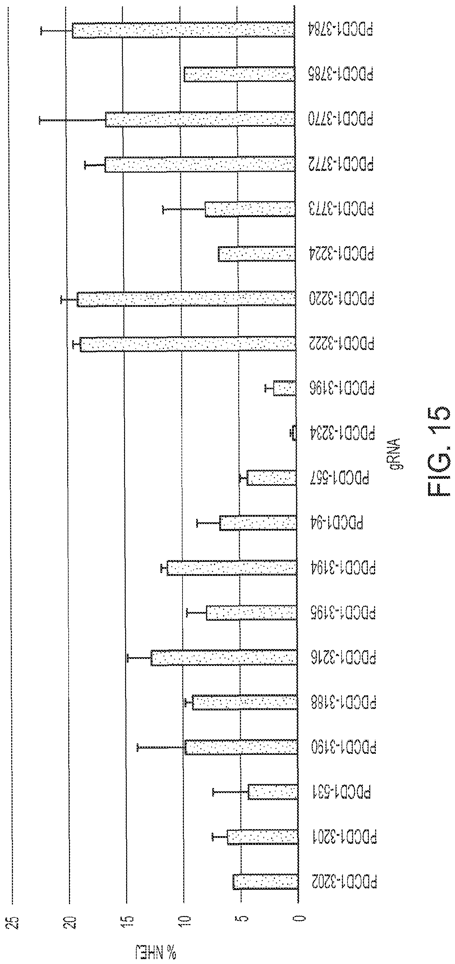

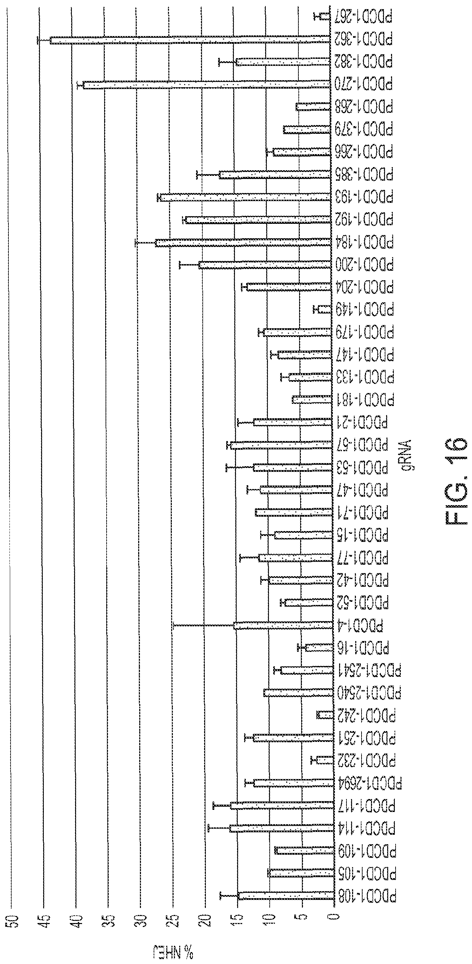

In order to identify gRNAs with the highest on target NHEJ efficiency, 48 *S. pyogenes* and 27 S. *aureus* gRNAs were selected (see Tables 31 and 32). A DNA template comprised of a U6 promoter, the gRNA target region and appropriate TRACR sequence *(S. pyogenes* or *S. aureus)* was generated by a PCR STITCHR reaction. This DNA template was subsequently transfected into 293 cells using Lipofectamine 3000 along with a DNA plasmid encoding the appropriate Cas9 *(S. pyogenes* or *S. aureus)* downstream of a CMV promoter. Genomic DNA was isolated from the cells 48-72 hours post transfection. To determine the rate of modification at the PD-1 gene (*PDCD1*), the target region was amplified using a locus PCR with the primers listed in Table 33. After PCR amplification, a T7E1 assay was performed on the PCR product. Briefly, this assay involves melting the PCR product followed by a re-annealing step. If gene modification has occurred, there will exist double stranded products that are not perfect matches due to some frequency of insertions or deletions. These double stranded products are sensitive to cleavage by a T7 endonuclease 1 enzyme at the site of mismatch. It is possible, therefore, to determine the efficiency of cutting by the Cas9/gRNA complex by analyzing the amount of T7E1 cleavage. The formula that is used to provide a measure of % NHEJ from the T7E1 cutting is the following: (100^{∗}(1-((1-(fraction cleaved))^0.5))). The results of this analysis for the gRNAs shown in Table 31 are shown in **Fig. 15** and **Fig. 16****.** Similar experiments can be performed with other gRNAs descrinbed herein, including those shown in Table 32.

**Table 31**

| gRNA Name | Targeting Domain | Cas9 species | SEQ ID NO: |
|---|---|---|---|
| PDCD1-3202 | GGUGCUACAACUGGGCUGGCGGC | *S. aureus* | 481 |
| PDCD1-3201 | GUGCUACAACUGGGCUGGCGGC | *S. aureus* | 482 |
| PDCD 1-531 | GCUACAACUGGGCUGGCGGC | *S. aureus* | 483 |
| PDCD1-3190 | GACGACUGGCCAGGGCGCCUG | *S. aureus* | 484 |
| PDCD1-3188 | GACUGGCCAGGGCGCCUG | *S. aureus* | 485 |
| PDCD1-3216 | GCAUGCCUGGAGCAGCCCCAC | *S. aureus* | 486 |
| PDCD1-3195 | GGGGGGUUCCAGGGCCUGUCUG | *S. aureus* | 487 |
| PDCD1-3194 | GGGGGUUCCAGGGCCUGUCUG | *S. aureus* | 488 |
| PDCD 1-94 | GGGGUUCCAGGGCCUGUCUG | *S. aureus* | 489 |
| PDCD1-557 | GAGAGCCUGCGGGCAGAGCU | *S. aureus* | 490 |
| PDCD1-3234 | GAGCCUGCGGGCAGAGCU | *S. aureus* | 491 |
| PDCD1-3196 | GGGGGGGUUCCAGGGCCUGUCUG | *S. aureus* | 492 |
| PDCD 1-3222 | GCAGGGCUGGGGAGAAGGUGG | *S. aureus* | 493 |
| PDCD 1-3220 | GGGCUGGGGAGAAGGUGG | *S. aureus* | 494 |
| PDCD 1-3224 | GAGCAGGGCUGGGGAGAAGGUGG | *S. aureus* | 495 |
| PDCD1-3773 | GGCCGCCCACGACACCAACCAC | *S. aureus* | 496 |
| PDCD 1-3772 | GCCGCCCACGACACCAACCAC | *S. aureus* | 497 |
| PDCD 1-3770 | GCCCACGACACCAACCAC | *S. aureus* | 498 |
| PDCD 1-3785 | GGUUUGGAACUGGCCGGCUGGC | *S. aureus* | 499 |
| PDCD1-3784 | GUUUGGAACUGGCCGGCUGGC | *S. aureus* | 500 |
| PDCD1-108 | GUCUGGGCGGUGCUACAACU | *S. pyogenes* | 508 |
| PDCD1-105 | GGCGCCCUGGCCAGUCGUCU | *S. pyogenes* | 509 |
| PDCD1-109 | GGGCGGUGCUACAACUGGGC | *S. pyogenes* | 510 |
| PDCD1-114 | GGCCAGGAUGGUUCUUAGGU | *S. pyogenes* | 511 |
| PDCD1-117 | GGAUGGUUCUUAGGUAGGUG | *S. pyogenes* | 512 |
| PDCD 1-2694 | GAAGGCGGCACUCUGGU | *S. pyogenes* | 513 |
| PDCD1-232 | GCCCUGGCCAGUCGUCU | *S. pyogenes* | 514 |
| PDCD1-251 | GCCCAGACGACUGGCCA | *S. pyogenes* | 515 |
| PDCD1-242 | GAUGGUUCUUAGGUAGG | *S. pyogenes* | 516 |
| PDCD 1-2540 | GGCGGAGGUGAGCGGAA | *S. pyogenes* | 517 |
| PDCD1-2541 | GGAAGGGAAACUGUCCC | *S. pyogenes* | 518 |
| PDCD1-16 | GCGUGACUUCCACAUGAGCG | *S. pyogenes* | 519 |
| PDCD 1-4 | GCCCUGCUCGUGGUGACCGA | *S. pyogenes* | 520 |
| PDCD1-52 | GGUGCCGCUGUCAUUGCGCC | *S. pyogenes* | 521 |
| PDCD1-42 | GCUCUCUUUGAUCUGCGCCU | *S. pyogenes* | 522 |
| PDCD1-77 | GAAGGUGGCGUUGUCCCCUU | *S.* p*yogenes* | 523 |
| PDCD1-15 | GUGUCACACAACUGCCCAAC | *S. pyogenes* | 524 |
| PDCD1-71 | GCUUGUCCGUCUGGUUGCUG | *S. pyogenes* | 525 |
| PDCD1-47 | GAUCUGCGCCUUGGGGGCCA | *S. pyogenes* | 526 |
| PDCD1-53 | GGGCCCUGACCACGCUCAUG | *S. pyogenes* | 527 |
| PDCD1-57 | GCAGUUGUGUGACACGGAAG | *S. pyogenes* | 528 |
| PDCD 1-21 | GACAGCGGCACCUACCUCUG | *S. pyogenes* | 529 |
| PDCD1-181 | GUGGAAGUCACGCCCGU | *S. pyogenes* | 530 |
| PDCD1-133 | GCUCGUGGUGACCGAAG | *S. pyogenes* | 531 |
| PDCD1-147 | GCCCGGCGCAAUGACAG | *S. pyogenes* | 532 |
| PDCD1-179 | GCCGCUGUCAUUGCGCC | *S. pyogenes* | 533 |
| PDCD1-149 | GCGGCACCUACCUCUGU | *S. pyogenes* | 534 |
| PDCD 1-204 | GGUGGCGUUGUCCCCUU | *S. pyogenes* | 535 |
| PDCD 1-200 | GAAGCUCUCCGAUGUGU | *S. pyogenes* | 536 |
| PDCD1-184 | GUUGUGUGACACGGAAG | *S. pyogenes* | 537 |
| PDCD1-192 | GCUGGCUGCGGUCCUCG | *S. pyogenes* | 538 |
| PDCD1-193 | GCUGCGGUCCUCGGGGA | *S. pyogenes* | 539 |
| PDCD1-385 | GACGUUACCUCGUGCGGCCC | *S. pyogenes* | 540 |
| PDCD 1-266 | GGUGUCGUGGGCGGCCUGCU | *S. pyogenes* | 541 |
| PDCD1-379 | GCCCACGACACCAACCACCA | *S. pyogenes* | 542 |
| PDCD1-268 | GCAGCCUGGUGCUGCUAGUC | *S. pyogenes* | 543 |
| PDCD1-270 | GGUGCUGCUAGUCUGGGUCC | *S. pyogenes* | 544 |
| PDCD1-382 | GGACCCAGACUAGCAGCACC | *S. pyogenes* | 545 |
| PDCD1-362 | GGCACUUCUGCCCUUCUCUC | *S. pyogenes* | 546 |
| PDCD1-267 | GGGCGGCCUGCUGGGCAGCC | *S. pyogenes* | 547 |

**Table 32**

| gRNA Name | Targeting Domain | Cas9 species | SEQ ID NO: |
|---|---|---|---|
| PDCD1-3856 | GGGCAGCCUGGUGCUGCUAGU | *S. aureus* | 501 |
| PDCD1-720 | GGCAGCCUGGUGCUGCUAGU | *S. aureus* | 502 |
| PDCD 1-3855 | GCAGCCUGGUGCUGCUAGU | *S. aureus* | 503 |
| PDCD1-369 | GGCUGGCCUGGGUGAGGGGC | *S. aureus* | 504 |
| PDCD1-3786 | GGGUUUGGAACUGGCCGGCUGGC | *S. aureus* | 505 |
| PDCD1-3859 | GCUGGGCAGCCUGGUGCUGCUAGU | *S. aureus* | 506 |
| PDCD1-3781 | GGCCGGCUGGCCUGGGUGAGGGGC | *S. aureus* | 507 |
| PDCD1-381 | GCAGCACCAGGCUGCCCAGC | *S. pyogenes* | 548 |
| PDCD1-368 | GCUGGCCUGGGUGAGGGGCU | *S. pyogenes* | 549 |
| PDCD 1-472 | GUUACCUCGUGCGGCCC | *S. pyogenes* | 550 |
| PDCD1-316 | GUCGUGGGCGGCCUGCU | *S. pyogenes* | 551 |
| PDCD1-462 | GUUUGGAACUGGCCGGC | *S. pyogenes* | 552 |
| PDCD1-322 | GGCCGUCAUCUGCUCCC | *S. pyogenes* | 553 |

| cRNA Name | Targeting Domain | Cas9 species | SEQ ID NO: |
|---|---|---|---|
| PDCD1-320 | GCUGCUAGUCUGGGUCC | *S. pyogenes* | 554 |
| PDCD1-318 | GCCUGGUGCUGCUAGUC | *S. pyogenes* | 555 |

**Table 33**

| Primer name | Sequence | Exon | SEQ ID NO: |
|---|---|---|---|
| GWED259 | CACTGCCTCTGTCACTCTCG | PD1_exon_1_5' | 556 |
| GWED260 | AGGGACTGAGAGTGAAAGGT | PD1_exon_1_3' | 557 |
| JFPR004 | CAGGATGCCCAAGGGTCAG | PD1_exon_2_5' | 558 |
| JFPR004R | GGAGCTCCTGATCCTGTGC | PD1_exon_2_3' | 559 |
| GWED257 | AATGGTGACCGGCATCTCTG | PD1_exon_3_5' | 560 |
| JFPR005 | CTGCACAGGATCAGGAGCTC | PD1_exon_3_5' | 561 |
| JFPR005R | AGAATGTGAGTCCTGCAGGC | PD1_exon_3_3' | 562 |

### Example 4: Enzymatic synthesis and delivery of gRNAs to primary T cells

### Delivery of Cas9 mRNA and gRNA as RNA molecules to T cells

To demonstrate Cas9-mediated cutting in primary CD4+ T cells, *S. pyogenes* Cas9 and a gRNA designed against the TCR beta chain (TRBC-210 (GCGCUGACGAUCUGGGUGAC) (SEQ ID NO:413)) or the TCR alpha chain (TRAC-4 (GCUGGUACACGGCAGGGUCA) (SEQ ID NO:453)) were delivered as RNA molecules to T cells via electroporation. In this embodiment, both the Cas9 and gRNA were in vitro transcribed using a T7 polymerase. A 5' ARCA cap was added to both RNA species simultaneous to transcription while a polyA tail was added after transcription to the 3' end of the RNA species by an *E. coli* polyA polymerase. To generate CD4+ T cells modified at the *TRBC1* and *TRBC2* loci, 10ug of Cas9 mRNA and 10ug of TRBC-210 (GCGCUGACGAUCUGGGUGAC) (SEQ ID NO:413) gRNA were introduced to the cells by electroporation. In the same experiment, we also targeted the *TRAC* gene by introducing 10ug of Cas9 mRNA with 10ug of TRAC-4 (GCUGGUACACGGCAGGGUCA) (SEQ ID NO:453) gRNA. A gRNA targeting the AAVS1 (GUCCCCUCCACCCCACAGUG) (SEQ ID NO:51201) genomic site was used as an experimental control. Prior to electroporation, the T cells were cultured in RPMI 1640 supplemented with 10% FBS and recombinant IL-2. The cells were activated using CD3/CD28 beads and expanded for at least 3 days. Subsequent to introduction of the mRNA to the activated T cells, CD3 expression on the cells was monitored at 24, 48 and 72 hours post electroporation by flow cytometry using a fluorescein (APC) conjugated antibody specific for CD3. At 72 hours, a population of CD3 negative cells was observed (**Fig. 17A** and **Fig. 17B**). To confirm that the generation of CD3 negative cells was a result of genome editing at the *TRBC* loci, genomic DNA was harvested and a T7E1 assay was performed. Indeed, the data confirm the presence of DNA modifications at the *TRBC2* locus and *TRAC* locus (Fig. 17C).

### Delivery of Cas9/gRNA RNP to T cells

To demonstrate Cas9-mediated cutting in Jurkat T cells, *S. aureus* Cas9 and a gRNA designed against the TCR alpha chain (TRAC-233 (GUGAAUAGGCAGACAGACUUGUCA) (SEQ ID NO:474)) were delivered as a ribonucleic acid protein complex (RNP) by electroporation. In this embodiment, the Cas9 was expressed in *E. coli* and purified. Specifically, the HJ29 plasmid encoding Cas9 was transformed into Rosetta™ 2 (DE3) chemically competent cells (EMD Millipore #71400-4) and plated onto LB plates with appropriate antibiotics for selection and incubated at 37°C overnight. A 10mL starter culture of Brain Heart Infusion Broth (Tcknova #B9993) with appropriate antibiotics was inoculated with 4 colonies and grown at 37°C with shaking at 220rpms. After growing overnight, the starter culture was added to 1L of Terrific Broth Complete (Teknova #T7060) with appropriate antibiotics plus supplements and grown at 37°C with shaking at 220rpms. The temperature was gradually reduced to 18°C and expression of the gene was induced by addition of IPTG to 0.5mM when the OD600 was greater than 2.0. The induction was allowed to continue overnight followed by harvesting the cells by centrifugation and resuspension in TG300 (50mM Tris pH8.0, 300mM NaCl, 20% glycerol, 1mM TCEP, protease inhibitor tablets (Thermo Scientific #88266)) and stored at -80°C.

The cells were lysed by thawing the frozen suspension, followed by two passes through a LM10 Microfluidizer® set to 18000psi. The extract was clarified via centrifugation and the soluble extract was captured via batch incubation with Ni-NTA Agarose resin (Qiagen #30230) at 4°C. The slurry was poured into a gravity flow column, washed with TG300+30mM Imidazole and then eluted the protein of interest with TG300+300mM Imidazole. The Ni eluent was diluted with an equal volume of HG100 (50mM Hepes pH7.5, 100mM NaCl, 10% glycerol, 0.5mM TCEP) and loaded onto a HiTrap SP HP column (GE Healthcare Life Sciences #17-1152-01) and eluted with a 30 column volume gradient from HG100 to HG1000 (50mM Hepes pH7.5, 1000mM NaCl, 10% glycerol, 0.5mM TCEP). Appropriate fractions were pooled after assaying with an SDS-PAGE gel and concentrated for loading onto a SRT10 SEC300 column (Sepax #225300-21230) equilibrated in HG150 (10mM Hepes pH7.5, 150mM NaCl, 20% glycerol, 1mM TCEP). Fractions were assayed by SDS-PAGE and appropriately pooled, concentrated to at least 5mg/ml.

The gRNA was generated by *in vitro* transcription using a T7 polymerase. A 5' ARCA cap was added to the RNA simultaneous to transcription while a polyA tail was added after transcription to the 3' end of the RNA species by an *E. coli* polyA polymerase. Prior to introduction into the cells, the purified Cas9 and gRNA were mixed and allowed to form complexes for 10 minutes. The RNP solution was subsequently introduced into Jurkat T cells by electroporation. Prior to and after electroporation, the cells were cultured in RPMI1640 media supplemented with 10% FBS. CD3 expression on the cells was monitored at 24, 48 and 72 hours post electroporation by flow cytometry using a fluorescein conjugated antibody specific for CD3. At 48 and 72 hours, a population of CD3 negative cells was observed (Fig. **18A** and **Fig. 18B**). To confirm that the generation of CD3 negative cells was a result of genome editing at the *TRAC* locus, genomic DNA was harvested and a T7E1 assay was performed. Indeed, the data confirm the presence of DNA modifications at the *TRAC* locus (**Fig. 18C**).

### Example 5: Assessing effect of gRNA modifications on T cell viability

In order to assess how gRNA modifications affect T cell viability, *S. pyogenes* Cas9 mRNA was delivered to Jurkat T cells in combination with an AAVS1 gRNA (GUCCCCUCCACCCCACAGUG) (SEQ ID NO:51201) with or without modification. Specifically, 4 different combinations of modification were analyzed, (1) gRNA with a 5' Anti-Reverse Cap Analog (ARCA) cap (see **Fig. 19**) and polyA tail, (2) gRNA with a 5' ARCA cap only, (3) gRNA with a polyA tail only, (4) gRNA without any modification. In order to generate all of four of the aforemention versions of modified gRNAs, a DNA template comprising a T7 promoter, AAVS1 gRNA target sequence (GUCCCCUCCACCCCACAGUG) (SEQ ID NO:51201), and *S. pyogenes* TRACR sequence. For all gRNAs, T7 polymerase was used to generate the gRNA in the presence of 7.5 mM UTP, 7.5 mM GTP, 7.5 mM CTP and 7.5 mM ATP. In order to modify the gRNAs with a 5' ARCA cap, 6.0 mM of ARCA analog was added to the NTP pool. Consequently, only 1.5 mM of GTP was added while the rest of the NTP pool remained at the same concentration: 7.5 mM UTP, 7.5 mM CTP and 7.5 mM ATP. In order to add a polyA tail to the gRNAs a recombinant polyA polymerase that was purified from *E. coli* was used to add a series of As to the end of the transcribed gRNA after the in vitro polymerase reaction was terminated. Termination was achieved by eliminating the DNA template with DNase I. The polyA tail reaction was performed for approximately 40 minutes. Regardless of the gRNA modification, all gRNA preparations were purified by phenol:choroform extraction followed by isopropanol precipitation. Once the gRNAs were generated, Jurkat T cells were electroporated with *S. pyogenes* Cas9 mRNA (modified with a 5' ARCA cap and polyA tail) and one of the 4 different modified AAVS1-specific gRNAs. Following electroporation cell viability was determined by performing an Annexin-V and propidium iodide double stain. The fraction of live cells, which do not stain for Annexin-V and PI was determined by flow cytometry. The results are quantified in **Fig. 20****.** Based on the fraction of live cells, it is concluded that gRNAs that have been modified wuth both a 5' ARCA cap and polyA tail are the least toxic to Jurkat T cells when introduced by electroporation.

### Example 6: Delivery of Cas9/sRNA RNP to naive T cells

To demonstrate Cas9-mediated cutting in naive T cells, *S. aureus* Cas9 and a gRNA designed against the TCR alpha chain with targeting domain GUGAAUAGGCAGACAGACUUGUCA (SEQ ID NO:474) were delivered as a ribonucleic acid protein complex (RNP) by electroporation. In this embodiment, the Cas9 was expressed in E. coli and purified. Specifically, the HJ29 plasmid encoding Cas9 was transformed into Rosetta™ 2 (DE3) chemically competent cells (EMD Millipore #71400-4) and plated onto LB plates with appropriate antibiotics for selection and incubated at 37°C overnight. A 10mL starter culture of Brain Heart Infusion Broth (Tcknova #B9993) with appropriate antibiotics was inoculated with 4 colonies and grown at 37°C with shaking at 220rpms. After growing overnight, the starter culture was added to 1L of Terrific Broth Complete (Teknova #T7060) with appropriate antibiotics plus supplements and grown at 37°C with shaking at 220rpms. The temperature was gradually reduced to 18°C and expression of the gene was induced by addition of IPTG to 0.5mM when the OD600 was greater than 2.0. The induction was allowed to continue overnight followed by harvesting the cells by centrifugation and resuspension in TG300 (50mM Tris pH8.0, 300mM NaCl, 20% glycerol, 1mM TCEP, protease inhibitor tablets (Thermo Scientific #88266)) and stored at -80°C.

The cells were lysed by thawing the frozen suspension, followed by two passes through a LM10 Microfluidizer® set to 18000psi. The extract was clarified via centrifugation and the soluble extract was captured via batch incubation with Ni-NTA Agarose resin (Qiagen #30230) at 4°C. The slurry was poured into a gravity flow column, washed with TG300+30mM Imidazole and then eluted the protein of interest with TG300+300mM Imidazole. The Ni eluent was diluted with an equal volume of HG100 (50mM Hepes pH7.5, 100mM NaCl, 10% glycerol, 0.5mM TCEP) and loaded onto a HiTrap SP HP column (GE Healthcare Life Sciences #17-1152-01) and eluted with a 30 column volume gradient from HG100 to HG1000 (50mM Hepes pH7.5, 1000mM NaCl, 10% glycerol, 0.5mM TCEP). Appropriate fractions were pooled after assaying with an SDS-PAGE gel and concentrated for loading onto a SRT10 SEC300 column (Sepax #225300-21230) equilibrated in HG150 (10mM Hepes pH7.5, 150mM NaCl, 20% glycerol, 1mM TCEP). Fractions were assayed by SDS-PAGE and appropriately pooled, concentrated to at least 5mg/ml.

The gRNA with targeting domain GUGAAUAGGCAGACAGACUUGUCA (SEQ ID NO:474) was generated by in vitro transcription using a T7 polymerase. A 5' ARCA cap was added to the RNA simultaneous to transcription while a polyA tail was added after transcription to the 3' end of the RNA species by an E. coli polyA polymerase. In this embodiment, the T cells were isolated from fresh cord blood by a Ficoll gradient followed by positive selection using CD3 magnetic beads. The cells were subsequently cultured in RPMI1640 media supplemented with 10% FBS, IL-7 (5ng/ml) and IL-15 (5ng/ml). 24 hours after isolation, the cells were electroporated with a RNP solution that was generated by incubating the purified Cas9 and gRNA for 10 minutes at room temperature. CD3 expression on the cells was monitored at 96 hours post electroporation by flow cytometry using an APC-conjugated antibody specific for CD3. A population of CD3 negative cells was observed in cells that in which a functional RNP complex was provided versus a negative control that received the gRNA and a non-functional Cas9 (**Fig**. **21A** and **Fig. 21B**). To confirm that the generation of CD3 negative cells was a result of genome editing at the *TRAC* locus, genomic DNA was harvested and a T7E1 assay was performed. Indeed, the data confirm the presence of DNA modifications at the *TRAC* locus (**Fig. 21C**).

### Example 7: Targeting of the PDCD1 locus by delivery of Cas9 mRNA and gRNA as RNA molecules or as Cas9/sRNA RNP to Jurkat T cells

### Delivery of Cas9 mRNA and gRNA as RNA molecules to Jurkat T cells

To demonstrate Cas9-mediated cutting at the *PDCD1* locus in Jurkat T cells, *S. pyogenes* Cas9 and a gRNA with a targeting domain GUCUGGGCGGUGCUACAACU (SEQ ID NO:508) designed against the *PDCD1* locus were delivered as RNA molecules to T cells via electroporation. In this embodiment, both the Cas9 and gRNA were in vitro transcribed using a T7 polymerase. A 5' ARCA cap was added to both RNA species simultaneous to transcription while a polyA tail was added after transcription to the 3' end of the RNA species by an E. coli polyA polymerase. To generate Jurkat T cells modified at the *PDCD1* locus, 10ug of Cas9 mRNA and 10ug of gRNA with a targeting domain GUCUGGGCGGUGCUACAACU (SEQ ID NO:508) were introduced to the cells by electroporation. Prior to electroporation, the T cells were cultured in RPMI 1640 supplemented with 10% FBS. At 24, 48 and 72 hours, genomic DNA was isolated and a T7E1 assay was performed at the *PDCD1* locus. Indeed, the data confirm the presence of DNA modifications at the *PDCD1* locus (**Fig. 22**).

### Delivery of Cas9/gRNA RNP to Jurkat T cells

To demonstrate Cas9-mediated cutting at the *PDCD1* locus in Jurkat T cells, *S. pyogenes* Cas9 and a gRNA designed against the *PDCD1* locus with the targeting domain GUCUGGGCGGUGCUACAACU (SEQ ID NO:508) were delivered as a ribonucleic acid protein complex (RNP) by electroporation. In this embodiment, the Cas9 was expressed in E. coli and purified. Specifically, the HJ29 plasmid encoding Cas9 was transformed into Rosetta™ 2 (DE3) chemically competent cells (EMD Millipore #71400-4) and plated onto LB plates with appropriate antibiotics for selection and incubated at 37°C overnight. A 10mL starter culture of Brain Heart Infusion Broth (Teknova #B9993) with appropriate antibiotics was inoculated with 4 colonies and grown at 37°C with shaking at 220rpms. After growing overnight, the starter culture was added to 1L of Terrific Broth Complete (Teknova #T7060) with appropriate antibiotics plus supplements and grown at 37°C with shaking at 220rpms. The temperature was gradually reduced to 18°C and expression of the gene was induced by addition of IPTG to 0.5mM when the OD600 was greater than 2.0. The induction was allowed to continue overnight followed by harvesting the cells by centrifugation and resuspension in TG300 (50mM Tris pH8.0, 300mM NaCl, 20% glycerol, 1mM TCEP, protease inhibitor tablets (Thermo Scientific #88266)) and stored at -80°C.

The cells were lysed by thawing the frozen suspension, followed by two passes through a LM10 Microfluidizer® set to 18000psi. The extract was clarified via centrifugation and the soluble extract was captured via batch incubation with Ni-NTA Agarose resin (Qiagen #30230) at 4°C. The slurry was poured into a gravity flow column, washed with TG300+30mM Imidazole and then eluted the protein of interest with TG300+300mM Imidazole. The Ni eluent was diluted with an equal volume of HG100 (50mM Hepes pH7.5, 100mM NaCl, 10% glycerol, 0.5mM TCEP) and loaded onto a HiTrap SP HP column (GE Healthcare Life Sciences #17-1152-01) and eluted with a 30 column volume gradient from HG100 to HG1000 (50mM Hepes pH7.5, 1000mM NaCl, 10% glycerol, 0.5mM TCEP). Appropriate fractions were pooled after assaying with an SDS-PAGE gel and concentrated for loading onto a SRT10 SEC300 column (Sepax #225300-21230) equilibrated in HG150 (10mM Hepes pH7.5, 150mM NaCl, 20% glycerol, 1mM TCEP). Fractions were assayed by SDS-PAGE and appropriately pooled, concentrated to at least 5mg/ml.

The gRNA with the targeting domain GUCUGGGCGGUGCUACAACU (SEQ ID NO:508) was generated by in vitro transcription using a T7 polymerase. A 5' ARCA cap was added to the RNA simultaneous to transcription while a polyA tail was added after transcription to the 3' end of the RNA species by an E. coli polyA polymerase. Prior to introduction into the cells, the purified Cas9 and gRNA were mixed and allowed to form complexes for 10 minutes. The RNP solution was subsequently introduced into Jurkat T cells by electroporation. Prior to and after electroporation, the cells were cultured in RPMI1640 media supplemented with 10% FBS. At 24, 48 and 72 hours, genomic DNA was isolated and a T7E1 assay was performed at the *PDCD1* locus. Indeed, the data confirm the presence of DNA modifications at the *PDCD1* locus (**Fig. 22**).

### INCORPORATION BY REFERENCE

All publications, patents, sequence listings, and patent applications mentioned herein are hereby incorporated by reference in their entirety as if each individual publication, patent, sequence listing, or patent application was specifically and individually indicated to be incorporated by reference. In case of conflict, the present application, including any definitions herein, will control.

### EQUIVALENTS

Those skilled in the art will recognize, or be able to ascertain using no more than routine experimentation, many equivalents to the specific embodiments of the invention described herein. Such equivalents are intended to be encompassed by the following claims. Other embodiments are also within the following claims.

The invention will now be defined by reference to the following clauses:
1. A gRNA molecule comprising a targeting domain which is complementary with a target domain from the *FAS, BID, CTLA4, PDCD1, CBLB, PTPN6, TRAC* or *TRBC* gene.
2. The gRNA molecule of clause 1, wherein the targeting domain is configured to provide a cleavage event selected from a double strand break and a single strand break, within 500, 400, 300, 200, 100, 50, 25, or 10 nucleotides of a T cell target knockout position.
3. The gRNA molecule of clause 1, wherein the targeting domain is configured to target an enzymatically inactive Cas9 (eiCas9) or an eiCas9 fusion protein, sufficiently close to a T cell target knockdown position to reduce, decrease or repress expression of the *FAS, BID, CTLA4, PDCD1, CBLB,* or *PTPN6* gene.
4. The gRNA molecule of clause 3, wherein the targeting domain is configured to target the promoter region of the *FAS, BID, CTLA4, PDCD1, CBLB, or PTPN6* gene.
5. The gRNA molecule of clause 1, wherein the targeting domain comprises a sequence that is the same as, or differs by no more than 3 nucleotides from, a targeting domain sequence from any of Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
6. The gRNA molecule of clause 1, wherein the targeting domain is selected from those in Tables 1A-F or Tables 13A-K.
7. The gRNA molecule of clause 1, wherein the targeting domain is selected from those in Tables 2A-I or Tables 14A-K.
8. The gRNA molecule of clause 1, wherein the targeting domain is selected from those in Tables 3A-H or Tables 15A-F.
9. The gRNA molecule of clause 1, wherein the targeting domain is selected from those in Tables 4A-I or Tables 16A-K.
10. The gRNA molecule of clause 1, wherein the targeting domain is selected from those in Tables 5A-I or Tables 17A-K.
11. The gRNA molecule of clause 1, wherein the targeting domain is selected from those in Tables 6A-I or Tables 18A-K.
12. The gRNA molecule of clause 1, wherein the targeting domain is selected from those in Tables 7A-H, Tables 19A-J, Table 31 or Table 32.
13. The gRNA molecule of clause 1, wherein the targeting domain is selected from those in Tables 8A-H or Tables 20A-J.
14. The gRNA molecule of clause 1, wherein the targeting domain is selected from those in Tables 9A-I or Tables 21A-K.
15. The gRNA molecule of clause 1, wherein the targeting domain is selected from those in Tables 10A-I or Tables 22A-K.
16. The gRNA molecule of clause 1, wherein the targeting domain is selected from those in Tables 11A-I or Tables 23A-J.
17. The gRNA molecule of clause 1, wherein the targeting domain is selected from those in Tables 12A-I or Tables 24A-K.
18. The gRNA molecule of clause 1, wherein the targeting domain is selected from those in Tables 25A-G or Table 29.
19. The gRNA molecule of clause 1, wherein the targeting domain is selected from those in Tables 26A-G or Table 27.
20. The gRNA molecule of any of clauses 1-19, wherein the gRNA is a modular gRNA molecule.
21. The gRNA molecule of any of clauses 1-19, wherein the gRNA is a chimeric gRNA molecule.
22. The gRNA molecule of any of clauses 1-19, wherein the targeting domain is 16 or 17 nucleotides or more in length.
23. The gRNA molecule of any of clauses 1-19, wherein the targeting domain is 16 or 17 nucleotides in length.
24. The gRNA molecule of any of clauses 1-19, wherein the targeting domain is 18 nucleotides in length.
25. The gRNA molecule of any of clauses 1-19, wherein the targeting domain is 19 nucleotides in length.
26. The gRNA molecule of any of clauses 1-19, wherein the targeting domain is 20 nucleotides in length.
27. The gRNA molecule of any of clauses 1-19, wherein the targeting domain is 21 nucleotides in length.
28. The gRNA molecule of any of clauses 1-19, wherein the targeting domain is 22 nucleotides in length.
29. The gRNA molecule of any of clauses 1-19, wherein the targeting domain is 23 nucleotides in length.
30. The gRNA molecule of any of clauses 1-19, wherein the targeting domain is 24, 25, or 26 nucleotides in length.
31. The gRNA molecule of any of clauses 1-30, comprising from 5' to 3':
   a targeting domain;
   a first complementarity domain;
   a linking domain;
   a second complementarity domain;
   a proximal domain; and
   a tail domain.
32. The gRNA molecule of any of clauses 1-30, comprising:
   a linking domain of no more than 25 nucleotides in length;
   a proximal and tail domain, that taken together, are at least 20 nucleotides in length; and
   a targeting domain of 17, 18, 19, 20, 21, 22, 23 or 24 nucleotides in length.
33. The gRNA molecule of any of clauses 1-30, comprising:
   a linking domain of no more than 25 nucleotides in length;
   a proximal and tail domain, that taken together, are at least 30 nucleotides in length; and
   a targeting domain of 17, 18, 19, 20, 21, 22, 23 or 24 nucleotides in length.
34. The gRNA molecule of any of clauses 1-30, comprising:
   a linking domain of no more than 25 nucleotides in length;
   a proximal and tail domain, that taken together, are at least 35 nucleotides in length; and
   a targeting domain of 17, 18, 19, 20, 21, 22, 23 or 24 nucleotides in length.
35. The gRNA molecule of any of clauses 1-30, comprising:
   a linking domain of no more than 25 nucleotides in length;
   a proximal and tail domain, that taken together, are at least 40 nucleotides in length; and
   a targeting domain of 17, 18, 19, 20, 21, 22, 23 or 24 nucleotides in length.
36. A nucleic acid that comprises: (a) sequence that encodes a gRNA molecule comprising a targeting domain that is complementary with a T cell target domain in the *FAS, BID, CTLA4, PDCD1, CBLB, PTPN6, TRAC* or *TRBC* gene.
37. The nucleic acid of clause 36, wherein the gRNA molecule is a gRNA molecule of any of clauses 1-35.
38. The nucleic acid of clause 36, wherein the targeting domain is configured to provide a cleavage event selected from a double strand break and a single strand break, within 500, 400, 300, 200, 100, 50, 25, or 10 nucleotides of the T cell target knockout position.
39. The nucleic acid of clause 36, wherein the targeting domain is configured to target an enzymatically inactive Cas9 (eiCas9) or an eiCas9 fusion protein, sufficiently close to a T cell target knockdown position to reduce, decrease or repress expression of the *FAS, BID, CTLA4, PDCD1, CBLB,* or *PTPN6* gene.
40. The nucleic acid of clause 36, wherein the targeting domain is configured to target the promoter region of the *FAS, BID, CTLA4, PDCD1, CBLB,* or *PTPN6* gene.
41. The nucleic acid of clause 36, wherein the targeting domain comprises a sequence that is the same as, or differs by no more than 3 nucleotides from, a targeting domain sequence from any of Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
42. The nucleic acid of clause 36, wherein the targeting domain is selected from those in Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
43. The nucleic acid of any of clauses 36-42, wherein the gRNA is a modular gRNA molecule.
44. The nucleic acid of any of clauses 36-42, wherein the gRNA is a chimeric gRNA molecule.
45. The nucleic acid of any of clauses 36-42, wherein the targeting domain is 16 or 17 nucleotides or more in length.
46. The nucleic acid of any of clauses 36-42, wherein the targeting domain is 16 or 17 nucleotides in length.
47. The nucleic acid of any of clauses 36-42, wherein the targeting domain is 18 nucleotides in length.
48. The nucleic acid of any of clauses 36-42, wherein the targeting domain is 19 nucleotides in length.
49. The nucleic acid of any of clauses 36-42, wherein the targeting domain is 20 nucleotides in length.
50. The nucleic acid of any of clauses 36-42, wherein the targeting domain is 21 nucleotides in length.
51. The nucleic acid of any of clauses 36-42, wherein the targeting domain is 22 nucleotides in length.
52. The nucleic acid of any of clauses 36-42, wherein the targeting domain is 23 nucleotides in length.
53. The nucleic acid of any of clauses 36-42, wherein the targeting domain is 24, 25, or 26 nucleotides in length.
54. The nucleic acid of any of clauses 36-54, comprising from 5' to 3':
   a targeting domain;
   a first complementarity domain;
   a linking domain;
   a second complementarity domain;
   a proximal domain; and
   a tail domain.
55. The nucleic acid of any of clauses 36-54, comprising:
   a linking domain of no more than 25 nucleotides in length;
   a proximal and tail domain, that taken together, are at least 20 nucleotides in length; and
   a targeting domain of 17, 18, 19, 20, 21, 22, 23 or 24 nucleotides in length.
56. The nucleic acid of any of clauses 36-54, comprising:
   a linking domain of no more than 25 nucleotides in length;
   a proximal and tail domain, that taken together, are at least 30 nucleotides in length; and
   a targeting domain of 17, 18, 19, 20, 21, 22, 23 or 24 nucleotides in length.
57. The nucleic acid of any of clauses 36-54, comprising:
   a linking domain of no more than 25 nucleotides in length;
   a proximal and tail domain, that taken together, are at least 35 nucleotides in length; and
   a targeting domain of 17, 18, 19, 20, 21, 22, 23 or 24 nucleotides in length.
58. The nucleic acid of any of clauses 36-54, comprising:
   a linking domain of no more than 25 nucleotides in length;
   a proximal and tail domain, that taken together, are at least 40 nucleotides in length; and
   a targeting domain of 17, 18, 19, 20, 21, 22, 23 or 24 nucleotides in length.
59. The nucleic acid of any of clauses 36-58, further comprising: (b) sequence that encodes a Cas9 molecule.
60. The nucleic acid of clause 59, wherein the Cas9 molecule is an eaCas9 molecule.
61. The nucleic acid of clause 60, wherein the eaCas9 molecule comprises a nickase molecule.
62. The nucleic acid of clause 60, wherein the eaCas9 molecule forms a double strand break in a target nucleic acid.
63. The nucleic acid of clause 60, wherein the eaCas9 molecule forms a single strand break in a target nucleic acid.
64. The nucleic acid of clause 63, wherein the single strand break is formed in the strand of the target nucleic acid to which the targeting domain of the gRNA molecule is complementary.
65. The nucleic acid of clause 63, wherein the single strand break is formed in the strand of the target nucleic acid other than the strand to which to which the targeting domain of the gRNA is complementary.
66. The nucleic acid of clause 60, wherein the eaCas9 molecule comprises HNH-like domain cleavage activity but has no, or no significant, N-terminal RuvC-like domain cleavage activity.
67. The nucleic acid of clause 60, wherein the eaCas9 molecule is an HNH-like domain nickase.
68. The nucleic acid of clause 60, wherein the eaCas9 molecule comprises a mutation at D10.
69. The nucleic acid of clause 60, wherein the eaCas9 molecule comprises N-terminal RuvC-like domain cleavage activity but has no, or no significant, HNH-like domain cleavage activity.
70. The nucleic acid of clause 60, wherein the eaCas9 molecule is an N-terminal RuvC-like domain nickase.
71. The nucleic acid of clause 60, wherein the eaCas9 molecule comprises a mutation at H840.
72. The nucleic acid of clause 59, wherein the Cas9 molecule is an eiCas9 molecule.
73. The nucleic acid of clause 72, wherein the Cas9 molecule is an eiCas9-fusion protein molecule (e.g., an eiCas9-transcription repressor domain fusion, e.g., an eiCas9-KRAB domain fusion).
74. The nucleic acid of any of clauses 36-73, further comprising: (c) sequence that encodes a second gRNA molecule described herein having a targeting domain that is complementary to a second target domain of the *FAS, BID, CTLA4, PDCD1, CBLB, PTPN6, TRAC* or *TRBC* gene.
75. The nucleic acid of clause 74, wherein the second gRNA molecule is a gRNA molecule of any of clauses 1-35.
76. The nucleic acid of clause 74, wherein the targeting domain of the second gRNA is configured to provide a cleavage event selected from a double strand break and a single strand break, within 500, 400, 300, 200, 100, 50, 25, or 10 nucleotides of the T cell target knockout position.
77. The nucleic acid of clause 74, wherein the targeting domain of the second gRNA is configured to target an enzymatically inactive Cas9 (eiCas9) or an eiCas9 fusion protein, sufficiently close to a T cell target knockdown position to reduce, decrease or repress expression of the *FAS, BID, CTLA4, PDCD1, CBLB,* or *PTPN6* gene.
78. The nucleic acid of clause 74, wherein the targeting domain of the second gRNA is configured to target the promoter region of the *FAS, BID, CTLA4, PDCD1, CBLB,* or *PTPN6* gene.
79. The nucleic acid of clause 74, wherein the targeting domain of the second gRNA comprises a sequence that is the same as, or differs by no more than 3 nucleotides from, a targeting domain sequence from any of Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
80. The nucleic acid of clause 74, wherein the targeting domain of the second gRNA is selected from those in Tables 1A-I, Tables 2A-I, Tables 3A-H, Tables 4A-I, Tables 5A-I, Tables 6A-I, Tables 7AH, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
81. The nucleic acid of any of clauses 74-80, wherein the second gRNA molecule is a modular gRNA molecule.
82. The nucleic acid of any of clauses 74-80, wherein the second gRNA molecule is a chimeric gRNA molecule.
83. The nucleic acid of any of clauses 74-80, wherein the targeting domain is 16 or 17 nucleotides or more in length.
84. The nucleic acid of any of clauses 74-80, wherein the targeting domain is 16 or 17 nucleotides in length.
85. The nucleic acid of any of clauses 74-80, wherein the targeting domain is 18 nucleotides in length.
86. The nucleic acid of any of clauses 74-80, wherein the targeting domain is 19 nucleotides in length.
87. The nucleic acid of any of clauses 74-80, wherein the targeting domain is 20 nucleotides in length.
88. The nucleic acid of any of clauses 74-80, wherein the targeting domain is 21 nucleotides in length.
89. The nucleic acid of any of clauses 74-80, wherein the targeting domain is 22 nucleotides in length.
90. The nucleic acid of any of clauses 74-80, wherein the targeting domain is 23 nucleotides in length.
91. The nucleic acid of any of clauses 74-80, wherein the targeting domain is 24, 25, or 26 nucleotides in length.
92. The nucleic acid of any of clauses 74-91, wherein the second gRNA molecule comprises from 5' to 3':
   a targeting domain;
   a first complementarity domain;
   a linking domain;
   a second complementarity domain;
   a proximal domain; and
   a tail domain.
93. The nucleic acid of any of clauses 74-92, wherein the second gRNA molecule comprises:
   a linking domain of no more than 25 nucleotides in length;
   a proximal and tail domain, that taken together, are at least 20 nucleotides in length; and
   a targeting domain of 17, 18, 19, 20, 21, 22, 23 or 24 nucleotides in length.
94. The nucleic acid of any of clauses 74-92, wherein the second molecule gRNA molecule comprises:
   a linking domain of no more than 25 nucleotides in length;
   a proximal and tail domain, that taken together, are at least 30 nucleotides in length; and
   a targeting domain of 17, 18, 19, 20, 21, 22, 23 or 24 nucleotides in length.
95. The nucleic acid of any of clauses 74-92, wherein the second gRNA molecule comprises:
   a linking domain of no more than 25 nucleotides in length;
   a proximal and tail domain, that taken together, are at least 35 nucleotides in length; and
   a targeting domain of 17, 18, 19, 20, 21, 22, 23 or 24 nucleotides in length.
96. The nucleic acid of any of clauses 74-92, wherein the second gRNA molecule comprises:
   a linking domain of no more than 25 nucleotides in length;
   a proximal and tail domain, that taken together, are at least 40 nucleotides in length; and
   a targeting domain of 17, 18, 19, 20, 21, 22, 23 or 24 nucleotides in length.
97. The nucleic acid of any of clauses 74-96, further comprising a third gRNA molecule.
98. The nucleic acid of clause 97, further comprising a fourth gRNA molecule.
99. The nucleic acid of clause 36, further comprising: (b) sequence that encodes a Cas9 molecule of any of clauses 59-73.
100. The nucleic acid of clause 99, wherein the nucleic acid does not comprise (c) a sequence that encodes a second gRNA molecule.
101. The nucleic acid of clause 100, wherein each of (a) and (b) is present on the same nucleic acid molecule.
102. The nucleic acid of clause 100, wherein the nucleic acid molecule is an AAV vector.
103. The nucleic acid of clauses 101, wherein: (a) is present on a first nucleic acid molecule; and (b) is present on a second nucleic acid molecule.
104. The nucleic acid of clause 103, wherein the first and second nucleic acid molecules are AAV vectors.
105. The nucleic acid of clause 36, further comprising:
   (b) sequence that encodes a Cas9 molecule of any of clauses 59-73; and
   (c) sequence that encode a second gRNA molecule of clams 74-96.
106. The nucleic acid of clause 105, wherein each of (a), (b), and (c) are present on the same nucleic acid molecule.
107. The nucleic acid of clause 106, wherein the nucleic acid molecule is an AAV vector.
108. The nucleic acid of clause 105, wherein:
   one of (a), (b), and (c) is encoded on a first nucleic acid molecule; and
   a second and third of (a), (b), and (c) is encoded on a second nucleic acid molecule.
109. The nucleic acid of clause 108, wherein the first and second nucleic acid molecules are AAV vectors.
110. The nucleic acid of clause 108, wherein: (a) is present on a first nucleic acid molecule; and (b) and (c) are present on a second nucleic acid molecule.
111. The nucleic acid of clause 110, wherein the first and second nucleic acid molecules are AAV vectors.
112. The nucleic acid of clause 108, wherein: (b) is present on a first nucleic acid molecule; and (a) and (c) are present on a second nucleic acid molecule.
113. The nucleic acid of clause 112, wherein the first and second nucleic acid molecules are AAV vectors.
114. The nucleic acid of clause 108, wherein: (c) is present on a first nucleic acid molecule; and (b) and (a) are present on a second nucleic acid molecule.
115. The nucleic acid of clause 114, wherein the first and second nucleic acid molecules are AAV vectors.
116. The nucleic acid of any of clauses 103, 108, 110, 112, or 114, wherein the first nucleic acid molecule is other than an AAV vector and the second nucleic acid molecule is an AAV vector.
117. The nucleic acid of any of clauses 36-58, wherein the nucleic acid comprises a promoter operably linked to the sequence that encodes the gRNA molecule of (a).
118. The nucleic acid of any of clauses 74-96, wherein the nucleic acid comprises a second promoter operably linked to the sequence that encodes the second gRNA molecule of (c).
119. The nucleic acid of any of clauses 117 or 118, wherein the promoter and second promoter differ from one another.
120. The nucleic acid of any of clauses 117 or 118, wherein the promoter and second promoter are the same.
121. The nucleic acid of any of clauses 59-73, wherein the nucleic acid comprises a promoter operably linked to the sequence that encodes the Cas9 molecule of (b).
122. The nucleic acid of any of clauses 36-96, further comprising a gRNA molecule targeting a second gene selected from the group consisting of *FAS, BID, CTLA4, PDCD1, CBLB, PTPN6, TRAC* and *TRBC* genes.
123. The nucleic acid of any of clauses 36-96, further comprising a gRNA molecule targeting at least one additional gene selected from the group consisting of *FAS, BID, CTLA4, PDCD1, CBLB, PTPN6, TRAC* and *TRBC* genes.
124. A composition comprising the (a) gRNA molecule of any of clauses 1-35.
125. The composition of clause 124, further comprising (b) a Cas9 molecule of any of clauses 59-73.
126. The composition of any of clauses 124 or 125, further comprising (c) a second gRNA molecule of any of clauses 74-96.
127. The composition of clause 126, further comprising a third gRNA molecule.
128. The composition of clause 127, further comprising a fourth gRNA molecule.
129. A composition comprising at least two gRNA molecules to target two or more of *FAS, BID, CTLA4, PDCD1, CBLB, PTPN6, TRAC* and *TRBC* genes.
130. The composition of clause 129, wherein the at least two gRNA molecules to two or more of *FAS, BID, CTLA4, PDCD1, CBLB, PTPN6, TRAC* and *TRBC* genes comprise a sequence that is the same as, or differs by no more than 3 nucleotides from, a targeting domain sequence from any of Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
131. The composition of clause 129, wherein the at least two gRNA molecules to two or more of *FAS, BID, CTLA4, PDCD1, CBLB, PTPN6, TRAC* and *TRBC* genes are selected from those in Tables 1A-I, Tables 2A-I, Tables 3A-H, Tables 4A-I, Tables 5A-I, Tables 6A-I, Tables 7A-H, Tables 8A-H, Tables 9A-I, Tables 10A-I, Tables 11A-I, Tables 12A-I, Tables 13A-K, Tables 14A-K, Tables 15A-F, Tables 16A-K, Tables 17A-K, Tables 18A-K, Tables 19A-J, Tables 20A-J, Tables 21A-K, Tables 22A-K, Tables 23A-J, Tables 24A-K, Tables 25A-G, Tables 26A-G, Table 27, Table 29, Table 31, or Table 32.
132. The composition of any of clauses 127-129, further comprising (b) a Cas9 molecule of any of clauses 59-73.
133. The composition of any of clauses 129-132, further comprising (c) a second gRNA molecule of any of clauses 74-96 targeting two or more of *FAS, BID, CTLA4, PDCD1, CBLB, PTPN6, TRAC* and *TRBC* genes.
134. The composition of clause 133, further comprising a third gRNA molecule.
135. The composition of clause 134, further comprising a fourth gRNA molecule.
136. A method of altering a cell comprising contacting the cell with:
   (a) a gRNA of any of clauses 1-35;
   (b) a Cas9 molecule of any of clauses 59-73; and
   optionally, (c) a second gRNA molecule of any of clauses 74-96.
137. The method of clause 136, further comprising a third gRNA molecule.
138. The method of clause 137, further comprising a fourth gRNA molecule.
139. The method of any of clauses 136-138, comprising contacting the cell with (a), (b), and optionally (c).
140. The method of any of clauses 136-139, wherein the gRNA molecule of (a) is selected from any of clauses 1-35.
141. The method of any of clauses 136-140, wherein the cell is from a subject suffering from cancer.
142. The method of any of clauses 136-141, wherein the cell is from a subject having cancer or which could otherwise benefit from a mutation at a T cell target position of the *FAS, BID, CTLA4, PDCD1, CBLB, PTPN6, TRAC* or *TRBC* gene.
143. The method of any of clauses 136-142, wherein the cell is a T cell.
144. The method of clause 143, wherein the T cell is an engineered T cell.
145. The method of clause 144, wherein the engineered T cell is an engineered chimeric antigen receptor (CAR) T cell.
146. The method of clause 144, wherein the engineered T cell is an engineered TCR (T-cell receptor) T cell.
147. The method of clause 143, wherein the T cell is engineered to express a TCR or a CAR prior to introducing a T cell target position mutation in one or more of the *FAS, BID, CTLA4, PDCD1, CBLB, PTPN6, TRAC* or *TRBC* gene.
148. The method of clause 143, wherein the T cell is engineered to express a TCR or a CAR after introducing a T cell target position mutation in one or more of the *FAS, BID, CTLA4, PDCD1, CBLB, PTPN6, TRAC* or *TRBC* gene.
149. The method of clause 143, wherein the T cell is engineered to express a TCR or a CAR at the same time as introducing a T cell target position mutation in one or more of the *FAS, BID, CTLA4, PDCD1, CBLB, PTPN6, TRAC* or *TRBC* gene.
150. The method of clause 136-149, wherein the contacting is performed *ex vivo.*
151. The method of clause 136-150, wherein the contacted cell is returned to the subject's body.
152. The method of any of clauses 136-151, wherein the cell is from a subject that has cancer.
153. The method of any of clauses 136-152, comprising acquiring knowledge of the sequence of the T cell target position in the cell.
154. The method of clause 153, comprising acquiring knowledge of the sequence of the T cell target position in the cell by sequencing a portion of the *FAS, BID, CTLA4, PDCD1, CBLB, PTPN6, TRAC* or *TRBC* gene.
155. The method of any of clauses 136-154, comprising inducing a T cell target position mutation.
156. The method of any of clauses 136-155, wherein contacting comprises contacting the cell with a nucleic acid that encodes at least one of (a), (b), and (c).
157. The method of any of clauses 136-156, wherein contacting comprises contacting the cell with a nucleic acid of any of clauses 36-123.
158. The method of any of clauses 136-157, wherein contacting comprises delivering to the cell the Cas9 molecule of (b) and a nucleic acid which encodes (a) and optionally (c).
159. The method of any of clauses 136-158, wherein contacting comprises delivering to the cell the Cas9 molecule of (b), the gRNA molecule of (a) and optionally the second gRNA molecule of (c).
160. The method of any of clauses 136-159, wherein contacting comprises delivering to the cell the gRNA molecule of (a), optionally the second gRNA molecule of (c) and a nucleic acid that encodes the Cas9 molecule of (b).
161. A method of treating a subject, comprising contacting a subject (or a cell from the subject) with:
   (a) a gRNA of any of clauses 1-35;
   (b) a Cas9 molecule of any of clauses 59-73; and
   optionally, (c) a second gRNA of any of clauses 74-96.
162. The method of clause 161, further comprising a third gRNA molecule.
163. The method of clause 162, further comprising a fourth gRNA molecule.
164. The method of any of clauses 161-163, further comprising contacting the subject with (a), (b), and optionally (c).
165. The method of any of clauses 161-164, wherein the subject is suffering from cancer.
166. The method of clause 165, wherein the cancer is selected from the group consisting of: lymphoma, chronic lymphocytic leukemia (CLL), B cell acute lymphocytic leukemia (B-ALL), acute lymphoblastic leukemia, acute myeloid leukemia, non-Hodgkin's lymphoma (NHL), diffuse large cell lymphoma (DLCL), multiple myeloma, renal cell carcinoma (RCC), neuroblastoma, colorectal cancer, breast cancer, ovarian cancer, melanoma, sarcoma, prostate cancer, lung cancer, esophageal cancer, hepatocellular carcinoma, pancreatic cancer, astrocytoma, mesothelioma, head and neck cancer, and medulloblastoma.
167. The method of any of clauses 161-166, wherein the subject would benefit from a mutation at the T cell target position of the *FAS, BID, CTLA4, PDCD1, CBLB, PTPN6, TRAC* or *TRBC* gene.
168. The method of any of clauses 161-167, wherein the subject would benefit from a mutation in one or more of the *FAS, BID, CTLA4, PDCD1, CBLB, PTPN6, TRAC* and *TRBC* genes.
169. The method of any of clauses 161-167, wherein the subject would benefit from a mutation in two or more of the *FAS, BID, CTLA4, PDCD1, CBLB, PTPN6, TRAC* and *TRBC* genes.
170. The method of any of clauses 161-167, wherein the subject would benefit from a mutation in three or more of the *FAS, BID, CTLA4, PDCD1, CBLB, PTPN6, TRAC* and *TRBC* genes.
171. The method of any of clauses 161-167, wherein the subject would benefit from a mutation in four or more of the *FAS, BID, CTLA4, PDCD1, CBLB, PTPN6, TRAC* and *TRBC* genes.
172. The method of any of clauses 161-167, wherein the subject would benefit from a mutation in five or more of the *FAS, BID, CTLA4, PDCD1, CBLB, PTPN6, TRAC* and *TRBC* genes.
173. The method of any of clauses 161-167, wherein the subject would benefit from a mutation in six or more of the *FAS, BID, CTLA4, PDCD1, CBLB, PTPN6, TRAC* and *TRBC* genes.
174. The method of any of clauses 161-167, wherein the subject would benefit from a mutation in seven or more of the *FAS, BID, CTLA4, PDCD1, CBLB, PTPN6, TRAC* and *TRBC* genes.
175. The method of any of clauses 161-167, wherein the subject would benefit from a mutation in each of the *FAS, BID, CTLA4, PDCD1, CBLB, PTPN6, TRAC* and *TRBC* genes.
176. The method of any of clauses 161-175, comprising acquiring knowledge of the sequence of the T cell target position in the subject.
177. The method of clause 176, comprising acquiring knowledge of the sequence of the T cell target position in the subject by sequencing one or more of the *FAS, BID, CTLA4, PDCD1, CBLB, PTPN6, TRAC* or *TRBC* gene or a portion thereof.
178. The method of any of clauses 161-177, comprising inducing a T cell target position mutation in the *FAS, BID, CTLA4, PDCD1, CBLB, PTPN6, TRAC* or *TRBC* gene.
179. The method of any of clauses 161-178, wherein a cell of the subject is contacted *ex vivo* with (a), (b), and optionally (c).
180. The method of clause 179, wherein the cell of the subject is a T cell.
181. The method of clause 180, wherein the T cell is engineered to express a TCR or a CAR.
182. The method of clause 180, wherein the T cell is engineered to express a TCR or a CAR prior to inducing a T cell target position mutation in one or more of the *FAS, BID, CTLA4, PDCD1, CBLB, PTPN6, TRAC* or *TRBC* gene.
183. The method of clause 180, wherein the T cell is engineered to express a TCR or a CAR after to inducing a T cell target position mutation in one or more of the *FAS, BID, CTLA4, PDCD1, CBLB, PTPN6, TRAC* or *TRBC* gene.
184. The method of clause 180, wherein the T cell is engineered to express a TCR or a CAR at the same time as inducing a T cell target position mutation in one or more of the *FAS, BID, CTLA4, PDCD1, CBLB, PTPN6, TRAC* or *TRBC* gene.
185. The method of any of clauses 179-184, wherein the cell is returned to the subject's body.
186. The method of any of clauses 179-185, wherein treatment comprises introducing a cell into the subject's body, wherein the cell subject is contacted *ex vivo* with (a), (b), and optionally (c).
187. The method of any of clauses 161-186, wherein contacting comprises contacting the subject with a nucleic acid that encodes at least one of (a), (b), and (c).
188. The method of any of clauses 161-187, wherein contacting comprises contacting the subject with a nucleic acid of any of any of clauses 36-123.
189. The method of any of clauses 161-188, wherein contacting comprises delivering to the subject the Cas9 molecule of (b) and a nucleic acid which encodes and (a) and optionally (c).
190. The method of any of clauses 161-188, wherein contacting comprises delivering to the subject the Cas9 molecule of (b), and the gRNA of (a) and optionally the second gRNA of (c).
191. The method of any of clauses 161-188, wherein contacting comprises delivering to the subject the gRNA of (a), optionally the second gRNA of (c) and a nucleic acid that encodes the Cas9 molecule of (b).
192. A reaction mixture comprising a gRNA, a nucleic acid, or a composition described herein, and a cell from a subject having cancer, or a subject which would benefit from a mutation at a T cell target position in one or more of the *FAS, BID, CTLA4, PDCD1, CBLB, PTPN6, TRAC* or *TRBC* genes.
193. A kit comprising, (a) gRNA molecule of any of clauses 1-35, or nucleic acid that encodes the gRNA, and one or more of the following:
   (b) a Cas9 molecule of any of clauses 59-73;
   (c) a second gRNA molecule of any of clauses 74-96; and
   (d) nucleic acid that encodes one or more of (b) and (c).
194. The kit of clause 193, comprising nucleic acid that encodes one or more of (a), (b) and (c).
195. The kit of clause 194, further comprising a third gRNA molecule targeting a T cell target position.
196. The kit of clause 195, further comprising a fourth gRNA molecule targeting a T cell target position.
197. A gRNA of any of clauses 1-35, wherein the gRNA comprises a modification at or near its 5' end (e.g., within 1-10, 1-5, or 1-2 nucleotides of its 5' end).
198. A gRNA of any of clauses 1-35, wherein the gRNA comprises a modification at or near its 3' end (e.g., within 1-10, 1-5, or 1-2 nucleotides of its 3' end).
199. A gRNA of any of clauses 1-35, wherein the gRNA comprises a modification at or near its 5' end (e.g., within 1-10, 1-5, or 1-2 nucleotides of its 5' end) and a modification at or near its 3' end (e.g., within 1-10, 1-5, or 1-2 nucleotides of its 3' end).
200. A gRNA of any of clauses 197-199, wherein the modification causes the gRNA to exhibit increase stability towards nucleases when introduced into a T cell.
201. A gRNA of any of clauses 197-199, wherein the modification causes the gRNA to exhibit a reduced innate immune response when introduced into a T cell.
202. A gRNA of clause 201, wherein the innate immune response involves the induction of cytokine expression.

## Claims

1. A gRNA for an *S. pyogenes* Cas9 comprising a first targeting domain that is complementary to a target sequence on a target nucleic acid comprising a TRAC gene, wherein the first targeting domain comprises a sequence that is the same as, or differs by no more than 3 nucleotides from, a targeting domain sequence from any one of SEQ ID NOs: 49274-49291.

2. The gRNA of claim 1, wherein the gRNA is a modular gRNA, or, wherein the gRNA is a unimolecular or chimeric gRNA.

3. The gRNA of any one of the preceding claims, wherein the gRNA comprises a 3' polyA tract.

4. A composition comprising the gRNA of any one of claims 1-3.

5. The composition of claim 4, further comprising an *S. pyogenes* Cas9.

6. A composition comprising a ribonucleoprotein (RNP) complex comprising the composition of claim 5.

7. The composition of any one of claims 4-6, further comprising a second gRNA comprising a second targeting domain that is complementary to a target sequence on a nucleic acid comprising a gene selected from the group consisting of FAS, *BID,* CTLA4, PDCD1, CBLB, PTPN6, and TRBC.

8. The composition of claim 7, wherein the gene is a TRBC gene.

9. The gRNA according to any one of claims 1-3, or the composition of any one of claims 4-8, for use in treating a subject suffering from cancer.

10. An engineered T cell comprising the composition of any one of claims 6-8.

11. The engineered T cell of claim 10 for use in cancer immunotherapy.

12. A modified cell comprising a modification in the TRAC gene made by delivering the composition of claim 6.

13. The modified cell of claim 12, wherein the modification comprises a deletion or mutation of all or part of the TRAC gene.

14. The modified cell of claim 12, wherein expression of TRAC in the modified cell is modulated.

15. The modified cell of claim 12, wherein the cell is a T-cell, or, wherein the cell is a lymphoid progenitor cell, a hematopoietic stem cell, a natural killer cell, or a dendritic cell.
